# EUROPEAN PATENT SPECIFICATION

(11) **EP 1 307 226 B1**
(45) Date of publication and mention of the grant of the patent: **19.11.2008**
(21) Application number: 01952180.6
(22) Date of filing: 21.06.2001
(51) Int. Cl.: A61K 41/00, A61K 51/04, A61P 35/00

(54) **VITRONECTIN RECEPTOR ANTAGONIST PHARMACEUTICALS**
VITRONECTIN REZEPTOR ANTAGONIST PHARMAKA
AGENTS PHARMACEUTIQUES ANTAGONISTES DU RECEPTEUR DE VITRONECTINE

(30) Priority: 21.06.2000 US 213210 P
(43) Date of publication of application: 07.05.2003
(73) Proprietor: Lantheus Medical Imaging, Inc., North Billerica, MA 01862 (US)
(72) Inventor: HARRIS, Thomas, D., Salem, NH 03079 (US); BARRETT, John, A., Groton, MA 01450 (US); CARPENTER, Alan, P., Jr., Carlisle, MA 01741 (US); RAJOPADHYE, Milind, Westford, MA 01886 (US)
(74) Representative: Howard, Paul Nicholas
(86) International application number: PCT/US2001/019793
(87) International publication number: WO 2001/097848

(56) References cited:
- WO-A-00/35492
- WO-A-01/97860
- WO-A-01/97861
- WO-A-01/98294
- WO-A-02/04030
- WO-A-99/59640
- KAWAI KAZUYOSHI ET AL: "Enhancement of anticancer effects of radiation and conventional anticancer agents by a quinolinone derivative, vesnarinone: Studies on human gastric cancer tissue xenografts in nude mice." ANTICANCER RESEARCH, vol. 18, no. 1A, January 1998 (1998-01), pages 405-412, XP008009504 ISSN: 0250-7005
- HARRIS T D ET AL: "Radiolabeled indazole-based alphavbeta3 antagonists as potential tumor imaging agents." JOURNAL OF LABELLED COMPOUNDS AND RADIOPHARMACEUTICALS, vol. 44, no. Supplement 1, May 2001 (2001-05), pages S60-S62, XP008009503 Fourteenth International Symposium on Radiopharmaceutical Chemistry;Interlaken, Switzerland; June 10-15, 2001 ISSN: 0362-4803

## Description

### FIELD OF THE INVENTION

The present invention provides novel pharmaceuticals useful for the diagnosis and treatment of cancer, methods of imaging tumors in a patient, and methods of treating cancer in a patient. The invention is also directed to novel pharmaceutical compositions and combination therapy comprising a compound of the invention or a pharmaceutically acceptable salt thereof, and at least one agent selected from the group consisting of an anti-cancer agent and a radiosensitizer agent. In addition, the invention is directed to novel pharmaceutical compositions and combination therapy comprising a compound of the invention or a pharmaceutically acceptable salt thereof, and a photosensitizing agent. The pharmaceuticals are comprised of a targeting moiety that binds to the vitronectin receptor that is expressed in tumor vasculature, an optional linking group, and a therapeutically effective radioisotope or diagnostically effective imageable moiety. The therapeutically effective radioisotope emits a gamma ray or alpha particle sufficient to be cytotoxic. The imageable moiety is a,gamma ray or positron emitting radioisotope, a magnetic resonance imaging contrast agent, an X-ray contrast agent, or an ultrasound contrast agent.

### BACKGROUND OF THE INVENTION

Cancer is a major public health concern in the United States and around the world. It is estimated that over 1 million new cases of invasive cancer will be diagnosed in the United States in 1998. The most prevalent forms of the disease are solid tumors of the lung, breast, prostate, colon and rectum. Cancer is typically diagnosed by a combination of in vitro tests and imaging procedures. The imaging procedures include X-ray computed tomography, magnetic resonance imaging, ultrasound imaging and radionuclide scintigraphy. Frequently, a contrast agent is administered to the patient to enhance the image obtained by X-ray CT, MRI and ultrasound, and the administration of a radiopharmaceutical that localizes in tumors is required for radionuclide scintigraphy.

Treatment of cancer typically involves the use of external beam radiation therapy and chemotherapy, either alone or in combination, depending on the type and extent of the disease. A number of chemotherapeutic agents are available, but generally they all suffer from a lack of specificity for tumors versus normal tissues, resulting in considerable side-effects. The effectiveness of these treatment modalities is also limited, as evidenced by the high mortality rates for a number of cancer types, especially the more prevalent solid tumor diseases. More effective and specific treatment means continue to be needed.

Despite the variety of imaging procedures available for the diagnosis of cancer, there remains a need for improved methods. In particular, methods that can better differentiate between cancer and other pathologic conditions or benign physiologic abnormalities are needed. One means of achieving this desired improvement would be to administer to the patient a metallopharmaceutical that localizes specifically in the tumor by binding to a receptor expressed only in tumors or expressed to a significantly greater extent in tumors than in other tissue. The location of the metallopharmaceutical could then be detected externally either by its imageable emission in the case of certain radiopharmaceuticals or by its effect on the relaxation rate of water in the immediate vicinity in the case of magnetic resonance imaging contrast agents.

This tumor specific metallopharmaceutical approach can also be used for the treatment of cancer when the metallopharmaceutical is comprised of a particle emitting radioisotope. The radioactive decay of the isotope at the site of the tumor results in sufficient ionizing radiation to be toxic to the tumor cells. The specificity of this approach for tumors minimizes the amount of normal tissue that is exposed to the cytotoxic agent and thus may provide more effective treatment with fewer side-effects.

Previous efforts to achieve these desired improvements in cancer imaging and treatment have centered on the use of radionuclide labeled monoclonal antibodies, antibody fragments and other proteins or polypeptides that bind to tumor cell surface receptors. The specificity of these radiopharmaceuticals is frequently very high, but they suffer from several disadvantages. First, because of their high molecular weight, they are generally cleared from the blood stream very slowly, resulting in a prolonged blood background in the images. Also, due to their molecular weight they do not extravasate readily at the site of the tumor and then only slowly diffuse through the extravascular space to the tumor cell surface. This results in a very limited amount of the radiopharmaceutical reaching the receptors and thus very low signal intensity in imaging and insufficient cytotoxic effect for treatment.

Alternative approaches to cancer imaging and therapy have involved the use of small molecules, such as peptides, that bind to tumor cell surface receptors. An In-111 labeled somatostatin receptor binding peptide, In-111-DTPA-D-Phe¹-octeotide, is in clinical use in many countries for imaging tumors that express the somatostatin receptor (Baker, et al. Life Sci., 1991, 49, 1583-91 and Krenning, et al., Eur. J. Nucl. Med., 1993, 20, 716-31). Higher doses of this radiopharmaceutical have been investigated for potential treatment of these types of cancer (Krenning, et al., Digestion, 1996, 57, 57-61). Several groups are investigating the use of Tc-99m labeled ananlogs of In-111-DTPA-D-Phe¹-octeotide for imaging and Re-186 labeled analogs for therapy (Flanagan, et al., U.S. 5,556,939, Lyle, et al., U.S. 5,382,654, and Albert et al.,U.S. 5,650,134).

Angiogenesis is the process by which new blood vessels are formed from pre-existing capillaries or post capillary venules; it is an important component of a variety of physiological processes including ovulation, embryonic development, wound repair, and collateral vascular generation in the myocardium. It is also central to a number of pathological conditions such as tumor growth and metastasis, diabetic retinopathy, and macular degeneration. The process begins with the activation of existing vascular endothelial cells in response to a variety of cytokines and growth factors. Tumor released cytokines or angiogenic factors stimulate vascular endothelial cells by interacting with specific cell surface receptors for the factors. The activated endothelial cells secrete enzymes that degrade the basement membrane of the vessels. The endothelial cells then proliferate and invade into the tumor tissue. The endothelial cells differentiate to form lumens, making new vessel offshoots of pre-existing vessels. The new blood vessels then provide nutrients to the tumor permitting further growth and a route for metastasis.

Under normal conditions, endothelial cell proliferation is a very slow process, but it increases for a short period of time during embryogenesis, ovulation and wound healing. This temporary increase in cell turnover is governed by a combination of a number of growth stimulatory factors and growth suppressing factors. In pathological angiogenesis, this normal balance is disrupted resulting in continued increased endothelial cell proliferation. Some of the proangiogenic factors that have been identified include basic fibroblast growth factor (bFGF), angiogenin, TGF-alpha, TGF-beta, and vascular endothelium growth factor (VEGF). While interferon-alpha, interferon-beta and thrombospondin are examples of angiogenesis suppressors.

The proliferation and migration of endothelial cells in the extracellular matrix is mediated by interaction with a variety of cell adhesion molecules (Folkman, J., Nature Medicine , 1995, 1, 27-31). Integrins are a diverse family of heterodimeric cell surface receptors by which endothelial cells attach to the extracellular matrix, each other and other cells. The integrin αᵥβ₃ is a receptor for a wide variety for a wide variety of extracellular matrix proteins with an exposed tripeptide Arg-Gly-Asp moiety and mediates cellular adhesion to its ligand: vitronectin, fibronectin, and fibrinogen, among others. The integrin αᵥβ₃ is minimally expressed on normal blood vessels, but is significantly upregulated on vascular cells within a variety of human tumors. The role of the αᵥβ₃ receptors is to mediate the interaction of the endothelial cells and the extracellular matrix and facilitate the migration of the cells in the direction of the angiogenic signal, the tumor cell population. Angiogenesis induced by bFGF or TNF-alpha depend on the agency of the integrin αᵥβ₃, while angiogenesis induced by VEGF depends on the integrin αᵥβ₃ (Cheresh et. al., Science, 1955, 270, 1500-2). Induction of expression of the integrins α₁β₁ and α₂β₁ on the endothelial cell surface is another important mechanism by which VEGF promotes angiogenesis (Senger, et. al., Proc. Natl. Acad, Sci USA, 1997, 84, 13612-7).

Angiogenic factors interact with endothelial cell surface receptors such as the receptor tyrosine kinases EGFR, FGFR, PDGFR, Flk-1/KDR, Flt-1, Tek, tie, neuropilin-1, endoglin, endosialin, and Axl. The receptors Flk-1/KDR, neuropilin-1, and Flt-1 recognize VEGF and these interactions play key roles in VEGF-induced angiogenesis. The Tie subfamily of receptor tyrosine kinases are also expressed prominently during blood vessel formation.

Because of the importance of angiogenesis to tumor growth and metastasis, a number of chemotherapeutic approaches are being developed to interfere with or prevent this process. One of these approaches, involves the use of anti-angiogenic proteins such as angiostatin and endostatin. Angiostatin is a 38 kDa fragment of plasminogen that has been shown in animal models to be a potent inhibitor of endothelial cell proliferation. (O'Reilly et. al. , Cell, 1994, 79, 315-328) Endostatin is a 20 kDa C-terminal fragment of collagen XVIII that has also been shown to be a potent inhibitor. (O'Reilly et. al., Cell, 1997, 88, 277-285) Systemic therapy with endostatin has been shown to result in strong anti-tumor activity in animal models. However, human clinical trials of these two chemotherapeutic agents of biological origin have been hampered by lack of availability.

Another approach to anti-angiogenic therapy is to use targeting moieties that interact with endothelial cell surface receptors expressed in the angiogenic vasculature to which are attached chemotherapeutic agents. Burrows and Thorpe (Proc. Nat. Acad. Sci, USA, 1993, 90, 8996-9000) described the use of an antibody-immunotoxin conjugate to eradicate tumors in a mouse model by destroying the tumor vasculature. The antibody was raised against an endothelial cell class II antigen of the major histocompatibility complex and was then conjugated with the cytotoxic agent, deglycosylated ricin A chain. The same group (Clin. Can. Res., 1995, 1, 1623-1634) investigated the use of antibodies raised against the endothelial cell surface receptor, endoglin, conjugated to deglycosylated ricin A chain. Both of these conjugates exhibited potent anti-tumor activity in mouse models. However, both still suffer drawbacks to routine human use. As with most antibodies or other large, foreign proteins, there is considerable risk of immunologic toxicity which could limit or preclude administration to humans. Also, while the vasculature targeting may improve the local concentration of the attached chemotherapeutic agents, the agents still must be cleaved from the antibody carrier and be transported or diffuse into the cells to be cytotoxic.

Thus, it is desirable to provide anti-angiogenic pharmaceuticals and tumor or new vasculature imaging agents which do not suffer from poor diffusion or transportation, possible immunologic toxicity, limited availability, and/or a lack of specificity.

There continues to be a need for more effective treatment options for patients with solid tumors. This is especially true in cases of metastatic cancer in which current standard chemotherapy and external beam radiation regimens only result in marginal survival improvements.

Although improvements in cytotoxic chemotherapeutics have been made in recent years, the toxicity of these compounds to normal tissues has continued to severely limit their utility in extending survival in patients with solid tumors. Recently developed combinations of different therapeutic modalities, such as external beam irradiation and chemotherapy (i.e. chemoradiation), has provided some incremental benefit to the control of tumor progression and quality of life. Combination treatment of brachytherapy with laser therapy in treating carcinomas have already been reported (Allen et al., Am. J. Surg. 150:71, 1985; Schray et al., Int. J. Radiat. Oncol. Biol. Phys. 11:403, 1985--both incorporated in their entirety herein by reference), as well as the general use of brachytherapy in cancer by Nori et al., Surg. Clin. N. Amer. 67:1093, 1987, incorporated herein in its entirety by reference. However, neither systemic chemotherapeutics nor external beam irradiation have acceptable therapeutic indices, and are often limited due to unacceptable toxicity to normal tissues. The concept of combined therapy of cancer using anti-angiogenesis drugs in combination with chemotherapeutics is not new. Further, the concept of combining targeted in-vivo radiotherapy using radiolabeled antibodies and antibody fragments with chemotherapy has been reported (Stein R, Juweid M, Zhang C, et al., Clin. Cancer Res., 5: 3199s-3206s, 1999. However, the combination of a angiogenesis-targeted therapeutic radiopharmaceutical which is targeted to receptors, which are then upregulated in the neovasculature of tumors, together with chemotherapy has not been described before. Therefore, there is a need for a combination of a therapeutic radiopharmaceutical, which is targeted to localize in the neovasculature of tumors, with anti-cancer agentss or a radiosensitizer agent, or a pharmaceutically acceptable salt thereof, to provide additive or synergistic therapeutic response without unacceptable additive toxicity in the treatment of solid tumors.

The major advantage of combined chemotherapy and angiogenesis-targeted therapeutic radiopharmaceuticals, over each therapeutic modality alone, is improved tumor response without substantial increases in toxicity over either treatment alone. The advantage of using neovascular-specific radiopharmaceuticals, versus a tumor-cell targeted antibody, is that there is much lower systemic radiation exposure to the subject being treated.

Further, if the receptor targets for the radiopharmaceutical compounds, used in this method of treatment, are expressed on the luminal side of tumor vessels, there is no requirement that these compounds traverse the capillary bed and bind to the tumor itself.

Thus, it is desirable to provide a combination of angiogenesis-targeted therapeutic radiopharmaceuticals and an anti-cancer agents or a radiosensitizer agent, or a pharmaceutically acceptable salt thereof, which target the luminal side of the neovasculature of tumors, to provide a surprising, and enhanced degree of tumor suppression relative to each treatment modality alone without significant additive toxicity.

Photodynamic therapy has also been used in the treatment of cancer. Photodynamic therapy involves the administration of a photosensitive agent and subsequent irradiation with light to excite the photosensitizer, thus producing a cytotoxic effect. Spears, U.S. Pat. No. 4,512,762, and U.S. Pat. No. 4,566,636, Kelly, et al. United States Patent 6,235,767.

In photodynamic therapy, the photosensitizers used are capable of localizing in malignant cells, either by natural tendency or because they have been intentionally targeted to a specific type of tissue, or both. When irradiated, they may be capable of fluorescing and, thus, may also be useful in diagnostic methods related to detecting target tissue. However, even more importantly, the photosensitizer has the capacity, when irradiated with light at a wavelength which the compound absorbs, of causing a cytotoxic effect against whatever cells or other tissue in which the photosensitizer has localized.

In one form of this therapy, a photosensitizer agent having a characteristic light absorption waveband is first administered to the patient, typically either orally or by injection. Abnormal tissue in the body is known to selectively absorb certain photosensitizer agents to a much greater extent than normal tissue. More effective selectivity can be achieved using a photoreactive agent that is bound to an antibody, which links with antigens on targeted cells. The cancerous or abnormal tissue that has absorbed or linked with the photosensitizer dye is then destroyed by administering light of an appropriate wavelength or waveband corresponding to the absorption wavelength or waveband of the photosensitizer agent.

Photosensitizing agents such as Photofrin, a haematoporphyrin derivative, are known. (Dougherty, T. J. (1987) Photosensitizers: therapy and detection of malignant tumours. Photochem. Photobiol., 45, 879-889, and Boyle R. W. and D. David (1996) Structure and biodistribution relationships of photodynamic sensitizers. Photochem. Photobiol. 64, 469-485) Also, Rodgers,et al., United States Patent No. 6,225,333, discloses treating cancers with a variety of photosensitizing agents for example naphthalocyanine photosensitizing agents; tetrapyrrole-based photosensitizers; including porphyins; chlorins;, phthalocyanines; napthalocyanines; coumarins and psoralens. Furthermore, Mazur, et al., United States Patent No. 6,229,048, discloses a method for treatment of solid tumors by photodynamic therapy comprising administering a photosensitizer selected from the group consisting of: 1,3,4,6-tetrahydroxyhelianthrone; 1,3,4,6-tetramethoxyhelianthrone; 10,13-dimethyl-1,3,4,6-tetrahydroxyhelianthrone; 10,13-di(methoxycarbonyl)-1,3,4,6-tetramethoxyhelianthrone; 1,6-di-N-butylamino-3,4-dimethoxy-helianthrone; 1,6-diN-butylamino-3,4-dimethoxy-10,13-dimethyl-helianthrone; 1,6-di-(N-hydroxyethylamino)-3,4-dimethoxy-helianthrone; 2,5-dibromo-1,3,4,6-tetrahydroxyhelianthrone; and 2,5-dibromo-10,13-dimethyl-1,3,4,6-tetrahydroxyhelianthrone.

In another method, "green porphyrins" have been used in photodynamic therapy with light having a wavelength range around 670-780 nm. See for example, Levy et al., U.S. Pat. No. 5,399,583 Levy et al., U.S. Pat. No. 4,920,143, Levy et al., U.S. Pat. No. 5,095,030; and Levy et al., and U.S. Pat. No. 5,171,749.

In most photodynamic therapy protocols, a method must be found for the irradiating light to reach the targeted tissue where the photosensitizer has been localized. For example, a light-emitting balloon catheter may be used or alternatively, a form of "liquid light" may be injected into the vascular tree such that the "liquid light", perfuses the vasculature at the target site. Spears, U.S. Pat. No. 4,512,762. Alternatively

The targeted tissues are visually located by imaging the treatment site through a fiber optic system so that light from a laser source can be accurately directed through the optical fiber to destroy the abnormal tissue. Even when the internal treatment site is accessible through natural body orifices, an endoscope is usually required to visualize the targeted tissue and accurately direct the light therapy administered to the treatment site. Chen, United States Patent No. 6,210,425 discloses an apparatus and a method to identify an internal treatment site within a patient's body for administration of light therapy and treatment of the site.

Thus, it is also desirable to provide a combination of a photosensitizer agent (as part of photodynamic therapy), an angiogenesis-targeted therapeutic radiopharmaceutical and an anti-cancer agent or a radiosensitizer agent, or a pharmaceutically acceptable salt thereof, which target the luminal side of the neovasculature of tumors, to provide a surprising, and enhanced degree of tumor suppression relative to each treatment modality alone without significant additive toxicity.

Another application of anti-angiogenic therapy is in treating rheumatoid arthritis (RA). In RA, the ingrowth of a highly vascularized pannus is caused by the excessive production of angiogenic factors by the infiltrating macrophages, immune cells, or inflammatory cells. Therefore, it is desirable to have new pharmaceuticals to destroy the highly vascularized pannus that results and thus treat the disease.

There is also a growing interest in therapeutic angiogenesis to improve blood flow in regions of the body that have become ischemic or poorly perfused. Several investigators are using growth factors administered locally to cause new vasculature to form either in the limbs or the heart. The growth factors VEGF and bFGF are the most common for this application. Recent publications include: Takeshita, S., et. al., J. Clin. Invest., 1994, 93, 662-670; and Schaper, W. and Schaper, J., Collateral Circulation:Heart, Brain, Kidney, Limbs, Kluwer Academic Publishers, Boston, 1993. The main applications that are under investigation in a number of laboratories are for improving cardiac blood flow and in improving peripheral vessal blood flow in the limbs. For example, Henry, T. et. al. (J. Amer. College Cardiology, 1998, 31, 65A) describe the use of recombinant human VEGF in patients for improving myocardial perfusion by therapeutic angiogenesis. Patients received infusions of rhVEGF and were monitored by nuclear perfusion imaging 30 and 60 days post treatment to determine improvement in myocardial perfusion. About 50% of patients showed improvement by nuclear perfusion imaging whereas 5/7 showed new collatoralization by angiography. Thus, it is desirable to discover a method of monitoring improved cardiac blood flow which is targeted to new collatoral vessels themselves and not, as in nuclear perfusion imaging, a regional consequence of new collatoral vessels.

The detection, imaging and diagnosis of a number of cardiovascular diseases need to be improved, including restenosis, atherosclerosis, myocardial reperfusion injury, and myocardial ischemia, stunning or infarction. It has recently been determined that in all of these disease conditions, the integrin receptor αvβ3 plays an important role.

For example, in the restenosis complication that occurs in -30-50% of patients having undergone angioplasty or stent placement, neointimal hyperplasia and ultimate reocclusion is caused by aggressively proliferating vascular smooth muscle cells that express αvβ3. (Cardiovascular Res., 1997, 36, 408-428; DDT, 1997, 2, 187-199; Current Pharm. Design, 1997, 3, 545-584)

Atherosclerosis proceeds from an intial endothelial damage that results in the recruitment and subintimal migration of monocytes at the site of the injury. Growth factors are released which induce medial smooth muscle cells to proliferate and migrate to the intimal layer. The migrating smooth muscle cells express αvβ3.

In reperfusion injury, neutrophil transmigration is integrin dependent and the integrins moderate initial infiltration into the viable border zone. The induction of α5β1, α4β1 and αvβ5 in infiltrating neutrophils occurs within 3 to 5 hours after reperfusion as neutrophils move from the border zone to the area of necrosis. (Circulation, 1999, 100, I-275)

Acute or chronic occlusion of a coronary artery is known to result in angiogenesis in the heart as native collateral vessels are recruited to attempt to relieve the ischemia. However, even a gradual occlusion usually results in areas of infarction as the resulting angiogenesis is not sufficient to prevent damage. Cardiac angiogenesis has been associated with increased expression of the growth factors VEGF and FGF and the upregulation of the growth factor receptors flt-1 and flk-1/KDR. (Drugs, 1999, 58, 391-396).

### SUMMARY OF THE INVENTION

It is one object of the present invention to provide improved anti-angiogenic pharmaceuticals, comprised of a targeting moiety that binds to the vitronectin receptor that is expressed in tumor neovasculature, an optional linking group, and a radioisotope. The vitronectin receptor binding compounds target the radioisotope to the tumor neovasculature. The beta or alpha-particle emitting radioisotope emits a cytotoxic amount of ionizing radiation which results in cell death. The penetrating ability of radiation obviates the requirement that the cytotoxic agent diffuse or be transported into the cell to be cytotoxic.

It is another object of the present invention to provide pharmaceuticals to treat rheumatoid arthritis. These pharmaceuticals comprise a targeting moiety that binds to a receptor that is upregulated during angiogenesis, an optional linking group, and a radioisotope that emits cytotoxic radiation (i.e., beta particles, alpha particles and Auger or Coster-Kronig electrons). In rheumatoid arthritis, the ingrowth of a highly vascularized pannus is caused by the excessive production of angiogenic factors by the infiltrating macrophages, immune cells, or inflammatory cells. Therefore, the radiopharmaceuticals of the present invention that emit cytotoxic radiation could be used to destroy the new angiogenic vasculature that results and thus treat the disease.

It is another object of the present invention to provide kits and therapeutic radiopharmacutical compositions for use in combination therapy comprising a radiopharmacutical of the invention and at least one agents selected from the group consisting of an anticancer agent and a radiosensitizer agent.

It is another object of the present invention to provide kits and therapeutic radiopharmacutical compositions for use in combination therapy comprising a radiopharmacutical of the invention and a photosensitising agent.

It is another object of the present invention to provide for the use of a therapeutic radiopharmaceutical composition of the invention for the manufacture of a medicament to be administered to a patient in need thereof, in combination with photodynamic therapy, for treating cancer.

It is another object of the present invention to provide compounds useful for preparing the pharmaceuticals of the present invention. These compounds are comprised of a non-peptide quinolone containing targeting moiety that binds to a receptor that is upregulated during angiogenesis or during cardiovascular diseases, Q, an optional linking group, Lₙ, and a metal chelator or bonding moiety, Cₕ. The compounds may have one or more protecting groups attached to the metal chelator or bonding moiety. The protecting groups provide improved stability to the reagents for long-term storage and are removed either immediately prior to or concurrent with the synthesis of the radiopharmaceuticals. Alternatively, the compounds of the present invention are comprised of a peptide or peptidomimetic targeting moiety that binds to a receptor that is upregulated during angiogenesis or during cardiovascular diseases, Q, an optional linking group, Lₙ, and a surfactant, S_{f}.

The pharmaceuticals of the present invention may be used for diagnostic and/or therapeutic purposes. Diagnostic radiopharmaceuticals of the present invention are pharmaceuticals comprised of a diagnostically useful radionuclide (i.e., a radioactive metal ion that has imageable gamma ray or positron emissions). Therapeutic radiopharmaceuticals of the present invention are pharmaceuticals comprised of a therapeutically useful radionuclide, a radioactive metal ion that emits ionizing radiation such as beta particles, alpha particles and Auger or Coster-Kronig electrons.

The pharmaceuticals comprising a gamma ray or positron emitting radioactive metal ion are useful for imaging tumors and by gamma scintigraphy or positron emission tomography. The pharmaceuticals comprising a gamma ray or positron emitting radioactive metal ion are also useful for imaging therapeutic angiogenesis, natural angiogenic processes in response to acute or chronic coronary vessel occlusion, restenosis post-angioplasty, atherosclerosis and plaque formation, and reperfusion injury by gamma scintigraphy or positron emission tomography. The pharmaceuticals comprising a particle emitting radioactive metal ion are useful for treating cancer by delivering a cytotoxic dose of radiation to the tumors. The pharmaceuticals comprising a particle emitting radioactive metal ion are also useful for treating rheumatoid arthritis by destroying the formation of angiogenic vasculature. The pharmaceuticals comprising a paramagnetic metal ion are useful as magnetic resonance imaging contrast agents. The pharmaceuticals comprising one or more X-ray absorbing or "heavy" atoms of atomic number 20 or greater are useful as X-ray contrast agents. The pharmaceuticals comprising a microbubble of a biocompatible gas, a liquid carrier, and a surfactant microsphere, are useful as ultrasound contrast agents.

### DETAILED DESCRIPTION OF THE INVENTION

[1] Thus, in a first embodiment, the present invention provides a kit for treating cancer, comprising a compound of the formula (I) and at least one agent selected from the group consisting of an anti-cancer agent and a radiosensitizer agent, or a pharmaceutically acceptable salt thereof, and a pharmaceutically acceptable carrier, wherein the compound of the formula (I) is:

   (Q)_{d}-Lₙ-Cₕ or (Q)_{d}-Lₙ-(Cₕ)_{d'} (I)

   wherein, Q is a compound of Formula (II): 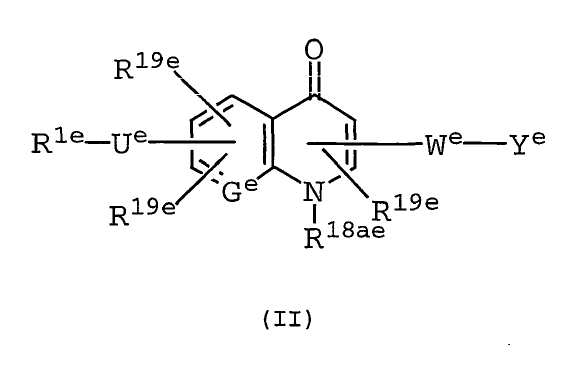 including stereoisomeric forms thereof, or mixtures of stereoisomeric forms thereof, or pharmaceutically acceptable salt or prodrug forms thereof wherein:
   - R^{1e}: is selected from: 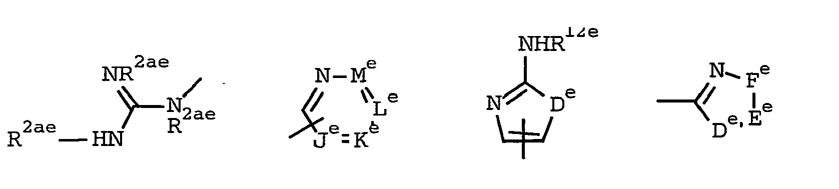 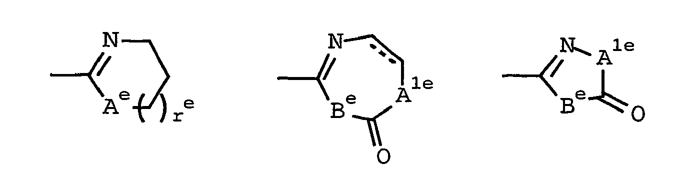 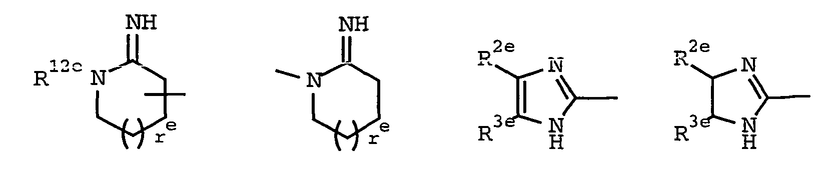 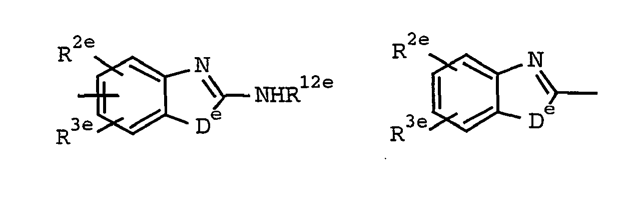 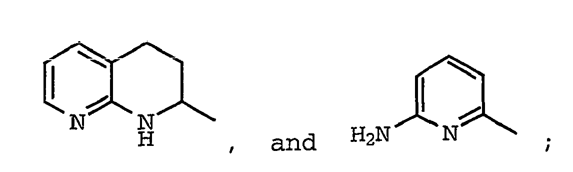
   - A^{e}: is -CH₂- or -N(R^{10e})-;
   - A^{1e} and B^{e}: are independently -CH₂- or -N(R^{10e})-;
   - D^{e}: is -N(R^{10e})- or -S-;
   - E^{e}-F^{e}: is -C(R^{2e})=C(R^{3e})- or -C(R^{2e})₂C(R^{3e})₂-;
   - J^{e}: is -C(R^{2e})- or -N-;
   - K^{e}, L^{e} and M^{e}: are independently -C(R^{2e})- or -C(R^{3e})-;
   - R^{2e} and R^{3e}: are independently selected from: H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
   alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano amino, CF₃ and NO₂;
   - R^{2ae}: is selected from:
   H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
   - R^{7e}: is selected from:
   H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
   - U^{e}: is selected from:
   -(CH₂)ₙ^{e}-, -(CH₂)ₙ^{e}O(CH₂)ₘ^{e}-, - (CH₂)ₙ^{e}N(R¹²)(CH₂)ₘ^{e}-, -NH(CH₂)ₙ^{e}-, - (CH₂)ₙ^{e}C(=O)(CH₂)ₘ^{e}-, -(CH₂)ₙ^{e}S(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})-, and -N(R^{10e})S(O)ₚ^{e}-;
   - G^{e}: is N or CR^{19e};
   - W^{e}: is-C(=O)-N(R^{10e})-(C₁-C₃ alkylene)-, in which the alkylene group is substituted by R^{8e} and by R^{9e}:
   - R^{8e} and R^{9e}: are independently selected from:
   H, CO₂R^{18be}, C(=O)R^{18be}, CONR¹⁷R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl,
   C₃-C₁₀ cycloalkyl(C₁-C₄ alkyl)-, phenyl substituted with 0-3 R^{6e}, naphthyl substituted with 0-3 R^{6e},
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e},
   C₁-C₁₀ alkoxy substituted with 0-2 R^{7e}, hydroxy, nitro, -N(R^{10e})R^{11e}, -N(R^{16e})R^{17e}, aryl(C₀-C₆ alkyl)carbonyl, aryl(C₃-C₆ alkyl), heteroaryl(C₁-C₆ alkyl), CONR^{18ae}R^{20e}, SO₂R^{18ae}, and SO₂NR^{18ae}R^{20e},
   providing that any of the above alkyl, cycloalkyl, aryl or heteroaryl groups may be unsubstituted or substituted independently with 1-2 R^{7e};
   - R^{6e}: is selected from:
   H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
   aryl substituted with 0-3 groups selected from halogen, C₁-C₆ alkoxy, C₁-C₆ alkyl, CF₃, S(O)ₘ^{e}Me, and -NMe₂,
   aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   - R^{10e}: is selected from:
   H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
   - R^{11e}: is selected from:
   H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl (C₁-C₄ alkyl), adamantylmethyl, and C₁-C₁₀ alkyl substituted with 0-2 R^{4e};
   - R^{4e}: is selected from:
   H, C₁-C₆ alkyl, C₃-C₇ cycloalkyl, C₃-C₇ cycloalkyl(C₁-C₄ alkyl)-, (C₁-C₁₀ alkyl)carbonyl, aryl, heteroaryl, aryl (C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
   alternatively, when R^{10e} and R^{11e} are both substituents on the same nitrogen atom (as in -NR^{10e}R^{11e}) they may be taken together with the nitrogen atom to which they are attached to form a heterocycle selected from: 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl, and 1-piperazinyl;
   said heterocycle being substituted with 0-3 groups selected from: C₁-C₆ alkyl, aryl, heteroaryl, aryl(C₁-C₄ alkyl)-, (C₁-C₆ alkyl)carbonyl, (C₃-C₇ cycloalkyl)carbonyl, (C₁-C₆ alkoxy)carbonyl, aryl(C₁-C₄ alkoxy)carbonyl, C₁-C₆ alkylsulfonyl, and arylsulfonyl;
   - R^{12e}: is selected from:
   H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy)carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
   - R^{16e}: is selected from:
   -C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, and -SO₂NHC(=O)OR^{18be};
   - R^{17e}: is selected from:
   H, C₁-C₆ alkyl, C₃-C₇ cycloalkyl, C₃-C₇ cycloalkyl(C₁-C₄ alkyl)-, aryl, aryl(C₁-C₆ alkyl)-, and heteroaryl(C₁-C₆ alkyl);
   - R^{18ae}: is selected from:
   C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, biaryl optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
   - R^{18be}: is H or R^{18ae},
   - R^{19e}: is selected from:
   H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
   - R^{20e}: is selected from:
   hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
   (5-(C₁-C₅ alkyl)-1,3-dioxa-cyclopenten-2-one-yl)methyloxy,
   (5-aryl-1,3-dioxa-cyclopenten-2-one-yl)methyloxy, and
   (R^{10e})(R^{11e})N-(C₁-C₁₀ alkoxy)-;
   - R^{21e}: is selected from:
   C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and C₁-C₁₀ alkyl substituted with 0-2 R^{7e};
   - R^{22e}: is selected from:
   -C(=O)-R^{18be}, -C(=O)N(R^{18be})₂, -C(=O)NHSO₂R^{18ae},-C(=O)NHC(=O)R^{18be}, -C(=O)NHC(=O)OR^{18ae}, and-C(=O)NHSO₂NHR^{18be};
   - Y^{e}: is selected from:
   -COR^{20e}, -SO₃H, -PO₃H, -CONHNHSO₂CF₃,-CONHSO₂R^{18ae}, -CONHSO₂NHR^{18be}, -NHCOCF₃,-NHCONHSO₂R^{18ae}, -NHSO₂R^{18ae}, -OPO₃H₂, -OSO₃H,-PO₃H₂, -SO₂NHCOR^{18ae},
   -SO₂NHCO₂R^{18ae}, 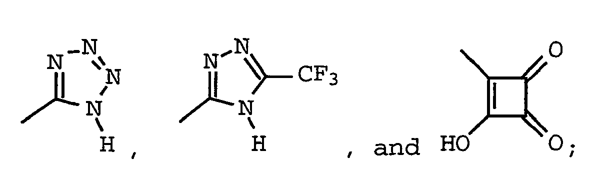
   - m^{e}: is 0-2;
   - n^{e}: is 0-4;
   - p^{e}: is 0-2;
   - r^{e}: is 0-2;
   with the following proviso: n^{e} and m^{e} are chosen such that the number of atoms connecting R^{1e} and Y^{e} is in the range of 8-14;
   - d: is selected from 1, 2, 3, 4, 5, 6, 7, 8, 9, and 10;
   - d': is 1-100;
   - Lₙ: is a linking group having the formula:

   ((W)ₕ-(CR⁶R⁷)_{g})ₓ-(Z)ₖ-((CR^{6a}R^{7a})_{g'}-(W)_{h'})_{x'};
   - W: is independently selected at each occurrence from the group: O, S, NH, NHC(=O), C(=O)NH, NR⁸C(=O), C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ, and (aa)_{t'};
   - aa: is independently at each occurrence an amino acid;
   - Z: is selected from the group: aryl substituted with 0-3 R¹⁰, C₃₋₁₀ cycloalkyl substituted with 0-3 R¹⁰, and a 5-10 membered heterocyclic ring system containing 1-4 heteroatoms independently selected from N, S, and O and substituted with 0-3 R¹⁰;
   - R⁶, R^{6a}, R⁷, R^{7a}, and R⁸: are independently selected at each occurrence from the group: H, =O, COOH, SO₃H, P03H, C₁-C₅ alkyl substituted with 0-3 R¹⁰, aryl substituted with 0-3 R¹⁰, benzyl substituted with 0-3 R¹⁰, and C₁-C₅ alkoxy substituted with 0-3 R¹⁰, NHC(=O)R¹¹, C(=O)NHR¹¹, NHC(=O)NHR¹¹, NHR¹¹, R¹¹, and a bond to Cₕ;
   - R¹⁰: is independently selected at each occurrence from the group: a bond to Cₕ, COOR¹¹, C(=O)NHR¹¹, NHC(=O)R¹¹, OH, NHR¹¹, SO₃H, PO₃H, -OPO₃H₂, -OSO₃H, aryl substituted with 0-3 R¹¹, C₁₋₅ alkyl substituted with 0-1 R¹², C₁₋₅ alkoxy substituted with 0-1 R¹², and a 5-10 membered heterocyclic ring system containing 1-4 heteroatoms independently selected from N, S, and 0 and substituted with 0-3 R¹¹;
   - R¹¹: is independently selected at each occurrence from the group: H, -OPO₃H₂,, -OSO₃H C₁₋₅ alkyl substituted with 0-1 R¹², aryl substituted with 0-1 R¹², a 5-10 membered heterocyclic ring system containing 1-4 heteroatoms independently selected from N, S, and O and substituted with 0-1 R¹², C₃₋₁₀ cycloalkyl substituted with 0-1 R¹², polyalkylene glycol substituted with 0-1 R¹², C₁₋₅ alkyl substituted with carbohydrate substituted with 0-1 R¹², cyclodextrin substituted with 0-1 R¹², amino acid substituted with 0-1 R¹², polycarboxyalkyl substituted with 0-1 R¹², polyazaalkyl substituted with 0-1 R¹², peptide substituted with 0-1 R¹², wherein the peptide is comprised of 2-10 amino acids, C₁₋₅ alkyl substituted with 3,6-O-disulfo-B-D-galactopyranosyl, bis(phosphonomethyl)glycine, and a bond to Cₕ;
   - R¹²: is a bond to Cₕ;
   - k: is selected from 0, 1, and 2;
   - h: is selected from 0, 1, and 2;
   - h': is selected from 0, 1, and 2;
   - g: is selected from 0, 1, 2, 3, 4, 5, 6, 7, 8, 9, and 10;
   - g': is selected from 0, 1, 2, 3, 4, 5, 6, 7, 8, 9, and 10;
   - s: is selected from 0, 1, 2, 3, 4, 5, 6, 7, 8, 9, and 10;
   - s': is selected from 0, 1, 2, 3, 4, 5, 6, 7, 8, 9, and 10;
   - s": is selected from 0, 1, 2, 3, 4, 5, 6, 7, 8, 9, and 10;
   - t: is selected from 0, 1, 2, 3, 4, 5, 6, 7, 8, 9, and 10;
   - t': is selected from 0, 1, 2, 3, 4, 5, 6, 7, 8, 9, and 10;
   - x: is selected from 0, 1, 2, 3, 4, 5, and 6;
   - x': is selected from 0, 1, 2, 3, 4, 5 and 6;
   - Cₕ: is a metal bonding unit having a formula selected from the group: 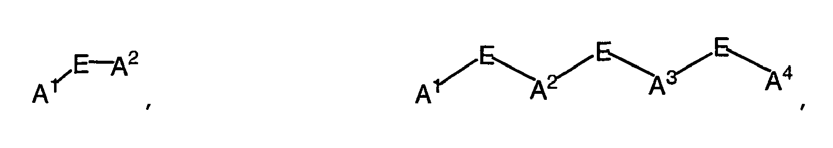 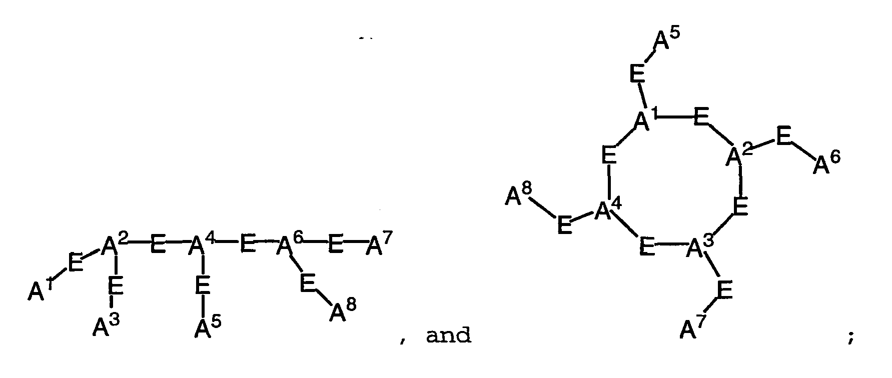
   - A¹, A², A³, A⁴, A⁵, A⁶, A⁷, and A⁸: are independently selected at each occurrence from the group: NR¹³, NR¹³R¹⁴, S, SH, S (Pg), 0, OH, PR¹³, PR¹³R¹⁴, P(O)R¹⁵R¹⁶, and a bond to Lₙ;
   - E: is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
   - R¹³: and R¹⁴ are each independently selected from the group: a bond to Lₙ, hydrogen, C₁-C₁₀ alkyl substituted with 0-3 R¹⁷, aryl substituted with 0-3 R¹⁷, C₁₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
   alternatively, R¹³ and R¹⁴ combine to form =C(R²⁰)(R²¹);
   - R¹⁵ and R¹⁶: are each independently selected from the group; a bond to Lₙ, -OH, C₁-C₁₀ alkyl substituted with 0-3 R¹⁷, C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
   - R¹⁷: is independently selected at each occurrence from the group: a bond to Lₙ, =0, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H,
   2-(1-morpholino)ethoxy, C₁-C₅ alkyl, C₂-C₄ alkenyl, C₃-C₆ cycloalkyl, C₃-C₆ cycloalkylmethyl, C₂-C₆ alkoxyalkyl, aryl substituted with 0-2 R¹⁸, and a 5-10 membered heterocyclic ring system containing 1-4 heteroatoms independently selected from N, S, and O;
   - R¹⁸, R^{18a}, and R¹⁹: are independently selected at each occurrence from the group: a bond to Lₙ, H, C₁-C₆ alkyl, phenyl, benzyl, C₁-C₆ alkoxy, halide, nitro, cyano, and trifluoromethyl;
   - Pg: is a thiol protecting group;
   - R²⁰ and R²¹: are independently selected from the group: H, C₁-C₁₀ alkyl, -CN, -CO₂R²⁵, -C(=O)R²⁵, -C(=O)N(R²⁵)₂, C₂-C₁₀ 1-alkene substituted with 0-3 R²³, C₂-C₁₀ 1-alkyne substituted with 0-3 R²³, aryl substituted with 0-3 R²³, unsaturated 5-10 membered heterocyclic ring system containing 1-4 heteroatoms independently selected from N, S, and 0 and substituted with 0-3 R²³, and unsaturated C₃₋₁₀ carbocycle substituted with 0-3 R²³;
   alternatively, R²⁰ and R²¹, taken together with the divalent carbon radical to which they are attached form: 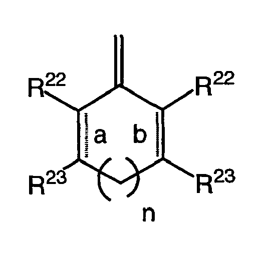
   - R²² and R²³: are independently selected from the group: H, R²⁴, C₁-C₁₀ alkyl substituted with 0-3 R²⁴, C₂-C₁₀ alkenyl substituted with 0-3 R²⁴, C₂-C₁₀ alkynyl substituted with 0-3 R²⁴, aryl substituted with 0-3 R²⁴, a 5-10 membered heterocyclic ring system containing 1-4 heteroatoms independently selected from N, S, and 0 and substituted with 0-3 R²⁴, and C₃₋₁₀ carbocycle substituted with 0-3 R²⁴;
   alternatively, R²², R²³ taken together form a fused aromatic or a 5-10 membered heterocyclic ring system containing 1-4 heteroatoms independently selected from N, S, and O;
   - a and b: indicate the positions of optional double bonds and n is 0 or 1;
   - R²⁴: is independently selected at each occurrence from the group: =O, F, Cl, Br, I, -CF₃, -CN, -CO₂R²⁵, -C(=O)R²⁵, -C(=O)N(R²⁵)₂, -N(R²⁵)₃+, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, and
   2-(1-morpholino)ethoxy; and,
   - R²⁵, R^{25a}, and R²⁶: are each independently selected at each occurrence from the group: hydrogen and C₁-C₆ alkyl; or a pharmaceutically acceptable salt thereof.
[2] In another embodiment, the present invention provides a kit according to Embodiment [1] wherein said kit comprises a plurality of separate containers,
   wherein at least one of said containers contains one or more agents selected from the group consisting of an anti-cancer agent and a radiosensitizer agent, or a pharmaceutically acceptable salt thereof, and a pharmaceutically acceptable carrier, and another of said containers contains a compound of formula (I):

   (Q)_{d}-Lₙ-Cₕ or (Q)_{d}-Lₙ-(Cₕ)_{d'} (I)

   wherein, Q is a compound of Formula (II): 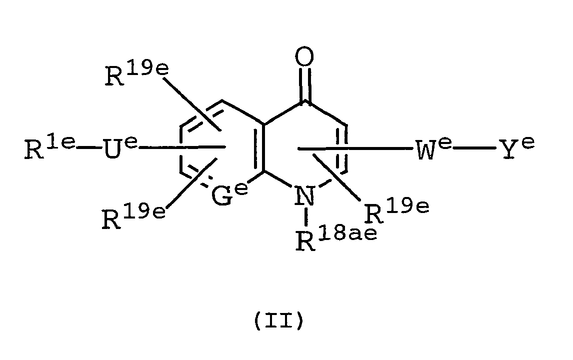 including stereoisomeric forms thereof, or mixtures of stereoisomeric forms thereof, or pharmaceutically acceptable salt or prodrug forms thereof wherein:
   - R^{1e}: is selected from: 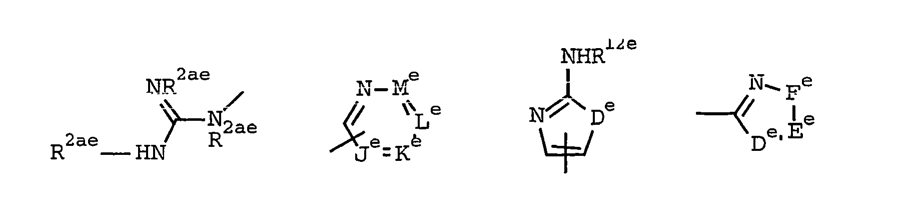 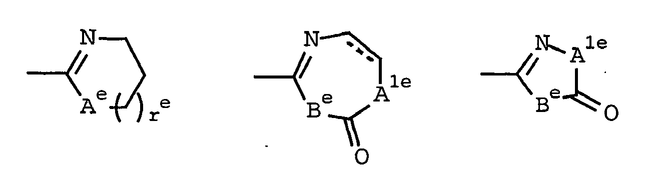 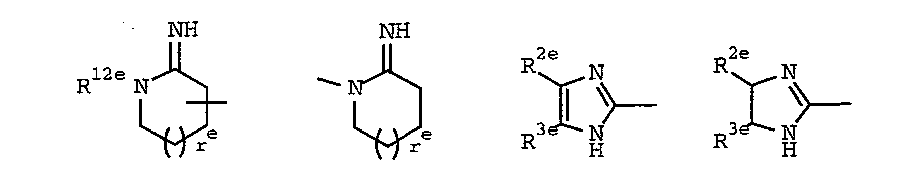 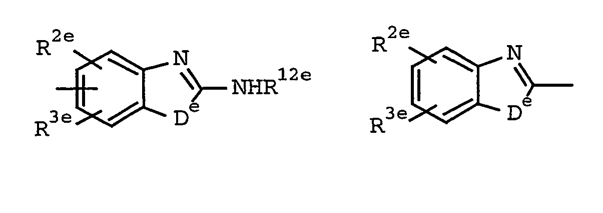 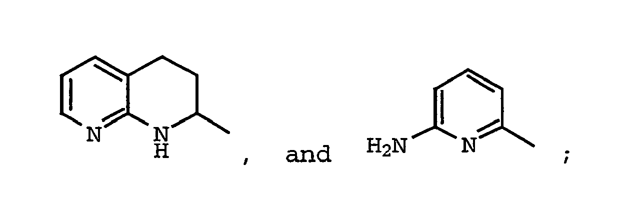
   - A^{e}: is -CH₂- or -N(R^{10e})-;
   - A^{1e} and B^{e}: are independently -CH₂- or -N(R^{10e})-;
   - D^{e}: is -N(R^{10e})- or -S-;
   - E^{e}-F^{e}: is -C(R^{2e})=C(R^{3e})- or -C(R^{2e})₂C(R^{3e})₂-;
   - J^{e}: is -C(R^{2e}) - or -N- ;
   - K^{e}, L^{e} and M^{e}: are independently -C(R^{2e})- or -C(R^{3e})-;
   - R^{2e} and R^{3e}: are independently selected from:
   H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
   alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
   - R^{2ae}: is selected from:
   H; C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
   - R^{7e}: is selected from:
   H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
   - U^{e}: is selected from:
   -(CH₂)ₙ^{e}-, -(CH₂)ₙ^{e}O(CH₂)ₘ^{e}-,-(CH₂)ₙ^{e}N(R¹²)(CH₂)ₘ^{e}-, -NH(CH₂)ₙ^{e}-,-(CH₂)ₙ^{e}C(=O)(CH₂)ₘ^{e}-,
   -(CH₂)ₙ^{e}S(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})-, and -N(R^{10e})S(O)ₚ^{e}-;
   - G^{e}: is N or CR^{19e};
   - W^{e}: is-C(=O)-N(R^{10e})-(C₁-C₃ alkylene)-, in which the alkylene group is substituted by R^{8e} and by R^{9e}:
   - R^{8e} and R^{9e}: are independently selected from:
   H, CO₂R^{18be}, C(=O)R^{18be}, CONR¹⁷R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl,
   C₃-C₁₀ cycloalkyl (C₁-C₄ alkyl) -, phenyl substituted with 0-3 R^{6e}, naphthyl substituted with 0-3 R^{6e},
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e},
   C₁-C₁₀ alkoxy substituted with 0-2 R^{7e}, hydroxy, nitro, -N(R^{10e})R^{11e}, -N(R^{16e})R^{17e}, aryl(C₀-C₆ alkyl)carbonyl, aryl(C₃-C₆ alkyl), heteroaryl(C₁-C₆ alkyl), CONR^{18ae}R^{20e}, SO₂R^{18ae}, and SO₂NR^{18ae}R^{20e},
   providing that any of the above alkyl, cycloalkyl, aryl or heteroaryl groups may be unsubstituted or substituted independently with 1-2 R^{7e};
   - R^{6e}: is selected from:
   H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=C)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
   aryl substituted with 0-3 groups selected from halogen, C₁-C₆ alkoxy, C₁-C₆ alkyl, CF₃, S(O)ₘ^{e}Me, and -NMe₂,
   aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   - R^{10e}: is selected from:
   H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
   - R^{11e}: is selected from:
   H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
   - R^{4e}: is selected from:
   H, C₁-C₆ alkyl, C₃-C₇ cycloalkyl, C₃-C₇ cycloalkyl(C₁-C₄ alkyl)-, (C₁-C₁₀ alkyl)carbonyl, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
   alternatively, when R^{10e} and R^{11e} are both substituents on the same nitrogen atom (as in -NR^{10e}R¹¹e) they may be taken together with the nitrogen atom to which they are attached to form a heterocycle selected from: 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl, and 1-piperazinyl;
   said heterocycle being substituted with 0-3 groups selected from: C₁-C₆ alkyl, aryl, heteroaryl, aryl(C₁-C₄ alkyl)-, (C₁-C₆ alkyl)carbonyl, (C₃-C₇ cycloalkyl)carbonyl, (C₁-C₆ alkoxy)carbonyl, aryl(C₁-C₄ alkoxy)carbonyl, C₁-C₆ alkylsulfonyl, and arylsulfonyl;
   - R^{12e}: is selected from:
   H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy)carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
   - R^{16e}: is selected from:
   -C(=O)OR^{18ae}, -C(=O)R^{18be}, -C (=O)N (R^{18be})₂, -C(=O)NHSO₂R^{18ae}, -C(=O)NHC(=O)R^{18be}, -C (=O) NHC (=O)OR^{18ae}, -C(=O)NHSO₂NHR^{18be}, -SO₂R^{18ae}, -SO₂N(R^{18be})₂, and -SO₂NHC(=O)OR^{18be};
   - R^{17e}: is selected from:
   H, C₁-C₆ alkyl, C₃-C₇ cycloalkyl, C₃-C₇ cycloalkyl(C₁-C₄ alkyl)-, aryl, aryl(C₁-C₆ alkyl)-, and heteroaryl(C₁-C₆ alkyl);
   - R^{18ae}: is selected from:
   C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e}.
   - R^{18be}: is H or R^{18ae},
   - R^{19e}: is selected from:
   H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆-alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
   - R^{20e}: is selected from:
   hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
   (5-(C₁-C₅ alkyl)-1,3-dioxa-cyclopenten-2-one-yl)methyloxy.
   (5-aryl-1,3-dioxa-cyclopenten-2-one-yl)methyloxy, and
   (R^{10e})(R^{11e})N-(C₁-C₁₀ alkoxy)-;
   - R^{21e}: is selected from:
   C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and C₁-C₁₀ alkyl substituted with 0-2 R^{7e};
   - R^{22e}: is selected from:
   -C(=O)-R^{18be}, -C(=O)N(R^{18be})₂, -C(=O)NHSO₂R^{18ae}, C(=O)NHC(=O)R^{18be}, -C(=O)NHC(=O)OR^{18ae}, and C(=O)NHSO₂NHR^{18be};
   - Y^{e}: is selected from:
   -COR^{20e}, -SO₃H, -PO₃H, -CONHNHSO₂CF₃,-CONHSO₂R^{18ae}, -CONHSO₂NHR^{18be}, -NHCOCF₃,-NHCONHSO₂R^{18ae}, -NHSO₂R^{18ae}, -OPO₃H₂, -OSO₃H,-PO3H₂, -SO₂NHCOR^{18ae},
   -SO₂NHCO₂R^{18ae}, 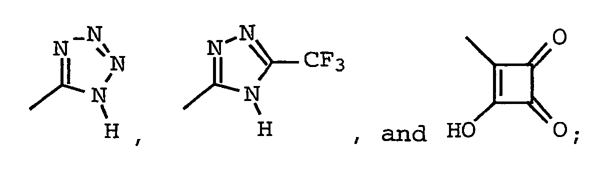
   - m^{e}: is 0-2;
   - n^{e}: is 0-4;
   - pe: is 0-2;
   - r^{e}: is 0-2;
   with the following proviso: n^{e} and m^{e} are chosen such that the number of atoms connecting R^{1e} and Y^{e} is in the range of 8-14;
   - d: is selected from 1, 2, 3, 4, 5, 6, 7, 8, 9, and 10;
   - d': is 1-100;
   - Lₙ: is a linking group having the formula:

   ((W)ₕ- (CR⁶R⁷)_{g})ₓ-(Z)ₖ-((CR^{6a}R^{7a})g'-(W)ₕ')ₓ';
   - W: is independently selected at each occurrence from the group: O, S, NH, NHC(=O), C(=O)NH, NR⁸C(=O), C (=O) N R⁸, C (=O) , C (=O) O, OC (=O) , NHC (=S) NH, NHC(=O)NH_{,} SO₂, SO₂NH, (OCH₂CH₂)ₛ, (CH₂CH₂O)_{s'}, (OCH₂CH₂CH2)ₛ", (CH₂CH₂CH₂O)ₜ, and (aa)_{t'};
   - aa: is independently at each occurrence an amino acid;
   - Z: is selected from the group: aryl substituted with 0-3 R¹⁰, C₃₋₁₀ cycloalkyl substituted with 0-3 R¹⁰, and a 5-10 membered heterocyclic ring system containing 1-4 heteroatoms independently selected from N, S, and 0 and substituted with 0-3 R¹⁰;
   - R⁶, R^{6a}, R⁷, R^{7a}, and R⁸: are independently selected at each occurrence from the group: H, =O, COOH, SO₃H, PO₃H, C₁-C₅ alkyl substituted with 0-3 R¹⁰, aryl substituted with 0-3 R¹⁰, benzyl substituted with 0-3 R¹⁰, and C₁-C₅ alkoxy substituted with 0-3 R¹⁰, NHC(=O)R¹¹, C(=O)NHR¹¹, NHC(=O)NHR¹¹, NHR¹¹, R¹¹, and a bond to Cₕ;
   - R¹⁰: is independently selected at each occurrence from the group: a bond to Cₕ, COOR¹¹, C(=O)NHR¹¹, NHC(=O)R¹¹, OH, NHR¹¹, SO₃H, PO₃H, -OPO₃H₂, -OS03H, aryl substituted with 0-3 R¹¹, C₁-₅ alkyl substituted with 0-1 R¹², C₁₋₅ alkoxy substituted with 0-1 R¹², and a 5-10 membered heterocyclic ring system containing 1-4 heteroatoms independently selected from N, S, and O and substituted with 0-3 R¹¹;
   - R¹¹: is independently selected at each occurrence from the group: H, -OPO₃H₂,, -OSO₃H C₁₋₅ alkyl substituted with 0-1 R¹², aryl substituted with 0-1 R¹², a 5-10 membered heterocyclic ring system containing 1-4 heteroatoms independently selected from N, S, and O and substituted with 0-1 R¹², C₃₋₁₀ cycloalkyl substituted with 0-1 R¹², polyalkylene glycol substituted with 0-1 R¹²,, C₁-₅ alkyl substituted with carbohydrate substituted with 0-1 R¹², cyclodextrin substituted with 0-1 R¹², amino acid substituted with 0-1 R¹², polycarboxyalkyl substituted with 0-1 R¹², polyazaalkyl substituted with 0-1 R¹², peptide substituted with 0-1 R¹², wherein the peptide is comprised of 2-10 amino acids, 3,6-O-disulfo-B-D-galactopyranosyl, bis(phosphonomethyl)glycine, and a bond to Cₕ;
   - R¹²: is a bond to Ch;
   - k: is selected from 0, 1, and 2;
   - h: is selected from 0, 1, and 2;
   - h': is selected from 0, 1, and 2;
   - g: is selected from 0, 1, 2, 3, 4, 5, 6, 7, 8, 9, and 10;
   - g': is selected from 0, 1, 2, 3, 4, 5, 6, 7, 8, 9, and 10;
   - s: is selected from 0, 1, 2, 3, 4, 5, 6, 7, 8, 9, and 10;
   - s': is selected from 0, 1, 2, 3, 4, 5, 6, 7, 8, 9, and 10;
   - s": is selected from 0, 1, 2, 3, 4, 5, 6, 7, 8, 9, and 10;
   - t: is selected from 0, 1, 2, 3, 4, 5, 6, 7, 8, 9, and 10;
   - t': is selected from 0, 1, 2, 3, 4, 5, 6, 7, 8, 9, and 10;
   - x: is selected from 0, 1, 2, 3, 4, 5, and 6;
   - x': is selected from 0, 1, 2, 3, 4, 5 and 6;
   - Cₕ: is a metal bonding unit having a formula selected from the group: 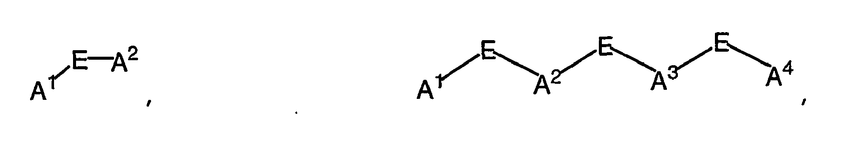 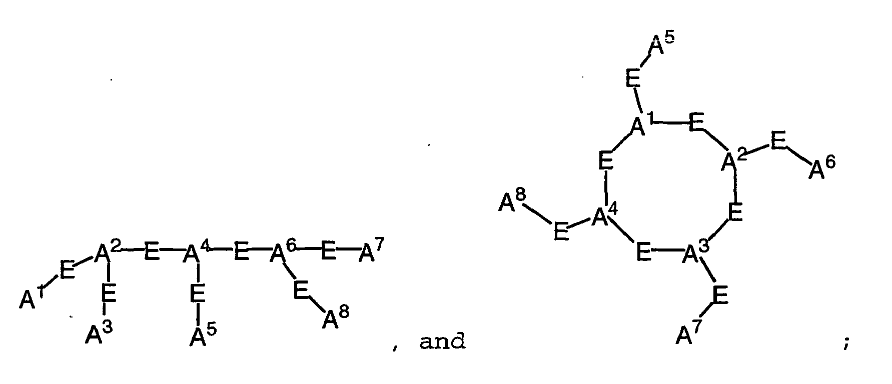
   - A¹, A², A³, A⁴, A⁵, A⁶, A⁷, and A⁸: are independently selected at each occurrence from the group: NR¹³, NR¹³R¹⁴, S, SH, S(Pg), O, OH, PR¹³, PR¹³R¹⁴, P(O)R¹⁵R¹⁶, and a bond to Lₙ;
   - E: is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and 0 and substituted with 0-3 R¹⁷;
   - R¹³ and R¹⁴: are each independently selected from the group: a bond to Lₙ, hydrogen, C₁-C₁₀ alkyl substituted with 0-3 R¹⁷, aryl substituted with 0-3 R¹⁷, C₁₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and 0, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
   alternatively, R¹³ and R¹⁴ combine to form =C(R²⁰)(R²¹) ;
   - R¹⁵ and R¹⁶: are each independently selected from the group: a bond to Lₙ, -OH, C₁-C₁₀ alkyl substituted with 0-3 R¹⁷, C₁-C₁₀ alkyl substituted with 0-3 R¹⁷, aryl substituted with 0-3 R¹⁷, C₃₋₁₀ cycloalkyl, substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and 0, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
   - R¹⁷: is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C (=O) NHNR¹⁸R^{18a}, -OCH₂CO₂H, 2-(1-morpholino)ethoxy, C₁-C₅ alkyl, C₂-C₄ alkenyl, C₃-C₆ cycloalkyl, C₃-C₆ cycloalkylmethyl, C₂-C₆ alkoxyalkyl, aryl substituted with 0-2 R¹⁸, and a 5-10 membered heterocyclic ring system containing 1-4 heteroatoms independently selected from N, S, and O;
   - R¹⁸, R^{18a}, and R¹⁹: are independently selected at each occurrence from the group: a bond to Lₙ, H, C₁-C₆ alkyl, phenyl, benzyl, C₁-C₆ alkoxy, halide, nitro, cyano, and trifluoromethyl;
   - Pg: is a thiol protecting group;
   - R²⁰ and R²¹: are independently selected from the group: H, C₁-C₁₀ alkyl, -CN, -CO₂R²⁵, -C(=O)R²⁵, -C(=O)N(R²⁵)₂, C₂-C₁₀ 1-alkene substituted with 0-3 R²³, C₂-C₁₀ 1-alkyne substituted with 0-3 R²³, aryl substituted with 0-3 R²³, unsaturated 5-10 membered heterocyclic ring system containing 1-4 heteroatoms independently selected from N, S, and O and substituted with 0-3 R²³, and unsaturated C₃-₁₀ carbocycle substituted with 0-3 R²³;
   alternatively, R²⁰ and R²¹, taken together with the divalent carbon radical to which they are attached form: 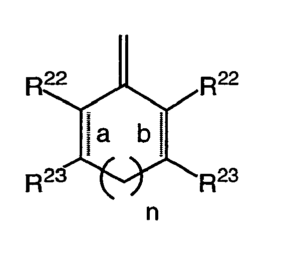
   - R²² and R²³: are independently selected from the group:
   H, R²⁴, C₁-C₁₀ alkyl substituted with 0-3 R²⁴, C₂-C₁₀ alkenyl substituted with 0-3 R²⁴, C₂-C₁₀ alkynyl substituted with 0-3 R²⁴, aryl substituted with 0-3 R²⁴, a 5-10 membered heterocyclic ring system containing 1-4 heteroatoms independently selected from N, S, and O and substituted with 0-3 R²⁴, and C₃₋₁₀ carbocycle substituted with 0-3 R²⁴;
   alternatively, R²², R²³ taken together form a fused aromatic or a 5-10 membered heterocyclic ring system containing 1-4 heteroatoms independently selected from N, S, and O;
   - a and b: indicate the positions of optional double bonds and n is 0 or 1 ;
   - R²⁴: is independently selected at each occurrence from the group: =O, F, Cl, Br, I, -CF₃, -CN, -CO₂R²⁵, -C(=O)R²⁵, -C(=O)N(R²⁵)₂, -N(R²⁵)₃⁺, -CH₂OR²⁵ , -OC(=O)R²⁵, -OC(=O)OR^{25a}, -OR²⁵, -OC(=O)N(R²⁵)², -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, and
   2-(1-morpholino)ethoxy; and,
   R²⁵, R^{25a}, and R²⁶ are each independently selected at each occurrence from the group: hydrogen and C₁-C₆ alkyl; or a pharmaceutically acceptable salt thereof.
[3] In another embodiment, the present invention provides a kit according to Embodiment [1], wherein the anti-cancer agent is selected from the group consisting of mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulatingfactor-1, colony stimulating factor-2, denileukin diftitox, interleukin-2, and leutinizing hormone releasing factor.
[7] In another embodiment, the present invention provides a kit according to Embodiment [1], wherein the radiosensitizer agent is selected from the group consisting of 2-(3-nitro-1,2,4-triazol-1-yl)-N-(2-methoxyethyl) acetamide, N-(3-nitro-4-quinolinyl)-4-morpholinecarboxamidine, 3-amino-1,2,4-benzotriazine-1,4-dioxide, N-(hydroxyethyl)-2-nitroimidazole-1-acetamide, 1-(2-nitroimidazol-1-yl)-3-(1-piperidinyl)-2-propanol, and 1-(2-nitro-1-imidazolyl)-3-1-aziridino)-2-propanol.
[8] In another embodiment, the present invention provides a kit according to Embodiment [1], wherein Q is a compound of formula (IV): 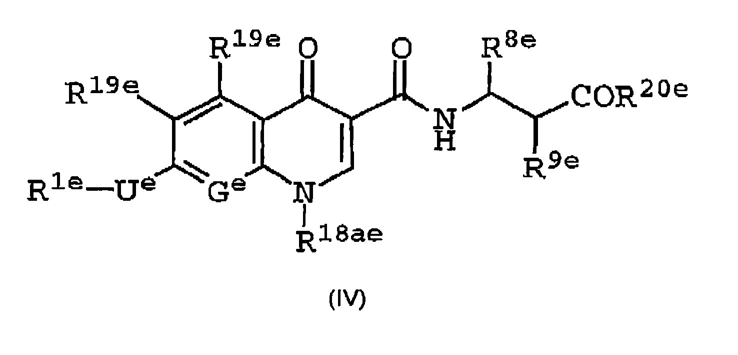 including stereoisomeric forms thereof, or mixtures of stereoisomeric forms thereof, or pharmaceutically acceptable salt or prodrug forms thereof wherein:
   - R^{1e}: is selected from: 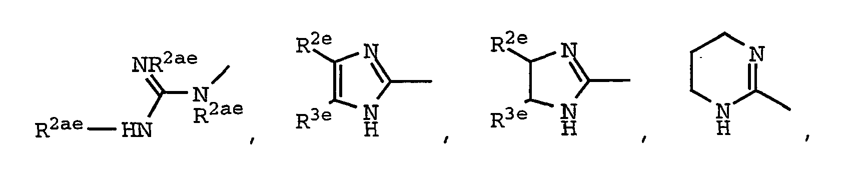 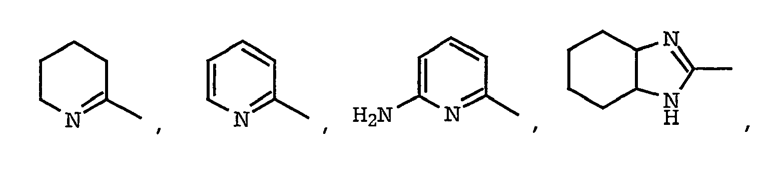 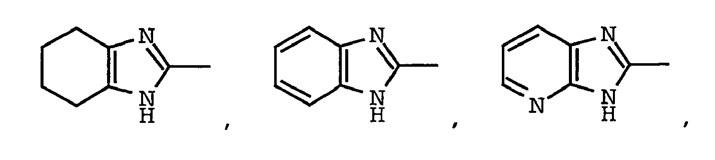 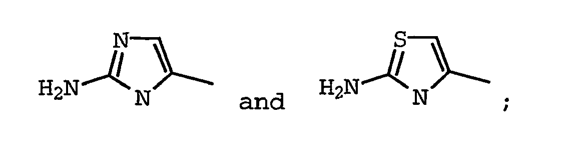
   - R^{2e} and R^{3e}: are independently selected from:
   H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
   alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
   - R^{2ae}: is selected from:
   H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl,
   (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
   - R^{7e}: is selected from:
   H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and CO₂R^{18ae}, SO₂R^{11e}, SO₂NR^{10e}R^{11e}, OR^{10e}, any N(R^{11e})R^{12e};
   - U^{e} is: selected from:
   -(CH₂)ₙ^{e}-, -(CH₂)ₙ^{e}O(CH₂)ₘ^{e}-, -NH(CH₂)ₙ^{e}-, -N(R^{10e})C(=O)-, -NHC(=O)(CH₂)ₙ^{e}-, and-C(=O)N(R^{10e})-;
   - G^{e}: is N or CR^{19e};
   - R^{8e}: is selected from:
   H, CO₂R^{18be}, C(=O)R^{18be}, CONR^{17e}R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl, C₃-C₁₀ cycloalkyl(C₁-C₄ alkyl)-, phenyl substituted with 0-3 R^{6e}, naphthyl substituted with 0-3 R^{6e},
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   - R^{9e}: is selected from:
   C₁-C₁₀ alkyl substituted with 0-1 R^{6e},
   C₁-C₁₀ alkoxy substituted with 0-2 R^{7e},
   H, nitro, N(R^{11e})R^{12e}, OC(=O)R10e, OR^{10e}, OC(=O)NR^{10e}R^{11e}, NR^{10e}C(=O) R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, hydroxy, OR^{22e}, -N(R^{10e})R^{11e}, -N(R^{16e})R^{17e}, aryl(C₀-C₆ alkyl)carbonyl, aryl(C₁-C₆ alkyl), heteroaryl(C₁-C₆ alkyl), CONR^{18ae}R^{20e}, SO₂R^{18ae}, and SO₂NR^{18ae}R^{20e},
   providing that any of the above alkyl, cycloalkyl, aryl or heteroaryl groups may be unsubstituted or substituted independently with 1-2 R^{7e};
   - R^{6e}: is selected from:
   H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚ^{e}R^{11e}, SO₂NR^{10e}R^{11e},
   aryl substituted with 0-3 groups selected from halogen, C₁-C₆ alkoxy, C₁-C₆ alkyl, CF₃, S(O)ₘ^{e}Me, and -NMe₂,
   aryl(C₁-C₄ alkyl)-, said aryl being substituted with 0-3 groups selected from halogen, C₂-C₆ alkoxy, C₁-C₆ alkyl, CF₃, S(O)p^{e}me, and -NMe₂, and
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   - R^{10e}: is selected from:
   H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
   - R^{11e}: is selected from:
   H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
   - R^{4e}: is selected from:
   H, C₁-C₆ alkyl, C₃-C₇ cycloalkyl, C₃-C₇ cycloalkyl(C₁-C₄ alkyl)-, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
   - R^{12e}: is selected from:
   H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy)carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
   - R^{16e}: is selected from:
   -C(=O)OR^{18ae}, -C(=O)R^{18be}, -C(=O)N(R^{18be})2,-SO₂R^{18ae}, and -SO₂N(R^{18be})₂;
   - R^{17e}: is selected from:
   H, C₁-C₆ alkyl, C₃-C₇ cycloalkyl, C₃-C₇ cycloalkyl(C₁-C₄ alkyl)-, aryl, aryl(C₁-C₆ alkyl)-, and heteroaryl(C₁-C₆ alkyl);
   - R^{18ae}: is selected from:
   C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
   - R^{18be}: is H or R^{18ae};
   - R^{19e}: is selected from:
   H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
   - R^{20e}: is selected from: hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
   (5-(C₁-C₅ alkyl)-1,3-dioxa-cyclopenten-2-one-yl)methyloxy,
   (5-aryl-1,3-dioxa-cyclopenten-2-one-yl)methyloxy, and
   (R^{10e}) (R^{11e})_{N}-(C₁-C₁₀ alkoxy)-;
   - R^{21e}: is selected from:
   C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and
   C₁-C₁₀ alkyl substituted with 0-2 R^{7e};
   - R^{22e}: is selected from:
   -C(=O)-R^{18be}, -C(=O)N(R^{18be})₂, -C(=O)NHSO₂R^{18ae}, -C(=O)NHC(=O)R^{18be},-C(=O)NHC(=O)OR^{18ae}, and -C(=O)NHSO₂NHR^{18be};
   - m^{e}: is 0-2;
   - n^{e}: is 0-4; and
   - p^{e}: is 0-2;
   with the following proviso: n^{e} and m^{e} are chosen such that the number of atoms connecting R¹ and -COR^{20e} in Formula (IV) is in the range of 8-14;
   - d: is selected from 1, 2, 3, 4, and 5;
   - d': is 1-50;
   - W: is independently selected at each occurrence from the group: O, NH, NHC(=O), C(=O)NH, NR⁸C(=O), C(=O)N R⁸, C(=O), C(=O)O, OC(=O), NHC(=S)NH, NHC (=O) NH, SO₂, (OCH₂CH₂)ₛ, (CH₂CH₂O)_{s'}, (OCH₂CH₂CH₂)_{s"}, (CH₂CH₂CH₂O)ₜ, and (aa)_{t'};
   - aa: is independently at each occurrence an amino acid;
   - Z: is selected from the group: aryl substituted with 0-1 R¹⁰. C₃-₁₀ cycloalkyl substituted with 0-1 R¹⁰, and a 5-10 membered heterocyclic ring system containing 1-4 heteroatoms independently selected from N, S, and O and substituted with 0-1 R¹⁰;
   - R⁶, R^{6a}, R⁷, R^{7a}, and R⁸: are independently selected at each occurrence from the group: H, =0, COOH, SO₃H, C₁-C₅ alkyl substituted with 0-1 R¹⁰, aryl substituted with 0-1 R¹⁰, benzyl substituted with 0-1 R¹⁰, and C₁-C₅ alkoxy substituted with 0-1 R¹⁰, NHC(=O)R¹¹, C(=O)NHR¹¹, NHC(=O)NHR¹¹, NHR¹¹, R¹¹, and a bond to Cₕ;
   - k: is 0 or 1;
   - s: is selected from 0, 1, 2, 3, 4, and 5;
   - s': is selected from 0, 1, 2, 3, 4, and 5;
   - s": is selected from 0, 1, 2, 3, 4, and 5;
   - t: is selected from 0, 1, 2, 3, 4, and 5;
   - A¹, A², A³, A⁴, A⁵, A⁶, A⁷, and A⁸: are independently selected at each occurrence from the group: NR¹³, NR¹³R¹⁴, S, SH, S(Pg), OH, and a bond to Lₙ;
   - E: is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, and a 5-10 membered heterocyclic ring system containing 1-4 heteroatoms independently selected from N, S, and 0 and substituted with 0-3 R¹⁷;
   - R¹³, and R¹⁴: are each independently selected from the group: a bond to Lₙ, hydrogen, C₁-C₁₀ alkyl substituted with 0-3 R¹⁷, aryl substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
   alternatively, R¹³ and R¹⁴ combine to form =C (R²⁰) (R²¹);
   - R¹⁷: is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CH₂OR¹⁸, -OC(=O)R¹⁸, -OC (=O) OR^{18a}, -OR¹⁸, -OC (=O) N (R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)₂, -N(R¹⁸)₂, -NHC(=S)NHR¹⁸, =NOR¹⁸, -C(=O)NHNR¹⁸R^{18a}, -OCH₂CO₂H, and 2-(1-morpholino) ethoxy;
   - R¹⁸, R^{18a}, and R¹⁹: are independently selected at each occurrence from the group: a bond to Lₙ, H, and C₁-C₆ alkyl;
   - R²⁰ and R²¹: are independently selected from the group:
   H, C₁-C₅ alkyl, -CO₂R²⁵ C₂-C₅ 1-alkene substituted with 0-3 R²³, C₂-C₅ 1-alkyne substituted with 0-3 R²³, aryl substituted with 0-3 R²³, and unsaturated 5-10 membered heterocyclic ring system containing 1-4 heteroatoms independently selected from N, S, and 0 and substituted with 0-3 R²³;
   alternatively, R²⁰ and R²¹, taken together with the divalent carbon radical to which they are attached form: 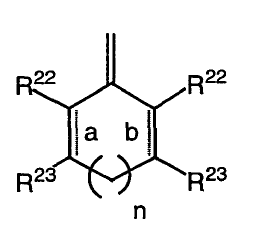
   - R²² and R²³: are independently selected from the group: H, and R²⁴;
   alternatively, R²², R²³ taken together form a fused aromatic or a 5-10 membered heterocyclic ring system containing 1-4 heteroatoms independently selected from N, S, and O;
   - R²⁴: is independently selected at each occurrence from the group: -CO₂R²⁵, -C(=O)N(R²⁵)₂, -CH₂OR²⁵, -OC(=O)R²⁵, -OR²⁵, -SO₃H, -N(R²⁵)₂, and -OCH₂CO₂H; and,
   - R²⁵: is independently selected at each occurrence from the group: H and C₁-C₃ alkyl.
[9] In another embodiment, the present invention provides a kit according to Embodiment [1] or [8],
   wherein:
   - R^{1e}: is selected from: 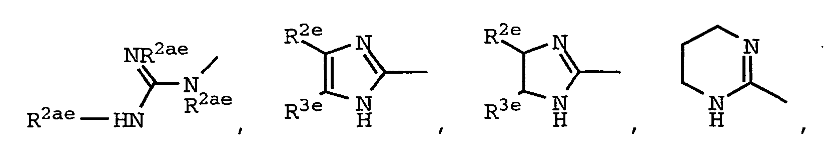 and 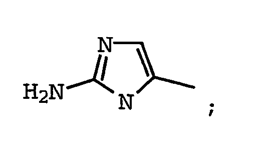
   - R^{2e} and R^{3e}: are independently selected from:
   H, C₁-C₄ alkoxy, NR^{11e}R^{12e}, halogen, NO₂, CN, CF_{3,} C₁-C₆ alkyl, C₃-C₆ alkenyl, C₃-C₇ cycloalkyl, C₃-C₇ cycloalkyl (C₁-C₄ alkyl), aryl (C₁-C₆ alkyl)-, (C₁-C₆ alkyl)carbonyl, (C₁-C₆ alkoxy)carbonyl, arylcarbonyl, and aryl substituted with 0-4 R^{7e}, alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
   - R^{2ae}: is selected from:
   H, C₁-C₁₀ alkyl, C₂-C₆ alkenyl, C₃-C₁₁ cycloalkyl, C₃-C₇ cycloalkyl (C₁-C₄ alkyl), aryl, aryl (C₁-C₄ alkyl)-, (C₂-C₇ alkyl)carbonyl, arylcarbonyl,
   (C₂-C₁₀ alkoxy)carbonyl, C₃-C₇ cycloalkoxycarbonyl, C₇-C₁₁ bicycloalkoxycarbonyl, aryloxycarbonyl, aryl (C₁-C₁₀ alkoxy) carbonyl, C₁-C₆ alkylcarbonyloxy (C₁-C₄ alkoxy)carbonyl, arylcarbonyloxy (C₁-C₄ alkoxy)carbonyl, and C₃-C₇ cycloalkylcarbonyloxy (C₁-C₄ alkoxy) carbonyl;
   - R^{7e}: is selected from:
   H, hydroxy, C₁-C₄ alkyl, C₁-C₄ alkoxy, aryl, aryl (C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e}) R^{12e};
   - U^{e}: is selected from:
   -(CH₂)ₙ^{e}-, -NH(CH₂)ₙ^{e}-, -N(R^{10e})C(=O)-, and
   -NHC(=O)(CH₂)ₙ^{e};
   G^{e} is N or CR^{19e};
   R^{8e} is H;
   R^{9e} is selected from:
   H, nitro, N (R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, hydroxy, OR^{22e}, -N(R^{10e}) R^{11e}, -N(R^{16e})R^{17e}, aryl(C₀-C₄ alkyl) carbonyl, aryl (C₁-C₄ alkyl), heteroaryl (C₁-C₄ alkyl), CONR^{18ae}R^{20e}, SO₂R^{18ae}, and SO₂NR^{18ae}R^{20e},
   providing that any of the above alkyl, cycloalkyl, aryl or heteroaryl groups may be unsubstituted or substituted independently with 1-2 R^{7e};
   - R^{10e}: is selected from:
   H, -OH, CF₃, C₃-C₆ alkenyl, C₃-C₆ cycloalkyl, aryl, (C₃-C₆ cycloalkyl)methyl, aryl (C₁-C₄ alkyl), and C₁-C₄ alkyl substituted with 0-2 R^{6e};
   - R^{6e}: is selected from:
   H, C₁-C₄ alkyl, hydroxy, C₁-C₄ alkoxy, nitro, C₁-C₄ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C (=O) R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
   aryl substituted with 0-3 groups selected from halogen, C₁-C₄ alkoxy, C₁-C₄ alkyl, CF₃, S(O)ₘ^{e}Me, and -NMe₂,
   aryl (C₁-C₄ alkyl)-, said aryl being substituted with 0-3 groups selected from halogen, C₁-C₄ alkoxy, C₁-C₄ alkyl, CF₃, S(O)ₚ^{e}Me, and -NMe₂, and
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   - R^{11e}: is selected from:
   H, hydroxy, C₁-C₄ alkyl, C₃-C₆ alkenyl, C₃-C₆ cycloalkyl, (C₃-C₆ cycloalkyl)methyl, C₁-C₄ alkoxy, benzyloxy, aryl, heteroaryl, heteroaryl(C₁-C₄ alkyl)-, aryl(C₁-C₄ alkyl), adamantylmethyl, and
   C₁-C₄ alkyl substituted with 0-2 R^{4e};
   - R^{4e}: is selected from:
   H, C₁-C₄ alkyl, C₃-C₇ cycloalkyl, C₃-C₇ cycloalkyl(C₁-C₄ alkyl)-, aryl, heteroaryl, aryl(C₁-C₄ alkyl)-, and heteroaryl(C₁-C₄ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
   - R^{12e}: is selected from:
   H, C₁-C₄ alkyl, (C₁-C₄ alkyl)carbonyl, (C₁-C₄ alkoxy)carbonyl, phenyl(C₁-C₄ alkyl)-, phenylsulfonyl, phenyloxycarbonyl, and phenyl(C₁-C₄ alkoxy)carbonyl, wherein said phenyl groups are substituted with 0-2 substituents selected from the group consisting of C₁-C₄ alkyl, C₁-C₄ alkoxy, halo, CF₃, and nitro;
   - R^{16e}: is selected from:
   -C(=O)OR^{18ae} -C(=O)R^{18be}, -C(=O)N(R^{18be})₂,-SO₂R^{18ae}, and -SO₂N(R^{18be})₂;
   - R^{17e}: is selected from:
   H, C₁-C₄ alkyl, C₃-C₆ cycloalkyl, C₃-C₆ cycloalkyl(C₁-C₄ alkyl)-, aryl, aryl(C₁-C₆ alkyl)-, and heteroaryl(C₁-C₆ alkyl);
   - R^{18ae}: is selected from:
   C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
   - R^{18be}: is H or R^{18ae};
   - R^{19e}: is selected from:
   H, halogen, CF₃, CO₂H, CN, NO₂, -NR^{11e}R^{12e}, OCF₃, C₁-C₆ alkyl, C₂-C₆ alkenyl, C₂-C₆ alkynyl, C₃-C₆ cycloalkyl, C₃-C₆ cycloalkyl(C₁-C₄ alkyl)-, aryl(C₁-C₄ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
   - R^{20e}: is selected from:
   hydroxy, C₁-C₆ alkyloxy, C₃-C₆ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl) oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
   (5-(C₁-C₅ alkyl)-1,3-dioxa-cyclopenten-2-one-yl)methyloxy,
   (5-aryl-1,3-dioxa-cyclopenten-2-one-yl)methyloxy, and
   (R^{10e}) (R^{11e})N-(C₁-C₁₀ alkoxy)-;
   - R^{21e}: is selected from:
   C₁-C₄ alkyl, C₂-C₆ alkenyl, C₃-C₆ cycloalkyl, (C₃-C₆ cycloalkyl)methyl, aryl, aryl(C₁-C₄ alkyl)-, and C₁-C₁₀ alkyl substituted with 0-2 R^{7e};
   - R^{22e}: is selected from:
   -C(=O)-R^{18be}, -C (=O) N (R^{18be})₂, -C(=O)NHSO₂R^{18ae}, -C(=O)NHC(=O)R^{18be}, -C(=O)NHC(=O)OR^{18ae}, and -C (=O) NHSO₂NHR^{18be};
   - m^{e}: is 0-2;
   - n^{e}: is 0-4;
   - p^{e}: is 0-2;
   - Cₕ: is 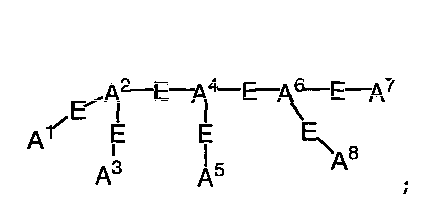
   - A¹: is selected from the group: OH, and a bond to Lₙ;
   - A²,: A⁴, and A⁶ are each N;
   - A³,: A⁵, and A⁸ are each OH;
   - A⁷: is a bond to Lₙ or NH-bond to Lₙ;
   - E: is a C₂ alkyl substituted with 0-1 R¹⁷;
   - R¹⁷: is =O;
   alternatively, Cₕ is 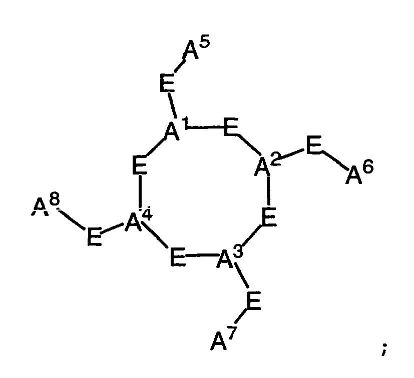
   - A¹: is selected from the group: OH, and a bond to Lₙ;
   - A², A³ and A⁴: are each N;
   - A⁵, A⁶ and A⁸: are each OH;
   - A⁷: is a bond to Lₙ;
   - E: is a C₂ alkyl substituted with 0-1 R¹⁷;
   - R¹⁷: is =O;
   alternatively, Cₕ is 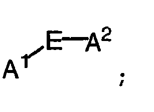
   - A¹: is NH₂ or N=C(R²⁰)(R²¹);
   - E: is a bond;
   - A²: is NHR¹³;
   - R¹³: is a heterocycle substituted with R¹⁷, the heterocycle being selected from pyridine and pyrimidine;
   - R¹⁷: is selected from a bond to Lₙ, C (=O) NHR¹⁸ and C(=O)R¹⁸;
   - R¹⁸: is a bond to Lₙ;
   - R²⁴: is selected from the group: -CO₂R²⁵, -OR²⁵, -SO₃H, and -N(R²⁵)₂; and,
   - R²⁵: is independently selected at each occurrence from the group: hydrogen and methyl.
[10] In another embodiment, the present invention provides a kit according to Embodiment [1], wherein:
   Q is selected from the group:
   3-[7-[(imidazolin-2-ylamino)methyl]-1-methyl-6,8-difluoroquinoline-4-one-3-ylcarbonylamino]-2-(3,5-dimethylisoxazol-4-ylsulfonylamino)propionic acid,
   3-[7-[(imidazolin-2-ylamino)methyl]-1-methyl-6,8-difluoroquinoline-4-one-3-ylcarbonylamino]-2-(benzyloxycarbonylamino)propionic acid,
   3-[7-[(imidazolin-2-ylamino)methyl]-1-methyl-6,8-difluoroquinoline-4-one-3-ylcarbonylamino]-2-(n-butyloxycarbonylamino)propionic acid,
   3-[7-[(imidazolin-2-ylamino)methyl]-1-methyl-6,8-difluoroquinoline-4-one-3-ylcarbonylamino]-2-(n-butylsulfonylamino)propionic acid,
   3-[7-[(tetrahydropyrimid-2-ylamino)methyl]-1-methyl-6,8-difluoroquinoline-4-one-3-ylcarbonylamino]-2-(benzyloxycarbonylamino)propionic acid,
   3-[7-[(tetrahydropyrimid-2-ylamino)methyl]-1-methyl-6,8-difluoroquinoline-4-one-3-ylcarbonylamino]-2-(n-butyloxycarbonylamino)propionic acid,
   3-[7-[(tetrahydropyrimid-2-ylamino)methyl]-1-methyl-6,8-difluoroquinoline-4-one-3-ylcarbonylamino]-2-(phenylsulfonylamino)propionic acid,
   3-[7-[(tetrahydropyrimid-2-ylamino)methyl]-1-methyl-6,8-difluoroquinoline-4-one-3-ylcarbonylamino]-2-(n-butylsulfonyl)aminopropionic acid,
   3-[7-[(2-aminothiazol-4-yl)methyl]-1-methyl-6,8-difluoroquinoline-4-one-3-ylcarbonylamino]-2-(benzyloxycarbonylamino)propionic acid,
   3-[7-[(imidazolin-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(tetrahydropyrimid-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(imidazol-2-ylamino)methyl]-1-methyl-6,8-difluoroquinoline-4-one-3-ylcarbonylamino]-2-(3,5-dimethylisoxazol-4-ylsulfonylamino)propionic acid,
   3-[7-[(imidazol-2-ylamino)methyl]-1-methyl-6,8-difluoroquinoline-4-one-3-ylcarbonylamino]-2-(benzyloxycarbonylamino)propionic acid,
   3-[7-[(imidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(imidazol-2-ylamino)methyl]-1-methyl-6,8-difluoroquinoline-4-one-3-ylcarbonylamino]-2-((4-biphenyl)sulfonylamino)propionic acid,
   3-[7-[(imidazol-2-ylamino)methyl]-1-methyl-6,8-difluoroquinoline-4-one-3-ylcarbonylamino]-2-(1-naphthylsulfonylamino)propionic acid,
   3-[7-[(benzimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(4-methylimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(4,5-dimethylimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(4,5,6,7-tetrahydrobenzimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(pyridin-2-ylamino)methyl]-1-methyl-6,8-difluoroquinoline-4-one-3-ylcarbonylamino]-2-((2,4,6-trimethylphenyl)sulfonylamino)propionic acid,
   3-[7-(2-aminopyridin-6-yl)-1-methyl-6,8-difluoroquinoline-4-one-3-ylcarbonylamino]-2-((2,4,6-trimethylphenyl)sulfonylamino)propionic acid,
   3-[7-[(7-azabenzimidazol-2-yl)methyl]-1-methyl-6,8-difluoroquinoline-4-one-3-ylcarbonylamino]-2-((2,4,6-trimethylphenyl)sulfonylamino)propionic acid,
   3-[7-[(benzimidazol-2-ylamino)methyl]-1-(2-phenylethyl)-6,8-difluoroquinoline-4-one-3-ylcarbonylamino]pro-pionic acid,
   3-[7-[(pyridin-2-ylamino)methyl]-1-(2-phenylethyl)-6,8-difluoroquinoline-4-one-3-ylcarbonylamino]propionic acid,
   3-[7-[(imidazolin-2-ylamino)methyl]-1-(2-phenylethyl)-6,8-difluoroquinoline-4-one-3-ylcarbonylamino]propionic acid,
   3-[7-[(imidazol-2-ylamino)methyl]-1-(2-phenylethyl)-6,8-difluoroquinoline-4-one-3-ylcarbonylamino]propionic acid,
   3-[7-[(imidazolin-2-ylamino)methyl]-1-(2-phenylethyl)-6,8-difluoroquinoline-4-one-3-ylcarbonylamino]-2-(benzyloxycarbonylamino)propionic acid,
   3-[7-[(imidazolin-2-ylamino)methyl]-1-(2-phenylethyl)-6,8-difluoroquinoline-4-one-3-ylcarbonylamino]-2-(n-butyloxycarbonylamino)propionic acid,
   3-[7-[(imidazolin-2-ylamino)methyl]-1-(2-phenylethyl)-6,8-difluoroquinoline-4-one-3-ylcarbonylamino]-2-(phenylsulfonylamino)propionic acid,
   3-[7-[(imidazolin-2-ylamino)methyl]-1-(2-phenylethyl)-6,8-difluoroquinoline-4-one-3-ylcarbonylamino]-2-(n-butylsulfonylamino)propionic acid,
   3-[7-[(tetrahydropyrimid-2-ylamino)methyl]-1-(2-phenylethyl)-6,8-difluoroquinoline-4-one-3-ylcarbonylamino]-2-(benzyloxycarbonylamino)propionic acid,
   3-[7-[(tetrahydropyrimid-2-ylamino)methyl]-1-(2-phenylethyl)-6,8-difluoroquinoline-4-one-3-ylcarbonylamino]-2-(n-butyloxycarbonylamino)propionic acid,
   3-[7-[(tetrahydropyrimid-2-ylamino)methyl]-1-(2-phenylethyl)-6,8-difluoroquinoline-4-one-3-ylcarbonylamino]-2-(phenylsulfonylamino)propionic acid,
   3-[7-[(tetrahydropyrimid-2-ylamino)methyl]-1-(2-phenylethyl)-6,8-difluoroquinoline-4-one-3-ylcarbonylamino]-2-(n-butylsulfonyl)aminopropionic acid,
   3-[7-[(2-aminothiazol-4-yl)methyl]-1-(2-phenylethyl)-6,8-difluoroquinoline-4-one-3-ylcarbonylamino]-2-(phenylsulfonylamino)propionic acid,
   3-[7-[(2-aminothiazol-4-yl)methyl]-1-(2-phenylethyl)-6,8-difluoroquinoline-4-one-3-ylcarbonylamino]-2-(benzyloxycarbonylamino)propionic acid,
   3-[7-[(imidazolin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(tetrahydropyrimid-2-ylamino)methyl]-1-(2-phenylethyl)-6,8-difluoroquinoline-4-one-3-ylcarbonylamino]-2-((2,4,6-trimethylphenyl)sulfon-ylamino)propionic acid,
   3-[7-[(imidazol-2-ylamino)methyl]-1-(2-phenylethyl)-6,8-difluoroquinoline-4-one-3-ylcarbonylamino]-2-(benzyloxycarbonylamino)propionic acid,
   3-[7-[(imidazol-2-ylamino)methyl]-1-(2-phenylethyl)-6,8-difluoroquinoline-4-one-3-ylcarbonylamino]-2-(phenylsulfonylamino)propionic acid,
   3-[7-[(imidazol-2-ylamino)methyl]-1-(2-phenylethyl)-6,8-difluoroquinoline-4-one-3-ylcarbonylamino]-2-((2,6,dichlorophenyl)sulfonylamino)propionic acid,
   3-[7-[(imidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(imidazol-2-ylamino)methyl]-1-(2-phenylethyl)-6,8-difluoroquinoline-4-one-3-ylcarbonylamino]-2-((4-biphenyl)sulfonylamino)propionic acid,
   3-[7-[(benzimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(4-methylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(4,5-dimethylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(4,5,6,7-tetrahydrobenzimidazol-2-ylamino)methyl]-1-(2-phenylethyl)-6,8-difluoroquinoline-4-one-3-ylcarbonylamino]-2-((2, 4, 6-trimethylphenyl)sulfonylamino)propionic acid,
   3-[7-[(pyridin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-(2-aminopyridin-6-yl)-1-(2-phenylethyl)-6,8-difluoroquinoline-4-one-3-ylcarbonylamino]-2-((2,4,6-trimethylphenyl)sulfonylamino)propionic acid, and
   3-[7-[(7-azabenzimidazol-2-yl)methyl]-1-(2-phenylethyl)-6,8-difluoroquinoline-4-one-3-ylcarbonylamino]-2-((2,4,6-trimethylphenyl)sulfonylamino)propionic acid.
[11] In another embodiment, the present invention provides a kit according to Embodiment [1], wherein the compound is selected from the group:
   2-(((4-(4-(((3-(2-(2-(3-((6-((1-aza-2-(2-sulfophenyl)vinyl)amino)(3-pyridyl))carbonylamino)-propoxy)ethoxy)-ethoxy)propyl)amino)sulfonyl)phenyl)phenyl)-sulfonyl)amino)-3-((7-((imidazol-2-ylamino)methyl)-1-methyl-4-oxo(3-hydroquinolyl))carbonylamino)propanoic acid;
   3-((7-((imidazol-2-ylamino)methyl)-1-methyl-4-oxo(3-hydroquinolyl))carbonylamino)-2-(((4-(4-(((3-(2-(2-(3-(2-(1,4,7,10-tetraaza-4,7,10-tris(carboxylmethyl)cyclododecyl)acetylamino)-propoxy)ethoxy)ethoxy)propyl)amino)sulfonyl)-phenyl)phenyl)sulfonyl)amino)propanoic acid;
   2-(((4-(3-(N-(3-(2-(2-(3-((6-((1-aza-2-(2-sulfophenyl)vinyl)amino)(3-pyridyl))carbonylamino)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))carbonylamino)propanoic;
   3-((1-(3-((6-((1-aza-2-(2-sulfophenyl)vinyl)amino)(3-pyridyl))carbonylamino)propyl)-7-((imidazole-2-ylamino)methyl)-4-oxo(3-hydroquinolyl)) carbonylamino) -2- (((2,4,6-trimethylphenyl)sulfonyl)amino)propanoic acid;
   3-((1-(3-((6-((1-aza-2-(2-sulfophenyl)vinyl)amino)(3-pyridyl))carbonylamino)propyl)-7-(((1-hydroxyimidazole-2-yl)amino)methyl)-4-oxo(3-hydroquinolyl))carbonylamino)-2-(((2,4,6-trimethylphenyl)sulfonyl)amino)propanoic acid;
   3-((1-(3-(3-(N-(3-(2-(2-(3-((6-((1-aza-2-(2-sulfophenyl)vinyl)amino)(3-pyridyl))carbonylamino)propoxy)ethoxy)-ethoxy) propyl) carbamoyl) propanoylamino) propyl)-7-((imidazole-2-ylamino)methyl)-4-oxo(3-hydroquinolyl))carbonylamino)-2-(((2,4,6-trimethylphenyl)sulfonyl)amino)propanoic acid;
   2-(2-aza-2-(5-(N-(1,3-bis (3- (2- (2- (3- (3- (N- (3- (3- (N- (3-carboxy-2-(((2,4,6-trimethylphenyl)sulfonyl)amino)-ethyl)carbamoyl)-7-((imidazole-2-ylamino)methyl)4-oxohydroquinolyl)propyl)carbamoyl)propanoylamino)pr opoxy)ethoxy)ethoxy)propyl)carbamoyl)(2-pyridyl))amino)vinyl)benzenesulfonic acid; 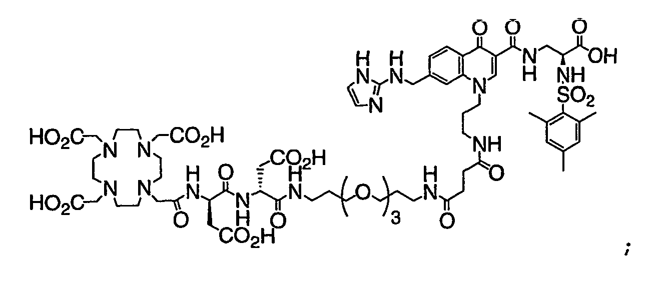 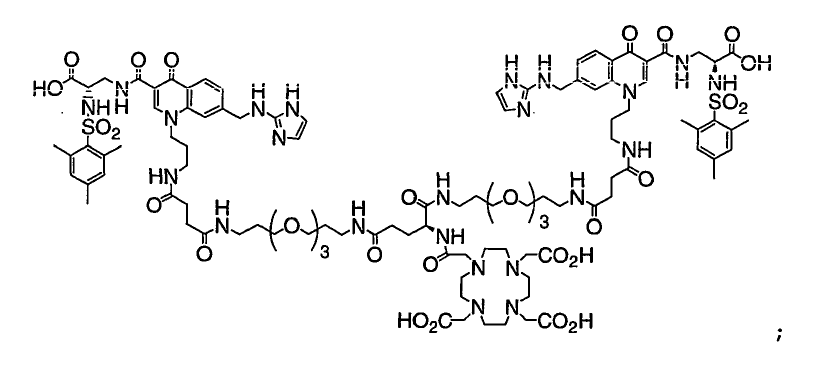 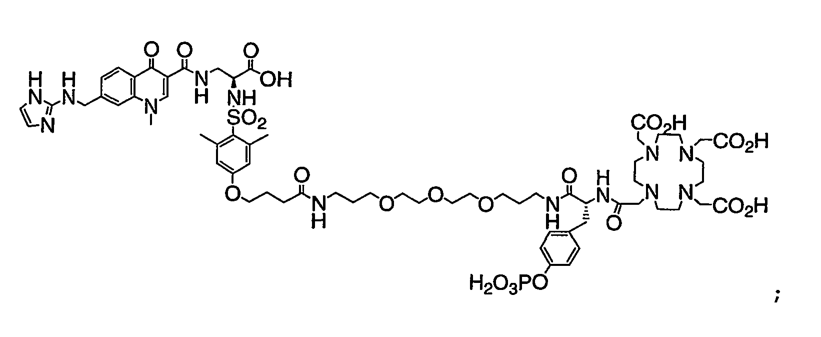 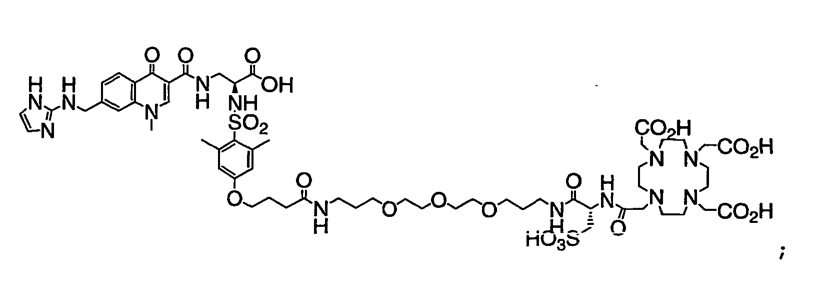 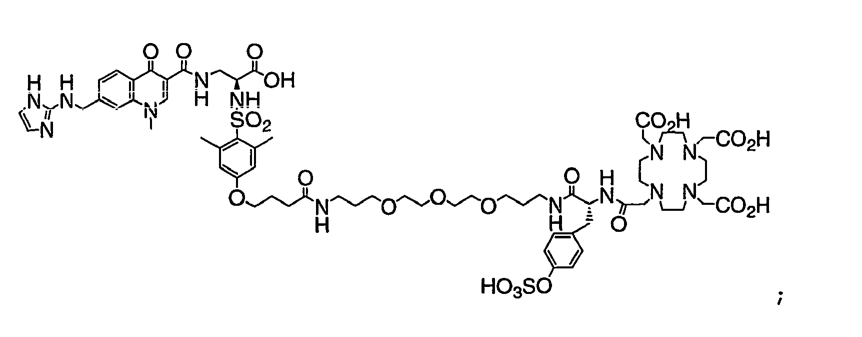 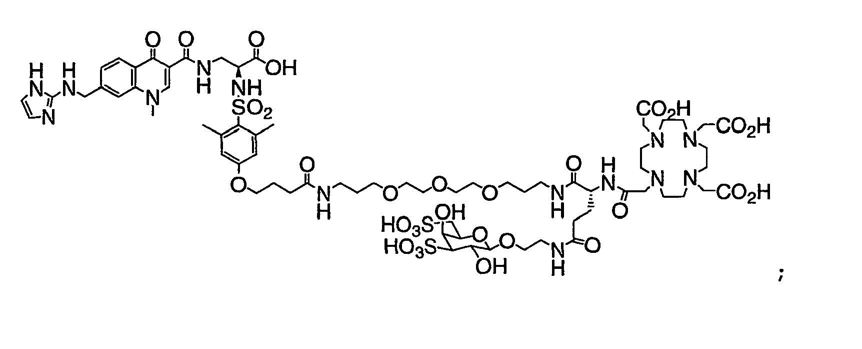 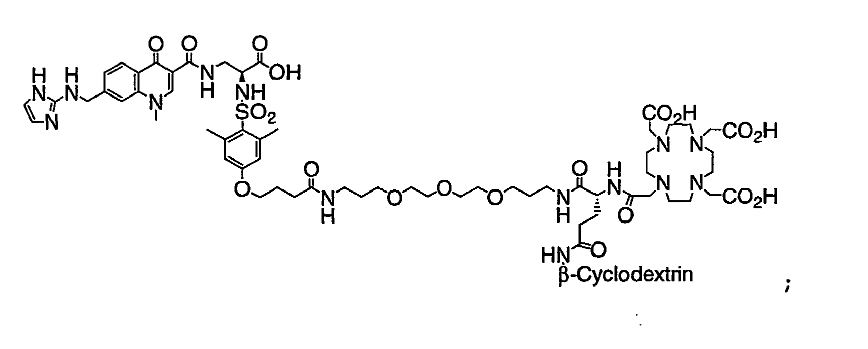 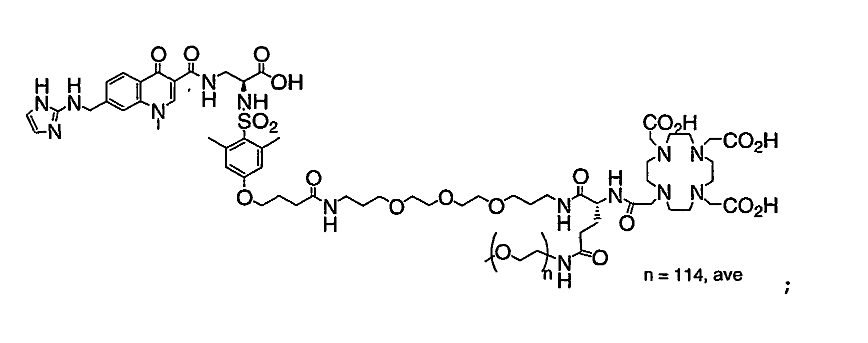
   2-(((4-(3-(N-(3-(2-(2-(3-(2-(1,4,7,10-tetraaza-4,7,10-tris (carboxymethyl) cyclododecylacetylamino)-6-aminohexanoylamino)propoxy)ethoxy)ethoxy)-propyl) carbamoyl) propoxy)-2,6-dimethylphenyl) sulfonyl) amino) -3-((7-((imidazol-2-ylamino)methyl)-1-methyl-4-oxo(3-hydroquinolyl))-carbonylamino)propanoic acid ;
   2-(((4-(3-(N-(3-(2-(2-(3-(2-(1,4,7,10-tetraaza-4,7,10-tris(carboxymethyl)cyclododecylacetylamino)-6-(2-(bis(phosphonomethyl)amino)acetylamino)hexanoylamin o)propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))carbonylamino)propanoic acid conjugate; and
   2- (((4- (3- (N- (3- (2- (2- (3- (2- (2- ((2- (2-(bis(carboxymethyl)-amino)ethyl) (carboxymethyl) amino) ethyl) (carboxymeth yl)amino)acetylamino)-3-sulfopropyl)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))carbonylamino)propanoic acid; 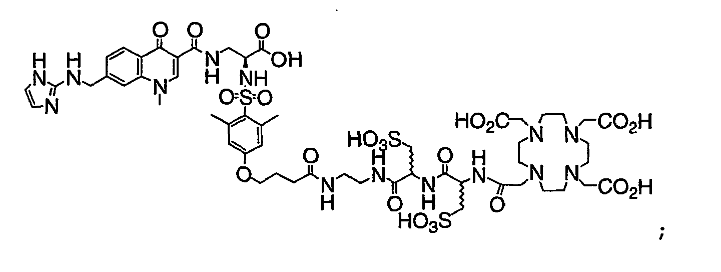 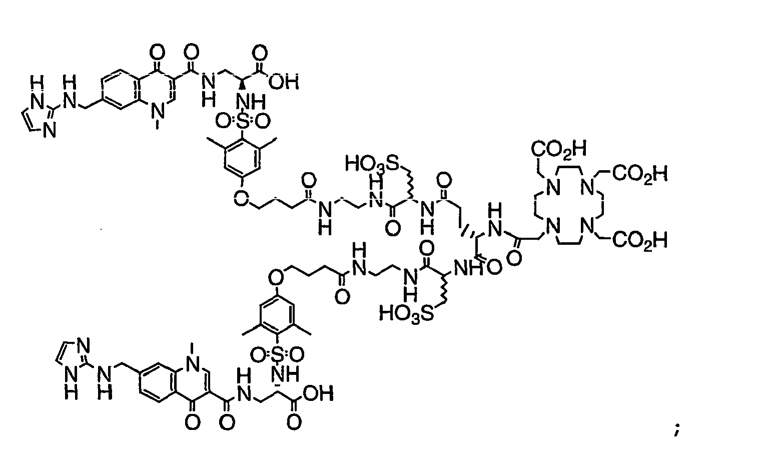 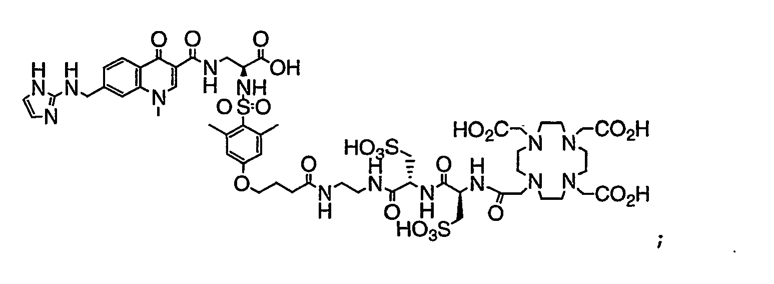 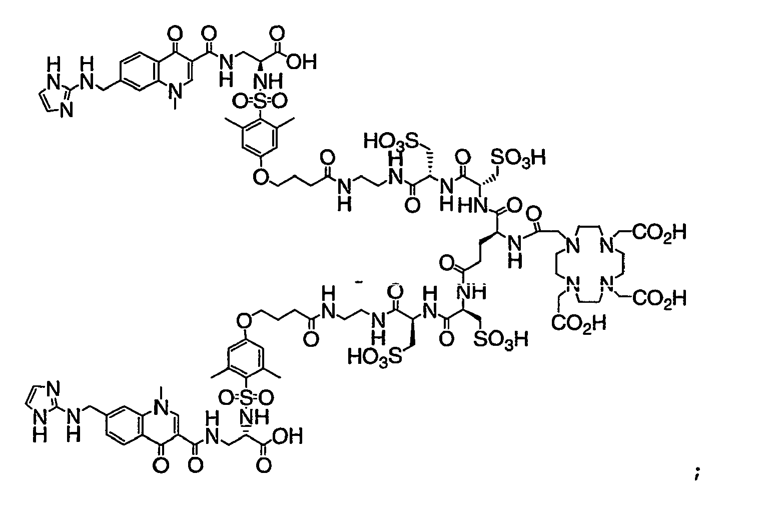 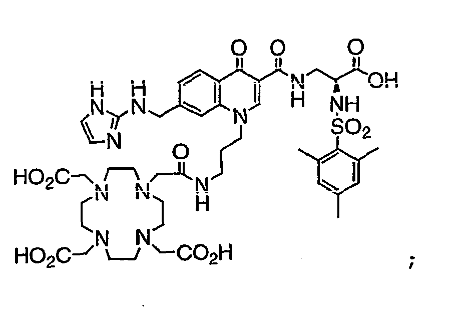 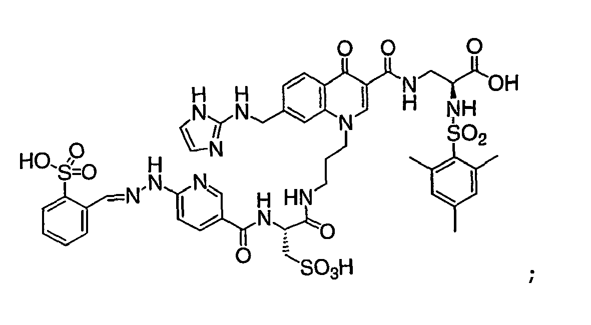 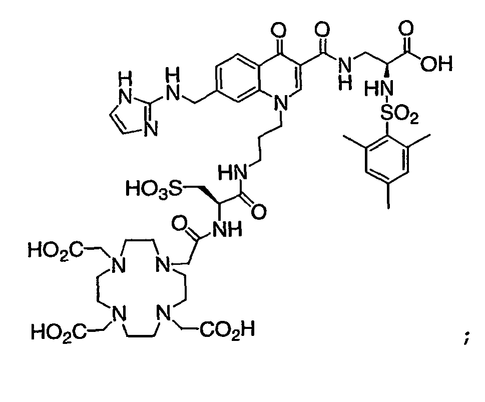 and 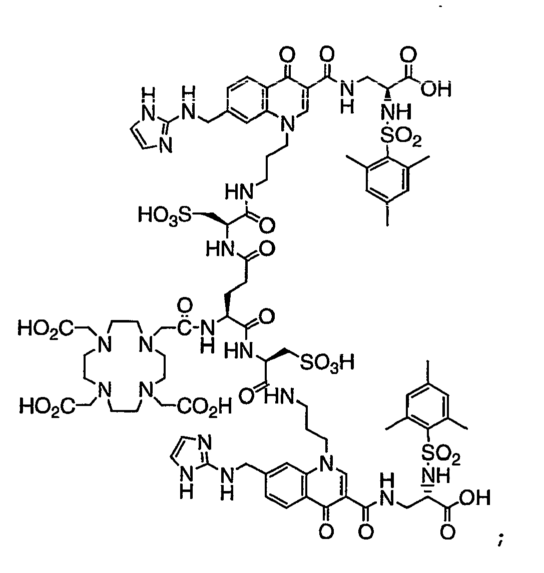
   or a pharmaceutically acceptable salt form thereof.
[12] In another embodiment, the present invention provides a kit according to Embodiment [1], wherein the kit further comprises one or more ancillary ligands and a reducing agent.
[13] In another embodiment, the present invention provides a kit according to Embodiment [12], wherein the ancillary ligands are tricine and TPPTS.
[14] In another embodiment, the present invention provides a kit according to Embodiment [12], wherein the reducing agent is tin(II).
[15] In another embodiment, the present invention provides a therapeutic radiopharmaceutical composition comprising at least one agent selected from the group consisting of an anti-cancer agent and a radiosensitizer agent, or a pharmaceutically acceptable salt thereof, and a radiopharmaceutical comprising:
   a) a metal;
   b) a chelator capable of chelating the metal; and
   c) a targeting moiety;
   wherein the targeting moiety is bound to the chelator through 0-1 linking groups, and the targeting moiety is a quinolone non-peptide that binds to a receptor that is upregulated during angiogenesis..
[16] In another embodiment, the present invention provides a therapeutic radiopharmaceutical composition according to Embodiment [15], wherein the anti-cancer agent is selected from the group consisting of mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, and leutinizing hormone releasing factor.
[17] In another embodiment, the present invention provides a therapeutic radiopharmaceutical composition according to Embodiment [15], wherein radiosensitizer agent is selected from the group consiting of 2-(3-nitro-1,2,4-triazol-1-yl)-N-(2-methoxyethyl)acetamide, N-(3-nitro-4-quinolinyl)-4-morpholinecarboxamidine, 3-amino-1,2,4-benzotriazine-1,4-dioxide, N-(2-hydroxyethyl)-2-nitroimidazole-1-acetamide, 1-(2-nitroimidazol-1-yl)-3-(1-piperidinyl)-2-propanol, and 1-(2-nitro-1-imidazolyl)-3-(1-aziridino)-2-propanol.
[18] In another embodiment, the present invention provides a therapeutic radiopharmaceutical composition according to Embodiment [15], wherein the metal is selected from the group ³³P, ¹²⁵I, ¹⁸⁶Re, ¹⁸⁸Re, ¹⁵³Sm, 166_{Ho}, 177_{Lu}, 149_{Pm}, 90_{Y}, 212_{Bi}, 103_{Pd}, 109_{Pd}, 159_{Gd}, 140_{La}, 198_{Au}, 199_{Au}, 169_{Yb}, 175_{Yb}, 165_{Dy}, 166_{Dy}, 67_{Cu}, 105_{Rh}, 111_{Ag}, and ¹⁹²Ir, and the linking group is present between the non-peptide targeting moiety and chelator.
[19] In another embodiment , the present invention provides a therapeutic radiopharmaceutical composition according to Embodiment [18], wherein the targeting moiety is a quinolone non-peptide and the receptor is αᵥβ₃ or αᵥβ₅.
[20] In another embodiment, the present invention provides a therapeutic radiopharmaceutical composition according to Embodiment [15], wherein the radiopharmaceutical comprises:
   a) a metal selected from the group ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir; and
   b) a compound of the formula (I):

      (Q)_{d}-Lₙ-Cₕ or (Q)_{d}-Lₙ(Cₕ)_{d'} (I)
   wherein, Q is a compound of Formula (II): 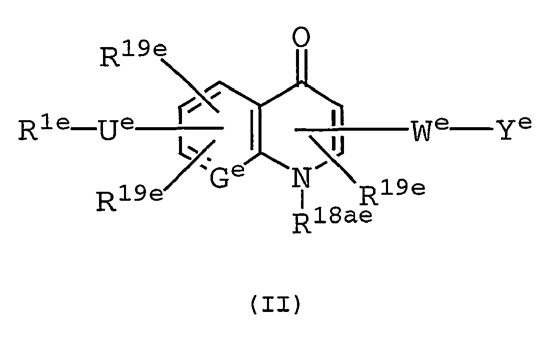 including stereoisomeric forms thereof, or mixtures of stereoisomeric forms thereof, or pharmaceutically acceptable salt or prodrug forms thereof wherein:
   - R^{1e}: is selected from: 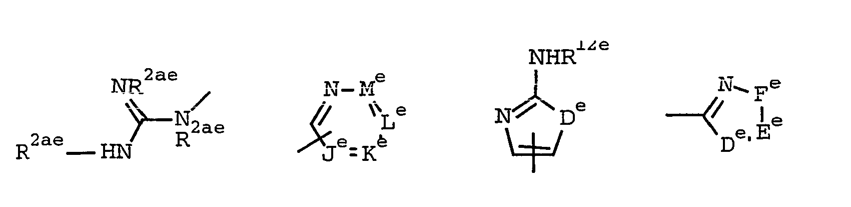 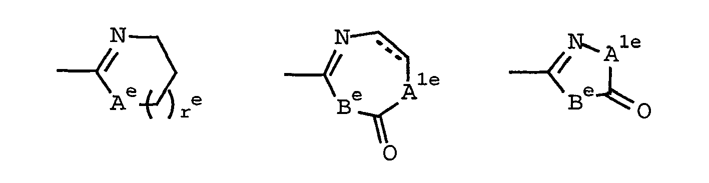 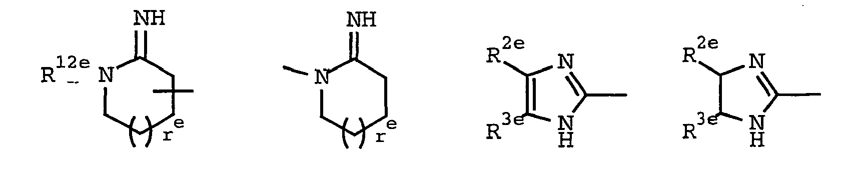 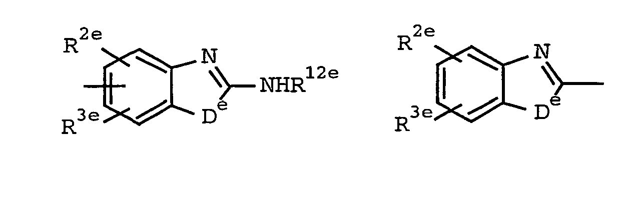 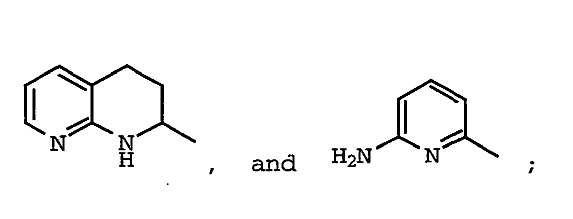
   - A^{e}: is -CH₂- or -N(R^{10e})-;
   - A^{1e} and B^{e}: are independently -CH₂- or -N(R^{10e})-;
   - D^{e}: is -N(R^{10e})- or -S-;
   - E^{e}-F^{e}: is -C (R^{2e}) =C (R^{3e}) - or -C(R^{2e})₂C(R^{3e})₂-;
   - J^{e}: is -C (R^{2e})- or -N-;
   - K^{e}, L^{e} and M^{e}: are independently -C(R^{2e})- or -C(R^{3e})-;
   - R^{2e} and R^{3e}: are independently selected from:
   H, C₁-C₄ alkoxy, NR^{11e}R^{12e}, halogen, NO₂, CN, -CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl (C₁-C₆ alkyl)carbonyl, (C₁-C₆ alkoxy)carbonyl, arylcarbonyl, and aryl substituted with 0-4 R^{7e},
   alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
   - R^{2ae}: is selected from:
   H, C₁-C₁₀ alkyl, C₂-C₆ alkenyl, C₃-C₁₁ cycloalkyl, C₃-C₇ cycloalkyl (C₁-C₄ alkyl), aryl, aryl (C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl (C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
   - R^{7e}: is selected from:
   H, hydroxy, C₁-C₄ alkyl, C₁-C₄ alkoxy, aryl, aryl (C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
   - U^{e}: is selected from:
   - (CH₂)ₙ^{e}-, - (CH₂)ₙ^{e}O(CH₂)ₘ^{e}-,-(CH₂)ₙ^{e}N(R¹²)(CH₂)ₘ^{e}- -NH(CH₂)ₙ^{e}-,-(CH₂)ₙ^{e}C(=O)(CH₂)ₘ^{e}-, -(CH₂)ₙ^{e}S(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})-, and -N(R^{10e})S(O)ₚ^{e}-;
   - G^{e}: is N or CR^{19e};
   - W^{e}: is-C(=O)-N(R^{10e})-(C₁-C₃ alkylene)-, in which the alkylene group is substituted by R^{8e} and by R^{9e}:
   - R^{8e} and R^{9e}: are independently selected from:
   H, CO₂R^{18be}, C(=O)R^{18be}, CONR¹⁷R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl,
   C₃-C₁₀ cycloalkyl (C₁-C₄ alkyl)-, phenyl substituted with 0-3 R^{6e}, naphthyl substituted with 0-3 R^{6e},
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e},
   C₁-C₁₀ alkoxy substituted with 0-2 R^{7e}, hydroxy, nitro, -N(R^{10e})R^{11e}, -N(R^{16e})R^{17e}, aryl(C₀-C₆ alkyl)carbonyl, aryl (C₃-C₆ alkyl), heteroaryl(C₁-C₆ alkyl), CONR^{18ae}R^{20e}, SO₂R^{18ae}, and SO₂NR^{18ae}R^{20e},
   providing that any of the above alkyl, cycloalkyl, aryl or heteroaryl groups may be unsubstituted or substituted independently with 1-2 R^{7e};
   - R^{6e}: is selected from:
   H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
   aryl substituted with 0-3 groups selected from halogen, C₁-C₆ alkoxy, C₁-C₆ alkyl, CF₃, S(O)ₘ^{e}Me, and -NMe₂,
   aryl (C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
   a 5-10 membered heterocyclic ring containing 1-3 N, 0, or S heteroatoms, wherein said heterocyclic ring may be saturated, partially saturated, or fully unsaturated, said heterocyclic ring being substituted with 0-2 R^{7e};
   - R^{10e}: is selected from:
   H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
   - R^{11e}: is selected from:
   H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
   - R^{4e}: is selected from:
   H, C₁-C₆ alkyl, C₃-C₇ cycloalkyl, C₃-C₇ cycloalkyl(C₁-C₄ alkyl)-, (C₁-C₁₀ alkyl)carbonyl, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
   alternatively, when R^{10e} and R^{11e} are both substituents on the same nitrogen atom (as in -NR^{10e}R^{11e}) they may be taken together with the nitrogen atom to which they are attached to form a heterocycle selected from: 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl, and 1-piperazinyl;
   said heterocycle being substituted with 0-3 groups selected from: C₁-C₆ alkyl, aryl, heteroaryl, aryl(C₁-C₄ alkyl)-, (C₁-C₆ alkyl)carbonyl, (C₃-C₇ cycloalkyl)carbonyl, (C₁-C₆ alkoxy)carbonyl, aryl(C₁-C₄ alkoxy)carbonyl, C₁-C₆ alkylsulfonyl, and arylsulfonyl;
   - R^{12e}: is selected from:
   H, C₁-C₆ alkyl, triphenylmethyl, methoxymethyl, methoxyphenyldiphenylmethyl, trimethylsilylethoxymethyl, (C₁-C₆ alkyl) carbonyl, (C₁-C₆ alkoxy)carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
   - R^{16e}: is selected from:
   -C (=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, and -SO₂NHC(=O)OR^{18be};
   - R^{17e}: is selected from:
   H, C₁-C₆ alkyl, C₃-C₇ cycloalkyl, C₃-C₇ cycloalkyl (C₁-C₄ alkyl)-, aryl, aryl (C₁-C₆ alkyl)-, and heteroaryl(C₁-C₆ alkyl);
   - R^{18ae}: is selected from:
   C₁-C₈ alkyl optionally substituted with a bond to Lₙ, C₃-C₁₁ cycloalkyl optionally substituted with a bond to Lₙ, aryl (C₁-C₆ alkyl)-optionally substituted with a bond to Lₙ, heteroaryl (C₁-C₆ alkyl)- optionally substituted with a bond to Lₙ, (C₁-C₆ alkyl)heteroaryl optionally substituted with a bond to Lₙ, biaryl (C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
   - R^{18be}: is H or R^{18ae};
   - R^{19e}: is selected from:
   H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl (C₁-C₄ alkyl)-, aryl (C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
   - R^{20e}: is selected from:
   hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy (C₁-C₂ alkyl)oxy-, C₃-C₁₀ cycloalkoxycarbonyloxy (C₁-C₂ alkyl)oxy-, C₃-C₁₀ cycloalkoxycarbonyl (C₁-C₂ alkyl)oxy-, aryloxycarbonyl (C₁-C₂ alkyl)oxy-, aryloxycarbonyloxy (C₁-C₂ alkyl)oxy-, arylcarbonyloxy (C₁-C₂ alkyl)oxy-, C₁-C₅ alkoxy (C₁-C₅ alkyl) carbonyloxy (C₁-C₂ alkyl)ox y,
   (5-(C₁-C₅ alkyl)-1,3-dioxa-cyclopenten-2-one-yl) methyloxy,
   (5-aryl-1,3-dioxa-cyclopenten-2-one-yl)methyloxy, and
   (R^{10e}) (R^{11e})N-(C₁-C₁₀ alkoxy)-:
   - R^{21e}: is selected from:
   C₁-C₈ alkyl, C₂-C₆ alkenyl, C₃-C₁₁ cycloalkyl, (C₃-C₁₁ cycloalkyl)methyl, aryl, aryl (C₁-C₄ alkyl)-, and C₁-C₁₀ alkyl substituted with 0-2 R^{7e};
   - R^{22e}: is selected from:
   -C(=O)-R^{18be}, -C(=O)N(R^{18be})₂, -C(=O)NHSO₂R^{18ae},-C(=O)NHC(=O)R^{18be}, -C(=O)NHC(=O)OR^{18ae}, and-C(=O)NHSO₂NHR^{18be},
   - Y^{e}: is selected from:
   -COR^{20e}, -SO₃H, -PO₃H, -CONHNHSO₂CF₃,-CONHSO₂R^{18ae}, -CONHSO₂NHR^{18be}, -NHCOCF₃,-NHCONHSO₂R^{18ae}, -NHSO₂R^{18ae}, -OPO₃H₂, -OSO₃H,-PO₃H₂, -SO₂NHCOR^{18ae},
   -SO₂NHCO₂R^{18ae}, 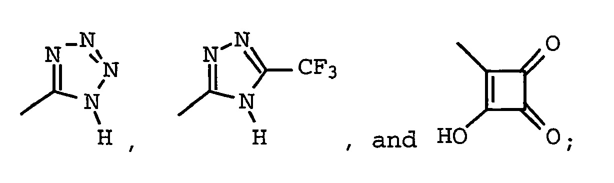
   - m^{e}: is 0-2;
   - n^{e}: is 0-4;
   - p^{e}: is 0-2;
   - r^{e}: is 0-2;
   with the following proviso: n^{e} and m^{e} are chosen such that the number of atoms connecting R^{1e} and Y^{e} is in the range of 8-14;
   - d: is selected from 1, 2, 3, 4, 5, 6, 7, 8, 9, and 10;
   - d': is 1-100;
   - Lₙ: is a linking group having the formula:

   ((W)ₕ-(CR⁶R⁷)g)ₓ-(Z)ₖ-((CR^{6a}R^{7a})g'-(W)_{h'})_{x'};
   - W: is independently selected at each occurrence from the group: O, S, NH, NHC(=O), C(=O)NH, NR⁸C(=O), C (=O)N R⁸, C(=O), C(=O)O, OC(=O), NHC (=S)NH, NHC(=O₎NH_{,} SO₂, SO₂NH, (OCH₂CH₂)ₛ, (CH₂CH₂O)_{s'}, (OCH₂CH₂CH₂)ₛ", (CH2CH₂CH₂O)ₜ, and (aa)t,;
   - aa: is independently at each occurrence an amino acid;
   - Z: is selected from the group: aryl substituted with 0-3 R¹⁰, C₃₋₁₀ cycloalkyl substituted with 0-3 R¹⁰, and a 5-10 membered heterocyclic ring system containing 1-4 heteroatoms independently selected from N, S, and O and substituted with 0-3 R¹⁰;
   - R⁶, R^{6a}, R⁷, R^{7a}, and R⁸: are independently selected at each occurrence from the group: H, =O, COOH, SO₃H, PO₃H, C₁-C₅ alkyl substituted with 0-3 R¹⁰, aryl substituted with 0-3 R¹⁰, benzyl substituted with 0-3 R¹⁰, and C₁-C₅ alkoxy substituted with 0-3 R¹⁰, NHC(=O)R¹¹, C(=O)NHR¹¹, NHC(=O)NHR¹¹, NHR¹¹, R¹¹, and a bond to Cₕ;
   - R¹⁰: is independently selected at each occurrence from the group: a bond to Cₕ, COOR¹¹, C(=O)NHR¹¹, NHC(=O)R¹¹, OH, NHR¹¹, SO₃H, PO₃H, -OPO₃H₂, -OS03H, aryl substituted with 0-3 R¹¹, C₁₋₅ alkyl substituted with 0-1 R¹², C₁₋₅ alkoxy substituted with 0-1 R¹², and a 5-10 membered heterocyclic ring system containing 1-4 heteroatoms independently selected from N, S, and O and substituted with 0-3 R¹¹;
   - R¹¹: is independently selected at each occurrence from the group: H, -OPO₃H₂,, -OS03H C₁₋₅ alkyl substituted with 0-1 R¹², aryl substituted with 0-1 R¹², a 5-10 membered heterocyclic ring system containing 1-4 heteroatoms independently selected from N, S, and O and substituted with 0-1 R¹², C₃₋₁₀ cycloalkyl substituted with 0-1 R¹², polyalkylene glycol substituted with 0-1 R¹², , C₁-5 alkyl substituted with carbohydrate substituted with 0-1 R¹², cyclodextrin substituted with 0-1 R¹², amino acid substituted with 0-1 R¹², polycarboxyalkyl substituted with 0-1 R¹², polyazaalkyl substituted with 0-1 R¹², peptide substituted with 0-1 R¹², wherein the peptide is comprised of 2-10 amino acids, 3,6-0-disulfo-B-D-galactopyranosyl, bis(phosphonomethyl)glycine, and a bond to Cₕ;
   - R¹²: is a bond to Ch;
   - k: is selected from 0, 1, and 2;
   - h: is selected from 0, 1, and 2;
   - h': is selected from 0, 1, and 2;
   - g: is selected from 0, 1, 2, 3, 4, 5, 6, 7, 8, 9, and 10;
   - g': is selected from 0, 1, 2, 3, 4, 5, 6, 7, 8, 9, and 10;
   - s: is selected from 0, 1, 2, 3, 4, 5, 6, 7, 8, 9, and 10;
   - s': is selected from 0, 1, 2, 3, 4, 5, 6, 7, 8, 9, and 10;
   - s": is selected from 0, 1, 2, 3, 4, 5, 6, 7, 8, 9, and 10;
   - t: is selected from 0, 1, 2, 3, 4, 5, 6, 7, 8, 9, and 10;
   - t': is selected from 0, 1, 2, 3, 4, 5, 6, 7, 8, 9, and 10;
   - x: is selected from 0, 1, 2, 3, 4, 5, and 6;
   - x': is selected from 0, 1, 2, 3, 4, 5, and 6;
   - Cₕ: is a metal bonding unit having a formula selected from the group: 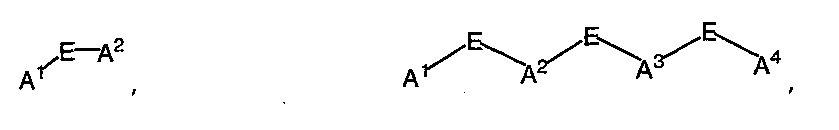 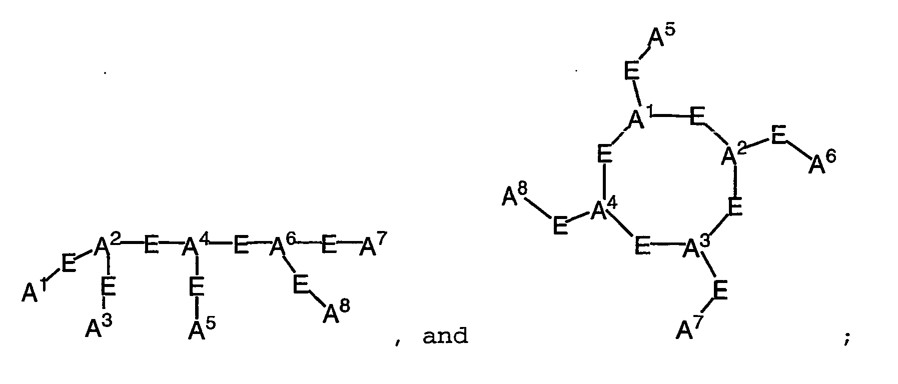
   - A¹, A² , A³ , A⁴ , A⁵ , A⁶ , A⁷, and A⁸: are independently selected at each occurrence from the group: NR¹³, NR¹³R¹⁴, S, SH, S(Pg), O, OH, PR¹³, PR¹³R¹⁴, P(O)R¹⁵R¹⁶, and a bond to Lₙ;
   - E: is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
   - R¹³ and R¹⁴: are each independently selected from the group: a bond to Lₙ, hydrogen, C₁-C₁₀ alkyl substituted with 0-3 R¹⁷, aryl substituted with 0-3 R¹⁷, C₁₋₁₀ cycloalkyl substituted with 0-3 R¹⁷, heterocyclo-C₁-₁₀ alkyl substituted with 0-3 R¹⁷, wherein the heterocyclo group is a 5-10 membered heterocyclic ring system containing 1-4 heteroatoms independently selected from N, S, and O, C₆-₁₀ aryl-C₁₋₁₀ alkyl substituted with 0-3 R¹⁷, C₁-₁₀ alkyl-C₆₋₁₀ aryl- substituted with 0-3 R¹⁷, a 5-10 membered heterocyclic ring system containing 1-4 heteroatoms independently selected from N, S, and 0 and substituted with 0-3 R¹⁷, and an electron, provided that when one of R¹³ or R¹⁴ is an electron, then the other is also an electron;
   alternatively, R¹³ and R¹⁴ combine to form =C(R²⁰)(R²¹);
   - R¹⁵ and R¹⁶: are each independently selected from the group: a bond to Lₙ, -OH, C₁-C₁₀ alkyl substituted with 0-3 R¹⁷, C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆-₁₀ aryl-C₁₋₁₀ alkyl substituted with 0-3 R¹⁷, C₁-₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
   - R¹⁷: is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O) OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H, 2-(1-morpholino)ethoxy, C₁-C₅ alkyl, C₂-C₄ alkenyl, C₃-C₆ cycloalkyl, C₃-C₆ cycloalkylmethyl, C₂-C₆ alkoxyalkyl, aryl substituted with 0-2 R¹⁸, and a 5-10 membered heterocyclic ring system containing 1-4 heteroatoms independently selected from N, S, and O;
   - R¹⁸, R^{18a}, and R¹⁹: are independently selected at each occurrence from the group: a bond to Lₙ, H, C₁-C₆ alkyl, phenyl, benzyl, C₁-C₆ alkoxy, halide, nitro, cyano, and trifluoromethyl;
   - Pg: is a thiol protecting group;
   - R²⁰ and R²¹: are independently selected from the group: H, C₁-C₁₀ alkyl, -CN, -CO₂R²⁵, -C(=O)R25, -C(=O)N(R²⁵)₂, C₂-C₁₀ 1-alkene substituted with 0-3 R²³, C₂-C₁₀ 1-alkyne substituted with 0-3 R²³, aryl substituted with 0-3 R²³, unsaturated 5-10 membered heterocyclic ring system containing 1-4 heteroatoms independently selected from N, S, and 0 and substituted with 0-3 R²³, and unsaturated C₃₋₁₀ carbocycle substituted with 0-3 R²³;
   alternatively, R²⁰ and R²¹, taken together with the divalent carbon radical to which they are attached form: 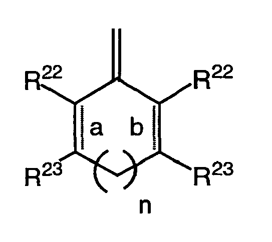
   - R²² and R²³: are independently selected from the group:
   H, R²⁴, C₁-C₁₀ alkyl substituted with 0-3 R²⁴, C₂-C₁₀ alkenyl substituted with 0-3 R²⁴, C₂-C₁₀ alkynyl substituted with 0-3 R²⁴, aryl substituted with 0-3 R²⁴, a 5-10 membered heterocyclic ring system containing 1-4 heteroatoms independently selected from N, S, and O and substituted with 0-3 R²⁴, and C₃₋₁₀ carbocycle substituted with 0-3 R²⁴;
   alternatively, R²², R²³ taken together form a fused aromatic or a 5-10 membered heterocyclic ring system containing 1-4 heteroatoms independently selected from N, S, and 0;
   - a and b: indicate the positions of optional double bonds and n is 0 or 1;
   - R²⁴: is independently selected at each occurrence from the group: =0, F, Cl, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, and
   2-(1-morpholino)ethoxy; and,
   - R²⁵, R^{25a}, and R²⁶: are each independently selected at each occurrence from the group: hydrogen and C₁-C₆ alkyl; or a pharmaceutically acceptable salt thereof.
[21] In another embodiment, the present invention provides a therapeutic radiopharmaceutical composition according to Embodiment [20], wherein Q is a compound of Formula (IV): 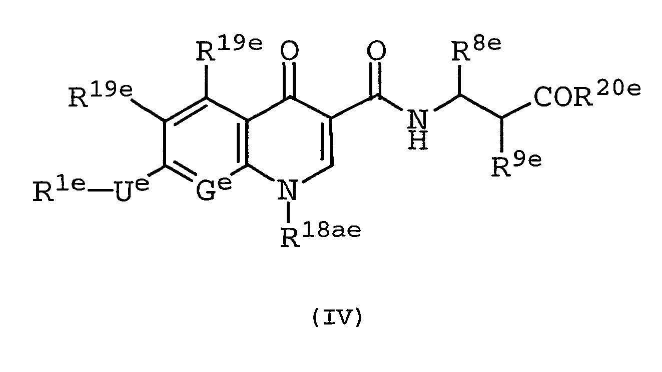 including stereoisomeric forms thereof, or mixtures of stereoisomeric forms thereof, or pharmaceutically acceptable salt or prodrug forms thereof wherein:
   - R^{1e}: is selected from: 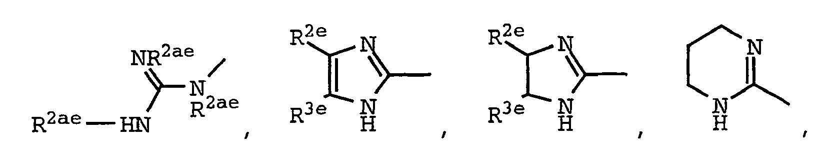 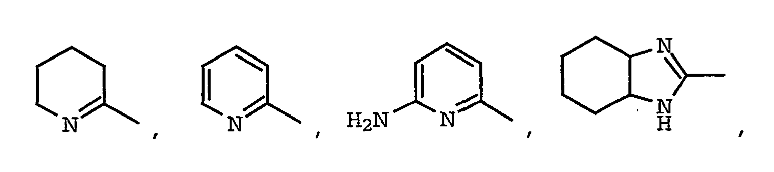 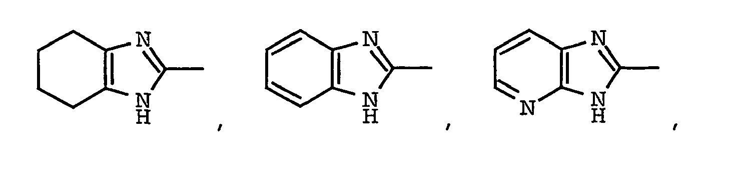 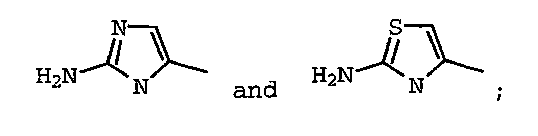
   - R^{2e} and R^{3e}: are independently selected from:
   H, Cₗ-_{C4} alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e}, alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
   - R^{2ae}: is selected from:
   H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl,
   (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
   - R^{7e}: is selected from:
   H, hydroxy. C₁-C₄ alkyl C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
   - U^{e}: is selected from:
   -(CH₂)ₙ^{e_}, -(CH₂)ₙ^{e}O(CH₂)ₘ^{e_}, -NH(CH₂)ₙ^{e_}, -N(R^{10e})(=O)-, -NHC(=O)(CH₂)ₙ^{e_}, and-C(=O)N(R^{10e})-;
   - G^{e}: is N or CR^{19e};
   - R^{8e}: is selected from:
   H, CO₂R^{18be}, C(=O)R^{18be}, CONR^{17e}R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl, C₃-C₁₀ cycloalkyl(C₁-C₄ alkyl)-, phenyl substituted with 0-3 R^{6e}, naphthyl substituted with 0-3 R^{6e},
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   - R^{9e}: is selected from:
   C₁-C₁₀ alkyl substituted with 0-1 R^{6e},
   C₁-C₁₀ alkoxy substituted with 0-2 R^{7e},
   H, nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, hydroxy, OR^{22e}, -N(R^{10e})R^{11e}, -N(R^{16e})R^{17e}, aryl (C₀-C₆ alkyl)carbonyl; aryl(C₁-C₆ alkyl), heteroaryl(C₁-C₆ alkyl), CONR^{18ae}R^{20e}, SO₂R^{18ae}, and SO₂NR^{18ae}R^{20e},
   providing that any of the above alkyl, cycloalkyl, aryl or heteroaryl groups may be unsubstituted or substituted independently with 1-2 R^{7e};
   - R^{6e}: is selected from:
   H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚ^{e}R^{11e}, SO₂NR^{10e}R^{11e},
   aryl substituted with 0-3 groups selected from halogen, C₁-C₆ alkoxy, C₁-C₆ alkyl, CF₃, S(O)ₘ^{e}Me, and -NMe₂ ,
   aryl(C₁-C₄ alkyl)-, said aryl being substituted with 0-3 groups selected from halogen, C₁-C₆ alkoxy, C₁-C₆ alkyl, CF₃ , S(O)ₚ^{e}Me, and -NMe₂, and
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   - R^{10e}: is selected from:
   H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
   - R^{11e}: is selected from:
   H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
   - R^{4e}: is selected from:
   H, C₁-C₆ alkyl, C₃-C₇ cycloalkyl, C₃-C₇ cycloalkyl(C₁-C₄ alkyl)-, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
   - R^{12e}: is selected from:
   H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy)carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl) sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
   - R^{16e}: is selected from:
   -C(=O)OR^{18ae}, -C (=O)R^{18be}, -C (=O) N (R^{18be})₂,-SO₂R^{18ae}, and -SO₂N(R^{18be})₂;
   - R^{17e}: is selected from:
   H, C₁-C₆ alkyl, C₃-C₇ cycloalkyl, C₃-C₇ cycloalkyl(C₁-C₄ alkyl)-, aryl, aryl(C₁-C₆ alkyl)-, and heteroaryl(C₁-C₆ alkyl);
   - R^{18ae}: is selected from:
   C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
   - R^{18be}: is H or R^{18ae},
   - R^{19e}: is selected from:
   H, halogen, CF₃, CO₂H, CN, NO₂, -NR^{11e}R^{12e}, OC_{F3}, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
   - R^{20e}: is selected from:
   hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-,
   C₁-C₅ alkoxy(C₁-C₅ alkyl)carbonyloxy(C₁-C₂ alkyl)ox
   (5-(C₁-C₅ alkyl)-1,3-dioxa-cyclopenten-2-one-yl)methyloxy,
   (5-aryl-1,3-dioxa-cyclopenten-2-one-yl)methyloxy, and
   (R^{10e}) (R^{11e})N-(C₁-C₁₀ alkoxy)-;
   - R^{21e}: is selected from:
   C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and
   C₁-C₁₀ alkyl substituted with 0-2 R^{7e};
   - R^{22e}: is selected from:
   -C(=O)-R^{18be}, -C(-O)N(R^{18be})2, -C(=O)NHSO₂R^{18ae}, -C(=O)NHC(=O)R^{18be}, -C (=O) NHC (=O) OR^{18ae}, and -C(=O)NHSO₂NHR^{18be};
   - m^{e}: is 0-2;
   - n^{e}: is 0-4; and
   - p^{e}: is 0-2;
   with the following proviso: n^{e} and m^{e} are chosen such that the number of atoms connecting R¹ and -COR^{20e} in Formula (IV) is in the range of 8-14;
   - d is: selected from 1, 2, 3, 4, and 5;
   - d': is 1-50;
   - W: is independently selected at each occurrence from the group: O, NH, NHC(=O), C(=O)NH, NR⁸C(=O), C(=O)N R⁸, C(=O), C(=O)O, OC(=O), NHC(=S)NH, NHC (=O) NH, SO₂, (OCH₂CH₂)ₛ, (CH₂CH₂O)_{s'}, (OCH₂CH₂CH2)_{s"}, (CH₂CH₂CH₂O)ₜ, and (aa)t,;
   - aa: is independently at each occurrence an amino acid;
   - Z: is selected from the group: aryl substituted with 0-1 R¹⁰, C₃₋₁₀ cycloalkyl substituted with 0-1 R¹⁰, and a 5-10 membered heterocyclic ring system containing 1-4 heteroatoms independently selected from N, S, and O and substituted with 0-1 R¹⁰;
   - R⁶, R^{6a}, R⁷, R^{7a}, and R⁸: are independently selected at each occurrence from the group: H, =0, COOH, SO₃H, C₁-C₅ alkyl substituted with 0-1 R¹⁰, aryl substituted with 0-1 R¹⁰, benzyl substituted with 0-1 R¹⁰, and C₁-C₅ alkoxy substituted with 0-1 R¹⁰, NHC(=O)R¹¹, C(=O)NHR¹¹, NHC(=O)NHR¹¹, NHR¹¹, R¹¹, and a bond to Cₕ;
   - k: is 0 or 1;
   - s: is selected from 0, 1, 2, 3, 4, and 5;
   - s': is selected from 0, 1, 2, 3, 4, and 5;
   - s": is selected from 0, 1, 2, 3, 4, and 5;
   - t: is selected from 0, 1, 2, 3, 4, and 5;
   - A¹, A², A³, A⁴, A⁵, A⁶, A⁷, and A⁸: are independently selected at each occurrence from the group: NR¹³, NR¹³R¹⁴, S, SH, S(Pg), OH, and a bond to Lₙ;
   - E: is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, and a 5-10 membered heterocyclic ring system containing 1-4 heteroatoms independently selected from N, S, and 0 and substituted with 0-3 R¹⁷;
   - R¹³, and R¹⁴: are each independently selected from the group: a bond to Lₙ, hydrogen, C₁-C₁₀ alkyl substituted with 0-3 R¹⁷, aryl substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
   alternatively, R¹³ and R¹⁴ combine to form =C(R²⁰)(R²¹);
   - R¹⁷: is independently selected at each occurrence from the group: a bond to Lₙ, =O, F, Cl, Br, I, -CF₃, -CN, -CO₂R¹⁸, -C (=O) R¹⁸, -C(=O)N(R¹⁸)₂, -CH₂OR¹⁸, -OC(=O)R¹⁸, -OC(=O)OR^{18a}, -OR¹⁸, -OC(=O)N(R¹⁸)_{2,} -NR¹⁹C(=O)R¹⁸, -NR¹⁹C(=O)OR^{18a}, -NR¹⁹C(=O)N(R¹⁸)₂, -NR¹⁹SO₂N(R¹⁸)2, -NR¹⁹SO₂R^{18a}, -SO₃H, -SO₂R^{18a}, -S(=O)R^{18a}, -SO₂N(R¹⁸)₂, -N(R¹⁸)₂, -NHC(=S)NHR¹⁸, =NOR¹⁸, -C(=O)NHNR¹⁸R^{18a}, -OCH₂CO₂H, and 2-(1-morpholino)ethoxy;
   - R¹⁸, R^{18a}, and R¹⁹: are independently selected at each occurrence from the group: a bond to Lₙ, H, and C₁-C₆ alkyl;
   - R²⁰ and R²¹: are independently selected from the group: H, C₁-C₅ alkyl, -CO₂R²⁵, C₂-C₅ 1-alkene substituted with 0-3 R²³, C₂-C₅ 1-alkyne substituted with 0-3 R²³, aryl substituted with 0-3 R²³, and unsaturated 5-10 membered heterocyclic ring system containing 1-4 heteroatoms independently selected from N, S, and O and substituted with 0-3 R²³;
   alternatively, R²⁰ and R²¹, taken together with the divalent carbon radical to which they are attached form: 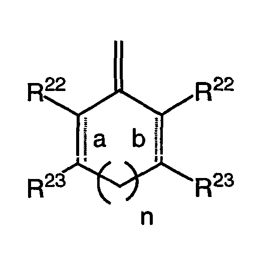
   - R²² and R²³: are independently selected from the group: H, and R²⁴;
   alternatively, R²², R²³ taken together form a fused aromatic or a 5-10 membered heterocyclic ring system containing 1-4 heteroatoms independently selected from N, S, and O;
   - R²⁴: is independently selected at each occurrence from the group: -CO₂R²⁵, -C(=O)N(R²⁵)₂, -CH₂OR²⁵, -OC(=O)R²⁵, -OR²⁵, -SO₃H, -N(R²⁵)₂, and -OCH₂CO₂H; and,
   - R²⁵: is independently selected at each occurrence from the group: H and C₁-C₃ alkyl.
[22] In another embodiment, the present invention provides a therapeutic radiopharmaceutical composition according to Embodiment [20], wherein:
   - R^{1e}: is selected from: 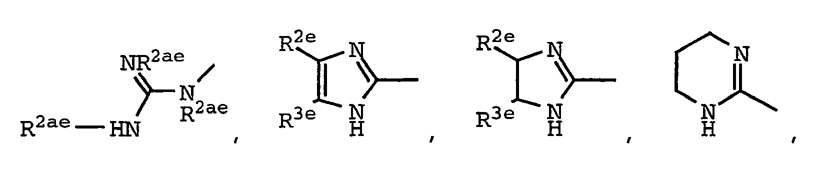 and 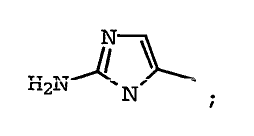
   - R^{2e} and R^{3e}: are independently selected from:
   H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
   alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
   - R^{2ae}: is selected from:
   H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl,
   (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cyclcalkylcarbonyloxy(C₁-C₄ alkoxy) carbonyl;
   - R^{7e}: is selected from:
   H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
   - U^{e}: is selected from:
   -(CH₂)ₙ^{e}-, -NH(CH₂)n^{e}-, -N(R^{10e})C(=O)-, and -NHC(=O)(CH₂)ₙ^{e};
   - G^{e}: is N or CR^{19e};
   - R^{8e}: is H;
   - R^{9e}: is selected from:
   H, nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, hydroxy, OR^{22e}, -N(R^{10e})R^{11e}, -N(R^{16e})R^{17e}, aryl(C₀-C₄ alkyl)carbonyl, aryl(C₁-C₄ alkyl), heteroaryl(C₁-C₄ alkyl), CONR^{18ae}R^{20e}, SO₂R^{18ae}, and SO₂NR^{18ae}R^{20e},
   providing that any of the above alkyl, cycloalkyl, aryl or heteroaryl groups may be unsubstituted or substituted independently with 1-2 R^{7e};
   - R^{10e}: is selected from:
   H, -OH, CF₃, C₃-C₆ alkenyl, C₃-C₆ cycloalkyl, aryl, (C₃-C₆ cycloalkyl)methyl, aryl(C₁-C₄ alkyl), and C₁-C₄ alkyl substituted with 0-2 R^{6e};
   - R^{6e}: is selected from:
   H, C₁-C₄ alkyl, hydroxy, C₁-C₄ alkoxy, nitro, C₁-C₄ alkylcarbonyl, -N(R^{11e})R^{12e}, cyano, halo, CF₃, CHO, CO₂R^{18be}, C(=O)R^{18be}, CONR^{17e}R^{18be} OC(=O)R^{10e}, OR^{10e}, OC(=O)NR^{10e}R^{11e}, NR^{10e}C(=O)R^{10e}, NR^{10e}C(=O)OR^{21e}, NR¹⁰C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
   aryl substituted with 0-3 groups selected from halogen, C₁-C₄ alkoxy, C₁-C₄ alkyl, CF₃, S(O)ₘ^{e}Me, and -NMe₂,
   aryl(C₁-C₄ alkyl)-, said aryl being substituted with 0-3 groups selected from halogen, C₁-C₄ alkoxy, C₁-C₄ alkyl, CF₃, S(O)_{P}^{e}Me, and -NMe₂, and
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   - R^{11e}: is selected from:
   H, hydroxy, C₁-C₄ alkyl, C₃-C₆ alkenyl, C₃-C₆ cycloalkyl, (C₃-C₆ cycloalkyl)methyl, C₁-C₄ alkoxy, benzyloxy, aryl, heteroaryl, heteroaryl(C₁-C₄ alkyl)-, aryl(C₁-C₄ alkyl); adamantylmethyl, and
   C₁-C₄ alkyl substituted with 0-2 R^{4e};
   - R^{4e}: is selected from:
   H, C₁-C₄ alkyl, C₃-C₇ cycloalkyl, C₃-C₇ cycloalkyl(C₁-C₄ alkyl)-, aryl, heteroaryl, aryl(C₁-C₄ alkyl)-, and heteroaryl(C₁-C₄ alkyl)-,
   wherein said aryl or heteroaryl groups are substituted with 0-2 substituents independently selected from the group consisting of C₁-C₄ alkyl, C₁-C₄ alkoxy, F, Cl, Br, CF₃, and NO₂,
   - R^{12e}: is selected from:
   H, C₁-C₄ alkyl, (C₁-C₄ alkyl)carbonyl, (C₁-C₄ alkoxy)carbonyl, phenyl(C₁-C₄ alkyl)-; phenylsulfonyl, phenyloxycarbonyl, and phenyl(C₁-C₄ alkoxy)carbonyl, wherein said phenyl groups are substituted with 0-2 substituents selected from the group consisting of C₁-C₄ alkyl, C₁-C₄ alkoxy, halo, CF₃, and nitro;
   - R^{16e}: is selected from:
   -C(=O)OR^{18ae} -C(=O)R^{18be}, -C(=O)N(R^{18be})₂,-SO₂R^{18ae}, and -SO₂N(R^{18be})₂,
   - R^{17e}: is selected from:
   H, C₁-C₄ alkyl, C₃-C₆ cycloalkyl, C₃-C₆ cycloalkyl(C₁-C₄ alkyl)-, aryl, aryl(C₁-C₆ alkyl)-, and heteroaryl(C₁-C₆ alkyl);
   - R^{18ae}: is selected from:
   C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
   - R^{18be}: is H or R^{18ae};
   - R^{19e}: is selected from:
   H, halogen, CF₃, CO₂H, CN, NO₂, -NR^{11e}R^{12e}, OCF₃, C₁-C₆ alkyl, C₂-C₆ alkenyl, C₂-C₆ alkynyl, C₃-C₆ cycloalkyl, C₃-C₆ cycloalkyl(C₁-C₄ alkyl)-, aryl(C₁-C₄ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
   - R^{20e}: is selected from:
   hydroxy, C₁-C₆ alkyloxy, C₃-C₆ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
   (5-(C₁-C₅ alkyl)-1,3-dioxa-cyclopenten-2-one-yl)methyloxy,
   (5-aryl-1,3-dioxa-cyclopenten-2-one-yl)methyloxy, and
   (R^{10e})(R^{11e})N-(C₁-C₁₀ alkoxy)-;
   - R^{21e}: is selected from:
   C₁-C₄ alkyl, C₂-C₆ alkenyl, C₃-C₆ cycloalkyl, (C₃-C₆ cycloalkyl)methyl, aryl, aryl(C₁-C₄ alkyl)-, and C₁-C₁₀ alkyl substituted with 0-2 R^{7e};
   - R^{22e}: is selected from:
   -C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, and -C(=O)NHSO₂NHR^{18be};
   - m^{e}: is 0-2;
   - n^{e}: is 0-4;
   - p^{e}: is 0-2;
   - Cₕ: is 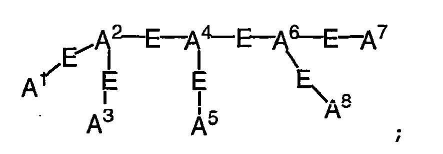
   - A¹: is selected from the group: OH, and a bond to Lₙ;
   - A², A⁴, and A⁶: are each N;
   - A³, A⁵, and A⁸: are each OH;
   - A⁷: is a bond to Lₙ or NH-bond to Lₙ;
   - E: is a C₂ alkyl substituted with 0-1 R¹⁷;
   - R¹⁷: is =O;
   alternatively, Cₕ is 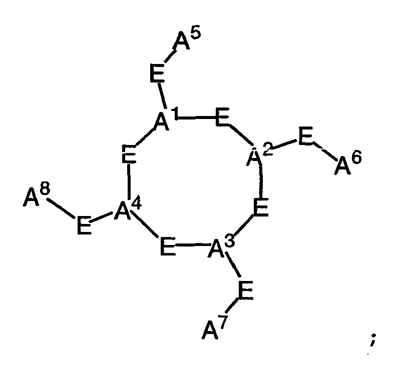
   - A¹: is selected from the group: OH, and a bond to Lₙ;
   - A², A³ and A⁴: are each N;
   - A⁵, A⁶ and A⁸: are each OH;
   - A⁷: is a bond to Lₙ;
   - E: is a C₂ alkyl substituted with 0-1 R¹⁷;
   - R¹⁷: is =O;
   alternatively, Cₕ is 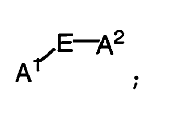
   - A¹: is NH₂ or N=C(R²⁰)(R²¹);
   - E: is a bond;
   - A²: is NHR¹³;
   - R¹³: is a heterocycle substituted with R¹⁷, the heterocycle being selected from pyridine and pyrimidine;
   - R¹⁷: is selected from a bond to Lₙ, C(=O)NHR¹⁸ and C(=O)R¹⁸;
   - R¹⁸: is a bond to Lₙ;
   - R²⁴: is selected from the group: -CO₂R²⁵, -OR²⁵, -SO₃H, and -N(R²⁵)₂; and,
   - R²⁵: is independently selected at each occurrence from the group: hydrogen and methyl.
[23] In another embodiment, the present invention provides a therapeutic radiopharmaceutical composition according to Embodiment [20], wherein Q is selected from the group:
   3-[7-[(imidazolin-2-ylamino)methyl]-1-methyl-6,8-difluoroquinoline-4-one-3-ylcarbonylamino]-2-(3,5-dimethylisoxazol-4-ylsulfonylamino)propionic acid,
   3-[7-[(imidazolin-2-ylamino)methyl]-1-methyl-6,8-difluoroquinoline-4-one-3-ylcarbonylamino]-2-(benzyloxycarbonylamino)propionic acid,
   3-[7-[(imidazolin-2-ylamino)methyl]-1-methyl-6,8-difluoroquinoline-4-one-3-ylcarbonylamino]-2-(n-butyloxycarbonylamino)propionic acid,
   3-[7-[(imidazolin-2-ylamino)methyl]-1-methyl-6,8-difluoroquinoline-4-one-3-ylcarbonylamino]-2-(n-butylsulfonylamino)propionic acid,
   3-[7-[(tetrahydropyrimid-2-ylamino)methyl]-1-methyl-6,8-difluoroquinoline-4-one-3-ylcarbonylamino]-2-(benzyloxycarbonylamino)propionic acid,
   3-[7-[(tetrahydropyrimid-2-ylamino)methyl]-1-methyl-6,8-difluoroquinoline-4-one-3-ylcarbonylamino]-2-(n-butyloxycarbonylamino)propionic acid,
   3-[7-[(tetrahydropyrimid-2-ylamino)methyl]-1-methyl-6,8-difluoroquinoline-4-one-3-ylcarbonylamino]-2-(phenylsulfonylamino)propionic acid,
   3-[7-[(tetrahydropyrimid-2-ylamino)methyl]-1-methyl-6,8-difluoroquinoline-4-one-3-ylcarbonylamino]-2-(n-butylsulfonyl)aminopropionic acid,
   3-[7-[(2-aminothiazol-4-yl)methyl]-1-methyl-6,8-difluoroquinoline-4-one-3-ylcarbonylamino]-2-(benzyloxycarbonylamino)propionic acid,
   3-[7-[(imidazolin-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(tetrahydropyrimid-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(imidazol-2-ylamino)methyl]-1-methyl-6,8-difluoroquinoline-4-one-3-ylcarbonylamino]-2-(3,5-dimethylisoxazol-4-ylsulfonylamino)propionic acid,
   3-[7-[(imidazol-2-ylamino)methyl]-1-methyl-6,8-difluoroquinoline-4-one-3-ylcarbonylamino]-2-(benzyloxycarbonylamino)propionic acid,
   3-[7-[(imidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(imidazol-2-ylamino)methyl]-1-methyl-6,8-difluoroquinoline-4-one-3-ylcarbonylamino]-2-((4-biphenyl)sulfonylamino)propionic acid,
   3-[7-[(imidazol-2-ylamino)methyl]-1-methyl-6,8-difluoroquinoline-4-one-3-ylcarbonylamino]-2-(1-naphthylsulfonylamino)propionic acid,
   3-[7-[(benzimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(4-methylimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(4,5-dimethylimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(4,5,6,7-tetrahydrobenzimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(pyridin-2-ylamino)methyl]-1-methyl-6,8-difluoroquinoline-4-one-3-ylcarbonylamino]-2-((2,4,6-trimethylphenyl)sulfonylamino)propionic acid,
   3-[7-(2-aminopyridin-6-yl)-1-methyl-6,8-difluoroquinoline-4-one-3-ylcarbonylamino]-2-((2,4,6-trimethylphenyl)sulfonylamino)propionic acid,
   3-[7-[(7-azabenzimidazol-2-yl)methyl]-1-methyl-6,8-difluoroquinoline-4-one-3-ylcarbonylamino]-2-((2,4,6-trimethylphenyl)sulfonylamino)propionic acid,
   3-[7-[(benzimidazol-2-ylamino)methyl]-1-(2-phenylethyl)-6,8-difluoroquinoline-4-one-3-ylcarbonylamino]pro-pionic acid,
   3-[7-[(pyridin-2-ylamino)methyl]-1-(2-phenylethyl)-6,8-difluoroquinoline-4-one-3-ylcarbonylamino]propionic acid,
   3-[7-[(imidazolin-2-ylamino)methyl]-1-(2-phenylethyl)-6,8-difluoroquinoline-4-one-3-ylcarbonylamino]propionic acid,
   3-[7-[(imidazol-2-ylamino)methyl]-1-(2-phenylethyl)-6,8-difluoroquinoline-4-one-3-ylcarbonylamino]propionic acid,
   3-[7-[(imidazolin-2-ylamino)methyl]-1-(2-phenylethyl)-6,8-difluoroquinoline-4-one-3-ylcarbonylamino]-2-(benzyloxycarbonylamino)propionic acid,
   3-[7-[(imidazolin-2-ylamino)methyl]-1-(2-phenylethyl)-6,8-difluoroquinoline-4-one-3-ylcarbonylamino]-2-(n-butyloxycarbonylamino)propionic acid,
   3-[7-[(imidazolin-2-ylamino)methyl]-1-(2-phenylethyl)-6,8-difluoroquinoline-4-one-3-ylcarbonylamino]-2-(phenylsulfonylamino)propionic acid,
   3-[7-[(imidazolin-2-ylamino)methyl]-1-(2-phenylethyl)-6,8-difluoroquinoline-4-one-3-ylcarbonylamino]-2-(n-butylsulfonylamino)propionic acid,
   3-[7-[(tetrahydropyrimid-2-ylamino)methyl]-1-(2-phenylethyl)-6,8-difluoroquinoline-4-one-3-ylcarbonylamino]-2-(benzyloxycarbonylamino)propionic acid,
   3-[7-[(tetrahydropyrimid-2-ylamino)methyl]-1-(2-phenylethyl)-6,8-difluoroquinoline-4-one-3-ylcarbonylamino]-2-(n-butyloxycarbonylamino)propionic acid,
   3-[7-[(tetrahydropyrimid-2-ylamino)methyl]-1-(2-phenylethyl)-6,8-difluoroquinoline-4-one-3-ylcarbonylamino]-2-(phenylsulfonylamino)propionic acid,
   3-[7-[(tetrahydropyrimid-2-ylamino)methyl]-1-(2-phenylethyl)-6,8-difluoroquinoline-4-one-3-ylcarbonylamino]-2-(n-butylsulfonyl)aminopropionic acid,
   3-[7-[(2-aminothiazol-4-yl)methyl]-1-(2-phenylethyl)-6,8-difluoroquinoline-4-one-3-ylcarbonylamino]-2-(phenylsulfonylamino)propionic acid,
   3-[7-[(2-aminothiazol-4-yl)methyl]-1-(2-phenylethyl)-6,8-difluoroquinoline-4-one-3-ylcarbonylamino]-2-(benzyloxycarbonylamino)propionic acid,
   3-[7-[(imidazolin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(tetrahydropyrimid-2-ylamino)methyl]-1-(2-phenylethyl)-6,8-difluoroquinoline-4-one-3-ylcarbonylamino]-2-((2,4,6-trimethylphenyl)sulfon-ylamino)propionic acid,
   3-[7-[(imidazol-2-ylamino)methyl]-1-(2-phenylethyl)-6,8-difluoroquinoline-4-one-3-ylcarbonylamino]-2-(benzyloxycarbonylamino)propionic acid,
   3-[7-[(imidazol-2-ylamino)methyl]-1-(2-phenylethyl)-6,8-difluoroquinoline-4-one-3-ylcarbonylamino]-2-(phenylsulfonylamino)propionic acid,
   3-[7-[(imidazol-2-ylamino)methyl]-1-(2-phenylethyl)-6,8-difluoroquinoline-4-one-3-ylcarbonylamino]-2-((2,6,dichlorophenyl)sulfonylamino)propionic acid,
   3-[7-[(imidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(imidazol-2-ylamino)methyl]-1-(2-phenylethyl)-6,8-difluoroquinoline-4-one-3-ylcarbonylamino]-2-((4-biphenyl)sulfonylamino)propionic acid,
   3-[7-[(benzimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(4-methylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(4,5-dimethylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(4,5,6,7-tetrahydrobenzimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(pyridin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-(2-aminopyridin-6-yl)-1-(2-phenylethyl)-6,8-difluoroquinoline-4-one-3-ylcarbonylamino]-2-((2,4,6-trimethylphenyl)sulfonylamino)propionic acid, and
   3-[7-[(7-azabenzimidazol-2-yl)methyl]-1-(2-phenylethyl)-6,8-difluoroquinoline-4-one-3-ylcarbonylamino]-2-((2,4,6-trimethylphenyl)sulfonylamino)propionic acid
[24] A composition according to Embodiment [18], wherein the radioisotope is ¹⁵³Sm.
[25] In another embodiment, the present invention provides a composition according to Embodiment [18], wherein the radioisotope is ¹⁷⁷Lu.
[26] In another embodiment, the present invention provides a composition according to Embodiment [25], wherein the radiopharmaceutical is selected from the group: 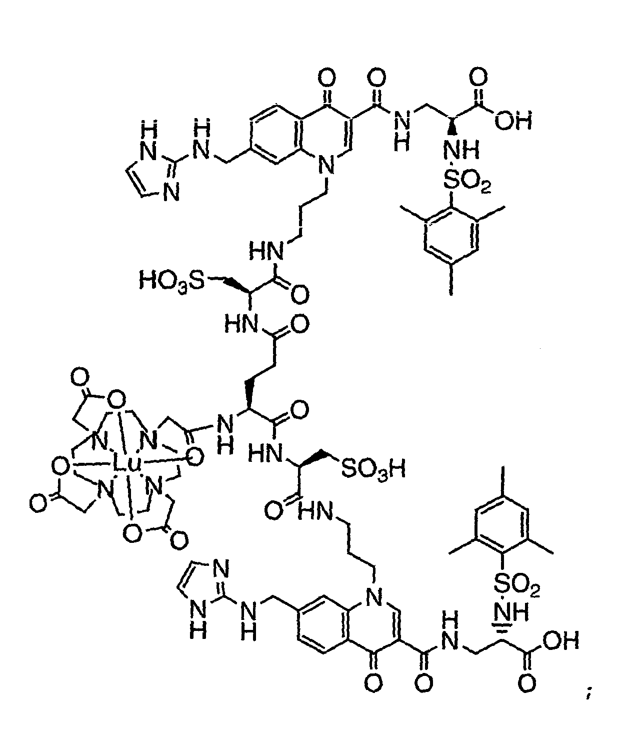 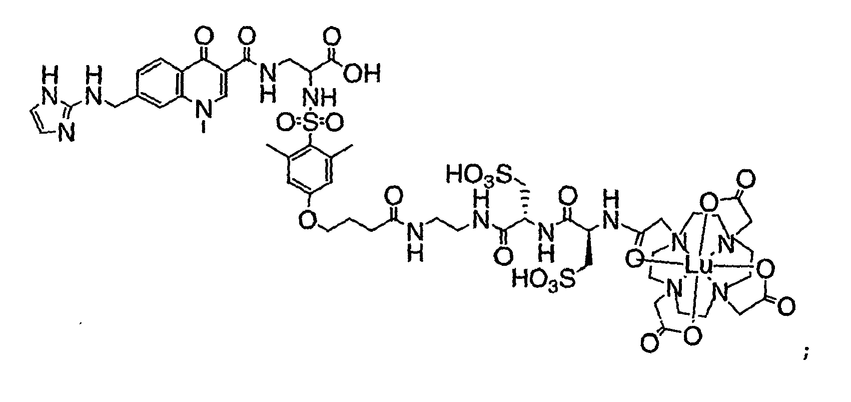 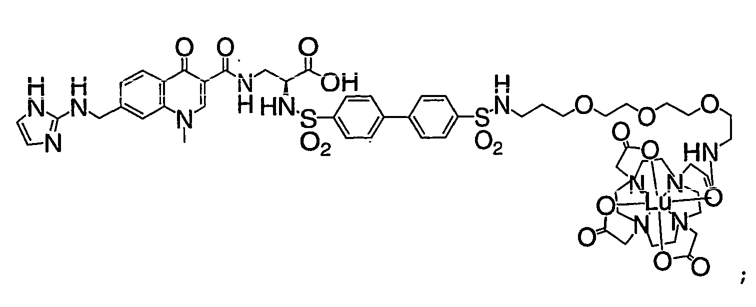 and 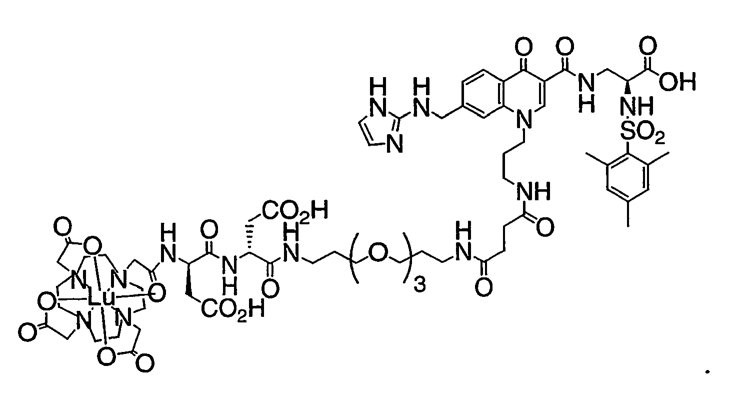
[27] In another embodiment, the present invention provides a composition according to Embodiment [18], wherein the radioisotope is ⁹⁰Y.
[28] In another embodiment, the present invention provides a composition according to Embodiment [27], wherein the radiopharmaceutical is selected from the group; 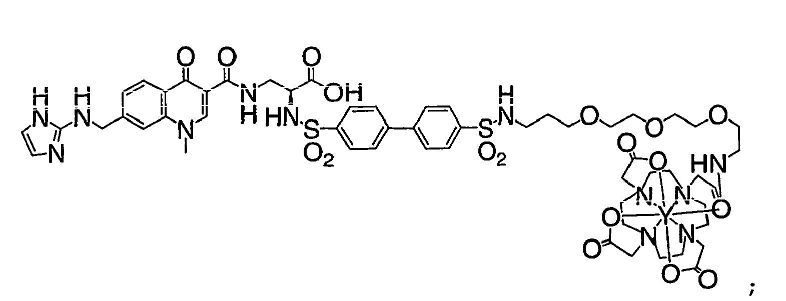 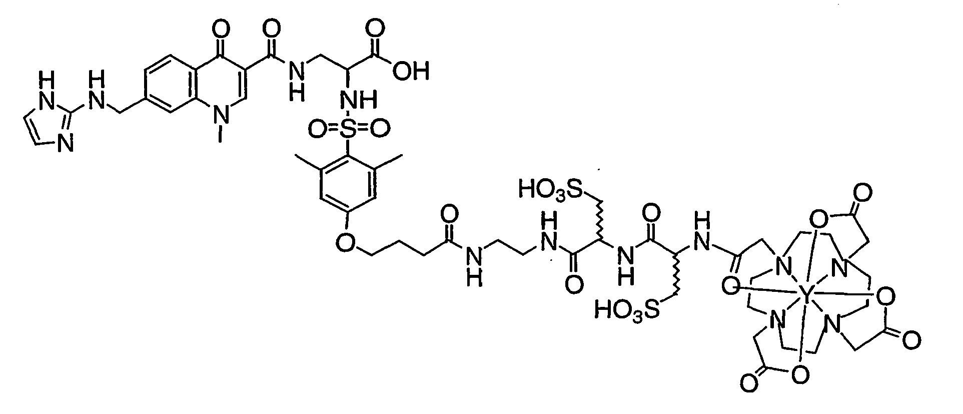 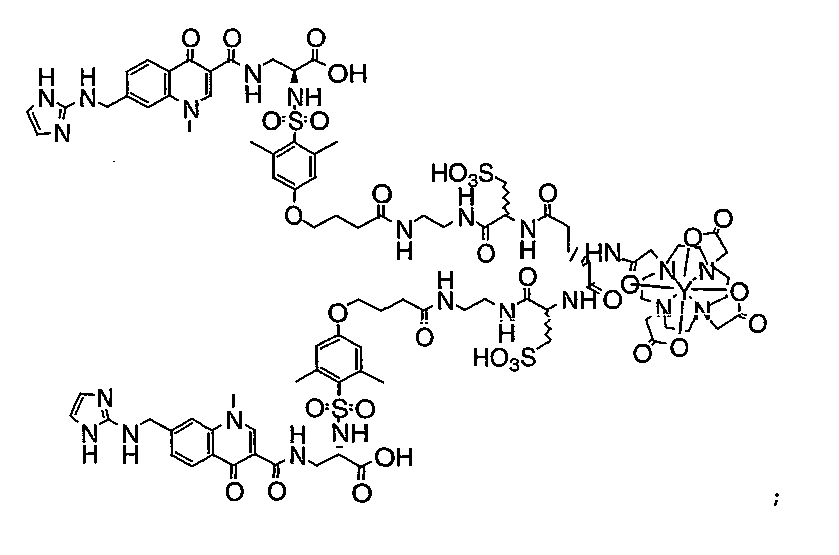 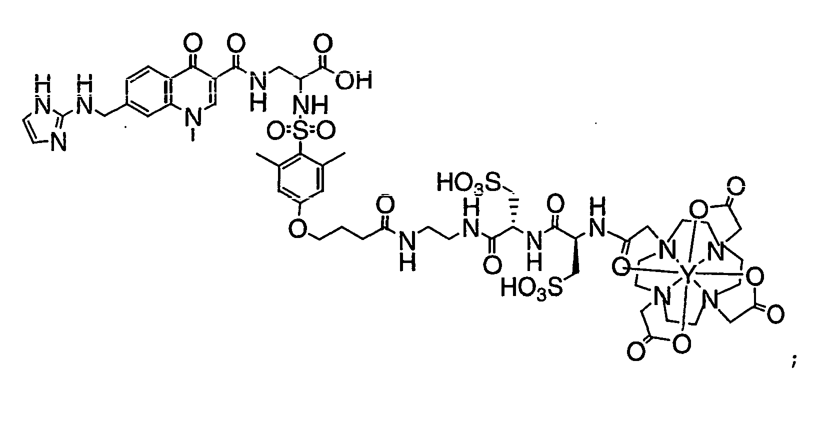 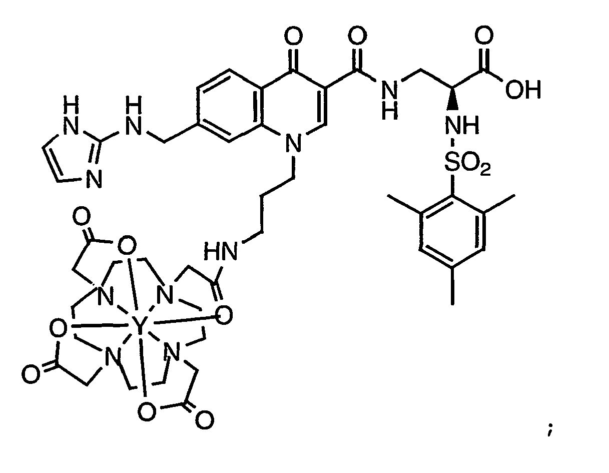 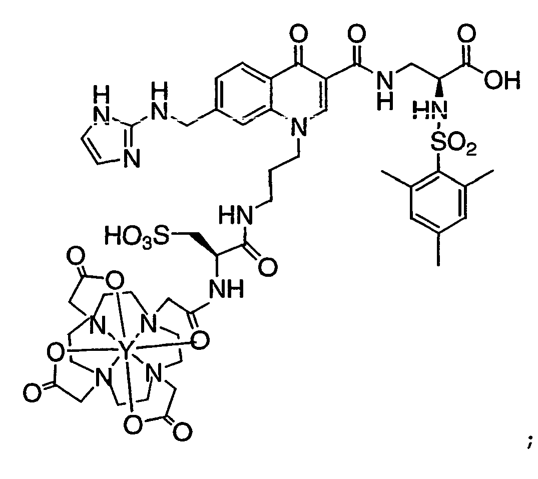 and 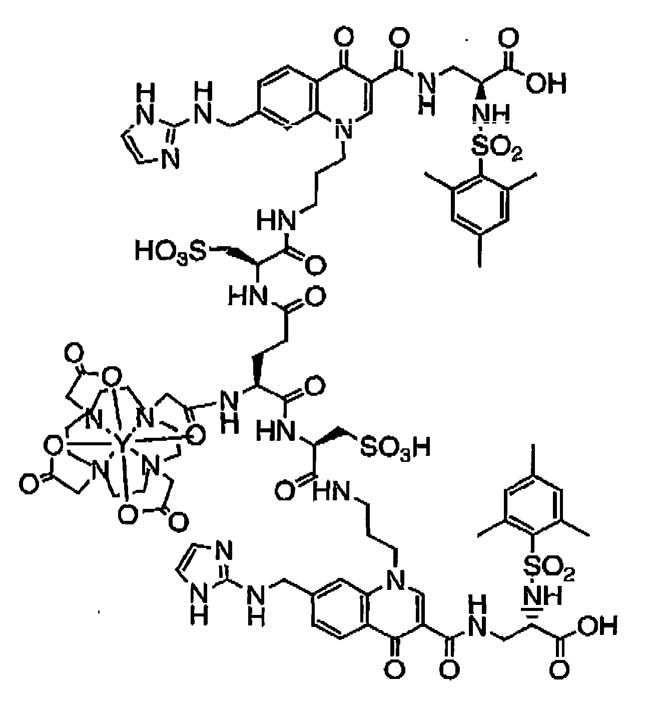
[29] In another embodiment, the present invention provides for the use of a therapeutic radiopharmaceutical comprising:
   a) a metal;
   b) a chelator capable of chelating the metal; and
   c) a targeting moiety; wherein the targeting moiety is bound to the chelator through a linking group, and the targeting moiety is a quinolone non-peptide that binds to a receptor that is upregulated during angiogenesis, and the metal is a radioisotope selected from the group: ³³P,¹²⁵I, ¹⁸⁶Re, ¹⁸⁸Re,¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁶⁹Gd, ¹⁴⁰La, ¹⁹⁸Au, ¹⁹⁹Au,¹⁸⁹Yb, ¹⁷⁵Yb, ¹⁶⁵Dy, ¹⁶⁶Dy, ⁶⁷Cu, ¹⁰⁵Rh, ¹¹¹Ag, and ¹⁸²Ir or a pharmaceutically acceptable salt thereof, and at least one agent selected from the group consisting of an anticancer agent and a radiosensitizer agent, or a pharmaceutically acceptable salt thereof; for the manufacture of a medicament to be administered to a patient in need thereof for treating cancer.
[30] In another embodiment, the present invention provides a use according to Embodiment [29] wherein administering the therapeutic radiopharmaceutical and agent is concurrent.
[31] In another embodiment, the present invention provides a use according to Embodiment [29] wherein administering the therapeutic radiopharmaceutical and agent is sequential.
[32] In another embodiment, the present invention provides a use according to Embodiment [29] wherein the cancer is selected from the group consisting of carcinomas of the lung, breast, ovary, stomach, pancreas, larynx, esophagus, testes, liver, parotid, biliary tract, colon, rectum, cervix, uterus, endometrium, kidney, bladder, prostate, thyroid, squamous cell carcinomas, adenocarcinomas, small cell carcinomas, melanomas, gliomas, and neuroblastomas.
[33] In another embodiment, the present invention provides a use according to Embodiment [29] wherein the anticancer agent is selected from the group consisting of mitomycin, tretinoin, ribomustin, gemcitabine, vincristine, etoposide, cladribine, mitobronitol, mathotrexate, doxorubicin, carboquone, pentostatin, nitracrine, zinostatin, cetrorelix letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, and leutinizing hormone releasing factor.
[34] In another embodiment, the present invention provides a use according to Embodiment [29] wherein the radiosensitizer agent is selected from the group consisting of 2-(3-nitro-1,2,4-triazol-1-yl)-N-(2-methoxyethyl)acetamide, N-(3-nitro-4-quinolinyl)-4-morpholinecarboxamidine, 3-amino-1,2,4-benzotriazine-1,4-dioxide, N-(2-hydroxyethyl)-2-nitroimidazole-1-acetamide, 1-(2-nitroimidazol-1-yl)-3-(1-piperidinyl)-2-propanol, and 1-(2-nitro-1-imidazolyl)-3-(1-aziridino)-2-propanol.
[35] In another embodiment, the present invention provides a use according to Embodiment [29] wherein the anti-cancer agent is a chemotherapeutic agent.
[36] In another embodiment, the present invention provides a use according to Embodiment [29], wherein the administration is by injection or infusion.
[37] In another embodiment, the present invention provides a use according to Embodiment [29], wherein the therapeutic radiopharmaceutical comprises:
   a) a metal;
   b) a chelator capable of chelating the metal; and
   c) a targeting moiety;
   wherein the targeting moiety is bound to the chelator through a linking group, and the targeting moiety is a quinolone non-peptide that binds to a receptor that is upregulated during angiogenesis, and the metal is a radioisotope selected from the group: ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir.
[38] In another embodiment, the present invention provides a use according to Embodiment [37], wherein the targeting moiety is a quinolone non-peptide and the receptor is αᵥβ₃ or αᵥβ₅.
[39] In another embodiment, the present invention provides a use according to Embodiment [37], wherein the therapeutic radiopharmaceutical comprises:
   a) a radioisotope selected from the group: ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir; and
   b) a compound of the formula (I):

      (Q)_{d}-Lₙ-Cₕ or (Q)_{d}-Lₙ-(Cₕ)_{d'} (I)
   wherein, Q is a compound of Formula (II): 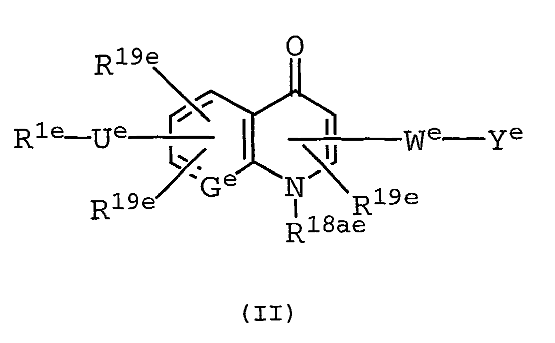 including stereoisomeric forms thereof, or mixtures of stereoisomeric forms thereof, or pharmaceutically acceptable salt or prodrug forms thereof wherein:
   - R^{1e}: is selected from: 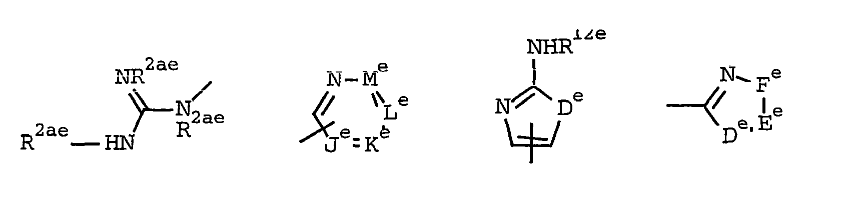 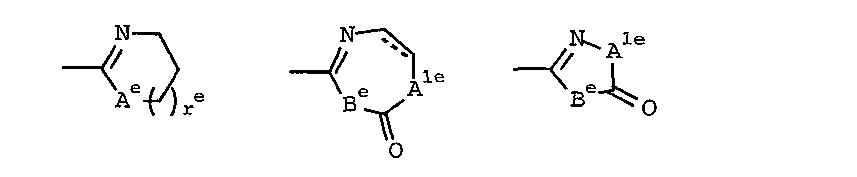 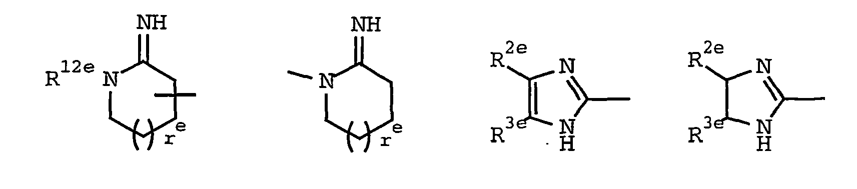 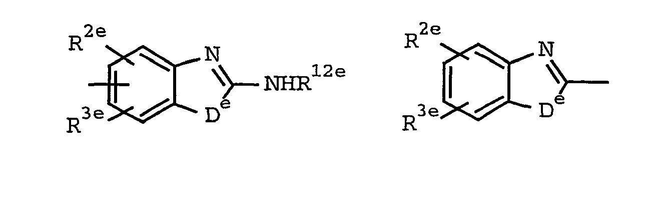 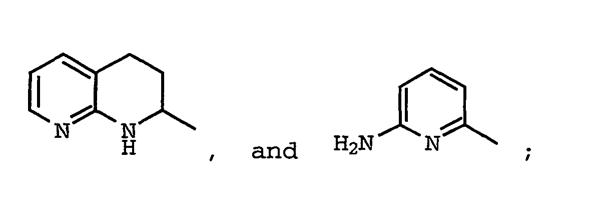
   A^{e} is -CH₂- or -N(R^{10e})-;
   A^{1e} and Be are independently -CH₂- or -N(R^{10e})-;
   D^{e} is -N(R^{10e})- or -S-;
   E^{e}-F^{e} is -C(R^{2e})=C(R^{3e})- or -C(R^{2e})₂C(R^{3e})₂-;
   J^{e} is -C(R^{2e})- or -N-;
   K^{e}, L^{e} and M^{e} are independently -C(R^{2e})- or -C(R^{3e})-;
   R^{2e} and R^{3e} are independently selected from:
   H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
   alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
   R^{2ae} is selected from:
   H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
   R^{7e} is selected from:
   H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
   U^{e} is selected from:
   -(CH₂)ₙ^{e}-, -(CH₂)ₙ^{e}O(CH₂)ₘ^{e}-,-(CH₂)ₙ^{e}N(R¹²)(CH₂)ₘ^{e}-, -NH(CH₂)ₙ^{e}-,-(CH₂)ₙ^{e}C(=O)(CH₂)ₘ^{e}-,
   -(CH₂)ₙ^{e}S(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})-, and -N(R^{10e})S(O)ₚ^{e}-;
   G^{e} is N or CR^{19e};
   W^{e} is -C(=O)-N(R^{10e})-(C₁-C₃ alkylene)-, in which the alkylene group is substituted by R^{8e} and by R^{9e}:
   R^{8e} and R^{9e} are independently selected from:
   H, CO₂R^{18be}, C(=O)R^{18be}, CONR¹⁷R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl, C₃-C₁₀ cycloalkyl(C₁-C₄ alkyl)-, phenyl substituted with 0-3 R^{6e}, naphthyl substituted with 0-3 R^{6e},
   a 5-10 membered heterocyclic ring containing 1-3 N, 0, or S heteroatoms, wherein said heterocyclic ring may be saturated, partially saturated, or fully unsaturated, said heterocyclic ring being substituted with 0-2 R^{7e},
   C₁-C₁₀ alkoxy substituted with 0-2 R^{7e}, hydroxy, nitro, -N(R^{10e})R^{11e}, -N(R^{16e})R^{17e}, aryl (C₀-C₆ alkyl)carbonyl, aryl (C₃-C₆ alkyl), heteroaryl (C₁-C₆ alkyl), CONR^{18ae}R^{20e}, SO₂R^{18ae}, and SO₂NR^{18ae}R^{20e},
   providing that any of the above alkyl, cycloalkyl, aryl or heteroaryl groups may be unsubstituted or substituted independently with 1-2 R^{7e};
   R^{6e} is selected from:
   H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
   aryl substituted with 0-3 groups selected from halogen, C₁-C₆ alkoxy, C₁-C₆ alkyl, CF₃, S(O)ₘ^{e}Me, and -NMe₂,
   aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   R^{10e} is selected from:
   H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
   R^{11e} is selected from:
   H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
   R^{4e} is selected from:
   H, C₁-C₆ alkyl, C₃-C₇ cycloalkyl, C₃-C₇ cycloalkyl(C₁-C₄ alkyl)-, (C₁-C₁₀ alkyl)carbonyl, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂, alternatively, when R^{10e} and R^{11e} are both substituents on the same nitrogen atom (as in -NR^{10e}R^{11e}) they may be taken together with the nitrogen atom to which they are attached to form a heterocycle selected from: 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl, and 1-piperazinyl;
   said heterocycle being substituted with 0-3 groups selected from: C₁-C₆ alkyl, aryl, heteroaryl, aryl(C₁-C₄ alkyl)-, (C₁-C₆ alkyl)carbonyl, (C₃-C₇ cycloalkyl)carbonyl, (C₁-C₆ alkoxy)carbonyl, aryl(C₁-C₄ alkoxy)carbonyl, C₁-C₆ alkylsulfonyl, and arylsulfonyl;
   R^{12e} is selected from:
   H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy)carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
   R^{16e} is selected from:
   -C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, and -SO₂NHC(=O)OR^{18be};
   R^{17e} is selected from:
   H, C₁-C₆ alkyl, C₃-C₇ cycloalkyl, C₃-C₇ cycloalkyl(C₁-C₄ alkyl)-, aryl, aryl(C₁-C₆ alkyl)-, and heteroaryl(C₁-C₆ alkyl);
   R^{18ae} is selected from:
   C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl (C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
   R^{18be} is H or R^{18ae};
   R^{19e} is selected from:
   H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
   R^{20e} is selected from:
   hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl) oxy-, C₂-C₁₀ alkoxycarbonyl(C₁-C₂ alkyl) oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl) carbonyloxy(C₁-C₂ alkyl)ox y,
   (5-(C₁-C₅ alkyl)-1,3-dioxa-cyclopenten-2-one-yl)methyloxy,
   (5-aryl-1,3-dioxa-cyclopenten-2-one-yl)methyloxy, and
   (R^{10e})(R^{11e})N-(C₁-C₁₀ alkoxy)-;
   R^{21e} is selected from:
   C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and C₁-C₁₀ alkyl substituted with 0-2 R^{7e};
   R^{22e} is selected from:
   -C(=O)-R^{18be}, -C(=O)N(R^{18be})₂, -C(=O)NHSO₂R^{18ae},-C(=O)NHC(=O)R^{18be}, -C(=O)NHC(=O)OR^{18ae}, and-C(=O)NHSO₂NHR^{18be};
   Y^{e} is selected from:
   -COR^{20e}, -SO₃H, -PO₃H, -CONHNHSO₂CF₃,-CONHSO₂R^{18ae}, -CONHSO₂NHR^{18be}, -NHCOCF₃,-NHCONHSO₂R^{18ae}, -NHSO₂R^{18ae}, -OPO₃H₂, -OSO₃H,-PO₃H₂, -SO₂NHCOR^{18ae},
   -SO₂NHCO₂R^{18ae}, 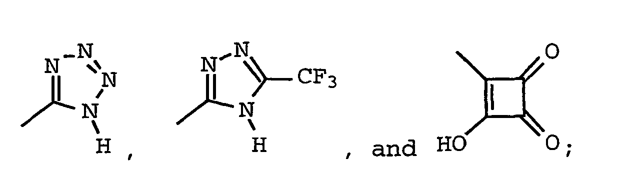
   m^{e} is 0-2;
   n^{e} is 0-4;
   p^{e} is 0-2;
   r^{e} is 0-2; with the following proviso: n^{e} and m^{e} are chosen such that the number of atoms connecting R^{1e} and Y^{e} is in the range of 8-14;
   - d: is selected from 1, 2, 3, 4, 5, 6, 7, 8, 9, and 10;
   - d': is 1-100;
   - Lₙ: is a linking group having the formula:

   ((W)ₕ-(CR⁶R⁷)_{g})ₓ-(Z)ₖ-((CR^{6a}R^{7a})_{g'}-(W)_{h'})_{x'};
   - W: is independently selected at each occurrence from the group: O, S, NH, NHC(=O), C(=O)NH, NR⁸C(=O), C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ, and (aa)_{t'};
   - aa: is independently at each occurrence an amino acid;
   - Z: is selected from the group: aryl substituted with 0-3 R¹⁰, C₃₋₁₀ cycloalkyl substituted with 0-3 R¹⁰, and a 5-10 membered heterocyclic ring system containing 1-4 heteroatoms independently selected from N, S, and O and substituted with 0-3 R¹⁰;
   - R⁶, R^{6a}, R⁷, R^{7a}, and R⁸: are independently selected at each occurrence from the group: H, =O, COOH, SO₃H, PO₃H, C₁-C₅ alkyl substituted with 0-3 R¹⁰, aryl substituted with 0-3 R¹⁰, benzyl substituted with 0-3 R¹⁰, and C₁-C₅ alkoxy substituted with 0-3 R¹⁰, NHC(=O)R¹¹, C(=O)NHR¹¹, NHC(=O)NHR¹¹, NHR¹¹, R¹¹, and a bond to Cₕ;
   - R¹⁰: is independently selected at each occurrence from the group: a bond to Cₕ, COOR¹¹, C(=O)NHR¹¹, NHC(=O)R¹¹, OH, NHR¹¹, SO₃H, PO₃H, -OPO₃H₂, -OSO₃H, aryl substituted with 0-3 R¹¹, C₁₋₅ alkyl substituted with 0-1 R¹², C₁₋₅ alkoxy substituted with 0-1 R¹², and a 5-10 membered heterocyclic ring system containing 1-4 heteroatoms independently selected from N, S, and O and substituted with 0-3 R¹¹;
   - R¹¹: is independently selected at each occurrence from the group: H, -OPO₃H₂,, -OSO₃H C₁₋₅ alkyl substituted with 0-1 R¹², aryl substituted with 0-1 R¹², a 5-10 membered heterocyclic ring system containing 1-4 heteroatoms independently selected from N, S, and 0 and substituted with 0-1 R¹², C₃₋₁₀ cycloalkyl substituted with 0-1 R¹², polyalkylene glycol substituted with 0-1 R¹²,, C₁-₅ alkyl substituted with carbohydrate substituted with 0-1 R¹², cyclodextrin substituted with 0-1 R¹², amino acid substituted with 0-1 R¹², polycarboxyalkyl substituted with 0-1 R¹², polyazaalkyl substituted with 0-1 R¹², peptide substituted with 0-1 R¹², wherein the peptide is comprised of 2-10 amino acids, 3,6-O-disulfo-B-D-galactopyranosyl, bis(phosphonomethyl)glycine, and a bond to Cₕ;
   - R¹²: is a bond to Cₕ;
   - k: is selected from 0, 1, and 2;
   - h: is selected from 0, 1, and 2;
   - h': is selected from 0, 1, and 2;
   - g: is selected from 0, 1, 2, 3, 4, 5, 6, 7, 8, 9, and 10;
   - g': is selected from 0, 1, 2, 3, 4, 5, 6, 7, 8, 9, and 10;
   - s: is selected from 0, 1, 2, 3, 4, 5, 6, 7, 8, 9, and 10;
   - s': is selected from 0, 1, 2, 3, 4, 5, 6, 7, 8, 9, and 10;
   - s": is selected from 0, 1, 2, 3, 4, 5, 6, 7, 8, 9, and 10;
   - t: is selected from 0, 1, 2, 3, 4, 5, 6, 7, 8, 9, and 10;
   - t': is selected from 0, 1, 2, 3, 4, 5, 6, 7, 8, 9, and 10;
   - x: is selected from 0, 1, 2, 3, 4, 5, and 6;
   - x': is selected from 0, 1, 2, 3, 4, 5, and 6;
   - Cₕ: is a metal bonding unit having a formula selected from the group: 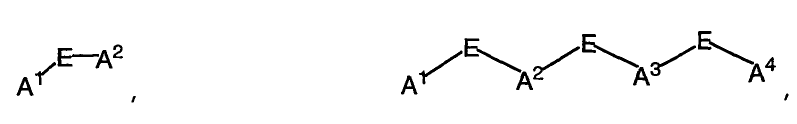 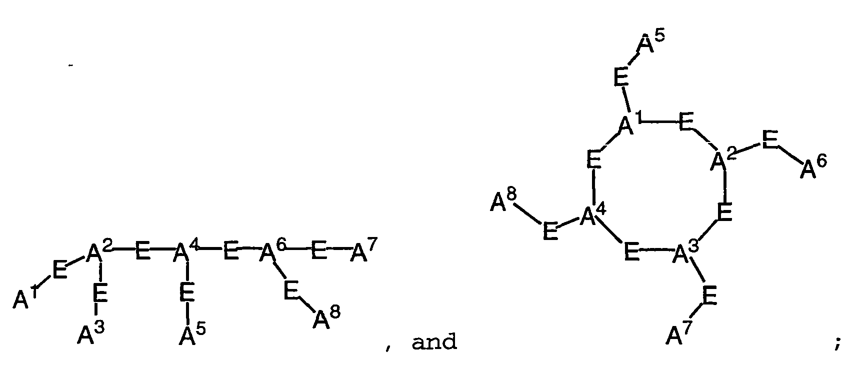
   - A¹, A², A³, A⁴, A⁵, A⁶, A⁷, and A⁸: are independently selected at each occurrence from the group: NR¹³, NR¹³R¹⁴, S, SH, S(Pg), O, OH, PR¹³, PR¹³R¹⁴, P(O)R¹⁵R¹⁶, and a bond to Lₙ;
   - E: is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
   - R¹³ and R¹⁴: are each independently selected from the group: a bond to Lₙ, hydrogen, C₁-C₁₀ alkyl substituted with 0-3 R¹⁷, aryl substituted with 0-3 R¹⁷, C₁₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
   alternatively, R¹³ and R¹⁴ combine to form =C(R²⁰)(R²¹);
   - R¹⁵ and R¹⁶: are each independently selected from the group: a bond to Lₙ, -OH, C₁-C₁₀ alkyl substituted with 0-3 R¹⁷, C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
   - R¹⁷: is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)2, -NHC(=S)NHR¹⁸, =NOR¹⁸, NO₂, -C(=O)NHOR¹⁸, -C (=O)NHNR¹⁸R^{18a}, -OCH₂CO₂H, 2-(1-morpholino)ethoxy, C₁-C₅ alkyl, C₂-C₄ alkenyl, C₃-C₆ cycloalkyl, C₃-C₆ cycloalkylmethyl, C₂-C₆ alkoxyalkyl, aryl substituted with 0-2 R¹⁸, and a 5-10 membered heterocyclic ring system containing 1-4 heteroatoms independently selected from N, S, and O;
   - R¹⁸, R^{18a}, and R¹⁹: are independently selected at each occurrence from the group: a bond to Lₙ, H, C₁-C₆ alkyl, phenyl, benzyl, C₁-C₆ alkoxy, halide, nitro, cyano, and trifluoromethyl;
   - Pg: is a thiol protecting group;
   - R²⁰ and R²¹: are independently selected from the group: H, C₁-C₁₀ alkyl, -CN, -CO₂R²⁵, -C(=O)R²⁵, -C(=O)N(R²⁵)₂, C₂-C₁₀ 1-alkene substituted with 0-3 R²³, C₂-C₁₀ 1-alkyne substituted with 0-3 R²³, aryl substituted with 0-3 R²³, unsaturated 5-10 membered heterocyclic ring system containing 1-4 heteroatoms independently selected from N, S, and 0 and substituted with 0-3 R²³, and unsaturated C₃-₁₀ carbocycle substituted with 0-3 R²³;
   alternatively, R²⁰ and R²¹, taken together with the divalent carbon radical to which they are attached form: 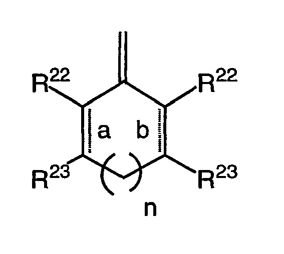
   - R²² and R²³: are independently selected from the group: H, R²⁴, C₁-C₁₀ alkyl substituted with 0-3 R²⁴, C₂-C₁₀ alkenyl substituted with 0-3 R²⁴, C₂-C₁₀ alkynyl substituted with 0-3 R²⁴, aryl substituted with 0-3 R²⁴, a 5-10 membered heterocyclic ring system containing 1-4 heteroatoms independently selected from N, S, and 0 and substituted with 0-3 R²⁴, and C₃-₁₀ carbocycle substituted with 0-3 R²⁴;
   alternatively, R²², R²³ taken together form a fused aromatic or a 5-10 membered heterocyclic ring system containing 1-4 heteroatoms independently selected from N, S, and O;
   - a and b: indicate the positions of optional double bonds and n is 0 or 1;
   - R²⁴: is independently selected at each occurrence from the group; =O, F, Cl, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)C)R^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, and 2-(1-morpholino)ethoxy; and,
   - R²⁵, R^{25a}, and R²⁶: are each independently selected at each occurrence from the group: hydrogen and C₁-C₆ alkyl; or a pharmaceutically acceptable salt thereof.
[40] In another embodiment, the present invention provides a use according to Embodiment [39], wherein: Q is a compound of Formula (IV): 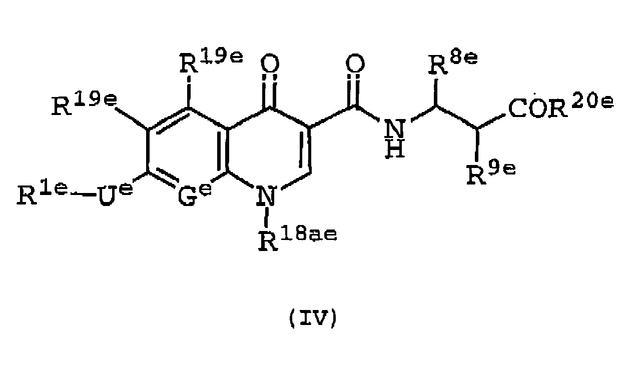 including stereoisomeric forms thereof, or mixtures of stereoisomeric forms thereof, or pharmaceutically acceptable salt or prodrug forms thereof wherein:
   - R^{1e}: is selected from: 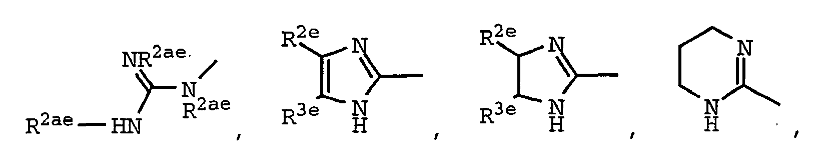 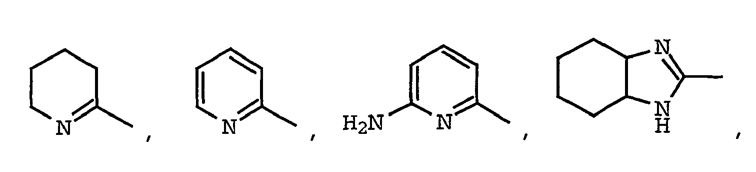 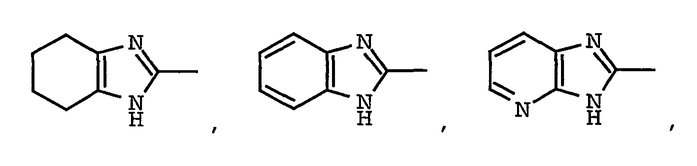 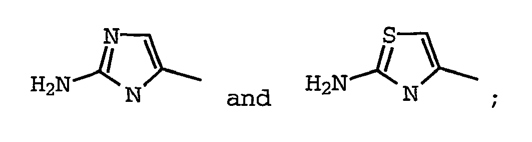
   - R^{2e} and R^{3e}: are independently selected from: . H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl (C₁-C₄ alkyl), aryl (C₁-C₆ alkyl) (C₁-C₆ alkyl)carbonyl, (C₁-C₆ alkoxy)carbonyl, arylcarbonyl, and aryl substituted with 0-4 R^{7e},
   alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and N02;
   - R^{2ae}: is selected from:
   H, C₁-C₁₀ alkyl, C₂-C₆ alkenyl, C₃-C₁₁ cycloalkyl, C₃-C₇ cycloalkyl (C₁-C₄ alkyl), aryl, aryl (C₁-C₄ alkyl)-, (C₂-C₇ alkyl)carbonyl, arylcarbonyl,
   (C₂-C₁₀ alkoxy)carbonyl, C₃-C₇ cycloalkoxycarbonyl, C₇-C₁₁ bicycloalkoxycarbonyl, aryloxycarbonyl, aryl (C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
   - R^{7e}: is selected from:
   H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
   - U^{e}: is selected from:
   -(CH₂)ₙ^{e}-, -(CH₂)ₙ^{e}O(CH₂)ₘ^{e}-, -NH(CH₂)n^{e}-, -N(R^{10e})C(=O)-, -NHC(=O)(CH₂)ₙ^{e}-, and-C(=O)N(R^{10e})-;
   - G^{e}: is N or CR^{19e};
   - R^{8e}: is selected from:
   H, CO₂R^{18be}, C(=O)R^{18be}, CONR^{17e}R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl, C₃-C₁₀ cycloalkyl(C₁-C₄ alkyl)-, phenyl substituted with 0-3 R^{6e}, naphthyl substituted with 0-3 R^{6e},
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   - R^{9e}: is selected from:
   C₁-C₁₀ alkyl substituted with 0-1 R^{6e},
   C₁-C₁₀ alkoxy substituted with 0-2 R^{7e},
   H, nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, hydroxy, OR^{22e}, -N(R^{10e})R^{11e}, -N(R^{16e})R^{17e}, aryl(C₀-C₆ alkyl)carbonyl, aryl(C₁-C₆ alkyl), heteroaryl(C₁-C₆ alkyl), CONR^{18ae}R^{20e}, SO₂R^{18ae}, and SO₂NR^{18ae}R^{20e},
   providing that any of the above alkyl, cycloalkyl, aryl or heteroaryl groups may be unsubstituted or substituted independently with 1-2 R^{7e};
   - R^{6e}: is selected from:
   H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚ^{e}R^{11e}, SO₂NR^{10e}R^{11e},
   aryl substituted with 0-3 groups selected from halogen, C₁-C₆ alkoxy, C₁-C₆ alkyl, CF₃, S(O)ₘ^{e}Me, and -NMe₂,
   aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
   a 5-10 membered heterocyclic ring containing 1-3 N, 0, or S heteroatoms, wherein said heterocyclic ring may be saturated, partially saturated, or fully unsaturated, said heterocyclic ring being substituted with 0-2 R^{7e};
   - R^{10e}: is selected from:
   H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
   - R^{11e}: is selected from:
   H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
   - R^{4e}: is selected from:
   H, C₁-C₆ alkyl, C₃-C₇ cycloalkyl, C₃-C₇ cycloalkyl(C₁-C₄ alkyl)-, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
   - R^{12e}: is selected from:
   H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy)carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl (C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
   - R^{16e}: is selected from:
   -C(=O)OR^{18ae}, -C(=O)R^{18be}, -C(=O)N(R^{18be})₂,-SO₂R^{18ae}, and -SO₂N(R^{18be})₂;
   - R^{17e}: is selected from:
   H, C₁-C₆ alkyl, C₃-C₇ cycloalkyl, C₃-C₇ cycloalkyl(C₁-C₄ alkyl)-, aryl, aryl(C₁-C₆ alkyl)-, and heteroaryl(C₁-C₆ alkyl);
   - R^{18ae}: is selected from:
   C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
   - R^{18be}: is H or R^{18ae},
   - R^{19e}: is selected from:
   H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
   - R^{20e}: is selected from:
   hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
   (5-(C₁-C₅ alkyl)-1,3-dioxa-cyclopenten-2-one-yl)methyloxy,
   (5-aryl-1,3-dioxa-cyclopenten-2-one-yl)methyloxy, and
   (R^{10e}) (R^{11e})N-(C₁-C₁₀ alkoxy)-;
   - R^{21e}: is selected from:
   C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and
   C₁-C₁₀ alkyl substituted with 0-2 R^{7e};
   - R^{22e}: is selected from:
   -C(=O)-R^{18be}, C(=O)N(R^{18be})₂, -C(=O)NHSO₂R^{18ae}, -C(=O)NHC(=O)R^{18be}, -C(=O)NHC(=O)OR^{18ae}, and -C(=O)NHSO₂NHR^{18be};
   - m^{e}: is 0-2;
   - n^{e}: is 0-4; and
   - p^{e}: is 0-2;
   with the following proviso: n^{e} and m^{e} are chosen such that the number of atoms connecting R¹ and -COR^{20e} in Formula (IV) is in the range of 8-14;
   - d: is selected from 1, 2, 3, 4, and 5;
   - d': is 1-50;
   - W: is independently selected at each occurrence from the group : O, NH, NHC(=O), C(=O)NH, NR⁸C(=O), C(=O)N R⁸, C(=O), C(=O)O, OC(=O), NHC(=S)NH, NHC(=O)NH, SO₂, (OCH₂CH₂)ₛ, (CH₂CH₂O)_{s'}, (OCH₂CH₂CH2)_{s"}, (CH₂CH₂CH₂O)ₜ, and (aa)_{t'};
   - aa: is independently at each occurrence an amino acid;
   - Z: is selected from the group: aryl substituted with 0-1 R¹⁰, C₃₋₁₀ cycloalkyl substituted with 0-1 R¹⁰, and a 5-10 membered heterocyclic ring system containing 1-4 heteroatoms independently selected from N, S, and O and substituted with 0-1 R¹⁰;
   - R⁶, R^{6a}, R⁷, R^{7a}, and R⁸: are independently selected at each occurrence from the group: H, =O, COOH, SO₃H, C₁-C₅ alkyl substituted with 0-1 R¹⁰, aryl substituted with 0-1 R¹⁰, benzyl substituted with 0-1 R¹⁰, and C₁-C₅ alkoxy substituted with 0-1 R¹⁰, NHC(=O)R¹¹, C(=O)NHR¹¹, NHC(=O)NHR¹¹, NHR¹¹, R¹¹, and a bond to Cₕ;
   - k: is 0 or 1;
   - s: is selected from 0, 1, 2, 3, 4, and 5;
   - s': is selected from 0, 1, 2, 3, 4, and 5;
   - s": is selected from 0, 1, 2, 3, 4, and 5;
   - t: is selected from 0, 1, 2, 3, 4, and 5;
   - A¹, A², A³, A⁴, A⁵, A⁶, A⁷, and A⁸: are independently selected at each occurrence from the group: NR¹³, NR¹³R¹⁴, S, SH, S(Pg), OH, and a bond to Lₙ;
   - E: is a bond, CH, or a spacer group independently selected at each occurrence from the group: C₁-C₁₀ alkyl substituted with 0-3 R¹⁷, aryl substituted with 0-3 R¹⁷, C₃-₁₀ cycloalkyl substituted with 0-3 R¹⁷, and a 5-10 membered heterocyclic ring system containing 1-4 heteroatoms independently selected from N, S, and 0 and substituted with 0-3 R¹⁷;
   - R¹³, and R¹⁴: are each independently selected from the group: a bond to Lₙ, hydrogen, C₁-C₁₀ alkyl substituted with 0-3 R¹⁷, aryl substituted with 0-3 R¹⁷, a 5-10 membered heterocyclic ring system containing 1-4 heteroatoms independently selected from N, S, and 0 and substituted with 0-3 R¹⁷, and an electron, provided that when one of R¹³ or R¹⁴ is an electron, then the other is also an electron;
   alternatively, R¹³ and R¹⁴ combine to form =C(R²⁰)(R²¹);
   - R¹⁷: is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CH₂OR¹⁸ -OC(=O)R¹⁸, -OC(=O)OR^{18a}, -OR¹⁸, -OC (=O) N(R¹⁸)₂, -NR¹⁹C(=O)R¹⁸, -NR¹⁹C(=O)OR¹⁸a, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)_{2,} -N(R¹⁸)₂, -NHC(=S)NHR¹⁸, =NOR¹⁸, -C(=O)NHNR¹⁸R^{18a}, -OCH₂CO₂H, and 2-(1-morpholino)ethoxy;
   - R¹⁸, R^{18a}, and R¹⁹: are independently selected at each occurrence from the group: a bond to Lₙ, H, and C₁-C₆ alkyl;
   - R²⁰ and R²¹: are independently selected from the group: H, C₁-C₅ alkyl, -CO₂R²⁵, C₂-C₅ 1-alkene substituted with 0-3 R²³, C₂-C₅ 1-alkyne substituted with 0-3 R²³, aryl substituted with 0-3 R²³, and unsaturated 5-10 membered heterocyclic ring system containing 1-4 heteroatoms independently selected from N, S, and O and substituted with 0-3 R²³;
   alternatively, R²⁰ and R²¹, taken together with the divalent carbon radical to which they are attached form: 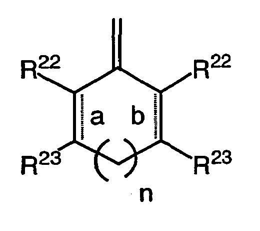
   - R²² and R²³: are independently selected from the group: H, and R²⁴;
   alternatively, R²², R²³ taken together form a fused aromatic or a 5-10 membered heterocyclic ring system containing 1-4 heteroatoms independently selected from N, S, and O;
   - R²⁴: is independently selected at each occurrence from the group: -CO₂R²⁵, -C(O)N(R²⁵)₂, -CH₂OR²⁵, -OC(=O)R²⁵, -OR²⁵ , -SO₃H, -N(R²⁵)₂, and -OCH₂CO₂H; and,
   - R²⁵: is independently selected at each occurrence from the group: H and C₁-C₃ alkyl.
[41] In another embodiment, the present invention provides a use according to Embodiment [39], wherein:
   - R^{1e}: is selected from: 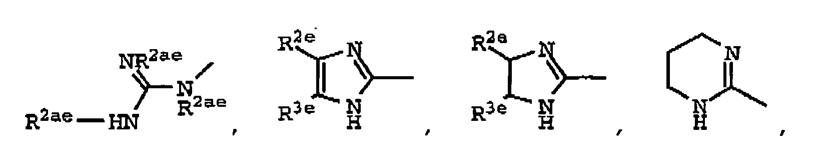 and 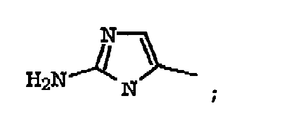
   - R^{2e} and R^{3e}: are independently selected from:
   H, C₁-C₄ alkoxy, NR^{11e}R¹⁸, halogen, NO₂, CN, CF₃, C₁-C₆ alkyl, C₃-C₆ alkenyl, C₃-C₇ cycloalkyl, C₃-C₇ cycloalkyl(C₁₋C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
   alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
   - R^{2ae}: is selected from:
   H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl,
   (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
   - R^{7e}: is selected from:
   H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
   - U^{e}: is selected from:
   -(CH₂₎ₙ^{e}-, -NH(CH₂)ₙ^{e}-, -N(R^{10e})C(=O)-, and -NHC(=O)(CH2)ₙ^{e};
   - G^{e}: is N or CR^{19e};
   - R^{8e}: is H;
   - R^{9e}: is selected from:
   H, nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C=O)NR^{10e}R^{11e}, NR^{10e}SO₂NR^{10e}R^{11e}, NR^{10e}SO₂R^{21e}, hydroxy, OR^{22e}, -N(R^{10e})R^{11e}, -N(R^{16e})R^{17e}, aryl(C₀-C₄ alkyl)carbonyl, aryl(C₁-C₄ alkyl), heteroaryl(C₁-C₄ alkyl), CONR^{18ae}R^{20e}, SO₂R^{18ae}, and SO₂NR^{18ae}R^{20e},
   providing that any of the above alkyl, cycloalkyl, aryl or heteroaryl groups may be unsubstituted or substituted independently with 1-2 R^{7e};
   - R^{10e}: is selected from:
   H, -OH, CF₃, C₃-C₆ alkenyl, C₃-C₆ cycloalkyl, aryl, (C₃-C₆ cycloalkyl)methyl, aryl(C₁-C₄ alkyl), and C₁-C₄ alkyl substituted with 0-2 R^{6e};
   - R^{6e}: is selected from:
   H, C₁-C₄ alkyl; hydroxy, C₁-C₄ alkoxy, nitro, C₁-C₄ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
   aryl substituted with 0-3 groups selected from halogen, C₁-C₄ alkoxy, C₁-C₄ alkyl, CF₃, S(O)ₘ^{e}Me, and -NMe₂,
   aryl(C₁-C₄ alkyl)-, said aryl being substituted with 0-3 groups selected from halogen, C₁-C₄ alkoxy, C₁-C₄ alkyl, CF₃, S(O)ₚ^{e}Me, and -NMe₂, and
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
   - R^{11e}: is selected from:
   H, hydroxy, C₁-C₄ alkyl, C₃-C₆ alkenyl, C₃-C₆ cycloalkyl, (C₃-C₆ cycloalkyl)methyl, C₁-C₄ alkoxy, benzyloxy, aryl, heteroaryl, heteroaryl(C₁-C₄ alkyl)-, aryl(C₁-C₄ alkyl), adamantylmethyl, and
   C₁-C₄ alkyl substituted with 0-2 R^{4e};
   - R^{4e}: is selected from:
   H, C₁-C₄ alkyl, C₃-C₇ cycloalkyl, C₃-C₇ cycloalkyl(C₁-C₄ alkyl)-, aryl, heteroaryl, aryl(C₁-C₄ alkyl)-, and heteroaryl(C₁-C₄ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
   - R^{12e}: is selected from:
   H, C₁-C₄ alkyl, (C₁-C₄ alkyl)carbonyl, (C₁-C₄ alkoxy)carbonyl, phenyl (C₁-C₄ alkyl)-, phenylsulfonyl, phenyloxycarbonyl, and phenyl(C₁-C₄ alkoxy)carbonyl, wherein said phenyl groups are substituted with 0-2 substituents selected from the group consisting of C₁-C₄ alkyl, C₁-C₄ alkoxy, halo, CF₃ and nitro;
   - R^{16e}: is selected from:
   -C(=O)OR^{18ae} -C (=O)R^{18be}, -C(=O)N(R^{18be})₂,-SO₂R^{18ae}, and -SO₂N(R^{18be})2;
   - R^{17e}: is selected from:
   H, C₁-C₄ alkyl, C₃-C₆ cycloalkyl, C₃-C₆ cycloalkyl(C₁-C₄ alkyl)-, aryl, aryl(C₁-C₆ alkyl)-, and heteroaryl(C₁-C₆ alkyl);
   - R^{18ae}: is selected from:
   C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
   - R^{18be}: is H or R^{18ae};
   - R^{19e}: is selected from:
   H, halogen, CF₃, CO₂H, CN, NO₂, -NR^{11e}R^{12e}, OCF₃, C₁-C₆ alkyl, C₂-C₆ alkenyl, C₂-C₆ alkynyl, C₃-C₆ cycloalkyl, C₃-C₆ cycloalkyl(C₁-C₄ alkyl)-, aryl(C₁-C₄ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
   - R^{20e}: is selected from:
   hydroxy, C₁-C₆ alkyloxy, C₃-C₆ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy (C₁-C₅ alkyl) carbonyloxy (C₁-C₂ alkyl)ox y,
   (5-(C₁-C₅ alkyl)-1,3-dioxa-cyclopenten-2-one-yl)methyloxy,
   (5-aryl-1,3-dioxa-cyclopenten-2-one-yl)methyloxy, and
   (R^{10e}) (R^{11e})N-(C₁-C₁₀ alkoxy)-;
   - R^{21e}: is selected from:
   C₁-C₄ alkyl, C₂-C₆ alkenyl, C₃-C₆ cycloalkyl, (C₃-C₆ cycloalkyl)methyl, aryl, aryl(C₁-C₄ alkyl)-, and C₁-C₁₀ alkyl substituted with 0-2 R^{7e};
   - R^{22e}: is selected from:
   -C(=O)-R^{18be}, -C(=O)_{N}(R^{18be})₂, -C(=O)NHSO₂R^{18ae}, -C(=O)NHC(=O)R^{18be}, -C(=O)NHC (=O)OR^{18ae}, and -C(=O)NHSO₂NHR^{18be};
   - m^{e}: is 0-2;
   - n^{e}: is 0-4;
   - p^{e}: is 0-2;
   - Cₕ: is 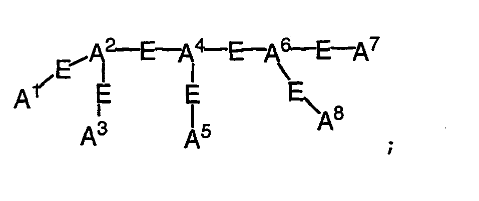
   - A¹: is selected from the group: OH, and a bond to Lₙ;
   - A², A⁴, and A⁶: are each N;
   - A³, A⁵, and A⁸: are each OH;
   - A⁷: is a bond to Lₙ or NH-bond to Lₙ;
   - E: is a C₂ alkyl substituted with 0-1 R¹⁷;
   - R¹⁷: is =O;
   alternatively, Cₕ is 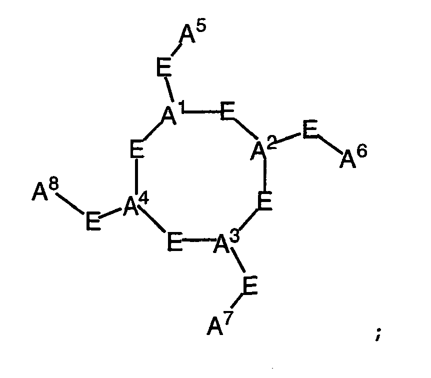
   - A¹: is selected from the group: OH, and a bond to Lₙ;
   - A², A³ and A⁴: are each N;
   - A⁵, A⁶ and A⁸: are each OH;
   - A⁷: is a bond to Lₙ;
   - E: is a C₂ alkyl substituted with 0-1 R¹⁷;
   - R¹⁷: is =0;
   alternatively, Cₕ is 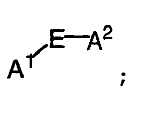
   - A¹: is NH₂ or N=C(R²⁰) (R²¹);
   - E: is a bond;
   - A²: is NHR¹³;
   - R¹³: is a heterocycle substituted with R¹⁷, the heterocycle being selected from pyridine and pyrimidine;
   - R¹⁷: is selected from a bond to Lₙ, C(=O)NHR¹⁸ and C(=O)R¹⁸;
   - R¹⁸: is a bond to Ln;
   - R²⁴: is selected from the group: -CO₂R²⁵, -OR²⁵, -SO₃H, and -N(R²⁵)₂; and,
   - R²⁵: is independently selected at each occurrence from the group: hydrogen and methyl.
[42] In another embodiment, the present invention provides a use according to Embodiment [39], wherein:
   Q is selected from the group:
   3-[7-[(imidazolin-2-ylamino)methyl]-1-methyl-6,8-difluoroquinoline-4-one-3-ylcarbonylamino]-2-(3,5-dimethylisoxazol-4-ylsulfonylamino)propionic acid,
   3-[7-[(imidazolin-2-ylamino)methyl]-1-methyl-6,8-difluoroquinoline-4-one-3-ylcarbonylamino]-2-(benzyloxycarbonylamino)propionic acid,
   3-[7-[(imidazolin-2-ylamino)methyl]-1-methyl-6,8-difluoxoquinoline-4-one-3-ylcarbonylamino]-2-(n-butyloxycarbonylamino)propionic acid,
   3-[7-[(imidazolin-2-ylamino)methyl]-1-methyl-6,8-difluoxoquinoline-4-one-3-ylcaxbonylamino]-2-(n-butylsulfonylamino)propionic acid,
   3-[7-[(tetrahydropyrimid-2-ylamino)methyl]-1-methyl-6,8-difluoroquinoline-4-one-3-ylcarbonylamino]-2-(benzyloxycarbonylamino)propionic acid,
   3-[7-[(tetrahydropyrimid-2-ylamino)methyl]-1-methyl-6,8-difluoroquinoline-4-one-3-ylcarbonylamino]-2-(n-butyloxycarbonylamino)propionic acid,
   3-[7-[(tetrahydropyrimid-2-ylamino)methyl]-1-methyl-6,8-difluoroquinoline-4-one-3-ylcarbonylaminol-2-(phenylsulfonylamino)propionic acid,
   3-[7-[(tetrahydropyrimid-2-ylamino)methyl]-1-methyl-6,8-difluoroquinoline-4-one-3-ylcarbonylamino]-2-(n-butylsulfonyl)aminopropionic acid,
   3-[7-[(2-aminothiazol-4-yl)methyl]-1-methyl-6,8-difluorocruinoline-4-one-3-ylcarbonylamino]-2-(benzyloxycarbonylamino)propionic acid,
   3-[7-[(imidazolin-2-ylamino)methyl]-1-methyl-6,8-difluoroquinoline-4-one-3-ylcarbonylamino]-2-((2, 4, 6-trimethylphenyl)sulfonylamino)propionic acid,
   3-[7-[(tetrahydropyrimid-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(imidazol-2-ylamino)methyl]-1-methyl-6,8-difluoroquinoline-4-one-3-ylcarbonylamino]-2-(3,5-dimethylisoxazol-4-ylsulfonylamino)propionic acid,
   3-[7-[(imidazol-2-ylamino)methyl]-1-methyl-6,8-difluoroquinoline-4-one-3-ylcarbonylamino]-2-(benzyloxycarbonylamino)propionic acid,
   3-[7-[(imidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(imidazol-2-ylamino)methyl]-1-methyl-6,8-difluoroquinoline-4-one-3-ylcarbonylamino]-2-((4-biphenyl)sulfonylamino)propionic acid,
   3-[7-[(imidazol-2-ylamino)methyl]-1-methyl-6,8-difluoroquinoline-4-one-3-ylcarbonylamino]-2-(1-naphthylsulfonylamino)propionic acid,
   3-[7-[(benzimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(4-methylimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(4,5-dimethylimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(4,5,6,7-tetrahydrobenzimidazol-2-ylamino)methyl]-1-methyl-6,8-difluoroquinoline-4-one-3-ylcarbonylamino]-2-((2,4,6-trimethylphenyl)sulfonylamino)propionic acid,
   3-[7-[(pyridin-2-ylamino)methyl]-1-methyl-6,8-difluoroquinoline-4-one-3-ylcarbonylamino]-2-((2,4,6-trimethylphenyl)sulfonylamino)propionic acid,
   3-[7-(2-aminopyridin-6-yl)-1-methyl-6,8-difluoroquinoline-4-one-3-ylcarbonylamino]-2-((2,4,6-trimethylphenyl)sulfonylamino)propionic acid,
   3-[7-[(7-azabenzimidazol-2-yl)methyl]-1-methyl-6,8-difluoroquinoline-4-one-3-ylcarbonylamino]-2-((2,4,6-trimethylphenyl)sulfonylamino)propionic acid,
   3-[7-[(benzimidazol-2-ylamino)methyl]-1-(2-phenylethyl)-6,8-difluoroquinoline-4-one-3-ylcarbonylamino]pro-pionic acid,
   3-[7-[(pyridin-2-ylamino)methyl]-1-(2-phenylethyl)-6,8-difluoroquinoline-4-one-3-ylcarbonylamino]propionic acid,
   3-[7-[(imidazolin-2-ylamino)methyl]-1-(2-phenylethyl)-6,8-difluoroquinoline-4-one-3-ylcarbonylamino]propionic acid,
   3-[7-[(imidazol-2-ylamino)methyl]-1-(2-phenylethyl)-6,8-difluoroquinoline-4-one-3-ylcarbonylamino]propionic acid,
   3-[7-[(imidazolin-2-ylamino)methyl]-1-(2-phenylethyl)-6,8-difluoroquinoline-4-one-3-ylcarbonylamino]-2-(benzyloxycarbonylamino)propionic acid,
   3-[7-[(imidazolin-2-ylamino)methyl]-1-(2-phenylethyl)-6,8-difluoroquinoline-4-one-3-ylcarbonylamino]-2-(n-butyloxycarbonylamino)propionic acid,
   3-[7-[(imidazolin-2-ylamino)methyl]-1-(2-phenylethyl)-6,8-difluoroquinoline-4-one-3-ylcarbonylamino]-2-(phenylsulfonylamino)propionic acid,
   3-[7-[(imidazolin-2-ylamino)methyl]-1-(2-phenylethyl)-6,8-difluoroquinoline-4-one-3-ylcarbonylamino]-2-(n-butylsulfonylamino)propionic acid,
   3-[7-[(tetrahydropyrimid-2-ylamino)methyl]-1-(2-phenylethyl)-6,8-difluoroquinoline-4-one-3-ylcarbonylamino]-2-(benzyloxycarbonylamino)propionic acid,
   3-[7-[(tetrahydropyrimid-2-ylamino)methyl]-1-(2-phenylethyl)-6,8-difluoroquinoline-4-one-3-ylcarbonylamino]-2-(n-butyloxycarbonylamino)propionic acid,
   3-[7-[(tetrahydropyrimid-2-ylamino)methyl]-1-(2-phenylethyl)-6,8-difluoroquinoline-4-one-3-ylcarbonylamino]-2-(phenylsulfonylamino)propionic acid,
   3-[7-[(tetrahydropyrimid-2-ylamino)methyl]-1-(2-phenylethyl)-6,8-difluoroquinoline-4-one-3-ylcarbonylamino]-2-(n-butylsulfonyl)aminopropionic acid,
   3-[7-[(2-aminothiazol-4-yl)methyl]-1-(2-phenylethyl)-6,8-difluoroquinoline-4-one-3-ylcarbonylamino]-2-(phenylsulfonylamino)propionic acid,
   3-[7-[(2-aminothiazol-4-yl)methyl]-1-(2-phenylethyl)-6,8-difluoroquinoline-4-one-3-ylcarbonylamino]-2-(benzyloxycarbonylamino)propionic acid,
   3-[7-[(imidazolin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(tetrahydropyrimid-2-ylamino)methyl]-1-(2-phenylethyl)-6,8-difluoroquinoline-4-one-3-ylcarbonylamino]-2-((2,4,6-trimethylphenyl)sulfon-ylamino)propionic acid,
   3-[7-[(imidazol-2-ylamino)methyl]-1-(2-phenylethyl)-6,8-difluoroquinoline-4-one-3-ylcarbonylamino]-2-(benzyloxycarbonylamino)propionic acid,
   3-[7-[(imidazol-2-ylamino)methyl]-1-(2-phenylethyl)-6,8-difluoroquinoline-4-one-3-ylcarbonylamino]-2-(phenylsulfonylamino)propionic acid,
   3-[7-[(imidazol-2-ylamino)methyl]-1-(2-phenylethyl)-6,8-difluoroquinoline-4-one-3-ylcarbonylamino]-2-((2,6,dichlorophenyl)sulfonylamino)propionic acid,
   3-[7-[(imidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(imidazol-2-ylamino)methyl]-1-(2-phenylethyl)-6,8-difluoroquinoline-4-one-3-ylcarbonylamino]-2-((4-biphenyl)sulfonylamino)propionic acid,
   3-[7-[(benzimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(4-methylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(4,5-dimethylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(4,5,6,7-tetrahydrobenzimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-[(pyridin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
   3-[7-(2-aminopyridin-6-yl)-1-(2-phenylethyl)-6,8-difluoroquinoline-4-one-3-ylcarbonylamino]-2-((2,4,6-trimethylphenyl)sulfonylamino)propionic acid, and
   3-[7-[(7-azabenzimidazol-2-yl)methyl]-1-(2-phenylethyl)-6,8-difluoroquinoline-4-one-3-ylcarbonylamino]-2-((2,4,6-trimethylphenyl)sulfonylamino)propionic acid.
[43] In another embodiment, the present invention provides a use according to Embodiment [37], wherein the radioisotope is ¹⁵³Sm.
[44] In another embodiment, the present invention provides a use according to Embodiment [37], wherein the radioisotope is ¹⁷⁷Lu.
[45] In another embodiment, the present invention provides a use according to Embodiment [44], wherein the radiopharmaceutical is selected from the group: 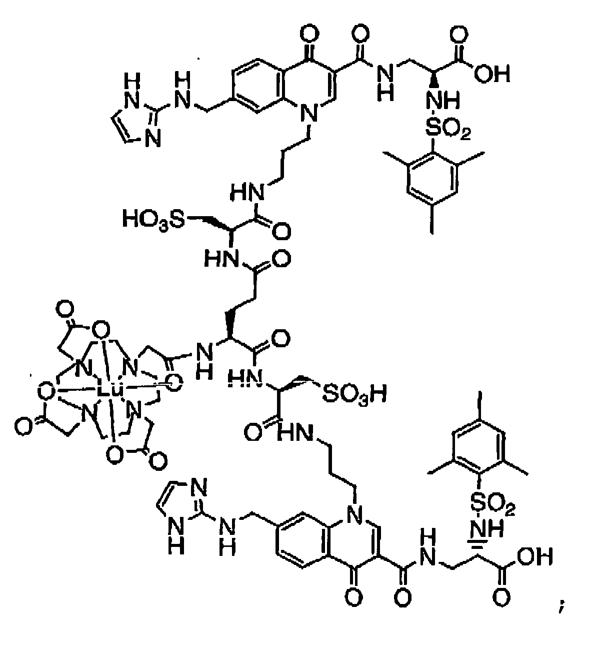 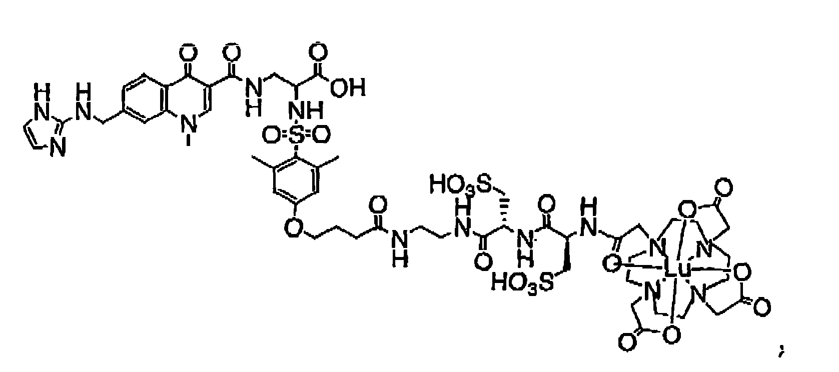 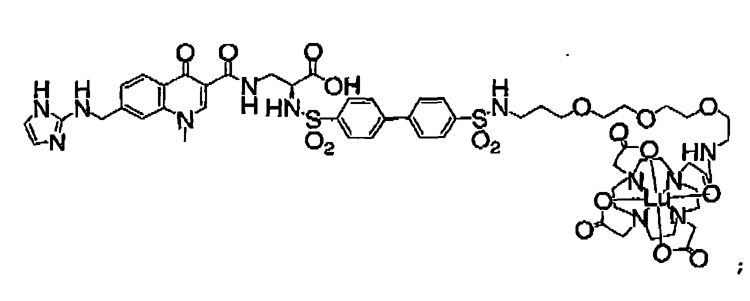 and 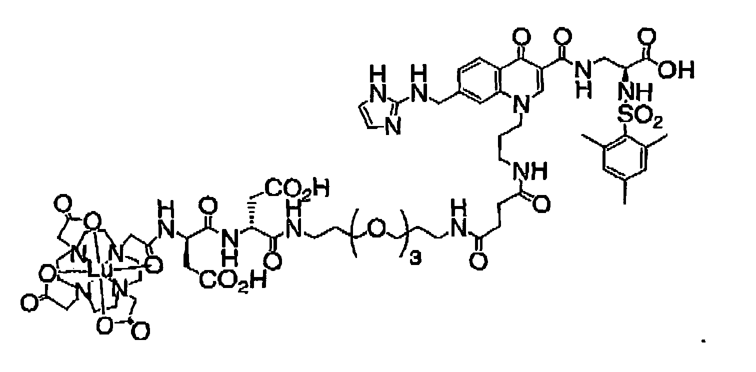
[46] In another embodiment, the present invention provides a use according to Embodiment [37], wherein the radioisotope is ⁹⁰Y.
[47] In another embodiment, the present invention provides a use according to Embodiment [46], wherein the radiopharmaceutical is selected from the group; 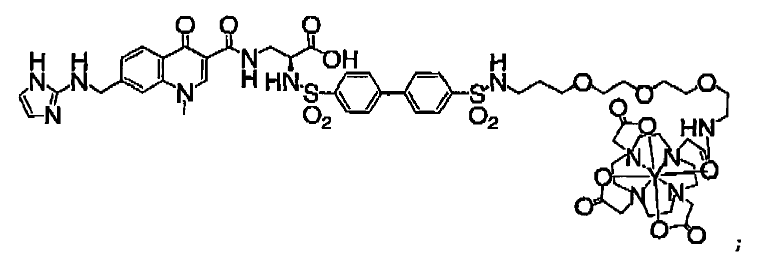 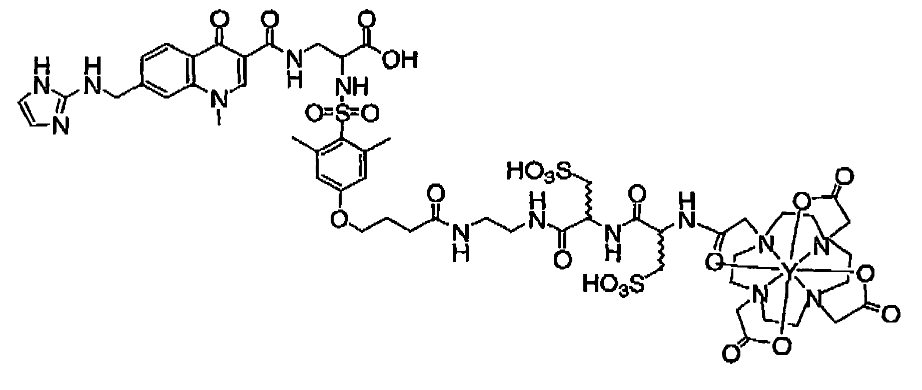 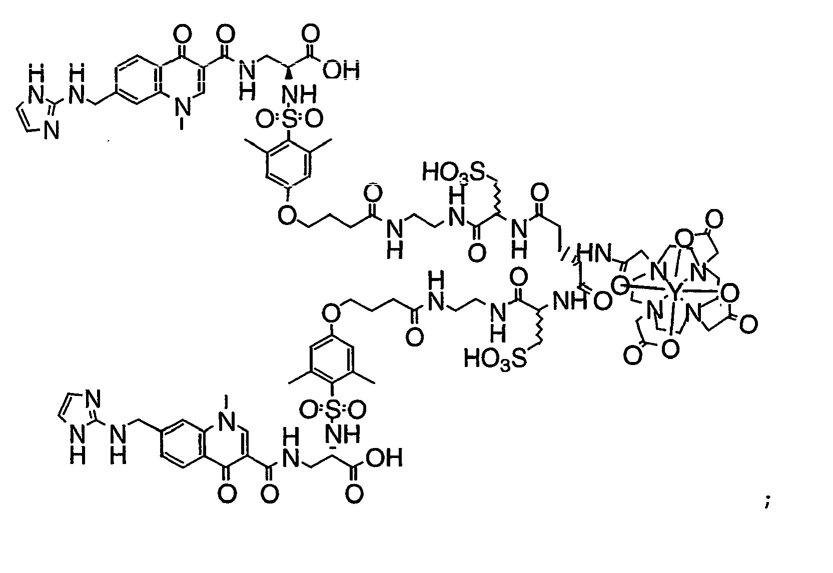 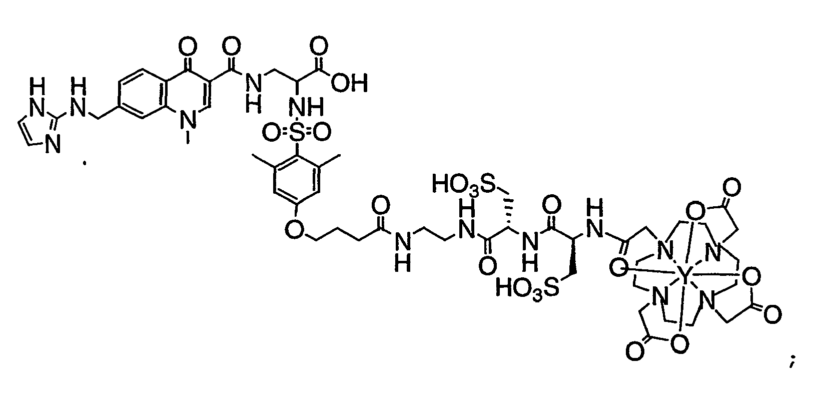 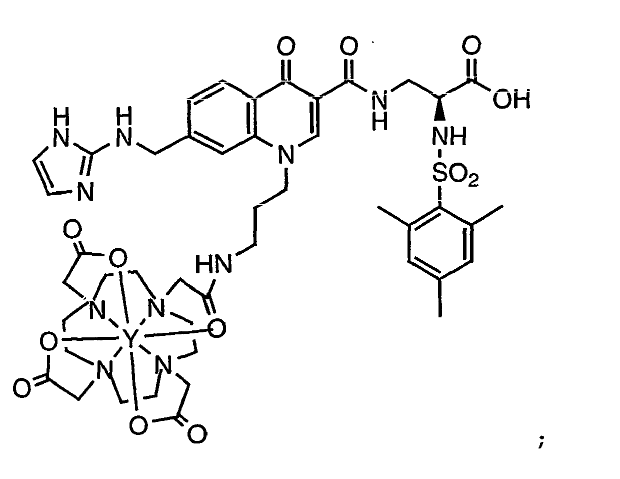 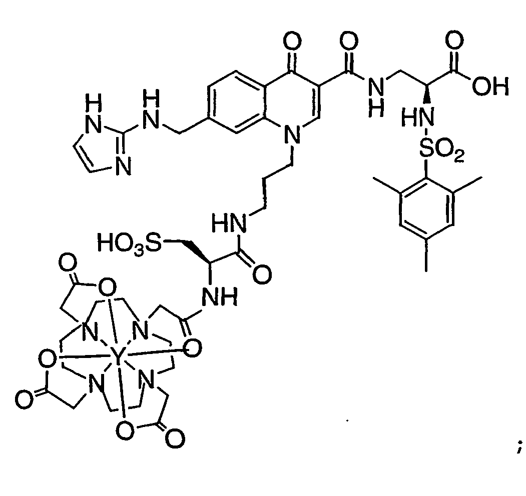 and 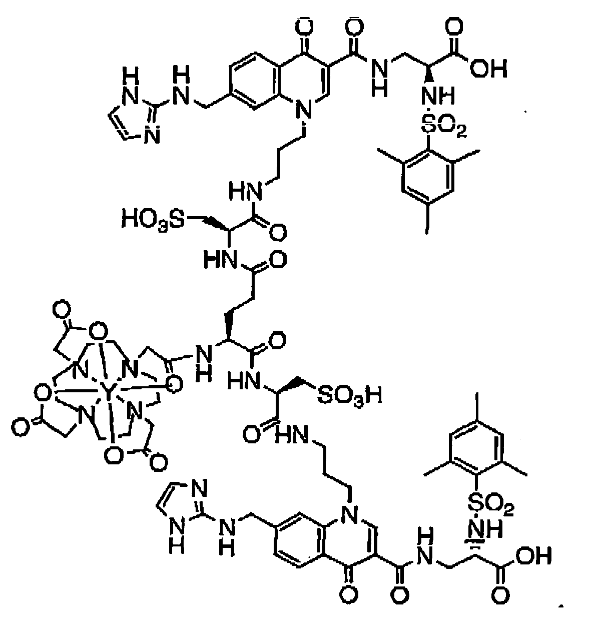
[48] In another embodiment, the present invention provides a use of Embodiment [29], further comprising treating the cancer by brachytherapy, external beam radiation, laser therapy or surgical removal.
[49] In another embodiment, the present invention provides a kit comprising packaging material, and a therapeutic radiopharmaceutical composition of Embodiment [15], contained within said packaging material, wherein the packaging material comprises a label or package insert which indicates that said therapeutic radiopharmaceutical composition can be used for treating cancer.
[50] In another embodiment, the present invention provides a therapeutic radiopharmaceutical composition of Embodiment [15], further comprising a photosensitizing agent.
[51] In another embodiment, the present invention provides a therapeutic radiopharmaceutical composition according to Embodiment [50], wherein the photosensitizing agent is selected from the group consisting of photofrin; naphthalocyanine photosensitizing agents; tetrapyrrole-based photosensitizers; porphyins; chlorins;, phthalocyanines; napthalocyanines; coumarins, psoralens, 1,3,4,6-tetramethoxyhelianthrone; 10,13-dimethyl-1,3,4,6-tetrahydroxyhelianthrone; 10,13-di(methoxycarbonyl)-1,3,4,6-tetramethoxyhelianthrone; 1,6-di-N-butylamino-3,4-dimethoxy-helianthrone; 1,6-di-N-butylamino-3,4-dimethoxy-10,13-dimethyl-helianthrone; 1,6-di-(N-hydroxyethylamino)-3,4-dimethoxy-helianthrone; 2,5-dibromo-1,3,4,6-tetrahydroxyhelianthrone; and 2,5-dibromo-10,13-dimethyl-1,3,4,6-tetrahydroxyhelianthrone.
[52] In another embodiment, the present invention provides a kit according to Embodiment [49], further comprising a photosensitizing agent.
[53] In another embodiment, the present invention provides a kit according to Embodiment [52], wherein the photosensitizing agent is selected from the group consisting of photofrin; naphthalocyanine photosensitizing agents; tetrapyrrole-based photosensitizers; porphyins; chlorins;, phthalocyanines; napthalocyanines; coumarins, psoralens, 1,3,4,6-tetramethoxyhelianthrone; 10,13-dimethyl-1,3,4,6-tetrahydroxyhelianthrone; 10,13-di(methoxycarbonyl)-1,3,4,6-tetramethoxyhelianthrone; 1,6-di-N-butylamino-3,4-dimethoxy-helianthrone; 1,6-di-N-butylamino-3,4-dimethoxy-10,13-dimethyl-helianthrone; 1,6-di-(N-hydroxyethylamino)-3,4-dimethoxy-helianthrone; 2,5-dibromo-1,3,4,6-tetrahydroxyhelianthrone; and 2,5-dibromo-10,13-dimethyl-1,3,4,6-tetrahydroxyhelianthrone.
[54] In another embodiment, the present invention provides a use according to Embodiment [29], further comprising treating the patient with photodynamic therapy.
[55] In another embodiment, the present invention provides a use according to Embodiment [54], wherein the Photodynamic therapy comprises:
   a) administering a therapeutic radiopharmaceutical of the present invention and a photosensitive agent (photoreactive agent) to a patient, said photosensitive agent having a characteristic light absorption waveband and being preferentially absorbed by abnormal tissue;
   b) providing an imaging device that is integral with a plurality of light sources and produces a signal used for imaging abnormal tissue at the internal treatment site, said light sources emitting light in a waveband corresponding to the characteristic light absorption waveband of the photosensitive agent, said waveband including wavelengths sufficiently long to penetrate through a dermal layer of the patient to the internal treatment site;
   (c) determining a location of the abnormal tissue at the internal targeted site within the body of the patient with the imaging device, by viewing an image of the abnormal tissue at the targeted site developed in response to the signal produced by the imaging device; and
   (d) energizing the light sources to administer light therapy to the internal targeted site at the location determined with the imaging device.
[56] In another embodiment, the present invention provides a use according to Embodiment [55], wherein the photosensitive agent (photoreactive agent) is specifically targeted at the targeted tissue by including a binding agent that selectively links the photosensitive agent to the targeted tissue.
[57] In another embodiment, the present invention provides a use according to Embodiment [55], wherein the photosensitizing agent is selected from the group consisting of photofrin; naphthalocyanine photosensitizing agents; tetrapyrrole-based photosensitizers; porphyins; chlorins;, phthalocyanines; napthalocyanines; coumarins, psoralens, 1,3,4,6-tetramethoxyhelianthrone; 10,13-dimethyl-1,3,4,6-tetrahydroxyhelianthrone; 10,13-di(methoxycarbonyl)-1,3,4,6-tetramethoxyhelianthrone; 1,6-di-N-butylamino-3,4-dimethoxy-helianthrone; 1,6-diN-butylamino-3,4-dimethoxy-10,13-dimethyl-helianthrone; 1,6-di-(N-hydroxyethylamino)-3,4-dimethoxy-helianthrone; 2,5-dibromo-1,3,4,6-tetrahydroxyhelianthrone; and 2,5-dibromo-10,13-dimethyl-1,3,4,6-tetrahydroxyhelianthrone.

Another aspect of the present invention are diagnostic kits for the preparation of radiopharmaceuticals useful as imaging agents for cancer. Diagnostic kits of the present invention comprise one or more vials containing the sterile, non-pyrogenic, formulation comprised of a predetermined amount of a reagent of the present invention, and optionally other components such as one or two ancillary ligands, reducing agents, transfer ligands, buffers, lyophilization aids, stabilization aids, solubilization aids and bacteriostats. The inclusion of one or more optional components in the formulation will frequently improve the ease of synthesis of the radiopharmaceutical by the practicing end user, the ease of manufacturing the kit, the shelf-life of the kit, or the stability and shelf-life of the radiopharmaceutical. The inclusion of one or two ancillary ligands is required for diagnostic kits comprising reagent comprising a hydrazine or hydrazone bonding moiety. The one or more vials that contain all or part of the formulation can independently be in the form of a sterile solution or a lyophilized solid.

Another aspect of the present invention contemplates a method of imaging cancer in a patient involving: (1) synthesizing a diagnostic radiopharmaceutical of the present invention, using a reagent of the present invention, capable of localizing in tumors; (2) administering said radiopharmaceutical to a patient by injection or infusion; (3) imaging the patient using planar or SPECT gamma scintigraphy, or positron emission tomography.

Another aspect of the present invention contemplates a method of imaging cancer in a patient involving: (1) administering a paramagnetic metallopharmaceutical of the present invention capable of localizing in tumors to a patient by injection or infusion; and (2) imaging the patient using magnetic resonance imaging.

Another aspect of the present invention contemplates a method of imaging cancer in a patient involving: (1) administering a X-ray contrast agent of the present invention capable of localizing in tumors to a patient by injection or infusion; and (2) imaging the patient using X-ray computed tomography.

Another aspect of the present invention contemplates a method of imaging cancer in a patient involving: (1) administering a ultrasound contrast agent of the present invention capable of localizing in tumors to a patient by injection or infusion; and (2) imaging the patient using sonography.

Another aspect of the present invention contemplates a method of treating cancer in a patient involving: (1) administering a therapeutic radiopharmaceutical of the present invention capable of localizing in tumors to a patient by injection or infusion.

Another aspect of the present invention contemplates the combination of anti-cancer agents and angiogenesis-targeted therapeutic radiopharmaceuticals of the invention, which target the luminal side of the neovasculature of tumors, to provide a surprising, and enhanced degree of tumor suppression relative to each treatment modality alone without significant additive toxicity.

Anti-cancer agents used in this invention also include those described in Swindell, et al. United States Patent No. 6,080,877, June 27, 2000, the contents of which is incorporated herein in its entirety.

Another aspect of the present invention contemplates the compounds of the present invention (i.e. a compound comprising: a targeting moiety and a chelator, wherein the targeting moiety is bound to the chelator, is a quinolone nonpeptide, and binds to a receptor that is upregulated during angiogenesis and the compound has 0-1 linking groups between the targeting moiety and chelator) which is administered in combination therapy, with one or more anti-cancer agent(s)selected from the group consisting of mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, and leutinizing hormone releasing factor.

This combination therapy may further, optionally, include a radiosensitizer agent, or a pharmaceutically acceptable salt thereof, to enhance the radiotherapeutic effect together with the anti-cancer agent, said radiosensitizer agent being selected from the group consisting of 2-(3-nitro-1,2,4-triazol-1-yl)-N-(2-methoxyethyl)acetamide, N-(3-nitro-4-quinolinyl)-4-morpholinecarboxamidine, 3-amino-1,2,4-benzotriazine-1,4-dioxide, N-(2-hydroxyethyl)-2-nitroimidazole-1-acetamide, 1-(2-nitroimidazol-1-yl)-3-(1-piperidinyl)-2-propanol, and 1-(2-nitro-1-imidazolyl)-3-(1-aziridino)-2-propanol. A thorough discussion of radiosensitizer agents is provided in the following: Rowinsky-EK, Oncology-Huntingt., 1999 Oct; 13(10 Suppl 5): 61-70; Chen-AY et al., Oncology-Huntingt. 1999 Oct; 13(10 Suppl 5): 39-46; Choy-H, Oncology-Huntingt. 1999 Oct; 13(10 Suppl 5): 23-38; and Herscher-LL et al, Oncology-Huntingt. 1999 Oct; 13(10 Suppl 5): 11-22, which are incorporated herein by reference.

It is a further aspect of the invention to provide kits having a plurality of active ingredients (with or without carrier) which, together, may be effectively utilized for carrying out the novel combination therapies of the invention.

It is another aspect of the invention to provide a novel pharmaceutical composition which is effective, in and of itself, for utilization in a beneficial combination therapy because it includes compounds of the present invention, and an anti-cancer agent or a radiosensitizer agent, which may be utilized in accordance with the invention.

In another aspect, the present invention provides a method for treating cancer in a patient in need of such treatment, said method including the steps of administering a therapeutically effective amount of a compound of the present invention and administering a therapeutically effective amount of at least one agent selected from the group consisting of an anti-cancer agent and a radiosensitizer agent.

In another aspect, the present invention provides a method of treating cancer in a patient by administering a therapeutic radiopharmaceutical of the present invention in conjunction with photodynamic therapy.

In another aspect, the present invention provides a method of treating cancer in a patient by:
a) administering a therapeutic radiopharmaceutical of the present invention and a photosensitive agent (photoreactive agent) to a patient, said photosensitive agent having a characteristic light absorption waveband and being preferentially absorbed by abnormal tissue;
b)providing an imaging device that is integral with a plurality of light sources and produces a signal used for imaging abnormal tissue at the internal treatment site, said light sources emitting light in a waveband corresponding to the characteristic light absorption waveband of the photosensitive agent, said waveband including wavelengths sufficiently long to penetrate through a dermal layer of the patient to the internal treatment site;
(c) determining a location of the abnormal tissue at the internal targeted site within the body of the patient with the imaging device, by viewing an image of the abnormal tissue at the targeted site developed in response to the signal produced by the imaging device; and
(d) energizing the light sources to administer light therapy to the internal targeted site at the location determined with the imaging device.

It is another aspect of the present invention to treat cancer in a patient by:
a) administering a therapeutic radiopharmaceutical of the present invention and a photosensitive agent (photoreactive agent) to a patient, said photosensitive agent having a characteristic light absorption waveband and being specifically targeted at the targeted tissue by including a binding agent that selectively links the photosensitive agent to the targeted tissue;
b)providing an imaging device that is integral with a plurality of light sources and produces a signal used for imaging abnormal tissue at the internal treatment site, said light sources emitting light in a waveband corresponding to the characteristic light absorption waveband of the photosensitive agent, said waveband including wavelengths sufficiently long to penetrate through a dermal layer of the patient to the internal treatment site;
(c) determining a location of the abnormal tissue at the internal targeted site within the body of the patient with the imaging device, by viewing an image of the abnormal tissue at the targeted site developed in response to the signal produced by the imaging device; and
(d) energizing the light sources to administer light therapy to the internal targeted site at the location determined with the imaging device.

Methods for carrying out photodynamic therapy, and photosensitizers which can be used, are well known in the art. For example, they are described in the following patents which are herein incorporated in their entirety:U.S. Patent No.s 6,248,741, 6,248,734, 6,248,727, 6,248,117, 6,245,811, 6,238,426, 6,238,392, 6,233,481, 6,229,048, 6,232,613, 6,225,333, 6,223,071, 6,219,577, 6,219,575, 6,217,869, 6,217,848, 6,216,540, 6,212,425, 6,211,626, 6,208,886, 6,207,464, 6,207,107, 6,198,532, 6,194,415, and 6,186,628.

It is appreciated that certain features of the invention, which are, for clarity, described in the context of separate embodiments, may also be provided in combination in a single embodiment. Conversely, various features of the invention which are for brevity, described in the context of a single embodiment, may also be provided separately or in any subcombination.

### DEFINITIONS

The compounds herein described may have asymmetric centers. Unless otherwise indicated, all chiral, diastereomeric and racemic forms are included in the present invention. Many geometric isomers of olefins, C=N double bonds, and the like can also be present in the compounds described herein, and all such stable isomers are contemplated in the present invention. It will be appreciated that compounds of the present invention contain asymmetrically substituted carbon atoms, and may be isolated in optically active or racemic forms. It is well known in the art how to prepare optically active forms, such as by resolution of racemic forms or by synthesis from optically active starting materials. Two distinct isomers (cis and trans) of the peptide bond are known to occur; both can also be present in the compounds described herein, and all such stable isomers are contemplated in the present invention. The D and L-isomers of a particular amino acid are designated herein using the conventional 3-letter abbreviation of the amino acid, as indicated by the following examples: D-Leu, or L-Leu.

When any variable occurs more than one time in any substituent or in any formula, its definition on each occurrence is independent of its definition at every other occurrence. Thus, for example, if a group is shown to be substituted with 0-2 R⁵², then said group may optionally be substituted with up to two R⁵², and R⁵² at each occurrence is selected independently from the defined list of possible R⁵². Also, by way of example, for the group -N(R⁵³)₂, each of the two R⁵³ substituents on N is independently selected from the defined list of possible R⁵³. Combinations of substituents and/or variables are permissible only if such combinations result in stable compounds. When a bond to a substituent is shown to cross the bond connecting two atoms in a ring, then such substituent may be bonded to any atom on the ring.

The term "nonpeptide" means preferably less than three amide bonds in the backbone core of the targeting moiety or preferably less than three amino acids or amino acid mimetics in the targeting moiety.

The term "metallopharmaceutical" means a pharmaceutical comprising a metal. The metal is the cause of the imageable signal in diagnostic applications and the source of the cytotoxic radiation in radiotherapeutic applications. Radiopharmaceuticals are metallopharmaceuticals in which the metal is a radioisotope.

By "reagent" is meant a compound of this invention capable of direct transformation into a metallopharmaceutical of this invention. Reagents may be utilized directly for the preparation of the metallopharmaceuticals of this invention or may be a component in a kit of this invention.

The term "binding agent" means a metallopharmaceutical of this invention having affinity for and capable of binding to the vitronectin receptor. The binding agents of this invention have Ki < 1000nM.

By "stable compound" or "stable structure" is meant herein a compound that is sufficiently robust to survive isolation to a useful degree of purity from a reaction mixture, and formulation into an efficacious pharmaceutical agent.

The term "substituted", as used herein, means that one or more hydrogens on the designated atom or group is replaced with a selection from the indicated group, provided that the designated atom's or group's normal valency is not exceeded, and that the substitution results in a stable compound. When a substituent is keto (i.e., =O), then 2 hydrogens on the atom are replaced.

The term "bond", as used herein, means either a single or double bond.

The term "salt", as used herein, is used as defined in the CRC Handbook of Chemistry and Physics, 65th Edition, CRC Press, Boca Raton, Fla, 1984, as any substance which yields ions, other than hydrogen or hydroxyl ions. As used herein, "pharmaceutically acceptable salts" refer to derivatives of the disclosed compounds modified by making acid or base salts. Examples of pharmaceutically acceptable salts include, but are not limited to, mineral or organic acid salts of basic residues such as amines; alkali or organic salts of acidic residues such as carboxylic acids; and the like.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable prodrugs" as used herein means those prodrugs of the compounds useful according to the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" means compounds that are rapidly transformed *in vivo* to yield the parent compound of the above formula, for example by hydrolysis in blood. Functional groups which may be rapidly transformed, by metabolic cleavage, in vivo form a class of groups reactive with the carboxyl group of the compounds of this invention. They include, but are not limited to such groups as alkanoyl (such as acetyl, propionyl, butyryl, and the like), unsubstituted and substituted aroyl (such as benzoyl and substituted benzoyl), alkoxycarbonyl (such as ethoxycarbonyl), trialkylsilyl (such as trimethyl- and triethysilyl), monoesters formed with dicarboxylic acids (such as succinyl), and the like. Because of the ease with which the metabolically cleavable groups of the compounds useful according to this invention are cleaved in vivo, the compounds bearing such groups act as pro-drugs. The compounds bearing the metabolically cleavable groups have the advantage that they may exhibit improved bioavailability as a result of enhanced solubility and/or rate of absorption conferred upon the parent compound by virtue of the presence of the metabolically cleavable group. A thorough discussion of prodrugs is provided in the following: Design of Prodrugs, H. Bundgaard, ed., Elsevier, 1985; Methods in Enzymology, K. Widder et al, Ed., Academic Press, 42, p.309-396, 1985; A Textbook of Drug Design and Development, Krogsgaard-Larsen and H. Bundgaard, ed., Chapter 5; "Design and Applications of Prodrugs" p.113-191, 1991; Advanced Drug Delivery Reviews, H. Bundgard, 8, p.1-38, 1992; Journal of Pharmaceutical Sciences, 77, p. 285, 1988; Chem. Pharm. Bull., N. Nakeya et al, 32, p. 692, 1984; Pro-drugs as Novel Delivery Systems, T. Higuchi and V. Stella, Vol. 14 of the A.C.S. Symposium Series, and Bioreversible Carriers in Drug Design, Edward B. Roche, ed., American Pharmaceutical Association and Pergamon Press, 1987, which are incorporated herein by reference.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

As used herein, "alkyl" is intended to include both branched and straight-chain saturated aliphatic hydrocarbon groups having the specified number of carbon atoms, examples of which include, but are not limited to, methyl, ethyl, n-propyl, i-propyl , n-butyl, i-butyl, sec-butyl, t-butyl, pentyl, hexyl, heptyl, octyl, nonyl, and decyl; "cycloalkyl" or "carbocycle" is intended to include saturated and partially unsaturated ring groups, including mono-, bi- or poly-cyclic ring systems, such as cyclopropyl, cyclobutyl, cyclopentyl, cyclohexyl, cycloheptyl, cyclooctyl and adamantyl; "bicycloalkyl" or "bicyclic" is intended to include saturated bicyclic ring groups such as [3.3.0]bicyclooctane, [4.3.0]bicyclononane, [4.4.0]bicyclodecane (decalin), [2.2.2]bicyclooctane, and so forth.

As used herein, the term "alkene" or "alkenyl" is intended to include hydrocarbon chains having the specified number of carbon atoms of either a straight or branched configuration and one or more unsaturated carbon-carbon bonds which may occur in any stable point along the chain, such as ethenyl, propenyl, and the like.

As used herein, the term "alkyne" or "alkynyl" is intended to include hydrocarbon chains having the specified number of carbon atoms of either a straight or branched configuration and one or more unsaturated carbon-carbon triple bonds which may occur in any stable point along the chain, such as propargyl, and the like.

As used herein, "aryl" or "aromatic residue" is intended to mean phenyl or naphthyl, which when substituted, the substitution can be at any position.

As used herein, the term "heterocycle" or "heterocyclic system" is intended to mean a stable 5- to 7- membered monocyclic or bicyclic or 7- to 10-membered bicyclic heterocyclic ring which is saturated partially unsaturated or unsaturated (aromatic), and which consists of carbon atoms and from 1 to 4 heteroatoms independently selected from the group consisting of N, O and S and including any bicyclic group in which any of the above-defined heterocyclic rings is fused to a benzene ring. The nitrogen and sulfur heteroatoms may optionally be oxidized. The heterocyclic ring may be attached to its pendant group at any heteroatom or carbon atom which results in a stable structure. The heterocyclic rings described herein may be substituted on carbon or on a nitrogen atom if the resulting compound is stable. If specifically noted, a nitrogen in the heterocycle may optionally be quaternized. It is preferred that when the total number of S and O atoms in the heterocycle exceeds 1, then these heteroatoms are not adjacent to one another. It is preferred that the total number of S and O atoms in the heterocycle is not more than 1. As used herein, the term "aromatic heterocyclic system" is intended to mean a stable 5- to 7- membered monocyclic or bicyclic or 7- to 10-membered bicyclic heterocyclic aromatic ring which consists of carbon atoms and from 1 to 4 heteroatoms independently selected from the group consisting of N, O and S. It is preferred that the total number of S and O atoms in the aromatic heterocycle is not more than 1.

Examples of heterocycles include, but are not limited to, 1H-indazole, 2-pyrrolidonyl, 2H,6H-1,5,2-dithiazinyl, 2H-pyrrolyl, 3H-indolyl, 4-piperidonyl, 4aH-carbazole, 4H-quinolizinyl, 6H-1,2,5-thiadiazinyl, acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazalonyl, carbazolyl, 4aH-carbazolyl, .-carbolinyl, chromanyl, chromenyl, cinnolinyl, decahydroquinolinyl, 2H,6H-1,5,2-dithiazinyl, dihydrofuro[2,3-b]tetrahydrofuran, furanyl, furazanyl, imidazolidinyl, imidazolinyl, imidazolyl, 1H-indazolyl, indolenyl, indolinyl, indolizinyl, indolyl, isobenzofuranyl, isochromanyl, isoindazolyl, isoindolinyl, isoindolyl, isoquinolinyl, isothiazolyl, isoxazolyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl., oxazolyl, oxazolidinylperimidinyl, phenanthridinyl, phenanthrolinyl, phenarsazinyl, phenazinyl, phenothiazinyl, phenoxathiinyl, phenoxazinyl, phthalazinyl, piperazinyl, piperidinyl, pteridinyl, piperidonyl, 4-piperidonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, pyrrolyl, quinazolinyl, quinolinyl, 4H-quinolizinyl, quinoxalinyl, quinuclidinyl, carbolinyl, tetrahydrofuranyl, tetrahydroisoquinolinyl, tetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, xanthenyl. Preferred heterocycles include, but are not limited to, pyridinyl, furanyl, thienyl, pyrrolyl, pyrazolyl, imidazolyl, indolyl, benzimidazolyl, 1H-indazolyl, oxazolidinyl, benzotriazolyl, benzisoxazolyl, oxindolyl, benzoxazolinyl, or isatinoyl. Also included are fused ring and spiro compounds containing, for example, the above heterocycles.

As used herein, the term "alkaryl" means an aryl group bearing an alkyl group of 1-10 carbon atoms; the term "aralkyl" means an alkyl group of 1-10 carbon atoms bearing an aryl group; the term "arylalkaryl" means an aryl group bearing an alkyl group of 1-10 carbon atoms bearing an aryl group; and the term "heterocycloalkyl" means an alkyl group of 1-10 carbon atoms bearing a heterocycle.

A "polyalkylene glycol" is a polyethylene glycol, polypropylene glycol or polybutylene glycol having a molecular weight of less than about 5000, terminating in either a hydroxy or alkyl ether moiety.

A "carbohydrate" is a polyhydroxy aldehyde, ketone, alcohol or acid, or derivatives thereof, including polymers thereof having polymeric linkages of the acetal type.

A "cyclodextrin" is a cyclic oligosaccharide. Examples of cyclodextrins include, but are not limited to, α-cyclodextrin, hydroxyethyl-α-cyclodextrin, hydroxypropyl-α-cyclodextrin, β-cyclodextrin, hydroxypropyl-β-cyclodextrin, carboxymethyl-β-cyclodextrin, dihydroxypropyl-β-cyclodextrin, hydroxyethyl-β-cyclodextrin, 2,6 di-O-methyl-β-cyclodextrin, sulfated-β-cyclodextrin, γ-cyclodextrin, hydroxypropyl-γ-cyclodextrin, dihydroxypropyl-γ-cyclodextrin, hydroxyethyl-γ-cyclodextrin, and sulfated γ-cyclodextrin.

As used herein, the term "polycarboxyalkyl" means an alkyl group having between two and about 100 carbon atoms and a plurality of carboxyl substituents; and the term "polyazaalkyl" means a linear or branched alkyl group having between two and about 100 carbon atoms, interrupted by or substituted with a plurality of amine groups.

A "reducing agent" is a compound that reacts with a radionuclide, which is typically obtained as a relatively unreactive, high oxidation state compound, to lower its oxidation state by transferring electron(s) to the radionuclide, thereby making it more reactive. Reducing agents useful in the preparation of radiopharmaceuticals and in diagnostic kits useful for the preparation of said radiopharmaceuticals include but are not limited to stannous chloride, stannous fluoride, formamidine sulfinic acid, ascorbic acid, cysteine, phosphines, and cuprous or ferrous salts. Other reducing agents are described in Brodack et. al., PCT Application 94/22496, which is incorporated herein by reference.

A "transfer ligand" is a ligand that forms an intermediate complex with a metal ion that is stable enough to prevent unwanted side-reactions but labile enough to be converted to a metallopharmaceutical. The formation of the intermediate complex is kinetically favored while the formation of the metallopharmaceutical is thermodynamically favored. Transfer ligands useful in the preparation of metallopharmaceuticals and in diagnostic kits useful for the preparation of diagnostic radiopharmaceuticals include but are not limited to gluconate, glucoheptonate, mannitol, glucarate, N,N,N',N'-ethylenediaminetetraacetic acid, pyrophosphate and methylenediphosphonate. In general, transfer ligands are comprised of oxygen or nitrogen donor atoms.

The term "donor atom" refers to the atom directly attached to a metal by a chemical bond.

"Ancillary" or "co-ligands" are ligands that are incorporated into a radiopharmaceutical during its synthesis. They serve to complete the coordination sphere of the radionuclide together with the chelator or radionuclide bonding unit of the reagent. For radiopharmaceuticals comprised of a binary ligand system, the radionuclide coordination sphere is composed of one or more chelators or bonding units from one or more reagents and one or more ancillary or co-ligands, provided that there are a total of two types of ligands, chelators or bonding units. For example, a radiopharmaceutical comprised of one chelator or bonding unit from one reagent and two of the same ancillary or co-ligands and a radiopharmaceutical comprised of two chelators or bonding units from one or two reagents and one ancillary or co-ligand are both considered to be comprised of binary ligand systems. For radiopharmaceuticals comprised of a ternary ligand system, the radionuclide coordination sphere is composed of one or more chelators or bonding units from one or more reagents and one or more of two different types of ancillary or co-ligands, provided that there are a total of three types of ligands, chelators or bonding units. For example, a radiopharmaceutical comprised of one chelator or bonding unit from one reagent and two different ancillary or co-ligands is considered to be comprised of a ternary ligand system.

Ancillary or co-ligands useful in the preparation of radiopharmaceuticals and in diagnostic kits useful for the preparation of said radiopharmaceuticals are comprised of one or more oxygen, nitrogen, carbon, sulfur, phosphorus, arsenic, selenium, and tellurium donor atoms. A ligand can be a transfer ligand in the synthesis of a radiopharmaceutical and also serve as an ancillary or co-ligand in another radiopharmaceutical. Whether a ligand is termed a transfer or ancillary or co-ligand depends on whether the ligand remains in the radionuclide coordination sphere in the radiopharmaceutical, which is determined by the coordination chemistry of the radionuclide and the chelator or bonding unit of the reagent or reagents.

A "chelator" or "bonding unit" is the moiety or group on a reagent that binds to a metal ion through the formation of chemical bonds with one or more donor atoms.

The term "binding site" means the site in vivo or in vitro that binds a biologically active molecule.

A "diagnostic kit" or "kit" comprises a collection of components, termed the formulation, in one or more vials which are used by the practicing end user in a clinical or pharmacy setting to synthesize diagnostic radiopharmaceuticals. The kit provides all the requisite components to synthesize and use the diagnostic radiopharmaceutical except those that are commonly available to the practicing end user, such as water or saline for injection, a solution of the radionuclide, equipment for heating the kit during the synthesis of the radiopharmaceutical, if required, equipment necessary for administering the radiopharmaceutical to the patient such as syringes and shielding, and imaging equipment.

Therapeutic radiopharmaceuticals, X-ray contrast agent pharmaceuticals, ultrasound contrast agent pharmaceuticals and metallopharmaceuticals for magnetic resonance imaging contrast are provided to the end user in their final form in a formulation contained typically in one vial, as either a lyophilized solid or an aqueous solution. The end user reconstitutes the lyophilized with water or saline and withdraws the patient dose or just withdraws the dose from the aqueous solution formulation as provided.

A "lyophilization aid" is a component that has favorable physical properties for lyophilization, such as the glass transition temperature, and is added to the formulation to improve the physical properties of the combination of all the components of the formulation for lyophilization.

A "stabilization aid" is a component that is added to the metallopharmaceutical or to the diagnostic kit either to stabilize the metallopharmaceutical or to prolong the shelf-life of the kit before it must be used. Stabilization aids can be antioxidants, reducing agents or radical scavengers and can provide improved stability by reacting preferentially with species that degrade other components or the metallopharmaceutical.

A "solubilization aid" is a component that improves the solubility of one or more other components in the medium required for the formulation.

A "bacteriostat" is a component that inhibits the growth of bacteria in a formulation either during its storage before use of after a diagnostic kit is used to synthesize a radiopharmaceutical.

The following abbreviations are used herein:
- Acm: acetamidomethyl
- b-Ala, beta-Ala or bAla: 3-aminopropionic acid
- ATA: 2-aminothiazole-5-acetic acid or 2-aminothiazole-5-acetyl group
- Boc: t-butyloxycarbonyl
- CBZ, Cbz or Z: Carbobenzyloxy
- Cit: citrulline
- Dap: 2,3-diaminopropionic acid
- DCC: dicyclohexylcarbodiimide
- DIEA: diisopropylethylamine
- DMAP: 4-dimethylaminopyridine
- EOE: ethoxyethyl
- HBTU: 2-(1H-Benzotriazol-1-yl)-1,1,3,3-tetramethyluronium hexafluorophosphate hynic boc-hydrazinonicotinyl group or 2-[[[5- [carbonyl]-2-pyridinyl]hydrazono]methyl]-benzenesulfonic acid,
- NMeArg: or MeArga-N-methyl arginine
- NMeAsp: a-N-methyl aspartic acid
- NMM: N-methylmorpholine
- OcHex: O-cyclohexyl
- OBzl: O-benzyl
- oSu: O-succinimidyl
- TBTU: 2-(1H-Benzotriazol-1-yl)-1,1,3,3-tetramethyluronium tetrafluoroborate
- THF: tetrahydrofuranyl
- THP: tetrahydropyranyl
- Tos: tosyl
- Tr: trityl

The following conventional three-letter amino acid abbreviations are used herein; the conventional one-letter amino acid abbreviations are NOT used herein:
- Ala =: alanine
- Arg =: arginine
- Asn =: asparagine
- Asp =: aspartic acid
- Cys =: cysteine
- Gln =: glutamine
- Glu =: glutamic acid
- Gly =: glycine
- His =: histidine
- Ile =: isoleucine
- Leu =: leucine
- Lys =: lysine
- Met =: methionine
- Nle =: norleucine
- Orn =: ornithine
- Phe =: phenylalanine
- Phg =: phenylglycine
- Pro =: proline
- Sar =: sarcosine
- Ser =: serine
- Thr =: threonine
- Trp =: tryptophan
- Tyr =: tyrosine
- Val =: valine

As used herein, the term "bubbles", as used herein, refers to vesicles which are generally characterized by the presence of one or more membranes or walls surrounding an internal void that is filled with a gas or precursor thereto. Exemplary bubbles include, for example, liposomes, micelles and the like.

As used herein, the term "lipid" refers to a synthetic or naturally-occurring amphipathic compound which comprises a hydrophilic component and a hydrophobic component. Lipids include, for example, fatty acids, neutral fats, phosphatides, glycolipids, aliphatic alchols and waxes, terpenes and steroids.

As used herein, the term "lipid composition" refers to a composition which comprises a lipid compound. Exemplary lipid compositions include suspensions, emulsions and vesicular compositions.

As used herein, the term "lipid formulation" refers to a composition which comprises a lipid compound and a bioactive agent.

As used herein, the term "vesicle" refers to a spherical entity which is characterized by the presence of an internal void. Preferred vesicles are formulated from lipids, including the various lipids described herein. In any give vesicle, the lipids may be in the form of a monolayer or bilayer, and the mono- or bilayer lipids may be used to form one of more mono- or bilayers. In the case of more than one mono- or bilayer, the mono- or bilayers are generally concentric. The lipid vesicles described herein include such entities commonly referred to as liposomes, micelles, bubbles, microbubbles, microspheres and the like. Thus, the lipids may be used to form a unilamellar vesicle (comprised of one monolayer or bilayer), an oligolamellar vesicle (comprised of about two or about three monolayers or bilayers) or a multilamellar vesicle (comprised of more than about three monolayers or bilayers). The internal void of the vesicles may be filled with a liquid, including, for example, an aqueous liquid, a gas, a gaseous precursor, and/or a solid or solute material, including, for example, a bioactive agent, as desired.

As used herein, the term "vesicular composition" refers to a composition which is formulate from lipids and which comprises vesicles.

As used herein, the term "vesicle formulation" refers to a composition which comprises vesicles and a bioactive agent.

As used herein, the term "lipsomes" refers to a generally spherical cluster or aggregate of amphipathic compounds, including lipid compounds, typically in the form of one or more concentric layers, for example, bilayers. They may also be referred to herein as lipid vesicles.

Angiogenesis is the process of formation of new capillary blood vessels from existing vasculature. It is an important component of a variety of physiological processes including ovulation, embryonic development, wound repair, and collateral vascular generation in the myocardium. It is also central to a number of pathological conditions such as tumor growth and metastasis, diabetic retinopathy, and macular degeneration. The process begins with the activation of existing vascular endothelial cells in response to a variety of cytokines and growth factors. The activated endothelial cells secrete enzymes that degrade the basement membrane of the vessels. The endothelial cells then proliferate and migrate into the extracellular matrix first forming tubules and subsequently new blood vessels.

Under normal conditions, endothelial cell proliferation is a very slow process, but it increases for a short period of time during embryogenesis, ovulation and wound healing. This temporary increase in cell turnover is governed by a combination of a number of growth stimulatory factors and growth suppressing factors. In pathological angiogenesis, this normal balance is disrupted resulting in continued increased endothelial cell proliferation. Some of the proangiogenic factors that have been identified include basic fibroblast growth factor (bFGF), angiogenin, TGF-alpha, TGF-beta, and vascular endothelium growth factor (VEGF), while interferon-alpha, interferon-beta and thrombospondin are examples of angiogenesis suppressors.

Angiogenic factors interact with endothelial cell surface receptors such as the receptor tyrosine kinases EGFR, FGFR, PDGFR, Flk-1/KDR, Flt-1, Tek, Tie, neuropilin-1, endoglin, endosialin, and Axl. The receptors Flk-1/KDR, neuropilin-1, and Flt-1 recognize VEGF and these interactions play key roles in VEGF-induced angiogenesis. The Tie subfamily of receptor tyrosine kinases are also expressed prominently during blood vessel formation.

The proliferation and migration of endothelial cells in the extracellular matrix is mediated by interaction with a variety of cell adhesion molecules. Integrins are a diverse family of heterodimeric cell surface receptors by which endothelial cells attach to the extracellular matrix, each other and other cells. Angiogenesis induced by bFGF or TNF-alpha depend on the agency of the integrin avb3, while angiogenesis induced by VEGF depends on the integrin avb5 (Cheresh et. al., Science, 1995, 270, 1500-2). Induction of expression of the integrins alb1 and a2b1 on the endothelial cell surface is another important mechanism by which VEGF promotes angiogenesis (Senger, et. al., Proc. Natl. Acad, Sci USA, 1997, 94, 13612-7).

The pharmaceuticals of the present invention are comprised of a non-peptide targeting moiety for the vitronectin receptor that is expressed or upregulated in angiogenic tumor vasculature.

The ultrasound contrast agents of the present invention comprise a plurality of vitronectin receptor targeting moieties attached to or incorporated into a microbubble of a biocompatible gas, a liquid carrier, and a surfactant microsphere, further comprising an optional linking moiety, Lₙ, between the targeting moieties and the microbubble. In this context, the term liquid carrier means aqueous solution and the term surfactant means any amphiphilic material which produces a reduction in interfacial tension in a solution. A list of suitable surfactants for forming surfactant microspheres is disclosed in EP0727225A2, herein incorporated by reference. The term surfactant microsphere includes nanospheres, liposomes, vesicles and the like. The biocompatible gas can be air, or a fluorocarbon, such as a C₃-C₅ perfluoroalkane, which provides the difference in echogenicity and thus the contrast in ultrasound imaging. The gas is encapsulated or contained in the microsphere to which is attached the biodirecting group, optionally via a linking group. The attachment can be covalent, ionic or by van der Waals forces. Specific examples of such contrast agents include lipid encapsulated perfluorocarbons with a plurality of tumor neovasculature receptor binding peptides, polypeptides or peptidomimetics.

X-ray contrast agents of the present invention are comprised of one or more vitronectin receptor targeting moieties attached to one or more X-ray absorbing or "heavy" atoms of atomic number 20 or greater, further comprising an optional linking moiety, Lₙ, between the targeting moieties and the X-ray absorbing atoms. The frequently used heavy atom in X-ray contrast agents is iodine. Recently, X-ray contrast agents comprised of metal chelates (Wallace, R., U.S. 5,417,959) and polychelates comprised of a plurality of metal ions (Love, D., U.S. 5,679,810) have been disclosed. More recently, multinuclear cluster complexes have been disclosed as X-ray contrast agents (U.S. 5,804,161, PCT WO91/14460, and PCT WO 92/17215).

MRI contrast agents of the present invention are comprised of one or more vitronectin receptor targeting moieties attached to one or more paramagnetic metal ions, further comprising an optional linking moiety, Lₙ, between the targeting moieties and the paramagnetic metal ions. The paramagnetic metal ions are present in the form of metal complexes or metal oxide particles. U.S. 5,412,148, and 5,760,191, describe examples of chelators for paramagnetic metal ions for use in MRI contrast agents. U.S. 5,801,228, U.S. 5,567,411, and U.S. 5,281,704, describe examples of polychelants useful for complexing more than one paramagnetic metal ion for use in MRI contrast agents. U.S. 5,520,904, describes particulate compositions comprised of paramagnetic metal ions for use as MRI contrast agents.

Administration of a compound of the present invention in combination with such additional therapeutic agents, may afford an efficacy advantage over the compounds and agents alone, and may do so while permitting the use of lower doses of each. A lower dosage minimizes the potential of side effects, thereby providing an increased margin of safety. The combination of a compound of the present invention with such additional therapeutic agents is preferably a synergistic combination. Synergy, as described for example by Chou and Talalay, Adv. Enzyme Regul. 22:27-55 (1984), occurs when the therapeutic effect of the compound and agent when administered in combination is greater than the additive effect of the either the compound or agent when administered alone. In general, a synergistic effect is most clearly demonstrated at levels that are (therapeutically) sub-optimal for either the compound of the present invention, an anti-cancer agent or a radiosensitizer agent alone, but which are highly efficacious in combination. Synergy can be in terms of improved tumor response without substantial increases in toxicity over individual treatments alone, or some other beneficial effect of the combination compared with the individual components.

The compounds of the present invention, and an anti-cancer agent or a radiosensitizer agent, utilized in combination therapy may be administered simultaneously, in either separate or combined formulations, or at different times e.g., sequentially, such that a combined effect is achieved. The amounts and regime of administration will be adjusted by the practitioner, by preferably initially lowering their standard doses and then titrating the results obtained.

The invention also provides kits or single packages combining two or more active ingredients useful in treating cancer. A kit may provide (alone or in combination with a pharmaceutically acceptable diluent or carrier), the compound of the present invention and additionally at least one agent selected from the group consisting of an anti-cancer agent and a radiosensitizer agent (alone or in combination with diluent or carrier).

The pharmaceuticals of the present invention have the formulae, (Q)_{d}-Lₙ-(Cₕ-X), (Q)_{d}-Lₙ- (Cₕ-X¹)_{d'}, (Q)_{d}-Lₙ-(X²)_{d"}, and (Q)_{d}-Lₙ-(X³), wherein Q represents a non-peptide that binds to a receptor expressed in angiogenic tumor vasculature, d is 1-10, Lₙ represents an optional linking group, Cₕ represents a metal chelator or bonding moiety, X represents a radioisotope, X¹ represents paramagnetic metal ion, X² represents a paramagnetic metal ion or heavy atom containing insoluble solid particle, d" is 1-100, and X³ represents a surfactant microsphere of an echogenic gas. The interaction of the non-peptide recognition sequences of the vitronectin receptor binding portion of the pharmaceuticals with the αvβ3 receptor results in localization of the pharmaceuticals in angiogenic tumor vasculature, which express the αvβ3 receptor.

The pharmaceuticals of the present invention can be synthesized by several approaches. One approach involves the synthesis of the targeting non-peptide moiety, Q, and direct attachment of one or more moieties, Q, to one or more metal chelators or bonding moieties, Cₕ, or to a paramagnetic metal ion or heavy atom containing solid particle, or to an echogenic gas microbubble. Another approach involves the attachment of one or more moieties, Q, to the linking group, Lₙ, which is then attached to one or more metal chelators or bonding moieties, Cₕ, or to a paramagnetic metal ion or heavy atom containing solid particle, or to an echogenic gas microbubble. Another approach involves the synthesis of a non-peptide, Q, bearing a fragment of the linking group, Lₙ, one or more of which are then attached to the remainder of the linking group and then to one or more metal chelators or bonding moieties, Cₕ, or to a paramagnetic metal ion or heavy atom containing solid particle, or to an echogenic gas microbubble.

The non-peptide vitronectin binding moieties, Q, optionally bearing a linking group, Lₙ, or a fragment of the linking group, can be synthesized using standard synthetic methods known to those skilled in the art. Preferred methods include but are not limited to those methods described below.

The attachment of linking groups, Lₙ, to the non-peptides, Q; chelators or bonding units, Cₕ, to the non-peptides, Q, or to the linking groups, Lₙ; and non-peptides, bearing a fragment of the linking group to the remainder of the linking group, in combination forming the moiety, (Q)_{d}-Lₙ, and then to the moiety Cₕ; can all be performed by standard techniques. These include, but are not limited to, amidation, esterification, alkylation, and the formation of ureas or thioureas. Procedures for performing these attachments can be found in Brinkley, M., Bioconjugate Chemistry 1992, 3(1), which is incorporated herein by reference.

A number of methods can be used to attach the non-peptides, Q, to paramagnetic metal ion or heavy atom containing solid particles, X², by one of skill in the art of the surface modification of solid particles. In general, the targeting moiety Q or the combination (Q)_{d}Lₙ is attached to a coupling group that react with a constituent of the surface of the solid particle. The coupling groups can be any of a number of silanes which react with surface hydroxyl groups on the solid particle surface, as described in co-pending United States Patent Application Serial No. 09/356,178, and can also include polyphosphonates, polycarboxylates, polyphosphates or mixtures thereof which couple with the surface of the solid particles, as described in U.S. 5,520,904.

A number of reaction schemes can be used to attach the non-peptides, Q, to the surfactant microsphere, X³. These are illustrated in following reaction schemes where S_{f} represents a surfactant moiety that forms the surfactant microsphere.

### Acylation Reaction:

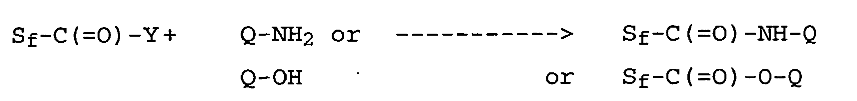

Y is a leaving group or active ester

### Disulfide Coupling:

S_{f}-SH + Q-SH -------> S_{f}-S-S-Q

### Sulfonamide Coupling:

S_{f}-S (=O)₂-Y + Q-NH₂ ----------- > S_{f}-S(=O)₂- NH-Q

### Reductive Amidation:

S_{f}-CHO + Q-NH₂ -----------> S_{f}-NH-Q

In these reaction schemes, the substituents S_{f} and Q can be reversed as well.

The linking group Lₙ can serve several roles. First it provides a spacing group between the metal chelator or bonding moiety, Cₕ, the paramagnetic metal ion or heavy atom containing solid particle, X², and the surfactant microsphere, X³, and the one or more of the non-peptides, Q, so as to minimize the possibility that the moieties Cₕ-X, Cₕ-X¹, X², and X³, will interfere with the interaction of the recognition sequences of Q with angiogenic tumor vasculature receptors. The necessity of incorporating a linking group in a reagent is dependent on the identity of Q, Cₕ-X, Cₕ-X¹, X², and X³. If Cₕ-X, Cₕ-X¹, X², and X³, cannot be attached to Q without substantially diminishing its affinity for the receptors, then a linking group is used. A linking group also provides a means of independently attaching multiple non-peptides, Q, to one group that is attached to Cₕ-X, Cₕ-X¹ , _{X}² , or X³.

The linking group also provides a means of incorporating a pharmacokinetic modifier into the pharmaceuticals of the present invention. The pharmacokinetic modifier serves to direct the biodistibution of the injected pharmaceutical other than by the interaction of the targeting moieties, Q, with the vitronectin receptors expressed in the tumor neovasculature. A wide variety of functional groups can serve as pharmacokinetic modifiers, including, but not limited to, carbohydrates, polyalkylene glycols, peptides or other polyamino acids, and cyclodextrins. The modifiers can be used to enhance or decrease hydrophilicity and to enhance or decrease the rate of blood clearance. The modifiers can also be used to direct the route of elimination of the pharmaceuticals. Preferred pharmacokinetic modifiers are those that result in moderate to fast blood clearance and enhanced renal excretion.

The metal chelator or bonding moiety, Cₕ, is selected to form stable complexes with the metal ion chosen for the particular application. Chelators or bonding moieties for diagnostic radiopharmaceuticals are selected to form stable complexes with the radioisotopes that have imageable gamma ray or positron emissions, such as ^{99m}Tc, ⁹⁵Tc, ¹¹¹In ⁶²Cu, ⁶⁰Cu, ⁶⁴Cu, ⁶⁷Ga, ⁶⁸Ga, ⁸⁶Y.

Chelators for technetium, copper and gallium isotopes are selected from diaminedithiols, monoamine-monoamidedithiols, triamide-monothiols, monoamine-diamide-monothiols, diaminedioximes, and hydrazines. The chelators are generally tetradentate with donor atoms selected from nitrogen, oxygen and sulfur. Preferred reagents are comprised of chelators having amine nitrogen and thiol sulfur donor atoms and hydrazine bonding units. The thiol sulfur atoms and the hydrazines may bear a protecting group which can be displaced either prior to using the reagent to synthesize a radiopharmaceutical or preferably in situ during the synthesis of the radiopharmaceutical.

Exemplary thiol protecting groups include those listed in Greene and Wuts, "Protective Groups in Organic Synthesis" John Wiley & Sons, New York (1991), the disclosure of which is hereby incorporated by reference. Any thiol protecting group known in the art can be used. Examples of thiol protecting groups include, but are not limited to, the following: acetamidomethyl, benzamidomethyl, 1-ethoxyethyl, benzoyl, and triphenylmethyl.

Exemplary protecting groups for hydrazine bonding units are hydrazones which can be aldehyde or ketone hydrazones having substituents selected from hydrogen, alkyl, aryl and heterocycle. Particularly preferred hydrazones are described in co-pending U.S.S.N. 08/476,296 the disclosure of which is herein incorporated by reference in its entirety.

The hydrazine bonding unit when bound to a metal radionuclide is termed a hydrazido, or diazenido group and serves as the point of attachment of the radionuclide to the remainder of the radiopharmaceutical. A diazenido group can be either terminal (only one atom of the group is bound to the radionuclide) or chelating. In order to have a chelating diazenido group at least one other atom of the group must also be bound to the radionuclide. The atoms bound to the metal are termed donor atoms.

Chelators for ¹¹¹In and ⁸⁶Y are selected from cyclic and acyclic polyaminocarboxylates such as DTPA, DOTA, D03A, 2-benzyl-DOTA, alpha-(2-phenethyl)1,4,7,10-tetraazazcyclododecane-1-acetic-4,7,10-tris(methylacetic)acid, 2-benzylcyclohexyldiethylenetriaminepentaacetic acid, 2-benzyl-6-methyl-DTPA, and 6,6"-bis[N,N,N",N"-tetra(carboxymethyl)aminomethyl)-4'-(3-amino-4-methoxyphenyl)-2,2':6',2"-terpyridine. Procedures for synthesizing these chelators that are not commercially available can be found in Brechbiel, M. and Gansow, O., J. Chem. Soc. Perkin Trans. 1992, 1, 1175; Brechbiel, M. and Gansow, 0., Bioconjugate Chem. 1991, 2, 187; Deshpande, S., et. al., J. Nucl. Med. 1990, 31, 473; Kruper, J., U.S. Patent 5,064,956, and Toner, J., U.S. Patent 4,859,777, the disclosures of which are hereby incorporated by reference in their entirety.

The coordination sphere of metal ion includes all the ligands or groups bound to the metal. For a transition metal radionuclide to be stable it typically has a coordination number (number of donor atoms) comprised of an integer greater than or equal to 4 and less than or equal to 8; that is there are 4 to 8 atoms bound to the metal and it is said to have a complete coordination sphere. The requisite coordination number for a stable radionuclide complex is determined by the identity of the radionuclide, its oxidation state, and the type of donor atoms. If the chelator or bonding unit does not provide all of the atoms necessary to stabilize the metal radionuclide by completing its coordination sphere, the coordination sphere is completed by donor atoms from other ligands, termed ancillary or co-ligands, which can also be either terminal or chelating.

A large number of ligands can serve as ancillary or co-ligands, the choice of which is determined by a variety of considerations such as the ease of synthesis of the radiopharmaceutical, the chemical and physical properties of the ancillary ligand, the rate of formation, the yield, and the number of isomeric forms of the resulting radiopharmaceuticals, the ability to administer said ancillary or co-ligand to a patient without adverse physiological consequences to said patient, and the compatibility of the ligand in a lyophilized kit formulation. The charge and lipophilicity of the ancillary ligand will effect the charge and lipophilicity of the radiopharmaceuticals. For example, the use of 4,5-dihydroxy-1,3-benzene disulfonate results in radiopharmaceuticals with an additional two anionic groups because the sulfonate groups will be anionic under physiological conditions. The use of N-alkyl substituted 3,4-hydroxypyridinones results in radiopharmaceuticals with varying degrees of lipophilicity depending on the size of the alkyl substituents.

Preferred technetium radiopharmaceuticals of the present invention are comprised of a hydrazido or diazenido bonding unit and an ancillary ligand, A_{L1}, or a bonding unit and two types of ancillary A_{L1} and A_{L2}, or a tetradentate chelator comprised of two nitrogen and two sulfur atoms. Ancillary ligands A_{L1} are comprised of two or more hard donor atoms such as oxygen and amine nitrogen (sp³ hybridized). The donor atoms occupy at least two of the sites in the coordination sphere of the radionuclide metal; the ancillary ligand A_{L1} serves as one of the three ligands in the ternary ligand system. Examples of ancillary ligands A_{L1} include but are not limited to dioxygen ligands and functionalized aminocarboxylates. A large number of such ligands are available from commercial sources.

Ancillary dioxygen ligands include ligands that coordinate to the metal ion through at least two oxygen donor atoms. Examples include but are not limited to: glucoheptonate, gluconate, 2-hydroxyisobutyrate, lactate, tartrate, mannitol, glucarate, maltol, Kojic acid, 2,2-bis(hydroxymethyl)propionic acid, 4,5-dihydroxy-1,3-benzene disulfonate, or substituted or unsubstituted 1,2 or 3,4 hydroxypyridinones. (The names for the ligands in these examples refer to either the protonated or non-protonated forms of the ligands.)

Functionalized aminocarboxylates include ligands that have a combination of amine nitrogen and oxygen donor atoms. Examples include but are not limited to: iminodiacetic acid, 2,3-diaminopropionic acid, nitrilotriacetic acid, N,N'-ethylenediamine diacetic acid, N,N,N'-ethylenediamine triacetic acid, hydroxyethylethylenediamine triacetic acid, and N,N'-ethylenediamine bis-hydroxyphenylglycine. (The names for the ligands in these examples refer to either the protonated or non-protonated forms of the ligands.)

A series of functionalized aminocarboxylates are disclosed by Bridger et. al. in U.S. Patent 5,350,837, herein incorporated by reference, that result in improved rates of formation of technetium labeled hydrazino modified proteins. We have determined that certain of these aminocarboxylates result in improved yields of the radiopharmaceuticals of the present invention. The preferred ancillary ligands A_{L1} functionalized aminocarboxylates that are derivatives of glycine; the most preferred is tricine (tris(hydroxymethyl)methylglycine).

The most preferred technetium radiopharmaceuticals of the present invention are comprised of a hydrazido or diazenido bonding unit and two types of ancillary designated A_{L1} and A_{L2}, or a diaminedithiol chelator. The second type of ancillary ligands A_{L2} are comprised of one or more soft donor atoms selected from the group: phosphine phosphorus, arsine arsenic, imine nitrogen (sp² hybridized), sulfur (sp² hybridized) and carbon (sp hybridized); atoms which have p-acid character. Ligands A_{L2} can be monodentate, bidentate or tridentate, the denticity is defined by the number of donor atoms in the ligand. One of the two donor atoms in a bidentate ligand and one of the three donor atoms in a tridentate ligand must be a soft donor atom. We have disclosed in co-pending U.S.S.N. 08/415,908, and U.S.S.N. 60/013360 and 08/646,886, the disclosures of which are herein incorporated by reference in their entirety, that radiopharmaceuticals comprised of one or more ancillary or co-ligands A_{L2} are more stable compared to radiopharmaceuticals that are not comprised of one or more ancillary ligands, A_{L2}; that is, they have a minimal number of isomeric forms, the relative ratios of which do not change significantly with time, and that remain substantially intact upon dilution.

The ligands A_{L2} that are comprised of phosphine or arsine donor atoms are trisubstituted phosphines, trisubstituted arsines, tetrasubstituted diphosphines and tetrasubstituted diarsines. The ligands A_{L2} that are comprised of imine nitrogen are unsaturated or aromatic nitrogen-containing, 5 or 6-membered heterocycles. The ligands that are comprised of sulfur (sp² hybridized) donor atoms are thiocarbonyls, comprised of the moiety C=S. The ligands comprised of carbon (sp hybridized) donor atoms are isonitriles, comprised of the moiety CNR, where R is an organic radical. A large number of such ligands are available from commercial sources. Isonitriles can be synthesized as described in European Patent 0107734 and in U.S. Patent 4,988,827, herein incorporated by reference.

Preferred ancillary ligands A_{L2} are trisubstituted phosphines and unsaturated or aromatic 5 or 6 membered heterocycles. The most preferred ancillary ligands A_{L2} are trisubstituted phosphines and unsaturated 5 membered heterocycles.

The ancillary ligands A_{L2} may be substituted with alkyl, aryl, alkoxy, heterocycle, aralkyl, alkaryl and arylalkaryl groups and may or may not bear functional groups comprised of heteroatoms such as oxygen, nitrogen, phosphorus or sulfur. Examples of such functional groups include but are not limited to: hydroxyl, carboxyl, carboxamide, nitro, ether, ketone, amino, ammonium, sulfonate, sulfonamide, phosphonate, and phosphonamide. The functional groups may be chosen to alter the lipophilicity and water solubility of the ligands which may affect the biological properties of the radiopharmaceuticals, such as altering the distribution into non-target tissues, cells or fluids, and the mechanism and rate of elimination from the body.

Chelators or bonding moieties for therapeutic radiopharmaceuticals are selected to form stable complexes with the radioisotopes that have alpha particle, beta particle, Auger or Coster-Kronig electron emissions, such as ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir. Chelators for rhenium, copper, palladium, platinum, iridium, rhodium, silver and gold isotopes are selected from diaminedithiols, monoamine-monoamidedithiols, triamide-monothiols, monoamine-diamide-monothiols, diaminedioximes, and hydrazines. Chelators for yttrium, bismuth, and the lanthanide isotopes are selected from cyclic and acyclic polyaminocarboxylates such as DTPA, DOTA, D03A, 2-benzyl-DOTA, alpha-(2-phenethyl)1,4,7,10-tetraazacyclododecane-1-acetic-4,7,10-tris(methylacetic)acid, 2-benzyl-cyclohexyldiethylenetriaminepentaacetic acid, 2-benzyl-6-methyl-DTPA, and 6,6"-bis[N,N,N",N"-tetra(carboxymethyl)aminomethyl)-4'-(3-amino-4-methoxyphenyl)-2,2':6',2"-terpyridine.

Chelators for magnetic resonance imaging contrast agents are selected to form stable complexes with paramagnetic metal ions, such as Gd(III), Dy(III), Fe(III), and Mn(II), are selected from cyclic and acyclic polyaminocarboxylates such as DTPA, DOTA, D03A, 2-benzyl-DOTA, alpha-(2-phenethyl)1,4,7,10-tetraazacyclododecane-1-acetic-4,7,10-tris(methylacetic)acid, 2-benzyl-cyclohexyldiethylenetriaminepentaacetic acid, 2-benzyl-6-methyl-DTPA, and 6,6"-bis[N,N,N",N"-tetra(carboxymethyl)aminomethyl)-4'-(3-amino-4-methoxyphenyl)-2,2':6',2"-terpyridine.

The technetium and rhenium radiopharmaceuticals of the present invention comprised of a hydrazido or diazenido bonding unit can be easily prepared by admixing a salt of a radionuclide, a reagent of the present invention, an ancillary ligand A_{L1}, an ancillary ligand A_{L2}, and a reducing agent, in an aqueous solution at temperatures from 0 to 100 °C. The technetium and rhenium radiopharmaceuticals of the present invention comprised of a tetradentate chelator having two nitrogen and two sulfur atoms can be easily prepared by admixing a salt of a radionuclide, a reagent of the present invention, and a reducing agent, in an aqueous solution at temperatures from 0 to 100 °C.

When the bonding unit in the reagent of the present invention is present as a hydrazone group, then it must first be converted to a hydrazine, which may or may not be protonated, prior to complexation with the metal radionuclide. The conversion of the hydrazone group to the hydrazine can occur either prior to reaction with the radionuclide, in which case the radionuclide and the ancillary or co-ligand or ligands are combined not with the reagent but with a hydrolyzed form of the reagent bearing the chelator or bonding unit, or in the presence of the radionuclide in which case the reagent itself is combined with the radionuclide and the ancillary or co-ligand or ligands. In the latter case, the pH of the reaction mixture must be neutral or acidic.

Alternatively, the radiopharmaceuticals of the present invention comprised of a hydrazido or diazenido bonding unit can be prepared by first admixing a salt of a radionuclide, an ancillary ligand A_{L1}, and a reducing agent in an aqueous solution at temperatures from 0 to 100 °C to form an intermediate radionuclide complex with the ancillary ligand A_{L1} then adding a reagent of the present invention and an ancillary ligand A_{L2} and reacting further at temperatures from 0 to 100 °C.

Alternatively, the radiopharmaceuticals of the present invention comprised of a hydrazido or diazenido bonding unit can be prepared by first admixing a salt of a radionuclide, an ancillary ligand A_{L1}, a reagent of the present invention, and a reducing agent in an aqueous solution at temperatures from 0 to 100 °C to form an intermediate radionuclide complex, and then adding an ancillary ligand A_{L2} and reacting further at temperatures from 0 to 100 °C.

The technetium and rhenium radionuclides are preferably in the chemical form of pertechnetate or perrhenate and a pharmaceutically acceptable cation. The pertechnetate salt form is preferably sodium pertechnetate such as obtained from commercial Tc-99m generators. The amount of pertechnetate used to prepare the radiopharmaceuticals of the present invention can range from 0.1 mCi to 1 Ci, or more preferably from 1 to 200 mCi.

The amount of the reagent of the present invention used to prepare the technetium and rhenium radiopharmaceuticals of the present invention can range from 0.01 µg to 10 mg, or more preferably from 0.5 µg to 200 µg. The amount used will be dictated by the amounts of the other reactants and the identity of the radiopharmaceuticals of the present invention to be prepared.

The amounts of the ancillary ligands A_{L1} used can range from 0.1 mg to 1 g, or more preferably from 1 mg to 100 mg. The exact amount for a particular radiopharmaceutical is a function of identity of the radiopharmaceuticals of the present invention to be prepared, the procedure used and the amounts and identities of the other reactants. Too large an amount of A_{L1} will result in the formation of by-products comprised of technetium labeled A_{L1} without a biologically active molecule or by-products comprised of technetium labeled biologically active molecules with the ancillary ligand A_{L1} but without the ancillary ligand A_{L2}. Too small an amount of A_{L1} will result in other by-products such as technetium labeled biologically active molecules with the ancillary ligand A_{L2} but without the ancillary ligand A_{L1}, or reduced hydrolyzed technetium, or technetium colloid.

The amounts of the ancillary ligands A_{L2} used can range from 0.001 mg to 1 g, or more preferably from 0.01 mg to 10 mg. The exact amount for a particular radiopharmaceutical is a function of the identity of the radiopharmaceuticals of the present invention to be prepared, the procedure used and the amounts and identities of the other reactants. Too large an amount of A_{L2} will result in the formation of by-products comprised of technetium labeled A_{L2} without a biologically active molecule or by-products comprised of technetium labeled biologically active molecules with the ancillary ligand A_{L2} but without the ancillary ligand A_{L1}. If the reagent bears one or more substituents that are comprised of a soft donor atom, as defined above, at least a ten-fold molar excess of the ancillary ligand A_{L2} to the reagent of formula 2 is required to prevent the substituent from interfering with the coordination of the ancillary ligand A_{L2} to the metal radionuclide.

Suitable reducing agents for the synthesis of the radiopharmaceuticals of the present invention include stannous salts, dithionite or bisulfite salts, borohydride salts, and formamidinesulfinic acid, wherein the salts are of any pharmaceutically acceptable form. The preferred reducing agent is a stannous salt. The amount of a reducing agent used can range from 0.001 mg to 10 mg, or more preferably from 0.005 mg to 1 mg.

The specific structure of a radiopharmaceutical of the present invention comprised of a hydrazido or diazenido bonding unit will depend on the identity of the reagent of the present invention used, the identity of any ancillary ligand A_{L1}, the identity of any ancillary ligand A_{L2}, and the identity of the radionuclide. Radiopharmaceuticals comprised of a hydrazido or diazenido bonding unit synthesized using concentrations of reagents of <100 µg/mL, will be comprised of one hydrazido or diazenido group. Those synthesized using >1 mg/mL concentrations will be comprised of two hydrazido or diazenido groups from two reagent molecules. For most applications, only a limited amount of the biologically active molecule can be injected and not result in undesired side-effects, such as chemical toxicity, interference with a biological process or an altered biodistribution of the radiopharmaceutical. Therefore, the radiopharmaceuticals which require higher concentrations of the reagents comprised in part of the biologically active molecule, will have to be diluted or purified after synthesis to avoid such side-effects.

The identities and amounts used of the ancillary ligands A_{L1} and A_{L2} will determine the values of the variables y and z. The values of y and z can independently be an integer from 1 to 2. In combination, the values of y and z will result in a technetium coordination sphere that is made up of at least five and no more than seven donor atoms. For monodentate ancillary ligands A_{L2}, z can be an integer from 1 to 2; for bidentate or tridentate ancillary ligands A_{L2}, z is 1. The preferred combination for monodentate ligands is y equal to 1 or 2 and z equal to 1. The preferred combination for bidentate or tridentate ligands is y equal to 1 and z equal to 1.

The indium, copper, gallium, silver, palladium, rhodium, gold, platinum, bismuth, yttrium and lanthanide radiopharmaceuticals of the present invention can be easily prepared by admixing a salt of a radionuclide and a reagent of the present invention, in an aqueous solution at temperatures from 0 to 100 °C. These radionuclides are typically obtained as a dilute aqueous solution in a mineral acid, such as hydrochloric, nitric or sulfuric acid. The radionuclides are combined with from one to about one thousand equivalents of the reagents of the present invention dissolved in aqueous solution. A buffer is typically used to maintain the pH of the reaction mixture between 3 and 10.

The gadolinium, dysprosium, iron and manganese metallopharmaceuticals of the present invention can be easily prepared by admixing a salt of the paramagnetic metal ion and a reagent of the present invention, in an aqueous solution at temperatures from 0 to 100 °C. These paramagnetic metal ions are typically obtained as a dilute aqueous solution in a mineral acid, such as hydrochloric, nitric or sulfuric acid. The paramagnetic metal ions are combined with from one to about one thousand equivalents of the reagents of the present invention dissolved in aqueous solution. A buffer is typically used to maintain the pH of the reaction mixture between 3 and 10.

The total time of preparation will vary depending on the identity of the metal ion, the identities and amounts of the reactants and the procedure used for the preparation. The preparations may be complete, resulting in > 80% yield of the radiopharmaceutical, in 1 minute or may require more time. If higher purity metallopharmaceuticals are needed or desired, the products can be purified by any of a number of techniques well known to those skilled in the art such as liquid chromatography, solid phase extraction, solvent extraction, dialysis or ultrafiltration.

Buffers useful in the preparation of metallopharmaceuticals and in diagnostic kits useful for the preparation of said radiopharmaceuticals include but are not limited to phosphate, citrate, sulfosalicylate, and acetate. A more complete list can be found in the United States Pharmacopeia.

Lyophilization aids useful in the preparation of diagnostic kits useful for the preparation of radiopharmaceuticals include but are not limited to mannitol, lactose, sorbitol, dextran, Ficoll, and polyvinylpyrrolidine(PVP).

Stabilization aids useful in the preparation of metallopharmaceuticals and in diagnostic kits useful for the preparation of radiopharmaceuticals include but are not limited to ascorbic acid, cysteine, monothioglycerol, sodium bisulfite, sodium metabisulfite, gentisic acid, and inositol.

Solubilization aids useful in the preparation of metallopharmaceuticals and in diagnostic kits useful for the preparation of radiopharmaceuticals include but are not limited to ethanol, glycerin, polyethylene glycol, propylene glycol, polyoxyethylene sorbitan monooleate, sorbitan monoloeate, polysorbates, poly(oxyethylene)poly(oxypropylene)poly(oxyethylene) block copolymers (Pluronics) and lecithin. Preferred solubilizing aids are polyethylene glycol, and Pluronics.

Bacteriostats useful in the preparation of metallopharmaceuticals and in diagnostic kits useful for the preparation of radiopharmaceuticals include but are not limited to benzyl alcohol, benzalkonium chloride, chlorbutanol, and methyl, propyl or butyl paraben.

A component in a diagnostic kit can also serve more than one function. A reducing agent can also serve as a stabilization aid, a buffer can also serve as a transfer ligand, a lyophilization aid can also serve as a transfer, ancillary or co-ligand and so forth.

The diagnostic radiopharmaceuticals are administered by intravenous injection, usually in saline solution, at a dose of 1 to 100 mCi per 70 kg body weight, or preferably at a dose of 5 to 50 mCi. Imaging is performed using known procedures.

The therapeutic radiopharmaceuticals are administered by intravenous injection, usually in saline solution, at a dose of 0.1 to 100 mCi per 70 kg body weight, or preferably at a dose of 0.5 to 5 mCi per 70 kg body weight.

The magnetic resonance imaging contrast agents of the present invention may be used in a similar manner as other MRI agents as described in U.S. Patent 5,155,215; U.S. Patent 5,087,440; Margerstadt et al., Magn. Reson. Med., 1986, 3, 808; Runge et al., Radiology, 1988, 166, 835; and Bousquet et al., Radiology, 1988, 166, 693. Generally, sterile aqueous solutions of the contrast agents are administered to a patient intravenously in dosages ranging from 0.01 to 1.0 mmoles per kg body weight.

For use as X-ray contrast agents, the compositions of the present invention should generally have a heavy atom concentration of 1 mM to 5 M, preferably 0.1 M to 2 M. Dosages, administered by intravenous injection, will typically range from 0.5 mmol/kg to 1.5 mmol/kg, preferably 0.8 mmol/kg to 1.2 mmol/kg. Imaging is performed using known techniques, preferably X-ray computed tomoaraphy.

The ultrasound contrast agents of the present invention are administered by intravenous injection in an amount of 10 to 30 µL of the echogenic gas per kg body weight or by infusion at a rate of approximately 3 µL/kg/min. Imaging is performed using known techniques of sonography.

Other features of the invention will become apparent in the course of the following descriptions of exemplary embodiments which are given for illustration of the invention and are not intended to be limiting thereof.

### EXAMPLES

Representative materials and methods that may be used in preparing the compounds of the invention are described further below. 1-methyl-4-oxo-7-(((1-(triphenylmethyl)imidazol-2-yl)amino)methyl)hydroquinoline-3-carboxylic acid, ethyl 7-bromo-4-oxohydroquinoline-3-carboxylate, 1-(triphenylmethyl)imidazole-2-ylamine, and methyl 3-amino-2-(((2,4,6-trimethylphenyl)sulfonyl)amino)propanoate hydrochloride were prepared as described in PCT WO 98/23608. Boc-L-cysteic acid, Boc-L-cysteic acid N-hydroxyphenyl ester, and Boc-L-cysteic acid p-nitrophenyl ester were prepared as described in Liebigs Ann. Chem. 1979, 776-783. Benzotriazole-1-yloxy-tris-pyrrolidinophosphonium hexafluorophosphate (PyBOP) was purchased from Novabiochem.
(tert-butoxy)-N-(3-bromopropyl)formamide and 2-(2-aza-2-((5-((2,5-dioxopyrrolidinyl)carbonyl)(2-pyridyl))-amino)vinyl)benzenesulfonic acid were prepared as described in PCT WO 96/40637. All other chemicals and solvents (reagent grade) were used as supplied from the vendor cited without further purification. t-Butyloxycarbonyl (Boc) amino acids and other starting amino acids may be obtained commercially from Bachem Inc., Bachem Biosciences Inc. (Philadelphia, PA), Advanced ChemTech (Louisville, KY), Peninsula Laboratories (Belmont, CA), or Sigma (St. Louis, MO). 2-(1H-Benzotriazol-1-yl)-1,1,3,3-tetramethyluronium hexafluorophosphate (HBTU) and TBTU were purchased from Advanced ChemTech. N-methylmorpholine (NMM), m-cresol, D-2-aminobutyric acid (Abu), trimethylacetylchloride, diisopropylethylamine (DIEA), 1,2,4-triazole, stannous chloride dihydrate, 1-(3-dimethylaminopropyl)-3-ethylcarbodiimide hydrochloride (EDC), triethylsilane (Et₃SiH) and tris(3-sulfonatophenyl)phosphine trisodium salt (TPPTS) were purchased from Aldrich Chemical Company. Bis(3-sulfonatophenyl)phenylphosphine disodium salt (TPPDS) was prepared by the published procedure (Kuntz, E., U.S. Patent 4,248,802). (3-Sulfonatophenyl)diphenylphosphine monosodium salt (TPPMS)was purchased from TCI America, Inc. Tricine was obtained from Research Organics, Inc. Technetium-99m-pertechnetate (^{99m}TcO₄⁻) was obtained from a DuPont Pharma ⁹⁹Mo/^{99m}Tc Technelite® generator. In-111-chloride (Indichlor®) was obtained from Amersham Medi-Physics, Inc. Sm-153-chloride and Lutetium-177-chloride were obtained from the University of Missouri Research Reactor (MURR). Yttrium-90 chloride was obtained from the Pacific Northwest Research Laboratories. Dimethylformamide (DMF), ethyl acetate, chloroform (CHCl₃), methanol (MeOH), pyridine and hydrochloric acid (HCl) were obtained from Baker. Acetonitrile, dichloromethane (DCM), acetic acid (HOAc), trifluoroacetic acid (TFA) ethyl ether, triethylamine, acetone, and magnesium sulfate were commercially obtained. Absolute ethanol was obtained from Quantum Chemical Corporation.

### Synthesis of Boc-Glu-(OTFP)-OTFP

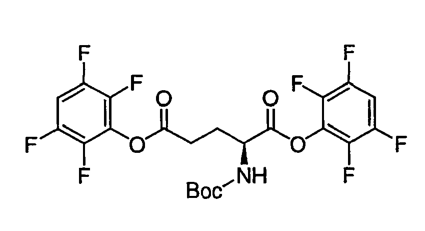

To a solution of Boc-Glu-OH (28.9 g, 117 mmol) in DMF (500 mL) at room temperature, and under nitrogen, was added a solution of 2,3,5,6-tetrafluorophenol (48.2 g, 290 mmol) in DMF (50 mL). After stirring for 10 min. EDC (55.6 g, 290 mmol) was added and the reaction mixture was stirred for about 96 h. The volatiles were removed in vacuo and the residue was triturated in 0.1 N HCl (750 mL). To this mixture was added ethyl acetate (600 mL), the layers separated. The aqueous layer was extracted with ethyl acetate (3 x ~500 mL), and all the ethyl acetate fractions were combined, washed with water (300 mL) and brine (300 mL), dried (MgSO₄), and concentrated to give a tan solid (62 g). The tan solid was washed with acetonitrile to give the title compound (45.5 g, 73%) in purified form.
ESMS: Calculated for C₂₂H₁₇F₈NO₆, 543.09; found, 566.0 [M+Na]⁺¹.

### Example 1

### 2-(((4-(4-(((3-(2-(2-(3-((6-((1-Aza-2-(2-sulfophenyl)vinyl)amino)(3-pyridyl))carbonylamino)-propoxy)ethoxy)-ethoxy)propyl)amino)sulfonyl)phenyl)phenyl)sulfonyl)-amino)-3-((7-((imidazol-2-ylamino)methyl)-1-methyl-4-oxo(3-hydroquinolyl))carbonylamino)propanoic Acid Trifluoroacetate Salt

### Part A - N-(3-(2-(2-(3-aminopropoxy)ethoxy)ethoxy)-propyl)(phenylmethoxy)formamide

A solution of 4,7,10-trioxa-1,13-tridecanediamine (158 mL, 0.72 mol), TEA (16.7 mL, 0.12 mol), and MeOH (300 mL) in peroxide-free THF (1,000 mL) was placed in a 3 liter 3-neck flask fitted with a mechanical stirrer, a thermometer, and an addition funnel with nitrogen line. The addition funnel was charged with a solution of benzyl chloroformate (17.1 mL, 0.12 mol) in peroxide-free THF (1,000 mL). The contents of the flask were cooled below 5 °C. The contents of the addition funnel were added to the flask with rapid stirring over 4 h while keeping the temperature below 5 °C. The solution was stirred an additional 30 min and concentrated to give a thick syrup. This syrup was taken up in saturated NaCl (1800 mL) and 10% Na₂CO₃ (200 mL) and extracted with ether (3 x 1,000 mL). The combined ether extracts were washed with saturated NaCl (500 mL), dried (MgSO₄), and concentrated to give a pale yellow oil (36.74 g). Flash chromatography on a 7 x 29 cm silica gel column (DCM/MeOH/TEA, 20/15/0.5) gave the title compound as a colorless syrup (19.14 g, 45%). ¹H NMR (CDCl₃): 7.33-7.25 (m, 5H), 5.59 (s, 1H), 5.06 (s, 2H), 3.62-3.45 (m, 12H), 3.32-3.25 (m, 2H), 2.74 (t, J = 6.7 Hz, 2H), 1.75 (pentet, J = 6.0 Hz, 2H), 1.67 (pentet, J = 6.4 Hz, 2H), 1.33 (s, 2H); MS: m/e 355.4 [M+H]; High Resolution MS: Calcd for C₁₈H₃₁N₂O₅ [M+H]: 355.2233, Found: 355.2222.

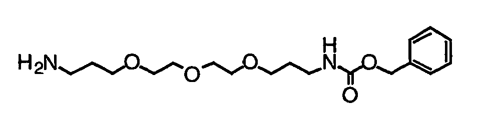

### Part B - Methyl 3-((tert-Butoxy)carbonylamino)-2-(((4-(4-(((3-(2-(2-(3-((phenylmethoxy)carbonylamino)propoxy)ethoxy)-ethoxy)propyl)amino)sulfonyl)phenyl)phenyl)sulfonyl)-amino)propanoate

Biphenyl-4,4'-disulfonyl chloride (2.64 g, 7.5 mmol, freshly recrystallized from CHCl₃) and DCM (200 mL) were placed in a 500 mL 3-neck flask fitted with a thermometer, an addition funnel, and a nitrogen line. The addition funnel was charged with a solution of N-(3-(2-(2-(3-aminopropoxy)ethoxy)ethoxy)propyl)-(phenylmethoxy)formamide (1.77 g, 5.0 mmol) and DIEA (0.87 mL, 5.0 mmol) in DCM (40 mL). The contents of the flask were cooled below 5 °C. The contents of the addition funnel were added to the flask with rapid stirring over 3 h while keeping the temperature of the flask below 5 °C. The addition funnel was charged with a solution of N-β-Boc-L-α,β,-diaminopropionic acid methyl ester hydrochloride (2.55 g, 10 mmol) and DIEA (3.8 mL, 22 mmol) in DCM (25 mL). This solution was added to the flask with stirring at 5 °C over 15 min, and stirred at ambient temperatures for an additional 20 h. The reaction solution was washed consecutively with 0.1 N HCl (100 mL) and water (2 x 100 mL), dried (MgSO₄), and concentrated to give a viscous oil (5.79 g). Flash chromatography on a 5 x 21 cm silica gel column (85/15 EtOAc/hexanes, followed by 100% EtOAc) gave a colorless amorphous solid. Recrystallization from toluene (85 mL) gave the title compound as a colorless solid (2.52 g, 59%). MP: 104.5-106.5 °C; ¹H NMR (CDCl₃): 8.00-7.90 (m, 4H), 7.72-7.64 (m, 4H), 7.46-7.24 (m, 5H), 5.96-5.88 (m, 1H), 5.86-5.73 (m, 1H), 5.41 (s, 1H), 5.16-5.00 (m, 3H), 4.15-4.02 (m, 1H), 3.68-3.39 (m, 17H), 3.34-3.22 (m, 2H), 3.13-3.03 (m, 2H), 1.80-1.62 (m, 4H), 1.39 (s, 9H); ¹³C NMR (CDCl₃): 170.2, 156.5, 156.1, 143.9, 143.0, 140.4, 139.4, 136.7, 128.4, 128.1, 128.0, 127.9, 127.9, 127.8, 127.3, 80.1, 70.6, 70.5, 70.2, 70.1, 70.0, 69.6, 66.5, 56.1, 52.9, 43.2, 42.4, 39.3, 29.4, 28.5, 28.2; MS: m/e 868.3 [M+NH₄]; High Resolution MS: Calcd for C₃₉H₅₅N₄O₁₃S_{2 [}M+H]: 851.3207, Found: 851.3226.

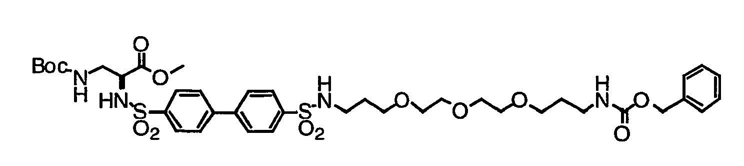

### Part C - Methyl 3-((1-Methyl-4-oxo-7-(((1-(triphenylmethyl) imidazol-2-yl)amino)methyl) (3-hydroquinolyl))carbonylamino)-2-(((4-(4-(((3-(2-(2-(3-((phenylmethoxy)carbonylamino)-propoxy)ethoxy)ethoxy)propyl)amino)sulfonyl)phenyl)pheny 1) sulfonyl)amino)propanoate

The product of Part B, above (748 mg, 0.88 mmol) was dissolved in 25/75 TFA/DCM (15 mL) and allowed to stand at ambient temperatures under nitrogen for 15 min. The TFA was removed under vacuum and the resulting amber oil was taken up in 50/50 ACN/water (50 mL), and treated portion wise with Bio-Rad AG-3-X4A resin, hydroxide form, to raise the pH from 2 to 6. The resin was removed by filtration and the filtrate was lyophilized to give a sticky pale yellow foam.

In a separate flask, 1-methyl-4-oxo-7-(((1-(triphenylmethyl)imidazol-2-yl)amino)methyl)hydroquinoline-3-carboxylic acid (432 mg, 0.80 mmol), TEA (0.33 mL), and HBTU (364 mg, 0.96 mmol) were dissolved in anhydrous DMF (25 mL). The resulting solution was stirred at ambient temperatures under a nitrogen atmosphere for 10 min and combined with a solution of the yellow foam in anhydrous DMF (15 mL). The DMF was removed under vacuum after 18 h to give a viscous yellow oil. This oil was taken up in EtOAc (175 mL), washed consecutively with water (25 mL), saturated NaHCO₃ (50 mL), and saturated NaCl (25 mL), dried (MgSO₄), and concentrated to give a viscous yellow oil. Purification by flash chromatography on a 7 x 25 cm silica gel column using a CHCl₃/EtOAc/MeOH step gradient (47/47/6, 46/46/8, 60/30/10) gave the title compound as a pale yellow solid (510 mg, 50%). MP: 136-140 °C; MS: m/e 1273.4 [M+H]; High Resolution MS: Calcd for C₆₈H₇₃N₈O₁₃S₂ [M+H]: 1273.4738, Found: 1273.4730.

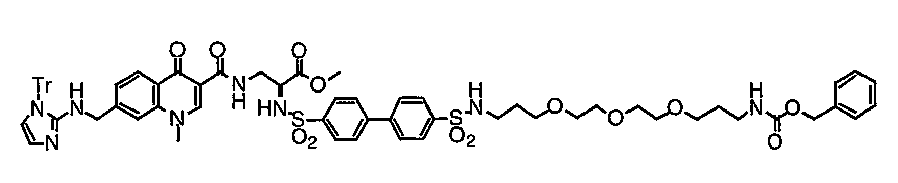

### Part D - 3-((1-Methyl-4-oxo-7-(((1-(triphenylmethyl)-imidazol-2-yl)amino)methyl) (3-hydroquinolyl))carbonylamino)-2-(((4-(4-(((3-(2-(2-(3-((phenylmethoxy)carbonylamino)propoxy)ethoxy)-ethoxy)propyl)amino)sulfonyl)phenyl)phenyl)sulfonyl)-amino)propanoic Acid

The product form Part C, above (295 mg, 0.232 mmol) was dissolved in a mixture of peroxide-free THF (12 mL), water (1.8 mL), and 3 N LiOH (1.2 mL), and stirred at ambient temperatures under a nitrogen atmosphere for 30 min. The THF was removed under vacuum and the resulting mixture was dissolved in CHCl₃ (75 mL) and water (50 mL). The aqueous layer was adjusted to pH 3 with 0.5 N HCl and the layers were thoroughly mixed. The aqueous layer was extracted with additional CHCl₃ (2 x 25 mL). The combined CHCl₃ extracts were washed with saturated NaCl (50 mL), dried (MgSO₄), and concentrated to give the title compound as a pale yellow solid (291 mg, 100%). MS: m/e 1259.3 [M+H]; High Resolution MS: Calcd for C₆₇H₇₁N₈O₁₃S₂ [M+H]: 1259.4582, Found: 1259.4610.

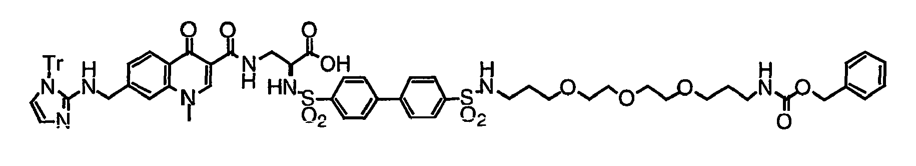

### Part E - 2-(((4-(4-(((3-(2-(2-(3-Aminopropoxy)ethoxy)-ethoxy)propyl)amino)sulfonyl)phenyl)phenyl)sulfonyl)-amino)-3-((7-((imidazol-2-yl)methyl)-1-methyl-4-oxo(3-hydroquinolyl))carbonylamino)propanoic Acid

The product from Part D, above (279 mg, 0.222 mmol) was dissolved in degassed TFA (30 mL) and treated with Et₃SiH (0.424 mL, 2.66 mmol). The solution was heated at 70°C under a nitrogen atmosphere for 1 h and concentrated to a viscous oil. This oil was dissolved in water (20 mL) and washed with ether (2 x 20 mL). The combined ether washings were back-extracted with water (10 mL). The combined water extracts were diluted with an equal volume of ACN and treated with Bio-Rad AG-3-X4A resin, hydroxide form to raise the pH from 4 to 6. The resin was removed by filtration and the filtrate was lyophilized to give the title compound as a colorless solid (220 mg). MS: m/e 883.4 [M+H], 442.5 [M+2H]; High Resolution MS: Calcd for C₄₀H₅₁N₈O₁₁S₂ [M+H]: 833.3118, Found: 833.3118.

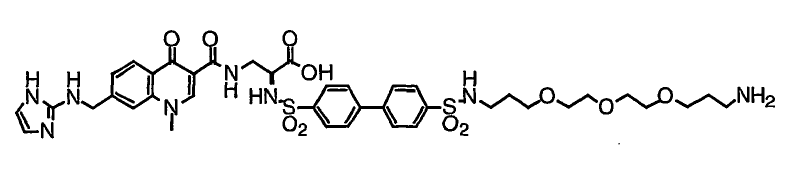

### Part F - 2-(((4-(4-(((3-(2-(2-(3-((6-((1-Aza-2-(2-sulfophenyl)vinyl)amino)(3-pyridyl))carbonylamino)-propoxy)ethoxy)-ethoxy)propyl)amino)sulfonyl)phenyl)phenyl)sulfonyl)-amino)-3-((7-((imidazol-2-ylamino)methyl)-1-methyl-4-oxo(3-hydroquinolyl))carbonylamino)propanoic Acid Trifluoroacetate Salt

A solution of the product from Part F, above (15 mg, 0.0135 mmol), TEA (0.007 mL), and 2-(2-aza-2-((5-((2,5-dioxopyrrolidinyl)carbonyl)(2-pyridyl))-amino)vinyl)benzenesulfonic acid (9.0 mg, 0.0204 mmol) in anhydrous DMF (2.5 mL) was allowed to stand at ambient temperatures under a nitrogen atmosphere for 22 h. The DMF was removed under vacuum and the glassy solid was dissolved in 20% ACN and purified by preparative HPLC on a Vydac C-18 column (22 x 250 mm) using 0.1% TFA in water for 5 min followed by a 2.52%/min gradient of 0 to 63% ACN containing 0.1% TFA at a flow rate of 20 mL/min. The main product peak eluting at 21.2 min was collected and lyophilized to give the title compound as a colorless powder (3.5 mg, 20%). MS: m/e 1186.7 [M+H]; High Resolution MS: Calcd for C₅₃H₆₀N₁₁O₁₅S₃ [M+H]: 1186.3432, Found: 1186.3410.

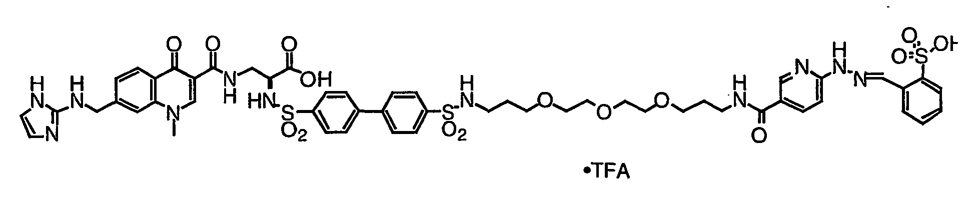

### Example 2

### 3-((7-((Imidazol-2-ylamino)methyl)-1-methyl-4-oxo(3-hydroquinolyl))carbonylamino)-2-(((4-(4-(((3-(2-(2-(3-(2-(1,4,7,10-tetraaza-4,7,10-tris(carboxylmethyl)cyclododecyl)acetylamino)-propoxy)ethoxy)ethoxy)propyl)amino)sulfonyl)-phenyl)phenyl)sulfonyl)amino)propanoic Acid Bis(trifluoroacetate) Salt

### Part A - Phenylmethyl 2-(1,4,7,10-Tetraaza-4,7,10-tris(((tert-butyl)oxycarbonyl)methyl)cyclododecyl)-acetate

A solution of tert-butyl (1,4,7,10-tetraaza-4,7-bis(((tert-butyl)oxycarbonyl)methyl)cyclododecyl)acetate (0.922 g, 1.79 mmol), TEA (1.8 mL) and benzyl bromoacetate (0.86 mL, 5.37 mmol) in anhydrous DMF (24 mL) was stirred at ambient temperatures under a nitrogen atmosphere for 24 h. The DMF was removed under vacuum and the resulting oil was dissolved in EtOAc (300 mL). This solution was washed consecutively with water (2 x 50 mL) and saturated NaCl (50 mL), dried (MgSO₄), and concentrated to give the title compound as an amorphous solid (1.26 g). MS: m/e 663.5 [M+H].

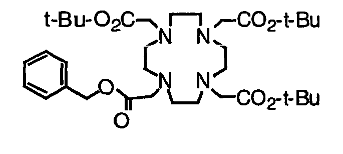

### Part B - 2-(1,4,7,10-tetraaza-4,7,10-tris(((tert-butyl)oxycarbonyl)methyl)cyclododecyl)acetic acid

The product from Part A, above (165 mg, 0.25 mmol) was hydrogenolyzed over 10% Pd on carbon (50 mg) in EtOH (15 mL) at 60 psi for 24 h. The catalyst was removed by filtration through filter aid and washed with EtOH. The filtrates were concentrated to give the title compound as an amorphous solid (134 mg, 94%). MS: m/e 573.5 [M+H].

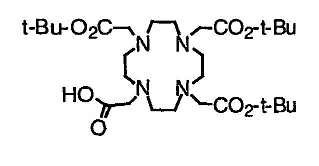

### Part C - Methyl 3-((7-((Imidazol-2-ylamino)methyl)-1-methyl-4-oxo(3-hydroquinolyl))carbonylamino)-2-(((4-(4-(((3-(2-(2-(3-(2-(1,4,7,10-tetraaza-4,7,10-tris(((tert-butyl)oxycarbonyl)methyl)cyclododecyl)acetylamino)-propoxy)ethoxy)-ethoxy)propyl)amino)sulfonyl)phenyl)phenyl)sulfonyl)-amino)propanoate Pentakis(trifluoroacetate) Salt

A solution of the product of Example 1, Part C (68 mg, 0.0534 mmol) and Et₃SiH (0.051 mL, 0.32 mmol) in degassed TFA (5.0 mL) was stirred at 70°C under a nitrogen atmosphere for 1 h and concentrated to dryness. The resulting amber oil was dissolved in anhydrous DMF (2 mL) and treated with TEA until basic to pH paper. A solution of the product of Part B, above (46 mg, 0.080 mmol) in anhydrous DMF (1.0 mL) was added, followed by HBTU (24 mg, 0.064 mmol), and the solution was stirred at ambient temperatures under a nitrogen atmosphere for 3 h. The DMF was removed under vacuum and the residue was dissolved in 50% ACN and purified by preparative HPLC on a Vydac C-18 column (22 x 250 mm) using a 2.1%/min gradient of 0 to 63% ACN containing 0.1% TFA at a flow rate of 20 mL/min. The main product peak eluting at 23.8 min was collected and lyophilized to give the title compound as a colorless powder (16 mg, 15%). MS: m/e 1451.7 [M+H]; High Resolution MS: Calcd for C₆₉H₁₀₃N₁₂O₁₈S₂ [M+H]: 1451.6954, Found: 1451.698.

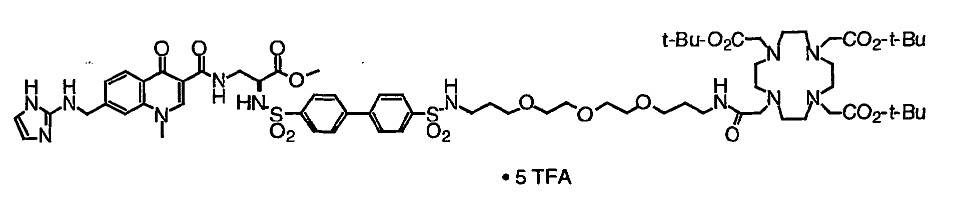

### Part D - 3-((7-((Imidazol-2-ylamino)methyl)-1-methyl-4-oxo(3-hydroquinolyl))carbonylamino)-2-(((4-(4-(((3-(2-(2-(3-(2-(1,4,7,10-tetraaza-4,7,10-tris(carboxylmethyl)cyclododecyl)acetylamino)-propoxy)ethoxy)ethoxy)propyl)amino)sulfonyl)-phenyl)phenyl)sulfonyl)amino)propanoic Acid Bis(trifluoroacetate) Salt

The product of Part C, above (16 mg, 0.0102 mmol) was dissolved in a mixture of peroxide-free THF (1 mL), water (0.115 mL), and 3 N LiOH (0.075 mL), and stirred at ambient temperatures under a nitrogen atmosphere for 24 h. The reaction was concentrated to give an oily solid. This solid was dissolved in 50% ACN and purified by preparative HPLC on a Vydac C-18 column (22 x 250 mm) using a 2.52%/min gradient of 0 to 63% ACN containing 0.1% TFA at a flow rate of 20 mL/min. The main product peak eluting at 24.0 min was collected and lyophilized to give a colorless powder (6.0 mg). This solid was dissolved in degassed TFA (2.0 mL) and Et₃SiH (0.050 mL), stirred at 70 °C under a nitrogen atmosphere for 4.5 h, and concentrated to dryness. The resulting oil was dissolved in 25% ACN and purified by preparative HPLC on a Vydac C-18 column (22 x 250 mm) using a 1.5%/min gradient of 0 to 45% ACN containing 0.1% TFA at a flow rate of 20 mL/min. The main product peak eluting at 19.0 min was collected and lyophilized to give the title compound as a colorless powder (2.0 mg, 17%). MS: m/e 1269.5 [M+H], 635.5 [M+2H], 424.3 [M+3H]; High Resolution MS: Calcd for C₅₆H₇₇N₁₂O₁₈S₂ [M+H]: 1269.4920, Found: 1269.4950.

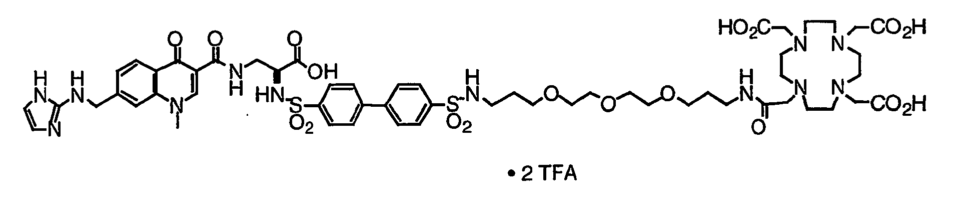

### Example 3

### 2-(((4-(3-(N-(3-(2-(2-(3-((6-((1-Aza-2-(2-sulfophenyl)vinyl)amino)(3-pyridyl))carbonylamino)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid Trifluoroacetate Salt

### Part A - Ethyl 4-(3,5-Dimethylphenoxy)butanoate

Sodium metal (17.12 g, 0.744 mol) was added to anhydrous EtOH (350 mL) and stirred until dissolved. 3,5-Dimethylphenol was added and the solution was stirred 15 min at ambient temperature. Ethyl 4-bromoacetate (58.7 mL, 0.41 mol) was added and the solution was stirred at ambient temperatures under a nitrogen atmosphere for 28 h. The EtOH was removed under vacuum and the oily solid was partitioned between water (1 L) and EtOAc (500 mL). The aqueous layer was extracted with additional EtOAc (500 mL). The combined EtOAc extracts were washed consecutively with saturated NaHCO₃ (300 mL) and saturated NaCl (300 mL), dried (MgSO₄), and concentrated to give an amber liquid. This liquid was vacuum fractional distilled through a 15 cm Vigreux column. The main fraction was collected from 91-117 °C/6 mm Hg to gave the title compound as a colorless liquid (77.77 g, 89%). ¹H NMR (CDCl₃): 6.59 (s, 1H), 6.52 (s, 2H), 4.16 (q, J - 7.16 Hz, 2H), 3.98 (t, J = 6.14 Hz, 2H), 2.49 (t, J = 7.34 Hz, 2H), 2.28 (s, 6H), 2.11-2.07 (m, 2H), 1.26 (t, J = 7.16 Hz, 3H); Anal. calcd for C₁₄H₂₀O₃: C, 71.16; H, 8.53, Found: C, 71.35; H, 8.59.

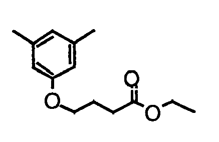

### Part B - 4-(3,5-Dimethylphenoxy)butanoic Acid

The product of part A, above (75.52 g, 0.320 mol) and KOH pellets (38.5 g, 0.584 mol) were dissolved in absolute EtOH (1.50 L) and heated at reflux for 3 h. The solution was concentrated to a colorless solid, which was taken up in water (2.0 L) and washed with ether (2 x 750 mL). The aqueous layer was adjusted to pH 1 with concd HCl (55 mL) and the resulting oily port was extracted into EtOAc (2 x 500 mL). The combined EtOAc extracts were washed consecutively with water (300 mL) and saturated NaCl, dried (MgSO₄), and concentrated to give a colorless solid (64.13 g). Recrystallization from hexanes (500 mL) gave the title compound as a colorless solid (59.51 g, 89%). MP: 66-68.5 °C; ¹H NMR (CDCl₃) : 11.70 (bs, 1H), 6.59 (s, 1H), 6.52 (s, 2H), 3.99 (t, J = 6.06 Hz, 2H), 2.57 (t, J = 7.29 Hz, 2H), 2.28 (s, 6H), 2.12-2.08 (m, 2H); Anal. calcd for C₁₂H₁₆O_{3:} C, 69.21; H, 7.74, Found: C, 69.23; H, 7.40.

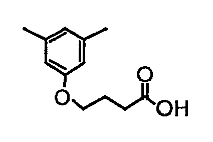

### Part C - 4-(4-(Chlorosulfonyl)-3,5-dimethylphenoxy)butanoic Acid

A solution of the product of Part B, above (20.8 g, 0.100 mol) in CHCl₃ (100 mL) was cooled to 0 °C and treated with chlorosulfonic acid (36 mL, 0.54 mol) dropwise and with rapid stirring while keeping the temperature of the reaction at 0 °C. The resulting gelatinous mixture was stirred an additional 10 min and poured onto an ice/water mixture (600 mL). The resulting solid ppt was collected by filtration, washed with water (3 x 75 mL), and dried under vacuum to give a colorless solid (12.52 g). MP: 114-115 °C (with decomp); ¹H NMR (CDCl₃): 13.84 (bs, 1H), 6.50 (s, 2H), 3.91 (t, J = 6.48 Hz, 2H), 2.48 (s, 6H), 2.32 (t, J = 7.32 Hz, 2H), 1.89-1.84 (m, 2H); IR (KBr cm⁻¹): 1705 (s), 1370 (s), 1175 (s); MS: m/e 305.1 [M-H].

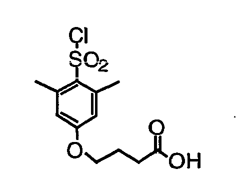

### Part D - 4-(4-(((2-((tert-Butoxy)carbonylamino)-1-(methoxycarbonyl)ethyl)amino)sulfonyl)-3,5-dimethylphenoxy)butanoic Acid

A solution of N-β-Boc-L-α,β,-diaminopropionic acid methyl ester hydrochloride (568 mg, 2.10 mmol) and DIEA (0.73 mL, 4.2 mmol) in DCM (5 mL) was cooled to 0 °C and treated with a suspension of the product of Part C, above (656 mg, 2.10 mmol) in DCM (20 mL) in small portions over a 15 min period. The reaction was stirred at ambient temperatures under a nitrogen atmosphere for 18 h. The reaction was diluted with DCM (100 mL) and washed with water (3 x 75 mL). The organic phase was dried (MgSO₄), and concentrated to give crude product (698 mg), which was purified by preparative HPLC on a Vydac C-18 column (50 x 250 mm) using a 0.96%/min gradient of 18 to 58.5% ACN containing 0.1% TFA at a flow rate of 80 mL/min. The main product fraction eluting at 23.8 min was collected adjusted to pH 3, partially concentrated to remove ACN, and extracted with DCM (2 x 100 mL). The DCM extracts were dried (MgSO₄) and concentrated to give the title compound as a colorless solid (297 mg, 29%). ¹H NMR (CDCl₃): δ 6.61 (s, 2H), 5.66 (d, J = 7.2 Hz, 1H), 4.90 (s, 1H), 4.03 (bs, 2H), 3.86 (bs, 1H), 3.59 (s, 3H), 3.49 (bs, 2H), 2.62 (s, 6H), 2.58-2.51 (m, 2H), 2.18-2.07 (m, 2H), 1.41 (s, 9H); MS: m/e 489.4 [M+H]; High Resolution MS: Calcd for C₂₁H₃₃N₂O₉S [M+Na]: 511.1726, Found: 511.1747; Anal. calcd for C₂₁H₃₂N₂O₉S: C., 51.62; H, 6.61; N, 5.74, Found: C, 51.47; H, 6.27; N, 5.48.

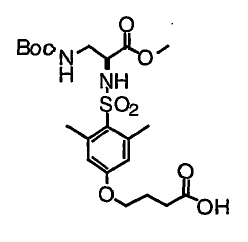

### Part E - Methyl 3-((tert-Butoxy)carbonylamino)-2-(((2,6-dimethyl-4-(3-(N-(3-(2-(2-(3-((phenylmethoxy)carbonylamino)propoxy)ethoxy)-ethoxy)propyl)carbamoyl)propoxy)phenyl)-sulfonyl)amino)propanoate

A solution of the product from Part D, above (233 mg, 0.477 mmol), the product of Example 1, Part A (190 mg, 0.536 mmol), TEA (0.2 mL, 1.43 mmol), and HBTU (226 mg, 0.701 mmol) in anhydrous DMF (8 mL) was stirred at ambient temperatures under a nitrogen atmosphere for 1 h. The DMF was removed under vacuum and the oily residue was taken up in EtOAc (50 mL) and washed consecutively with 0.1 N HCl (35 mL), water (35 mL), and saturated NaCl (35 mL), dried (MgSO₄), and concentrated to give crude product as a yellow viscous oil. Flash chromatography on a 3 x 18 cm silica gel column (EtOAc/MeOH, 95/5) gave the title compound as a colorless viscous oil (393 mg, 100%). ¹H NMR (CDCl₃): δ 7.34-7.28 (m, 5H), 6.60 (s, 2H), 6.26 (bs, 1H), 5.67 (bs, 1H), 5.29 (bs, 1H), 5.08 (s, 2H), 4.88 (bs, 1H), 3.99 (t, J = 6.1 Hz, 2H), 3.88-3.84 (m, 1H), 3.62-3.40 (m, 17H), 3.37-3.26 (m, 4H), 2.62 (s, 6H), 2.32 (t, J = 7.2 Hz, 2H), 2.08 (t, J = 6.3 Hz, 2H), 1.79-1.70 (m, 4H), 1.41 (s, 9H); MS: m/e 825.5 [M+H]; High Resolution MS: Calcd for C₃₉H₆₁N₄O₁₃S [M+H]: 825.3955, Found: 825.3940.

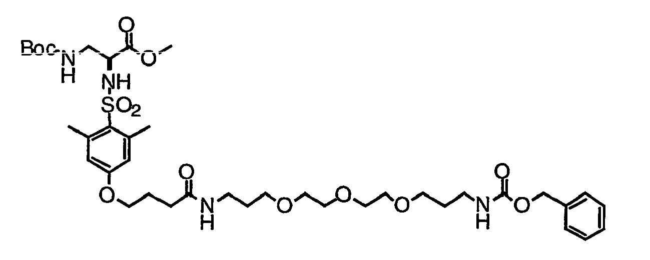

### Part F - Methyl 3-Amino-2-(((2,6-dimethyl-4-(3-(N-(3-(2-(2-(3-((phenylmethoxy)carbonylamino)propoxy)ethoxy)-ethoxy)propyl)carbamoyl)propoxy)phenyl)-sulfonyl)amino)propanoate

The product of Part E, above (750 mg, 0.91 mmol) was dissolved in 4 M HCl/dioxane (25 mL) and stirred at ambient temperatures for 1 h. The solution was diluted with ether (500 mL) and the resulting gummy ppt was triturated with fresh ether (2 x 250 mL). The gummy solid was dissolved in water (100 mL) and adjusted to pH 9 with NaHCO₃, causing an oily ppt to form. This ppt was extracted into DCM (2 x 75 mL). The DCM extracts were dried (MgSO₄) and concentrated to give the title compound as a colorless oil (386 mg, 56%). MS: m/e 725.5 [M+H].

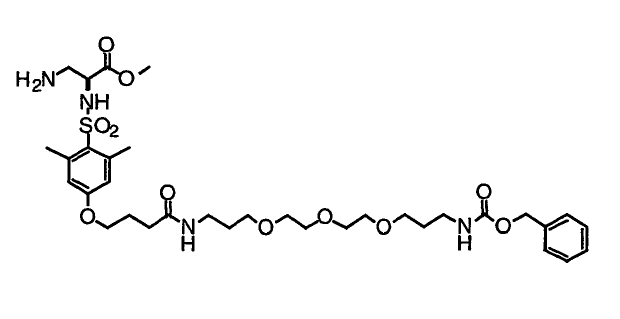

### Part G - Methyl 2-(((2,6-Dimethyl-4-(3-(N-(3-(2-(2-(3-((phenylmethoxy)carbonylamino)propoxy)ethoxy)ethoxy)-propyl)carbamoyl)propoxy)phenyl)sulfonyl)amino)-3-((1-methyl-4-oxo-7-(((1-(triphenylmethyl)imidazol-2-yl)amino)methyl)(3-hydroquinolyl))carbonylamino)propanoate

A solution of 1-methyl-4-oxo-7-(((1-(triphenylmethyl)imidazol-2-yl)amino)methyl)hydroquinoline-3-carboxylic acid (274 mg, 0.51 mmol), TEA (0.22 mL, 1.52 mmol), and HBTU (192 mg, 0.51 mmol) in anhydrous DMF (3 mL) was stirred at ambient temperatures for 5 min. A solution of the product of Part F, above (367 mg, (0.51 mmol) in anhydrous DMF (7 mL) was added and the resulting solution was stirred at ambient temperatures under a nitrogen atmosphere for 2 h. The DMF was removed under vacuum and the resulting oily solid was dissolved in EtOAc (150 mL). The EtOAc solution was washed consecutively with water (50 mL), saturated NaHCO₃ (25 mL), and saturated NaCl (25 mL), dried (MgSO₄), and concentrated to give a yellow solid. Purification by flash chromatography on a silica gel column using a EtOAc/MeOH step gradient (95/5, 92.5/7.5) gave the title compound as a pale yellow solid (254 mg, 43%). MS: m/e 1247.7 [M+H], 624.6 [M+2H].

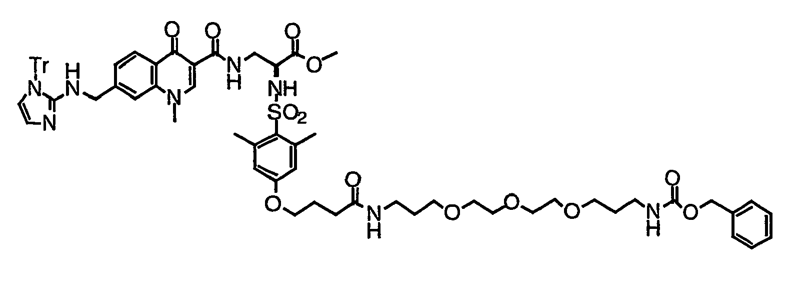

### Part H - 2-(((2,6-Dimethyl-4-(3-(N-(3-(2-(2-(3-((phenylmethoxy)carbonylamino)propoxy)ethoxy)ethoxy)-propyl)carbamoyl)propoxy)phenyl)sulfonyl)amino)-3-((1-methyl-4-oxo-7-(((1-(triphenylmethyl)imidazol-2-yl)amino)methyl)(3-hydroquinolyl))carbonylamino)propanoic Acid

The product of Part G, above (60.0 mg, 0.048 mmol) was dissolved in a mixture of peroxide-free THF (2.5 mL), water (0.37 mL), and 3 N LiOH (0.244 mL), and stirred at ambient temperatures under a nitrogen atmosphere for 30 min. The THF was removed under vacuum and the resulting mixture was dissolved in CHCl₃ (25 mL) and water (20 mL). The aqueous layer was adjusted to pH 3 with 0.1 N HCl and the layers were thoroughly mixed. The aqueous layer was extracted with additional CHCl₃ (2 x 20 mL). The combined CHCl₃ extracts were washed with saturated NaCl (30 mL), dried (MgSO₄), and concentrated to give the title compound as a pale yellow solid (44.0 mg, 74%). MS: m/e 1233.7 [M+H]; High Resolution MS: Calcd for C₆₇H₇₇N₈O₁₃S [M+H]: 1233.5330, Found: 1233.5330.

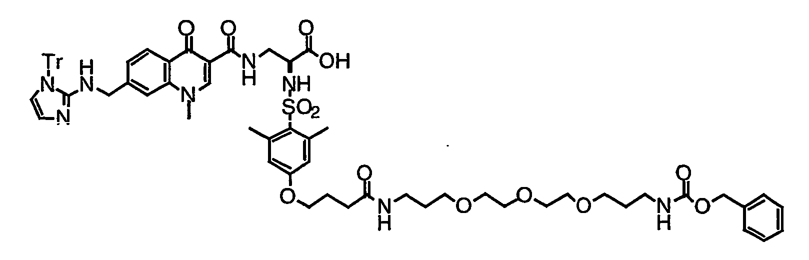

### Part I - 2-(((4-(3-(N-(3-(2-(2-(3-Aminopropoxy)ethoxy)-ethoxy)propyl)carbamoyl)propoxy)-2,6-dimethylphenyl)-sulfonyl)amino)-3-((7-((imidazol-2-ylamino)methyl)-1-methyl-4-oxo(3-hydroquinolyl))carbonylamino)propanoic Acid

The product of Part H, above (42.1 mg, 0.0341 mmol) and Et₃SiH (0.033 mL, 0.205 mmol) were dissolved in degassed TFA (3.5 mL), heated at 70 °C under a nitrogen atmosphere for 1 h, and concentrated to give a viscous amber oil. This oil was dissolved in water (20 mL) and washed with ether (2 x 20 mL). The combined ether washings were back-extracted with water (10 mL). The combined water extracts were diluted with an equal volume of ACN and treated with Bio-Rad AG-3-X4A resin, hydroxide form to raise the pH from 4 to 6. The resin was removed by filtration and the filtrate was lyophilized to give the title compound as a colorless solid (34 mg). MS: m/e 857.5 [M+H], 429.4 [M+2H].

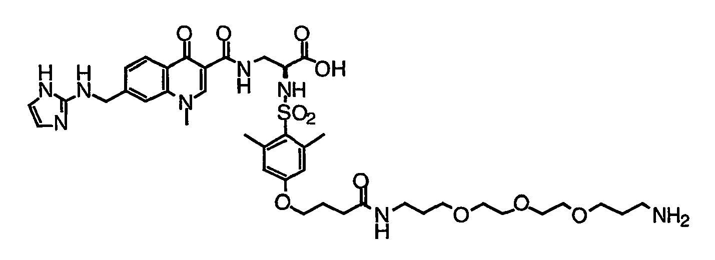

### Part J - 2-(((4-(3-(N-(3-(2-(2-(3-((6-((1-Aza-2-(2-sulfophenyl)vinyl)amino)(3-pyridyl))carbonylamino)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid Trifluoroacetate Salt

A solution of the product from Part I, above (30 mg, 0.035 mmol), DIEA (0.018 mL, 0.105 mmol) and 2-(2-aza-2-((5-((2,5-dioxopyrrolidinyl)carbonyl)(2-pyridyl))-amino)vinyl)benzenesulfonic acid (18.5 mg, 0.042 mmol) in anhydrous DMF (1.5 mL) was allowed to stand at ambient temperatures under a nitrogen atmosphere for 20 h. The DMF was removed under vacuum and the amber oil was dissolved in 50% ACN and purified by preparative HPLC on a Zorbax C-18 RX column (21.2 x 250 mm) using a 1.5%/min gradient of 0 to 45% ACN containing 0.1% TFA at a flow rate of 20 mL/min. The main product peak eluting at 21.0 min was collected and lyophilized to give the title compound as a colorless powder (8.9 mg, 20%). MS: m/e 1160.6 [M+H], 581.0 [M+2H].

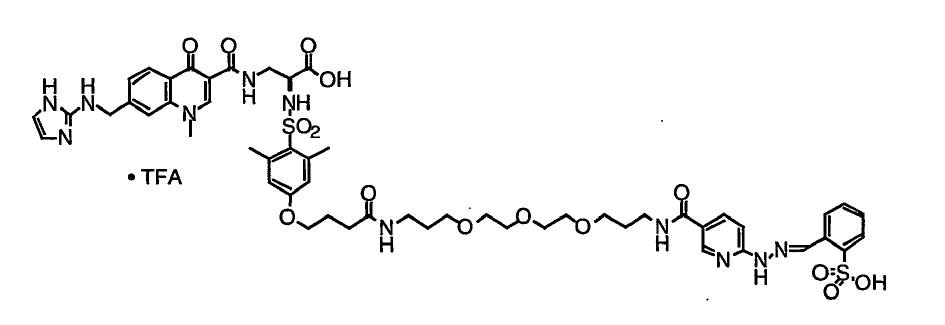

### Example 4

### 3-((1-(3-((6-((1-Aza-2-(2-sulfophenyl)vinyl)amino)(3-pyridyl))carbonylamino)propyl)-7-((imidazole-2-ylamino)-methyl)-4-oxo(3-hydroquinolyl))carbonylamino)-2-(((2,4,6-trimethylphenyl)sulfonyl)amino)propanoic Acid Trifluoroacetate Salt

### Part A - Ethyl 1-(3-((tert-Butoxy)carbonylamino)propyl)-7-bromo-4-oxohydroquinoline-3-carboxylate

A mixture of ethyl 7-bromo-4-oxohydroquinoline-3-carboxylate (6.28 g, 0.0212 mol), (tert-butoxy)-N-(3-bromopropyl)formamide (30.3 g, 0.127 mol), and anhydrous K₂CO₃ (12.5 g, 0.904 mol) in anhydrous DMF (200 mL) was stirred at 60 °C under a nitrogen atmosphere for 4 h, and then at ambient temperatures for 72 h. The DMF was removed under vacuum and the resulting oily solid was dissolved in EtOAc (500 mL). The EtOAc solution was washed consecutively with water (500 mL), saturated NaHCO₃ (500 mL), and saturated NaCl (500 mL), dried (MgSO₄), and concentrated to give a red oil. This oil was taken up in EtOAc (250 mL) and cooled, causing a solid ppt to form. This ppt was collected by filtration, washed with cold EtOAc, and dried to give the title compound as a colorless solid (6.25 g, 65%). MP: 140-142 °C; ¹H NMR (CDCl₃) : 8.49 (s, 1H), 8.39 (d, J = 8.6 Hz, 1H), 7.58 (s, 1H), 7.53 (d, J = 8.6 Hz, 1H), 4.72 (bs, 1H), 4.39 (q, J = 7.1 Hz, 2H), 4.20 (t, J = 7.6 Hz, 2H), 3.28-3.24 (m, 2H), 2.10-2.06 (m, 2H), 1.46 (s, 9H), 1.40 (t, J = 7.1 Hz, 3H); MS: m/e 455.2. [M+H]; High Resolution MS: Calcd for C₂₀H₂₆BrN₂O₅ [M+H]: 453.1025, Found: 453.1028.

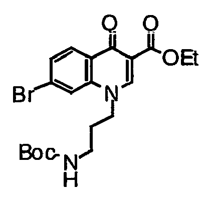

### Part B - Ethyl 1-(3-((tert-Butoxy)carbonylamino)propyl)-4-oxo-7-vinylhydroquinoline-3-carboxylate

The product from Part A, above (2.98 g, 6.60 mmol) was dissolved in toluene (50 mL) at a temperature of 100 °C and treated with tetrakis(triphenylphosphine)palladium(0) (152 mg, 0.132 mmol). After 5 min the mixture was treated with tributyl (vinyl) tin (1.93 mL, 6.60 mmol) and stirred 4.5 h at 100 °C under a nitrogen atmosphere, and 18 h at ambient temperatures. Additional tributyl(vinyl)tin (0.386 mL) and tetrakis(triphenylphosphine)palladium(0) (152 mg) were added and the mixture was heated at 100 °C for an additional 17 h. The toluene was removed under vacuum and the solid residue was triturated with ether to give the title compound as a pale green solid (1.67 g, 63%). MP: 133-135 °C; ¹H NMR (CDCl₃): 8.52 (d, J = 8.4 Hz, 1H), 8.51 (s, 1H), 7.55 (d, J = 8.4 Hz, 1H), 7.38 (s, 1H), 6.88-6.82 (m, 1H), 5.97 (d, J = 17.4 Hz, 1H), 5.51 (d, J = 10.8 Hz, 1H), 4.75 (bs, 1H), 4.42 (q, J = 7.2 Hz, 2H), 4.27 (t, J = 7.8 Hz, 2H), 3.6-3.25 (m, 2H), 2.16-2.11 (m, 2H), 1.49 (s, 9H), 1.45 (t, J = 7.2 Hz, 3H); MS: m/e 401.3 [M+H]; High Resolution MS: Calcd for C₂₂H₂₉N₂O₅ [M+H]: 401.2076, Found: 401.2075.

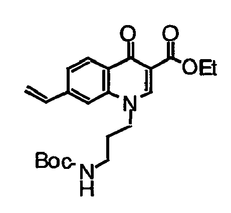

### Part C - Ethyl 1-(3-((tert-Butoxy)carbonylamino)propyl)-7-formyl-4-oxohydroquinoline-3-carboxylate

A solution of the product of Part B, above (1.50 g, 3.75 mmol) in dioxane (119 mL) and water (39 mL) was treated with a solution of osmium tetroxide (19.6 mg, 0.077 mmol) in dioxane (0.600 mL) and stirred at ambient temperatures under a nitrogen atmosphere for 5 min. Sodium periodate (2.40 g, 11.2 mmol) was added and the stirred at ambient temperatures for 2 h. The dioxane was removed under vacuum and the residue was taken up in DCM (500 mL). The DCM solution was washed consecutively with water (500 mL) and saturated NaCl (500 mL), dried (MgSO₄), and concentrated to give the title compound as an orange oily solid (1.52 g, 100%). ¹H NMR (CDCl₃): 10.17 (s, 1H), 8.68 (d, J = 8.2 Hz, 1H), 8.64 (s, 1H), 8.01 (s, 1H), 7.88 (d, J = 8.2 Hz, 1H), 4.82 (bs, 1H), 4.41-4.35 (m, 4H), 3.28 (s, 2H), 2.15-2.07 (m, 2H), 1.45 (s, 9H), 1.41 (t, J = 7.1 Hz, 3H); MS: m/e 403.3 [M+H]; High Resolution MS: Calcd for C₂₁H₂₇N₂O₆ [M+H]: 403.1870, Found: 403.1875.

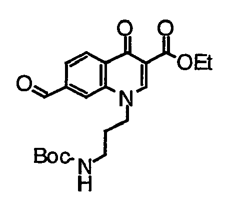

### Part D - Ethyl 1-(3-((tert-Butoxy)carbonylamino)propyl)-4-oxo-7-(((1-(triphenylmethyl)imidazole-2-yl)amino)methyl)hydroquinoline-3-carboxylate

A solution of the product of Part C, above (544 mg, 1.35 mmol) and 1-(triphenylmethyl)imidazole-2-ylamine (456 mg, 1.35 mmol) in toluene (60 mL) was heated at reflux under a nitrogen atmosphere with removal of water for 5 h. The solution was cooled, treated with Na(OAc)₃BH (1.14 g, 5.38 mmol) and stirred at ambient temperatures for 18 h. The mixture was diluted with EtOAc (400 mL), washed consecutively with water (500 mL) and saturated NaCl (500 mL), dried (MgSO₄), and concentrated to give an orange solid. This solid was dissolved in 50% ACN and purified by preparative HPLC on a Vydac C-18 column (50 x 250 mm) using a 0.60%/min gradient of 18 to 52% ACN containing 0.1% TFA at a flow rate of 49 mL/min. The main product peak eluting at 30.8 min was collected and lyophilized to give the title compound as a pale yellow solid (407 mg, 60%). MS: m/e 712.4 [M+H]; High Resolution MS: Calcd for C₄₃H₄₆N₅O₅ [M+H]: 712.3499, Found: 712.3485.

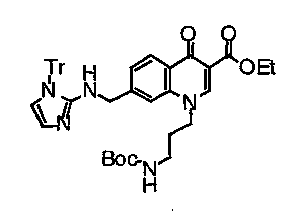

### Part E -1-(3-((tert-Butoxy)carbonylamino)propyl)-4-oxo-7-(((1-(triphenylmethyl)imidazole-2-yl)amino)methyl)-hydroquinoline-3-carboxylic Acid

A mixture of the product of Part D, above (997 mg, 1.40 mmol), water (7.3 mL), 3 N LiOH (3.5 mL), and THF (50 mL) was stirred at ambient temperatures under a nitrogen atmosphere for 3 h. The THF was removed under vacuum and the resulting mixture was dissolved in CHCl₃ (500 mL) and water (100 mL). The aqueous layer was adjusted to pH 3 with 1.0 N HCl and the layers were thoroughly mixed. The organic layer was washed consecutively with water (500 mL) and saturated NaCl (500 mL), dried (MgSO₄), and concentrated to give the title compound as a pale yellow solid (998 mg). MP: 153-160 °C; ¹H NMR (CDCl₃): δ 14.83 (s, 1H), 8.76 (s, 1H), 8.68 (s, 1H), 8.24 (d, J = 6 Hz, 1H), 7.49-7.35 (m, 9H), 7.12-7.10 (m, 6H), 6.82 (s, 1H), 6.52 (s, 1H), 6.24 (d, J = 6 Hz, 1H), 5.75 (bs, 1H), 4.87-4.83 (m, 2H), 4.77 (bs, 1H), 4.51 (t, J = 9 Hz, 2H), 3.38 (s, 2H), 2.23 (s, 2H), 1.42 (s, 9H); MS: m/e 684.3 [M+H]; High Resolution MS: Calcd for C₄₁H₄₂N₅O₅ [M+H]: 684.3186, Found: 684.3181.

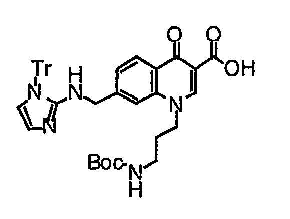

### Part F - Methyl 3-((1-(3-((tert-Butoxy)carbonylamino)propyl)-4-oxo-7-(((1-(triphenylmethyl)imidazole-2-yl)amino)methyl)(3-hydroquinolyl))carbonylamino)-2-(((2,4,6-trimethylphenyl)sulfonyl)amino)propanoate

A solution of the product of Part E, above (300 mg, 0.437 mmol), TEA (0.243 mL, 1.75 mmol), and HBTU (230 mg, 0.606 mmol) in anhydrous DMF (4 mL) was stirred at ambient temperatures for 5 min. A solution of methyl 3-amino-2-(((2,4,6-trimethylphenyl)sulfonyl)amino)propanoate hydrochloride (184 mg, 0.637 mmol) in anhydrous DMF (3 mL) was added and the solution was stirred at ambient temperatures under a nitrogen atmosphere for 2 h. The solution was diluted with EtOAc (200 mL) and washed consecutively with water (2 x 50 mL), saturated NaHCO₃ (50 mL), and saturated NaCl (50 mL), dried (MgSO₄), and concentrated to give a viscous amber oil. Purification by flash chromatography on a 2.5 x 24 cm silica gel column using a EtOAc/MeOH step gradient (98/2, 95/5, 75/25) gave the title compound as a pale yellow oil (330 mg, 78%). MS: m/e 966.6 [M+H]; High Resolution MS: Calcd for C₅₄H₆₀N₇O₈S [M+H]: 966.4224, Found: 966.4224.

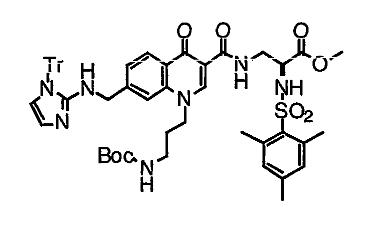

### Part G-3-((1-(3-((tert-Butoxy)carbonylamino)propyl)-4-oxo-7-(((1-(triphenylmethyl)imidazole-2-yl)amino)methyl)(3-hydroquinolyl))carbonylamino)-2-(((2,4,6-trimethylphenyl)sulfonyl)amino)propanoic Acid

A solution of the product of Part F, above (51 mg, 0.052 mmol), water (0.27 mL), and 3 N LiOH (0.13 mL) in MeOH (2 mL) was allowed to stand at ambient temperatures for 3.5 h and concentrated under vacuum. The resulting solid was dissolved in water (10 mL) and adjusted to pH 3 with 1.0 N HCl. The aqueous mixture was extracted with DCM (2 x 30 mL). The combined DCM extracts were washed with saturated NaCl (30 mL), dried (MgSO₄), and concentrated to give the title compound as a colorless solid (72 mg). MS: m/e 952.5 [M+H]; High Resolution MS: Calcd for C₅₃H₅₈N₇O₈S [M+H]: 952.4067, Found: 952.4056.

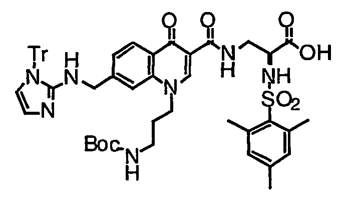

### Part H - 3-((1-(3-Aminopropyl)-7-((imidazole-2-ylamino)-methyl)-4-oxo(3-hydroquinolyl))carbonylamino)-2-(((2,4,6-trimethylphenyl)sulfonyl)amino)propanoic Acid Bis(trifluoroacetate) Salt

The product of Part I, above (0.052 mmol) and Et₃SiH (0.042 mL, 0.26 mmol) were dissolved in degassed TFA (2 mL), heated at 70 °C for 2.5 h, and concentrated to give an amber oil. This oil was dissolved in water (25 mL) and washed with ether (2 x 15 mL). The combined ether washings were back-extracted with water (15 mL). The combined water extracts were lyophilized to give the title compound as a colorless powder (34 mg, 78%). MS: m/e 610.4 [M+H]; High Resolution MS: Calcd for C₂₉H₃₆N₇O₆S [M+H]: 610.2448, Found: 610.2462.

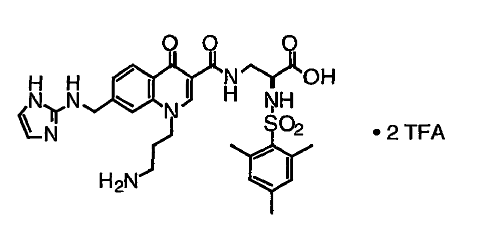

### Part I - 3-((1-(3-((6-((1-Aza-2-(2-sulfophenyl)vinyl)amino)(3-pyridyl))carbonylamino)propyl)-7-((imidazole-2-ylamino)-methyl)-4-oxo(3-hydroquinolyl))carbonylamino)-2-(((2,4,6-trimethylphenyl)sulfonyl)amino)propanoic Acid Trifluoroacetate Salt

A solution of the product of Part H, above (13.7 mg, 0.0163 mmol), TEA (0.015 mL, 0.108 mmol), and 2-(2-aza-2-((5-((2,5-dioxopyrrolidinyl)carbonyl)(2-pyridyl))-amino)vinyl)benzenesulfonic acid (8.2 mg, 0.0186 mmol) in anhydrous DMF (2.0 mL) was allowed to stand at ambient temperatures under a nitrogen atmosphere for 24 h. The DMF was removed under reduced pressure and the amber oil was dissolved in 50% ACN and purified by preparative HPLC on a Vydac C-18 column (22 x 250 mm) using 0.1% TFA in water for 5 min followed by a 2.52%/min gradient of 0 to 63% ACN containing 0.1% TFA at a flow rate of 20 mL/min. The main product peak eluting at 21.4 min was collected and lyophilized to give the title compound as a colorless powder (12.5 mg, 75%). MS: m/e 913.3 [M+H]; High Resolution MS: Calcd for C₄₂H₄₅N₁₀O₁₀S₂ [M+H]: 913.2761, Found: 913.2751.

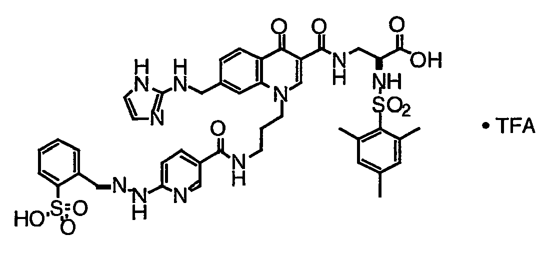

### Example 5

### 3-((1-(3-((6-((1-Aza-2-(2-sulfophenyl)vinyl)amino)(3-pyridyl))carbonylamino)propyl)-7-(((1-hydroxyimidazole-2-yl)amino)methyl)-4-oxo(3-hydroquinolyl))carbonylamino)-2-(((2,4,6-trimethylphenyl)sulfonyl)amino)propanoic Acid Trifluoroacetate Salt

### Part A - Methyl 3-((1-(3-Aminopropyl)-7-((imidazole-2-ylamino)methyl)-4-oxo(3-hydroquinolyl))carbonylamino)-2-(((2,4,6-trimethylphenyl)sulfonyl)amino)propanoate Bis(trifluoroacetate) Salt

A solution of the product of Example 4, Part F (120 mg, 0.124 mmol) and Et₃SiH (0.99 mL, 6.20 mmol) in TFA (10 mL) was heated at 70 °C for 1 h, and concentrated to give an amber oil. This oil was dissolved in water (50 mL) and washed with ether (2 x 30 mL). The combined ether washings were back-extracted with water (20 mL). The combined water extracts were lyophilized to give the title compound as a colorless powder (105 mg, 100%). MS: m/e 624.4 [M+H]; High Resolution MS: Calcd for C₃₀H₃₈N₇O₆S [M+H]: 624.2604, Found: 624.2608.

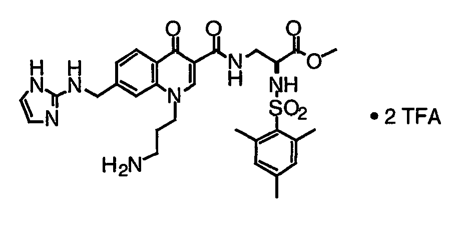

### Part B - 3-((1-(3-Aminopropyl)-7-(((1-hydroxyimidazol-2-yl)amino)methyl)-4-oxo(3-hydroquinolyl))carbonylamino)-2-(((2,4,6-trimethylphenyl)sulfonyl)amino)propanoic Acid Trifluoroacetate Salt

A mixture of the product of Part A, above (105 mg, 0.126 mmol), water (3.0 mL), and 3 N LiOH (1.82 mL) in peroxide-containing THF (4 mL) was allowed to stand at ambient temperatures for 1 h and concentrated under vacuum. The resulting solid was dissolved in water (10 mL) and adjusted to pH 5 with 1.0 N HCl. Insoluble impurities were removed by filtration and the filtrate was lyophilized to give a colorless solid. This solid was dissolved in water and purified by preparative HPLC on a Vydac C-18 column (22 x 250 mm) using 0.1% TFA in water for 5 min followed by a 2.52%/min gradient of 0 to 63% ACN containing 0.1% TFA at a flow rate of 20 mL/min. The main product peak eluting at 19.5 min was collected and lyophilized to give the title compound as a colorless powder (10.0 mg, 11%). MS: m/e 314.0 [M+2H]

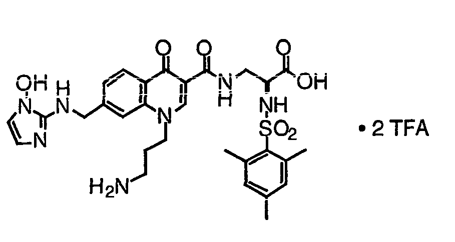

### Part C - 3-((1-(3-((6-((1-Aza-2-(2-sulfophenyl)vinyl)amino)(3-pyridyl))carbonylamino)propyl)-7-(((1-hydroxyimidazole-2-yl)amino)methyl)-4-oxo(3-hydroquinolyl))carbonylamino)-2-(((2,4,6-trimethylphenyl)sulfonyl)amino)propanoic Acid Trifluoroacetate Salt

A solution of the product of Part B, above (10.0 mg, 0.0135 mmol), TEA (0.018 mL, 0.129 mmol), and 2-(2-aza-2-((5-((2,5-dioxopyrrolidinyl)carbonyl)(2-pyridyl))-amino)vinyl)benzenesulfonic acid (7.2 mg, 0.0163 mmol) in anhydrous DMF (4 mL) was allowed to stand at ambient temperatures under a nitrogen atmosphere for 20 h. The DMF was removed under vacuum and the amber oil was dissolved in 30% ACN and purified by preparative HPLC on a Vydac C-18 column (22 x 250 mm) using 0.1% TFA in water for 5 min followed by a 2.52%/min gradient of 0 to 63% ACN containing 0.1% TFA at a flow rate of 20 mL/min. The main product peak eluting at 21.5 min was collected and lyophilized to give the title compound as a colorless powder (3.5 mg, 25%). MS: m/e 929.4 [M+H]; High Resolution MS: Calcd for C₄₂H₄₅N₁₀O₁₁S₂ [M+H]: 929.2710, Found: 929.2698.

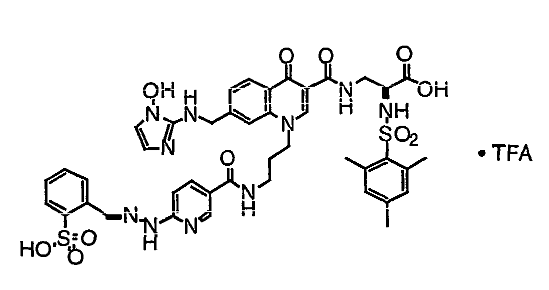

### Example 6

### 3-((1-(3-(3-(N-(3-(2-(2-(3-((6-((1-Aza-2-(2-sulfophenyl)vinyl) amino) (3-pyridyl))carbonylamino)propoxy)ethoxy)-ethoxy)propyl)carbamoyl)propanoylamino)propyl)-7-((imidazole-2-ylamino)methyl)-4-oxo(3-hydroquinolyl))-carbonylamino)-2-(((2,4,6-trimethylphenyl)sulfonyl)-amino)propanoic Acid Trifluoroacetate Salt

### Part A - 3-(N-(3-(2-(2-(3-((tert-Butoxy)carbonylamino)-propoxy)ethoxy)ethoxy)propyl)carbamoyl)propanoic Acid

A solution of N-(3-(2-(2-(3-aminopropoxy) ethoxy) ethoxy)propyl) (tert-butoxy) formamide (as described by D. S. Wilbur et al. in Bioconjugate Chem. 1998, 9, 322-330) (2.00 g, 6.24 mmol), TEA (1.0 mL, 7.49 mmol), and succinic anhydride (624 mg, 6.24 mmol) in anhydrous DMF (5 mL) was stirred at ambient temperatures under a nitrogen atmosphere for 4 h. The DMF was removed under reduced pressure to give the title compound as a pale yellow oil (2.80 g). MS: m/e 839.5 [2M-H], 419.4 [M-H].

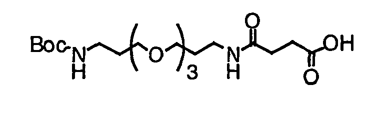

### Part B - Methyl 3-((1-(3-(3-(N-(3-(2-(2-(3-((tert-Butoxy) carbonylamino)propoxy)ethoxy)ethoxy)propyl)carbam oyl)propanoylamino)propyl-4-oxo-7-(((1-(triphenylmethyl) imidazole-2-yl)amino)methyl) (3-hydroquinolyl))carbonylamino)-2-(((2,4,6-trimethylphenyl)sulfonyl)amino)propanoate

The product of Example 4, Part F (46.1 mg, 0.477 mmol) was dissolved in 50% TFA/DCM (2.0 mL) for 15 min at ambient temperatures and concentrated to give a yellow oil. This oil was dissolved in anhydrous DMF (1.0 mL) and made basic to pH paper with TEA. In a separate flask, the product of Part A, above (26.1 mg, 0.062 mmol), TEA (0.014 mL, 0.099 mmol), and HBTU (27.7 mg, 0.074 mmol) were dissolved in anhydrous DMF (1.0 mL). The resulting solution was allowed to react for 5 min and combined with the DMF solution from the TFA deprotection reaction. The combined solutions were allowed to stand at ambient temperatures under a nitrogen atmosphere for 20 min and concentrated under vacuum. The resulting oil was dissolved in 50% ACN and purified by preparative HPLC on a Vydac C-18 column (22 x 250 mm) using a 1.8%/min gradient of 18 to 72% ACN containing 0.1% TFA at a flow rate of 20 mL/min. The main product peak eluting at 26.8 min was collected and lyophilized to give the title compound as a colorless powder (44.5 mg, 68%). MS: m/e 1268.6 [M+H] ; High Resolution MS: Calcd for C₆₈H₈₆N₉O₁₃S [M+H] : 1268.6065, Found: 1268.6070.

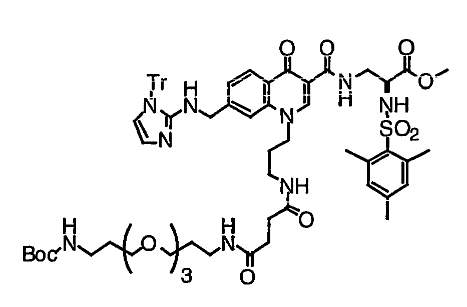

### Part C - 3-((1-(3-(3-(N-(3-(2-(2-(3-((tert-Butoxy)carbonylamino)propoxy)ethoxy)ethoxy)propyl)carbam oyl)propanoylamino)propyl-4-oxo-7-(((1-(triphenylmethyl)imidazole-2-yl)amino)methyl) (3-hydroquinolyl))carbonylamino)-2-(((2,4,6-trimethylphenyl)sulfonyl)amino)propanoic Acid

A solution of the product of Part B, above (31.1 mg, 0.0227 mmol), 3 n LiOH (0.091 mL), and water (0.117 mL) in MeOH (1.30 mL) was stirred at ambient temperatures for 8.5 h. The MeOH was removed under vacuum and the aqueous mixture was diluted with water (30 mL) and adjusted to pH 4 with 1.0 N HCl. The resulting aqueous mixture was extracted with DCM (2 x 50 mL). The combined DCM extracts were washed with saturated NaCl (50 mL), dried (MgSO₄), and concentrated to give the title compound as a colorless solid (24.6 mg, 86%).

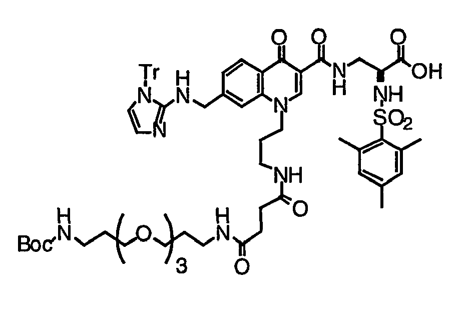

### Part D - 3-((1-(3-(3-(N-(3-(2-(2-(3-aminopropoxy)ethoxy)ethoxy)propyl)carbamoyl)-propanoylamino)propyl)-7-((imidazole-2-ylamino)methyl)-4-oxo(3-hydroquinolyl))carbonylamino)-2-(((2,4,6-trimethylphenyl)sulfonyl)amino)propanoic Acid Bis(trifluoroacetate) Salt

A solution of the product of Part C, above (24.6 mg, 0.0194 mmol) and Et₃SiH (0.016 mL, 0.097 mmol) in TFA (2.0 mL) was heated at 70 °C under a nitrogen atmosphere for 3 h, and concentrated to give a yellow solid. This solid was dissolved in water (50 mL) and washed with ether (2 x 25 mL). The aqueous layer was lyophilized to give the title compound as a pale yellow solid (20.7 mg, 93%). MS: m/e 912.5 [M+H].

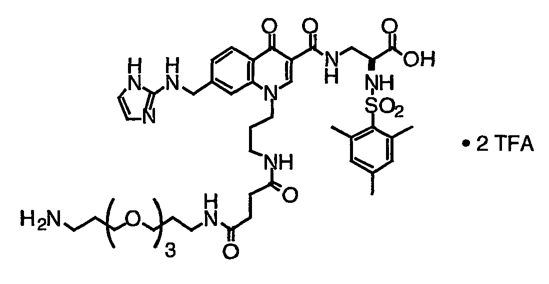

### Part E - 3-((1-(3-(3-(N-(3-(2-(2-(3-((6-((1-Aza-2-(2-sulfophenyl)vinyl)amino)(3-pyridyl))carbonylamino)propoxy)ethoxy)-ethoxy)propyl)carbamoyl)propanoylamino)propyl)-7-((imidazole-2-ylamino)methyl)-4-oxo(3-hydroquinolyl))-carbonylamino)-2-(((2,4,6-trimethylphenyl)sulfonyl)-amino)propanoic Acid Trifluoroacetate Salt

A solution of the product of Part D, above (15.5 mg, 0.0136 mmol), TEA (0.010 mL, 0.0746 mmol), and 2-(2-aza-2-((5-((2,5-dioxopyrrolidinyl)carbonyl)(2-pyridyl))-amino)vinyl)benzenesulfonic acid (8.0 mg, 0.0182 mmol) in anhydrous DMF (2.0 mL) was allowed to stand at ambient temperatures under a nitrogen atmosphere for 24 h. The DMF was removed under vacuum and the resulting yellow oil was dissolved in 50% ACN and purified by preparative HPLC on a Vydac C-18 column (22 x 250 mm) using 0.1% TFA in water for 5 min followed by a 2.52%/min gradient of 0 to 63% ACN containing 0.1% TFA at a flow rate of 20 mL/min. The main product peak eluting at 21.7 min was collected and lyophilized to give the title compound as a colorless powder (7.2 mg, 40%). MS: m/e 1215.5 [M+H]; High Resolution MS: Calcd for C₅₆H₇₁N₁₂O₁₅S₂ [M+H]: 1215.4603, Found: 1215.4580.

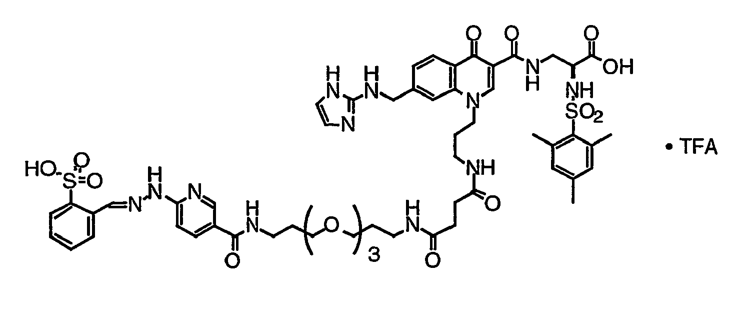

### Example 7

### 2-(2-Aza-2-(5-(N-(1,3-bis(3-(2-(2-(3-(3-(N-(3-(3-(N-(3-carboxy-2-(((2,4,6-trimethylphenyl)sulfonyl)amino)-ethyl)carbamoyl)-7-((imidazole-2-ylamino)methyl)4-oxohydroquinolyl)propyl)carbamoyl)propanoylamino)propoxy )ethoxy)ethoxy)propyl)carbamoyl)(2-pyridyl))amino)vinyl)benzenesulfonic Acid Bis(trifluoroacetate) Salt

### Part A - N,N'-Bis(3-(2-(2-(3-(3-(N-(3-(3-(N-(3-carbomethoxy-2-(((2,4,6-trimethylphenyl)-sulfonyl)amino)ethyl)carbamoyl)-4-oxo-7-(((1-(triphenylmethyl)imidazole-2-yl)amino)methyl)-hydroquinolyl)propyl)carbamoyl)propanoylamino)propoxy) et hoxy)ethoxy)propyl-2-((tert-butoxy)carbonylamino)pentane-1,5-diamide

A solution of the product of Example 6, Part B (50.5 mg, 0.0398 mmol) in 50/50 TFA/DCM (2 mL) was allowed to react for 20 min at ambient temperatures and concentrated to a viscous oil. This oil was taken up in anhydrous DMF and made basic to pH paper with TEA. This solution was treated with Boc-L-Glu-OH (4.5 mg, 0.0181 mmol) and HBTU (16.6 mg, 0.0438 mmol), and allowed to stand at ambient temperatures for 2 h. The DMF was removed under vacuum and the resulting oil was dissolved in 60% ACN and purified by preparative HPLC on a Vydac C-18 column (22 x 250 mm) using a 1.8%/min gradient of 18 to 72% ACN containing 0.1% TFA at a flow rate of 20 mL/min. The main product peak eluting at 21.5 min was collected and lyophilized to give the title compound as a colorless powder (38.8 mg, 84%). MS: m/e 2306.5 [M+H-Tr], 2064.4 [M+H-2Tr], 1275.0 [M+2H]; High Resolution MS: Calcd for C₁₁₇H₁₅₄N₁₉OS₂ [M+H-Tr]: 2305.0753, Found: 2305.0770.

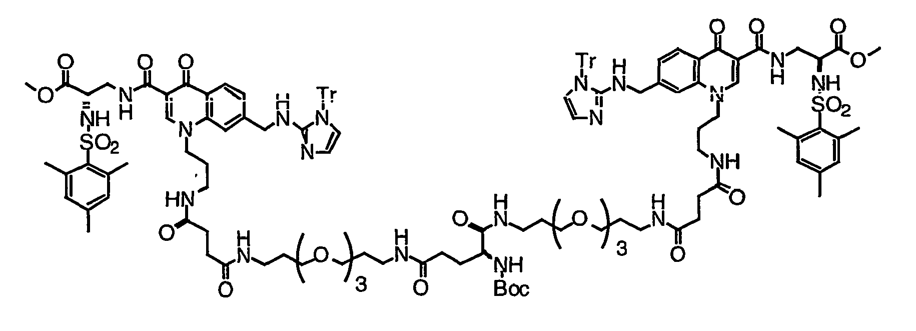

### Part B-2-Amino-N,N'-bis(3-(2-(2-(3-(3-(N-(3-(3-(N-(3-carboxy-2-(((2,4,6-trimethylphenyl)-sulfonyl)amino)ethyl)carbamoyl)-7-((imidazole-2-ylamino)methyl)4-oxohydroquinolyl)propyl)carbamoyl)propanoylamino)propoxy )ethoxy)ethoxy)propyl)pentane-1,5-diamide Tris(trifluoroacetate) Salt

A solution of the product from Part A, above (38.8 mg, 0.0152 mmol), 3 N LiOH (0.075 mL), and water (0.156 mL) in MeOH (2.0 mL) was stirred at ambient temperatures for 18 h. The MeOH was removed under vacuum and the aqueous mixture was diluted with water (50 mL) and adjusted to pH 3 using 0.5 N HCl. The mixture was extracted with DCM (2 x 50 mL). The combined DCM extracts were washed with saturated NaCl (50 mL), dried (MgSO₄), and concentrated to give a colorless solid. This solid was dissolved in TFA (3.0 mL) along with Et₃SiH (0.031 mL, 0.178 mol), heated at 70 °C under a nitrogen atmosphere for 11 h, and concentrated to give a yellow oil. This oil was dissolved in water (25 mL) and washed with ether (2 x 25 mL). The aqueous solution was lyophilized to give a pale yellow solid. This solid was dissolved in water and purified by preparative HPLC on a Vydac C-18 column (22 x 250 mm) using 0.1% TFA in water for 5 min followed by a 2.52%/min gradient of 0 to 63% ACN containing 0.1% TFA at a flow rate of 20 mL/min. The main product peak eluting at 22.4 min was collected and lyophilized to give the title compound as a colorless powder (5.1 mg, 18%). MS: m/e 968.2 [M+2H], 646.0 [M+3H].

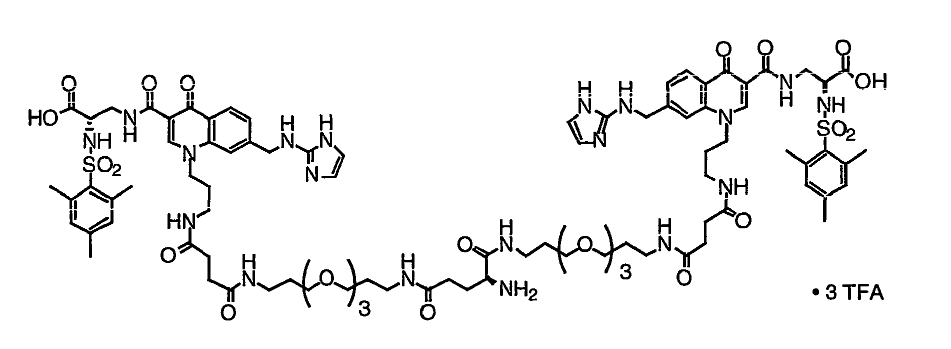

### Part C-2-(2-Aza-2-(5-(N-(1,3-bis(3-(2-(2-(3-(3-(N-(3-(3-(N-(3-carboxy-2-(((2,4,6-trimethylphenyl)-sulfonyl)amino)ethyl)carbamoyl)-7-((imidazole-2-ylamino)methyl)4-oxohydroquinolyl)propyl)carbamoyl)propanoylamino)propoxy )ethoxy)ethoxy)propyl)carbamoyl)(2-pyridyl))amino)vinyl)benzenesulfonic Acid Bis(trifluoroacetate) Salt

A solution of the product of Part B, above (5.1 mg, 0.00224 mmol), TEA (0.002 mL, 0.0115 mmol), and 2-(2-aza-2-((5-((2,5-dioxopyrrolidinyl)carbonyl)(2-pyridyl))-amino)vinyl)benzenesulfonic acid (1.2 mg, 0.00272 mmol) in anhydrous DMF (2.0 mL) was allowed to stand at ambient temperatures under nitrogen for 72 h. The DMF was removed under vacuum and the resulting oil was dissolved in 50% ACN and purified by preparative HPLC on a Vydac C-18 column (22 x 250 mm) using 0.1 % TFA in water for 5 min followed by a 2.52%/min gradient of 0 to 63% ACN containing 0.1% TFA at a flow rate of 20 mL/min. The main product peak eluting at 23.5 min was collected and lyophilized to give the title compound as a colorless powder (0.5 mg, 9.0%). MS: m/e 1120.0 [M+2H]; High Resolution MS: Calcd for C₁₀₄H₁₃₇N₂₂O₂₈S₃ [M⁺]: 2237.9055, Found: 2237.9120.

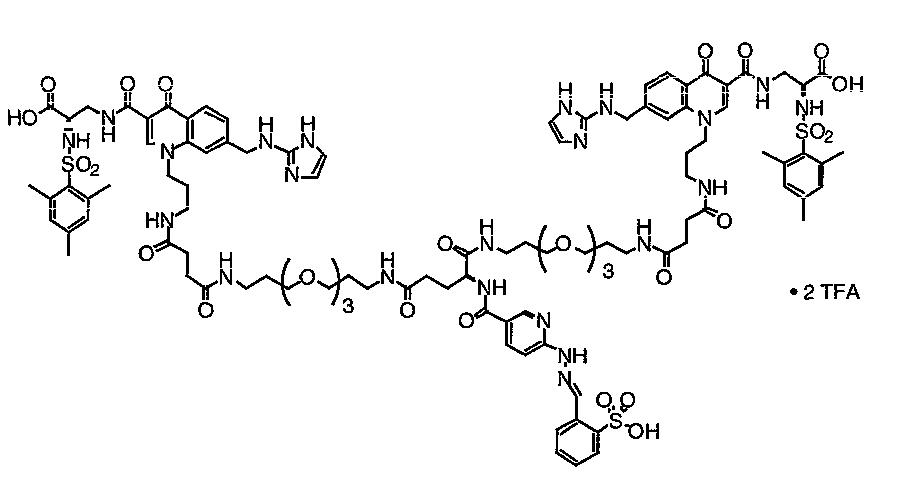

### Example 8

### DOTA Conjugate of 3-((1-(3-(3-(N-(3-(2-(2-(N-(L-Asp-L-Asp)3-aminopropoxy)ethoxy)ethoxy)propyl)carbamoyl)-propanoylamino)propyl-7-((imidazole-2-ylamino)methyl)-4-oxo(3-hydroquinolyl))carbonylamino)-2-(((2,4,6-trimethylphenyl)sulfonyl)amino)propanoic Acid Bis(trifluoroacetate Salt

### Part A - Carbobenzyloxy-L-Asp(O-t-Bu)-L-Asp(O-t-Bu)-OMe

A solution of Cbz-Asp(O-t-Bu)-OH (1.54 g, 4.76 mmol), H-Asp(O-t-Bu)-OMe•HCl (1.14 g, 4.76 mmol), DIEA (1.85 mL, 10.5 mmol), and HBTU (1.99 g, 5.24 mmol) in DMF (20 mL) was stirred at ambient temperatures for 18 h. Water (100 mL) and EtOAc (50 mL) were added and the layers were separated. The water layer was extracted with EtOAc (2 x 50 mL). The combined EtOAc extracts were washed consecutively with water (50 mL), 10% KHSO₄ (2 x 50 mL), and 10% NaHCO₃ (50 mL). The organic phase was dried (MgSO₄), and concentrated to give an oily solid. This material was triturated with ether to give the title compound as a colorless solid (2.14g, 89%). MS: m/e 1017.6 [2M+H], 509.4 [M+H].

### Part B - Carbobenzyloxy-L-Asp(O-t-Bu)-L-Asp(O-t-Bu)-OH

A mixture of the product of Part A, above (200 mg, 0.393 mmol), LiOH (38 mg, 0.865 mmol), water (40 mL), and THF (200 mL) was stirred at ambient temperatures for 28 h, and concentrated to remove THF. The aqueous mixture was diluted with additional water (20 mL) and washed with EtOAc (20 mL). The aqueous phase was adjusted to pH 4 with 1.0 N HCl and extracted with EtOAc (20 mL). The EtOAc extract was washed with saturated NaCl (15 mL), dried (MgSO₄), and concentrated to give a colorless solid. This solid was dissolved in 60% ACN and purified by preparative HPLC on a Vydac C-18 column (22 x 250 mm) using a 2.4%/min gradient of 18 to 90% ACN containing 0.1% TFA at a flow rate of 20 mL/min. The main product peak eluting at 19.0 min was collected and lyophilized to give the title compound as a colorless powder (95 mg, 49%).

### Part C - Methyl 3-((1-(3-(3-(N-(3-(2-(2-(N-(benzyloxycarbonyl-L-Asp(O-t-Bu)-L-Asp(O-t-Bu))3-aminopropoxy)ethoxy)ethoxy)propyl)carbamoyl)-propanoylamino)propyl-4-oxo-7-(((1-(triphenylmethyl)-imidazole-2-yl)amino)methyl)(3-hydroquinolyl))carbonylamino)-2-(((2,4,6-trimethylphenyl)sulfonyl)amino)propanoate

The product of Example 6, Part B (44.0 mg, 0.0894 mmol) in TFA (1.5 mL) was allowed to stand at ambient temperatures for 45 min and concentrated to a yellow oil. This oil was dissolved in anhydrous DMF (2.0 mL) and made basic to pH paper with TEA. In a separate flask, the product of Part B, above (69.3 mg, 0.0547 mmol) was dissolved in anhydrous DMF (2.0 mL) and pre activated by treatment with TEA (0.015 mL, 0.104 mmol) and HBTU (32.6 mg, 0.0859 mmol). After 10 min this solution was added to the DMF solution from the TFA deprotection reaction, and the combined solutions were stirred at ambient temperatures for 30 min. The DMF was removed under vacuum and the resulting oil was dissolved in 60% ACN and purified by preparative HPLC on a Vydac C-18 column (22 x 250 mm) using a 1.54%/main gradient of 18 to 72% ACN containing 0.1% TFA at a flow rate of 20 mL/min. The main product peak eluting at 29.9 min was adjusted to pH 8 with saturated NaHCO₃ and concentrated to remove the ACN. The remaining aqueous mixture was extracted with EtOAc (2 x 40 mL). The combined EtOAc extracts were washed with saturated NaCl (40 mL), dried (MgSO₄), and concentrated to give the title compound as a colorless solid (56.4 mg, 63%). MS: m/e 1644.8 [M+H]; High Resolution MS: Calcd for C₈₇H₁₁₀N₁₁O₁₉S [M+H]: 1644.7700, Found: 1644.771.

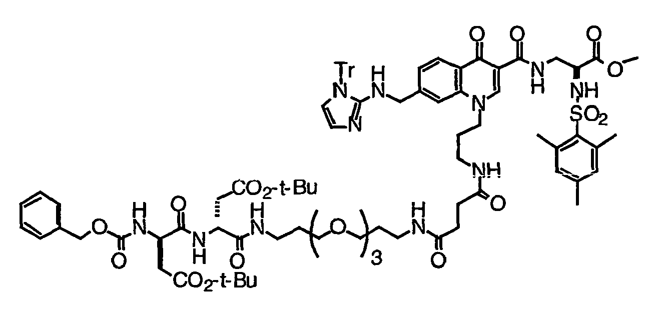

### Part D - Methyl 3-((1-(3-(3-(N-(3-(2-(2-(N-(L-Asp(O-t-Bu)-L-Asp(O-t-Bu))3-aminopropoxy)-ethoxy)ethoxy)propyl)carbamoyl)propanoylamino)propyl-4-oxo-7-(((1-(triphenylmethyl)imidazole-2-yl)amino)methyl)(3-hydroquinolyl))carbonylamino)-2-(((2,4,6-trimetriylphenyl)sulfonyl)amino)propanoate

The product of Part E, above (55.0 mg, 0.0335 mmol) was hydrogenolyzed over 10% Pd/C (25 mg) in MeOH (15 mL) at 40 psi for 3.5 h. The catalyst was removed by filtration through filter aid and the filtrate was concentrated to give the title compound as a pale yellow oil (41.8 mg, 83%). MS: m/e 1510.8 [M+H] .

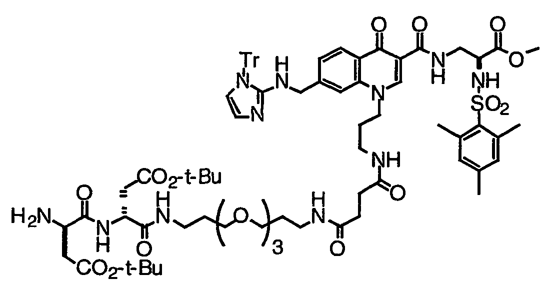

### Part E - DOTA-tri-t-butyl Ester Conjugate of Methyl 3-((1-(3-(3-(N-(3-(2-(2-(N-(L-Asp(O-t-Bu)-L-Asp(O-t-Bu))3-aminopropoxy)ethoxy)ethoxy)propyl)carbamoyl)-propanoylamino)propyl-4-oxo-7-(((1-(triphenylmethyl)-imidazole-2-yl)amino)methyl)(3-hydroquinolyl))carbonylamino)-2-(((2,4,6-trimethylphenyl)sulfonyl)amino)propanoate

A solution of the product of Part D, above (41.8 mg, 0.0277 mmol), the product of Example 2, Part B, 39.9 mg, 0.0436 mmol), TEA (0.023 mL , 0.166 mmol), and HBTU (15.6 mg, 0.0411 mmol) in anhydrous DMF (3.0 mL) was allowed to stand at ambient temperatures under a nitrogen atmosphere for 20 h. The DMF was removed under vacuum and the resulting oil was dissolved in 60% ACN and purified by preparative HPLC on a Vydac C-18 column (22 x 250 mm) using a 2.4%/min gradient of 18 to 90% ACN containing 0.1% TFA at a flow rate of 20 mL/min. The main product peak eluting at 21.2 min was collected and lyophilized to give the title compound as a colorless powder (24.8 mg, 43%). MS: m/e 2066.3 [M+H], 1033.6 [M+2H] ; High Resolution MS: Calcd for C₁₀₇H₁₅₄N₁₅O₂₄S [M+H]: 2065.1011, Found: 2065.1030.

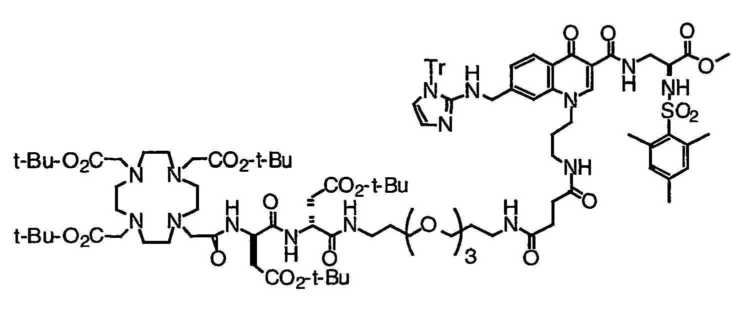

### Part F - DOTA Conjugate of 3-((1-(3-(3-(N-(3-(2-(2-(N-(L-Asp-L-Asp)3-aminopropoxy)-ethoxy)ethoxy)propyl)carbamoyl)propanoylamino)propyl-7-((imidazole-2-ylamino)methyl)-4-oxo(3-hydroquinolyl))carbonylamino)-2-(((2,4,6-trimethylphenyl)sulfonyl)amino)propanoic Acid Bis(trifluoroacetate Salt

A mixture of the product of Part G, above (18.8 mg. 0.0091 mmol), water (0.150 mL), 3 N LiOH (0.015 mL), and peroxide-free THF (1.5 mL) was stirred at ambient temperatures for 3 h. The THF was removed under vacuum and the aqueous mixture was diluted with water (40 mL) and adjusted to pH 7 with 0.1 N HCl. The mixture was extracted with DCM (2 x 30 mL) and the combined extracts were concentrated to give a yellow oil. This oil was dissolved in TFA (1.0 mL) along with Et₃SiH (0.030 mL, 0.184 mmol) and heated at 40 °C under a nitrogen atmosphere for 48 h. The solution was concentrated and the resulting oil was dissolved in water and purified by preparative HPLC on a Vydac C-18 column (22 x 250 mm) using 0.1% TFA in water for 5 min followed by a 2.52%/min gradient of 0 to 63% ACN containing 0.1% TFA at a flow rate of 20 mL/min. The main product peak eluting at 19.9 min was collected and lyophilized to give the title compound as a colorless powder (1.5 mg, 9.4%). MS: m/e 1528.9 [M+2H], 765.1 [M+2H], 510.7 [M+3H].

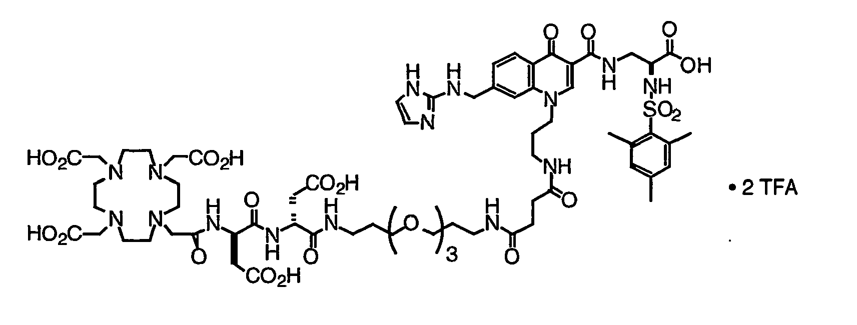

### Example 9

### DOTA/2-Amino-N,N'-bis(3-(2-(2-(3-(3-(N-(3-(3-(N-(3-carboxy-2-(((2,4,6-trimethylphenyl)-sulfonyl)amino)ethyl)carbamoyl)-7-((imidazole-2-ylamino)methyl)4-oxohydroquinolyl)-propyl)carbamoyl)propanoylamino)propoxy)ethoxy)ethoxy)-propyl)pentane-1,5-diamide Tris(trifluoroacetate) Salt Conjugate

### Part A - DOTA-tri-t-butyl Ester/2-Amino-N,N'-bis(3-(2-(2-(3-(3-(N-(3-(3-(N-(3-carboxy-2-(((2,4,6-trimethylphenyl)sulfonyl)amino)ethyl)carbamoyl)-7-((-imidazole-2-ylamino)methyl)4-oxohydroquinolyl)propyl)carbamoyl)-propanoylamino)propoxy)ethoxy)ethoxy)propyl)pentane-1,5-diamide Hexakis(trifluoroacetate) Salt Conjugate

A solution of the product of Example 2, Part B, HBTU, and DIEA in anhydrous DMF is stirred at ambient temperatures under nitrogen for 15 min and treated with the product of Example 7, Part B. The resulting solution is stirred an additional 18 h and the DMF is removed under vacuum. The resulting residue is purified by preparative HPLC on a C18 column using a water:ACN:0.1% TFA gradient. The product fraction is lyophilized to give the title compound.

### Part B - DOTA/2-Amino-N,N'-bis(3-(2-(2-(3-(3-(N-(3-(3-(N-(3-carboxy-2-(((2,4,6-trimethylphenyl)-sulfonyl)amino)ethyl)carbamoyl)-7-((imidazole-2-ylamino)methyl)4-oxohydroquinolyl)propyl)carbamoyl)-propanoylamino)propoxy)ethoxy)ethoxy)propyl)pentane-1,5-diamide Tris(trifluoroacetate) Salt Conjugate

The product of Part B, above, is dissolved in degassed TFA, treated with triethylsilane, and heated at 50 °C under nitrogen for 1 h. The solution is concentrated under vacuum and the resulting residue is purified by preparative HPLC on a C18 column using a water: ACN:0.1% TFA gradient. The product fraction is lyophilized to give the title compound.

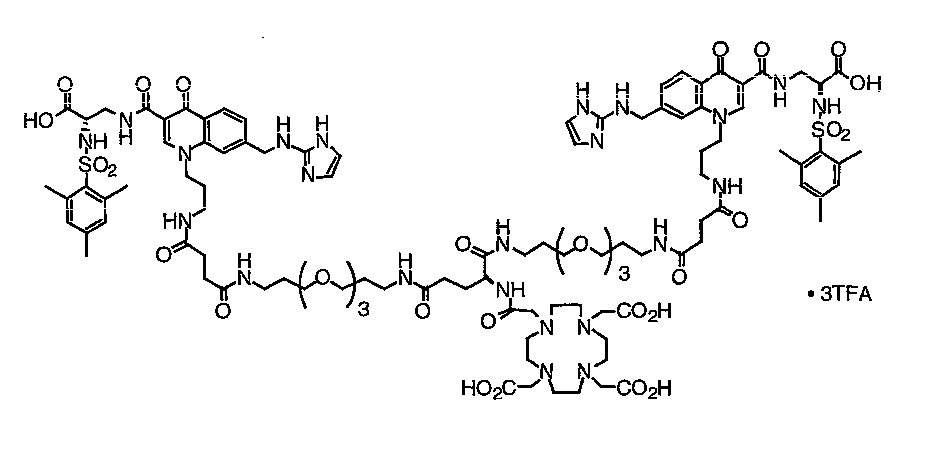

### Example 10

### DOTA/2-(((4-(3-(N-(3-(2-(2-(3-(2-Amino-3-sulfopropyl)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid Trifluoroacetate Salt Conjugate

### Part A - 2-(((4-(3-(N-(3-(2-(2-(3-(2-((tert-Butoxy)-carbonylamino)-3-sulfopropyl)propoxy)ethoxy)ethoxy)-propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid

The product of Example 3, Part I is dissolved in anhydrous DMF and treated with the N-hydroxysuccinimide ester of Boc-cysteic acid (as described in Liebigs Ann. Chem. 1979, 776-783) and DIEA. The solution is stirred at ambient temperatures under nitrogen for 18 h, and the DMF is removed under vacuum. The resulting residue is purified by preparative HPLC on a C18 column using a water:ACN:0.1% TFA gradient. The product fraction is lyophilized to give the title compound.

### Part B - DOTA-tri-t-butyl Ester/2-(((4-(3-(N-(3-(2-(2-(3-(2-Amino-3-sulfopropyl)propoxyl)ethoxy) ethoxy) propyl) - carbamoyl)propoxy)-2,6-dimethylphenyl)sulfonyl)amino)-3-((7-((imidazol-2-ylamino)methyl)-1-methyl-4-oxo(3-hydroquinolyl))carbonylamino)propanoic Acid Tetrakis(trifluoroacetate) Salt Conjugate

The product of Part A, above, is dissolved in degassed TFA and stirred at ambient temperatures for 15 min. The solution is concentrated under vacuum, and the resulting residue is dissolved in 50% ACN and lyophilized to remove the last traces of TFA.

In a separate flask, a solution of the product of Example 2, Part B and DIEA in anhydrous DMF are treated with HBTU and allowed to react 15 min at ambient temperatures under nitrogen. The deprotected product from above is added to this solution and stirring is continued at ambient temperatures under nitrogen for 18 h. The DMF is removed under vacuum and the resulting residue is purified by preparative HPLC on a C18 column using a water:ACN:0.1% TFA gradient. The product fraction is lyophilized to give the title compound.

### Part C - DOTA/2-(((4-(3-(N-(3-(2-(2-(3-(2-Amino-3-sulfopropyl)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid Trifluoroacetate Salt Conjugate

The product of Part B, above, and Et₃SiH are dissolved in degassed TFA and heated at 50°C under nitrogen for 1 h. The solution is concentrated and the resulting residue is purified by preparative HPLC on a C18 column using a water:ACN:0.1% TFA gradient. The product fraction is lyophilized to give the title compound.

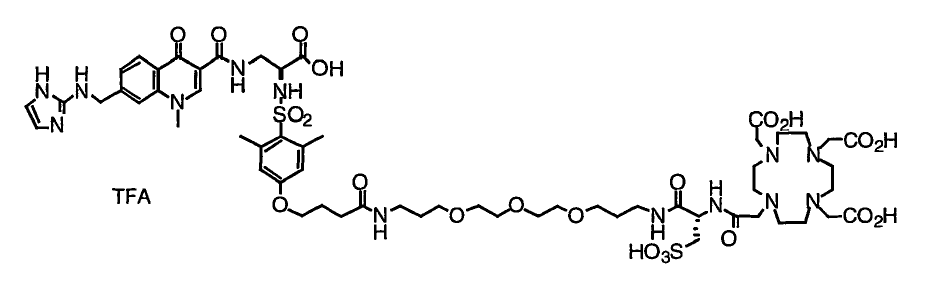

### Example 11

### DOTA/2-(((4-(3-(N-(3-(2-(2-(3-(2-Amino-3-(4-(phosphonooxy)-phenyl)propanoylamino)propoxy)ethoxy) ethoxy) propyl) carba moyl)propoxy)-2,6-dimethylphenyl)sulfonyl)amino)-3-((7-((imidazol-2-ylamino)methyl)-1-methyl-4-oxo(3-hydroquinolyl))carbonylamino)propanoic Acid Trifluoroacetate Salt Conjugate

The title compound is prepared by the same procedure described for Example 10 by substituting Boc-Tyr (PO₃H2) -OSu for Boc-Cys(O₃H)-OSu.

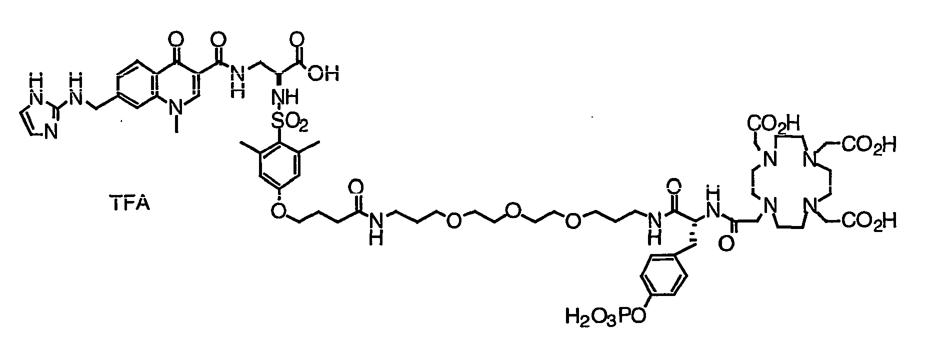

### Example 12

### DOTA/2-(((4-(3-(N-(3-(2-(2-(3-(2-Amino-3-(4-(sulfooxy)-phenyl)propanoylamino)propoxy) ethoxy) ethoxy) propyl) carba moyl)propoxy)-2,6-dimethylphenyl)sulfonyl)amino)-3-((7-((imidazol-2-ylamino)methyl)-1-methyl-4-oxo(3-hydroquinolyl))carbonylamino)propanoic Acid Trifluoroacetate Salt Conjugate

The title compound is prepared by the same procedure described for Example 10 by substituting Boc-Tyr(SO₃H)-OSu for Boc-Cys(O₃H)-OSu.

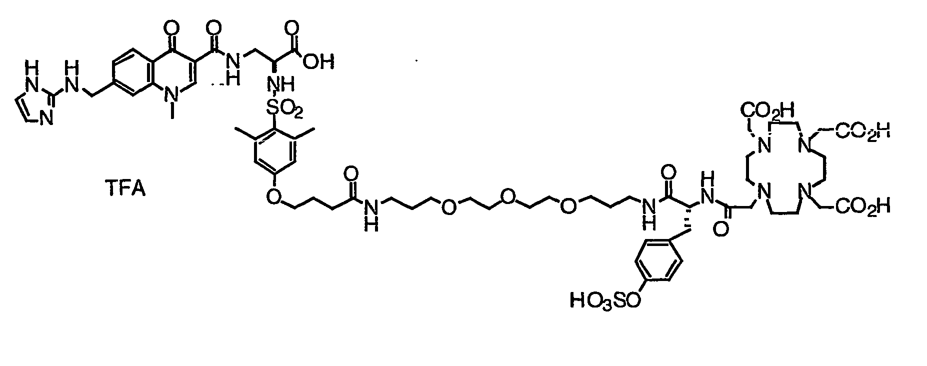

### Example 13

### DOTA/2-(((4-(3-(N-(3-(2-(2-(3-(2-Amino-4-(N-(ethyl-3,6-O-disulfo-β-D-galactopyranosyl)carbamoyl)butanoylamino)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid Conjugate

### Part A - Preparation of Boc-Glu(aminoethyl-3,6-0-disulfo-β-D-galactopyranosyl)-OSu

A solution of Boc-Glu-OMe, aminoethyl-3,6-0-disulfo-β-D-galactopyranoside (as described in Tet. Lett. 1997, 53, 11937-11952), DIEA, and HBTU in anhydrous DMF is stirred at ambient temperatures under nitrogen for 18 h. The DMF is removed under vacuum and the resulting residue is hydrolyzed using aqueous NaOH. The reaction solution is adjusted to pH 7 and purified by preparative anion exchange chromatography using a resin such as DEAE Cellulose and a Et₃NH₂CO₃ gradient. The product fraction is treated with a cation exchange resin, sodium form, to give the intermediate carboxylic acid as the sodium salt.

The above compound, N-hydroxysuccinimide, and DCC are dissolved in anhydrous DMF and stirred at ambient temperatures under nitrogen for 18 h. The DMF is removed under vacuum and the resulting residue is purified by preparative anion exchange chromatography as above to give the title compound as the triethylammonium salt.

### Part B - DOTA/2-(((4-(3-(N-(3-(2-(2-(3-(2-Amino-4-(N-(ethyl-3, 6-O-disulfo-β-D-galactopyranosyl)carbamoyl)butanoylamino)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid Conjugate

The title compound is prepared by the same procedure described for Example 10 by substituting Boc-Glu(aminoethyl-3,6-O-disulfo-β-D-galactopyranosyl)-OSu for Boc-Cys(O₃H)-OSu.

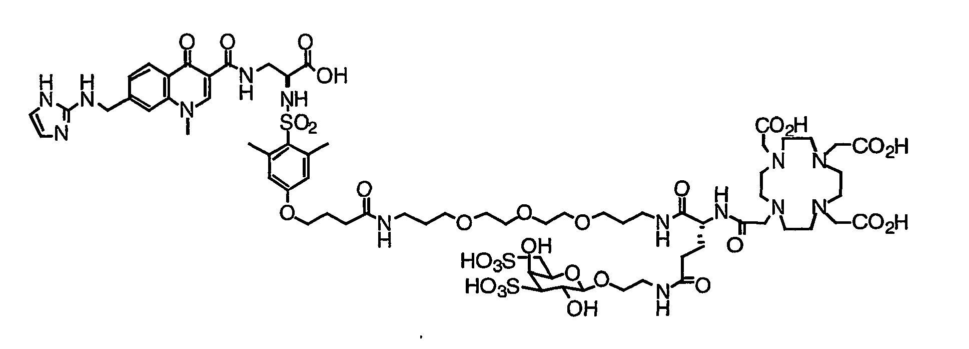

### Example 14

### DOTA/2-(((4-(3-(N-(3-(2-(2-(3-(2-Amino-4-(N-(6-deoxy-β-cyclodextryl)carbamoyl)butanoylamino)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid Bis(trifluoroacetate) Salt Conjugate

### Part A - Preparation of Boc-Glu(6-amino-6-deoxy-β-cyclodextryl)-OMe

A solution of Boc-Glu-OMe, 6-amino-6-deoxy-β-cyclodextrin (as described in J. Org. Chem. 1996, 61, 903-908), DIEA, and HBTU in anhydrous DMF is stirred at ambient temperatures under nitrogen for 18 h. The DMF is removed under vacuum and the resulting residue is purified by preparative HPLC on a C18 column using a water:ACN:0.1% TFA gradient. The product fraction is lyophilized to give the title compound.

### Part B - Preparation of Boc-Glu(6-amino-6-deoxy-β-cyclodextryl)-OSu

The product of Part A, above, is hydrolyzed by stirring in a mixture of LiOH, THF, and water at ambient temperatures under nitrogen for 4 h. The THF is removed under vacuum and the resulting mixture is diluted with water and adjusted to pH 3 using 0.1 N HCl. The mixture is extracted with EtOAc, and the combined extracts are dried (MgSO₄) and concentrated. The resulting material is dissolved in anhydrous DMF along with N-hydroxysuccinimide, and DCC, and stirred at ambient temperatures under nitrogen for 18 h. The DMF is removed under vacuum and the resulting residue is purified by preparative HPLC on a C18 column using a water:ACN:0.1% TFA gradient. The product fraction is lyophilized to give the title compound.

### Part C - DOTA/2-(((4-(3-(N-(3-(2-(2-(3-(2-Amino-4-(N-(6-deoxy-β-cyclodextryl)carbamoyl)butanoylamino)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid Bis(trifluoroacetate) Salt Conjugate

The title compound is prepared by the same procedure described for Example 10 by substituting Boc-Glu(6-amino-6-deoxy-β-cyclodextryl)-OSu for Boc-Cys(O₃H)-OSu.

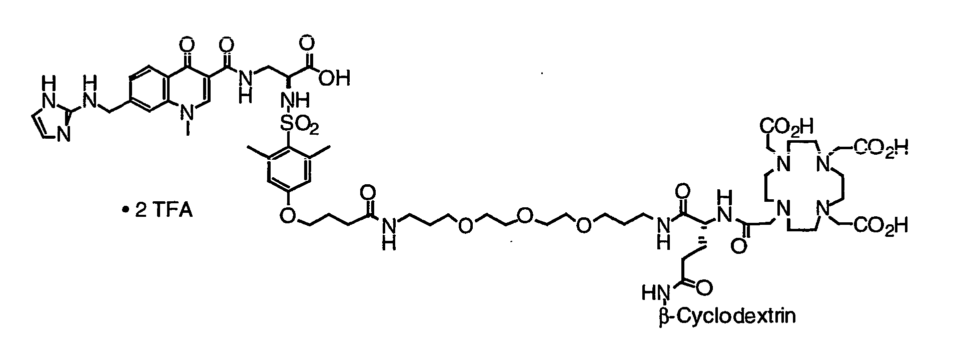

### Example 15

### DOTA/2-(((4-(3-(N-(3-(2-(2-(3-(2-Amino-4-(N-(ω-methoxypolyethylene(5,000)glycoxyethyl)carbamoyl)-butanoylamino) propoxy) ethoxy) ethoxy) propyl) carbamoyl) - propoxy)-2,6-dimethylphenyl)sulfonyl)amino)-3-((7-((imidazol-2-ylamino)methyl)-1-methyl-4-oxo(3-hydroquinolyl))carbonylamino)propanoic Acid Bis(trifluoroacetate) Salt Conjugate

### Part A - Preparation of Boc-Glu(amino-ω-methoxypolyethylene glycol)-OMe

A solution of Boc-Glu-OMe, amino-ω-methoxypolyethylene glycol, (MW = 5,000), DIEA, and HBTU in anhydrous DMF is stirred at ambient temperatures under nitrogen for 18 h. The DMF is removed under vacuum and the resulting residue is purified by preparative HPLC on a C18 column using a water:ACN:0.1% TFA gradient. The product fraction is lyophilized to give the title compound.

### Part B - Preparation of Boc-Glu(amino-ω-methoxypolyethylene glycol)-OSu

The product of Part A, above, is hydrolyzed by stirring in a mixture of LiOH, THE, and water at ambient temperatures under nitrogen for 4 h. The THF is removed under vacuum and the resulting solution is adjusted to pH 7 using 0.1 N HCl. The solution is desalted using a Sephadex PD-10 desalting column and the product eluant is lyophilized. The resulting material is dissolved in anhydrous DMF along with N-hydroxysuccinimide, and DCC, and stirred at ambient temperatures under nitrogen for 18 h. The DMF is removed under vacuum and the resulting residue is purified by preparative HPLC on a C18 column using a water:ACN:0.1% TFA gradient. The product fraction is lyophilized to give the title compound.

### Part C - DOTA/2-(((4-(3-(N-(3-(2-(2-(3-(2-Amino-4-(N-(ω-methoxypolyethylene(5,000)glycoxyethyl)carbamoyl)-butanoylamino)propoxy)ethoxy)ethoxy)propyl)carbamoyl)-propoxy)-2,6-dimethylphenyl)sulfonyl)amino)-3-((7-((imidazol-2-ylamino)methyl)-1-methyl-4-oxo(3-hydroquinolyl))carbonylamino)propanoic Acid Bis(trifluoroacetate) Salt Conjugate

The title compound is prepared by the same procedure described for Example 10 by substituting Boc-Glu(amino-ω)-methoxypolyethylene glycol)-OSu for Boc-Cys(O₃H)-OSu.

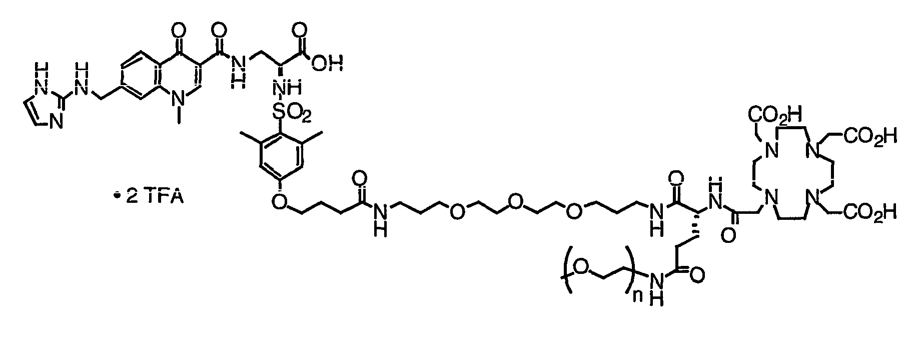

### Example 16

### 2-(((4-(3-(N-(3-(2-(2-(3-(2-(1,4,7,10-Tetraaza-4,7,10-tris(carboxymethyl)cyclododecylacetylamino)-6-aminohexanoylamino)propoxy)ethoxy)ethoxy)-propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid Tris(trifluoroacetate) Salt

The title compound is prepared by the same procedure described for Example 10 by substituting Boc-Lys(Cbz)-OSu for Boc-Cys(O₃H)-OSu.

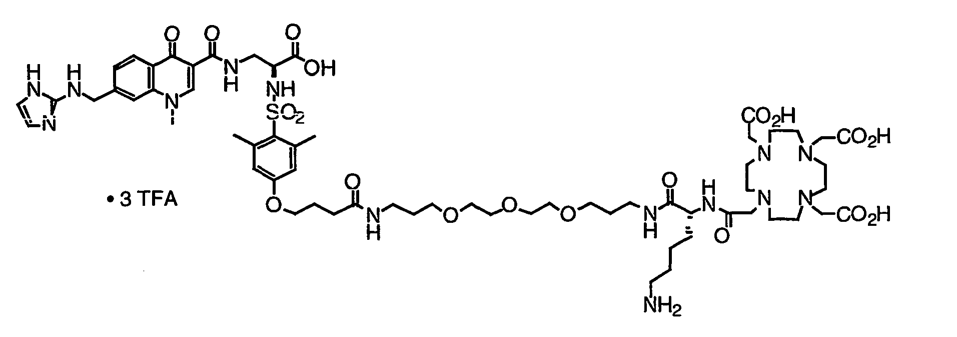

### Example 17

### 2-(((4-(3-(N-(3-(2-(2-(3-(2-(1,4,7,10-Tetraaza-4,7,10-tris(carboxymethyl)cyclododecylacetylamino)-6-(2-(bis(phosphonomethyl)amino)acetylamino)hexanoylamino)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic Acid Conjugate

A solution of bis(phosphonomethyl)glycine, DIEA, and HBTU in anhydrous DMF is stirred at ambient temperatures under nitrogen for 15 min, and treated with the product of Example 16. Stirring is continued for 18 h and the DMF is removed under vacuum. The resulting residue is purified by ion exchange chromatography.

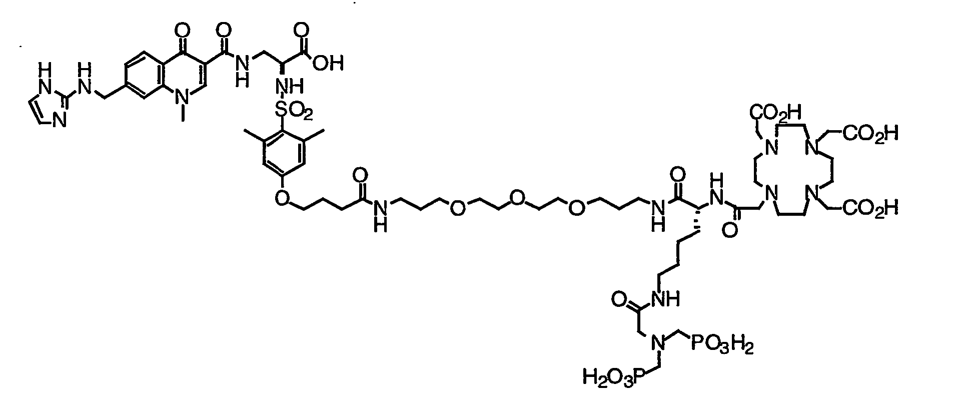

### Example 18

### 2-(((4-(3-(N-(3-(2-(2-(3-(2-(2-((2-((2-(bis(carboxymethyl)-amino)ethyl)(carboxymethyl)amino)ethyl)(carboxymethyl)am ino)acetylamino)-3-sulfopropyl)propoxy)ethoxy)-ethoxy)propyl)carbamoyl)propoxy)-2,6-dimethylphenyl)-sulfonyl)amino)-3-((7-((imidazol-2-ylamino)methyl)-1-methyl-4-oxo(3-hydroquinolyl))carbonylamino)propanoic Acid

The product of Example 10, Part A is dissolved in degassed TFA and stirred at ambient temperatures for 15 min. The solution is concentrated under vacuum, and the resulting residue is dissolved in 50% ACN and lyophilized to remove the last traces of TFA. The material is dissolved in anhydrous DMF along with DIEA and diethylenetriaminepentaacetic dianhydride. The resulting solution is stirred at ambient temperatures under nitrogen for 18 h. The DMF is removed under vacuum and the resulting residue is purified by preparative HPLC on a C18 column using a water:ACN:0.1% TFA gradient. The product fraction is lyophilized to give the title compound.

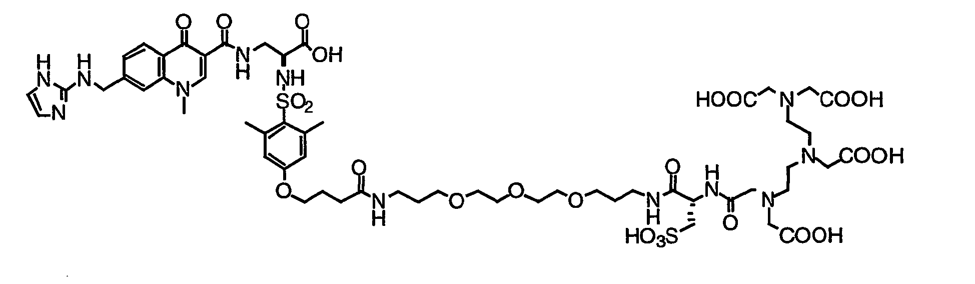

The following procedure describe the synthesis of radiopharmaceuticals of the present invention of the formula ^{99m}Tc(VnA)(tricine)(phosphine), in which (VnA) represents a vitronectin receptor antagonist compound of the present invention bonded to the Tc through a diazenido (-N=N-) or hydrazido (=N-NH-) moiety. The diazenido or hydrazido moiety results from the reaction of the hydrazinonicotinamido group, present either as the free hydrazine or protected as a hydrazone, with the Tc-99m. The other two ligands in the Tc coordination sphere are tricine and a phosphine.

### Examples 19 - 23

### Synthesis of Complexes [^{99m}Tc (HYNIC-VnA)(tricine)(TPPTS)].

To a lyophilized vial containing 4.84 mg TPPTS, 6.3 mg tricine, 40 mg mannitol, succinic acid buffer, pH 4.8, and 0.1% Pluronic F-64 surfactant, was added 1.1 mL sterile water for injection, 0.2 mL (20 µg) of the appropriate HYNIC-conjugated vitronectin antagonist (VnA) in deionized water or 50% aqueous ethanol, and 0.2 mL of ^{99m}TcO₄⁻ (50±5 mCi) in saline. The reconstituted kit was heated in a
100°C water bath for 15 minutes, and was allowed to cool 10 minutes at room temperature. A sample of the reaction mixture was analyzed by HPLC. The RCP results are listed in the Table 1.
HPLC Method
Column: Zorbax C18 , 25 cm x 4.6 mm
Flow rate,: 1.0 mL/min
Solvent A: 10 mM sodium phosphate buffer, pH 6.0
Solvent B : 100 % CH3CN

| Gradient A (Exs. 19, 20, 21) | | | | | | |
|---|---|---|---|---|---|---|
| t (min) | 0 | 20 | 21 | 30 | 31 | 40 |
| % Solvent B | 0 | 25 | 75 | 75 | 0 | 0 |
| | | | | | | |

| Gradient B (Ex. 22) | | | | | | |
|---|---|---|---|---|---|---|
| t (min) | 0 | 20 | 30 | 31 | 40 | |
| % Solvent B | 0 | 50 | 50 | 0 | 0 | |
| | | | | | | |

| Gradient C (Ex. 23) | | | | | | |
|---|---|---|---|---|---|---|
| t (min) | 0 | 20 | 21 | 30 | 31 | 40 |
| % Solvent B | 10 | 30 | 75 | 75 | 0 | 0 |

**Table 1. Analytical and Yield Data for 99^{m}Tc(VnA)(tricine)(TPPTS) Complexes**

| Example No. | Reagent No. | Ret. Time (min) | % Yield |
|---|---|---|---|
| 19 | 1 | 8.8 | 73 |
| 20 | 3 | 17.2 | 81 |
| 21 | 4 | 17.6 | 68 |
| 22 | 6 | 11.7 | 79 |
| 23 | 7 | 16.4 | 52 |

### Example 24

### Synthesis of the In-111 Complex of 3-((7-((Imidazol-2-ylamino)methyl)-1-methyl-4-oxo(3-hydroquinolyl))carbonylamino)-2-(((4-(4-(((3-(2-(2-(3-(2-(1,4,7,10-tetraaza-4,7,10-tris(carboxylmethyl)cyclododecyl)acetylamino)-propoxy)ethoxy)ethoxy)propyl)amino)sulfonyl)-phenyl)phenyl)sulfonyl)amino)propanoic Acid

To a lead shielded and crimped autosampler vial was added 35 µg of the conjugate of Example 2 and 1.0 mg gentisic acid, sodium salt dissolved in 70 µL ammonium acetate buffer (0.4 M, pH 4.7) followed by the addition of 2 mCi , 20 µL In-111 in 0.05 N HCl (specific activity: 17 µg/mCi). The reaction mixture was heated at 70 - 80 °C for 60 min and analyzed by HPLC and ITLC. The complex was formed in 93% yield and had a retention time of 19.6 min.
HPLC Method
Column: Zorbax Rx C18, 25 cm x 4.6 mm
Column Temperature: Ambient
Flow: 1.0 mL/min
Solvent A: 10 % Acetonitrile/0.1%TFA/H₂O
Solvent B: Acetonitrile
Detector: Sodium iodide (NaI) radiometric probe

| Gradient | | | | | | |
|---|---|---|---|---|---|---|
| t (min) | 0 | 25 | 26 | 35 | 36 | 45 |
| %B | 10 | 20 | 60 | 60 | 10 | 10 |

### Examples 25 - 26

### Synthesis of ¹⁷⁷Lu and ⁹⁰Y Complexes of 3-((7-((Imidazol-2-ylamino)methyl)-1-methyl-4-oxo(3-hydroquinolyl))carbonylamino)-2-(((4-(4-(((3-(2-(2-(3-(2-(1,4,7,10-tetraaza-4,7,10-tris(carboxylmethyl)cyclododecyl)acetylamino)-propoxy)ethoxy)ethoxy)propyl)amino)sulfonyl)-phenyl)phenyl)sulfonyl)amino)propanoic Acid.

To a clean sealed 5 mL vial was added 0.3 mL of a solution of the comjugate of Example 2 (200 µg/mL in 0.5 M ammonium acetate buffer, pH 6.9), followed by 0.05 mL of gentisic acid (sodium salt, 10 mg/mL in 0.5 M ammonium acetate buffer, pH 6.9) solution, 0.3 mL of 0.5 M ammonium acetate buffer (pH 6.9), and 0.010 mL of ¹⁷⁷LuCl₃ or ⁹⁰YCl₃ solution (1000 mCi/mL for ¹⁷⁷LuCl₃ and 500 mCi/mL for ⁹⁰YCl₃) in 0.05 N HCl. The resulting mixture was heated at 100 °C for 30 min. After cooling to room temperature, a sample of the resulting solution was analyzed by radio-HPLC and ITLC. The radiolabeling yields were = 90% (after correction for small amount of colloid) for both complex, and the retention time was 19.2 min.
HPLC Method
Column: Zorbax C18 , 25 cm x 4.6 mm
Flow rate : 1.0 mL/min
Solvent A: 0.1% TFA aqueous solution
Solvent B : 100 % CH₃CN

| | | | | | | |
|---|---|---|---|---|---|---|
| t (min) | 0 | 20 | 25 | 30 | 31 | 40 |
| % Solvent B | 10 | 25 | 60 | 60 | 10 | 10 |

The instant thin layer chromatography (ITLC) method used Gelman Sciences silica-gel strips and a 1:1 mixture of acetone and saline as eluant.

### Example 27

### Synthesis of ¹⁷⁷Lu Complex of the DOTA Conjugate of 3-((1-(3-(3-(N-(3-(2-(2-(N-(L-Asp-L-Asp)3-aminopropoxy)-ethoxy)ethoxy)propyl)carbamoyl)propanoylamino)propyl-7-((imidazole-2-ylamino) methyl)-4-oxo (3-hydroquinolyl))carbonylamino)-2-(((2,4,6-trimethylphenyl)sulfonyl)amino)propanoic Acid.

To a clean sealed 5 mL vial was added 0.5 mL of a solution of the conjugate of Example 8 (200 µg/mL in 0.5 M ammonium acetate buffer, pH 6.9), followed by 0.05 mL of gentisic acid (sodium salt, 10 mg/mL in 0,5 M ammonium acetate buffer, pH 6.9) solution, 0.25 mL of 0.5 M ammonium acetate buffer (pH 6.9), and 0.05 mL of ¹⁷⁷LuCl₃ solution (200 mCi/mL) in 0.05 N HCl. The resulting mixture was heated at 100 °C for 30 min. After cooling to room temperature, a sample of the resulting solution was analyzed by radio-HPLC and ITLC. The radiolabeling yield was 75% (after correction for colloid), and the retention time was 20 min.
HPLC Method
Column: Zorbax C18 , 25 cm x 4.6 mm
Flow rate : 1.0 mL/min
Solvent A: 10 mM phosphate buffer, pH = 6
Solvent B : 100 % CH₃CN

| | | | | | | |
|---|---|---|---|---|---|---|
| t (min) | 0 | 20 | 25 | 30 | 31 | 40 |
| % Solvent B | 0 | 20 | 50 | 50 | 0 | 0 |

### Example 28

### Synthesis of the Gadolinium Complex of 2-(((4-(3-(N-(3-(2-(2-(3-(2-(2-((2-((2-(bis(carboxymethyl)-amino) ethyl) (carboxymethyl) amino) ethyl) (carboxymethyl) am ino)acetylamino)-3-sulfopropyl)propoxy)ethoxy)-ethoxy)propyl)carbamoyl)propoxy)-2,6-dimethylphenyl)-sulfonyl)amino)-3-((7-((imidazol-2-ylamino)methyl)-1-methyl-4-oxo(3-hydroquinolyl))carbonylamino)propanoic Acid

The gadolinium complex of the conjugate of Example 18 is prepared according to the following procedure. 3-3.5 mg of the conjugate is dissolved in 2 mL 1 M ammonium acetate buffer at pH 7.0 , and one equivalent Gd(NO₃)₃ solution (0.02 M in water) is added to it. The reaction mixture is allowed to stay at room temperature for 3-5 hours and the product is isolated by HPLC. The fraction containing the complex is lyophilized and dissolved in 1 mL H₂O. The identity of the complex is confirmed by mass spectroscopy.

### Example 29

### Synthesis of (2S)-2-[({2,6-Dimethyl-4-[3-(N-{2-[3-sulfo-2-(3-sulfo-2-{2-[1,4,7,10-tetraaza-4,7,10-tris(carboxymethyl) cyclododecyl]acetylamino}propyl)propyl]ethyl}carbamoyl)-propoxy]phenyl}sulfonyl)amino]-3-({7-[(imidazol-2-ylamino)methyl]-1-methyl-4-oxo(3-hydroquinolyl)}-carbonylamino)propanoic Acid Trifluoroacetate Salt

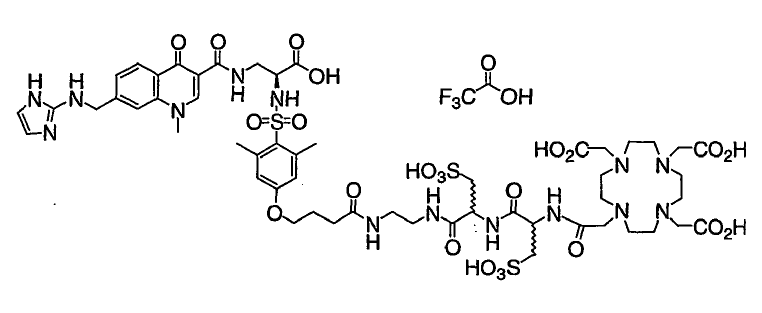

### Part A - Preparation of Methyl (2S)-3-[(tert-Butoxy)-carbonylamino]-2-[({2,6-dimethyl-4-[3-(N-{2-[(phenylmethoxy)carbonylamino]ethyl}carbamoyl)propoxy]-phenyl}sulfonyl)amino]propanoate

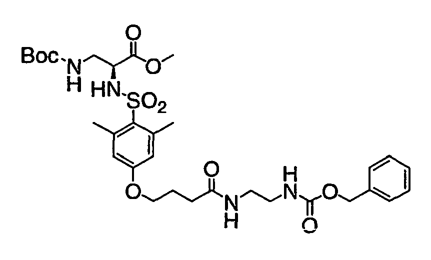

A solution of the product of Example 3, Part D (369 mg, 0.756 mmol), DIEA (0.52 mL, 3.0 mmol), and HBTU (315 mg, 0.832 mmol) in anhydrous DMF (14 mL) was stirred at ambient temperatures under nitrogen for 5 min, and treated with benzyl N-(2-aminoethyl)carbamate hydrochloride (192 mg, 0.832 mmol), and stirred an additional 1 h. The DMF was removed under vacuum, and the oily residue was taken up in EtOAc (150 mL), washed consecutively with 0.1 N HCl (40 mL)), water (40 mL), and saturated NaCl (40 mL), dried (MgSO₄), and concentrated to give a colorless viscous oil. Flash chromatography on a 3 x 16 cm silica gel column (EtOAc) gave the title compound as a colorless viscous oil (450 mg, 89.6%). ¹H NMR (CDCl₃): δ 7.34-7.27 (m, 5H), 6.58 (s, 2H), 6.31 (bs, 1H), 5.86 (bs, 1H), 5.36 (bs, 1H), 5.14-5.03 (m, 3H), 3.96 (t, J = 6.0 Hz, 2H), 3.88-3.83 (m, 1H), 3.56 (s, 3H), 3.47-3.25 (m, 6H), 2.59 (s, 6H), 2.31 (t, J = 6.9 Hz, 2H), 2.05 (p, J = 6.6 Hz, 2H), 1.39 (s, 9H); ¹³C NMR (CDCl₃): δ 172.9, 170.5, 160.6, 157.3, 155.9, 141.8, 136.3, 128.5, 128.2, 128.0, 116.6, 79.9, 66.9, 55.5, 52.8, 43.1, 40.9, 40.3, 32.4, 28.2, 24.9, 23.3; MS: m/e 665.4 [M+H]; 687.3 [M+Na]; High Resolution MS: Calcd for C₃₁H₄₅N₄O₁₀S [M+H]: 665.2856, Found: 665.2883.

### Part B - Preparation of Methyl (2S)-3-Amino-2-[({2,6-dimethyl-4-[3-(N-{2-[(phenylmethoxy)carbonylamino]ethyl} carbamoyl)propoxy]phenyl}sulfonyl)amino]propanoate Trifluoroacetate Salt

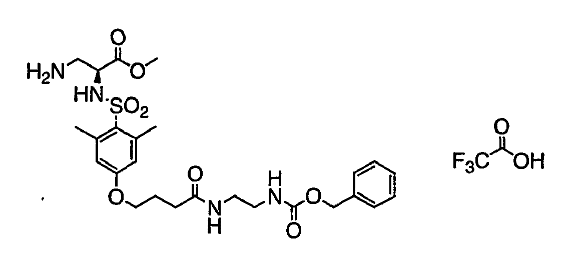

The product of Part A, above (420 mg, 0.632 mmol) was dissolved in 25/75 DCM/TFA (20 mL) and allowed to stand at ambient temperatures under nitrogen for 10 min. The solution was concentrated, and the resulting viscous oil was dissolved in 50% ACN and lyophilized to give the title compound as a colorless solid (437 mg, 102%). MS: m/e 565.3 [M+H].

### Part C - Preparation of Methyl (2S)-2-[({2,6-Dimethyl-4-[3-(N-{2-[(phenylmethoxy)carbonylamino]ethyl}carbamoyl)-propoxy]phenyl}sulfonyl)amino]-3-{[1-methyl-4-oxo-7-({[1-(triphenylmethyl)imidazol-2-yl]amino}methyl)(3-hydroquinolyl)]carbonylamino}propanoate

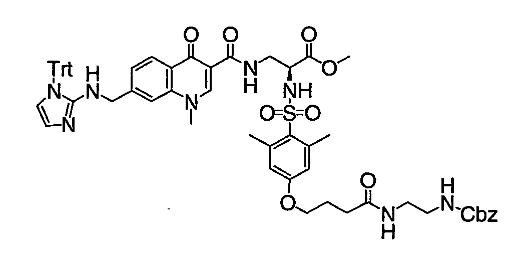

A solution of 1-methyl-4-oxo-7-(((1-(triphenylmethyl)imidazol-2-yl)amino)methyl)hydroquinoline-3-carboxylic acid (702 mg, 1.30 mmol), DIEA (0.678 mL, 3.90 mmol), and HBTU (542 mg, 1.43 mmol) in anhydrous DMF (60 mL) was stirred at ambient temperatures under nitrogen for 10 min, and treated with the product of Step B, above (881 mg, 1.30 mmol). After 75 min the DMF was removed under vacuum and the resulting oil was purified by HPLC on a Vydac C-18 column (50 x 250 mm) using a 1.24%/min gradient of 18 to 67.5% ACN containing 0.1% TFA at a flow rate of 80 mL/min. A peak eluting at 18.9 min was lyophilized to give unreacted 1-methyl-4-oxo-7-(((1-(triphenylmethyl)-imidazol-2-yl)amino)methyl)hydroquinoline-3-carboxylic acid (308 mg). The main product peak eluting at 23.7 min was lyophilized to give the title compound as a colorless solid (890 mg, 63.0%). ¹H NMR (CDCl₃/D₂O): δ 8.50 (s, 1H), 8.18 (d, J = 8.3 Hz, 1H), 7.70 (s, 1H), 7.51-7.25 (m, 15H), 7.25-7.12 (m, 5H), 6.97 (s, 1H), 6.58 (d, J = 2.3 Hz, 1H), 6.34 (s, 2H), 6.32 (d, J = 8.5 Hz, 1H), 5.09 (s, 2H), 4.65 (s, 2H), 4.29-4.23 (m, 1H), 3.88 (s, 3H), 3.80-3.50 (m, 7H), 3.41-3.28 (m, 4H), 2.61 (s, 6H), 2.26-2.11 (m, 2H), 1.92-1.76 (m, 2H); MS: m/e 1087.4 [M+H]; 845.3 [M+H-Tr]; High Resolution MS: Calcd for C₆₀H₆₃N₈O₁₀S [M+H] : 1087.4388; found: 1087.440.

### Part D - Preparation of Methyl (2S)-2-{[(4-{3-[N-(2-Aminoethyl)carbamoyl]propoxy}-2,6-dimethylphenyl)-sulfonyl]amino}-3-{[1-methyl-4-oxo-7-({[1-(triphenylmethyl)imidazol-2-yl]amino}methyl)(3-hydroquinolyl)]carbonylamino}propanoate

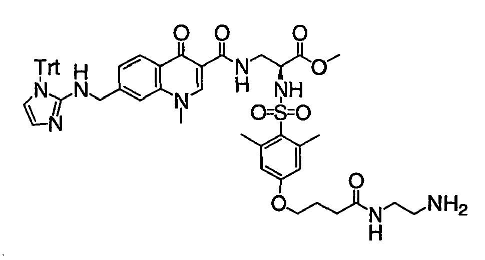

Hydrogenolysis of the product of Part C, above (468 mg, 0.431 mmol) was accomplished in MeOH (100 mL) over 10% Pd/C (95 mg) at 60 psi for 1 h. The catalyst was removed by filtration through Celite® and the filtrate was concentrated to give the title compound as a pale amber oil (405 mg, 98.7%). MS: m/e 953.3 [M+H], 711.3 [M+H-Trityl].

### Part E - Preparation of (2R)-N-{2-[4-(4-{[((1S)-1-(Methoxycarbonyl)-2-{[1-methyl-4-oxo-7-({[1-(triphenylmethyl)imidazol-2-yl]amino}methyl)(3-hydroquinolyl)]carbonylamino}ethyl)amino]sulfonyl}-3,5-dimethylphenoxy)butanoylamino]ethyl}-2-[(tert-butoxy)carbonylamino]propanesulfonic Acid

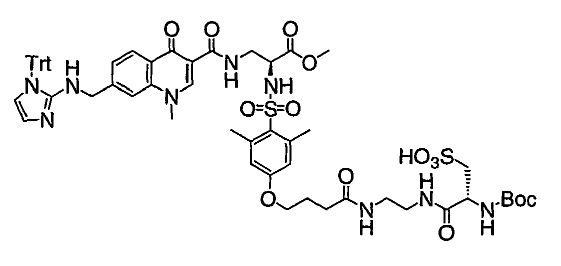

A solution of the product of Part E, above (405 mg, 0.425 mmol), the p-nitrophenyl ester of Boc-L-cysteic acid (425 mg, 1.03 mmol), and DIEA (0.435 mL, 2.55 mmol) in anhydrous DMF (20 mL) was stirred at ambient temperatures under nitrogen for 3 h. The DMF was removed under vacuum and the resulting oil was purified by HPLC on a Vydac C-18 column (50 x 250 mm) using a 1.12%/min gradient of 9 to 54% ACN containing 0.1% TFA at a flow rate of 80 mL/min. The main product peak eluting at 37.3 min was lyophilized to give the title compound as a colorless solid (410 mg, 80.2%). MS: m/e 1204.4 [M+H], 962.3 [M+H-Trt].

### Part F - Preparation of (2R)-N-{2-[4-(4-{[((1S)-1-(Methoxycarbonyl)-2-{[1-methyl-4-oxo-7-({[1-(triphenylmethyl)imidazol-2-yl]amino}methyl)(3-hydroquinolyl)]carbonylamino}ethyl)amino]sulfonyl}-3,5-dimethylphenoxy)butanoylamino]ethyl}-2-aminopropanesulfonic Acid

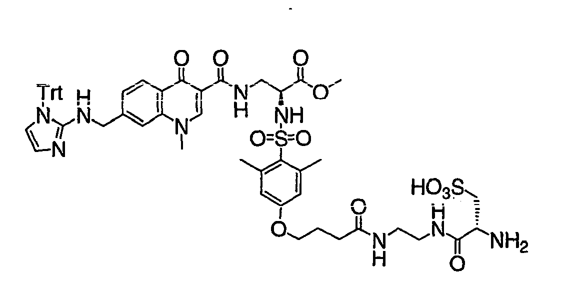

The product of Part E, above (410 mg, 0.341 mmol) was dissolved in 50/50 TFA/DCM (20 mL) and allowed to react at ambient temperatures for 10 min. The solution was concentrated and the resulting amber oil was dissolved in 50% ACN (50 mL) and lyophilized to give the title compound as a colorless solid (371 mg, 98.6%). MS: m/e 1104.4 [M+H], 862.3 [M+H-Trt]; High Resolution MS: Calcd for C₅₅H₆₂N₉O₁₂S₂ [M+H]: 1104.3959; Found: 1104.393.

### Part G - Preparation of (2R)-N-[(1R)-1-(N-{2-[4-(4-{[((1S)-1-(Methoxycarbonyl)-2-{[1-methyl-4-oxo-7-({[1-(triphenylmethyl)imidazol-2-yl]amino}methyl)(3-hydroquinolyl)]carbonylamino}ethyl)amino]sulfonyl}-3,5-dimethylphenoxy)butanoylamino]ethyl}carbamoyl)-2-sulfoethyl]-2-[(tert-butoxy)carbonylamino]propanesulfonic Acid

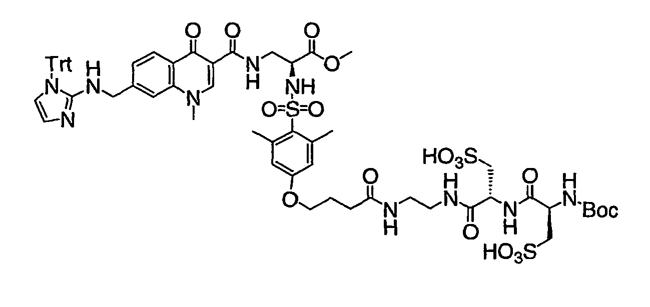

A solution of the product of Part F, above (110 mg, 0.100 mmol), the p-nitrophenyl ester of Boc-L-cysteic acid (82.4 mg, 0.200 mmol), and DIEA (0.104 mL, 0.600 mmol) in anhydrous DMF (5.0 mL) was stirred at ambient temperatures under nitrogen for 48 h. The DMF was removed under vacuum and the resulting amber oil was purified by HPLC on a Vydac C-18 column (50 x 250 mm) using a 1.12%/min gradient of 9 to 54% ACN containing 0.1% TFA at a flow rate of 80 mL/min. The main product peak eluting at 37.0 min was lyophilized to give the title compound as a colorless solid (96.0 mg, 70.9%). MS: m/e 1355.3 [M+H], 1113.3 [M-Trt+H], 1013.2 [M-Trt-Boc+H] .

### Part H - Preparation of (2R)-N-[(1R)-1-(N-{2-[4-(4-{[((1S)-1-(Methoxycarbonyl)-2-{[1-methyl-4-oxo-7-({[1-(triphenylmethyl)imidazol-2-yl]amino}methyl)(3-hydroquinolyl)]carbonylamino}ethyl)amino]sulfonyl}-3,5-dimethylphenoxy)butanoylamino]ethyl}carbamoyl)-2-sulfoethyl]-2-aminopropanesulfonic Acid

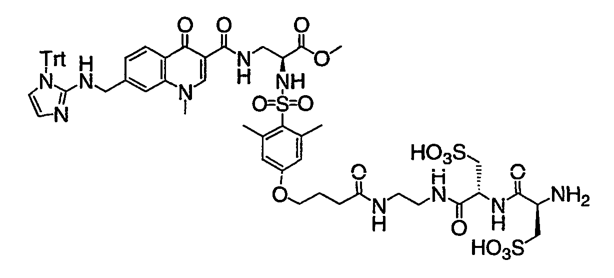

The product of Part G, above (21 mg, 0.0155 mmol) was dissolved in 50/50 TFA/DCM (5.0 mL) and allowed to react at ambient temperatures for 10 min. The solution was concentrated and the residue was taken up in 50% ACN (15 mL) and lyophilized to give the title compound as a colorless solid (18.7 mg, 96.2%). MS: m/e 1255.3 [M+H], 1013.2 [M+H-Trityl]; High Resolution MS: Calcd for C₅₈H₆₇N₁₀O₁₆S₃ [M+H]: 1255.3899; Found: 1255.391.

### Part I - Preparation of (2R)-N-[(1R)-1-(N-{2-[4-(4-{[((1S)-1-(Methoxycarbonyl)-2-{[1-methyl-4-oxo-7-({[1-(triphenylmethyl)imidazol-2-yl]amino}methyl) (3-hydroquinolyl)]carbonylamino}ethyl)amino]sulfonyl}-3,5-dimethylphenoxy)butanoylamino]ethyl}carbamoyl)-2-sulfoethyl]-2-(2-{1,4,7,10-tetraaza-4,7,10-tris[(tert-butoxycarbonyl)methyl]cyclododecyl}acetylamino) propanesulfonic Acid

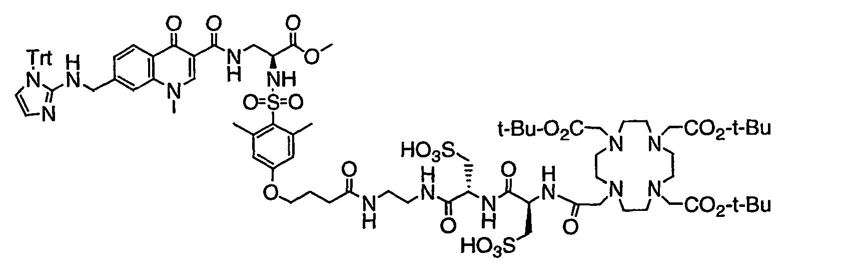

A solution of 2-(1,4,7,10-tetraaza-4,7,10-tris(((tert-butyl)oxycarbonyl)methyl)cyclododecyl)acetic acid (30.0 mg, 0.0327 mmol) (as described in DM-7003), DIEA (0.034 mL, 0.196 mmol), and HBTU (9.3 mg, 0.0245 mmol) in anhydrous DMF (1.5 mL) was stirred under nitrogen at ambient temperatures for 15 min and treated with the product of Part H, above (18.7 mg, 0.0137 mmol). The DMF was removed under vacuum after 75 min and the resulting amber oil was purified by HPLC on a Vydac C-18 column (22 x 250 mm) using a 0.9%/min gradient of 22.5 to 58.5% ACN containing 0.1% TFA at a flow rate of 20 mL/min. The main product peak eluting at 26.1 min was lyophilized to give the title compound as acolorless fluffy solid (7.5 mg, 53%). MS: m/e 1809.7 [M+H].

### Part J - Preparation of (2S)-2-[({2,6-Dimethyl-4-[3-(N-{2-[3-sulfo-2-(3-sulfo-2-{2-[1,4,7,10-tetraaza-4,7,10-tris(carboxymethyl)cyclododecyl]acetylamino}propyl)-propyl]ethyl}carbamoyl)propoxy]phenyl}sulfonyl)amino]-3-({7-[(imidazol-2-ylamino)methyl]-1-methyl-4-oxo(3-hydroquinolyl)}carbonylamino)propanoic Acid Trifluoroacetate Salt

The product of Step I, above (7.5 mg, 0.0039 mmol) was dissolved in a solution of peroxide-free THF (1.40 mL) and water (0.21 mL), and treated with 3 N LiOH (0.14 mL). The mixture was stirred at ambient temperatures under nitrogen for 1 h, and concentrated to dryness under vacuum. The resulting solid residue was dissolved in 95/5 TFA/Et₃SiH (2.0 mL) and heated at 70 °C under nitrogen for 1 h. The solution was concentrated under vacuum and the resulting solid residue was purified by HPLC on a Vydac C-18 column (22 x 250 mm) using a 0.90%/min gradient of 0 to 27% ACN containing 0.1% TFA at a flow rate of 20 mL/min. The main product peak eluting at 20.5 min was lyophilized to give the title compound as a colorless fluffy solid (4.2 mg, 71.9%). MS: m/e 1385.3 [M+H]; High Resolution MS: Calcd for C₅₄H₇₇N₁₄O₂₃S₃ [M+H]: 1385.4448; found: 1385.446.

### Example 30

### Synthesis of DOTA/(2S)-2-{[(4-{3-[N-(2-{2-[(4S)-4-(N-{1-[N-(2-{4-[4-({[(1S)-1-Carboxy-2-({7-[(imidazol-2-ylamino)methyl]-1-methyl-4-oxo(3-hydroquinolyl)}-carbonylamino)ethyl]amino}sulfonyl)-3,5-dimethylphenoxy]butanoylamino}ethyl)carbamoyl]-2-sulfoethyl}carbamoyl)-4-aminobutanoylamino]-3-sulfopropyl}ethyl)carbamoyl]propoxy}-2,6-dimethylphenyl)sulfonyl]amino}-3-({7-[(imidazol-2-ylamino)methyl]-1-methyl-4-oxo(3-hydroquinolyl)}-carbonylamino)propanoic Acid Conjugate Bis(trifluoroacetate) Salt

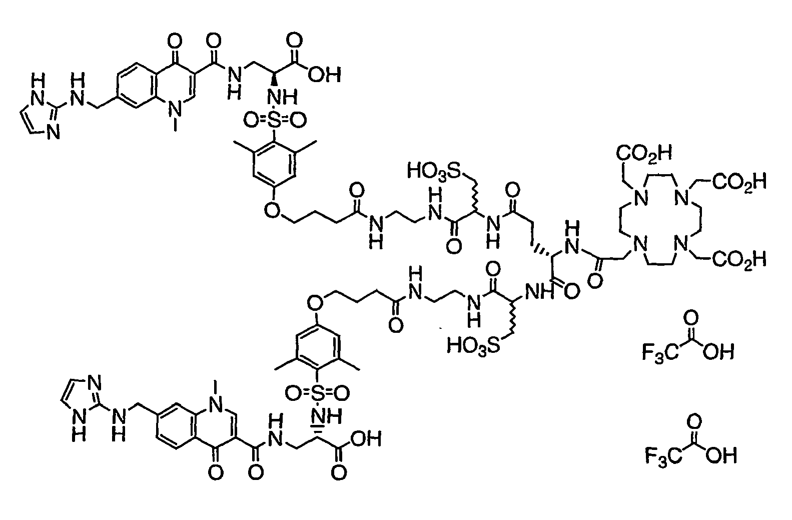

### Part A - Preparation of Di-2,3,5,6-tetrafluorophenyl (2S)-2-[(tert-Butoxy)carbonylamino]pentane-1,5-dioate

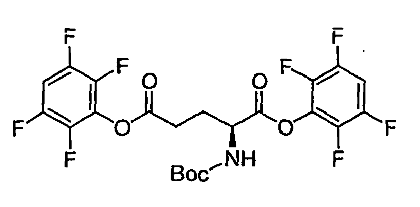

To a solution of Boc-L-Glu-OH (28.9 g, 117 mmol) in DMF (500 mL) at ambient temperatures and under nitrogen, was added a solution of 2,3,5,6-tetrafluorophenol (48.2 g, 290 mmol) in DMF (50 mL). After stirring for 10 min, EDC (55.6 g, 290 mmol) was added and the mixture was stirred for 96 h. The volatiles were removed under vacuum and the residue was triturated with 0.1 N HCl (750 mL). To this mixture was added EtOAc (600 mL) and the layers were separated. The aqueous layer was extracted with EtOAc (3 x 500 mL), and all EtOAc extracts were combined, washed consecutively with water (300 mL) and saturated NaCl (300 mL), dried (MgSO₃), and concentrated to give a tan solid (62 g). The tan solid was washed with ACN to give the title compound (45.5 g, 73.0%) in purified form. MS: m/e 566.0 [M+Na].

### Part B - Preparation of (2R)-2-[4-(N-{(1R)-1-[N-(2-{4-[4-({[(1S)-2-({7-[([1-(Triphenylmethyl)imidazol-2-yl]amino)methyl]-1-methyl-4-oxo(3-hydroquinolyl)}-carbonylamino)- 1-(methoxycarbonyl)ethyl]amino}sulfonyl)-3,5-dimethylphenoxy]butanoylamino}ethyl)carbamoyl]-2-sulfoethyl}carbamoyl)(4S)-4-[(tert-butoxy)carbonylamino]butanoylamino]-N-(2-{4-[4-({[(1S)-2-({7-[(imidazol-2-ylamino)methyl]-1-methyl-4-oxo(3-hydroquinolyl)}carbonylamino)-1-(methoxycarbonyl)ethyl]amino}sulfonyl)-3,5-dimethylphenoxy]butanoylamino}ethyl)propanesulfonic Acid

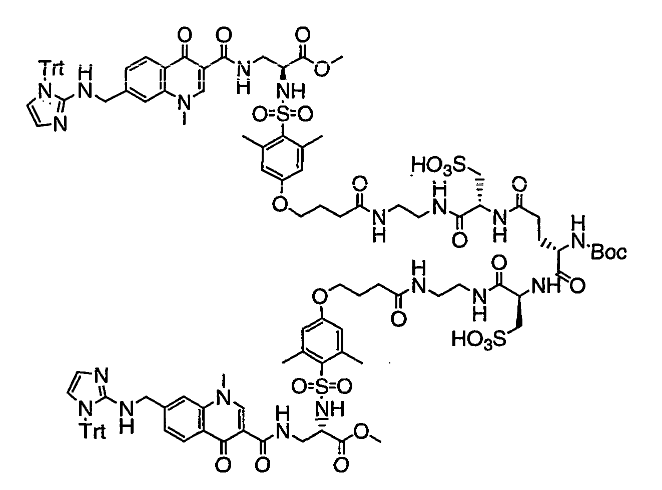

A solution of the product of Example 29, Part F (130 mg, 0.118 mmol), the product of Part A, above (27.2 mg, 0.050 mmol), and DIEA (0.070 mL, 0.40 mmol) in anhydrous DMF (4.0 mL) was stirred at ambient temperatures under nitrogen for 29 h. The DMF was removed under vacuum and the resulting amber oil was purified by HPLC on a Vydac C-18 column (50 x 250 mm) using a 0.90%/min gradient of 22.5 to 58.5% ACN containing 0.1% TFA at a flow rate of 80 mL/min. The main product peak eluting at 35.7 min was lyophilized to give the title compound as a colorless fluffy solid (108 mg, 89.3%). MS: m/e 2419.6 [M+H], 1210.4 [M+2H].

### Part C - Preparation of (2R)-2-[4-(N-{(1R)-1-[N-(2-{4-[4-({[(lS)-2-({7-[([1-(Triphenylmethyl)imidazol-2-yl]amino)methyl]-1-methyl-4-oxo(3-hydroquinolyl)}-carbonylamino)- 1-(methoxycarbonyl)ethyl]amino}sulfonyl)-3,5-dimethylphenoxy]butanoylamino}ethyl)carbamoyl]-2-sulfoethyl}carbamoyl)(4S)-4-aminobutanoylamino]-N-(2-{4-[4-({[(1S)-2-({7-[(imidazol-2-ylamino)methyl]-1-methyl-4-oxo(3-hydroquinolyl)}carbonylamino)-1-(methoxycarbonyl)ethyl]amino}sulfonyl)-3,5-dimethylphenoxy]butanoylamino}ethyl)propanesulfonic Acid

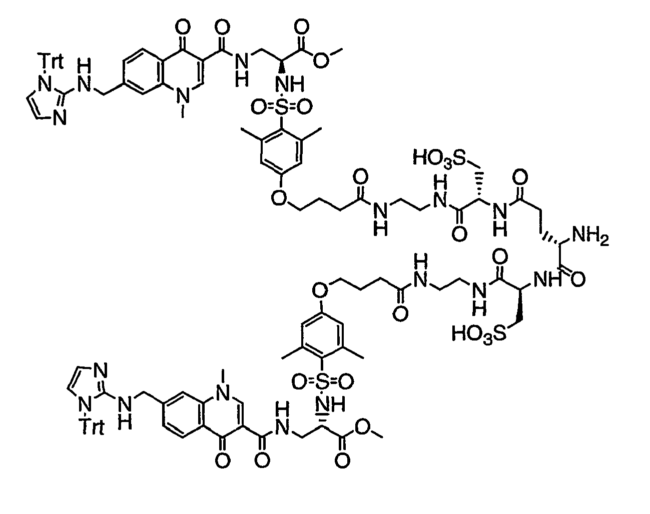

The product of Part B, above (107 mg, 0.0442 mmol) was dissolved in 50/50 TFA/DCM (5.0 mL) and allowed to react at ambient temperatures under nitrogen for 10 min. The solution was concentrated and the resulting amber oil was dissolved in 50% ACN (25 mL) and lyophilized to give the title compound as a pale yellow solid (105 mg, 98.0%). MS: m/e 1159.9 [M+2H], 1039.4 [M+2H-Trt].

### Part D - Preparation of DOTA tri-t-Butyl Ester/(2R)-2-[4-(N-{(1R)-1-[N-(2-{4-[4-({[(1S)-2-({7-[([1-(Triphenylmethyl)imidazol-2-yl]amino)methyl]-1-methyl-4-oxo(3-hydroquinolyl)}carbonylamino)-1-(methoxycarbonyl)ethyl]amino}sulfonyl)-3,5-dimethylphenoxy]butanoylamino}ethyl)carbamoyl]-2-sulfoethyl}carbamoyl)(4S)-4-aminobutanoylamino]-N-(2-{4-[4-({[(1S)-2-({7-[(imidazol-2-ylamino)methyl]-1-methyl-4-oxo(3-hydroquinolyl)}carbonylamino)-1-(methoxycarbonyl)ethyl]amino}sulfonyl)-3,5-dimethylphenoxy]butanoylamino}ethyl)propanesulfonic Acid Conjugate

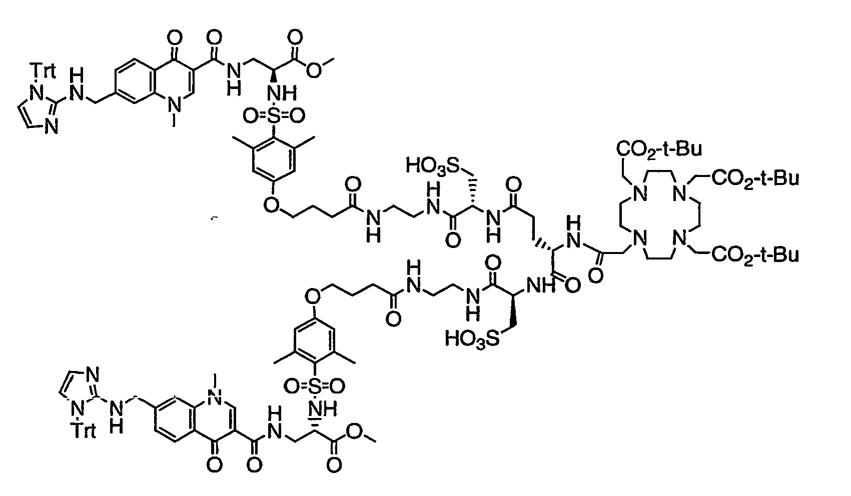

A solution of 2-(1,4,7,10-tetraaza-4,7,10-tris(((tert-butyl)oxycarbonyl)methyl)cyclododecyl)acetic acid (31.6 mg, 0.0346 mmol) (as described in DM-7003), DIEA (0.072 mL, 0.416 mmol), and HBTU (9.8 mg, 0.026 mmol) in anhydrous DMF (1.8 mL) was stirred under nitrogen at ambient temperatures for 15 min and treated with the product of Part C, above (40.0 mg, 0.0173 mmol). The DMF was removed under vacuum after 90 min and the resulting pale yellow oil was purified by HPLC on a Vydac C-18 column (50 x 250 mm) using a 1.01%/min gradient of 22.5 to 63.0% ACN containing 0.1% TFA at a flow rate of 80 mL/min. The main product peak eluting at 27.6 min was lyophilized to give the title compound as a colorless solid (29.0 mg, 62.4%). MS: m/e 1437.6 [M+2H], 1316.6 [M+2H-Trt].

### Part E - Preparation of DOTA/(2S)-2-{[(4-{3-[N-(2-{2-[(4S)-4-(N-{1-[N-(2-{4-[4-({[(1S)-1-Carboxy-2-({7-[(imidazol-2-ylamino)methyl]-1-methyl-4-oxo(3-hydroquinolyl)}carbonylamino)ethyl]amino}sulfonyl)-3,5-dimethylphenoxy]butanoylamino}ethyl)carbamoyl]-2-sulfoethyl}carbamoyl)-4-aminobutanoylamino]-3-sulfopropyl}ethyl)carbamoyl]propoxy}-2,6-dimethylphenyl)sulfonyl]amino}-3-({7-[(imidazol-2-ylamino)methyl]-1-methyl-4-oxo(3-hydroquinolyl)}-carbonylamino)propanoic Acid Conjugate Bis(trifluoroacetate) Salt

A mixture of the product of Part D, above (30.0 mg, 0.0104 mmol), peroxide-free THF (3.2 mL), water (0.485 mL), and 3 N LiOH (0.320 mL, 0.96 mmol) was stirred at ambient temperatures under nitrogen for 2 h. The solution was concentrated under vacuum and the resulting solid residue was dissolved in 95/5 TFA/Et₃SiH (5.0 mL). The solution was heated at 70 °C under nitrogen for 1 h and concentrated under vacuum. The resulting oily solid was purified by HPLC on a Vydac C-18 column (22 x 250 mm) using a 0.90%/min gradient of 0 to 27% ACN containing 0.1% TFA at a flow rate of 20 mL/min. The main product peak eluting at 27.8 min was lyophilized to give the title compound as a cololess fluffy solid (12.8 mg, 48.5%). MS: m/e 1096.8 [M+2H], 731.8 [M+3H]; High Resolution MS: Calcd for C₉₁H₁₂₂N₂₃O₃₃S₄ [M+H] : 2192.7458; Found: 2192.741.

### Example 31

### Synthesis of 2-[({4-[3-(N-{2-[(2R)-2-((2R)-3-Sulfo-2-{2-[1,4,7,10-tetraaza-4,7,10-tris(carboxymethyl)cyclododecyl] acetylamino}propyl)-3-sulfopropyl]ethyl}carbamoyl)propoxy]-2,6-dimethylphenyl}sulfonyl)amino](2S)-3-({7-[(imidazol-2-ylamino)methyl]-1-methyl-4-oxo(3-hydroquinolyl)}-carbonylamino)propanoic Acid Trifluoroacetate Salt

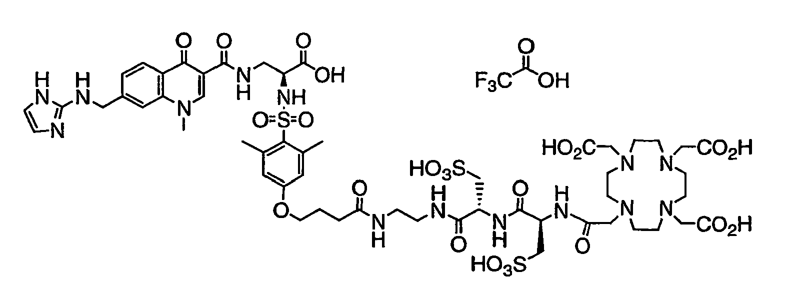

### Part A - Preparation of 2-({[4-(3-{N-[2-((2R)-2-Amino-3-sulfopropyl)ethyl]carbamoyl}propoxy)-2,6-dimethylphenyl]sulfonyl}amino)(2S)-3-{[1-methyl-4-oxo-7-({[1-(triphenylmethyl)imidazol-2-yl]amino}methyl)(3-hydroquinolyl)]carbonylamino}propanoic Acid

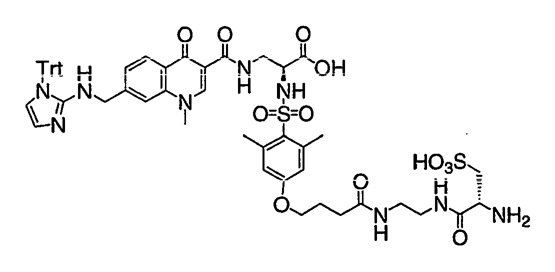

A mixture of the product of Example 29, Part F (125 mg, 0.113 mmol), peroxide-free THF (3.8 mL), water (0.57 mL), and 3 N LiOH (0.38 mL, 1.13 mmol) was stirred at ambient temperatures under nitrogen for 1 h. The mixture was adjusted to pH 1 using 1 N HCl (0.70 mL) and concentrated to dryness under vacuum. The resulting solid was purified by HPLC on a Vydac C-18 column (50 x 250 mm) using a 0.90%/min gradient of 18 to 54% ACN containing 0.1% TFA at a flow rate of 80 mL/min. The main product peak eluting at 21.0 min was lyophilized to give the title compound as a colorless solid (96.0 mg, 77.9%). MS: m/e 1090.3 [M+H], 848.2 [M+H-Trt]; High Resolution MS: Calcd for C₅₄H₆₀N₉O₁₂S₂ [M+H]: 1090.3808; Found: 1090.381.

### Part B - Preparation of 2-({[4-(3-{N-[2-((2R)-2-{(2R)-2-[(tert-Butoxy)carbonylamino]-3-sulfopropyl}-3-sulfopropyl)ethyl]carbamoyl}propoxy)-2,6-dimethylphenyl]sulfonyl}amino) (2S)-3-{[1-methyl-4-oxo-7-({[1-(triphenylmethyl)imidazol-2-yl]amino}methyl)(3-hydroquinolyl)]carbonylamino}propanoic Acid

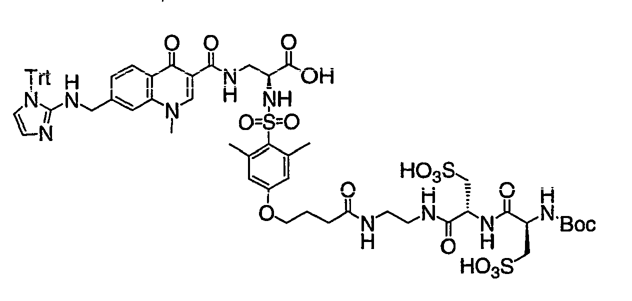

A solution of Boc-L-cysteic acid (37.0 mg, 0.128 mmol), DIEA (0.040 mL, 0.228 mmol), and PyBOP (53.0 mg, 0.102 mmol) in anhydrous DMF (1.0 mL) was stirred at ambient temperatures under nitrogen for 15 min, and added to a solution of the product of Part A, above (93.0 mg, 0.0854 mmol) and DIEA (0.045 mL, 0.256 mmol) in anhydrous DMF (3.0 mL). The resulting solution was stirred at ambient temperatures under nitrogen for 1.5 h and concentrated to a viscous amber oil. Purification by HPLC on a Vydac C-18 column (50 x 250 mm) using a 0.68%/min gradient of 18 to 45% ACN containing 0.1% TFA at a flow rate of 80 mL/min. The main product peak eluting at 36.4 min was lyophilized to give the title compound as a colorless solid (94.0 mg, 82.1%). MS: m/e 1341.2 [M+H], 1099.1 [M+H-Trt], 999.1 [M+H-Trt-Boc].

### Part C - Preparation of 2-{[(4-{3-[N-(2-{(2R)-2-[(2R)-3-Sulfo-2-(2-{1,4,7,10-tetraaza-4,7,10-tris[(tert-butoxycarbonyl)methyl]cyclododecyl}acetylamino)propyl]-3-sulfopropyl}ethyl)carbamoyl]propoxy}-2,6-dimethylphenyl)sulfonyl]amino}(2S)-3-{[1-methyl-4-oxo-7-({[1-(triphenylmethyl)imidazol-2-yl]amino}methyl)(3-hydroquinolyl)]carbonylamino}propanoic Acid

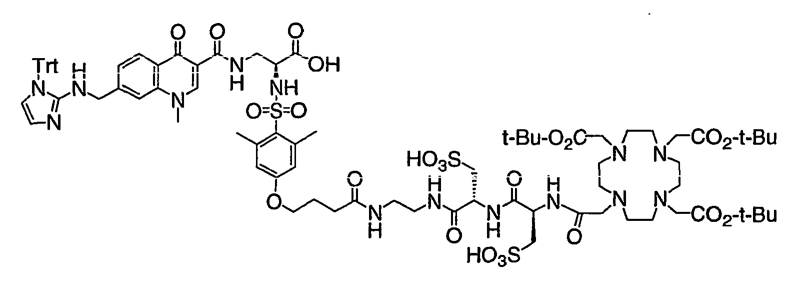

A solution of the product of Part B, above (90.0 mg, 0.0672 mmol) in 50/50 TFA/DCM (10.0 mL) was allowed to react at ambient temperatures under nitrogen for 10 min and concentrated under vacuum to give the intermediate amine as an amber oil. MS: m/e 1241.3 [M+H], 999.3 [M+H-Trt]; High Resolution MS: Calcd for C₅₇H₆₅N₁₀O₁₆S₃ [M+H]: 1241.3742; Found: 1241.375.

A solution of 2-(1,4,7,10-tetraaza-4,7,10-tris(((tert-butyl)oxycarbonyl)methyl)cyclododecyl)acetic acid (123 mg, 0.134 mmol) (as described in DM-7003), DIEA (0.092 mL, 0.538 mmol), and PyBOP (52.4 mg, 0.101 mmol) in anhydrous DMF (1.5 mL) was stirred under nitrogen at ambient temperatures for 15 min, and added to a solution of the free amine produced above (90.0 mg, 0.0672 mmol) and DIEA (0.046 mL, 0.269 mmol) in anhydrous DMF (1.5 mL). The DMF was removed under vacuum after 1 h and the resulting amber oil was purified by HPLC on a Vydac C-18 column (50 x 250 mm) using a 0.288%/min gradient of 30.6 to 45% ACN containing 0.1% TFA at a flow rate of 80 mL/min. The main product peak eluting at 25.8 min was lyophilized to give the title compound as a colorless solid (92.0 mg, 76.3%). MS: m/e 1795.6 [M+H], 1553.5 [M+H-Trt]; High Resolution MS: Calcd for C₈₅H₁₁₅N₁₄O₂₃S₃ [M+H]: 1795.7422; Found: 1795.744.

### Part D - Preparation of 2-[({4-[3-(N-{2-[(2R)-2-((2R)-3-Sulfo-2-{2-[1,4,7,10-tetraaza-4,7,10-tris(carboxymethyl)cyclododecyl] acetylamino}propyl)-3-sulfopropyl]ethyl}carbamoyl)propoxy]-2,6-dimethylphenyl}sulfonyl)amino](2S)-3-({7-[(imidazol-2-ylamino)methyl]-1-methyl-4-oxo(3-hydroquinolyl)}-carbonylamino)propanoic Acid Trifluoroacetate Salt

A solution of the product of Part C, above (89.0 mg, 0.0496 mmol) in 97/3 TFA/Et₃SiH (10.0 mL) was heated at 70 °C under nitrogen for 30 min and concentrated under vacuum. The resulting oily solid was purified by HPLC on a Vydac C-18 column (50 x 250 mm) using a 0.45%/min gradient of 4.5 to 22.5% ACN containing 0.1% TFA at a flow rate of 80 mL/min. The main product peak eluting at 19.5 min was lyophilized to give steochemically pure title compound as a colorless fluffy solid (65.0 mg, 87.5%). MS: m/e 1385.4 [M+H].

### Example 32

### Alternative Synthesis of Intermediate 2-({[4-(3-{N-[2-((2R)-2-Amino-3-sulfopropyl)ethyl]carbamoyl}propoxy)-2,6-dimethylphenyl]sulfonyl}amino)(2S)-3-{[1-methyl-4-oxo-7-({[1-(triphenylmethyl)imidazol-2-yl]amino}methyl)(3 - hydroquinolyl)]carbonylamino}propanoic Acid

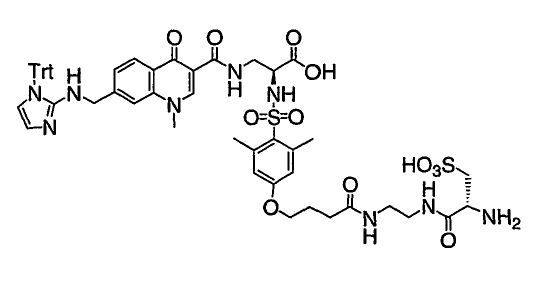

### Part A - Preparation of (2S)-2-{[(4-{3-[N-(2-Aminoethyl)carbamoyl]propoxy}-2,6-dimethylphenyl)-sulfonyl]amino}-3-{[1-methyl-4-oxo-7-({[1-(triphenylmethyl)imidazol-2-yl]amino}methyl)(3-hydroquinolyl)]carbonylamino}propanoic Acid

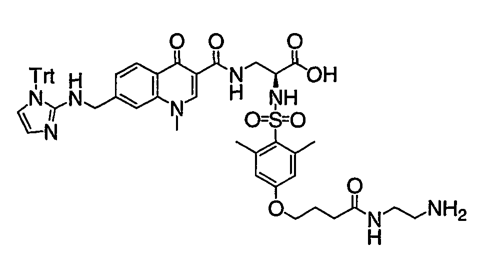

A mixture of the product of Example 29, Part D (956 mg, 1.004 mmol), peroxide-free THF (35 mL), water (5.3 mL), and 3 N LiOH (3.53 mL, 10.6 mmol) was stirred at ambient temperatures under nitrogen for 1 h, and adjusted to pH 5-6 using 1 N HCl (10 mL). The THF was removed under vacuum causing a gummy yellow solid to precipitate. The water layer was removed by decantation and the solid was washed with water (15 mL). The solid was dried under vacuum to give the title compound as a dry yellow solid

### Part B - Preparation of 2-{[(4-{3-[N-(2-{(2R)-2-[(tert-Butoxy)carbonylamino]-3-sulfopropyl}ethyl)carbamoyl]-propoxy}-2,6-dimethylphenyl)sulfonyl]amino}(2S)-3-{[1-methyl-4-oxo-7-({[1-(triphenylmethyl)imidazol-2-yl]amino}methyl)(3-hydroquinolyl)]carbonylamino}propanoic Acid

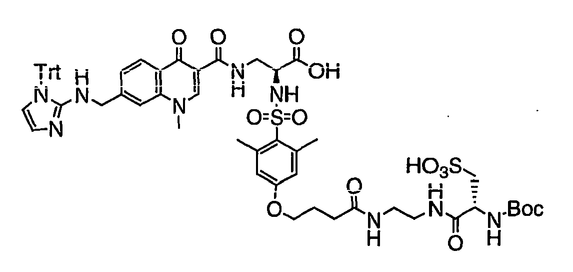

A solution of Boc-L-cysteic acid (175 mg, 0.60 mmol), DIEA (0.208 mL, 1.20 mmol), and PyBOP (250 mg, 0.480 mmol) in anhydrous DMF (5.0 mL) was stirred at ambient temperatures under nitrogen for 17 min, and added to a solution of the product of Part A, above (375 mg, 0.400 mmol) and DIEA (0.070 mL, 0.400 mmol) in anhydrous DMF (4.0 mL). The resulting solution was stirred at ambient temperatures under nitrogen for 45 min and concentrated under vacuum to give an amber oil. Purification by PLC on a Vydac C-18 column (50 x 250 mm) using a 0.292%/min gradient of 31.5 to 43.2% ACN containing 0.1% TFA at a flow rate of 80 mL/min. The main product peak eluting at 22.0 min was lyophilized to give the title compound as a colorless solid (430 mg, 90.4%). MS: m/e 1190.3 [M+H], 948.3 [M+H-Trt].

### Part C - Preparation of 2-({[4-(3-{N-[2-((2R)-2-Amino-3-sulfopropyl)ethyl]carbamoyl}propoxy)-2,6-dimethylphenyl]sulfonyl}amino)(2S)-3-{[1-methyl-4-oxo-7-({[1-(triphenylmethyl)imidazol-2-yl]amino}methyl)(3-hydroquinolyl)]carbonylamino}propanoic Acid

A solution of the product of Part B, above (430 mg, 0.362 mmol) in 50/50 TFA/DCM (15 mL) was allowed to react at ambient temperatures under nitrogen for 10 min and concentrated under vacuum. The resulting amber oil was taken up in 50% ACN (50 mL) and lyophilized to give the title compound as a pale yellow solid (398 mg, 100%). MS: m/e 1090.3 [M+H], 848.2 [M+H-Trt].

### Example 33

### Synthesis of DOTA/2-{[(4-{3-[N-(2-{(2R)-2-[(2R)-2-(4-{N-[(1R)-1-(N-{(1R)-1-[N-(2-{4-[4-({[(1S)-1-Carboxy-2-({7-[(imidazol-2-ylamino)methyl]-1-methyl-4-oxo(3-hydroquinolyl)}carbonylamino)-1-carboxyethyl]amino}-sulfonyl)-3,5-dimethylphenoxy]butanoylamino}ethyl)-carbamoyl]-2-sulfoethyl}carbamoyl)-2-sulfoethyl]carbamoyl}(2S)-2-aminobutanoylamino)-3-sulfopropyl]-3-sulfopropyl}ethyl)carbamoyl]propoxy}-2,6-dimethylphenyl)sulfonyl]amino}(2S)-3-({7-[(imidazol-2-ylamino)methyl]-1-methyl-4-oxo(3-hydroquinolyl)}-carbonylamino)propanoic Acid Conjugate

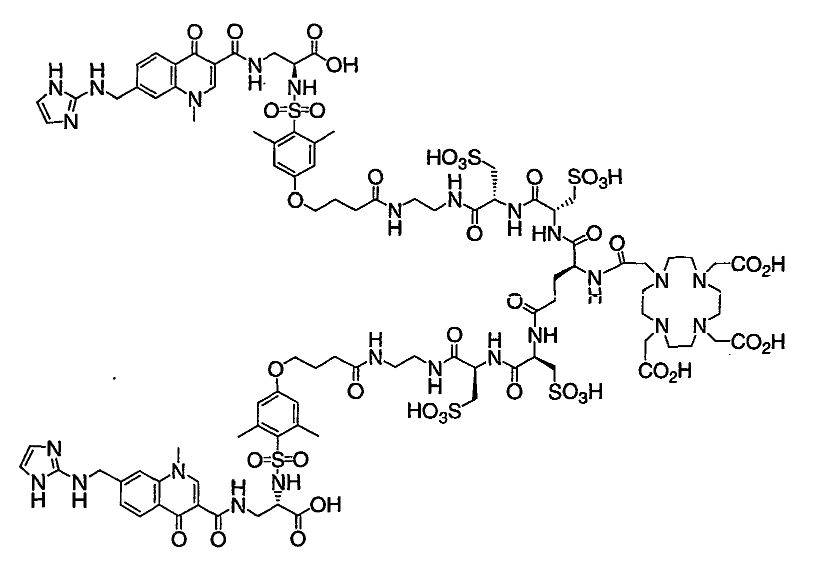

### Part A - Preparation of 2-{[(4-{3-[N-(2-{(2R)-2-[(2R)-2-(4-{N-[(1R)-1-(N-{(1R)-1-[N-(2-{4-[4-({[(1S)-1-Carboxy-2-({7-[({1-(triphenylmethyl)imidazol-2-yl}amino)methyl]-1-methyl-4-oxo(3-hydroquinolyl)}carbonylamino)-1-carboxyethyl]amino}sulfonyl)-3,5-dimethylphenoxy]butanoylamino}ethyl)carbamoyl]-2-sulfoethyl}carbamoyl)-2-sulfoethyl]carbamoyl}(2S)-2-[(tert-butoxy)carbonylamino]butanoylamino)-3-sulfopropyl]-3-sulfopropyl}ethyl)carbamoyl]propoxy}-2,6-dimethylphenyl)sulfonyl]amino}(2S)-3-({7-[({1-(triphenylmethyl)imidazol-2-yl}amino)methyl]-1-methyl-4-oxo(3-hydroquinolyl)}carbonylamino)propanoic Acid

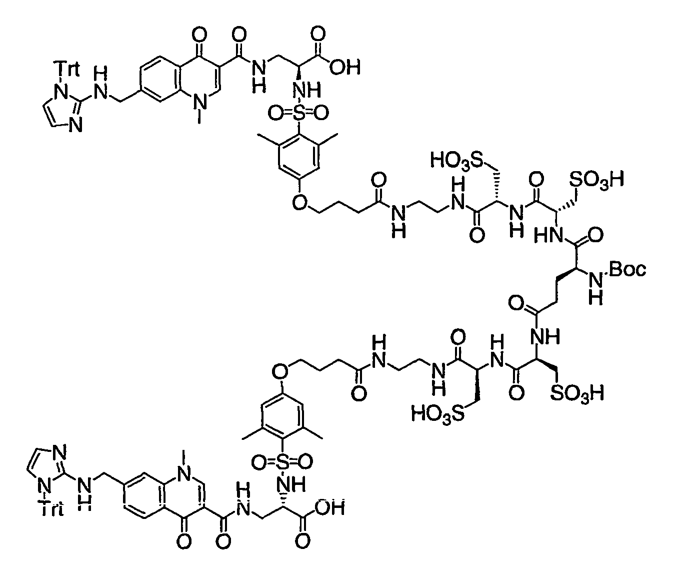

A solution of the product of the first half of Example 31, Part C (136 mg, 0.110 mmol), DIEA (0.076 mL, 0.44 mmol), and the product of Example 30, Part A (26.2 mg, 0.050 mmol) in anhydrous DMF (3.0 mL) was stirred at ambient temperatures under nitrogen for 7 h. The DMF was removed under vacuum and the viscous amber oil was purified by HPLC on a Vydac C-18 column (50 x 250 mm) using a 0.45%/min gradient of 27 to 45% ACN, followed by a 0.72% gradient of 45-63% ACN containing 0.1% TFA at a flow rate of 80 mL/min. The main product peak eluting at 75.2 min was lyophilized to give the title compound as a colorless solid (129 mg, 47.9%). MS: m/e 1347.3 [M+2H].

### Part B - Preparation of DOTA tri-t-Butyl Ester Conjugate of 2-{[(4-{3-[N-(2-{(2R)-2-[(2R)-2-(4-{N-[(1R)-1-(N-{(1R)-1-[N-(2-{4-[4-({[(1S)-1-Carboxy-2-({7-[({1-(triphenylmethyl)imidazol-2-yl}amino)methyl]-1-methyl-4-oxo(3-hydroquinolyl)}carbonylamino)-1-carboxyethyl]amino}sulfonyl)-3,5-dimethylphenoxy]butanoylamino}ethyl)carbamoyl]-2-sulfoethyl}carbamoyl)-2-sulfoethyl]carbamoyl}(2S)-2-aminobutanoylamino)-3-sulfopropyl]-3-sulfopropyl}-ethyl)carbamoyl]propoxy}-2,6-dimethylphenyl)sulfonyl]-amino}(2S)-3-({7-[({1-(triphenylmethyl)imidazol-2-yl}amino)methyl]-1-methyl-4-oxo(3-hydroquinolyl)}-carbonylamino)propanoic Acid

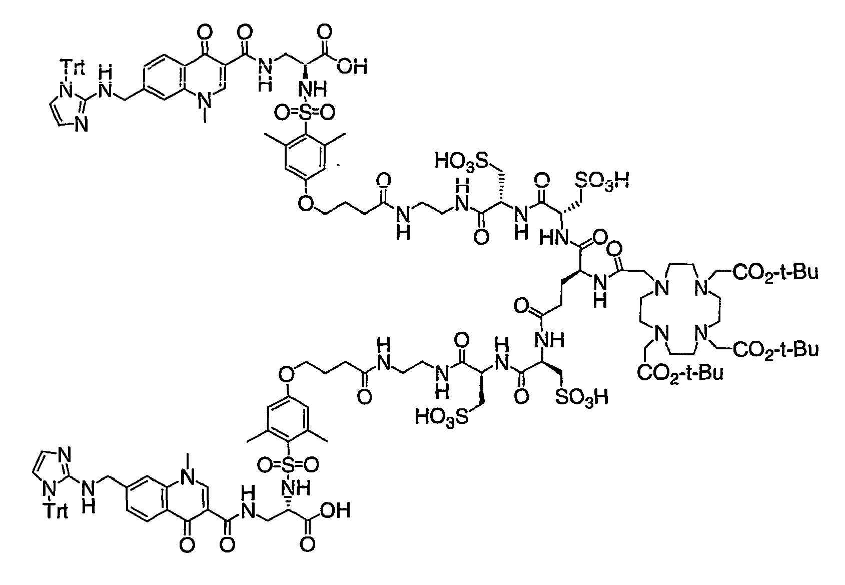

The product of Part A, above (34.0 mg, 0.0126 mmol) was dissolved in 50/50 TFA/DCM (12 mL) and allowed to react at ambient temperatures under nitrogen for 10 min. The solution was concentrated and the resulting amber oil was dried under vacuum.

A solution of 2-(1,4,7,10-tetraaza-4,7,10-tris(((tert-butyl)oxycarbonyl)methyl)cyclododecyl)acetic acid (23.1 mg, 0.0253 mmol), DIEA (0.020 mL, 0.115 mmol), and PyBOP (9.8 mg, 0.019 mmol) in anhydrous DMF (2.0 mL) was stirred under nitrogen at ambient temperatures for 15 min, and added to a solution of the product from the deprotection reaction, above and DIEA (0.020 mL, 0.115 mmol) in anhydrous DMF (2.0 mL). The DMF was removed under vacuum after 2 h, and the resulting residue was purified by HPLC on a Vydac C-18 column (50 x 250 mm) using a 0.45%/min gradient of 27 to 49.5% ACN containing 0.1% TFA at a flow rate of 80 mL/min. The main product peak eluting at 43.8 min was lyophilized to give the title compound as a colorless solid (16.0 mg, 40.4%). MS: m/e 1574.8 [M+2H], 1453.7 [M+2H-Trt], 1332.2 [M+2H-2Trt].

### Part C - Preparation of DOTA/2-{[(4-{3-[N-(2-{(2R)-2-[(2R)-2-(4-{N-[(1R)-1-(N-{(1R)-1-[N-(2-{4-[4-({[(1S}-1-Carboxy-2-({7-[(imidazol-2-ylamino)methyl]-1-methyl-4-oxo(3-hydroquinolyl)}carbonylamino)-1-carboxyethyl]amino}sulfonyl)-3,5-dimethylphenoxy]butanoylamino}ethyl)carbamoyl]-2-sulfoethyl}carbamoyl)-2-sulfoethyl]carbamoyl}(2S)-2-aminobutanoylamino)-3-sulfopropyl]-3-sulfopropyl}-ethyl)carbamoyl]propoxy}-2,6-dimethylphenyl)sulfonyl]-amino}(2S)-3-({7-[(imidazol-2-ylamino)methyl]-1-methyl-4-oxo(3-hydroquinolyl)}carbonylamino)propanoic Acid Conjugate

The product of Part B, above (14.0 mg, 0.00445 mmol) was dissolved in 95/5 TFA/Et₃SiH (8.0 mL) and heated at 70 °C under nitrogen for 1 h. The solution was concentrated under vacuum and the resulting yellow solid was purified by HPLC on a Vydac C-18 column (22 x 250 mm) using a 0.9%/min gradient of 0 to 27% ACN containing 0.1% TFA at a flow rate of 20 mL/min. The main product peak eluting at 24.5 min was lyophilized to give the title compound as a colorless solid (8.2 mg, 73.9%). MS: m/e 1247.7 [M+2H].

### Example 34

### Synthesis of (2S)-3-{[7-[(Imidazol-2-ylamino)methyl]-4-oxo-1-(3-{2-[1,4,7,10-tetraaza-4,7,10-tris(carboxymethyl)cyclododecyl]acetylamino}propyl)(3-hydroquinolyl)]carbonylamino}-2-{[(2,4,6-trimethylphenyl)sulfonyl]amino}propanoic Acid Tris(trifluoroacetate) Salt

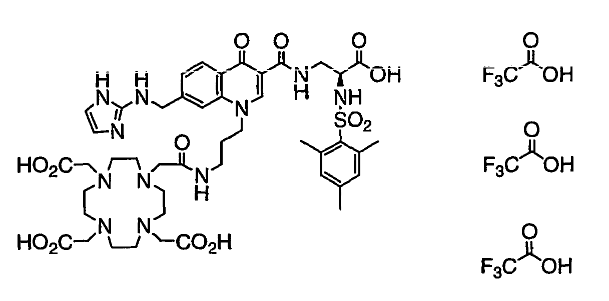

### Part A - Preparation of (2S)-3-({7-[(Imidazol-2-ylamino)methyl]-4-oxo-1-[3-(2-{1,4,7,10-tetraaza-4,7,10-tris[(tert-butoxycarbonyl)methyl]cyclododecyl}acetylamino)-propyl](3-hydroquinolyl)}carbonylamino)-2-{[(2,4,6-trimethylphenyl)sulfonyl]amino}propanoic Acid Tris(trifluoroacetate) Salt

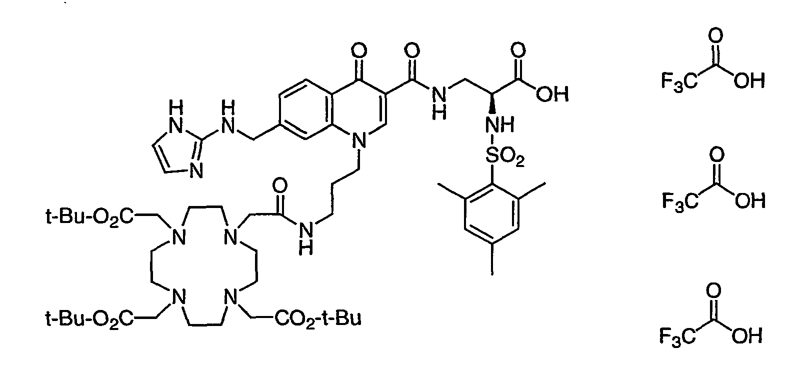

A solution of 2-(1,4,7,10-tetraaza-4,7,10-tris(((tert-butyl)oxycarbonyl)methyl)cyclododecyl)acetic acid (89 mg, 0.0974 mmol) (as described in DM-7003), DIEA (0.103 mL, 0.607 mmol), and HBTU (28.0 mg, 0.0735 mmol) in anhydrous DMF (1.0 mL) was stirred under nitrogen at ambient temperatures for 15 min and treated with a solution of the product of Example 4, Part H (30.0 mg, 0.049 mmol) in anhydrous DMF (1.0 mL). The DMF was removed under vacuum after 3 h and the residue was purified by HPLC on a Vydac C-18 column (22 x 250 mm) using a 1.08%/min gradient of 18 to 72% ACN containing 0.1% TFA at a flow rate of 20 mL/min. The main product peak eluting at 17.5 min was lyophilized to give the title compound as a colorless solid (48.0 mg, 65.0%). MS: m/e 1164.7 [M+H].

### Part B - Preparation of (2S)-3-{[7-[(Imidazol-2-ylamino)methyl]-4-oxo-1-(3-{2-[1,4,7,10-tetraaza-4,7,10-tris(carboxymethyl)cyclododecyl]acetylamino}propyl)(3-hydroquinolyl)]carbonylamino}-2-{[(2,4,6-trimethylphenyl)sulfonyl]amino}propanoic Acid Tris(trifluoroacetate) Salt

A solution of the product of Part A, above (48.0 mg, 0.0375 mmol) in 95/5 TFA/Et₃SiH (2.1 mL) was stirred at 50 °C under nitrogen for 2 h. The solution was concentrated under vacuum and the oily residue was purified by HPLC on a Vydac C-18 column (22 x 250 mm) using a 1.2%/min gradient of 0 to 36% ACN containing 0.1% TFA at a flow rate of 20 mL/min. The main product peak eluting at 18.6 min was lyophilized to give the title compound as a colorless solid (25.7 mg, 51.2%). MS: m/e 996.5 [M+H]; High Resolution MS: Calcd for C₄₅H₆₂N₁₁O₁₃S [M+H]: 996.4249; Found: 996.4278.

### Example 35

### Synthesis of 3-({1-[3-((2R)-3-Sulfo-2-{2-[1,4,7,10-tetraaza-4,7,10-tris(carboxymethyl)cyclododecyl]-acetylamino}propyl)propyl]-7-[(imidazol-2-ylamino)methyl]-4-oxo(3-hydroquinolyl)}carbonylamino)(2S)-2-{[(2,4,6-trimethylphenyl)sulfonyl]amino}propanoic Acid Bis(trifluoroacetate) Salt

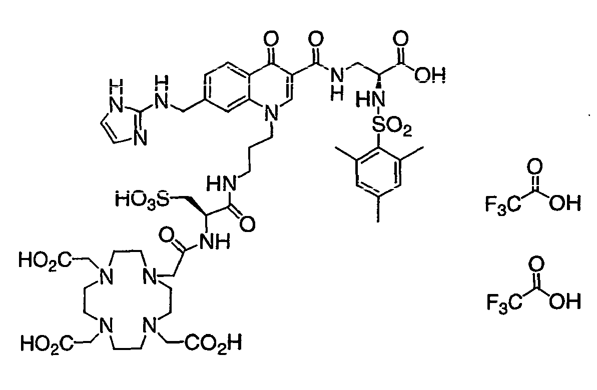

### Part A - Preparation of 3-{[1-(3-{(2R)-2-[(tert-Butoxy)carbonylamino]-3-sulfopropyl}propyl)-7-[(imidazol-2-ylamino)methyl]-4-oxo(3-hydroquinolyl)]carbonylamino}(2S)-2-{[(2,4,6-trimethylphenyl)sulfonyl]amino}propanoic Acid

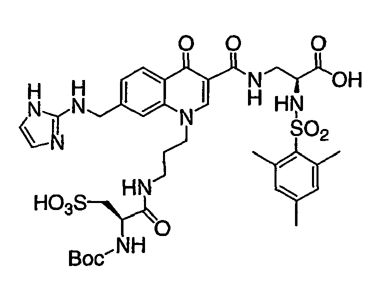

A solution of the product of Example 4, Part H (105 mg, 0.125 mmol), the N-hydroxysuccinimide ester of Boc-Cysteic acid (as described in Liebigs Ann. Chem. 1979, 776-783) (146 mg, 0.467 mmol), and DIEA (0.120 mL, 0.69 mmol) in anhydrous DMF (1.5 mL) was stirred at ambient temperatures under nitrogen for 24 h. The DMF was removed under vacuum and the resulting solid residue was purified by HPLC on a Vydac C-18 column (22 x 250 mm) using a 0.68%/min gradient of 9 to 36% ACN containing 0.1% TFA at a flow rate of 20 mL/min. The main product peak eluting at 30.3 min was lyophilized to give the title compound as a colorless solid (73.0 mg, 67.9%). MS: m/e 861.3 [M+H].

### Part B - Preparation of 3-({1-[3-((2R)-2-Amino-3-sulfopropyl)propyl]-7-[(imidazol-2-ylamino)methyl]-4-oxo(3-hydroquinolyl)}carbonylamino)(2S)-2-{[(2,4,6-trimethylphenyl)sulfonyl]amino}propanoic Acid Trifluoroacetate Salt

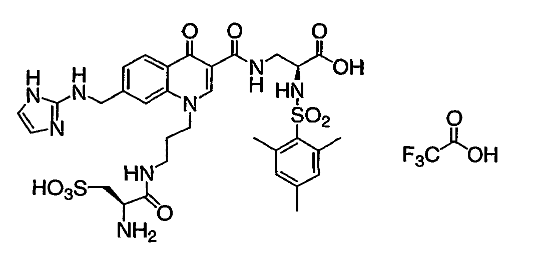

The product of Part B, above (70.0 mg, 0.0814 mmol) was dissolved in 2:1 DCM/TFA (1.5 mL) and allowed to react at ambient temperatures under nitrogen for 30 min. The solution was concentrated under vacuum and the amber oil was dissolved in 50% ACN (25 mL) and lyophilized to give the title compound as a colorless solid (70.8 mg, 99.5%). MS: m/e 761.2 [M+H] ; High Resolution MS: Calcd for C₃₂H₄₁N₈O₁₀S₂ [M+H]: 761.2387; Found: 761.2393.

### Part C - Preparation of 3-[(1-{3-[(2R)-3-Sulfo-2-(2-{1,4,7,10-tetraaza-4,7,10-tris[(tert-butoxycarbonyl)-methyl]cyclododecyl}acetylamino)propyl]propyl}-7-[(imidazol-2-ylamino)methyl]-4-oxo(3-hydroquinolyl))carbonylamino](2S)-2-{[(2,4,6-trimethylphenyl)sulfonyl]amino}propanoic Acid Bis(trifluoroacetate) Salt

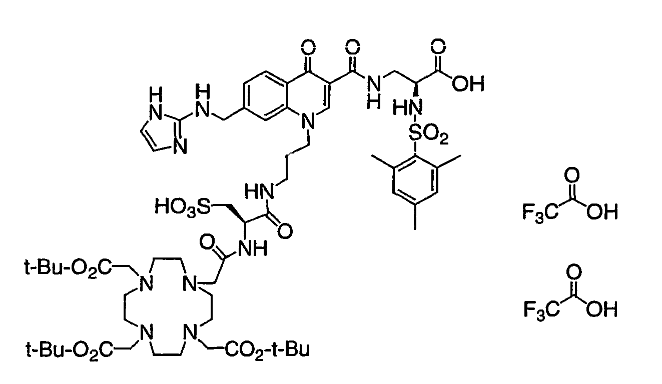

A solution of 2-(1,4,7,10-tetraaza-4,7,10-tris(((tert-butyl)oxycarbonyl)methyl)cyclododecyl)acetic acid (20.8 mg, 0.0228 mmol) (as described in DM-7003), DIEA (0.006 mL, 0.034 mmol), and HBTU (6.5 mg, 0.0171 mmol) in anhydrous DMF (0.5 mL) was stirred under nitrogen at ambient temperatures for 5 min and treated with a solution of the product of Part B, above (10.0 mg, 0.0114 mmol) and DIEA (0.006 mL, 0.034 mmol) in anhydrous DMF (0.5 mL). Stirring was continued at ambient temperatures for 24 h, and the reaction was diluted with water (3.0 mL), treated with concentrated ammonium hydroxide (0.003 mL), and stirred an additional 10 min. The solution was adjusted to pH 3 using 0.1 N HCl (6.0 mL) and diluted further with 10% ACN (5.5 mL). This solution was purified directly by HPLC on a Vydac C-18 column (22 x 250 mm) using a 0.68%/min gradient of 9 to 36% ACN containing 0.1% TFA at a flow rate of 20 mL/min. The main product peak eluting at 36.0 min was lyophilized to give the title compound as a colorless solid (12.0 mg, 68.3%). MS: m/e 1315.6 [M+H].

### Part D - Preparation of 3-({1-[3-((2R)-3-Sulfo-2-{2-[1,4,7,10-tetraaza-4,7,10-tris(carboxymethyl)cyclododecyl]-acetylamino}propyl)propyl]-7-[(imidazol-2-ylamino)methyl]-4-oxo(3-hydroquinolyl)}carbonylamino)(2S)-2-{[(2,4,6-trimethylphenyl)sulfonyl]amino}propanoic Acid Bis(trifluoroacetate) Salt

A solution of the product of Part C, above (12.0 mg, 0.00778 mmol) in 95/5 TFA/Et₃SiH (1.0 mL) was stirred at ambient temperatures under nitrogen for 18 h. The solution was concentrated under vacuum and the oily residue was purified by HPLC on a Vydac C-18 column (22 x 250 mm) using a 1.2%/min gradient of 0 to 36% ACN containing 0.1% TFA at a flow rate of 20 mL/min. The main product peak eluting at 21.1 min was lyophilized to give the title compound as a colorless solid (8.1 mg, 75.7%). MS: m/e 1147.3 [M+H] ; High Resolution MS: Calcd for C₄₈H₆₇N₁₂O₁₇S₂ [M+H]: 1147.4189 ; Found: 1147.418.

### Example 36

### Synthesis of 3-{[1-(3-{2-[(6-{[(1E)-1-Aza-2-(2-sulfophenyl)vinyl]amino}(3-pyridyl))carbonylamino] (2R)-3-sulfopropyl}propyl)-7-[(imidazol-2-ylamino)methyl]-4-oxo(3-hydroquinolyl)]carbonylamino}(2S)-2-{[(2,4,6-trimethylphenyl)sulfonyl]amino}propanoic Acid

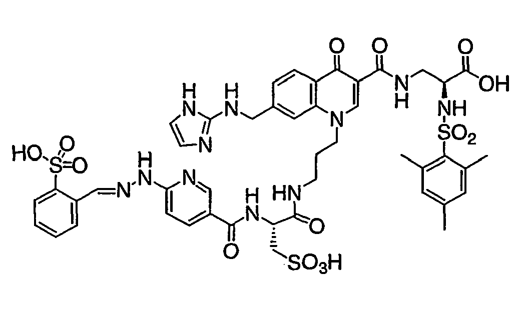

A solution of the product of Example 35, Part B (10.0 mg, 0.0101 mmol), DIEA (0.007 mL, 0.040 mmol), and 2-(2-aza-2-((5-((2,5-dioxopyrrolidinyl)carbonyl)(2-pyridyl))amino)vinyl)benzenesulfonic acid (5.3 mg, 0.0120 mmol) in anhydrous DMF (0.5 mL) was allowed to stand at ambient temperatures under a nitrogen atmosphere for 48 h. Additional 2-(2-aza-2-((5-((2,5-dioxopyrrolidinyl)carbonyl)(2-pyridyl))amino)vinyl)-benzenesulfonic acid (2.0 mg, 0.00455 mmol) was added and stirring was continued an additional 48 h. The DMF was removed under vacuum and the residue was purified by HPLC on a Vydac C-18 column (22 x 250 mm) using a 0.9%/min gradient of 0 to 36% ACN containing 0.1% TFA at a flow rate of 20 mL/min. The main product peak eluting at 30.0 min was lyophilized to give the title compound as a colorless solid (2.5 mg, 23.3%). MS: m/e 1064.3 [M+H] ; High Resolution MS: Calcd for C₄₅H₅₀N₁₁O₁₄S₃ [M+H]: 1064.27005; Found: 1064.272.

### Example 37

### Synthesis of 3-{[1-(3-{(2R)-2-[4-(N-{(1R)-1-[N-(3-{3-[N-((2S)-2-Carboxy-2-{[(2,4,6-trimethylphenyl)sulfonyl]-amino}ethyl)carbamoyl]-7-[(imidazol-2-ylamino)methyl]-4-oxohydroquinolyl}propyl)carbamoyl]-2-sulfoethyl}-carbamoyl)(2S)-2-{2-[1,4,7,10-tetraaza-4,7,10-tris(carboxymethyl)cyclododecyl]acetylamino}butanoylamin o]-3-sulfopropyl}propyl)-7-[(imidazol-2-ylamino)methyl]-4-oxo(3-hydroquinolyl)]carbonylamino}(2S)-2-{[(2,4,6-trimethylphenyl)sulfonyl]amino}propanoic Acid Bis(trifluoroacetate) Salt

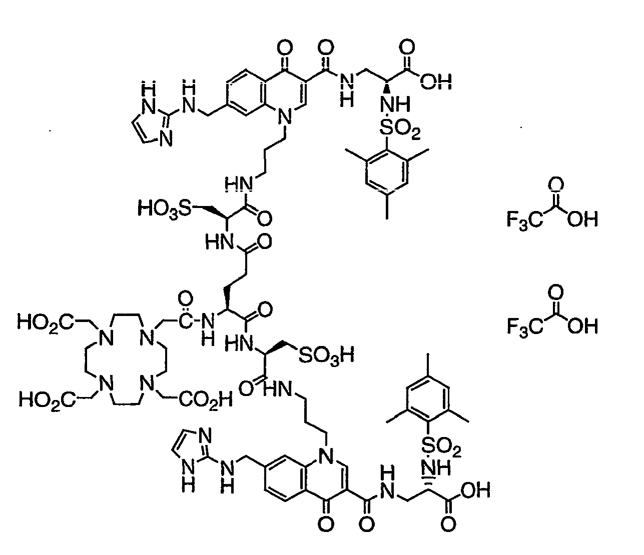

### Part A - Preparation of 3-{[1-(3-{(2R)-2-[4-(N-{(1R)-1-[N-(3-{3-[N-((2S)-2-Carboxy-2-{[(2,4,6-trimethylphenyl)-sulfonyl]amino}ethyl)carbamoyl]-7-[(imidazol-2-ylamino)methyl]-4-oxohydroquinolyl}propyl)carbamoyl]-2-sulfoethyl}carbamoyl)(2S)-2-[(tert-butoxy)carbonylamino]butanoylamino]-3-sulfopropyl}propyl)-7-[(imidazol-2-ylamino)methyl]-4-oxo(3-hydroquinolyl)]carbonylamino}(2S)-2-{[(2,4,6-trimethylphenyl)sulfonyl]amino}propanoic Acid

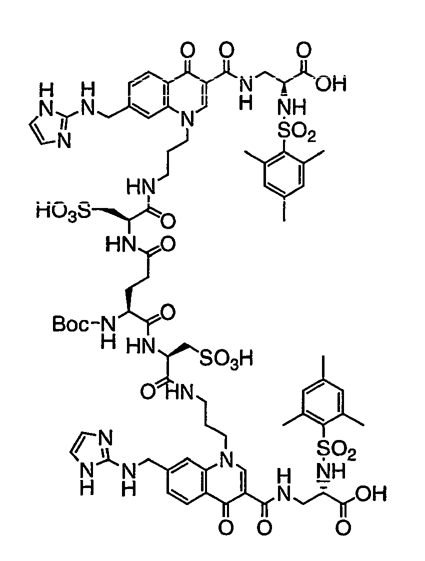

A solution of the product of Example 35, Part B (38.0 mg, 0.0434 mmol), DIEA (0.015 mL, 0.0869 mmol), and the product of Example 30, Part A (10.9 mg, 0.0202 mmol) in anhydrous DMF (1.0 mL) was stirred at ambient temperatures under nitrogen for 48 h. The DMF was removed under vacuum and the amber oil was purified by HPLC on a Vydac C-18 column (22 x 250 mm) using a 0.68%/min gradient of 9 to 36% ACN containing 0.1% TFA at a flow rate of 20 mL/min. The main product peak eluting at 36.1 min was lyophilized to give the title compound as a colorless solid (13.5 mg, 38.6%). MS: m/e 1732.4 [M+H], 1632.2 [M+H-Boc].

### Part B - Preparation of 3-{[1-(3-{(2R)-2-[4-(N-{(1R)-1-[N-(3-{3-[N-((2S)-2-Carboxy-2-{[(2,4,6-trimethylphenyl)-sulfonyl]amino}ethyl)carbamoyl]-7-[(imidazol-2-ylamino)methyl]-4-oxohydroquinolyl}propyl)carbamoyl]-2-sulfoethyl}carbamoyl)(2S)-2-aminobutanoylamino]-3-sulfopropyl}propyl)-7-[(imidazol-2-ylamino)methyl]-4-oxo(3-hydroquinolyl)]carbonylamino}(2S)-2-{[(2,4,6-trimethylphenyl)sulfonyl]amino}propanoic Acid Trifluoroacetate Salt

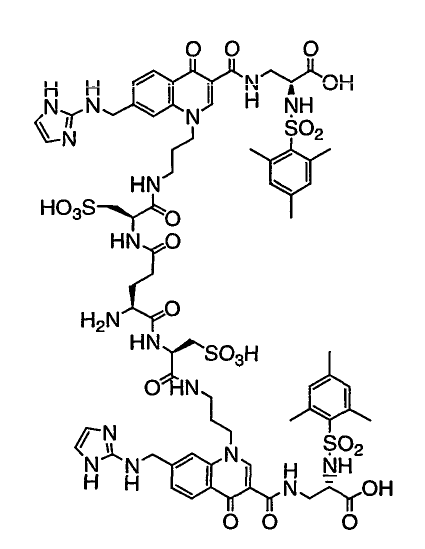

The product of Part A, above (13.5 mg, 0.00779 mmol) was dissolved in 50/50 TFA/DCM (1.0 mL) and allowed to react at ambient temperatures under nitrogen for 45 min. The solution was concentrated under vacuum to give the title compound as a pale amber oil. MS: m/e 1633.3 [M+H].

### Part C - Preparation of 3-{[1-(3-{(2R)-2-[4-(N-{(1R)-1-[N-(3-{3-[N-((2S)-2-Carboxy-2-{[(2,4,6-trimethylphenyl)-sulfonyl]amino}ethyl)carbamoyl]-7-[(imidazol-2-ylamino)methyl]-4-oxohydroquinolyl}propyl)carbamoyl]-2-sulfoethyl}carbamoyl)(2S)-2-{2-[1,4,7,10-tetraaza-4,7,10-tris[(tert-butoxycarbonyl)methyl]cyclododecyl]acetylamino}-butanoylamino]-3-sulfopropyl}propyl)-7-[(imidazol-2-ylamino)methyl]-4-oxo(3-hydroquinolyl)]carbonylamino}-(2S)-2-{[(2,4,6-trimethylphenyl)sulfonyl]amino}propanoic Acid Bis(trifluoroacetate) Salt

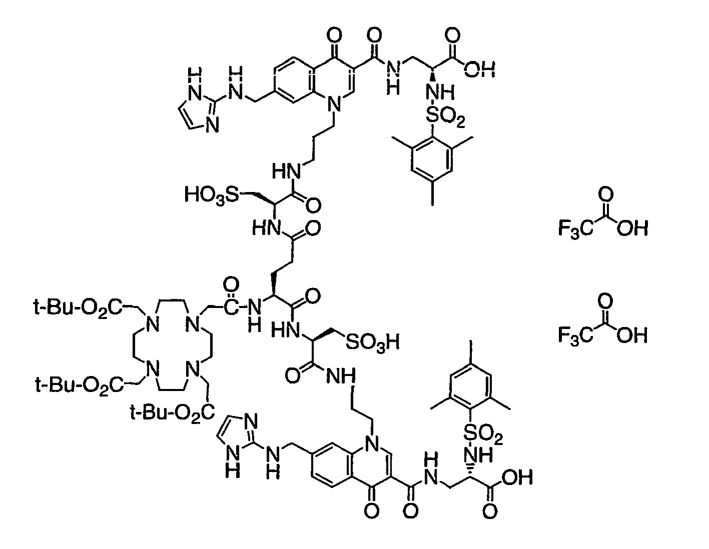

A solution of 2-(1,4,7,10-tetraaza-4,7,10-tris(((tert-butyl)oxycarbonyl)methyl)cyclododecyl)acetic acid (15.0 mg, 0.0164 mmol) (as described in DM-7003), DIEA (0.004 mL), and HBTU (4.7 mg, 0.0124 mmol) in anhydrous DMF (0.5 mL) was stirred under nitrogen at ambient temperatures for 8 min and treated with a solution of the product of Part B, above (0.00779 mmol) and DIEA (0.004 mL) in anhydrous DMF (0.5 mL). The solution was stirred at ambient temperatures for 24 h, treated with 0.1 N NaOH (0.33 mL), stirred an additional 5 min, and adjusted to pH 3 with 0.1 N HCl (0.60 mL). This solution was diluted with water (4.5 mL) and purified directly by HPLC on a Vydac C-18 column (22 x 250 mm) using a 1.01%/min gradient of 9 to 49.5% ACN containing 0.1% TFA at a flow rate of 20 mL/min. The main product peak eluting at 26.7 min was lyophilized to give the title compound as a colorless solid (7.0 mg, 37.2%). MS: m/e 1094.4 [M+2H]; High Resolution MS: Calcd for C₉₇H₁₃₆N₂₁O₂₉S₄ [M+H] : 2186.8696; Found: 2186.867.

### Part D - Preparation of 3-{[1-(3-{(2R)-2-[4-(N-{(1R)-1-[N-(3-{3-[N-((2S)-2-Carboxy-2-{[(2,4,6-trimethylphenyl)-sulfonyl]amino}ethyl)carbamoyl]-7-[(imidazol-2-ylamino)methyl]-4-oxohydroquinolyl}propyl)carbamoyl]-2-sulfoethyl}carbamoyl)(2S)-2-{2-[1,4,7,10-tetraaza-4,7,10-tris(carboxymethyl)cyclododecyl]acetylamino}butanoylamin o]-3-sulfopropyl}propyl)-7-[(imidazol-2-ylamino)methyl]-4-oxo(3-hydroquinolyl)]carbonylamino}(2S)-2-{[(2,4,6-trimethylphenyl)sulfonyl]amino}propanoic Acid Bis(trifluoroacetate) Salt

A solution of the product of Step C, above (7.0 mg, 0.00290 mmol) in 95/5 TFA/Et₃SiH (1.0 mL) was heated to reflux under nitrogen for 3 h. The solution was concentrated under vacuum and the oily residue was purified by HPLC on a Vydac C-18 column (22 x 250 mm) using a 1.2%/min gradient of 0 to 36% ACN containing 0.1% TFA at a flow rate of 20 mL/min. The main product peak eluting at 26.5 min was lyophilized to give the title compound as a colorless solid (4.5 mg, 66.1%). High Resolution MS: Calcd for C₈₅H₁₁₂N₂₁O₂₉S₄ [M+H] : 2018.6818; Found: 2018.683.

### Example 38

### Synthesis of the In-111 Complex of the Conjugate Example 29

To a shielded and crimped 2 cc autosampler vial was added 70 µg of the conjugate of Example 29 dissolved in 140 µl 0.5 M ammonium acetate buffer (pH 4.8) followed by the addition of 2 mg gentisic acid sodium salt and 2.6 mCi (7 µl) In-111 in 0.05M HCl. The reaction mixture (specific activity was heated at 85°C for 20 minutes and analyzed by HPLC. Yield: 87.9% (total for the two isomers); Ret. Time: 12.5, 13.1 min.
HPLC Method
Column: Zorbax Rx C18, 25 cm x 4.6 mm
Column Temperature: Ambient
Flow: 1.0 ml/min
Solvent A: 10 mM ammonium acetate
Solvent B: Acetonitrile
Detector: IN-US β-ram, and UV at 220 nm wavelength.

| Gradient | | | | | | |
|---|---|---|---|---|---|---|
| t (min) | 0 | 25 | 26 | 35 | 36 | 45 |
| %B | 7 | 7 | 60 | 60 | 7 | 7 |

### Example 39

### Synthesis of the In-111 Complex of the Conjugate Example 30

To a lead shielded and crimped 2 cc autosampler vial was added 120 µg of the conjugate of Example 30 dissolved in 240 µL ammonium acetate buffer (0.5 M, pH 4.7) followed by the addition of 2 mg of gentisic acid (sodium salt) dissolved in 20 µL of H₂O, and 2.3 mCi, (10 µL) In-111 (NEN) in 0.05 N HCl (specific activity: 52 µg/mCi). The reaction mixture was heated at 100 °C for 20 min and analyzed by HPLC. Yield: 94.7% (total for the two isomers), Ret. Time: 16.6 and 17.3 min.
HPLC Method
Column: Zorbax Rx C18, 25 cm x 4.6 mm
Column Temperature: Ambient
Flow: 1.0 ml/min
Solvent A: 10 mM ammonium acetate
Solvent B: Acetonitrile Detector: IN-US β-ram, and UV at 220 nm wavelength.

| Gradient | | | | | | |
|---|---|---|---|---|---|---|
| t (min) | 0 | 25 | 26 | 35 | 36 | 45 |
| %B | 10 | 15 | 60 | 60 | 10 | 10 |

### Example 40

### Synthesis of the In-111 Complex of the Conjugate Example 31

To a shielded and crimped 2 cc autosampler vial was added 70 µg of the conjugate of Example 31 dissolved in 140 µl 0.5 M ammonium acetate buffer (pH 4.8) followed by the addition of 2 mg gentisic acid sodium salt and 2.6 mCi (7 µl) In-111 in 0.05M HCl. The reaction mixture (specific activity was heated at 85°C for 20 minutes and analyzed by HPLC. Yield: 92.2%; Ret. Time: 12.9 min.
HPLC Method
Column: Zorbax Rx C18, 25 cm x 4.6 mm
Column Temperature: Ambient
Flow: 1.0 ml/min
Solvent A: 10 mM ammonium acetate
Solvent B: Acetonitrile Detector: IN-US β-ram, and UV at 220 nm wavelength.

| Gradient | | | | | | |
|---|---|---|---|---|---|---|
| t (min) | 0 | 25 | 26 | 35 | 36 | 45 |
| %B | 7 | 7 | 60 | 60 | 7 | 7 |

### Example 41

### Synthesis of the In-111 Complex of the Conjugate Example 33

To a shielded and crimped 2 cc autosampler vial was added 107 µg of the conjugate of Example 33 dissolved in 140 µl 0.5 M ammonium acetate buffer (pH 4.8) followed by the addition of 2 mg gentisic acid sodium salt and 2.6 mCi (7 µl) In-111 in 0.05M HCl. The reaction mixture (specific activity was heated at 85°C for 20 minutes and analyzed by HPLC. Yield: 77.9%; Ret. Time: 17.8 min.
HPLC Method
Column: Zorbax Rx C18, 25 cm x 4.6 mm
Column Temperature: Ambient
Flow: 1.0 ml/min
Solvent A: 10 mM ammonium acetate
Solvent B: Acetonitrile Detector: IN-US β-ram, and UV at 220 nm wavelength.

| Gradient | | | | | | |
|---|---|---|---|---|---|---|
| t (min) | 0 | 25 | 26 | 35 | 36 | 45 |
| %B | 9 | 11 | 60 | 60 | 9 | 9 |

### Example 42

### Synthesis of the In-111 Complex of the Conjugate Example 34

To a lead shielded and crimped autosampler vial was added 25 µg of the conjugate of Example 34 and 1.0 mg gentisic acid, sodium salt dissolved in 50 µL ammonium acetate buffer (0.4 M, pH 4.7) followed by the addition of 1.2 mCi, (5 µL) In-111 in 0.05 N HCl (specific activity: 21 µg/mCi). The reaction mixture was heated at 80 °C for 45 min and analyzed by HPLC and ITLC. 93.5% yield by HPLC, Ret. Time: 16.7 min.
HPLC Method
Column: Zorbax Rx C18, 25 cm x 4.6 mm
Column Temperature: Ambient
Flow: 1.0 ml/min
Solvent A: 25 mM sodium phosphate buffer at pH 6
Solvent B: Acetonitrile
Detector: Sodium iodide (NaI) radiometric probe, and UV at 220 nm wavelength.

| Gradient | | | | | | |
|---|---|---|---|---|---|---|
| t (min) | 0 | 25 | 26 | 35 | 36 | 45 |
| %B | 10 | 20 | 60 | 60 | 10 | 10 |

### Example 43

### Synthesis of the In-111 Complex of the Conjugate Example 35

To a lead shielded and crimped 1 cc autosampler vial was added 40-50 µg of the conjugate of Example 35 dissolved in 100 µL ammonium citrate buffer (0.4 M, pH 4.7) followed by the addition of 2 mCi, (5 µL) In-111 in 0.05 N HCl (specific activity: 25 µg/mCi). The reaction mixture was heated at 90-100 C for 30 min and analyzed by HPLC. Yield: 95%; Ret. Time 12.5 min.
HPLC Method
Column: Zorbax Rx C18, 25 cm x 4.6 mm
Column Temperature: Ambient
Flow: 1.0 ml/min
Solvent A: 25 mM sodium phosphate buffer at pH 6
Solvent B: Acetonitrile
Detector: Sodium iodide (NaI) radiometric probe, and UV at 220 nm wavelength.

| Gradient | | | | | | |
|---|---|---|---|---|---|---|
| t (min) | 0 | 25 | 26 | 35 | 36 | 45 |
| %B | 10 | 20 | 60 | 60 | 10 | 10 |

### Example 44

### Synthesis of the In-111 Complex of the Conjugate Example 37

To a lead shielded and crimped 2 cc autosampler vial was added 150 µg of the conjugate of Example 37 dissolved in 300 µL ammonium citrate buffer (0.3 M, pH 4.8) followed by the addition of 4.5 mCi, (25 µL) In-111 (NEN) in 0.05 N HCl (specific activity: 33 µg/mCi). The reaction mixture was heated at 100 °C for 20 min and analyzed by HPLC. RCP: 80%, Ret. Time: 21 min.
HPLC Method
Column: Zorbax Rx C18, 25 cm x 4.6 mm
Column Temperature: Ambient
Flow: 1.0 ml/min
Solvent A: 25 mM sodium phosphate buffer at pH 6
Solvent B: Acetonitrile
Detector: Sodium iodide (NaI) radiometric probe, and UV at 220 nm wavelength.

| Gradient | | | | | | |
|---|---|---|---|---|---|---|
| t (min) | 0 | 25 | 26 | 35 | 36 | 45 |
| %B | 17 | 19 | 60 | 60 | 17 | 17 |

### Examples 45 - 51

### Synthesis of Y-90 and Lu-177 Complexes of the Conjugates of Examples 30, 31, 34, 35 and 37

To a clean sealed 5 mL vial was added 0.5 -1.0 mL of the appropriate conjugate solution (200 µg/mL in 0.5 M ammonium acetate buffer, pH 7.0-8.0), followed by 0.05 mL of sodium gentisate (10 mg/mL in 0.5 M ammonium acetate buffer, pH 7.0-8.0) solution, and 10 - 40 µL of ⁹⁰YCl₃ or ¹⁷⁷LuCl₃ solution (10 - 20 mCi) in 0.05 N HCl. The reaction mixture was heated at 100 °C for 5-10 min. After cooling to room temperature, a sample of the resulting solution was analyzed by HPLC and by ITLC.

| Complex Ex # | Isotope | Conjugate Ex. # | Ret. Time (min) | % Yield | HPLC Method |
|---|---|---|---|---|---|
| 45 | Y-90 | 30 | 14.0, 16.0 | 90 | D |
| 46 | Y-90 | 31 | 14.0 | 90.5 | F |
| 47 | Lu-177 | 31 | 13.0 | 85 | D |
| 48 | Y-90 | 34 | 8.0 | 81.9 | A |
| 49 | Y-90 | 35 | 16.0 | 89 | B |
| 50 | Y-90 | 37 | 8.2 | 83.5 | B |
| 51 | Lu-177 | 37 | 14.0 | 70 | G |

HPLC Method A: The HPLC method using a reverse phase C₁₈ Zorbax column (4.6 mm x 25 cm, 80 Å pore size) at a flow rate of 1.0 mL/min with a gradient mobile phase from 85% A (25 mM pH 6.0 phosphate buffer) and 15% B (acetonitrile) to 75% A and 25% B at 20 min.
HPLC Method B: The HPLC method using a reverse phase C₁₈ Zorbax column (4.6 mm x 25 cm, 80 Å pore size) at a flow rate of 1.0 mL/min with a gradient mobile phase from 90% A (25 mM pH 6.0 phosphate buffer) and 10% B (acetonitrile) to 80% A and 20% B at 20 min.
HPLC Method D: The HPLC method using a reverse phase C₁₈ Zorbax column (4.6 mm x 25 cm, 80 Å pore size) at a flow rate of 1.0 mL/min with a gradient mobile phase from 87% A (25 mM pH 6.0 phosphate buffer) and 13% B (acetonitrile) to 86% A and 14% B at 20 min.
HPLC Method F: The HPLC method using a reverse phase C₁₈ Zorbax column (4.6 mm x 25 cm, 80 Å pore size) at a flow rate of 1.0 mL/min with a gradient mobile phase from 92% A (25 mM ammonium acetate buffer, pH = 6.8) and 8% B (acetonitrile) to 90% A and 10% B at 20 min.
HPLC Method G: The HPLC method using a reverse phase C₁₈ Zorbax column (4.6 mm x 25 cm, 80 Å pore size) at a flow rate of 1.0 mL/min with an isocratic mobile phase of 87% A (25 mM ammonium acetate buffer, pH = 6.8) and 13% B (acetonitrile) from 0 to 20 min.

### Example 52

### Synthesis of ^{99m}Tc(3-{[1-(3-{2-[(6-(diazenido) (3-pyridyl))carbonylamino](2R)-3-sulfopropyl}propyl)-7-[(imidazol-2-ylamino)methyl]-4-oxo(3-hydroquinolyl)]carbonylamino}(2S)-2-{[(2,4,6-trimethylphenyl)sulfonyl]amino}propanoic acid) (tricine)(TPPTS)

To a lyophilized vial containing 4.84 mg TPPTS, 6.3 mg tricine, 40 mg mannitol, succinic acid buffer, pH 4.8, and 0.1% Pluronic F-64 surfactant, was added 1.1 mL sterile water for injection, 0.2 mL (20 µg) of the the conjugate of Example 36 in deionized water or 50% aqueous ethanol, and 0.2 mL of ^{99m}TcO₄⁻ (50±5 mCi) in saline. The reconstituted kit was heated in a 100 °C water bath for 15 minutes, and was allowed to cool 10 minutes at room temperature. A sample of the reaction mixture was analyzed by HPLC. The yield was 89.0% and the retention time 12.8, 13.2 min (2 isomers).
HPLC Method
Column: Zorbax C18 , 25 cm x 4.6 mm
Flow rate : 1.0 mL/min
Solvent A: 10 mM sodium phosphate buffer, pH 6.0
Solvent B : 100 % CH3CN
Gradient 0 - 25% B over 20 min.

### Utility

The pharmaceuticals of the present invention are useful for imaging angiogenic tumor vasculature, therapeutic cardiovascular angiogenesis, and cardiac pathologies associated with the expression of vitronectin receptors in a patient or for treating cancer in a patient. The radiopharmaceuticals of the present invention comprised of a gamma ray or positron emitting isotope are useful for imaging of pathological processes involving angiogenic neovasculature, including cancer, diabetic retinopathy, macular degeneration, restenosis of blood vessels after angioplasty, and wound healing, as well as atherosclerotic plaque, myocardial reperfusion injury, and myocardial ischemia, stunning or infarction. The radiopharmaceuticals of the present invention comprised of a beta, alpha or Auger electron emitting isotope are useful for treatment of pathological processes involving angiogenic neovasculature, by delivering a cytotoxic dose of radiation to the locus of the angiogenic neovasculature. The treatment of cancer is affected by the systemic administration of the radiopharmaceuticals resulting in a cytotoxic radiation dose to tumors.

The compounds of the present invention comprised of one or more paramagnetic metal ions selected from gadolinium, dysprosium, iron, and manganese, are useful as contrast agents for magnetic resonance imaging (MRI) of pathological processes involving angiogenic neovasculature, as well as atherosclerotic plaque, myocardial reperfusion injury, and myocardial ischemia, stunning or infarction.

The compounds of the present invention comprised of one or more heavy atoms with atomic number of 20 or greater are useful as X-ray contrast agents for X-ray imaging of pathological processes involving angiogenic neovasculature, as well as atherosclerotic plaque, myocardial reperfusion injury, and myocardial ischemia, stunning or infarction.

The compounds of the present invention comprised of an echogenic gas containing surfactant microsphere are useful as ultrasound contrast agents for sonography of pathological processes involving angiogenic neovasculature, as well as atherosclerotic plaque, myocardial reperfusion injury, and myocardial ischemia, stunning or infarction.

Representative compounds of the present invention were tested in the following in vitro assays and in vivo models and were found to be active.

### Immobilized Human Placental avb3 Receptor Assay

The assay conditions were developed and validated using [I-125]vitronectin. Assay validation included Scatchard format analysis (n=3) where receptor number (Bmax) and Kd (affinity) were determined. Assay format is such that compounds are preliminarily screened at 10 and 100 nM final concentrations prior to IC50 determination. Three standards (vitronectin, anti-avB3 antibody, LM609, and anti-avB5, P1F6) and five reference peptides have been evaluated for IC50 determination. Briefly, the method involves immobilizing previously isolated receptors in 96 well plates and incubating overnight. The receptors were isolated from normal, fresh, non-infectious (HIV, hepatitis B and C, syphilis, and HTLV free) human placenta. The tissue was lysed and tissue debris removed via centrifugation. The lysate was filtered. The receptors were isolated by affinity chromatography using the immobilized avb3 antibody. The plates are then washed 3x with wash buffer. Blocking buffer is added and plates incubated for 120 minutes at room temperature. During this time compounds to be tested and [I-125]vitronectin are premixed in a reservoir plate. Blocking buffer is removed and compound mixture pipetted. Competition is carried out for 60 minutes at room temperature. Unbound material is then removed and wells are separated and counted via gamma scintillation.

### Oncomouse® Imaging

The study involves the use of the c-Neu Oncomouse® and FVB mice simultaneously as controls. The mice are anesthetized with sodium pentobarbital and injected with approximately 0.5 mCi of radiopharmaceutical. Prior to injection, the tumor locations on each Oncomouse® are recorded and tumor size measured using calipers. The animals are positioned on the camera head so as to image the anterior or posterior of the animals. 5 Minute dynamic images are acquired serially over 2 hours using a 256x256 matrix and a zoom of 2x. Upon completion of the study, the images are evaluated by circumscribing the tumor as the target region of interest (ROI) and a background site in the neck area below the carotid salivary glands.

This model can also be used to assess the effectiveness of the radiopharmaceuticals of the present invention comprised of a beta, alpha or Auger electron emitting isotope. The radiopharmaceuticals are administered in appropriate amounts and the uptake in the tumors can be quantified either non-invasively by imaging for those isotopes with a coincident imageable gamma emission, or by excision of the tumors and counting the amount of radioactivity present by standard techniques. The therapeutic effect of the radiopharmaceuticals can be assessed by monitoring the rate of growth of the tumors in control mice versus those in the mice administered the radiopharmaceuticals of the present invention.

This model can also be used to assess the compounds of the present invention comprised of paramagnetic metals as MRI contrast agents. After administration of the appropriate amount of the paramagnetic compounds, the whole animal can be placed in a commercially available magnetic resonance imager to image the tumors. The effectiveness of the contrast agents can be readily seen by comparison to the images obtain from animals that are not administered a contrast agent.

This model can also be used to assess the compounds of the present invention comprised of heavy atoms as X-ray contrast agents. After administration of the appropriate amount of the X-ray absorbing compounds, the whole animal can be placed in a commercially available X-ray imager to image the tumors. The effectiveness of the contrast agents can be readily seen by comparison to the images obtain from animals that are not administered a contrast agent.

This model can also be used to assess the compounds of the present invention comprised of an echogenic gas containing surfactant microsphere as ultrasound contrast agents. After administration of the appropriate amount of the echogenic compounds, the tumors in the animal can be imaging using an ultrasound probe held proximate to the tumors. The effectiveness of the contrast agents can be readily seen by comparison to the images obtain from animals that are not administered a contrast agent.

### Rabbit Matrigel Model

This model was adapted from a matrigel model intended for the study of angiogenesis in mice. Matrigel (Becton & Dickinson, USA) is a basement membrane rich in laminin, collagen IV, entactin, HSPG and other growth factors. When combined with growth factors such as bFGF [500 ng/ml] or VEGF [2 µg/ml] and injected subcutaneously into the mid-abdominal region of the mice, it solidifies into a gel and stimulates angiogenesis at the site of injection within 4-8 days. In the rabbit model, New Zealand White rabbits (2.5-3.0 kg) are injected with 2.0 ml of matrigel, plus 1 µg bFGF and 4 µg VEGF. The radiopharmaceutical is then injected 7 days later and the images obtained.

This model can also be used to assess the effectiveness of the radiopharmaceuticals of the present invention comprised of a beta, alpha or Auger electron emitting isotope. The radiopharmaceuticals are administered in appropriate amounts and the uptake at the angiogenic sites can be quantified either non-invasively by imaging for those isotopes with a coincident imageable gamma emission, or by excision of the angiogenic sites and counting the amount of radioactivity present by standard techniques. The therapeutic effect of the radiopharmaceuticals can be assessed by monitoring the rate of growth of the angiogenic sites in control rabbits versus those in the rabbits administered the radiopharmaceuticals of the present invention.

This model can also be used to assess the compounds of the present invention comprised of paramagnetic metals as MRI contrast agents. After administration of the appropriate amount of the paramagnetic compounds, the whole animal can be placed in a commercially available magnetic resonance imager to image the angiogenic sites. The effectiveness of the contrast agents can be readily seen by comparison to the images obtain from animals that are not administered a contrast agent.

This model can also be used to assess the compounds of the present invention comprised of heavy atoms as X-ray contrast agents. After administration of the appropriate amount of the X-ray absorbing compounds, the whole animal can be placed in a commercially available X-ray imager to image the angiogenic sites. The effectiveness of the contrast agents can be readily seen by comparison to the images obtain from animals that are not administered a contrast agent.

This model can also be used to assess the compounds of the present invention comprised of an echogenic gas containing surfactant microsphere as ultrasound contrast agents. After administration of the appropriate amount of the echogenic compounds, the angiogenic sites in the animal can be imaging using an ultrasound probe held proximate to the tumors. The effectiveness of the contrast agents can be readily seen by comparison to the images obtain from animals that are not administered a contrast agent.

### Canine Spontaneous Tumor Model

Adult dogs with spontaneous mammary tumors were sedated with xylazine (20 mg/kg)/atropine (1 ml/kg). Upon sedation the animals were intubated using ketamine (5 mg/kg)/diazepam (0.25 mg/kg) for full anethesia. Chemical restraint was continued with ketamine (3 mg/kg)/xylazine (6 mg/kg) titrating as necessary. If required the animals were ventilated with room air via an endotrachael tube (12 strokes/min, 25 ml/kg) during the study. Peripheral veins were catheterized using 20G I.V. catheters, one to serve as an infusion port for compound while the other for exfusion of blood samples. Heart rate and EKG were monitored using a cardiotachometer (Biotech, Grass Quincy, MA) triggered from a lead II electrocardiogram generated by limb leads. Blood samples-are generally taken at -10 minutes (control), end of infusion, (1 minute), 15 min, 30 min, 60 min, 90 min, and 120 min for whole blood cell number and counting. Radiopharmaceutical dose was 300 µCi/kg adminitered as an i.v. bolus with saline flush. Parameters were monitored continuously on a polygraph recorder (Model 7E Grass) at a paper speed of 10 mm/min or 10 mm/sec.

Imaging of the laterals were for 2 hours with a 256x256 matrix, no zoom, 5 minute dynamic images. A known source is placed in the image field (20-90 µCi) to evaluate region of interest (ROI) uptake. Images were also acquired 24 hours post injection to determine retention of the compound in the tumor. The uptake is determined by taking the fraction of the total counts in an inscribed area for ROI/source and multiplying the known µCi. The result is µCi for the ROI.

This model can also be used to assess the effectiveness of the radiopharmaceuticals of the present invention comprised of a beta, alpha or Auger electron emitting isotope. The radiopharmaceuticals are administered in appropriate amounts and the uptake in the tumors can be quantified either non-invasively by imaging for those isotopes with a coincident imageable gamma emission, or by excision of the tumors and counting the amount of radioactivity present by standard techniques. The therapeutic effect of the radiopharmaceuticals can be assessed by monitoring the size of the tumors over time. This model can also be used to assess the compounds of the present invention comprised of paramagnetic metals as MRI contrast agents. After administration of the appropriate amount of the paramagnetic compounds, the whole animal can be placed in a commercially available magnetic resonance imager to image the tumors. The effectiveness of the contrast agents can be readily seen by comparison to the images obtain from animals that are not administered a contrast agent.

This model can also be used to assess the compounds of the present invention comprised of heavy atoms as X-ray contrast agents. After administration of the appropriate amount of the X-ray absorbing compounds, the whole animal can be placed in a commercially available X-ray imager to image the tumors. The effectiveness of the contrast agents can be readily seen by comparison to the images obtain from animals that are not administered a contrast agent.

This model can also be used to assess the compounds of the present invention comprised of an echogenic gas containing surfactant microsphere as ultrasound contrast agents. After administration of the appropriate amount of the echogenic compounds, the tumors in the animal can be imaging using an ultrasound probe held proximate to the tumors. The effectiveness of the contrast agents can be readily seen by comparison to the images obtain from animals that are not administered a contrast agent.

Cardiovascular disease models that can be used to assess the diagnostic radiopharmaceuticals, magnetic resonance, X-ray and ultrasound contrast agents of the present invention are reviewed in J. Nucl. Cardiol., 1998, 5, 167-83. There are several well established rabbit models of atherosclerosis; one model produces predominantly proliferating smooth muscle cells by balloon deendothelialization of infradiaphragmatic abdominal aorta to simulate restenotic lesions; another model that produces simulated advanced human atherosclerotic plaque by balloon deendothelialization followed by a high cholesterol diet.
A model of congestive heart failure is described in Am. J. Physiol., 1998, 274, H1516-23. In general, Yorkshire pigs are randomly assigned to undergo 3 wks of rapid atrial pacing at 240 beats/min. or to be sham controls. The pigs are chronically instrumented to measure left ventricular function in the conscious state. The pigs are anesthetized. A shielded stimulating electrode is sutured onto the left atrium, connected to a modified programmable pace maker and buried in a subcutaneous pocket. The pericardium is closed loosely, the thoracotomy is closed, and the pleural space is evacuated of air. After a recovery period of 7-10 days, the pacemaker is activated in the animals selected to undergo chronic rapid pacing. The animals are sedated, the pacemaker is deactivated (pacing groups only. After a 30 min stabilization period, indexes of LV function and geometry are determined (by echocardiography as a control) by injecting the radiolabeled compound. For biodistribution, the animals are anesthetized, the heart extirpate and the LV apex and midventricular regions are evaluated.

A rat model of reversible coronary occlusion and reperfusion is described in McNulty et al., J. Am. Physiol., 1996, H2283-9.

## Claims

1. A kit for treating cancer, comprising a compound of the formula (I) and at least one agent selected from the group consisting of an anti-cancer agent and a radiosensitizer agent, or a pharmaceutically acceptable salt thereof, and a pharmaceutically acceptable carrier, wherein the compound of the formula (I) is:
(Q)_{d}-Lₙ-Cₕ or (Q)_{d}-Lₙ-(Cₕ)_{d'} (I)
wherein, Q is a compound of Formula (II): 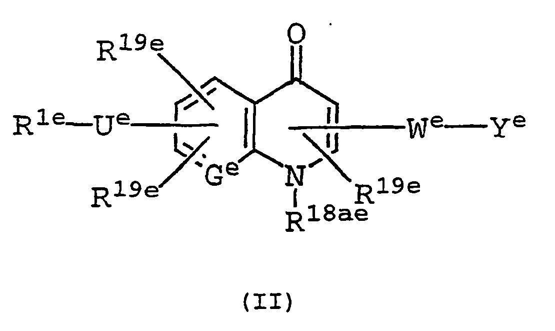 including stereoisomeric forms thereof, or mixtures of stereoisomeric forms thereof, or pharmaceutically acceptable salt or prodrug forms thereof wherein:
R^{1e} is selected from: 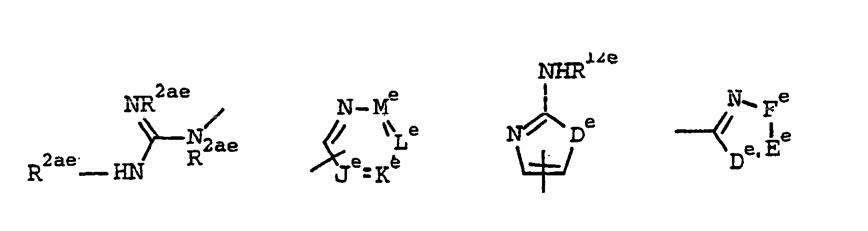 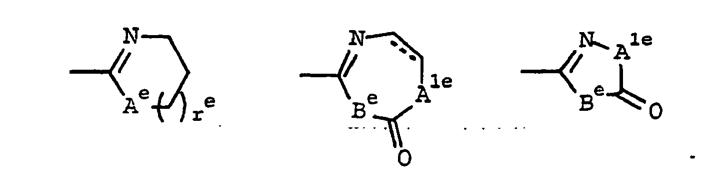 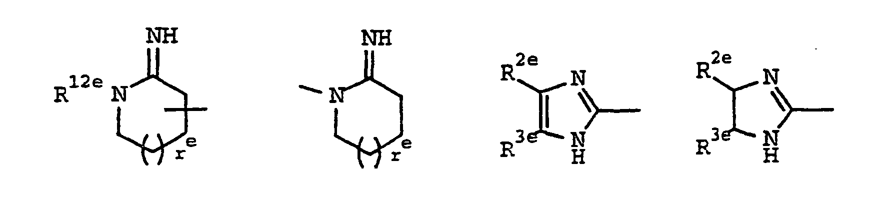 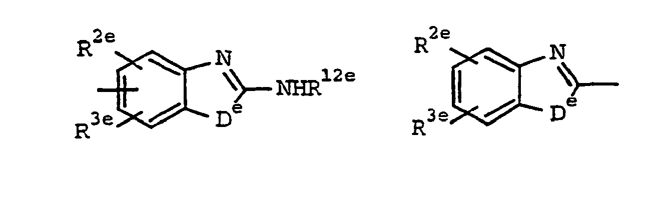 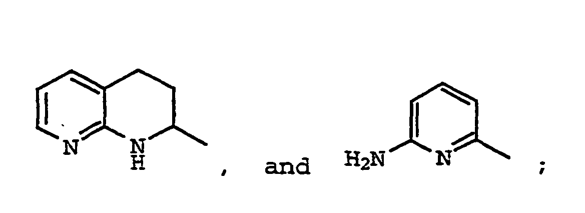
A^{e} is -CH₂- or -N(R^{10e})-;
A^{1e} and B^{e} are independently -CH₂- or -N(R^{10e})-;
D^{e} is -N(R^{10e})- or -S-;
E^{e}-F^{e} is -C(R^{2e})=C(R^{3e})- or -C(R^{2e})₂C(R^{3e})₂-;
J^{e} is -C(R^{2e})- or -N-;
K^{e}, L^{e} and M^{e} are independently -C(R^{2e})- or -C(R^{3e})-;
R^{2e} and R^{3e} are independently selected from:
H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂ ;
R^{2ae} is selected from:
H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy) carbonyl, and C₃-C₇ cycloalkylcarbonyloxy(C₁-C₄ alkoxy)carbonyl ;
R^{7e} is selected from:
H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
U^{e} is selected from:
-(CH₂)ₙ^{e}-, -(CH₂)ₙ^{e}O(CH₂)ₘ^{e}-,-(CH₂)ₙ^{e}N(R¹²)(CH₂)ₘ^{e}-, -NH(CH₂)ₙ^{e}-,-(CH₂)ₙ^{e}C(=O)(CH₂)ₘ^{e}-, -(CH₂)ₙ^{e}S(O)ₚ^{e}(CH2)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})-, and -N(R^{10e})S(O)ₚ^{e}-;
G^{e} is N or CR^{19e};
W^{e} is-C(=O)-N(R^{10e})-(C₁-C₃ alkylene)-, in which the alkylene group is substituted by R^{8e} and by R^{9e}:
R^{8e} and R^{9e} are independently selected from:
H, CO₂R^{18be}, C(=O)R^{18be}, CONR¹⁷R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e},
(C₁-C₁₀ alkyl)carbonyl,
C₃-C₁₀ cycloalkyl(C₁-C₄ alkyl)-, phenyl substituted with 0-3 R^{6e}, naphthyl substituted with 0-3 R^{6e},
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e},
C₁-C₁₀ alkoxy substituted with 0-2 R^{7e},
hydroxy, nitro, -N(R^{10e})R^{11e}, -N(R^{16e})R^{17e}, aryl(C₀-C₆ alkyl)carbonyl, aryl(C₃-C₆ alkyl), heteroaryl(C₁-C₆ alkyl), CONR^{18ae}R^{20e}, SO₂R^{18ae}, and SO₂NR^{18ae}R^{20e},
providing that any of the above alkyl, cycloalkyl, aryl or heteroaryl groups may be unsubstituted or substituted independently with 1-2 R^{7e};
R^{6e} is selected from:
H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
aryl substituted with 0-3 groups selected from halogen, C₁-C₆ alkoxy, C₁-C₆ alkyl, CF₃, S(O)ₘ^{e}Me, and -NMe₂,
aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
a 5-10 membered heterocyclic ring containing 1-3 N, 0, or S heteroatoms, wherein said heterocyclic ring may be saturated, partially saturated, or fully unsaturated, said heterocyclic ring being substituted with 0-2 R^{7e};
R^{10e} is selected from:
H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
R^{11e} is selected from:
H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
R^{4e} is selected from:
H, C₁-C₆ alkyl, C₃-C₇ cycloalkyl, C₃-C₇ cycloalkyl(C₁-C₄ alkyl)-, (C₁-C₁₀ alkyl)carbonyl, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
alternatively, when R^{10e} and R^{11e} are both substituents on the same nitrogen atom (as in -NR^{10e}R^{11e}) they may be taken together with the nitrogen atom to which they are attached to form a heterocycle selected from: 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl, and 1-piperazinyl ;
said heterocycle being substituted with 0-3 groups selected from: C₁-C₆ alkyl, aryl, heteroaryl, aryl(C₁-C₄ alkyl)-, (C₁-C₆ alkyl) carbonyl, (C₃-C₇ cycloalkyl) carbonyl, (C₁-C₆ alkoxy)carbonyl, aryl(C₁-C₄ alkoxy)carbonyl, C₁-C₆ alkylsulfonyl, and arylsulfonyl ;
R^{12e} is selected from:
H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy)carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
R^{16e} is selected from:
-C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, and -SO₂NHC(=O)OR^{18be};
R^{17e} is selected from:
H, C₁-C₆ alkyl, C₃-C₇ cycloalkyl, C₃-C₇ cycloalkyl (C₁-C₄ alkyl)-, aryl, aryl(C₁-C₆ alkyl)-, and heteroaryl (C₁-C₆ alkyl);
R^{18ae} is selected from:
C₁-C₈ alkyl optionally substituted with a bond to Ln, C₃-C₁₁ cycloalkyl optionally substituted with a bond to Lₙ, aryl(C₁-C₆ alkyl)-optionally substituted with a bond to Lₙ, heteroaryl (C₁-C₆ alkyl)- optionally substituted with a bond to Lₙ, (C₁-C₆ alkyl)heteroaryl optionally substituted with a bond to Lₙ, biaryl (C₁-C₆ alkyl) optionally substituted with a bond to Lₙ, biaryl optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
R^{18be} is H or R^{18ae};
R^{19e} is selected from:
H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
R^{20e} is selected from:
hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy (C₁-C₂ alkyl) oxy-, C₂-C₁₀ alkoxycarbonyl (C₁-C₂ alkyl) oxy-, C₃-C₁₀ cycloalkylcarbonyloxy (C₁-C₂ alkyl) oxy-, C₃-C₁₀ cycloalkoxycarbonyloxy (C₁-C₂ alkyl)oxy-, C₃-C₁₀ cycloalkoxycarbonyl (C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-_{C5} alkyl) carbonyloxy (C₁-C₂ alkyl)ox y,
(5-(C₁-C₅ alkyl)-1,3-dioxa-cyclopenten-2-one-yl)methyloxy,
(5-aryl-1,3-dioxa-cyclopenten-2-one-yl)methyloxy, and
(R^{10e})(R^{11e})N-(C₁-C₁₀ alkoxy)-;
R^{21e} is selected from:
C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and C₁-C₁₀ alkyl substituted with 0-2 R^{7e};
R^{22e} is selected from:
-C(=O)-R^{18be}, -C(=O)N(R^{18be})₂, -C(=O)NHSO₂R^{18ae},-C(=O)NHC(=O)R^{18be}, -C(=O)NHC(=O)OR^{18ae}, and-C(=O)NHSO₂NHR^{18be};
Y^{e} is selected from:
-COR^{20e}, -SO₃H, -PO₃H, -CONHNHSO₂CF₃,-CONHSO₂R^{18ae}, -CONHSO₂NHR^{18be}, -NHCOCF₃,-NHCONHSO₂R^{18ae}, -NHSO₂R^{18ae}, -OPO₃H₂, -OSO₃H,-PO₃H₂, -SO₂NHCOR^{18ae}, -SO₂NHCO₂R^{18ae}, 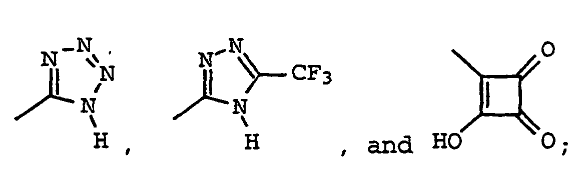
m^{e} is 0-2;
n^{e} is 0-4;
p^{e} is 0-2;
r^{e} is 0-2;
with the following proviso: n^{e} and m^{e} are chosen such that the number of atoms connecting R^{1e} and Y^{e} is in the range of 8-14;
d is selected from 1, 2, 3, 4, 5, 6, 7, 8, 9, and 10;
d' is 1-100;
Lₙ is a linking group having the formula:
((W)ₕ-(CR⁶R⁷)_{g})ₓ-(Z)ₖ-((CR^{6a}R^{7a})_{g'}-(W)_{h'})_{x'};
W is independently selected at each occurrence from the group: O, S, NH, NHC(=O), C(=O)NH, NR⁸C(=O), C(=O)N R⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ, and (aa)_{t'};
aa is independently at each occurrence an amino acid;
Z is selected from the group: aryl substituted with 0-3 R¹⁰, C₃-₁₀ cycloalkyl substituted with 0-3 R¹⁰, and a 5-10 membered heterocyclic ring system containing 1-4 heteroatoms independently selected from N, S, and 0 and substituted with 0-3 R¹⁰;
R⁶, R^{6a}, R⁷, R^{7a}, and R⁸ are independently selected at each occurrence from the group: H, =0, COOH, SO₃H, PO₃H, C₁-C₅ alkyl substituted with 0-3 R¹⁰, aryl substituted with 0-3 R¹⁰, benzyl substituted with 0-3 R¹⁰, and C₁-C₅ alkoxy substituted with 0-3 R¹⁰, NHC(=O)R¹¹, C(=O)NHR¹¹, NHC(=O)NHR¹¹, NHR¹¹, R¹¹, and a bond to Cₕ;
R¹⁰ is independently selected at each occurrence from the group: a bond to Cₕ, COOR¹¹, C(=O)NHR¹¹, NHC(=O)R¹¹, OH, NHR¹¹, SO₃H, PO₃H, -OPO₃H₂, -OSO₃H, aryl substituted with 0-3 R¹¹, C₁₋₅ alkyl substituted with 0-1 R¹², C₁-₅ alkoxy substituted with 0-1 R¹², and a 5-10 membered heterocyclic ring system containing 1-4 heteroatoms independently selected from N, S, and 0 and substituted with 0-3 R¹¹;
R¹¹ is independently selected at each occurrence from the group: H, -OPO₃H₂,, -OSO₃H C₁₋₅ alkyl substituted with 0-1 R¹², aryl substituted with 0-1 R¹², a 5-10 membered heterocyclic ring system containing 1-4 heteroatoms independently selected from N, S, and O and substituted with 0-1 R¹², C₃₋₁₀ cycloalkyl substituted with 0-1 R¹², polyalkylene glycol substituted with 0-1 R¹², C₁₋₅ alkyl substituted with carbohydrate substituted with 0-1 R¹², cyclodextrin substituted with 0-1 R¹², amino acid substituted with 0-1 R¹², polycarboxyalkyl substituted with 0-1 R¹², polyazaalkyl substituted with 0-1 R¹², peptide substituted with 0-1 R¹², wherein the peptide is comprised of 2-10 amino acids, C₁₋₅ alkyl substituted with 3,6-O-disulfo-B-D-galactopyranosyl, bis(phosphonomethyl)glycine, and a bond to Cₕ;
R¹² is a bond to Cₕ;
k is selected from 0, 1, and 2;
h is selected from 0, 1, and 2;
h' is selected from 0, 1, and 2;
g is selected from 0, 1, 2, 3, 4, 5, 6, 7, 8, 9, and 10;
g' is selected from 0, 1, 2, 3, 4, 5, 6, 7, 8, 9, and 10;
s is selected from 0, 1, 2, 3, 4, 5, 6, 7, 8, 9, and 10;
s' is selected from 0, 1, 2, 3, 4, 5, 6, 7, 8, 9, and 10 ;
s" is selected from 0, 1, 2, 3, 4, 5, 6, 7, 8, 9, and 10;
t is selected from 0, 1, 2, 3, 4, 5, 6, 7, 8, 9, and 10;
t' is selected from 0, 1, 2, 3, 4, 5, 6, 7, 8, 9, and 10;
x is selected from 0, 1, 2, 3, 4, 5, and 6;
x' is selected from 0, 1, 2, 3, 4, 5 and 6;
Cₕ is a metal bonding unit having a formula selected from the group: 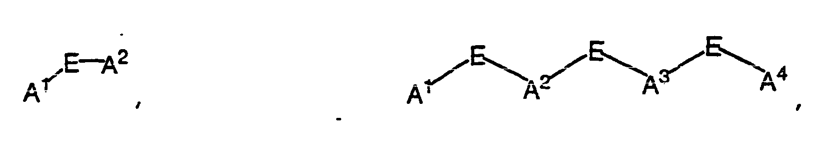 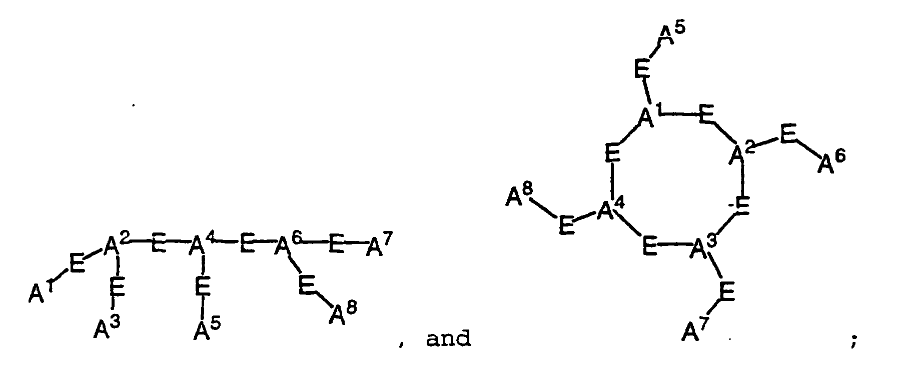
A¹, A², A³, A⁴, A⁵, A⁶, A⁷, and A⁸ are independently selected at each occurrence from the group: NR¹³, NR¹³R¹⁴, S, SH, S(Pg), O, OH, PR¹³, PR¹³R¹⁴, P(O)R¹⁵R¹⁶, and a bond to Lₙ;
E is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
R¹³ and R¹⁴ are each independently selected from the group: a bond to Lₙ, hydrogen, C₁-C₁₀ alkyl substituted with 0-3 R¹⁷, aryl substituted with 0-3 R¹⁷, C₁₋₁₀ cycloalkyl substituted with 0-3 _{R}¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
alternatively, R¹³ and R¹⁴ combine to form =C(R²⁰)(R²¹);
R¹⁵ and R¹⁶ are each independently selected from the group: a bond to Lₙ, -OH, C₁-C₁₀ alkyl substituted with 0-3 R¹⁷, C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
R¹⁷ is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H,
2-(1-morpholino)ethoxy, C₁-C₅ alkyl, C₂-C₄ alkenyl, C₃-C₆ cycloalkyl, C₃-C₆ cycloalkylmethyl, C₂-C₆ alkoxyalkyl, aryl substituted with 0-2 R¹⁸, and a 5-10 membered heterocyclic ring system containing 1-4 heteroatoms independently selected from N, S, and O;
R¹⁸, R^{18a}, and R¹⁹ are independently selected at each occurrence from the group: a bond to Lₙ, H, C₁-C₆ alkyl, phenyl, benzyl, C₁-C₆ alkoxy, halide, nitro, cyano, and trifluoromethyl;
Pg is a thiol protecting group;
R²⁰ and R²¹ are independently selected from the group: H, C₁-C₁₀ alkyl, -CN, -CO₂R²⁵, -C(=O)R²⁵, -C(=O)N(R²⁵)₂, C₂-C₁₀ 1-alkene substituted with 0-3 R²³, C₂-C₁₀ 1-alkyne substituted with 0-3 R²³, aryl substituted with 0-3 R²³, unsaturated 5-10 membered heterocyclic ring system containing 1-4 heteroatoms independently selected from N, S, and O and substituted with 0-3 R²³, and unsaturated C₃₋₁₀ carbocycle substituted with 0-3 R²³;
alternatively, R²⁰ and R²¹, taken together with the divalent carbon radical to which they are attached form: 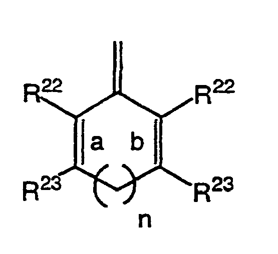
R²² and R²³ are independently selected from the group:
H, R²⁴, C₁-C₁₀ alkyl substituted with 0-3 R²⁴, C₂-C₁₀ alkenyl substituted with 0-3 R²⁴, C₂-C₁₀ alkynyl substituted with 0-3 R²⁴, aryl substituted with 0-3 R²⁴, a 5-10 membered heterocyclic ring system containing 1-4 heteroatoms independently selected from N, S, and O and substituted with 0-3 R²⁴, and C₃₋₁₀ carbocycle substituted with 0-3 R²⁴;
alternatively, R²², R²³ taken together form a fused aromatic or a 5-10 membered heterocyclic ring system containing 1-4 heteroatoms independently selected from N, S, and O;
a and b indicate the positions of optional double bonds and n is 0 or 1;
R²⁴ is independently selected at each occurrence from the group: =O, F, Cl, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, and 2-(1-morpholino)ethoxy; and,
R²⁵, R^{25a}, and R²⁶ are each independently selected at each occurrence from the group: hydrogen and C₁-C₆ alkyl; or a pharmaceutically acceptable salt thereof.

2. A kit according to claim 1 wherein said kit comprises a plurality of separate containers, wherein at least one of said containers contains one or more agents selected from the group consisting of an anti-cancer agent and a radiosensitizer agent, or a pharmaceutically acceptable salt thereof, and a pharmaceutically acceptable carrier, and another of said containers contains a compound of formula (I):
(Q)_{d}-Lₙ-Cₕ or (Q)_{d}-Lₙ-(Cₕ)_{d'} (I)
wherein, Q is a compound of Formula (II): 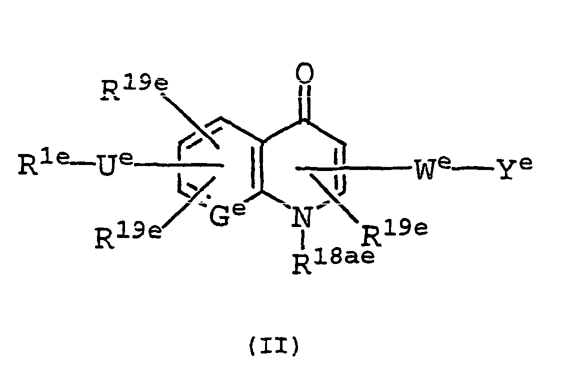 including stereoisomeric forms thereof, or mixtures of stereoisomeric forms thereof, or pharmaceutically acceptable salt or prodrug forms thereof wherein:
R^{1e} is selected from: 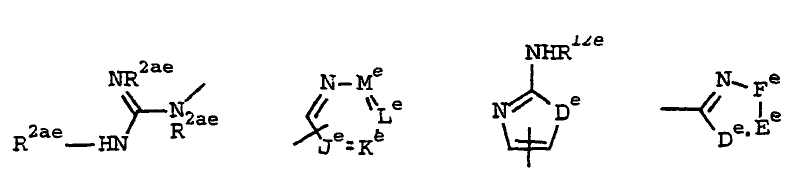 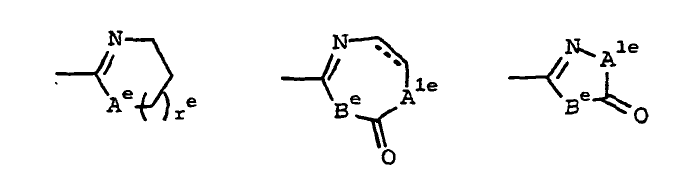 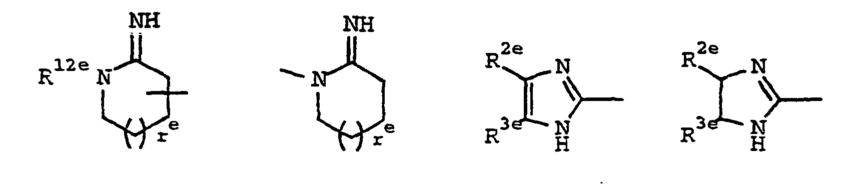 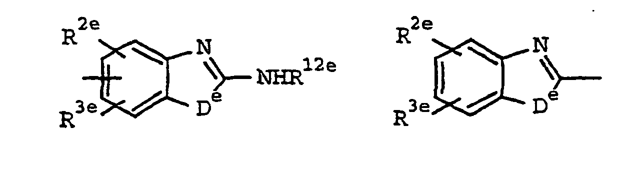 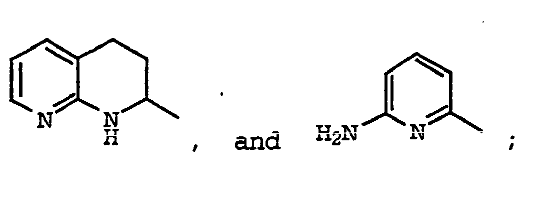
A^{e} is -CH₂- or -N(R^{10e})-;
A^{1e} and B^{e} are independently -CH₂- or -N(R^{10e})-;
D^{e} is -N(R^{10e})- or -S-;
E^{e}-F^{e} is -C(R^{2e})=C(R^{3e})- or -C(R^{2e})₂C(R^{3e})₂-;
J^{e} is -C(R^{2e})- or -N-;
K^{e}, L^{e} and M^{e} are independently -C(R^{2e})- or -C(R^{3e})-;
R^{2e} and R^{3e} are independently selected from:
H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
R^{2ae} is selected from:
H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy) carbonyl;
R^{7e} is selected from:
H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
U^{e} is selected from:
-(CH₂)ₙ^{e}-, -(CH₂)ₙ^{e}O(CH₂)ₘ^{e}-,-(CH₂)ₙ^{e}N(R¹²)(CH₂)ₘ^{e}-, -NH(CH₂)ₙ^{e}-,-(CH₂)ₙ^{e}C(=O)(CH₂)ₘ^{e}-, -(CH₂)ₙ^{e}S(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})-, and -N(R^{10e})S(O)ₚ^{e}-;
G^{e} is N or CR^{19e};
W^{e} is-C(=O)-N(R^{10e})-(C₁-C₃ alkylene)-, in which the alkylene group is substituted by R^{8e} and by R^{9e}:
R^{8e} and R^{9e} are independently selected from:
H, CO₂R^{18be}, C(=O)R^{18be}, CONR¹⁷R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl,
C₃-C₁₀ cycloalkyl(C₁-C₄ alkyl)-, phenyl substituted with 0-3 R^{6e}, naphthyl substituted with 0-3 R^{6e},
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e},
C₁-C₁₀ alkoxy substituted with 0-2 R^{7e}, hydroxy, nitro, -N(R^{10e})R^{11e}, -N(R^{16e})R^{17e}, aryl(C₀-C₆ alkyl)carbonyl, aryl(C₃-C₆ alkyl), heteroaryl(C₁-C₆ alkyl), CONR^{18ae}R^{20e}, SO₂R^{18ae}, and SO₂NR^{18ae}R^{20e},
providing that any of the above alkyl, cycloalkyl, aryl or heteroaryl groups may be unsubstituted or substituted independently with 1-2 R^{7e};
R^{6e} is selected from:
H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
aryl substituted with 0-3 groups selected from halogen, C₁-C₆ alkoxy, C₁-C₆ alkyl, CF₃, S(O)ₘ^{e}Me, and -NMe₂,
aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
R^{10e} is selected from:
H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
R^{11e} is selected from:
H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
R^{4e} is selected from:
H, C₁-C₆ alkyl, C₃-C₇ cycloalkyl, C₃-C₇ cycloalkyl (C₁-C₄ alkyl)-, (C₁-C₁₀ alkyl)carbonyl, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
alternatively, when R^{10e} and R^{11e} are both substituents on the same nitrogen atom (as in -NR^{10e}R^{11e}) they may be taken together with the nitrogen atom to which they are attached to form a heterocycle selected from: 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl, and 1-piperazinyl;
said heterocycle being substituted with 0-3 groups selected from: C₁-C₆ alkyl, aryl, heteroaryl, aryl(C₁-C₄ alkyl)-, (C₁-C₆ alkyl)carbonyl, (C₃-C₇ cycloalk-yl)carbonyl, (C₁-C₆ alkoxy)carbonyl, aryl(C₁-C₄ alkoxy)carbonyl, C₁-C₆ alkylsulfonyl, and arylsulfonyl;
R^{12e} is selected from:
H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy) carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
R^{16e} is selected from:
-C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, and -SO₂NHC(=O)OR^{18be};
R^{17e} is selected from:
H, C₁-C₆ alkyl, C₃-C₇ cycloalkyl, C₃-C₇ cycloalkyl(C₁-C₄ alkyl)-, aryl, aryl (C₁-C₆ alkyl)-, and heteroaryl(C₁-C₆ alkyl);
R^{18ae} is selected from:
C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
R^{18be} is H or R^{18ae};
R^{19e} is selected from:
H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
R^{20e} is selected from:
hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
(5-(C₁-C₅ alkyl)-1,3-dioxa-cyclopenten-2-one-yl)methyloxy,
(5-aryl-1,3-dioxa-cyclopenten-2-one-yl)methyloxy, and
(R^{10e})(R^{11e})N-(C₁-C₁₀ alkoxy)-;
R^{21e} is selected from:
C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and C₁-C₁₀ alkyl substituted with 0-2 R^{7e};
R^{22e} is selected from:
-C(=O)-R^{18be}, -C(=O)N(R^{18be})₂, -C(=O)NRSO₂R^{18ae},-C(=O)NHC(=O)R^{18be}, -C(=O)NHC(=O)OR^{18ae}, and-C(=O)NHSO₂NHR^{28be};
Y^{e} is selected from:
-COR^{20e}, -SO₃H, -PO₃H, -CONHNHSO₂CF₃,-CONHSO₂R^{18ae}, -CONHSO₂NHR^{18be}, -NHCOCF₃,-NHCONHSO₂R^{18ae}, -NHSO₂R^{18ae}, -OPO₃H₂, -OSO₃H,-PO₃H₂, -SO₂NHCOR^{18ae}, -SO₂NHCO₂R^{18ae}, 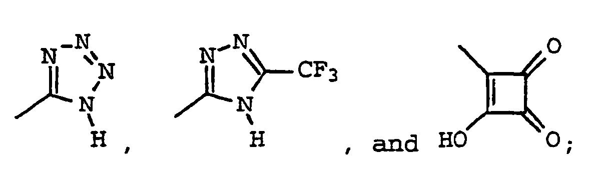
m^{e} is 0-2;
n^{e} is 0-4;
p^{e} is 0-2;
r^{e} is 0-2;
with the following proviso: n^{e} and m^{e} are chosen such that the number of atoms connecting R^{1e} and Y^{e} is in the range of 8-14;
d is selected from 1, 2, 3, 4, 5, 6, 7, 8, 9, and 10;
d' is 1-100;
Lₙ is a linking group having the formula:
((W)ₕ-(CR⁶R⁷)_{g})ₓ-(Z)ₖ-((CR^{6a}R^{7a})_{g'}-(W)_{h'})_{x'} ;
W is independently selected at each occurrence from the group: O, S, NH, NHC(=O), C(=O)NH, NR⁸C(=O), C(=O)N R⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ, and (aa)_{t'};
aa is independently at each occurrence an amino acid;
Z is selected from the group: aryl substituted with 0-3 R¹⁰, C₃₋₁₀ cycloalkyl substituted with 0-3 R¹⁰, and a 5-10 membered heterocyclic ring system containing 1-4 heteroatoms independently selected from N, S, and O and substituted with 0-3 R¹⁰;
R⁶, R^{6a}, R⁷, R^{7a}, and R⁸ are independently selected at each occurrence from the group: H, =O, COOH, SO₃H, PO₃H, C₁-C₅ alkyl substituted with 0-3 R¹⁰, aryl substituted with 0-3 R¹⁰, benzyl substituted with 0-3 R¹⁰, and C₁-C₅ alkoxy substituted with 0-3 R¹⁰, NHC(=O)R¹¹, C(=O)NHR¹¹, NHC(=O)NHR¹¹, NHR¹¹, R¹¹, and a bond to Cₕ;
R¹⁰ is independently selected at each occurrence from the group: a bond to Cₕ, COOR¹¹, C(=O)NHR¹¹, NHC(=O)R¹¹, OH, NHR¹¹, SO₃H, P03H, -OPO₃H₂, -OSO₃H, aryl substituted with 0-3 R¹¹, C₁₋₅ alkyl substituted with 0-1 R¹², C₁₋₅ alkoxy substituted with 0-1 R¹², and a 5-10 membered heterocyclic ring system containing 1-4 heteroatoms independently selected from N, S, and 0 and substituted with 0-3 R¹¹;
R¹¹ is independently selected at each occurrence from the group: H, -OPO₃H₂,, -OSO₃H C₁₋₅ alkyl substituted with 0-1 R¹², aryl substituted with 0-1 R¹², a 5-10 membered heterocyclic ring system containing 1-4 heteroatoms independently selected from N, S, and 0 and substituted with 0-1 R¹², C₃₋₁₀ cycloalkyl substituted with 0-1 R¹², polyalkylene glycol substituted with 0-1 R¹², , C₁-₅ alkyl substituted with carbohydrate substituted with 0-1 R¹², cyclodextrin substituted with 0-1 R¹², amino acid substituted with 0-1 R¹², polycarboxyalkyl substituted with 0-1 R¹², polyazaalkyl substituted with 0-1 R¹², peptide substituted with 0-1 R¹², wherein the peptide is comprised of 2-10 amino acids, 3,6-O-disulfo-B-D-galactopyranosyl, bis(phosphonomethyl)glycine, and a bond to Cₕ;
R¹² is a bond to Cₕ;
k is selected from 0, 1, and 2;
h is selected from 0, 1, and 2;
h' is selected from 0, 1, and 2;
g is selected from 0, 1, 2, 3, 4, 5, 6, 7, 8, 9, and 10;
g_{'} is selected from 0, 1, 2, 3, 4, 5, 6, 7, 8, 9, and 10;
s is selected from 0, 1, 2, 3, 4, 5, 6, 7, 8, 9, and 10;
s' is selected from 0, 1, 2, 3, 4, 5, 6, 7, 8, 9, and
s" is selected from 0, 1, 2, 3, 4, 5, 6, 7, 8, 9, and 10;
t is selected from 0, 1, 2, 3, 4, 5, 6, 7, 8, 9, and 10;
t' is selected from 0, 1, 2, 3, 4, 5, 6, 7, 8, 9, and 10;
x is selected from 0, 1, 2, 3, 4, 5, and 6;
x' is selected from 0, 1, 2, 3, 4, 5 and 6;
Cₕ is a metal bonding unit having a formula selected from the group: 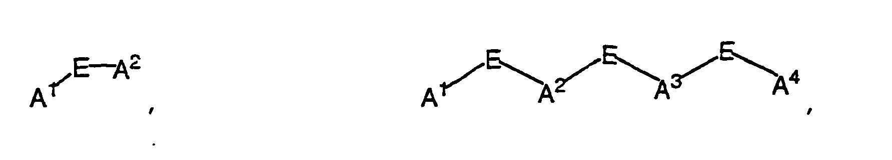 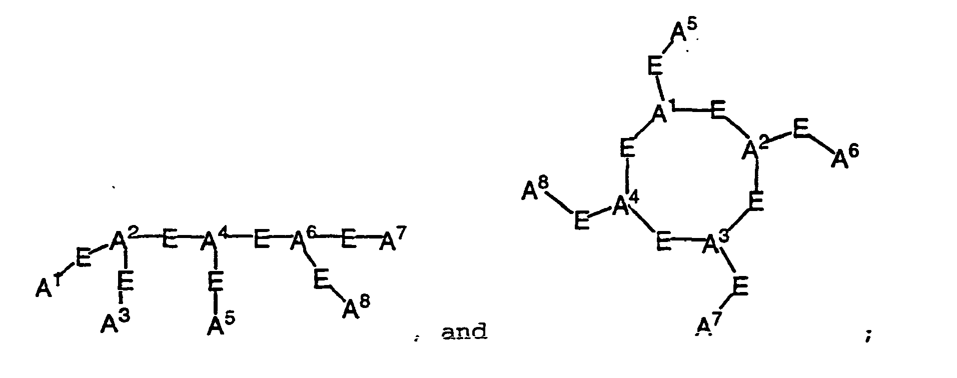
A¹, A², A³, A⁴, A⁵, A⁶, A⁷, and A⁸ are independently selected at each occurrence from the group: NR¹³, NR¹³R¹⁴, S, SH, S(Pg), O, OH, PR¹³, PR¹³R¹⁴, P(O)R¹⁵R¹⁶, and a bond to Lₙ;
E is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
R¹³ and R¹⁴ are each independently selected from the group: a bond to Lₙ, hydrogen, C₁-C₁₀ alkyl substituted with 0-3 R¹⁷, aryl substituted with 0-3 R¹⁷, C₁₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
alternatively, R¹³ and R¹⁴ combine to form =C(R²⁰)(R²¹);
R¹⁵ and R¹⁶ are each independently selected from the group: a bond to Lₙ, -OH, C₁-C₁₀ alkyl substituted with 0-3 R¹⁷, C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and 0 and substituted with 0-3 R¹⁷;
R¹⁷ is independently selected at each occurrence from the group: a bond to Lₙ, =0, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H, 2-(1-morpholino)ethoxy, C₁-C₅ alkyl, C₂-C₄ alkenyl, C₃-C₆ cycloalkyl, C₃-C₆ cycloalkylmethyl, C₂-C₆ alkoxyalkyl, aryl substituted with 0-2 R¹⁸, and a 5-10 membered heterocyclic ring system containing
1-4 heteroatoms independently selected from N, S, and O;
R¹⁸, R^{18a}, and R¹⁹ are independently selected at each occurrence from the group: a bond to Lₙ, H, C₁-C₆ alkyl, phenyl, benzyl, C₁-C₆ alkoxy, halide, nitro, cyano, and trifluoromethyl;
Pg is a thiol protecting group;
R²⁰ and R²¹ are independently selected from the group: H, C₁-C₁₀ alkyl, -CN, -CO₂R²⁵, -C(=O)R²⁵, -C(=O)N(R²⁵)₂, C₂-C₁₀ 1-alkene substituted with 0-3 R²³, C₂-C₁₀ 1-alkyne substituted with 0-3 R²³, aryl substituted with 0-3 R²³, unsaturated 5-10 membered heterocyclic ring system containing 1-4 heteroatoms independently selected from N, S, and O and substituted with 0-3 R²³, and unsaturated C₃₋₁₀ carbocycle substituted with 0-3 R²³;
alternatively, R²⁰ and R²¹, taken together with the divalent carbon radical to which they are attached form: 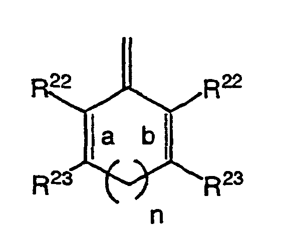
R²² and R²³ are independently selected from the group:
H, R²⁴, C₁-C₁₀ alkyl substituted with 0-3 R²⁴, C₂-C₁₀ alkenyl substituted with 0-3 R²⁴, C₂-C₁₀ alkynyl substituted with 0-3 R²⁴, aryl substituted with 0-3 R²⁴, a 5-10 membered heterocyclic ring system containing 1-4 heteroatoms independently selected from N, S, and 0 and substituted with 0-3 R²⁴, and C₃₋₁₀ carbocycle substituted with 0-3 R²⁴;
alternatively, R²², R²³ taken together form a fused aromatic or a 5-10 membered heterocyclic ring system containing 1-4 heteroatoms independently selected from N, S, and O;
a and b indicate the positions of optional double bonds and n is 0 or 1;
R²⁴ is independently selected at each occurrence from the group: =O, F, Cl, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, and 2-(1-morpholino)ethoxy; and,
R²⁵, R^{25a}, and R²⁶ are each independently selected at each occurrence from the group: hydrogen and C₁-C₆ alkyl; or a pharmaceutically acceptable salt thereof.

3. A kit according to Claim 1, wherein the anti-cancer agent is selected from the group consisting of mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, and leutinizing hormone releasing factor.

4. A kit according to Claim 1, wherein radiosensitizer agent is selected from the group consiting of 2-(3-nitro-1,2,4-triazol-1-yl)-N-(2-methoxyethyl)acetamide, N-(3-nitro-4-quinolinyl)-4-morpholinecarboxamidine, 3-amino-1,2,4-benzotriazine-1,4-dioxide, N-(2-hydroxyethyl)-2-nitroimidazole-1-acetamide, 1-(2-nitroimidazol-1-yl)-3-(1-piperidinyl)-2-propanol, and 1-(2-nitro-1-imidazolyl)-3-(1-aziridino)-2-propanol.

5. A kit according to claim 1, wherein Q is a compound of Formula (IV): 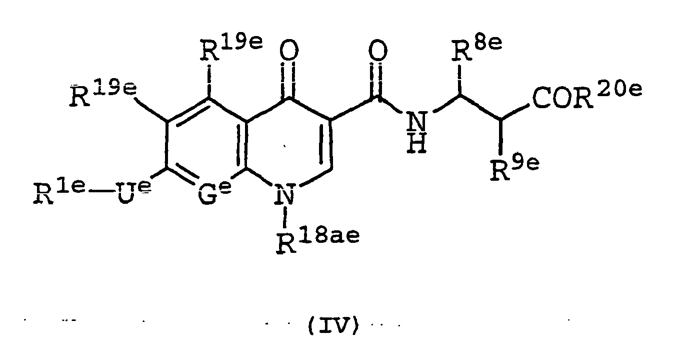 including stereoisomeric forms thereof, or mixtures of stereoisomeric forms thereof, or pharmaceutically acceptable salt or prodrug forms thereof wherein:
R^{1e} is selected from: 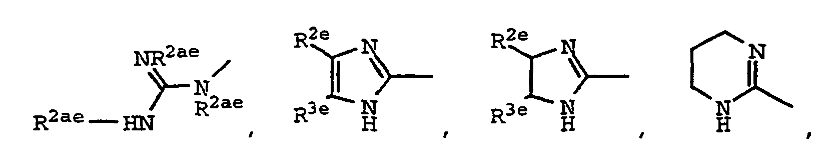 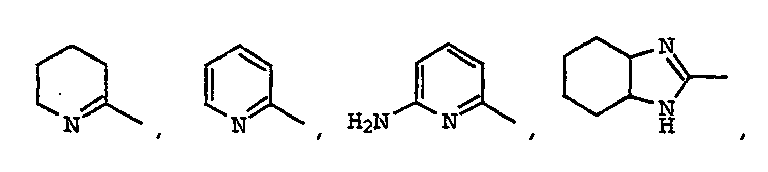 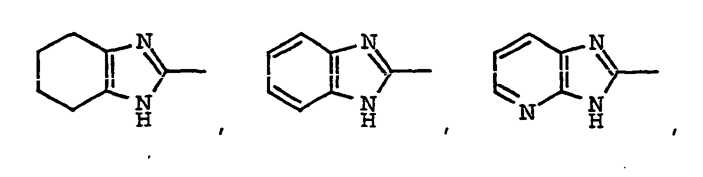 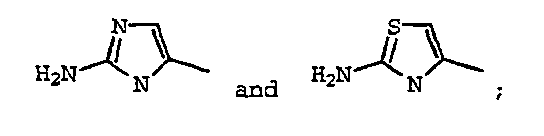
R^{2e} and R^{3e} are independently selected from: H, C₁-C₄ alkoxy, NR^{11e}R^{12e}, halogen, NO₂, CN, CF₃, alkyl, C₃-C₆ alkenyl, C₃-C₇ cycloalkyl, C₃-C₇ cycloalkyl (C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂ ;
R^{2ae} is selected from:
H, C₁-C₁₀ alkyl, C₂-C₆ alkenyl, C₃-C₁₁ cycloalkyl, C₃-C₇ cycloalkyl (C₁-C₄ alkyl), aryl, aryl(C₁-C₄ alkyl)-, (C₂-C₇ alkyl)carbonyl, arylcarbonyl, (C₂-C₁₀ alkoxy) carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
R^{7e} is selected from:
H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
U^{e} is selected from:
-(CH₂)ₙ^{e-}, -(CH₂)ₙ^{e}O(CH₂)ₘ^{e-}, -NH(CH₂)ₙ^{e-}, -N(R^{10e})C(=O)-, -NHC(=O)(CH₂)ₙ^{e-}, and-C(=O)N(R^{10e})- ;
G^{e} is N or CR^{19e};
R^{8e} is selected from:
H, CO₂R^{18be}, C(=O)R^{18be}, CONR^{17e}R^{18be}, C₁-C₁₀ alkyl substituted with 0-1R^{6e}, C₂-C₁₀ alkenyl substituted with 0-1R^{6e}, C₂-C₁₀ alkynyl substituted with 0-1R^{6e}, C₃-C₈ cycloalkyl substituted with 0-1R^{6e}, C₅-C₆ cycloalkenyl substituted with 0-1R^{6e}, (C₁-C₁₀ alkyl)carbonyl, C₃-C₁₀ cycloalkyl(C₁-C₄ alkyl)-, phenyl substituted with 0-3 R^{6e}, naphthyl substituted with 0-3 R^{6e},
a 5-10 membered heterocyclic ring containing 1-3 N, O, or S heteroatoms, wherein said heterocyclic ring may be saturated, partially saturated, or fully unsaturated, said heterocyclic ring being substituted with 0-2R^{7e};
R^{9e} is selected from:
C₁-C₁₀ alkyl substituted with 0-1R^{6e}, C₁-C₁₀ alkoxy substituted with 0-2 R^{7e},
H, nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, hydroxy, OR^{22e}, -N(R^{10e})R^{11e}, -N(R^{16e})R^{17e}, aryl(C₀-C₆ alkyl)carbonyl, aryl(C₁-C₆ alkyl), heteroaryl(C₁-C₆ alkyl), CONR^{18ae}R^{20e}, SO₂R^{18ae}, and SO₂NR^{18ae}R^{20e},
providing that any of the above alkyl, cycloalkyl, aryl or heteroaryl groups may be unsubstituted or substituted independently with 1-2 R^{7e};
R^{6e} is selected from:
H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚ^{e}R^{11e}, SO₂NR^{10e}R^{11e},
aryl substituted with 0-3 groups selected from halogen, C₁-C₆ alkoxy, C₁-C₆ alkyl, CF₃, S(O)ₘ^{e}Me, and -NMe₂,
aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
R^{10e} is selected from:
H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
R^{11e} is selected from:
H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
R^{4e} is selected from:
H, C₁₋C₆ alkyl, C₃-C₇ cycloalkyl, C₃-C₇ cycloalkyl(C₁-C₄ alkyl)-, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
R^{12e} is selected from:
H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy)carbonyl, (C₁-C₆ alkyl) aminocarbonyl, C₃-C₆ alkenyl, C₃-C₇ cycloalkyl, C₃-C₇ cycloalkyl(C₁-C₄ alkyl)-, aryl, heteroaryl (C₁-C₆ alkyl) carbonyl, heteroarylcarbonyl, aryl (C₁-C₆ alkyl)-, (C₁-C₆ alkyl)carbonyl, arylcarbonyl, C₁-C₆ alkylsulfonyle, arylsulfonyl, aryl (C₁-C₆ alkyl)sulfonyl, heteroarylsulfonyl, heteroaryl (C₁-C₆ alkyl)sulfonyl, aryloxycarbonyl, and aryl (C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
R^{16e} is selected from:
-C(=O)OR^{18ae}, -C(=O)R^{18be}, -C(=O)N(R^{18be})₂,-SO₂R^{18ae}, and -SO₂N(R^{18be})₂;
R^{17e} is selected from:
H, C₁-C₆ alkyl, C₃-C₇ cycloalkyl, C₃-C₇ cycloalkyl(C₁-C₄ alkyl)-, aryl, aryl(C₁-C₆ alkyl)-, and heteroaryl(C₁-C₆ alkyl);
R^{18ae} is selected from:
C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4R^{19e} and optionally substituted with a bond to Lₙ, naphthyl substituted with 0-4R^{19e} and optionally substituted with a bond to Lₙ, and a bond to Lₙ, wherein said aryl or heteroaryl groups are optionally substituted with 0-4 R^{19e} ;
R^{18be} is H or R^{18ae} ;
R^{19e} is selected from:
H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
R^{20e} is selected from:
hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁₋C₂ alkyl)oxy-, C₃-C₁₀ cycloalkylcarbonyloxy(C₁-C₂ alkyl) oxy-, C₃-C₁₀ cycloalkoxycarbonyloxy(C₁-C₂ alkyl) oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl) carbonyloxy(C₁-C₂ alkyl)ox Y,
(5-(C₁-C₅ alkyl)-1,3-dioxa-cyclopenten-2-one-yl)methyloxy,
(5-aryl-1,3-dioxa-cyclopenten-2-one-yl)methyloxy, and
(R^{10e})(R^{11e})N-(C₁-C₁₀ alkoxy)-;
R^{21e} is selected from:
C₁-C₈ alkyl, C₂-C₆ alkenyl, C₃-C₁₁ cycloalkyl, (C₃-C₁₁ cycloalkyl) methyl, aryl, aryl (C₁-C₄ alkyl)-, and
C₁-C₁₀ alkyl substituted with 0-2R^{7e};
R^{22e} is selected from:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, and -C(=O)NHSO₂NHR^{18be} ;
m^{e} is 0-2;
n^{e} is 0-4; and
pe is 0-2;
with the following proviso: n^{e} and m^{e} are chosen such that the number of atoms connecting R¹ and -COR^{20e} in Formula (IV) is in the range of 8-14;
d is selected from 1, 2, 3, 4, and 5;
d' is 1-50;
W is independently selected at each occurrence from the group: O, NH, NHC(=O), C(=O)NH, NR⁸C(=O), C(=O)N R⁸, C(=O), C(=O)O, OC(=O), NHC(=S)NH, NHC(=O)NH, SO₂, (OCH₂CH₂)ₛ,(CH₂CH₂O)_{s'}, (OCH₂CH₂CH₂)_{s"}, (CH₂CH₂CH₂O)ₜ, and (aa)_{t'} ;
aa is independently at each occurrence an amino acid;
Z is selected from the group: aryl substituted with 0-1 R¹⁰, C₃-₁₀ cycloalkyl substituted with 0-1 R¹⁰, and a 5-10 membered heterocyclic ring system containing 1-4 heteroatoms independently selected from N, S, and 0 and substituted with 0-1R¹⁰;
R⁶, R^{6a}, R⁷, R^{7a}, and R⁸ are independently selected at each occurrence from the group: H, =O, COOH, SO₃H, C₁-C₅ alkyl substituted with 0-1 R¹⁰, aryl substituted with 0-1 R¹⁰, benzyl substituted with 0-1 R¹⁰, and C₁-C₅ alkoxy substituted with 0-1 R¹⁰, NHC(=O)R¹¹, C(=O)NHR¹¹, NHC(=O)NHR¹¹, NHR¹¹, R¹¹, and a bond to Cₕ;
k is 0 or 1;
s is selected from 0, 1, 2, 3, 4, and 5;
s' is selected from 0, 1, 2, 3, 4, and 5;
s" is selected from 0. 1, 2, 3, 4, and 5;
t is selected from 0, 1, 2, 3, 4, and 5;
A¹, A², A³, A⁴, A⁵, A⁶, A⁷, and A⁸ are independently selected at each occurrence from the group: NR¹³, NR¹³R¹⁴, S, SH, S(Pg), OH, and a bond to Lₙ;
E is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, and a 5-10 membered heterocyclic ring system containing 1-4 heteroatoms independently selected from N, S, and O and substituted with 0-3 R¹⁷;
R¹³, and R¹⁴ are each independently selected from the group: a bond to Lₙ, hydrogen, C₁-C₁₀ alkyl substituted with 0-3 R¹⁷, aryl substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
alternatively, R¹³ and R¹⁴ combine to form =C(R²⁰)(R²¹);
R¹⁷ is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)₂, -N(R¹⁸)₂. -NHC(=S)NHR¹⁸, =NOR¹⁸, -C(=O)NHNR¹⁸R^{18a}, -OCH₂CO₂H, and 2-(1-morpholino)ethoxy;
R¹⁸, R^{18a}, and R¹⁹ are independently selected at each occurrence from the group: a bond to Lₙ, H, and C₁-C₆ alkyl;
R²⁰ and R²¹ are independently selected from the group:
H, C₁-C₅ alkyl, -CO₂R²⁵, C₂-C₅ 1-alkene substituted with 0-3 R²³, C₂-C₅ 1-alkyle substituted with 0-3 R²³, aryl substituted with 0-3 R²³, and unsaturated 5-10 membered heterocyclic ring system containing 1-4 heteroatoms independently selected from N, S, and O and substituted with 0-3 R²³;
alternatively, R²⁰ and R²¹, taken together with the divalent carbon radical to which they are attached form: 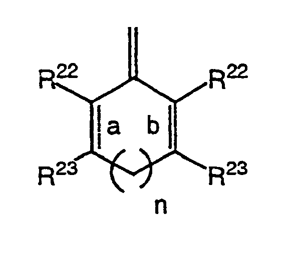
R²² and R²³ are independently selected from the group: H, and R²⁴ ;
alternatively, R²², R²³ taken together form a fused aromatic or a 5-10 membered heterocyclic ring system containing 1-4 heteroatoms independently selected from N, S, and O;
R²⁴ is independently selected at each occurrence from the group: -CO₂R²⁵, -C(=O)N(R²⁵)₂, -CH₂OR²⁵, -OC(=O)R²⁵, -OR²⁵ , -SO₃H, -N(R²⁵)_{2,} and -OCH₂CO₂H; and,
R²⁵ is independently selected at each occurrence from the group: H and C₁-C₃ alkyl.

6. A kit according to claim 1,
wherein:
R^{1e} is selected from: 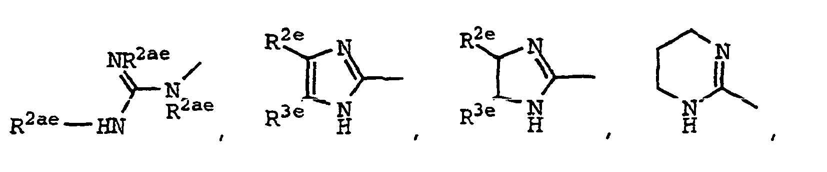 and 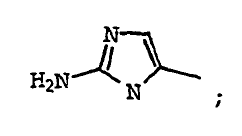
R^{2e} and R^{3e} are independently selected from:
H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4R^{7e},
alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂ ;
R^{2ae} is selected from:
H, C₁-C₁₀ alkyl, C₂-C₆ alkenyl, C₃-C₁₁ cycloalkyl, C₃-C₇ cycloalkyl (C₁-C₄ alkyl), aryl, aryl(C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy) carbonyl, arylcarbonyloxy(C₁-C₄ alkoxy)carbonyl, and C₃-C₇ cycloalkylcarbonyloxy(C₁-C₄ alkoxy) carbonyl;
R^{7e} is selected from:
H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e} ;
U^{e} is selected from:
-(CH₂)ₙ^{e}-, -NH(CH₂)ₙ^{e}-, -N(R^{10e})C(=O)-, and -NHC(=O) (CH₂)ₙ^{e} ;
G^{e} is N or CR^{19e};
R^{8e} is H;
R^{9e} is selected from:
H, nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, hydroxy, OR^{22e}, -N(R^{10e})R^{11e}, -N(R^{16e})R^{17e}, aryl (C₀-C₄ alkyl)carbonyl, aryl (C₁-C₄ alkyl), heteroaryl (C₁-C₄ alkyl),
CONR^{18ae}R^{20e}, SO₂R^{18ae}, and SO₂NR^{18ae}R^{20e}, providing that any of the above alkyl, cycloalkyl, aryl or heteroaryl groups may be unsubstituted or substituted independently with 1-2 R^{7e};
R^{10e} is selected from:
H, -OH, CF₃, C₃-C₆alkenyl, C₃-C₆cycloalkyl, aryl, (C₃-C₆ cycloalkyl)methyl, aryl (C₁-C₄alkyl), and C₁-C₄ alkyl substituted with 0-2 R^{6e};
R^{6e} is selected from:
H, C₁-C₄ alkyl, hydroxy, C₁-C₄ alkoxy, nitro, C₁-C₄ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
aryl substituted with 0-3 groups selected from halogen, C₁-C₄ alkoxy, C₁-C₄ alkyl, CF₃, S(O)ₘ^{e}Me, and -NMe₂,
aryl(C₁-C₄ alkyl)-, said aryl being substituted with 0-3 groups selected from halogen, C₁-C₄ alkoxy, C₁-C₄ alkyl, CF₃, S(O)ₚ^{e}Me, and -NMe₂, and
a 5-10 membered heterocyclic ring containing 1-3 N, O, or S heteroatoms, wherein said heterocyclic ring may be saturated, partially saturated, or fully unsaturated, said heterocyclic ring being substituted with 0-2R^{7e};
R^{11e} is selected from:
H, hydroxy, C₁-C₄ alkyl, C₃-C₆ alkenyl, C₃-C₆ cycloalkyl, (C₃-C₆ cycloalkyl)methyl, C₁-C₄ alkoxy, benzyloxy, aryl, heteroaryl, heteroaryl(C₁-C₄ alkyl)-, aryl(C₁-C₄ alkyl), adamantylmethyl, and C₁-C₄ alkyl substituted with 0-2 R^{4e};
R^{4e} is selected from:
H, C₁-C₄ alkyl, C₃-C₇ cycloalkyl, C₃-C₇ cycloalkyl(C₁-C₄ alkyl)-, aryl, heteroaryl, aryl(C₁-C₄ alkyl)-, and heteroaryl(C₁-C₄ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
R^{12e} is selected from:
H, C₁-C₄ alkyl, (C₁-C₄ alkyl)carbonyl, (C₁-C₄ alkoxy)carbonyl, phenyl(C₁-C₄ alkyl)-, phenylsulfonyl, phenyloxycarbonyl, and phenyl(C₁-C₄ alkoxy)carbonyl, wherein said phenyl groups are substituted with 0-2 substituents selected from the group consisting of C₁-C₄ alkyl, C₁-C₄ alkoxy, halo, CF₃, and nitro;
R^{16e} is selected from:
-C(=O)OR^{18ae} -C(=O)R^{18be}, -C(=O)N(R^{18be})₂,-SO₂R^{18ae}, and -SO₂N(R^{18be})_{2;}
R^{17e} is selected from:
H, C₁-C₄ alkyl, C₃-C₆ cycloalkyl, C₃-C₆ cycloalkyl(C₁-C₄ alkyl)-, aryl, aryl(C₁-C₆ alkyl)-, and heteroaryl(C₁-C₆ alkyl);
R^{18ae} is selected from:
C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e} ;
R^{18be} is H or R^{18ae};
R^{19e} is selected from:
H, halogen, CF₃, CO₂H, CN, NO₂, -NR^{11e}R^{12e}, OCF₃, C₁-C₆ alkyl, C₂-C₆ alkenyl, C₂-C₆ alkynyl, C₃-C₆ cycloalkyl, C₃-C₆ cycloalkyl(C₁-C₄ alkyl)-, aryl(C₁-C₄ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
R^{20e} is selected from:
hydroxy, C₁-C₆ alkyloxy, C₃-C₆ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
(5-(C₁-C₅ alkyl)-1,3-dioxa-cyclopenten-2-one-yl)methyloxy,
(5-aryl-1,3-dioxa-cyclopenten-2-one-yl)methyloxy, and
(R^{10e})(R^{11e})N-(C₁-C₁₀ alkoxy) -;
R^{21e} is selected from:
C₁-C₄ alkyl, C₂-C₆ alkenyl, C₃-C₆ cycloalkyl, (C₃-C₆ cycloalkyl)methyl, aryl, aryl(C₁-C₄ alkyl)-, and C₁-C₁₀ alkyl substituted with 0-2 R^{7e};
R^{22e} is selected from:
-C(=O)-R^{18be}, -C(=O)N(R^{18be})₂, C(=O)NHSO₂R^{18ae}, -C(=O)NHC(=O)R^{18be}, -C(=O)NHC(=O)OR^{18ae}, and -C(=O)NHSO₂NHR^{18be};
m^{e} is 0-2;
n^{e} is 0-4;
p^{e} is 0-2;
Cₕ is 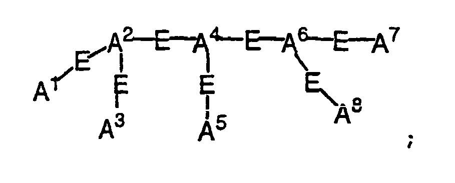
A¹ is selected from the group: OH, and a bond to Lₙ;
A², A⁴, and A⁶ are each N;
A³, A⁵, and A⁸ are each OH;
A⁷ is a bond to Lₙ or NH-bond to Lₙ;
E is a C₂ alkyl substituted with 0-1R¹⁷;
R¹⁷ is =O;
alternatively, Cₕ is 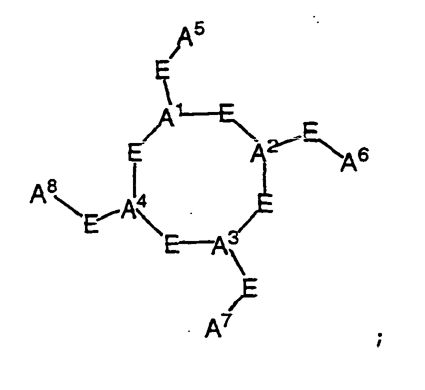
A¹ is selected from the group: OH, and a bond to Lₙ;
A², A³ and A⁴ are each N;
A⁵, A⁶ and A⁸ are each OH;
A⁷ is a bond to Lₙ;
E is a C₂ alkyl substituted with 0-1 R¹⁷;
R¹⁷ is =O;
alternatively, Cₕ is 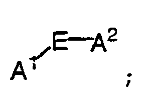
A¹ is NH₂ or N=C(R²⁰)(R²¹);
E is a bond;
A² is NHR¹³;
R¹³ is a heterocycle substituted with R¹⁷, the heterocycle being selected from pyridine and pyrimidine;
R¹⁷ is selected from a bond to Lₙ, C(=O)NHR¹⁸ and C(=O)R¹⁸;
R¹⁸ is a bond to Lₙ;
R²⁴ is selected from the group: -CO₂R²⁵, -OR²⁵, -SO₃H, and -N(R²⁵)₂; and,
R²⁵ is independently selected at each occurrence from the group: hydrogen and methyl.

7. A kit according to claim 1, wherein:
Q is selected from the group:
3-[7-[(imidazolin-2-ylamino)methyl]-1-methyl-6,8-difluoroquinoline-4-one-3-ylcarbonylamino]-2-(3,5-dimethylisoxazol-4-ylsulfonylamino)propionic acid,
3-[7-[(imidazolin-2-ylamino)methyl]-1-methyl-6,8-difluoroquinoline-4-one-3-ylcarbonylamino]-2-(benzyloxycarbonylamino)propionic acid,
3-[7-[(imidazolin-2-ylamino)methyl]-1-methyl-6,8-difluoroquinoline-4-one-3-ylcarbonylamino]-2-(n-butyloxycarbonylamino)propionic acid,
3-[7-[(imidazolin-2-ylamino)methyl]-1-methyl-6,8-difluoroquinoline-4-one-3-ylcarbonylamino]-2-(n-butylsulfonylamino)propionic acid,
3-[7-[(tetrahydropyrimid-2-ylamino)methyl]-1-methyl-6,8-difluoroquinoline-4-one-3-ylcarbonylamino]-2-(benzyloxycarbonylamino)propionic acid,
3-[7-[(tetrahydropyrimid-2-ylamino)methyl]-1-methyl-6,8-difluoroquinoline-4-one-3-ylcarbonylamino]-2-(n-butyloxycarbonylamino)propionic acid,
3-[7-[(tetrahydropyrimid-2-ylamino)methyl]-1-methyl-6,8-difluoroquinoline-4-one-3-ylcarbonylamino]-2-(phenylsulfonylamino)propionic acid,
3-[7-[(tetrahydropyrimid-2-ylamino)methyl]-1-methyl-6,8-difluoroquinoline-4-one-3-ylcarbonylamino]-2-(n-butylsulfonyl)aminopropionic acid,
3-[7-[(2-aminothiazol-4-yl)methyl]-1-methyl-6,8-difluoroquinoline-4-one-3-ylcarbonylamino]-2-(benzyloxycarbonylamino)propionic acid,
3-[7-[(imidazolin-2-ylamino)methyl]-1-methyl-6,8-difluoroquinoline-4-one-3-ylcarbonylamino]-2-((2,4,6-trimethylphenyl)sulfonylamino)propionic acid,
3-[7-[(tetrahydropyrimid-2-ylamino)methyl]-1-methyl-6,8-difluoroquinoline-4-one-3-ylcarbonylamino]-2-((2,4,6-trimethylphenyl)sulfonylamino)propionic acid,
3-[7-[(imidazol-2-ylamino)methyl]-1-methyl-6,8-difluoroquinoline-4-one-3-ylcarbonylamino]-2-(3,5-dimethylisoxazol-4-ylsulfonylamino)propionic acid,
3-[7-[(imidazol-2-ylamino)methyl]-1-methyl-6,8-difluoroquinoline-4-one-3-ylcarbonylamino]-2-(benzyloxycarbonylamino)propionic acid,
3-[7-[(imidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(imidazol-2-ylamino)methyl]-1-methyl-6,8-difluoroquinoline-4-one-3-ylcarbonylamino]-2-((4-biphenyl)sulfonylamino)propionic acid,
3-[7-[(imidazol-2-ylamino)methyl]-1-methyl-6,8-difluoroquinoline-4-one-3-ylcarbonylamino]-2-(1-naphthylsulfonylamino)propionic acid,
3-[7-[(benzimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(4-methylimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(4,5-dimethylimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(4,5,6,7-tetrahydrobenzimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(pyridin-2-ylamino)methyl]-1-methyl-6,8-difluoroquinoline-4-one-3-ylcarbonylamino]-2-((2,4,6-trimethylphenyl)sulfonylamino)propionic acid,
3-[7-(2-aminopyridin-6-yl)-1-methyl-6,8-difluoroquinoline-4-one-3-ylcarbonylamino]-2-((2,4,6-trimethylphenyl)sulfonylamino)propionic acid,
3-[7-[(7-azabenzimidazol-2-yl)methyl]-1-methyl-6,8-difluoroquinoline-4-one-3-ylcarbonylamino]-2-((2,4,6-trimethylphenyl)sulfonylamino)propionic acid,
3-[7-[(benzimidazol-2-ylamino)methyl]-1-(2-phenylethyl)-6,8-difluoroquinoline-4-one-3-ylcarbonylamino]pro-pionic acid,
3-[7-[(pyridin-2-ylamino)methyl]-1-(2-phenylethyl)-6,8-difluoroquinoline-4-one-3-ylcarbonylamino]propionic acid,
3-[7-[(imidazolin-2-ylamino)methyl]-1-(2-phenylethyl)-6,8-difluoroquinoline-4-one-3-ylcarbonylamino]propionic acid,
3-[7-[(imidazol-2-ylamino)methyl]-1-(2-phenylethyl)-6,8-difluoroquinoline-4-one-3-ylcarbonylamino]propionic acid,
3-[7-[(imidazolin-2-ylamino)methyl]-1-(2-phenylethyl)-6,8-difluoroquinoline-4-one-3-ylcarbonylamino]-2-(benzyloxycarbonylamino)propionic acid,
3-[7-[(imidazolin-2-ylamino)methyl]-1-(2-phenylethyl)-6,8-difluoroquinoline-4-one-3-ylcarbonylamino]-2-(n-butyloxycarbonylamino)propionic acid,
3-[7-[(imidazolin-2-ylamino)methyl]-1-(2-phenylethyl)-6,8-difluoroquinoline-4-one-3-ylcarbonylamino]-2-(phenylsulfonylamino)propionic acid,
3-[7-[(imidazolin-2-ylamino)methyl]-1-(2-phenylethyl)-6,8-difluoroquinoline-4-one-3-ylcarbonylamino]-2-(n-butylsulfonylamino)propionic acid,
3-[7-[(tetrahydropyrimid-2-ylamino)methyl]-1-(2-phenylethyl)-6,8-difluoroquinoline-4-one-3-ylcarbonylamino]-2-(benzyloxycarbonylamino)propionic acid,
3-[7-[(tetrahydropyrimid-2-ylamino)methyl]-1-(2-phenylethyl)-6,8-difluoroquinoline-4-one-3-ylcarbonylamino]-2-(n-butyloxycarbonylamino)propionic acid,
3-[7-[(tetrahydropyrimid-2-ylamino)methyl]-1-(2-phenylethyl)-6,8-difluoroquinoline-4-one-3-ylcarbonylamino]-2-(phenylsulfonylamino)propionic acid,
3-[7-[(tetrahydropyrimid-2-ylamino)methyl]-1-(2-phenylethyl)-6,8-difluoroquinoline-4-one-3-ylcarbonylamino]-2-(n-butylsulfonyl)aminopropionic acid,
3-[7-[(2-aminothiazol-4-yl)methyl]-1-(2-phenylethyl)-6,8-difluoroquinoline-4-one-3-ylcarbonylamino]-2-(phenylsulfonylamino)propionic acid,
3-[7-[(2-aminothiazol-4-yl)methyl]-1-(2-phenylethyl)-6,8-difluoroquinoline-4-one-3-ylcarbonylamino]-2-(benzyloxycarbonylamino)propionic acid,
3-[7-[(imidazolin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(tetrahydropyrimid-2-ylamino)methyl]-1-(2-phenylethyl)-6,8-difluoroquinoline-4-one-3-ylcarbonylamino]-2-((2,4,6-trimethylphenyl)sulfon-ylamino)propionic acid,
3-[7-[(imidazol-2-ylamino)methyl]-1-(2-phenylethyl)-6,8-difluoroquinoline-4-one-3-ylcarbonylamino]-2-(benzyloxycarbonylamino)propionic acid,
3-[7-[(imidazol-2-ylamino)methyl]-1-(2-phenylethyl)-6,8-difluoroquinoline-4-one-3-ylcarbonylamino]-2-(phenylsulfonylamino)propionic acid,
3-[7-[(imidazol-2-ylamino)methyl]-1-(2-phenylethyl)-6,8-difluoroquinoline-4-one-3-ylcarbonylamino]-2-((2,6,dichlorophenyl)sulfonylamino)propionic acid,
3-[7-[(imidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(imidazol-2-ylamino)methyl]-1-(2-phenylethyl)-6,8-difluoroquinoline-4-one-3-ylcarbonylamino]-2-((4-biphenyl)sulfonylamino)propionic acid,
3-[7-[(benzimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(4-methylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(4,5-dimethylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(4,5,6,7-tetrahydrobenzimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(pyridin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-(2-aminopyridin-6-yl)-1-(2-phenylethyl)-6,8-difluoroquinoline-4-one-3-ylcarbonylamino]-2-((2,4,6-trimethylphenyl)sulfonylamino)propionic acid, and
3-[7-[(7-azabenzimidazol-2-yl)methyl]-1-(2-phenylethyl)-6,8-difluoroquinoline-4-one-3-ylcarbonylamino]-2-((2,4,6-trimethylphenyl)sulfonylamino)propionic acid.

8. A kit according to claim 1, wherein the compound is selected from the group:
2-(((4-(4-(((3-(2-(2-(3-((6-((1-aza-2-(2-sulfophenyl)vinyl)amino)(3-pyridyl))carbonylamino)-propoxy)ethoxy)-ethoxy)propyl)amino)sulfonyl)phenyl)phenyl)-sulfonyl)amino)-3-((7-((imidazol-2-ylamino)methyl)-1-methyl-4-oxo(3-hydroquinolyl))carbonylamino)propanoic acid;
3-((7-((imidazol-2-ylamino)methyl)-1-methyl-4-oxo(3-hydroquinolyl))carbonylamino)-2-(((4-(4-(((3-(2-(2-(3-(2-(1,4,7,10-tetraaza-4,7,10-tris(carboxylmethyl)cyclododecyl)acetylamino)-propoxy)ethoxy)ethoxy)propyl)amino)sulfonyl)-phenyl)phenyl)sulfonyl)amino)propanoic acid;
2-(((4-(3-(N-(3-(2-(2-(3-((6-((1-aza-2-(2-sulfophenyl)vinyl)amino)(3-pyridyl))carbonylamino)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))carbonylamino)propanoic;
3-((1-(3-((6-((1-aza-2-(2-sulfophenyl)vinyl)amino)(3-pyridyl))carbonylamino)propyl)-7-((imidazole-2-ylamino)methyl)-4-oxo(3-hydroquinolyl))carbonylamino)-2-(((2,4,6-trimethylphenyl)sulfonyl)amino)propanoic acid;
3-((1-(3-((6-((1-aza-2-(2-sulfophenyl)vinyl)amino) (3-pyridyl))carbonylamino)propyl)-7-(((1-hydroxyimidazole-2-yl)amino)methyl)-4-oxo(3-hydroquinolyl))carbonylamino)-2-(((2,4,6-trimethylphenyl)sulfonyl)amino)propanoic acid;
3-((1-(3-(3-(N-(3-(2-(2-(3-((6-((1-aza-2-(2-sulfophenyl)vinyl)amino) (3-pyridyl))carbonylamino)propoxy)ethoxy)-ethoxy)propyl)carbamoyl)propanoylamino)propyl)-7-((imidazole-2-ylamino)methyl)-4-oxo(3-hydroquinolyl))carbonylamino)-2-(((2,4,6-trimethylphenyl)sulfonyl)amino)propanoic acid;
2-(2-aza-2-(5-(N-(1,3-bis(3-(2-(2-(3-(3-(N-(3-(3-(N-(3-carboxy-2-(((2,4,6-trimethylphenyl)sulfonyl)amino)-ethyl)carbamoyl)-7-((imidazole-2-ylamino)methyl)4-oxohydroquinolyl)propyl)carbamoyl)propanoylamino)pr opoxy)ethoxy)ethoxy)propyl)carbamoyl) (2-pyridyl))amino)vinyl)benzenesulfonic acid; 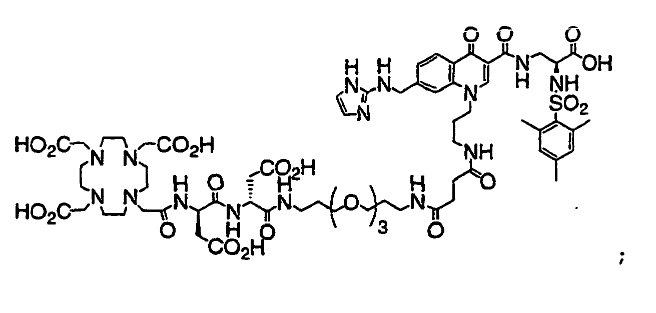 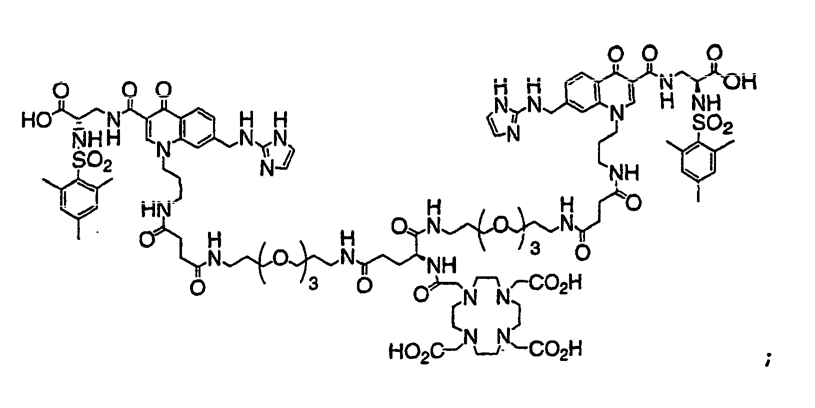 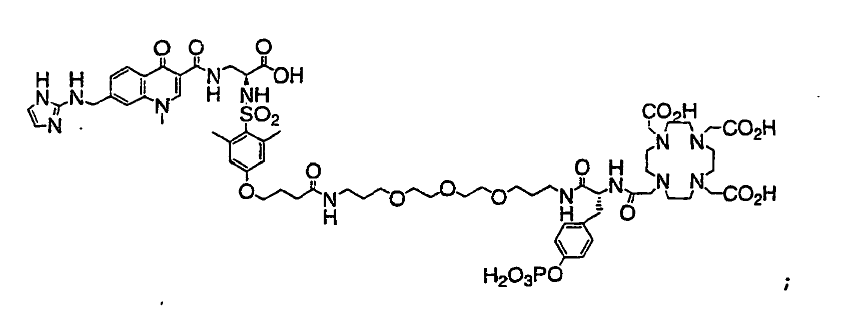 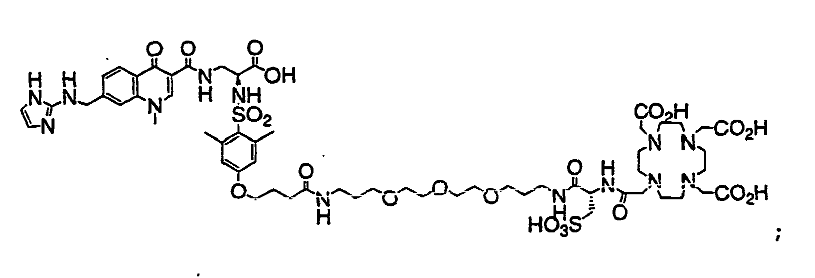 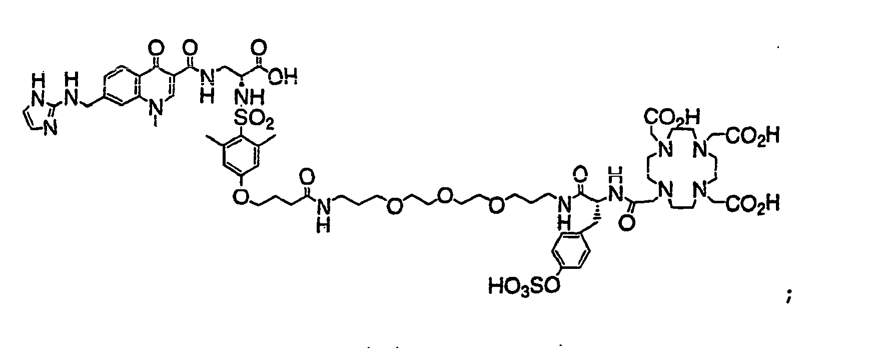 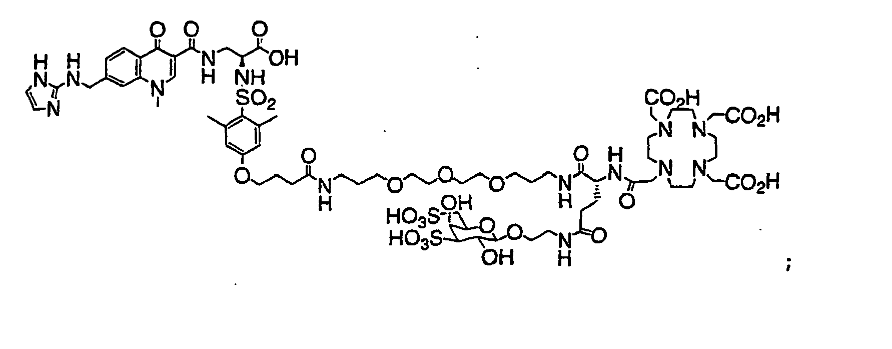 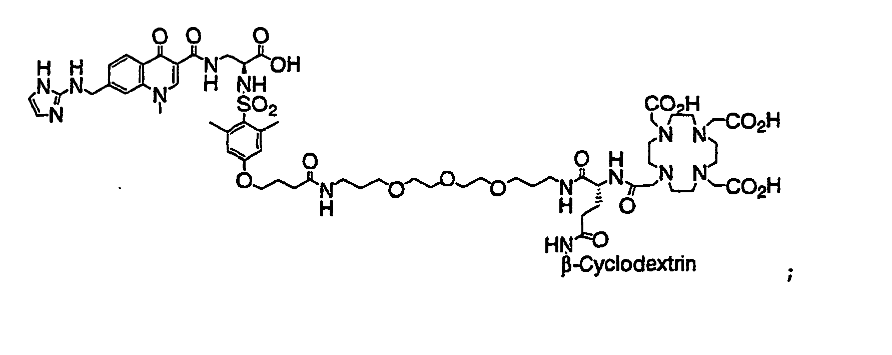 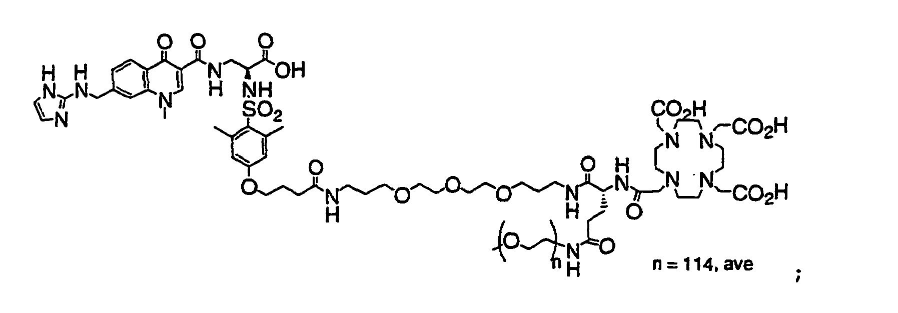
2-(((4-(3-(N-(3-(2-(2-(3-(2-(1,4,7,10-tetraaza-4,7,10-tris(carboxymethyl)cyclododecylacetylamino)-6-aminohexanoylamino)propoxy)ethoxy)ethoxy)-propyl)carbamoyl)propoxy)-2,6-dimethylphenyl)sulfonyl)amino)-3-((7-((imidazol-2-ylamino)methyl)-1-methyl-4-oxo(3-hydroquinolyl))-carbonylamino)propanoic acid ;
2-(((4-(3-(N-(3-(2-(2-(3-(2-(1,4,7,10-tetraaza-4,7,10-tris(carboxymethyl)cyclododecylacetylamino)-6-(2-(bis(phosphonomethyl)amino)acetylamino)hexanoylamin o)propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))carbonylamino)propanoic acid conjugate; and
2-(((4-(3-(N-(3-(2-(2-(3-(2-(2-((2-((2-(bis(carboxymethyl)-amino)ethyl)(carboxymethyl)amino)ethyl)(carboxymeth yl)amino)acetylamino)-3-sulfopropyl)-propoxy)ethoxy)ethoxy)propyl)carbamoyl)propoxy)-2,6-dimethylphenyl)sulfonyl)amino)-3-((7-((imidazol-2-ylamino)methyl)-1-methyl-4-oxo(3-hydroquinolyl))carbonylamino)propanoic acid; 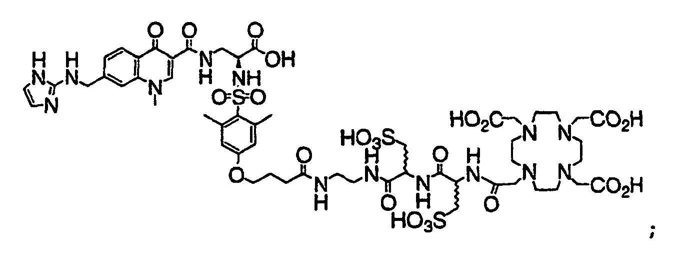 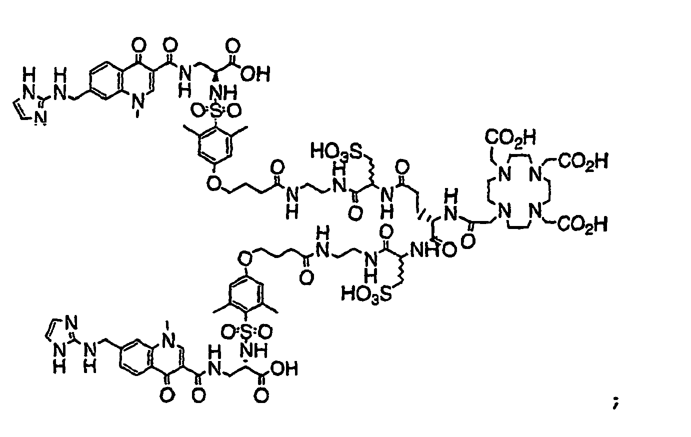 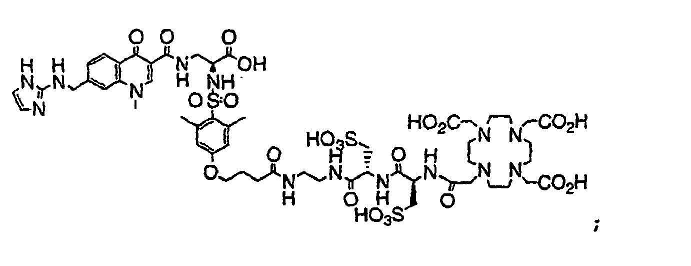 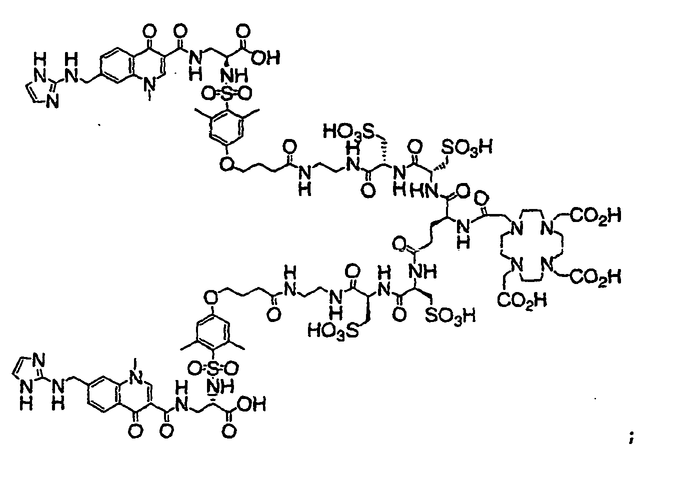 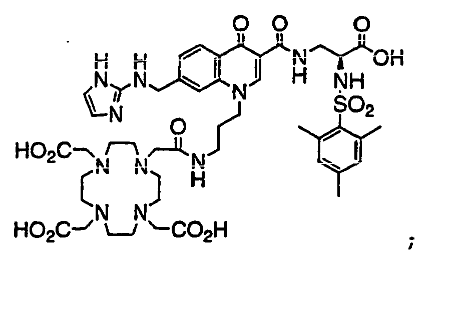 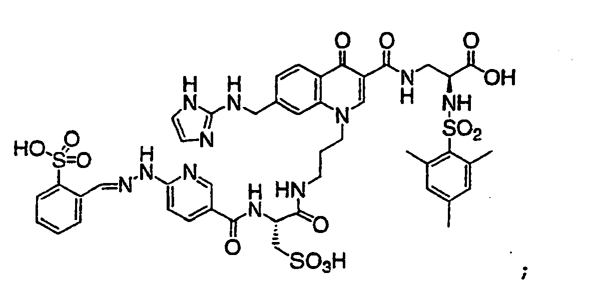 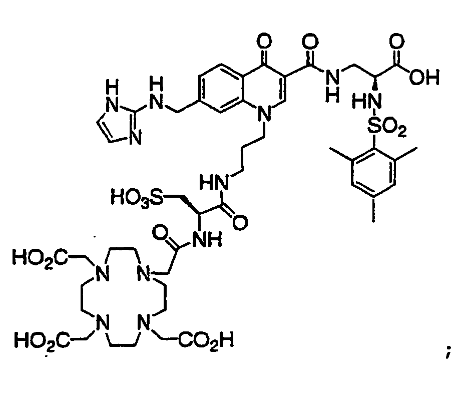 and 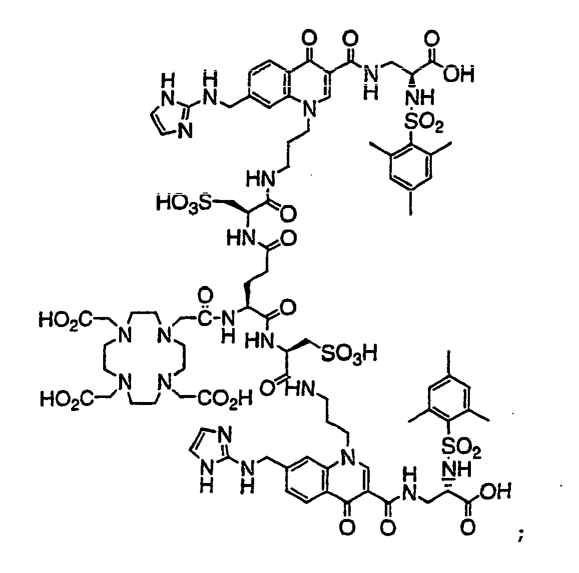
or a pharmaceutically acceptable salt form thereof.

9. A kit according to Claim 1, wherein the kit further comprises one or more ancillary ligands and a reducing agent.

10. A kit according to Claim 9, wherein the ancillary ligands are tricine and TPPTS.

11. A kit according to Claim 9, wherein the reducing agent is tin(II).

12. A therapeutic radiopharmaceutical composition comprising at least one agent selected from the group consisting of an anti-cancer agent and a radiosensitizer agent, or a pharmaceutically acceptable salt thereof, and a radiopharmaceutical comprising:
a) a metal;
b) a chelator capable of chelating the metal; and
c) a targeting moiety;
wherein the targeting moiety is bound to the chelator through 0-1 linking groups, and the targeting moiety is a quinolone non-peptide that binds to a receptor that is upregulated during angiogenesis..

13. A therapeutic radiopharmaceutical composition according to claim 12, wherein the anti-cancer agent is selected from the group consisting of mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, and leutinizing hormone releasing factor.

14. A therapeutic radiopharmaceutical composition according to claim 12, wherein radiosensitizer agent is selected from the group consiting of 2-(3-nitro-1,2,4-triazol-1-yl)-N-(2-methoxyethyl)acetamide, N-(3-nitro-4-quinolinyl)-4-morpholinecarboxamidine, 3-amino-1,2,4-benzotriazine-1,4-dioxide, N-(2-hydroxyethyl)-2-nitroimidazole-1-acetamide, 1-(2-nitroimidazol-1-yl) -3-(1-piperidinyl)-2-propanol, and 1-(2-nitro-1-imidazolyl)-3-(1-aziridino)-2-propanol.

15. A therapeutic radiopharmaceutical composition according to claim 12, wherein the metal is selected from the group ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir, and the linking group is present between the non-peptide targeting moiety and chelator.

16. A therapeutic radiopharmaceutical composition according to Claim 15, wherein the targeting moiety is a quinolone non-peptide and the receptor is αᵥβ₃ or αᵥβ₅.

17. A therapeutic radiopharmaceutical composition according to claim 12, wherein the radiopharmaceutical comprises:
b) a metal selected from the group ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir; and
b) a compound of the formula (I):
(Q)_{d}-Lₙ-Cₕ or (Q)_{d}-Lₙ-(Cₕ)_{d'} (I)
wherein, Q is a compound of Formula (II): 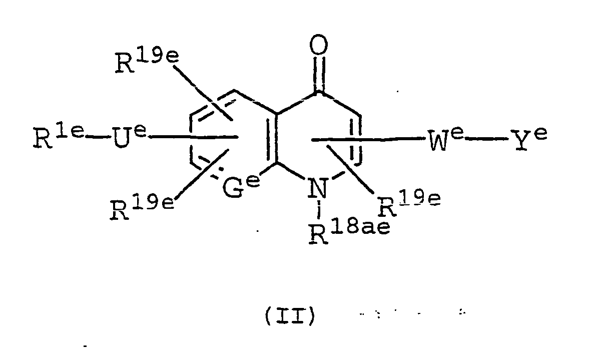 including stereoisomeric forms thereof, or mixtures of stereoisomeric forms thereof, or pharmaceutically acceptable salt or prodrug forms thereof wherein:
R^{1e} is selected from: 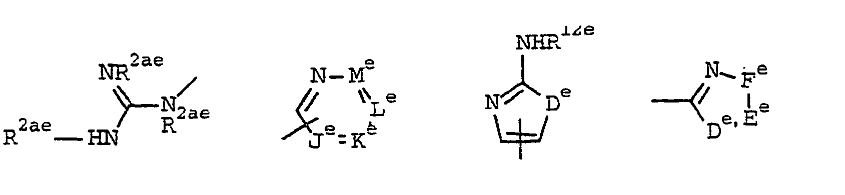 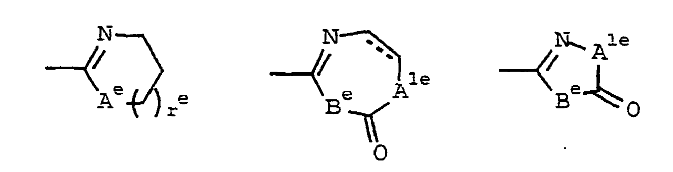 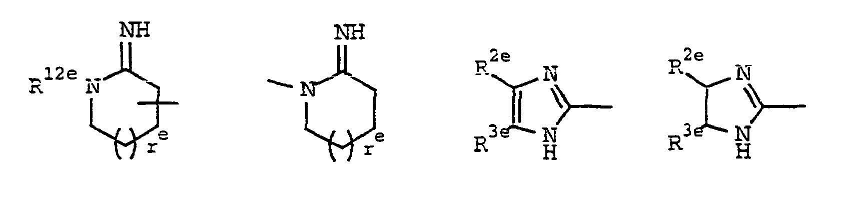 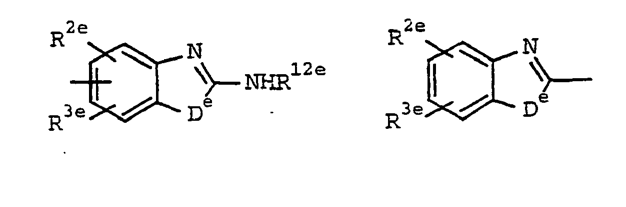 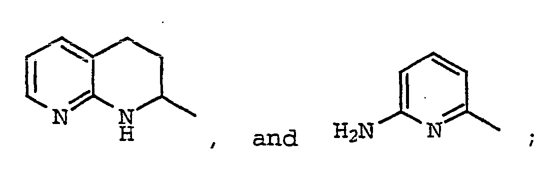
A^{e} is -CH₂- or -N(R^{10e})-;
A^{1e} and B^{e} are independently -CH₂- or -N(R^{10e})-;
D^{e} is -N(R^{10e})- or -S-;
E^{e}-F^{e} is -C(R^{2e})=C(R^{3e})- or -C(R^{2e})₂C(R^{3e})₂-;
J^{e} is -C(R^{2e}) - or -N-;
K^{e}, L^{e} and M^{e} are independently -C(R^{2e})- or -C(R^{3e})-;
R^{2e} and R^{3e} are independently selected from: . H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl (C₁-C₄ alkyl), aryl (C₁-C₆ alkyl)-, (C₁-C₆ alkyl)carbonyl, (C₁-C₆ alkoxy) carbonyl, arylcarbonyl, and aryl substituted with 0-4 R^{7e},
alternatively, when R^{2e} and R^{3e} are substituents on adjacent atoms, they can be taken together with the carbon atoms to which they are attached to form a . 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
R^{2ae} is selected from:
H, C₁-C₁₀ alkyl, C₂-C₆ alkenyl, C₃-C₁₁ cycloalkyl, C₃-C₇ cycloalkyl (C₁-C₄ alkyl), aryl, aryl (C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl (C₁-C₁₀ alkoxy) carbonyl, C₁-C₆ alkylcarbonyloxy (C₁-C₄ alkoxy) carbonyl, arylcarbonyloxy(C₁-C₄ alkoxy) carbonyl, and C₃-C₇ cycloalkylcarbonyloxy (C₁-C₄ alkoxy) carbonyl;
R^{7e} is selected from:
H, hydroxy, C₁-C₄ alkyl, C₁-C₄ alkoxy, aryl, aryl (C₁-C₄ alkyl)-, (C₁-C₄ alkyl) carbonyl, CO₂R^{18ae}, SO₂R^{11e}, SO₂NR^{10e}R^{11e}, OR^{10e}, and . N(R^{11e})R^{12e};
U^{e} is selected from:
-(CH₂)ₙ^{e}-, -(CH₂)ₙ^{e}O(CH₂)ₘ^{e}-,-(CH₂)ₙ^{e}N(R¹²)(CH₂)ₘ^{e}-, -NH(CH₂)ₙ^{e}-,-(CH₂)ₙ^{e}C(=O)(CH₂)ₘ^{e}-, -(CH₂)ₙ^{e}S(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})-, and -N(R^{10e})S(O)ₚ^{e}-;
G^{e} is N or CR^{19e};
W^{e} is-C(=O)-N(R^{10e})-(C₁-C₃ alkylene)-, in which the alkylene group is substituted by R^{8e} and by R^{9e}:
R^{8e} and R^{9e} are independently selected from:
H, CO₂R^{18be}, C(=O)R^{18be}, CONR¹⁷R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl) carbonyl, C₃-C₁₀ cycloalkyl (C₁-C₄ alkyl)-, phenyl substituted with 0-3 R^{6e}, naphthyl substituted with 0-3 R^{6e},
a 5-10 membered heterocyclic ring containing 1-3 N, 0, or S heteroatoms, wherein said heterocyclic ring may be saturated, partially saturated, or fully unsaturated, said heterocyclic ring being substituted with 0-2 R^{7e},
C₁-C₁₀ alkoxy substituted with 0-2 R^{7e}, hydroxy, nitro, -N(R^{10e})R^{11e}, -N(R^{16e})R^{17e}, aryl (C₀-C₆ alkyl)carbonyl, aryl(C₃-C₆ alkyl), heteroaryl (C₁-C₆ alkyl), CONR^{18ae}R^{20e}, SO₂R^{18ae}, and SO₂NR^{18ae}R^{20e},
providing that any of the above alkyl, cycloalkyl, aryl or heteroaryl groups may be unsubstituted or substituted independently with 1-2 R^{7e};
R^{6e} is selected from:
H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
aryl substituted with 0-3 groups selected from halogen, C₁-C₆ alkoxy, C₁-C₆ alkyl, CF₃, S(O)ₘ^{e}Me, and -NMe₂,
aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
R^{10e} is selected from:
H, -OH, CF₃, C₃-C₆ alkenyl, C₃-C₁₁ cycloalkyl, aryl, (C₃-C₁₁ cycloalkyl)methyl, aryl (C₁-C₄ alkyl), and C₁-C₁₀ alkyl substituted with 0-2 R^{6e};
R^{11e} is selected from:
H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl (C₁-C₄ alkyl)-, aryl (C₁-C₄ alkyl), adamantylmethyl, and C₁-C₁₀ alkyl substituted with 0-2 R^{4e};
R^{4e} is selected from:
H, C₁-C₆ alkyl, C₃-C₇ cycloalkyl, C₃-C₇ cycloalkyl (C₁-C₄ alkyl)-, (C₁-C₁₀ alkyl)carbonyl, aryl, heteroaryl, aryl (C₁-C₆ alkyl)-, and heteroaryl (C₁-C₆ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
alternatively, when R^{10e} and R^{11e} are both substituents on the same nitrogen atom (as in -NR^{10e}R^{11e}) they may be taken together with the nitrogen atom to which they are attached to form a heterocycle selected from: 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl, and 1-piperazinyl;
said heterocycle being substituted with 0-3 groups selected from: C₁-C₆ alkyl, aryl, heteroaryl, aryl (C₁-C₄ alkyl)-, (C₁-C₆ alkyl)carbonyl, (C₃-C₇ cycloalkyl)carbonyl, (C₁-C₆ alkoxy)carbonyl, aryl (C₁-C₄ alkoxy)carbonyl, C₁-C₆ alkylsulfonyl, and arylsulfonyl;
R^{12e} is selected from:
H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy) carbonyl, (C₁-C₆ alkyl)aminocarbonyl, C₃-C₆ alkenyl, C₃-C₇ cycloalkyl, C₃-C₇ cycloalkyl (C₁-C₄ alkyl)-, aryl, heteroaryl (C₁-C₆ alkyl)carbonyl, heteroarylcarbonyl, aryl (C₁-C₆ alkyl)-, (C₁-C₆ alkyl)carbonyl, arylcarbonyl, C₁-C₆ alkylsulfonyl, arylsulfonyl, aryl (C₁-C₆-alkyl) sulfonyl, heteroarylsulfonyl, heteroaryl (C₁-C₆ alkyl)sulfonyl, aryloxycarbonyl, and aryl (C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
R^{16e} is selected from:
-C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, and -SO₂NHC(=O)OR^{18be};
R^{17e} is selected from:
H, C₁-C₆ alkyl, C₃-C₇ cycloalkyl, C₃-C₇ cycloalkyl (C₁-C₄ alkyl)-, aryl, aryl (C₁-C₆ alkyl)-, and heteroaryl (C₁-C₆ alkyl);
R^{18ae} is selected from:
C₁-C₈ alkyl optionally substituted with a bond to Lₙ, C₃-C₁₁ cycloalkyl optionally substituted with a bond to Lₙ, aryl (C₁-C₆ alkyl)-optionally substituted with a bond to Lₙ, heteroaryl (C₁-C₆ alkyl)- optionally substituted with a bond to Lₙ, (C₁-C₆ alkyl)heteroaryl optionally substituted with a bond to Lₙ, biaryl (C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
R^{18be} is H or R^{18ae};
R^{19e} is selected from:
H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl (C₁-C₄ alkyl)-, aryl (C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
R^{20e} is selected from:
hydroxy, C₁-C₁₀ alkyloxy, C₃-C₁₁ cycloalkyloxy, aryloxy, aryl (C₁-C₄ alkyl)oxy, C₂-C₁₀ alkylcarbonyloxy (C₁-C₂ alkyl)oxy-, C₂-C₁₀ alkoxycarbonyloxy (C₁-C₂ alkyl)oxy-, C₂-C₁₀ alkoxycarbonyl (C₁-C₂ alkyl)oxy-, C₃-C₁₀ cycloalkylcarbonyloxy (C₁-C₂ alkyl)oxy-, C₃-C₁₀ cycloalkoxycarbonyloxy (C₁-C₂ alkyl)oxy-, C₃-C₁₀ cycloalkoxycarbonyl (C₁-C₂ alkyl)oxy-, aryloxycarbonyl (C₁-C₂ alkyl)oxy-, aryloxycarbonyloxy (C₁-C₂ alkyl)oxy-, arylcarbonyloxy (C₁-C₂ alkyl)oxy-, C₁-C₅ alkoxy (C₁-C₅ alkyl) carbonyloxy (C₁-C₂ alkyl) ox y,
(5-(C₁-C₅ alkyl)-1,3-dioxa-cyclopenten-2-one-yl)methyloxy,
(5-aryl-1,3-dioxa-cyclopenten-2-one-yl)methyloxy, and
(R^{10e})(R^{11e})N-(C₁-C₁₀ alkoxy)-;
R^{21e} is selected from:
C₁-C₈ alkyl, C₂-C₆ alkenyl, C₃-C₁₁ cycloalkyl, (C₃-C₁₁ cycloalkyl)methyl, aryl, aryl (C₁-C₄ alkyl)-, and C₁-C₁₀ alkyl substituted with 0-2 R^{7e};
R^{22e} is selected from:
-C(=O)-R^{18be}, -C(=O)N(R^{18be})₂, -C(=O)NHSO₂R^{18ae},-C(=O)NHC(=O)R^{18be}, -C(=O)NHC(=O)OR^{18ae}, and-C(=O)NHSO₂NHR^{18be};
Y^{e} is selected from:
-COR^{20e}, -SO₃H, -PO₃H, -CONHNHSO₂CF₃,-CONHSO₂R^{18ae}, -CONHSO₂NHR^{18be}, -NHCOCF₃,-NHCONHSO₂R^{18ae}, -NHSO₂R^{18ae}, -OPO₃H₂, -OSO₃H,-PO₃H₂, -SO₂NHCOR^{18ae}, -SO₂NHCO₂R^{18ae}, 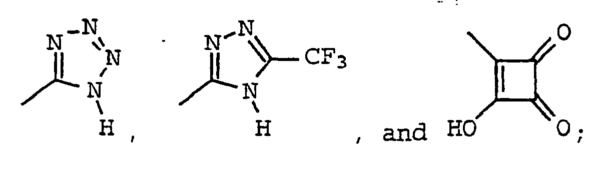
m^{e} is 0-2;
n^{e} is 0-4;
p^{e} is 0-2;
r^{e} is 0-2;
with the following proviso: n^{e} and m^{e} are chosen such that the number of atoms connecting R^{1e} and Y^{e} is in the range of 8-14;
d is selected from 1, 2, 3, 4, 5, 6, 7, 8, 9, and 10;
d' is 1-100;
Lₙ is a linking group having the formula:
((W)ₕ-(CR⁶R⁷)_{g})ₓ-(Z)ₖ-((CR^{6a}R^{7a})_{g'}-(W)_{h'})_{x'};
W is independently selected at each occurrence from the group: O, S, NH, NH(=O), C(=O)NH, NR⁸C(=O), C(=O)NR⁸, C(=O), C(=O)O, OC(=O), NHC (=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ, and (aa)_{t'};
aa is independently at each occurrence an amino acid;
Z is selected from the group: aryl substituted with 0-3 R¹⁰, C₃₋₁₀ cycloalkyl substituted with 0-3 R¹⁰, and a 5-10 membered heterocyclic ring system containing 1-4 heteroatoms independently selected from N, S, and 0 and substituted with 0-3 R¹⁰;
R⁶, R^{6a}, R⁷, R^{7a}, and R⁸ are independently selected at each occurrence from the group: H, =O, COOH, SO₃H, PO₃H, C₁-C₅ alkyl substituted with 0-3 R¹⁰, aryl substituted with 0-3 R¹⁰, benzyl substituted with 0-3 R¹⁰, and C₁-C₅ alkoxy substituted with 0-3 R¹⁰, NHC(=O)R¹¹, C(=O)NHR¹¹, NHC(=O)NHR¹¹, NHR¹¹, R¹¹, and a bond to Cₕ;
R¹⁰ is independently selected at each occurrence from the group: a bond to Cₕ, COOR¹¹, C(=O)NHR¹¹, NHC(=O)R¹¹, OH, NHR¹¹, SO₃H, PO₃H, -OPO₃H₂, -OSO₃H, aryl substituted with 0-3 R¹¹, C₁₋₅ alkyl substituted with 0-1 R¹², C₁₋₅ alkoxy substituted with 0-1 R¹², and a 5-10 membered heterocyclic ring system containing 1-4 heteroatoms independently selected from N, S, and 0 and substituted with 0-3 R¹¹;
R¹¹ is independently selected at each occurrence from the group: H, -OPO₃H₂,, -OSO₃H C₁₋₅ alkyl substituted with 0-1 R¹², aryl substituted with 0-1 R¹², a 5-10 membered heterocyclic ring system containing 1-4 heteroatoms independently selected from N, S, and O and substituted with 0-1 R¹², C₃₋₁₀ cycloalkyl substituted with 0-1 R¹², polyalkylene glycol substituted with 0-1 R¹²,, C₁-₅ alkyl substituted with carbohydrate substituted with 0-1 R¹², cyclodextrin substituted with 0-1 R¹², amino acid substituted with 0-1 R¹², polycarboxyalkyl substituted with 0-1 R¹², polyazaalkyl substituted with 0-1 R¹², peptide substituted with 0-1 R¹², wherein the peptide is comprised of 2-10 amino acids, 3, 6-O-disulfo-B-D-galactopyranosyl, bis(phosphonomethyl)glycine, and a bond to Cₕ;
R¹² is a bond to Cₕ;
k is selected from 0, 1, and 2;
h is selected from 0, 1, and 2;
h' is selected from 0, 1, and 2;
g is selected from 0, 1, 2, 3, 4, 5, 6, 7, 8, 9, and 10;
g' is selected from 0, 1, 2, 3, 4, 5, 6, 7, 8, 9, and 10;
s is selected from 0, 1, 2, 3, 4, 5, 6, 7, 8, 9, and 10;
s' is selected from 0, 1, 2, 3, 4, 5, 6, 7, 8, 9, and 10;
s" is selected from 0, 1, 2, 3, 4, 5, 6, 7, 8, 9, and 10;
t is selected from 0, 1, 2, 3, 4, 5, 6, 7, 8, 9, and 10;
t' is selected from 0, 1, 2, 3, 4, 5, 6, 7, 8, 9, and 10;
x is selected from 0, 1, 2, 3, 4, 5, and 6;
x' is selected from 0, 1, 2, 3, 4, 5, and 6;
Cₕ is a metal bonding unit having a formula selected from the group: 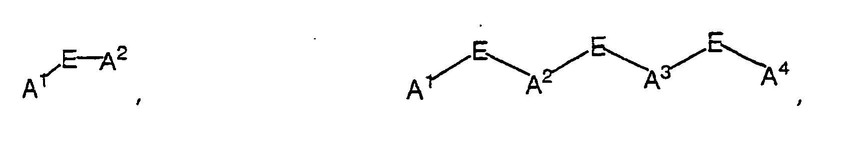 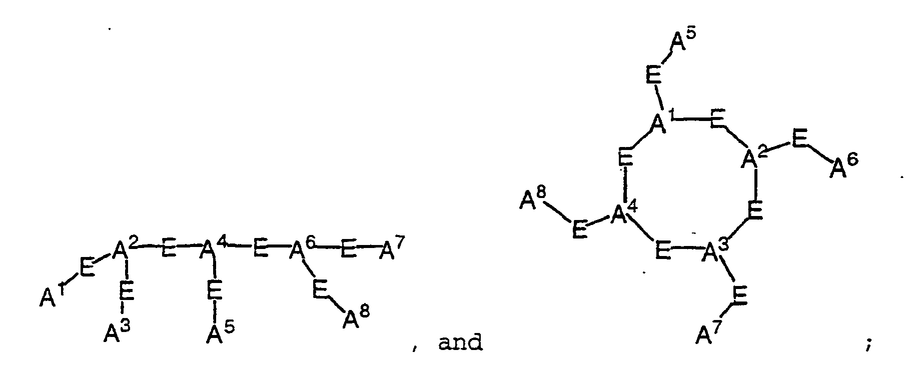
A¹, A², A³, A⁴, A⁵, A⁶, A⁷, and A⁸ are independently selected at each occurrence from the group: NR¹³, NR¹³R¹⁴, S, SH, S(Pg), O, OH, PR¹³, PR¹³R¹⁴, P(O)R¹⁵R¹⁶, and a bond to Lₙ;
E is a bond, CH, or a spacer group independently selected at each occurrence from the group: C₁-C₁₀ alkyl substituted with 0-3 -R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
R¹³ and R¹⁴ are each independently selected from the group: a bond to Lₙ, hydrogen, C₁-C₁₀ alkyl substituted with 0-3 R¹⁷, aryl substituted with 0-3 R¹⁷, C₁₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
alternatively, R¹³ and R¹⁴ combine to form =C(R²⁰) (R²¹);
R¹⁵ and R¹⁶ are each independently selected from the group: a bond to Lₙ, -OH, C₁-C₁₀ alkyl substituted with 0-3 R¹⁷, C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and 0 and substituted with 0-3 R¹⁷;
R¹⁷ is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H, 2-(1-morpholino)ethoxy, C₁-C₅ alkyl, C₂-C₄ alkenyl, C₃-C₆ cycloalkyl, C₃-C₆ cycloalkylmethyl, C₂-C₆ alkoxyalkyl, aryl substituted with 0-2 R¹⁸, and a 5-10 membered heterocyclic ring system containing 1-4 heteroatoms independently selected from N, S, and O ;
R¹⁸, R^{18a}, and R¹⁹ are independently selected at each occurrence from the group: a bond to Lₙ, H, C₁-C₆ alkyl, phenyl, benzyl, C₁-C₆ alkoxy, halide, nitro, cyano, and trifluoromethyl;
Pg is a thiol protecting group;
R²⁰ and R²¹ are independently selected from the group: H, C₁-C₁₀ alkyl, -CN, -CO₂R²⁵, -C(=O)R²⁵, -C(=O)N(R²⁵)₂, C₂-C₁₀ 1-alkene substituted with 0-3 R²³, C₂-C₁₀ 1-alkyle substituted with 0-3 R²³, aryl substituted with 0-3R²³, unsaturated 5-10 membered heterocyclic ring system containing 1-4 heteroatoms independently selected from N, S, and 0 and substituted with 0-3 R²³, and unsaturated C₃₋₁₀ carbocycle substituted with 0-3 R²³;
alternatively, R²⁰ and R²¹, taken together with the divalent carbon radical to which they are attached form: 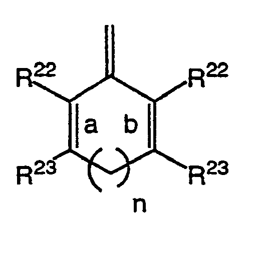
R²² and R²³ are independently selected from the group: H, R²⁴, C₁-C₁₀ alkyl substituted with 0-3 R²⁴, C₂-C₁₀ alkenyl substituted with 0-3 R²⁴, C₂-C₁₀ alkynyl substituted with 0-3 R²⁴, aryl substituted with 0-3 R²⁴, a 5-10 membered heterocyclic ring system containing 1-4 heteroatoms independently selected from N, S, and 0 and substituted with 0-3 R²⁴, and C₃₋₁₀ carbocycle substituted with 0-3 R²⁴;
alternatively, R²², R²³ taken together form a fused aromatic or a 5-10 membered heterocyclic ring system containing 1-4 heteroatoms independently selected from N, S, and O;
a and b indicate the positions of optional double bonds and n is 0 or 1;
R²⁴ is independently selected at each occurrence from the group: =0, F, Cl, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, and 2-(1-morpholino)ethoxy; and,
R²⁵, R^{25a}, and R²⁶ are each independently selected at each occurrence from the group: hydrogen and C₁-C₆ alkyl; or a pharmaceutically acceptable salt thereof.

18. A therapeutic radiopharmaceutical composition according to claim 17, wherein Q is a compound of Formula (IV): 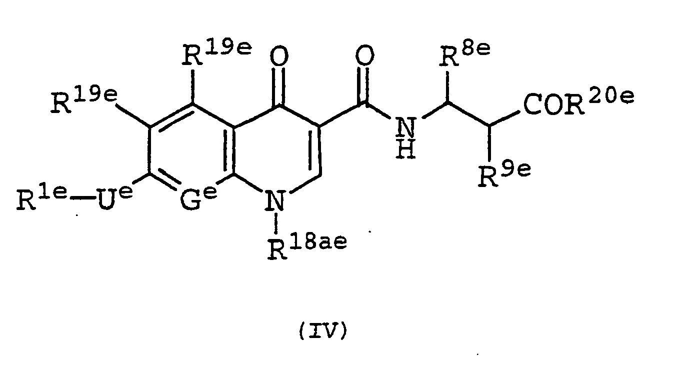 including stereoisomeric forms thereof, or mixtures of stereoisomeric forms thereof, or pharmaceutically acceptable salt or prodrug forms thereof wherein:
R^{1e} is selected from: 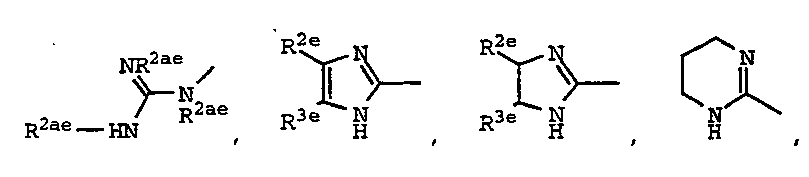 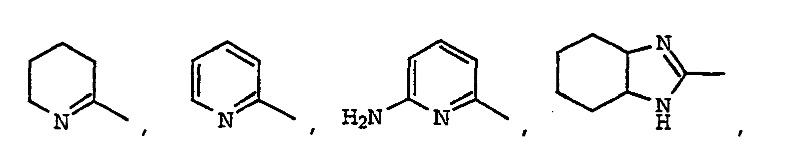 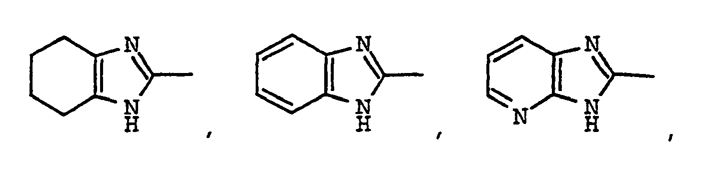 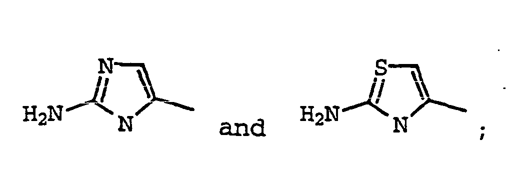
R^{2e} and R^{3e} are independently selected from: H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
R^{2ae} is selected from:
H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
R^{7e} is selected from:
H, hydroxy, C₁-C₄ alkyl, C₁-C₄ alkoxy, aryl, aryl(C₁-C₄ alkyl)-, (C₁-C₄ alkyl)carbonyl, C0₂R^{18ae}, SO₂R^{11e}, SO₂NR^{10e}R^{11e}, OR^{10e}, and N(R^{11e})R^{12e};
U^{e} is selected from:
- (CH₂)ₙ^{e}-, - (CH₂)ₙ^{e}O(CH₂)ₘ^{e}-, -NH(CH₂)ₙ^{e}-, -N(R^{10e})C(=O)-, -NHC(=O)(CH₂)ₙ^{e}-, and-C(=O)N(R^{10e})-;
G^{e} is N or CR^{19e};
R^{8e} is selected from:
H, CO₂R^{18be}, C(=O)R^{18be}, CONR^{17e}R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl, C₃-C₁₀ cycloalkyl(C₁-C₄ alkyl)-, phenyl substituted with 0-3 R^{6e}, naphthyl substituted with 0-3 R^{6e},
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
R^{9e} is selected from:
C₁-C₁₀ alkyl substituted with 0-1 R^{6e},
C₁-C₁₀ alkoxy substituted with 0-2 R^{7e},
H, nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, hydroxy, OR^{22e}, -N(R^{10e})R^{11e}, -N(R^{16e})R^{17e}, aryl(C₀-C₆ alkyl)carbonyl, aryl(C₁-C₆ alkyl), heteroaryl(C₁-C₆ alkyl), CONR^{18ae}R^{20e}, SO₂R^{18ae}, and SO₂NR^{18ae}R^{20e},
providing that any of the above alkyl, cycloalkyl, aryl or heteroaryl groups may be unsubstituted or substituted independently with 1-2 R^{7e};
R^{6e} is selected from:
H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O) R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚ^{e}R^{11e}, SO₂NR^{10e}R^{11e},
aryl substituted with 0-3 groups selected from halogen, C₁-C₆ alkoxy, C₁-C₆ alkyl, CF₃, S(O)ₘ^{e}Me, and -NMe₂,
aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
a 5-10 membered heterocyclic ring containing 1-3 N, 0, or S heteroatoms, wherein said heterocyclic ring may be saturated, partially saturated, or fully unsaturated, said heterocyclic ring being substituted with 0-2 R^{7e};
R^{10e} is selected from:
H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
R^{11e} is selected from:
H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
R^{4e} is selected from:
H, C₁-C₆ alkyl, C₃-C₇ cycloalkyl, C₃-C₇ cycloalkyl(C₁-C₄ alkyl)-, aryl, heteroaryl, aryl(C₁-C₆ alkyl)-, and heteroaryl(C₁-C₆ alkyl)-,
wherein said aryl or heteroaryl groups are substituted with 0-2 substituents independently selected from the group consisting of C₁-C₄ alkyl, C₁-C₄ alkoxy, F, Cl, Br, CF₃, and NO₂,
R^{12e} is selected from:
H, C₁-C₆ alkyl, triphenylmethyl, methoxymethyl, methoxyphenyldiphenylmethyl, trimethylsilylethoxymethy , (C₁-C₆ alkyl)carbonyl, (C₁-C₆ alkoxy)carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
R^{16e} is selected from:
-C(=O)OR^{18ae}, -C(=O)R^{18be}, -C(=O)N(R^{18be})₂,-SO₂R^{18ae}, and -SO₂N(R^{18be})₂;
R^{17e} is selected from:
H, C₁-C₆ alkyl, C₃-C₇ cycloalkyl, C₃-C₇ cycloalkyl(C₁-C₄ alkyl)-, aryl, aryl(C₁-C₆ alkyl)-, and heteroaryl(C₁-C₆ alknyl) ;
R^{18ae} is selected from:
C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
R^{18be} is H or R^{18ae};
R^{19e} is selected from:
H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and hetercaryl-SO₂-, wherein said aryl and heteroaryl groups are substituted with 0-4 groups selected from hydrogen, halogen, CF₃, C₁-C₃ alkyl, and C₁-C₃ alkoxy;
R^{20e} is selected from:
hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
(5-(C₁-C₅ alkyl)-1,3-dioxa-cyclopenten-2-one-yl)methyloxy,
(5-aryl-1,3-dioxa-cyclopenten-2-one-yl)methyloxy, and
(R^{10e}) (R^{11e})N-(C₁-C₁₀ alkoxy)-;
R^{21e} is selected from:
C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and
C₁-C₁₀ alkyl substituted with 0-2 R^{7e};
R^{22e} is selected from:
-C(=O)-R^{18be},-C(=O)N(R^{18be})₂,-C(=O)NHSO₂R^{18ae}, -C(=O)NHC(=O)R^{18be}, -C(=O)NHC(=O)OR^{18ae}, and -C(=O)NHSO₂NHR^{18be};
m^{e} is 0-2;
n^{e} is 0-4; and
p^{e} is 0-2;
with the following proviso: n^{e} and m^{e} are chosen such that the number of atoms connecting R¹ and -COR^{20e} in Formula (IV) is in the range of 8-14;
d is selected from 1, 2, 3, 4, and 5;
d' is 1-50;
W is independently selected at each occurrence from the group: O, NH, NHC(=O), C(=O)NH, NR⁸C(=O), C(=O)N R⁸, C(=O), C (=O) O, OC(=O), NHC (=S)NH, NHC(=O)NH, SO₂, (OCH₂CH₂)ₛ, (CH₂CH₂O)_{s'}, (OCH₂CH₂CH₂)_{s"}, (CH₂CH₂CH₂O)ₜ, and (aa)_{t'} ;
aa is independently at each occurrence an amino acid;
Z is selected from the group: aryl substituted with 0-1 R¹⁰, C₃₋₁₀ cycloalkyl substituted with 0-1 R¹⁰, and a 5-10 membered heterocyclic ring system containing 1-4 heteroatoms independently selected from N, S, and O and substituted with 0-1 R¹⁰;
R⁶, R^{6a}, R⁷, R^{7a}, and R⁸ are independently selected at each occurrence from the group: H, =O, COOH, SO₃H, C₁-C₅ alkyl substituted with 0-1 R¹⁰, aryl substituted with 0-1 R¹⁰, benzyl substituted with 0-1 R¹⁰, and C₁-C₅ alkoxy substituted with 0-1 R¹⁰, NHC(=O)R¹¹, C(=O)NHR¹¹, NHC(=O)NHR¹¹, NHR¹¹, R¹¹, and a bond to Cₕ;
k is 0 or 1;
s is selected from 0, 1, 2, 3, 4, and 5;
s' is selected from 0, 1, 2, 3, 4, and 5;
s" is selected from 0, 1, 2, 3, 4, and 5;
t is selected from 0, 1, 2, 3, 4, and 5;
A¹, A², A³, A⁴, A⁵, A⁶, A⁷, and A⁸ are independently selected at each occurrence from the group: NR¹³, NR¹³R¹⁴, S, SH, S(Pg), OH, and a bond to Lₙ;
E is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, and a 5-10 membered heterocyclic ring system containing 1-4 heteroatoms independently selected from N, S, and 0 and substituted with 0-3 R¹⁷;
R¹³, and R¹⁴ are each independently selected from the group: a bond to Lₙ, hydrogen, C₁-C₁₀ alkyl substituted with 0-3 R¹⁷, aryl substituted with 0-3 R¹⁷, a 5-10 membered heterocyclic ring system containing 1-4 heteroatoms independently selected from N, S, and 0 and substituted with 0-3 R¹⁷, and an electron, provided that when one of R¹³ or R¹⁴ is an electron, then the other is also an electron;
alternatively, R¹³ and R¹⁴ combine to form =C(R²⁰) (R²¹);
R¹⁷ is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)₂, -N(R¹⁸)₂, -NHC(=S)NHR¹⁸, =NOR¹⁸, -C(=O)NHNR¹⁸R^{18a}, -OCH₂CO₂H, and 2-(1-morpholino)ethoxy;
R¹⁸, R^{18a}, and R¹⁹ are independently selected at each occurrence from the group: a bond to Lₙ, H, and C₁-C₆ alkyl;
R²⁰ and R²¹ are independently selected from the group: H, C₁-C₅ alkyl, -CO₂R²⁵, C₂-C₅ 1-alkene substituted with 0-3 R²³, C₂-C₅ 1-alkyne substituted with 0-3 R²³, aryl substituted with 0-3 R²³, and unsaturated 5-10 membered heterocyclic ring system containing 1-4 heteroatoms independently selected from N, S, and O and substituted with 0-3 R²³;
alternatively, R²⁰ and R²¹, taken together with the divalent carbon radical to which they are attached form: 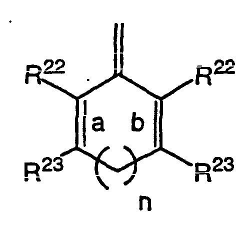
R²² and R²³ are independently selected from the group: H, and R²⁴;
alternatively, R²², R²³ taken together form a fused aromatic or a 5-10 membered heterocyclic ring system containing 1-4 heteroatoms independently selected from N, S, and O;
R²⁴ is independently selected at each occurrence from the group: -CO₂R²⁵, -C(=O)N(R²⁵)₂, -CH₂OR²⁵, -OC(=O)R²⁵, -OR²⁵, -SO₃H, -N(R²⁵)₂, and -OCH₂CO₂H; and,
R²⁵ is independently selected at each occurrence from the group: H and C₁-C₃ alkyl.

19. A therapeutic radiopharmaceutical composition according to claim 17, wherein:
R^{1e} is selected from: 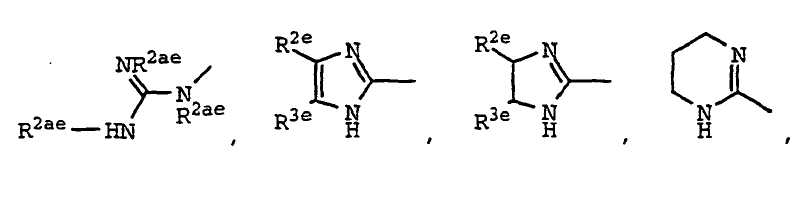 and 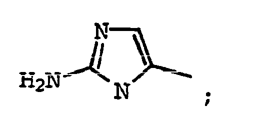
R^{2e} and R^{3e} are independently selected from: H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl (C₁-C₆ alkyl)-, (C₁-C₆ alkyl)carbonyl, (C₁-C₆ alkoxy)carbonyl, arylcarbonyl, and aryl substituted with 0-4 R^{7e},
alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
R^{2ae} is selected from:
H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
R^{7e} is selected from:
H, hydroxy, C₁-C₄ alkyl, C₁-C₄ alkoxy, aryl, aryl (C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
U^{e} is selected from:
-(CH₂)ₙ^{e}-, -NH(CH₂)ₙ^{e}-, -N(R^{10e})C(=O)-, and -NHC(=O)(CH₂)ₙ^{e};
G^{e} is N or CR^{19e};
R^{8e} is H;
R^{9e} is selected from:
H, nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, hydroxy, OR^{22e}, -N(R^{10e})R^{11e}, -N(R^{16e})R^{17e}, aryl(C₀-C₄ alkyl)carbonyl, aryl(C₁-C₄ alkyl), heteroaryl(C₁-C₄ alkyl), CONR^{18ae}R^{20e}, SO₂R^{18ae}, and SO₂NR^{18ae}R^{20e},
providing that any of the above alkyl, cycloalkyl, aryl or heteroaryl groups may be unsubstituted or substituted independently with 1-2 R^{7e};
R^{10e} is selected from:
H, -OH, CF₃, C₃-C₆ alkenyl, C₃-C₆ cycloalkyl, aryl, (C₃-C₆ cycloalkyl) methyl, aryl(C₁-C₄ alkyl), and C₁-C₄ alkyl substituted with 0-2 R^{6e};
R^{6e} is selected from:
H, C₁-C₄ alkyl, hydroxy, C₁-C₄ alkoxy, nitro, C₁-C₄ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
aryl substituted with 0-3 groups selected from halogen, C₁-C₄ alkoxy, C₁-C₄ alkyl, CF₃, S(O)ₘ^{e}Me, and -NMe₂,
aryl(C₁-C₄ alkyl)-, said aryl being substituted with 0-3 groups selected from halogen, C₁-C₄ alkoxy, C₁-C₄ alkyl, CF₃, S(O)ₚ^{e}Me, and -NMe₂, and
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
R^{11e} is selected from:
H, hydroxy, C₁-C₄ alkyl, C₃-C₆ alkenyl, C₃-C₆ cycloalkyl, (C₃-C₆ cycloalkyl)methyl, C₁-C₄ alkoxy, benzyloxy, aryl, heteroaryl, heteroaryl(C₁-C₄ alkyl)-, aryl(C₁-C₄ alkyl), adamantylmethyl, and C₁-C₄ alkyl substituted with 0-2 R^{4e};
R^{4e} is selected from:
H, C₁-C₄ alkyl, C₃-C₇ cycloalkyl, C₃-C₇ cycloalkyl (C₁-C₄ alkyl)-, aryl, heteroaryl, aryl(C₁-C₄ alkyl)-, and heteroaryl(C₁-C₄ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
R^{12e} is selected from:
H, C₁-C₄ alkyl, (C₁-C₄ alkyl)carbonyl, (C₁-C₄ alkoxy)carbonyl, phenyl(C₁-C₄ alkyl)-, phenylsulfonyl, phenyloxycarbonyl, and phenyl(C₁-C₄ alkoxy)carbonyl, wherein said phenyl groups are substituted with 0-2 substituents selected from the group consisting of C₁-C₄ alkyl, C₁-C₄ alkoxy, halo, CF₃, and nitro;
R^{16e} is selected from:
-C(=O)OR^{18ae} -C(=O)R^{18be}, -C(=O)N(R^{18be})₂,-SO₂R^{18ae}, and -SO₂N(R^{18be})₂;
R^{17e} is selected from:
H, C₁-C₄ alkyl, C₃-C₆ cycloalkyl, C₃-C₆ cycloalkyl(C₁-C₄ alkyl)-, aryl, aryl(C₁-C₆ alkyl)-, and heteroaryl(C₁-C₆ alkyl);
R^{18ae} is selected from:
C₁-C₈ alkyl optionally substituted with a bond to Lₙ, C₃-C₁₁ cycloalkyl optionally substituted with a bond to Lₙ, aryl (C₁-C₆ alkyl)-optionally substituted with a bond to Lₙ, heteroaryl (C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
R^{18be} is H or R^{18ae};
R^{19e} is selected from:
H, halogen, CF₃, CO₂H, CN, NO₂, -NR^{11e}R^{12e}, OCF3, C₁-C₆ alkyl, C₂-C₆ alkenyl, C₂-C₆ alkynyl, C₃-C₆ cycloalkyl, C3-C6 cycloalkyl(C₁-C₄ alkyl)-, aryl(C₁-C₄ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
R^{20e} is selected from:
hydroxy, C₁-C₆ alkyloxy, C₃-C₆ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
(5-(C₁-C₅ alkyl)-1,3-dioxa-cyclopenten-2-one-yl)methyloxy,
(5-aryl-1,3-dioxa-cyclopenten-2-one-yl)methyloxy, and
(R^{10e}) (R^{11e})N-(C₁-C₁₀ alkoxy)-;
R^{21e} is selected from:
C₁-C₄ Alkyl, C₂-C₆ alkenyl, C₃-C₆ cycloalkyl, (C₃-C₆ cycloalkyl)methyl, aryl, aryl(C₁-C₄ alkyl)-, and C₁-C₁₀ alkyl substituted with 0-2 R^{7e};
R^{22e} is selected from:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, and -C(=O)NHSO₂NHR^{18be};
m^{e} is 0-2;
n^{e} is 0-4;
p^{e} is 0-2;
Cₕ is 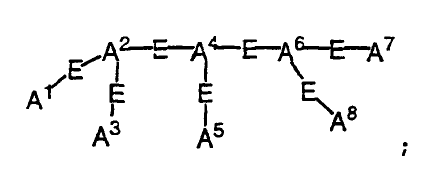
A¹ is selected from the group: OH, and a bond to Lₙ;
A², A⁴, and A⁶ are each N;
A³, A⁵, and A⁸ are each OH;
A⁷ is a bond to Lₙ or NH-bond to Lₙ;
E is a C₂ alkyl substituted with 0-1 R¹⁷;
R¹⁷ is =O;
alternatively, Cₕ is 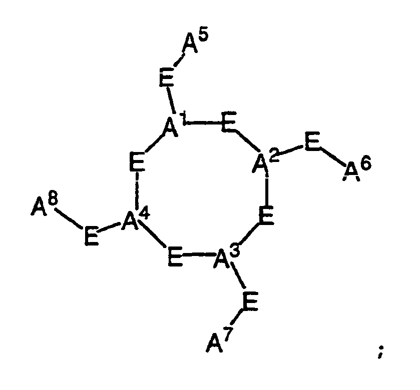
A¹ is selected from the group: OH, and a bond to Lₙ;
A², A³ and A⁴ are each N;
A⁵, A⁶ and A⁸ are each OH;
A⁷ is a bond to Lₙ;
E is a C₂ alkyl substituted with 0-1 R¹⁷;
R¹⁷ is =O;
alternatively, Cₕ is 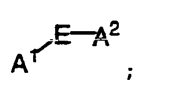
A¹ is NH₂ or N=C(R²⁰)(R²¹);
E is a bond;
A² is NHR¹³;
R¹³ is a heterocycle substituted with R¹⁷, the heterocycle being selected from pyridine and pyrimidine;
R¹⁷ is selected from a bond to Lₙ, C(=O)NHR¹⁸ and C(=O)R¹⁸;
R¹⁸ is a bond to Lₙ;
R²⁴ is selected from the group: -CO₂R²⁵, -OR²⁵, -SO₃H, and -N(R²⁵)₂; and,
R²⁵ is independently selected at each occurrence from the group: hydrogen and methyl.

20. A therapeutic radiopharmaceutical composition according to claim 17, wherein Q is selected from the group:
3-[7-[(imidazolin-2-ylamino)methyl]-1-methyl-6,8-difluoroquinoline-4-one-3-ylcarbonylamino]-2-(3,5-dimethylisoxazol-4-ylsulfonylamino)propionic acid,
3-[7-[(imidazolin-2-ylamino)methyl]-1-methyl-6,8-difluoroquinoline-4-one-3-ylcarbonylamino]-2-(benzyloxycarbonylamino)propionic acid,
3-[7-[(imidazolin-2-ylamino)methyl]-1-methyl-6,8-difluoroquinoline-4-one-3-ylcarbonylamino]-2-(n-butyloxycarbonylamino)propionic acid,
3-[7-[(imidazolin-2-ylamino)methyl]-1-methyl-6,8-difluoroquinoline-4-one-3-ylcarbonylamino]-2-(n-butylsulfonylamino)propionic acid,
3-[7-[(tetrahydropyrimid-2-ylamino)methyl]-1-methyl-6, 8-difluoroquinoline-4-one-3-ylcarbonylamino]-2-(benzyloxycarbonylamino)propionic acid,
3-[7-[(tetrahydropyrimid-2-ylamino)methyl]-1-methyl-6,8-difluoroquinoline-4-one-3-ylcarbonylamino]-2-(n-butyloxycarbonylamino)propionic acid,
3-[7-[(tetrahydropyrimid-2-ylamino)methyl]-1-methyl-6,8-difluoroquinoline-4-one-3-ylcarbonylamino]-2-(phenylsulfonylamino)propionic acid,
3-[7-[(tetrahydropyrimid-2-ylamino)methyl]-1-methyl-6,8-difluoroquinoline-4-one-3-ylcarbonylamino]-2-(n-butylsulfonyl)aminopropionic acid,
3-[7-[(2-aminothiazol-4-yl)methyl]-1-methyl-6,8-difluoroquinoline-4-one-3-ylcarbonylamino]-2-(benzyloxycarbonylamino)propionic acid,
3-[7-[(imidazolin-2-ylamino)methyl]-1-methyl-6,8-difluoroquinoline-4-one-3-ylcarbonylamino]-2-((2,4,6-trimethylphenyl)sulfonylamino)propionic acid,
3-[7-[(tetrahydropyrimid-2-ylamino)methyl]-1-methyl-6,8-difluoroquinoline-4-one-3-ylcarbonylamino]-2-((2,4,6-trimethylphenyl) sulfonylamino) propionic acid,
3-[7-[(imidazol-2-ylamino)methyl]-1-methyl-6,8-difluoroquinoline-4-one-3-ylcarbonylamino]-2-(3,5-dimethylisoxazol-4-ylsulfonylamino)propionic acid,
3-[7-[(imidazol-2-ylamino)methyl]-1-methyl-6,8-difluoroquinoline-4-one-3-ylcarbonylamino]-2-(benzyloxycarbonylamino)propionic acid,
3-[7-[(imidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(imidazol-2-ylamino)methyl]-1-methyl-6,8-difluoroquinoline-4-one-3-ylcarbonylamino]-2-((4-biphenyl)sulfonylamino)propionic acid,
3-[7-[(imidazol-2-ylamino)methyl]-1-methyl-6,8-difluoroquinoline-4-one-3-ylcarbonylamino]-2-(1-naphthylsulfonylamino)propionic acid,
3-[7-[(benzimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(4-methylimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(4,5-dimethylimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(4,5,6,7-tetrahydrobenzimidazol-2-ylamino)methyl]-1-methyl-6,8-difluoroquinoline-4-one-3-ylcarbonylainino]-2-((2,4,6-trimethylphenyl)sulfonylamino)propionic acid,
3-[7-[(pyridin-2-ylamino)methyl]-1-methyl-6,8-difluoroquinoline-4-one-3-ylcarbonylamino]-2-((2,4,6-trimethylphenyl)sulfonylamino)propionic acid,
3-[7-(2-aminopyridin-6-yl)-1-methyl-6,8-difluoroquinoline-4-one-3-ylcarbonylamino]-2-((2,4,6-trimethylphenyl)sulfonylamino)propionic acid,
3-[7-[(7-azabenzimidazol-2-yl)methyl]-1-methyl-6,8-difluoroquinoline-4-one-3-ylcarbonylaminol-2-((2,4,6-trimethylphenyl)sulfonylamino)propionic acid,
3-[7-[(benzimidazol-2-ylamino)methyl]-1-(2-phenylethyl)-6,8-difluoroquinoline-4-one-3-ylcarbonylamino]pro-pionic acid,
3-[7-[(pyridin-2-ylamino)methyl]-1-(2-phenylethyl)-6,8-difluoroquinoline-4-one-3-ylcarbonylamino]propionic acid,
3-[7-[(imidazolin-2-ylamino)methyl]-1-(2-phenylethyl)-6,8-difluoroquinoline-4-one-3-ylcarbonylamino]propionic acid,
3-[7-[(imidazol-2-ylamino)methyl]-1-(2-phenylethyl)-6,8-difluoroquinoline-4-one-3-ylcarbonylamino]propionic acid,
3-[7-[(imidazolin-2-ylamino)methyl]-1-(2-phenylethyl)-6,8-difluoroquinoline-4-one-3-ylcarbonylamino]-2-(benzyloxycarbonylamino)propionic acid,
3-[7-[(imidazolin-2-ylamino)methyl]-1-(2-phenylethyl)-6,8-difluoroquinoline-4-one-3-ylcarbonylaminol-2-(n-butyloxycarbonylamino)propionic acid,
3-[7-[(imidazolin-2-ylamino)methyl]-1-(2-phenylethyl)-6,8-difluoroquinoline-4-one-3-ylcarbonylamino]-2-(phenylsulfonylamino)propionic acid,
3-[7-[(imidazolin-2-ylamino)methyl]-1-(2-phenylethyl)-6,8-difluoroquinoline-4-one-3-ylcarbonylamino]-2-(n-butylsulfonylamino)propionic acid,
3-[7-[(tetrahydropyrimid-2-ylamino)methyl]-1-(2-phenylethyl)-6,8-difluoroquinoline-4-one-3-ylcarbonylamino]-2-(benzyloxycarbonylamino)propionic acid,
3-[7-[(tetrahydropyrimid-2-ylamino)methyl]-1-(2-phenylethyl)-6,8-difluoroquinoline-4-one-3-ylcarbonylamino]-2-(n-butyloxycarbonylamino)propionic acid,
3-[7-[(tetrahydropyrimid-2-ylamino)methyl]-1-(2-phenylethyl)-6,8-difluoroquinoline-4-one-3-ylcarbonylamino]-2-(phenylsulfonylamino)propionic acid,
3-[7-[(tetrahydropyrimid-2-ylamino)methyl]-1-(2-phenylethyl)-6,8-difluoroquinoline-4-one-3-ylcarbonylamino]-2-(n-butylsulfonyl)aminopropionic acid,
3-[7-[(2-aminothiazol-4-yl)methyl]-1-(2-phenylethyl)-6,8-difluoroquinoline-4-one-3-ylcarbonylamino]-2-(phenylsulfonylamino)propionic acid,
3-[7-[(2-aminothiazol-4-yl)methyl]-1-(2-phenylethyl)-6,8-difluoroquinoline-4-one-3-ylcarbonylamino]-2-(benzyloxycarbonylamino)propionic acid,
3-[7-[(imidazolin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(tetrahydropyrimid-2-ylamino)methyl]-1-(2-phenylethyl)-6,8-difluoroquinoline-4-one-3-ylcarbonylamino]-2-((2,4,6-trimethylphenyl)sulfon-ylamino)propionic acid,
3-[7-[(imidazol-2-ylamino)methyl]-1-(2-phenylethyl)-6,8-difluoroquinoline-4-one-3-ylcarbonylamino]-2-(benzyloxycarbonylamino)propionic acid,
3-[7-[(imidazol-2-ylamino)methyl]-1-(2-phenylethyl)-6,8-difluoroquinoline-4-one-3-ylcarbonylamino]-2-(phenylsulfonylamino)propionic acid,
3-[7-[(imidazol-2-ylamino)methyl]-1-(2-phenylethyl)-6,8-difluoroquinoline-4-one-3-ylcarbonylamino]-2-((2,6,dichlorophenyl)sulfonylamino)propionic acid,
3-[7-[(imidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(imidazol-2-ylamino)methyl]-1-(2-phenylethyl)-6,8-difluoroquinoline-4-one-3-ylcarbonylamino]-2-((4-biphenyl)sulfonylamino)propionic acid,
3-[7-[(benzimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(4-methylimidazol-2-ylamino)methyl]-1-(2-phenylethyl)-6,8-difluoroquinoline-4-one-3 ylcarbonylamino]-2-((2,4,6-trimethylphenyl)sulfonylamino)propionic acid,
3-[7-[(4,5-dimethylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(4,5,6,7-tetrahydrobenzimidazol-2-ylamino)methyl]-1-(2-phenylethyl)-6,8-difluoroquinoline-4-one-3-ylcarbonylamino]-2-((2,4,6-(trimethylphenyl) sulfonylamino) propionic acid,
3-[7-[(pyridin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-(2-aminopyridin-6-yl)-1-(2-phenylethyl)-6,8-difluoroquinoline-4-one-3-ylcarbonylamino]-2-((2,4,6-trimethylphenyl)sulfonylamino)propionic acid, and
3-[7-[(7-azabenzimidazol-2-yl)methyl]-1-(2-phenylethyl)-6,8-difluoroquinoline-4-one-3-ylcarbonylamino]-2-((2,4,6-trimethylphenyl)sulfonylamino)propionic acid

21. A composition according to Claim 15, wherein the radioisotope is ¹⁵³Sm.

22. A composition according to Claim 15, wherein the radioisotope is ¹⁷⁷Lu.

23. A composition according to Claim 22, wherein the radiopharmaceutical is selected from the group: 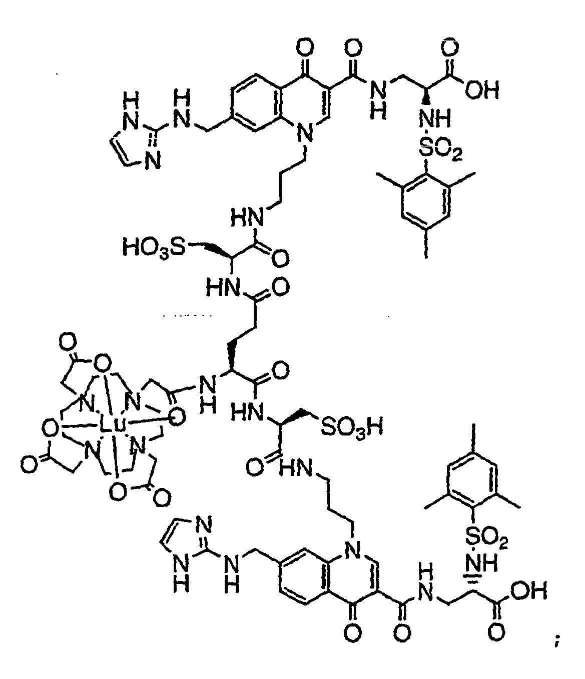 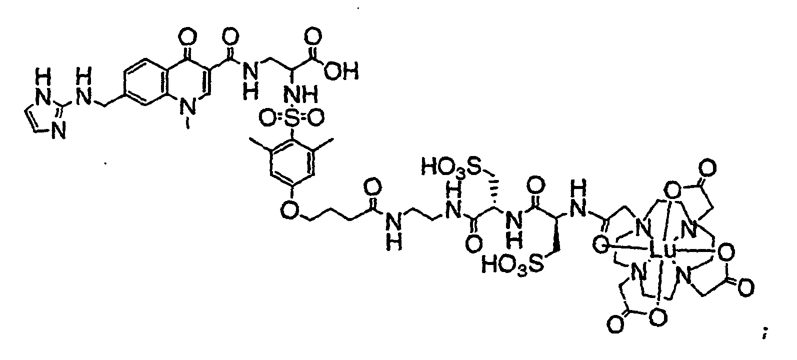 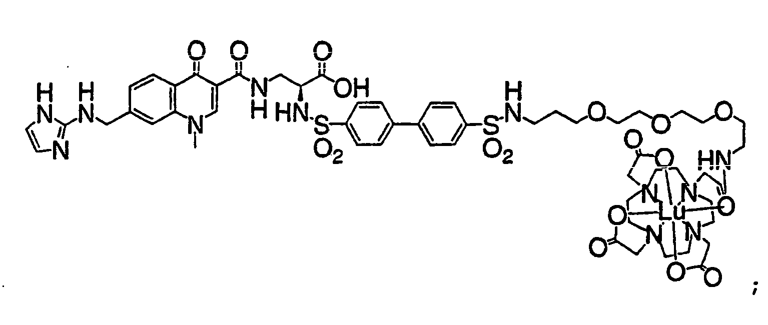 and

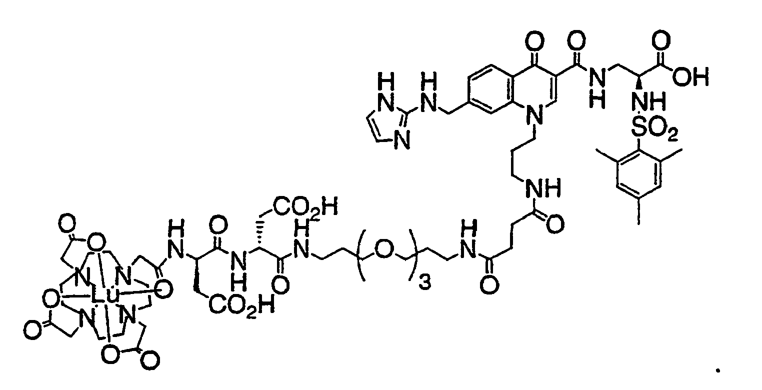

24. A composition according to Claim 15, wherein the radioisotope is ⁹⁰Y.

25. A composition according to Claim 24, wherein the radiopharmaceutical is selected from the group; 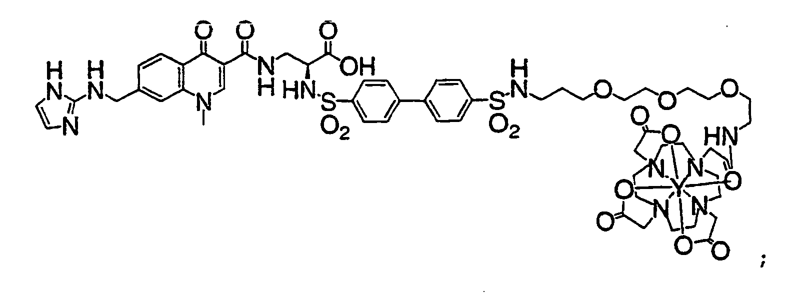 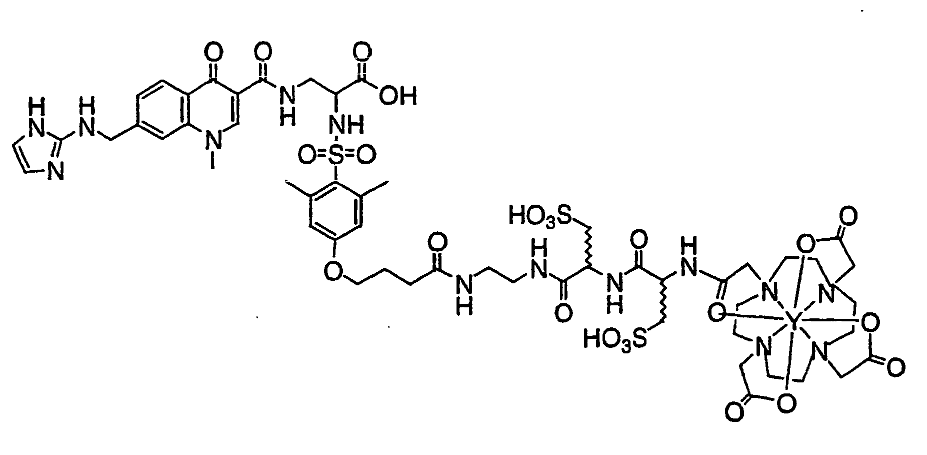 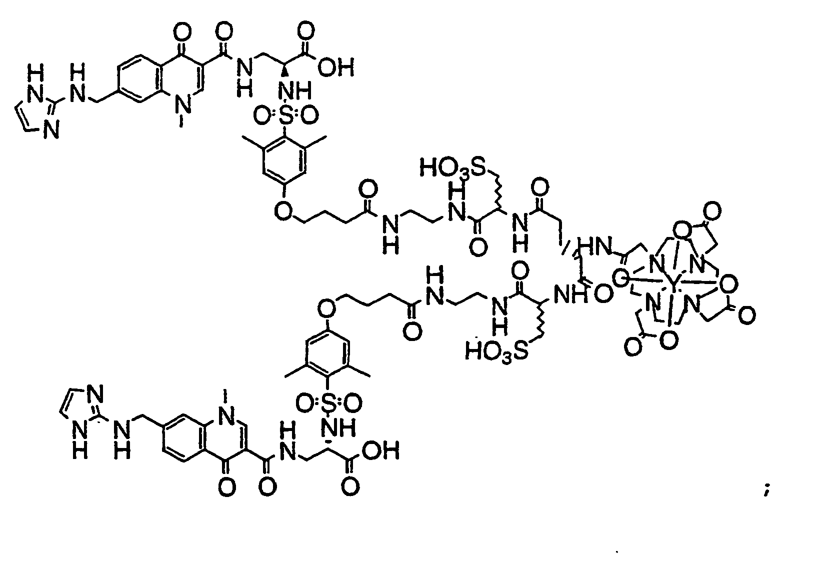 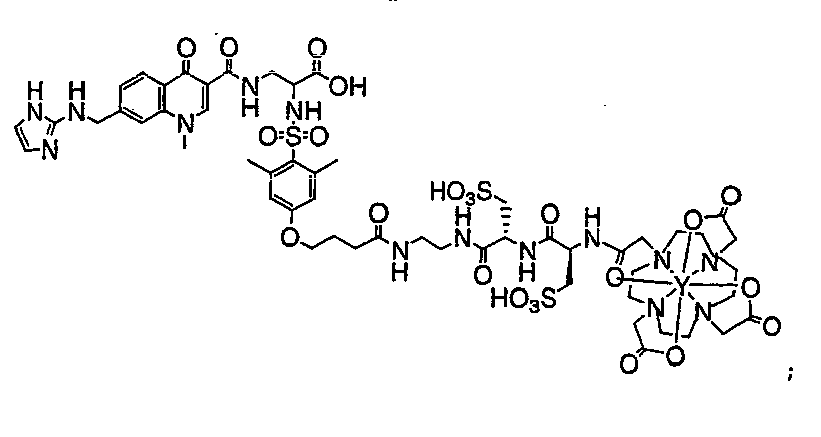 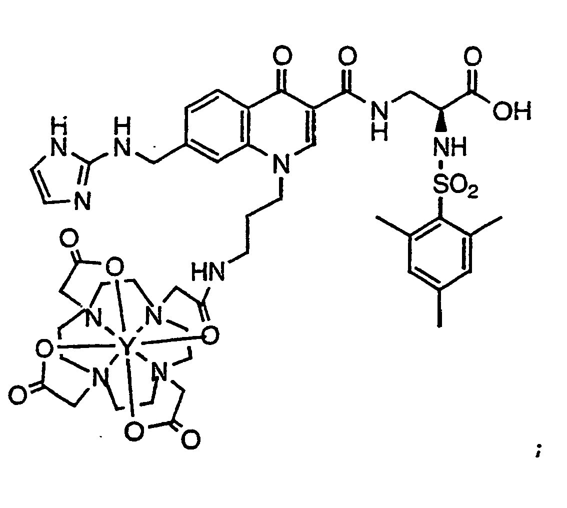 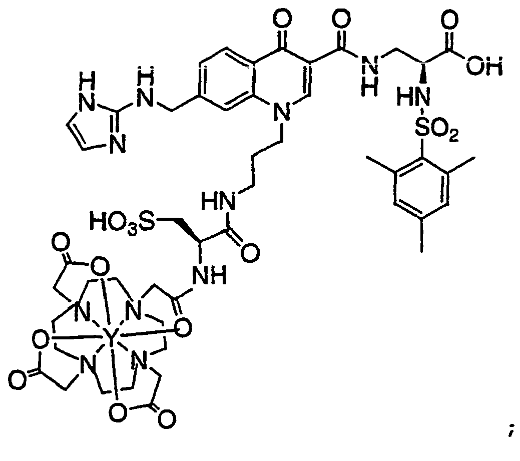 and

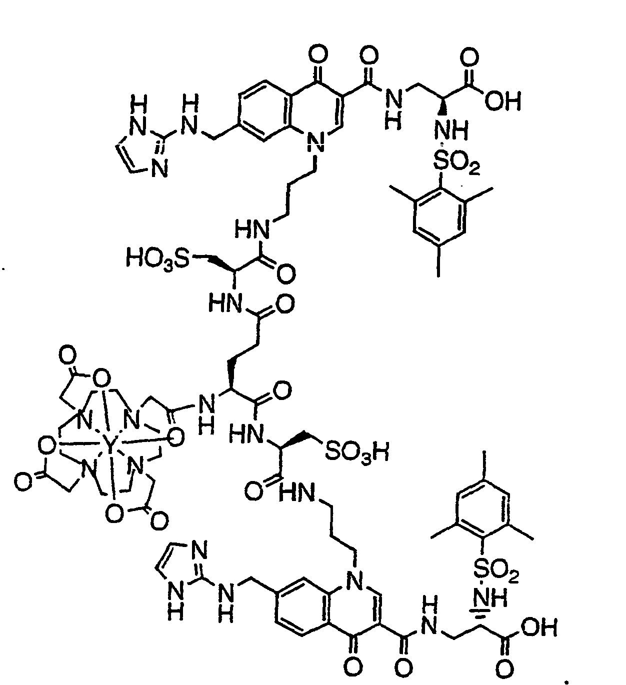

26. Use of for the manufacture of a medicament to be administered to a patient in need thereof for treating cancer. a therapeutic radiopharmaceutical comprising:
a) a metal;
b) chelator capable of chelating the metal; and
c) a targeting moiety;
wherein the targeting moiety is bound to the chelator through a linking group, and the targeting moiety is a quinolone non-peptide that binds to a receptor that is upregulated during angiogenesis, and the metal is a radioisotope selected from the group: ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir or a pharmaceutically acceptable salt thereof, and at least one agent selected from the group consisting of an anti-cancer agent and a radiosensitizer agent, or a pharmaceutically acceptable salt thereof

27. Use according to claim 26 wherein administering the therapeutic radiopharmaceutical and agent is concurrent.

28. Use according to claim 26 wherein administering the therapeutic radiopharmaceutical and agent is sequential.

29. Use according to claim 26 wherein the cancer is selected from the group consisting of carcinomas of the lung, breast, ovary, stomach, pancreas, larynx, esophagus, testes, liver, parotid, biliary tract, colon, rectum, cervix, uterus, endometrium, kidney, bladder, prostate,thyroid, squamous cell carcinomas, adenocarcinomas, small cell carcinomas, melanomas, gliomas, and neuroblastomas.

30. Use according to claim 26 wherein the anti-cancer agent is selected from the group consisting of mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, and leutinizing hormone releasing factor.

31. Use according to claim 26 wherein the radiosensitizer agent is selected from the group consisting of 2-(3-nitro-1,2,4-triazol-1-yl)-N-(2-methoxyethyl)acetamide, N-(3-nitro-4-quinolinyl)-4-morpholinecarboxamidine, 3-amino-1,2,4-benzotriazine-1,4-dioxide, N-(2-hydroxyethyl)-2-nitroimidazole-1-acetamide, 1-(2-nitroimidazol-1-yl)-3-(1-piperidinyl)-2-propanol, and 1-(2-nitro-1-imidazolyl)-3- (1-aziridino)-2-propanol.

32. Use according to claim 26 wherein the anti-cancer agent is a chemotherapeutic agent.

33. Use according to claim 26, wherein the administration is by injection or infusion.

34. Use according to claim 26, wherein the therapeutic radiopharmaceutical comprises:
a) a metal;
b) a chelator capable of chelating the metal; and
c) a targeting moiety;
wherein the targeting moiety is bound to the chelator through a linking group, and the targeting moiety is a quinolone non-peptide that binds to a receptor that is upregulated during angiogenesis, and the metal is a radioisotope selected from the group: ³³P ¹²⁵I, ¹⁸⁶Re, ¹⁸⁸Re, ¹⁵³Sm, ¹⁶⁶Ho, ¹⁷⁷Lu, ¹⁴³Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir.

35. Use according to claim 34, wherein the targeting moiety is a quinolone non-peptide and the receptor is αᵥβ₃ or αᵥβ₅.

36. Use according to claim 34, wherein the therapeutic radiopharmaceutical comprises:
a) a radioisotope selected from the group: ³³P, ¹²⁵I, ¹⁸⁶Re, ¹⁸⁸Re, ¹⁵³Sm, ¹⁶⁶Ho, ¹⁷⁷Lu ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir; and
b) a compound of the formula (I):
(Q)_{d}-Lₙ-Cₕ or (Q)_{d}-Lₙ-(Cₕ)_{d'} (I)
wherein, Q is a compound of Formula (II): 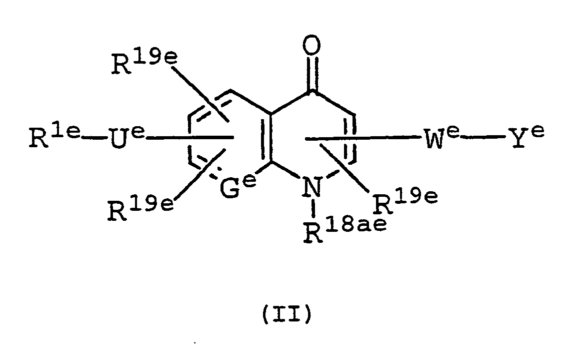 including stereoisomeric forms thereof, or mixtures of stereoisomeric forms thereof, or pharmaceutically acceptable salt or prodrug forms thereof wherein:
R^{1e} is selected from: 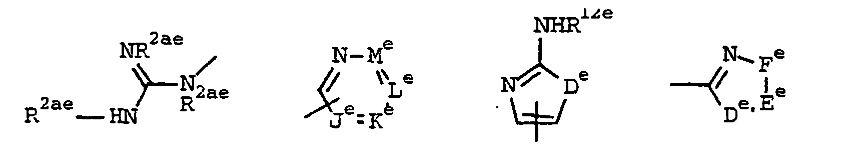 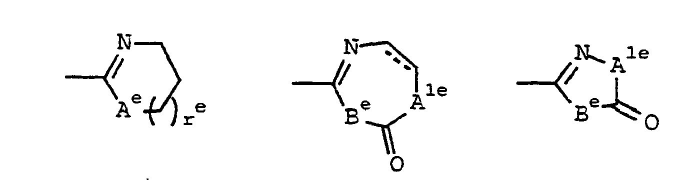 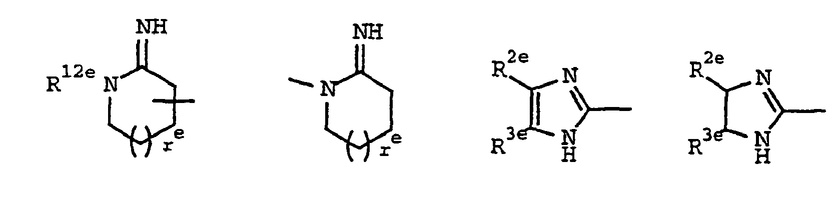 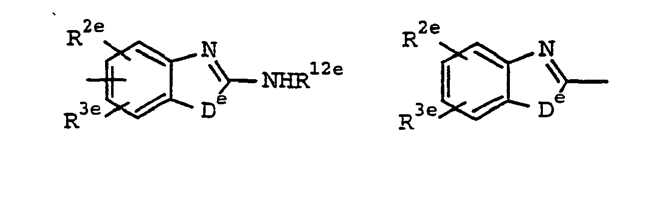 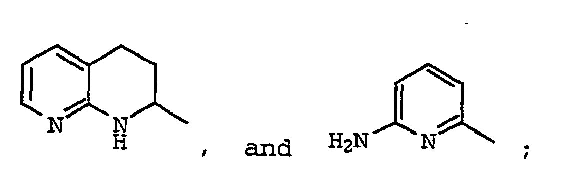
A^{e} is -CH₂- or -N(R^{10e})-;
A^{1e} and B^{e} are independently -CH₂- or -N(R^{10e})-;
D^{e} is -N(R^{10e})- or -S-;
E^{e}-F^{e} is -C(R^{2e})=C(R^{3e})- or -C(R^{2e})₂C(R^{3e})₂-;
J^{e} is -C(R^{2e})- or -N-;
K^{e}, L^{e} and M^{e} are independently -C(R^{2e})- or -C(R^{3e})-;
R^{2e} and R^{3e} are independently selected from:
H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy)carbonyl, arylcarbonyl, and aryl substituted with 0-4 R^{7e},
alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
R^{2ae} is selected from:
H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
R^{7e} is selected from:
H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
U^{e} is selected from:
-(CH₂)ₙ^{e}-, -(CH₂)ₙ^{e}O(CH₂)ₘ^{e}-,-(CH₂)ₙ^{e}N(R¹²)(CH₂)ₘ^{e}-,-NH(CH₂)ₙ^{e}-, -(CH₂)ₙ^{e}C(=O)(CH₂)m^{e}-, -(CH₂)ₙ^{e}S(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})-, and -N(R^{10e})S(O)ₚ^{e}-;
G^{e} is N or CR^{19e};
W^{e} is-C(=O)-N(R^{10e})-(C₁-C₃ alkylene)-, in which the alkylene group is substituted by R^{8e} and by R^{9e}:
R^{8e} and R^{9e} are independently selected from:
H, CO₂R^{18be}, C(=O)R^{18be}, CONR¹⁷R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl, C₃-C₁₀ cycloalkyl(C₁-C₄ alkyl)-, phenyl substituted with 0-3 R^{6e}, naphthyl substituted with 0-3 R^{6e},
a 5-10 membered heterocyclic ring containing 1-3 N, 0, or S heteroatoms, wherein said heterocyclic ring may be saturated, partially saturated, or fully unsaturated, said heterocyclic ring being substituted with 0-2 R^{7e},
C₁-C₁₀ alkoxy substituted with 0-2 R^{7e}, hydroxy, nitro, -N(R^{10e})R^{11e}, -N(R^{16e})R^{17e}, aryl(C₀-C₆ alkyl)carbonyl, aryl(C₃-C₆ alkyl), heteroaryl(C₁-C₆ alkyl), CONR^{18ae}R^{20e}, SO₂R^{18ae}, and SO₂NR^{18ae}R^{20e},
providing that any of the above alkyl, cycloalkyl, aryl or heteroaryl groups may be unsubstituted or substituted independently with 1-2 R^{7e};
R^{6e} is selected from:
H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR¹⁷R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
aryl substituted with 0-3 groups selected from halogen, C₁-C₆ alkoxy, C₁-C₆ alkyl, CF₃, S(O)m^{e}Me, and -NMe₂,
aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
R^{10e} is selected from:
H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
R^{11e} is selected from:
H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e}_{;}
R^{4e} is selected from:
H, C₁-C₆ alkyl, C₃-C₇ cycloalkyl, C₃-C₇ cycloalkyl(C₁-C₄ alkyl)-, (_{C1}-C₁₀ alkyl)carbonyl, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₄ alkoxy, F, C1, Br, CF₃, and NO₂,
alternatively, when R^{10e} and R^{11e} are both substituents on the same nitrogen atom (as in -NR^{10e}R^{11e}) they may be taken together with the nitrogen atom to which they are attached to form a heterocycle selected from: 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl, and 1-piperazinyl;
said heterocycle being substituted with 0-3 groups selected from: C₁-C₆ alkyl, aryl, heteroaryl, aryl(C₁-C₄ alkyl)-, (C₁-C₆ alkyl)carbonyl, (C₃-C₇ cycloalkyl)carbonyl, (C₁-C₆ alkoxy)carbonyl; aryl(C₁-C₄ alkoxy)carbonyl, C₁-C₆ alkylsulfonyle, and arylsulfonyl;
R^{12e} is selected from:
H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy)carbonyl, (C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl)carbonyl, heteroarylcarbonyl, aryl(C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
R^{16e} is selected from:
-C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, and -SO₂NHC(=O)OR^{18be};
R^{17e} is selected from:
H, C₁-C₆ alkyl, C₃-C₇ cycloalkyl, C₃-C₇ cycloalkyl(C₁-C₄ alkyl)-, aryl, aryl(C₁-C₆ alkyl)-, and heteroaryl(C₁-C₆ alkyl);
R^{18ae} is selected from:
C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
R^{18be} is H or R^{18ae};
R^{19e} is selected from:
H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl , and C₁-C₃ alkoxy;
R^{20e} is selected from:
hydroxy, C₁-C₁₀ alkyloxy, C₃-C₁₁ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-,
C₁-C₅ alkoxy(C₁-C₅ alkyl)carbonyloxy(C₁-C₂ alkyl)ox y,
(5-(C₁-C₅ alkyl)-1,3-dioxa-cyclopenten-2-one-yl)methyloxy,
(5-aryl-1,3-dioxa-cyclopenten-2-one-yl)methyloxy, and
(R^{10e})(R^{11e})N-(C₁-C₁₀ alkoxy)-;
R^{21e} is selected from:
C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and C₁-C₁₀ alkyl substituted with 0-2 R^{7e};
R^{22e} is selected from:
-C(=O)-R^{18be}, -C(=O)N(R^{18be})₂, -C(=O)NHSO₂R^{18ae},-C(=O)NHC(=O)R^{18be}, -C(=O)NHC(=O)OR^{18ae}, and-C(=O)NHSO₂NHR^{18be};
Y^{e} is selected from:
-COR^{20e}, -SO₃H, -PO₃H, -CONHNHSO₂CF₃,-CONHSO₂R^{18ae}, -CONHSO₂NHR^{18be}, -NHCOCF₃,-NHCONHSO₂R^{18ae}, -NHSO₂R^{18ae}, -OPO₃H₂, -OSO₃H,-PO₃H₂, -SO₂NHCOR^{18ae}, -SO₂NHCO₂R^{18ae}, 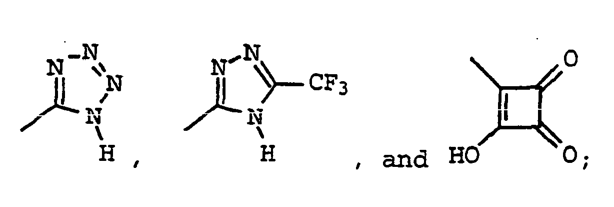
m^{e} is 0-2;
n^{e} is 0-4;
p^{e} is 0-2;
r^{e} is 0-2;
with the following proviso: n^{e} and m^{e} are chosen such that the number of atoms connecting R^{1e} and Y^{e} is in the range of 8-14;
d is selected from 1, 2, 3, 4, 5, 6, 7, 8, 9, and 10;
d' is 1-100;
Lₙ is a linking group having the formula:
((W)ₕ-(CR⁶R⁷)_{g})ₓ-(Z)ₖ-((CR^{6a}R^{7a})_{g'}-(W)_{h'})_{x'};
W is independently selected at each occurrence from the group: O, S, NH, NHC(=O), C(=O)NH, NR⁸C(=O) , C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ, and (aa)_{t'};
aa is independently at each occurrence an amino acid;
Z is selected from the group: aryl substituted with 0-3 R¹⁰, C₃-₁₀ cycloalkyl substituted with 0-3 R¹⁰, and a 5-10 membered heterocyclic ring system containing 1-4 heteroatoms independently selected from N, S, and O and substituted with 0-3 R¹⁰;
R⁶, R^{6a}, R⁷, R^{7a}, and R⁸ are independently selected at each occurrence from the group: H, =O, COOH, SO₃H, PO₃H, C₁-C₅ alkyl substituted with 0-3 R¹⁰, aryl substituted with 0-3 R¹⁰, benzyl substituted with 0-3 R¹⁰, and C₁-C₅ alkoxy substituted with 0-3 R¹⁰, NHC(=O)R¹¹, C(=O)NHR¹¹, NHC(=O)NHR¹¹, NHR¹¹, R¹¹, and a bond to Cₕ;
R¹⁰ is independently selected at each occurrence from the group: a bond to Cₕ, COOR¹¹, C(=O)NHR¹¹, NHC(=O)R¹¹, OH, NHR¹¹, SO₃H, PO₃H, -OPO₃H₂, -OSO₃H, aryl substituted with 0-3 R¹¹, C₁-₅ alkyl substituted with 0-1 R¹², C₁-₅ alkoxy substituted with 0-1 R¹², and a 5-10 membered heterocyclic ring system containing 1-4 heteroatoms independently selected from N, S, and O and substituted with 0-3 R¹¹;
R¹¹ is independently selected at each occurrence from the group: H, -OPO₃H₂,, -OSO₃H C₁₋₅ alkyl substituted with 0-1 R¹², aryl substituted with 0-1 R¹², a 5-10 membered heterocyclic ring system containing 1-4 heteroatoms independently selected from N, S, and 0 and substituted with 0-1 R¹², C₃₋₁₀ cycloalkyl substituted with 0-1 R¹², polyalkylene glycol substituted with 0-1 R¹²,, C₁-₅ alkyl substituted with carbohydrate substituted with 0-1 R¹², cyclodextrin substituted with 0-1 R¹², amino acid substituted with 0-1 R¹², polycarboxyalkyl substituted with 0-1 R¹², polyazaalkyl substituted with 0-1 R¹², peptide substituted with 0-1 R¹², wherein the peptide is comprised of 2-10 amino acids, 3,6-O-disulfo-B-D-galactopyranosyl, bis(phosphonomethyl)glycine, and a bond to Cₕ;
R¹² is a bond to Cₕ;
k is selected from 0, 1, and 2;
h is selected from 0, 1, and 2;
h' is selected from 0, 1, and 2;
g is selected from 0, 1, 2, 3, 4, 5, 6, 7, 8, 9, and 10;
g' is selected from 0, 1, 2, 3, 4, 5, 6, 7, 8, 9, and 10;
s is selected from 0, 1, 2, 3, 4, 5, 6, 7, 8, 9, and 10;
s' is selected from 0, 1, 2, 3, 4, 5, 6, 7, 8, 9, and 10;
s" is selected from 0, 1, 2, 3, 4, 5, 6, 7, 8, 9, and 10;
t is selected from 0, 1, 2, 3, 4, 5, 6, 7, 8, 9, and 10;
t' is selected from 0, 1, 2, 3, 4, 5, 6, 7, 8, 9, and 10;
x is selected from 0, 1, 2, 3, 4, 5, and 6;
x' is selected from 0, 1, 2, 3, 4, 5, and 6;
Cₕ is a metal bonding unit having a formula selected from the group: 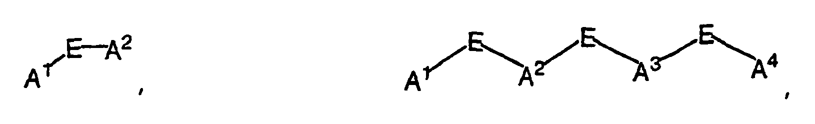 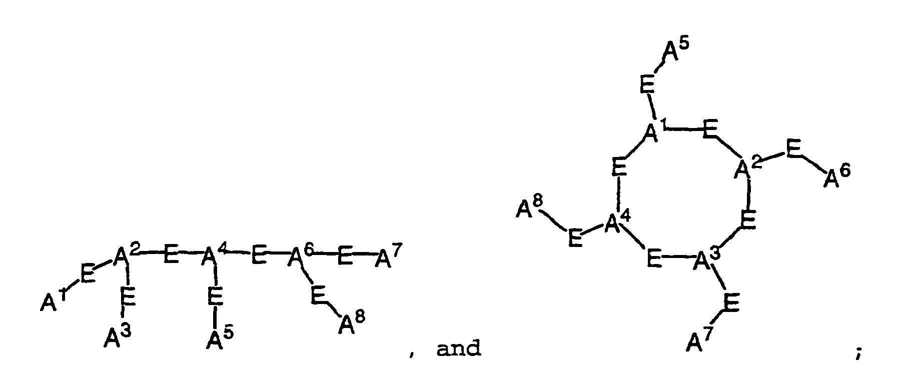
A¹, A², A³, A⁴, A⁵, A⁶, A⁷, and A⁸ are independently selected at each occurrence from the group: NR¹³, NR¹³R¹⁴, S, SH, S(Pg), O, OH, PR¹³, PR¹³R¹⁴, P(O)R¹⁵R¹⁶, and a bond to Lₙ;
E is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and O and substituted with 0-3 R¹⁷;
R¹³ and R¹⁴ are each independently selected from the group: a bond to Lₙ, hydrogen, C₁-C₁₀ alkyl substituted with 0-3 R¹⁷, aryl substituted with 0-3 R¹⁷, C₁₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
alternatively, R¹³ and R¹⁴ combine to form =C(R²⁰)(R²¹);
R¹⁵ and R¹⁶ are each independently selected from the group: a bond to Lₙ, -OH, C₁-C₁₀ alkyl substituted with 0-3 R¹⁷, C₁-C₁₀ alkyl substituted with 0-3 R¹⁷, aryl substituted with 0-3 R¹⁷, C₃₋₁₀ cycloalkyl substituted with 0-3 R¹⁷, heterocyclo-C₁₋₁₀ alkyl substituted with 0-3 R¹⁷, wherein the heterocyclo group is a 5-10 membered heterocyclic ring system containing 1-4 heteroatoms independently selected from N, S, and O, C₆₋₁₀ aryl-C₁₋₁₀ alkyl substituted with 0-3 R¹⁷, C₁₋₁₀ alkyl-C₆₋₁₀ aryl- substituted with 0-3 R¹⁷, and a 5-10 membered heterocyclic ring system containing 1-4 heteroatoms independently selected from N, S, and 0 and substituted with 0-3 R¹⁷;
R¹⁷ is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H,
2-(1-morpholino)ethoxy, C₁-C₅ alkyl,C₂-C₄ alkenyl, C₃-C₆ cycloalkyl, C₃-C₆ cycloalkylmethyl, C₂-C₆ alkoxyalkyl, aryl substituted with 0-2 R¹⁸, and a 5-10 membered heterocyclic ring system containing 1-4 heteroatoms independently selected from N, S, and O;
R¹⁸, R^{18a}, and R¹⁹ are independently selected at each occurrence from the group: a bond to Lₙ, H, C₁-C₆ alkyl, phenyl, benzyl, C₁-C₆ alkoxy, halide, nitro, cyano, and trifluoromethyl;
Pg is a thiol protecting group;
R²⁰ and R²¹ are independently selected from the group: H, C₁-C₁₀ alkyl, -CN, -CO₂R²⁵, -C(=O)R²⁵, -C(=O)N(R²⁵)₂, C₂-C₁₀ 1-alkene substituted with 0-3 R²³, C₂-C₁₀ 1-alkyne substituted with 0-3 R²³, aryl substituted with 0-3 R²³, unsaturated 5-10 membered heterocyclic ring system containing 1-4 heteroatoms independently selected from N, S, and 0 and substituted with 0-3 R²³, and unsaturated C₃-₁₀ carbocycle substituted with 0-3 R²³;
alternatively, R²⁰ and R²¹, taken together with the divalent carbon radical to which they are attached form: 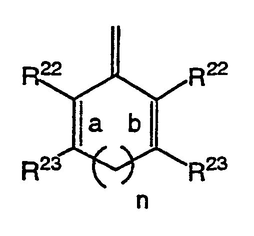
R²² and R²³ are independently selected from the group:
H, R²⁴, C₁-C₁₀ alkyl substituted with 0-3 R²⁴, C₂-C₁₀ alkenyl substituted with 0-3 R²⁴, C₂-C₁₀ alkynyl substituted with 0-3 R²⁴, aryl substituted with 0-3 R²⁴, a 5-10 membered heterocyclic ring system containing 1-4 heteroatoms independently selected from N, S, and O and substituted with 0-3 R²⁴, and C₃-₁₀ carbocycle substituted with 0-3 R²⁴;
alternatively, R²², R²³ taken together form a fused aromatic or a 5-10 membered heterocyclic ring system containing 1-4 heteroatoms independently selected from N, S, and O;
a and b indicate the positions of optional double bonds and n is 0 or 1;
R²⁴ is independently selected at each occurrence from the group: =O, F, Cl, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, and 2- (1-morpholino) ethoxy; and,
R²⁵, R^{25a}, and R²⁶ are each independently selected at each occurrence from the group: hydrogen and C₁-C₆ alkyl; or a pharmaceutically acceptable salt thereof.

37. Use according to claim 36, wherein:
Q is a compound of Formula (IV): 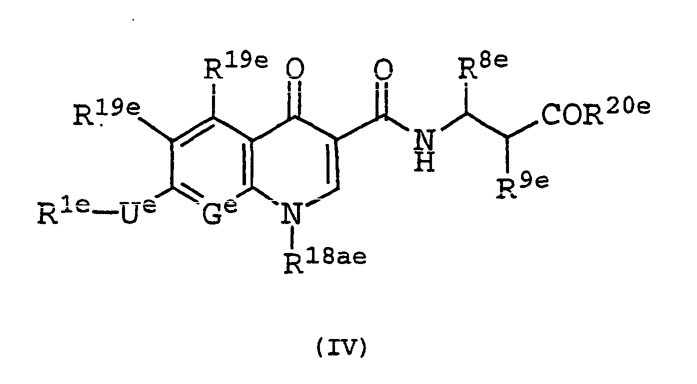 including stereoisomeric forms thereof, or mixtures of stereoisomeric forms thereof, or pharmaceutically acceptable salt or prodrug forms thereof wherein:
R^{1e} is selected from: 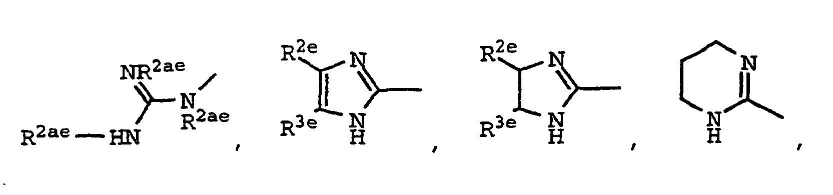 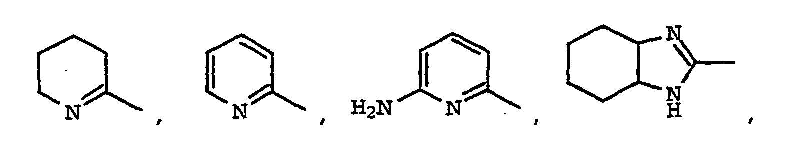 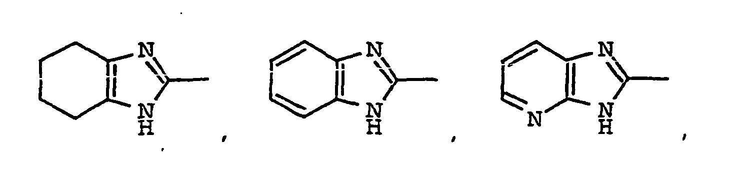 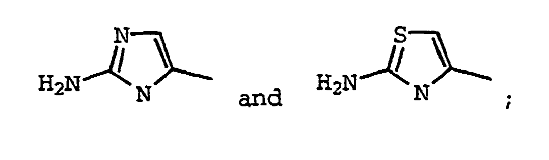
R^{2e} and R^{3e} are independently selected from: H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-, (C₁-C₆ alkyl)carbonyl, (C₁-C₆ alkoxy) carbonyl, arylcarbonyl, and aryl substituted with 0-4 R^{7e},
alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
R^{2ae} is selected from:
H, C₁-C₁₀ alkyl, C₂-C₆ alkenyl, C₃-C₁₁ cycloalkyl, C₃-C₇ cycloalkyl(C₁-C₄ alkyl), aryl, aryl(C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
R^{7e} is selected from:
H, hydroxy, C₁-C₄ alkyl, C₁-C₄ alkoxy, aryl, aryl(C₁-C₄ alkyl)-, (C₁-C₄ alkyl) carbonyl, CO₂R^{18ae},SO₂R^{11e}, SO₂NR^{10e}R^{11e}, OR^{10e}, and N(R^{11e})R^{12e};
U^{e} is selected from:
-(CH₂)ₙ^{e}-, -(CH₂)ₙ^{e}O(CH₂)ₘ^{e}-, -NH(CH₂)ₙ^{e}-, -N (R^{10e})C (=O) -, -NHC(=O)(CH₂)ₙ^{e}-, and-C(=O)N(R^{10e})-;
G^{e} is N or CR^{19e};
R^{8e} is selected from:
H, CO₂R^{18be}, C(=O)R^{18be}, CONR^{17e}R^{18be}, C₁-C₁₀ alkyl substituted with 0-1 R^{6e}, C₂-C₁₀ alkenyl substituted with 0-1 R^{6e}, C₂-C₁₀ alkynyl substituted with 0-1 R^{6e}, C₃-C₈ cycloalkyl substituted with 0-1 R^{6e}, C₅-C₆ cycloalkenyl substituted with 0-1 R^{6e}, (C₁-C₁₀ alkyl)carbonyl, C₃-C₁₀ cycloalkyl(C₁-C₄ alkyl)-, phenyl substituted with 0-3 R^{6e}, naphthyl substituted with 0-3 R^{6e},
a 5-10 membered heterocyclic ring containing 1-3 N, 0, or S heteroatoms, wherein said heterocyclic ring may be saturated, partially saturated, or fully unsaturated, said heterocyclic ring being substituted with 0-2 R^{7e};
R^{9e} is selected from:
C₁-C₁₀ alkyl substituted with 0-1 R^{6e},
C₁-C₁₀ alkoxy substituted with 0-2 R^{7e},
H, nitro, N(R^{11e})R^{12e}, OC(=O)R^{10e}, OR^{10e}, OC(=O)NR^{10e}R^{11e}, NR^{10e}C(=O)R^{10e}, NR^{10e}C (=O) OR^{21e}, NR^{10e}C(=O)NR^{10e}R^{11e}, NR^{10e}SO₂NR¹⁰R^{11e}, NR^{10e}SO₂R^{21e}, hydroxy, OR^{22e}, -N(R^{10e})R^{11e}, -N(R^{16e})R^{17e}, aryl (C₀-C₆ alkyl) carbonyl, aryl(C₁-C₆ alkyl), heteroaryl(C₁-C₆ alkyl), CONR^{18ae}R^{20e}, SO₂R^{18ae}, and SO₂NR^{18ae}R^{20e},
providing that any of the above alkyl, cycloalkyl, aryl or heteroaryl groups may be unsubstituted or substituted independently with 1-2 R^{7e};
R^{6e} is selected from:
H, C₁-C₁₀ alkyl, hydroxy, C₁-C₁₀ alkoxy, nitro, C₁-C₁₀ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚ^{e}R^{11e}, SO₂NR^{10e}R^{11e},
aryl substituted with 0-3 groups selected from halogen, C₁-C₆ alkoxy, C₁-C₆ alkyl, CF₃, S(O)ₘ^{e}Me, and -NMe₂,
aryl(C₁-C₄ alkyl)-, said aryl being substituted with 0-3 groups selected from halogen, C₁-C₆ alkoxy, C₁-C₆ alkyl, CF₃, S(O)ₚ^{e}Me, and -NMe₂, and
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
R^{10e} is selected from:
H, -OH, CF₃, C₃-C₆ alkenyl, C₃-C₁₁ cycloalkyl, aryl, (C₃-C₁₁ cycloalkyl)methyl, aryl(C₁-C₄ alkyl), and C₁-C₁₀ alkyl substituted with 0-2 R^{6e};
R^{11e} is selected from:
H, hydroxy, C₁-C₈ alkyl, C₃-C₆ alkenyl, C₃-C₁₁ cycloalkyl, (C₃-C₁₁ cycloalkyl)methyl, C₁-C₆ alkoxy, benzyloxy, aryl, heteroaryl, heteroaryl(C₁-C₄ alkyl)-, aryl(C₁-C₄ alkyl), adamantylmethyl, and C₁-C₁₀ alkyl substituted with 0-2 R^{4e};
R^{4e} is selected from:
H, C₁-C₆ alkyl, C₃-C₇ cycloalkyl, C₃-C₇ cycloalkyl(C₁-C₄ alkyl)-, aryl, heteroaryl, aryl(C₁-C₆ alkyl)-, and heteroaryl(C₁-C₆ alkyl)-, wherein said aryl or heteroaryl groups are substituted with 0-2 substituents independently selected from the group consisting of C₁-C₄ alkyl, C₁-C₁ alkoxy, F, Cl, Br, CF₃, and NO₂,
R^{12e} is selected from:
H, C₁-C₆ alkyl, triphenylmethyl, methoxymethyl, methoxyphenyldiphenylmethyl, trimethylsilylethoxymethyl, (C₁-C₆ alkyl)carbonyl, (C₁-C₆ alkoxy)carbonyl,(C₁-C₆ alkyl)aminocarbonyl, C₃-C₆ alkenyl, C₃-C₇ cycloalkyl, C₃-C₇ cycloalkyl(C₁-C₄ alkyl)-, aryl, heteroaryl(C₁-C₆ alkyl) carbonyl, heteroarylcarbonyl, aryl (C₁-C₆ alkyl)-, (C₁-C₆ alkyl)carbonyl, arylcarbonyl, C₁-C₆ alkylsulfonyl, arylsulfonyl, aryl(C₁-C₆ alkyl)sulfonyl, heteroarylsulfonyl, heteroaryl(C₁-C₆ alkyl)sulfonyl, aryloxycarbonyl, and aryl(C₁-C₆ alkoxy)carbonyl, wherein said aryl groups are substituted with 0-2 substituents selected from the group consisting of C₁-C₄ alkyl, C₁-C₄ alkoxy, halo, CF₃, and nitro;
R^{16e} is selected from:
-C(=O)OR^{18ae}, -C(=O)R^{18be}, -C(=O)N(R^{18be})₂,-SO₂R^{18ae}, and -SO₂N(R^{18be})_{2;}
R^{17e} is selected from:
H, C₁-C₆ alkyl, C₃-C₇ cycloalkyl, C₃-C₇ cycloalkyl(C₁-C₄ alkyl)-, aryl, aryl(C₁-C₆ alkyl)-, and heteroaryl(C₁-C₆ alkyl);
R^{18ae} is selected from:
C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Ln, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
R^{18be} is H or R^{18ae};
R^{19e} is selected from:
H, halogen, CF₃, CO₂H, CN, NO₂, -NR^{11e}R^{12e}, OCF₃, C₁-C₈ alkyl, C₂-C₆ alkenyl, C₂-C₆ alkynyl, C₃-C₁₁ cycloalkyl, C₃-C₇ cycloalkyl(C₁-C₄ alkyl)-, aryl(C₁-C₆ alkyl)-, C₁-C₆ alkoxy, C₁-C₄ alkoxycarbonyl, aryl, aryl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
R^{20e} is selected from:
hydroxy, C₁-C₁₀ alkyloxy, C₃-C₁₁ cycloalkyloxy, aryloxy, aryl (C₁-C₄ alkyl) oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃-C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, aryloxycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
(5-(C₁-C₅ alkyl)-1,3-dioxa-cyclopenten-2-one-yl)methyloxy,
(5-aryl-1,3-dioxa-cyclopenten-2-one-yl)methyloxy, and
(R^{10e}) (R^{11e}) N- (C₁-C₁₀ alkoxy)-;
R^{21e} is selected from:
C₁-C₈ alkyl, C₂-C₆ alkenyl, C₃-C₁₁ cycloalkyl, (C₃-C₁₁ cycloalkyl)methyl, aryl, aryl(C₁-C₄ alkyl)-, and C₁-C₁₀ alkyl substituted with 0-2 R^{7e};
R^{22e} is selected from:
-C(=O)-R^{18be}, -C(=O)N(R^{18be})₂, -C(=O)NHSO₂R^{18ae}, -C(=O)NHC(=O)R^{18be}, -C(=O) NHC(=O)OR^{18ae}, and -C(=O)NHSO₂NHR^{18be};
m^{e} is 0-2;
n^{e} is 0-4; and
p^{e} is 0-2;
with the following proviso: n^{e} and m^{e} are chosen such that the number of atoms connecting R¹ and -COR^{20e} in Formula (IV) is in the range of 8-14;
d is selected from 1, 2, 3, 4, and 5;
d' is 1-50;
W is independently selected at each occurrence from the group: O, NH, NHC(=O), C(=O)NH, NR⁸C(=O), C(=O)N R⁸, C(=O), C(=O)O, OC(=O)*,* NHC(=S)NH, NHC(=O)NH, SO₂, (OCH₂CH₂)ₛ, (CH₂CH₂O)_{s'}, (OCH₂CH₂CH₂)_{s"}, (CH₂CH₂CH₂O)ₜ, and (aa)_{t'};
aa is independently at each occurrence an amino acid;
Z is selected from the group: aryl substituted with 0-1 R¹⁰, C₃₋₁₀ cycloalkyl substituted with 0-1 R¹⁰, and a 5-10 membered heterocyclic ring system containing 1-4 heteroatoms independently selected from N, S, and 0 and substituted with 0-1 R¹⁰;
R⁶, R^{6a}, R⁷, R^{7a}, and R⁸ are independently selected at each occurrence from the group: H, =0, COOH, SO₃H, C₁-C₅ alkyl substituted with 0-1 R¹⁰, aryl substituted with 0-1 R¹⁰, benzyl substituted with 0-1 R¹⁰, and C₁-C₅ alkoxy substituted with 0-1 R¹⁰, NHC(=O)R¹¹, C(=O)NHR¹¹, NHC(=O)NHR¹¹, NHR¹¹, R¹¹, and a bond to Cₕ;
k is 0 or 1;
s is selected from 0, 1, 2, 3, 4, and 5;
s' is selected from 0, 1, 2, 3, 4, and 5;
s" is selected from 0, 1, 2, 3, 4, and 5;
t is selected from 0, 1, 2, 3, 4, and 5;
A¹, A², A³, A⁴, A⁵, A⁶, A⁷, and A⁸ are independently selected at each occurrence from the group: NR¹³, NR¹³R¹⁴, S, SH, S(Pg), OH, and a bond to Lₙ;
E is a bond, CH, or a spacer group independently selected at each occurrence from the group: C₁-C₁₀ alkyl substituted with 0-3 R¹⁷, aryl substituted with 0-3 R¹⁷, C₃₋₁₀ cycloalkyl substituted with 0-3 R¹⁷, and a 5-10 membered heterocyclic ring system containing 1-4 heteroatoms independently selected from N, S, and 0 and substituted with 0-3 R¹⁷;
R¹³, and R¹⁴ are each independently selected from the group: a bond to Lₙ, hydrogen, C₁-C₁₀ alkyl substituted with 0-3 R¹⁷, aryl substituted with 0-3 R¹⁷, a 5-10 membered heterocyclic ring system containing 1-4 heteroatoms independently selected from N, S, and O and substituted with 0-3 R¹⁷, and an electron, provided that when one of R¹³ or R¹⁴ is an electron, then the other is also an electron;
alternatively, R¹³ and R¹⁴ combine to form =C(R²⁰)(R²¹);
R¹⁷ is independently selected at each occurrence from the group: a bond to Lₙ, =O, F, Cl, Br, I, -CF₃, -CN, -CO₂R¹⁸, -C(=O)R¹⁸, -C(=O)N(R¹⁸)₂, -CH₂OR¹⁸, -OC(=O)R¹⁸, -OC (=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)₂, -N(R¹⁸)₂, -NHC(=S)NHR¹⁸, =NOR¹⁸, -C(=O)NHNR¹⁸R^{18a}, -OCH₂CO₂H, and 2-(1-morpholino) ethoxy;
R¹⁸, R^{18a}, and R¹⁹ are independently selected at each occurrence from the group: a bond to Lₙ, H, and C₁-C₆ alkyl;
R²⁰ and R²¹ are independently selected from the group: H, C₁-C₅ alkyl, -CO₂R²⁵, C₂-C₅ 1-alkene substituted with 0-3 R²³, C₂-C₅ 1-alkyne substituted with 0-3 R²³, aryl substituted with 0-3 R²³, and unsaturated 5-10 membered heterocyclic ring system containing 1-4 heteroatoms independently selected from N, S, and O and substituted with 0-3 R²³;
alternatively, R²⁰ and R²¹, taken together with the divalent carbon radical to which they are attached form: 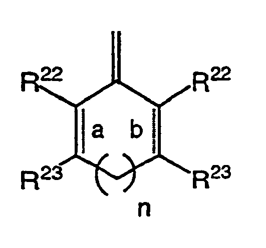
R²² and R²³ are independently selected from the group: H, and R²⁴;
alternatively, R²², R²³ taken together form a fused aromatic or a 5-10 membered heterocyclic ring system containing 1-4 heteroatoms independently selected from N, S, and O;
R²⁴ is independently selected at each occurrence from the group: -CO₂R²⁵, -C(=O)N(R²⁵)₂, -CH₂OR²⁵, -OC(=O)R²⁵, -OR²⁵, -SO₃H, -N(R²⁵)₂, and -OCH₂CO₂H; and,
R²⁵ is independently selected at each occurrence from the group: H and C₁-C₃ alkyl.

38. Use according to claim 36, wherein:
R^{1e} is selected from: 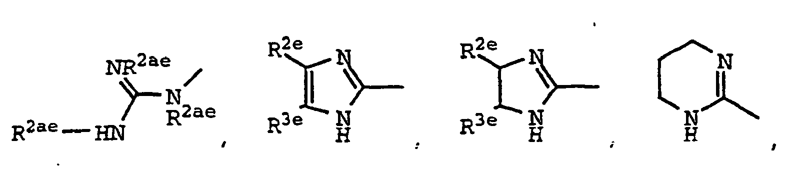 and 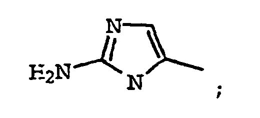
R^{2e} and R^{3e} are independently selected from:
H, C₁-C₄ alkoxy, NR^{11e}R^{12e}, halogen, NO₂, CN, CF₃, C₁-C₆ alkyl, C₃-C₆ alkenyl, C₃-C₇ cycloalkyl, C₃-C₇ cycloalkyl(C₁-C₄ alkyl), aryl(C₁-C₆ alkyl)-(C₁-C₆ alkyl)carbonyl, (C₁-C₆ alkoxy)carbonyl, arylcarbonyl, and aryl substituted with 0-4 R^{7e},
alternatively, when R^{2e} and R^{3e} are substituents on adjacent atoms, they can be taken together with the carbon atoms to which they are attached to form a 5-7 membered carbocyclic or 5-7 membered heterocyclic aromatic or nonaromatic ring system, said carbocyclic or heterocyclic ring being substituted with 0-2 groups selected from C₁-C₄ alkyl, C₁-C₄ alkoxy, halo, cyano, amino, CF₃ and NO₂;
R^{2ae} is selected from:
H, C₁-C₁₀ alkyl, C₂-C₆ alkenyl, C₃-C₁₁ cycloalkyl, C₃-C₇ cycloalkyl(C₁-C₄ alkyl), aryl, aryl (C₁-C₄ alkyl)-, (C₂-C₇ alkyl)carbonyl, arylcarbonyl, (C₂-C₁₀ alkoxy)carbonyl, C₃-C₇ cycloalkoxycarbonyl, C₇-C₁₁ bicycloalkoxycarbonyl, aryloxycarbonyl, aryl(C₁-C₁₀ alkoxy)carbonyl, C₁-C₆ alkylcarbonyloxy(C₁-C₄ alkoxy)carbonyl, arylcarbonyloxy(C₁-C₄ alkoxy)carbonyl, and C₃-C₇ cycloalkylcarbonyloxy(C₁-C₄ alkoxy)carbonyl;
R^{7e} is selected from:
H, hydroxy, C₁-C₄ alkyl, C₁-C₄ alkoxy, aryl, aryl(C₁-C₄ alkyl)-, (C₁-C₄ alkyl)carbonyl, CO₂R^{18ae}, SO₂R^{11e}, SO₂NR^{10e}R^{11e}, OR^{10e}, and N(R^{11e})R^{12e};
U^{e} is selected from:
-(CH₂)ₙ^{e}-, -NH(CH₂)ₙ^{e}-, -N(R^{10e})C(=O)-, and
-NHC(=O) (CH₂)ₙ^{e}
G^{e} is N or CR^{19e};
R^{8e} is H;
R^{9e} is selected from:
H, nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, hydroxy, OR^{22e}, -N(R^{10e})R^{11e}, -N(R^{16e})R^{17e}, aryl(C₀-C₄ alkyl)carbonyl, aryl(C₁-C₄ alkyl), heteroaryl(C₁-C₄ alkyl), CONR^{18ae}R^{20e}, SO₂R^{18ae}, and SO₂NR^{18ae}R^{20e},
providing that any of the above alkyl, cycloalkyl, aryl or heteroaryl groups may be unsubstituted or substituted independently with 1-2 R^{7e};
R^{10e} is selected from:
H, -OH, CF₃, C₃-C₆ alkenyl, C₃-C₆ cycloalkyl, aryl, (C₃-C₆ cycloalkyl)methyl, aryl(C₁-C₄ alkyl), and C₁-C₄ alkyl substituted with 0-2 R^{6e};
R^{6e} is selected from:
H, C₁-C₄ alkyl, hydroxy, C₁-C₄ alkoxy, nitro, C₁-C₄ alkylcarbonyl, -N(R^{11e})R^{12e}, cyano, halo, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e}
aryl substituted with 0-3 groups selected from halogen, C₁-C₄ alkoxy, C₁-C₄ alkyl, CF₃, S(O)ₘ^{e}Me, and -NMe₂,
aryl(C₁-C₄ alkyl)-, said aryl being substituted with 0-3 groups selected from halogen, C₁-C₄ alkoxy, C₁-C₄ alkyl, CF₃, S(O)ₚ^{e}Me, and -NMe₂, and
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R^{7e};
R^{11e} is selected from:
H, hydroxy, C₁-C₄ alkyl, C₃-C₆ alkenyl, C₃-C₆ cycloalkyl, (C₃-C₆ cycloalkyl)methyl, C₁-C₄ alkoxy, benzyloxy, aryl, heteroaryl, heteroaryl(C₁-C₄ alkyl)-, aryl(C₁-C₄ alkyl), adamantylmethyl, and
C₁-C₄ alkyl substituted with 0-2 R^{4e};
R^{4e} is selected from:
H, C₁-C₄ alkyl, C₃-C₇ cycloalkyl, C₃-C₇ cycloalkyl(C₁-C₄ alkyl)-, aryl, heteroaryl, aryl(C₁-C₄ alkyl)-, and heteroaryl(C₁-C₄ alkyl)-, wherein said aryl or heteroaryl groups are substituted with 0-2 substituents independently selected from the group consisting of C₁-C₄ alkyl, C₁-C₄ alkoxy, F, Cl, Br, CF₃, and NO₂,
R^{12e} is selected from:
H, C₁-C₄ alkyl, (C₁-C₄ alkyl)carbonyl, (C₁-C₄ alkoxy)carbonyl, phenyl(C₁-C₄ alkyl)-, phenylsulfonyl, phenyloxycarbonyl, and phenyl(C₁-C₄ alkoxy)carbonyl, wherein said phenyl groups are substituted with 0-2 substituents selected from the group consisting of C₁-C₄. alkyl, C₁-C₄ alkoxy, halo, CF₃, and nitro;
R^{16e} is selected from:
-C(=O)OR^{18ae} -C(=O)R^{18be}, -C(=O)N(R^{18be})₂, SO₂R^{18ae}, and -SO₂N(R^{18be})₂,
R^{17e} is selected from:
H, C₁-C₄ alkyl, C₃-C₆ cycloalkyl, C₃-C₆ cycloalkyl(C₁-C₄ alkyl)-, aryl, axyl(C₁-C₆ alkyl)-, and heteroaryl(C₁-C₆ alkyl);
R^{18ae} is selected from:
C₁-C₈ alkyl optionally substituted with a bond to Lₙ, C₃-C₁₁ cycloalkyl optionally substituted with a bond to Lₙ, aryl(C₁-C₆ alkyl)-optionally substituted with a bond to Lₙ, heteroaryl(C₁-C₆ alkyl)- optionally substituted with a bond to Lₙ, (C₁-C₆ alkyl)heteroaryl optionally substituted with a bond to Lₙ, biaryl(C₁-C₆ alkyl) optionally substituted with a bond to Lₙ, heteroaryl optionally substituted with a bond to Lₙ, phenyl substituted with 3-4 R^{19e} and optionally substituted with a bond to Lₙ, naphthyl substituted with 0-4 R^{19e} and optionally substituted with a bond to Lₙ, and a bond to Lₙ, wherein said aryl or heteroaryl groups are optionally substituted with 0-4 R^{19e};
R^{18be} is H or R^{18ae};
R^{19e} is selected from:
H, halogen, CF₃, CO₂H, CN, NO₂, -NR^{11e}R^{12e}, OCF₃, C₁-C₆ alkyl, C₂-C₆ alkenyl, C₂-C₆ alkynyl, C3-C6 cycloalkyl, C₃-C₆ cycloalkyl(C₁-C₄ alkyl)-, aryl(C₁-C₄ alkyl)-, C₁-C₆ alkoxy, C₁-C₄ alkoxycarbonyl, aryl, axyl-O-, aryl-SO₂-, heteroaryl, and heteroaryl-SO₂-, wherein said aryl and heteroaryl groups are substituted with 0-4 groups selected from hydrogen, halogen, CF₃, C₁-C₃ alkyl, and C₁-C₃ alkoxy;
R^{20e} is selected from:
hydroxy, C₁-C₆ alkyloxy, C₃-C₆ cycloalkyloxy, aryloxy, aryl(C₁-C₄ alkyl)oxy, C₂-C₁₀ alkylcarbonyloxy(C₁-C₂ alkyl)oxy-, C₂-C₁₀ alkoxycarbonyloxy(C₁-C₂ alkyl)oxy-, C₂-C₁₀ alkoxycarbonyl(C₁-C₂ alkyl)oxy-, C₃₋C₁₀ cycloalkylcarbonyloxy(C₁-C₂ alkyl)oxy-, C₃-C₁₀ cycloalkoxycarbonyloxy(C₁-C₂ alkyl)oxy-, C₃-C₁₀ cycloalkoxycarbonyl(C₁-C₂ alkyl)oxy-, arylohycarbonyl(C₁-C₂ alkyl)oxy-, aryloxycarbonyloxy(C₁-C₂ alkyl)oxy-, arylcarbonyloxy(C₁-C₂ alkyl)oxy-, C₁-C₅ alkoxy(C₁-C₅ alkyl)carbonyloxy(C₁-C₂ alkyl)ox y,
(5-(C₁-C₅ alkyl)-1,3-dioxa-cyclopenten-2-one-yl)methyloxy,
(5-aryl-1,3-dioxa-cyclopenten-2-one-yl)methyloxy, and
(R^{10e}) (R^{11e})N-(C₁-C₁₀ alkoxy)-:
R^{21e} is selected from:
C₁-C₄ alkyl, C₂-C₆ alkenyl, C₃-C₆ cycloalkyl, (C₃-C₆ cycloalkyl)methyl, aryl, aryl(C₁-C₄ alkyl)-, and C₁-C₁₀ alkyl substituted with 0-2 R^{7e};
R^{22e} is selected from:
-C(=O)-R^{18be}, -C(=O)N(R^{18be})₂, -C(=O)NHSO₂R^{18ae}, -C(=O)NHC(=O)R^{18be}, -C(-O)NHC(=O)OR^{18ae}, and -C(=O)NHSO₂NHR^{18e};
m^{e} is 0-2;
n^{e} is 0-4;
p^{e} is 0-2;
Cₕ is 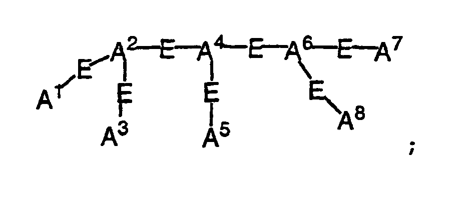
A¹ is selected from the group: OH, and a bond to Lₙ;
A², A⁴, and A⁶ are each N;
A³, A⁵, and A⁸ are each OH;
A⁷ is a bond to Lₙ or NH-bond to Lₙ;
E is a C₂ alkyl substituted with 0-1 R¹⁷;
R¹⁷ is =O;
alternatively, Cₕ is 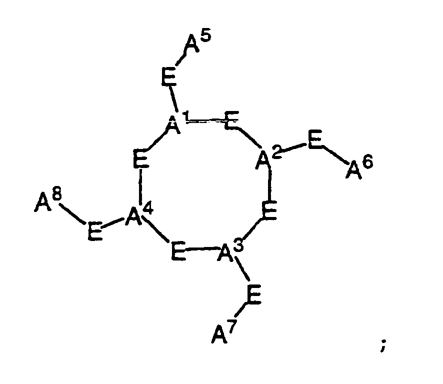
A¹ is selected from the group: OH, and a bond to Lₙ;
A², A³ and A⁴ are each N;
A⁵, A⁶ and A⁸ are each OH;
A⁷ is a bond to Lₙ;
E is a C₂ alkyl substituted with 0-1 R¹⁷;
R¹⁷ is =O;
alternatively, Cₕ is 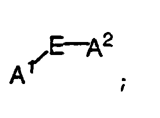
A¹ is NH₂ or N=C(R²⁰) (R²¹);
E is a bond;
A² is NHR¹³;
R¹³ is a heterocycle substituted with R¹⁷, the heterocycle being selected from pyridine and pyrimidine;
R¹⁷ is selected from a bond to Lₙ, C(=O)NHR¹⁸ and C(=O)R¹⁸;
R¹⁸ is a bond to Lₙ;
R²⁴ is selected from the group: -CO₂R²⁵, -OR²⁵, -SO₃H, and -N(R²⁵)₂; and,
R²⁵ is independently selected at each occurrence from the group: hydrogen and methyl.

39. Use according to claim 36, wherein:
Q is selected from the group:
3-[7-[(imidazolin-2-ylamino)methyl]-1-methyl-6,8-difluoroquinoline-4-one-3-ylcarbonylamino]-2-(3,5-dimethylisoxazol-4-ylsulfonylamino)propionic acid,
3-[7-[(imidazolin-2-ylamino)methyl]-1-methyl-6,8-difluoroquinoline-4-one-3-ylcarbonylamino]-2-(benzyloxycarbonylamino)propionic acid,
3-[7-[(imidazolin-2-ylamino)methyl]-1-methyl-6,8-difluoroquinoline-4-one-3-ylcarbonylamino]-2-(n-butyloxycarbonylamino)propionic acid,
3-[7-[(imidazolin-2-ylamino)methyl]-1-methyl-6,8-difluoroquinoline-4-one-3-ylcarbonylamino]-2-(n-butylsulfonylamino)propionic acid,
3-[7-[(tetrahydropyrimid-2-ylamino)methyl]-1-methyl-6,8-difluoroquinoline-4-one-3-ylcarbonylamino]-2-(benzyloxycarbonylamino)propionic acid,
3-[7-[(tetrahydropyrimid-2-ylamino)methyl]-1-methyl-6,8-difluoroquinoline-4-one-3-ylcarbonylamino]-2-(n-butyloxycarbonylamino)propionic acid,
3-[7-[(tetrahydropyrimid-2-ylamino)methyl]-1-methyl-6,8-difluoroquinoline-4-one-3-ylcarbonylamino]-2-(phenylsulfonylamino)propionic acid,
3-[7-[(tetrahydropyrimid-2-ylamino)methyl]-1-methyl-6,8-difluoroquinoline-4-one-3-ylcarbonylamino]-2-(n-butylsulfonyl)aminopropionic acid,
3-[7-[(2-aminothiazol-4-yl)methyl]-1-methyl-6,8-difluoroquinoline-4-one-3-ylcarbonylamino]-2-(benzyloxycarbonylamino)propionic acid,
3-[7-[(imidazolin-2-ylamino)methyl]-1-methyl-6,8-difluoroquinoline-4-one-3-ylcarbonylamino]-2-((2,4,6-trimethylphenyl)sulfonylamino)propionic acid,
3-[7-[(tetrahydropyrimid-2-ylamino)methyl]-1-methyl-6,8-difluoroquinoline-4-one-ylcarbonylamino]-2-((2,4,6-trimethylphenyl)sulfonylamino)propionic acid,
3-[7-[(imidazol-2-ylamino)methyl]-1-methyl-6,8-difluoroquinoline-4-one-3-ylcarbonylamino]-2-(3,5-dimethylisoxazol-4-ylsulfonylamino)propionic acid,
3-[7-[(imidazol-2-ylamino)methyl]-1-methyl-6,8-difluoroquinoline-4-one-3-ylcarbonylamino]-2-(benzyloxycarbonylamino)propionic acid,
3-[7-[(imidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(imidazol-2-ylamino)methyl]-1-methyl-6,8-difluoroquinoline-4-one-3-ylcarbonylamino]-2-((4-biphenyl)sulfonylamino)propionic acid,
3-[7-[(imidazol-2-ylamino)methyl]-1-methyl-6,8-difluoroquinoline-4-one-3-ylcarbonylamino]-2-(1-naphthylsulfonylamino)propionic acid,
3-[7-[(benzimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(4-methylimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(4,5-dimethylimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(4,5,6,7-tetrahydrobenzimidazol-2-ylamino)methyl]-1-methyl-6,8-difluoroquinoline-4-one-3-ylcarbonylamino]-2-((2,4,6-trimethylphenyl)sulfonylamino)propionic acid,
3-[7-[(pyridin-2-ylamino)methyl]-1-methyl-6,8-difluoroquinoline-4-one-3-ylcarbonylamino]-2-((2,4,6-trimethylphenyl)sulfonylamino)propionic acid,
3-[7-(2-aminopyridin-6-yl)-1-methyl-6,8-difluoroquinoline-4-one-3-ylcarbonylamino]-2-((2,4,6-trimethylphenyl)sulfonylamino)propionic acid,
3-[7-[(7-azabenzimidazol-2-yl)methyl]-1-methyl-6,8-difluoroquinoline-4-one-3-ylcarbonylamino]-2-((2,4,6-trimethylphenyl)sulfonylamino)propionic acid,
3-[7-[(benzimidazol-2-ylamino)methyl]-1-(2-phenylethyl)-6,8-difluoroquinoline-4-one-3-ylcarbonylamino]pro-pionic acid,
3-[7-[(pyridin-2-ylamino)methyl]-1-(2-phenylethyl)-6,8-difluoroquinoline-4-one-3-ylcarbonylamino]propionic acid,
3-[7-[(imidazolin-2-ylamino)methyl]-1-(2-phenylethyl)-6,8-difluoroquinoline-4-one-3-ylcarbonylamino]propionic acid,
3-[7-[(imidazol-2-ylamino)methyl]-1-(2-phenylethyl)-6,8-difluoroquinoline-4-one-3-ylcarbonylamino]propionic acid,
3-[7-[(imidazolin-2-ylamino)methyl]-1-(2-phenylethyl)-6,8-difluoroquinoline-4-one-3-ylcarbonylamino]-2-(benzyloxycarbonylamino)propionic acid,
3-[7-[(imidazolin-2-ylamino)methyl]-1-(2-phenylethyl)-6,8-difluoroquinoline-4-one-3-ylcarbonylamino]-2-(n-butyloxycarbonylamino)propionic acid,
3-[7-[(imidazolin-2-ylamino)methyl]-1-(2-phenylethyl)-6,8-difluoroquinoline-4-one-3-ylcarbonylamino]-2-(phenylsulfonylamino)propionic acid,
3-[7-[(imidazolin-2-ylamino)methyl]-1-(2-phenylethyl)-6,8-difluoroquinoline-4-one-3-ylcarbonylamino]-2-(n-butylsulfonylamino)propionic acid,
3-[7-[(tetrahydropyrimid-2-ylamino)methyl]-1-(2-phenylethyl)-6,8-difluoroquinoline-4-one-3-ylcarbonylamino]-2-(benzyloxycarbonylamino) propionic acid,
3-[7-[(tetrahydropyrimid-2-ylamino)methyl]-1-(2-phenylethyl)-6,8-difluoroquinoline-4-one-3-ylcarbonylamino]-2-(n-butyloxycarbonylamino)propionic acid,
3-[7-[(tetrahydropyrimid-2-ylamino)methyl]-1-(2-phenylethyl)-6,8-difluoroquinoline-4-one-3-ylcarbonylamino]-2-(phenylsulfonylamino)propionic acid,
3-[7-[(tetrahydropyrimid-2-ylamino)methyl]-1-(2-phenylethyl)-6,8-difluoroquinoline-4-one-3-ylcarbonylamino]-2-(n-butylsulfonyl)aminopropionic acid,
3-[7-[(2-aminothiazol-4-yl)methyl]-1-(2-phenylethyl)-6,8-difluoroquinoline-4-one-3-ylcarbonylamino]-2-(phenylsulfonylamino)propionic acid,
3-[7-[(2-aminothiazol-4-yl)methyl]-1-(2-phenylethyl)-6,8-difluoroquinoline-4-one-3-ylcarbonylamino]-2-(benzyloxycarbonylamino)propionic acid,
3-[7-[(imidazolin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(tetrahydropyrimid-2-ylamino)methyl]-1-(2-phenylethyl)-6,8-difluoroquinoline-4-one-3-ylcarbonylamino]-2-((2,4,6-trimethylphenyl)sulfon-ylamino)propionic acid,
3-[7-[(imidazol-2-ylamino)methyl]-1-(2-phenylethyl)-6,8-difluoroquinoline-4-one-3-ylcarbonylamino]-2-(benzyloxycarbonylamino)propionic acid,
3- [7-[(imidazol-2-ylamino)methyl]-1-(2-phenylethyl)-6,8-difluoroquinoline-4-one-3-ylcarbonylamino]-2-(phenylsulfonylamino)propionic acid,
3-[7-[(imidazol-2-ylamino)methyl]-1-(2-phenylethyl)-6,8-difluoroquinoline-4-one-3-ylcarbonylamino]-2-((2,6,dichlorophenyl)sulfonylamino)propionic acid,
3-[7-[(imidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(imidazol-2-ylamino)methyl]-1-(2-phenylethyl)-6,8-difluoroquinoline-4-one-3-ylcarbonylamino]-2-((4-biphenyl)sulfonylamino)propionic acid,
3-[7-[(benzimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(4-methylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(4,5-dimethylimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(4,5,6,7-tetrahydrobenzimidazol-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-[(pyridin-2-ylamino)methyl]-1-(2-phenylethyl)-6,8-difluoroquinoline-4-one-3-ylcarbonylamino]-2-((2,4,6-trimethylphenyl)sulfonylamino)propionic acid,
3-[7-(2-aminopyridin-6-yl)-1-(2-phenylethyl)-6,8-difluoroquinoline-4-one-3-ylcarbonylamino]-2-((2,4,6-trimethylphenyl)sulfonylamino)propionic acid, and
3-[7-[(7-azabenzimidazol-2-yl)methyl]-1-(2-phenylethyl)-6,8-difluoroquinoline-4-one-3-ylcarbonylamino]-2-((2,4,6-trimethylphenyl)sulfonylamino)propionic acid.

40. Use according to claim 34, wherein the radioisotope is 153_{Sm.}

41. Use according to claim 34, wherein the radioisotope is 177_{Lu}.

42. Use according to claim 41, wherein the radiopharmaceutical is selected from the group: 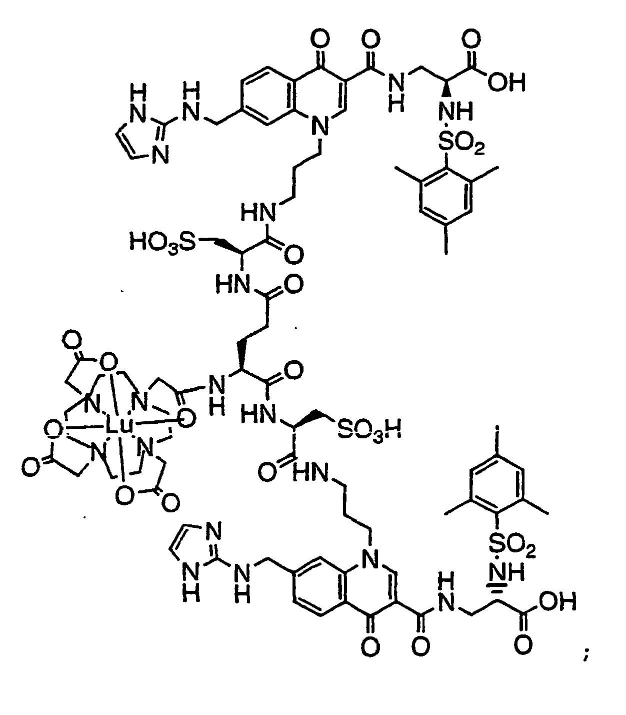 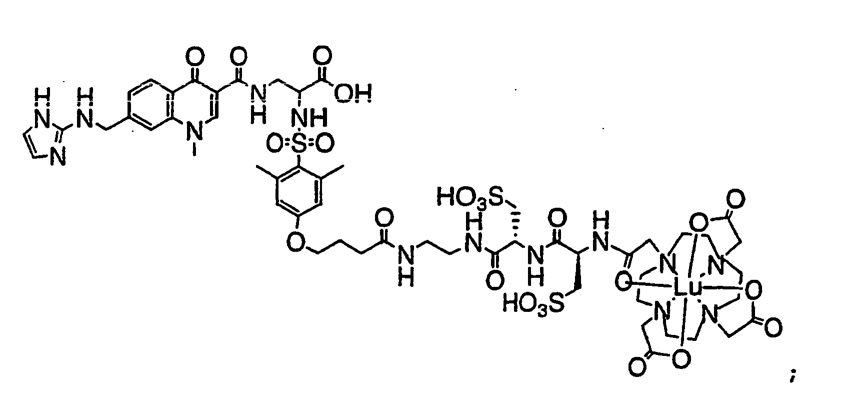 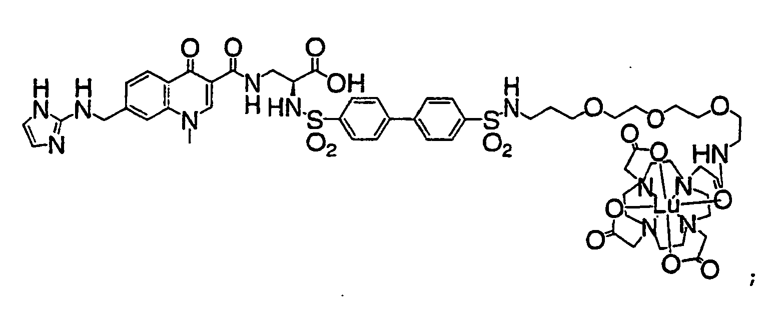 and

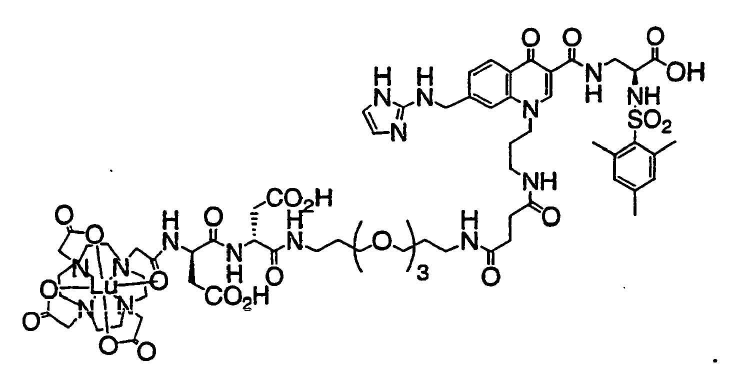

43. Use according to claim 34, wherein the radioisotope is ⁹⁰Y.

44. Use according to claim 43, wherein the radiopharmaceutical is selected from the group; 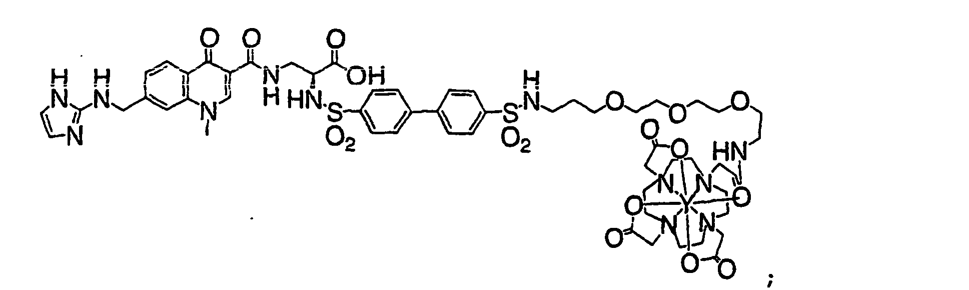 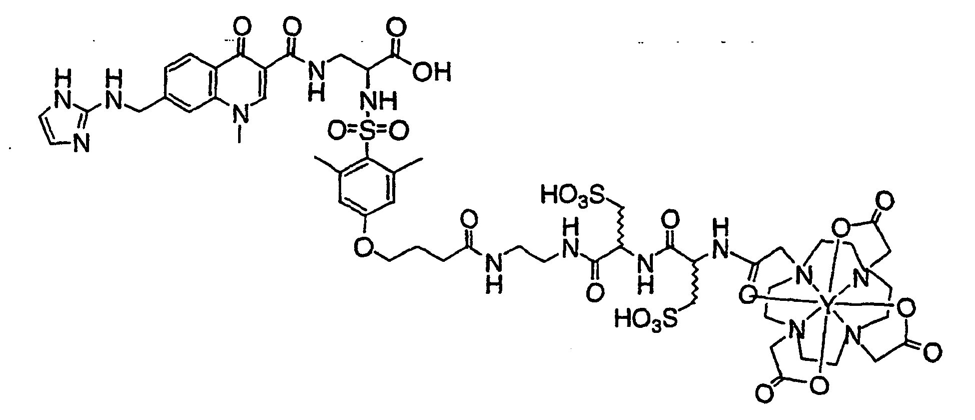 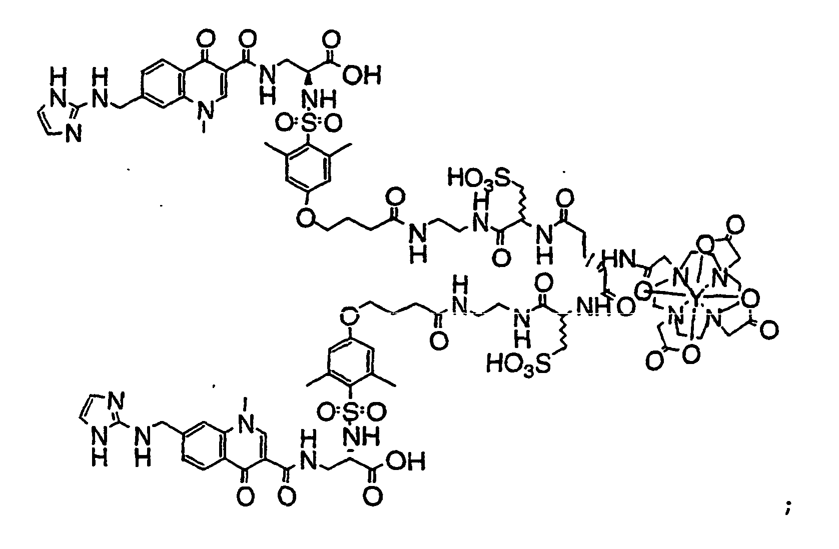 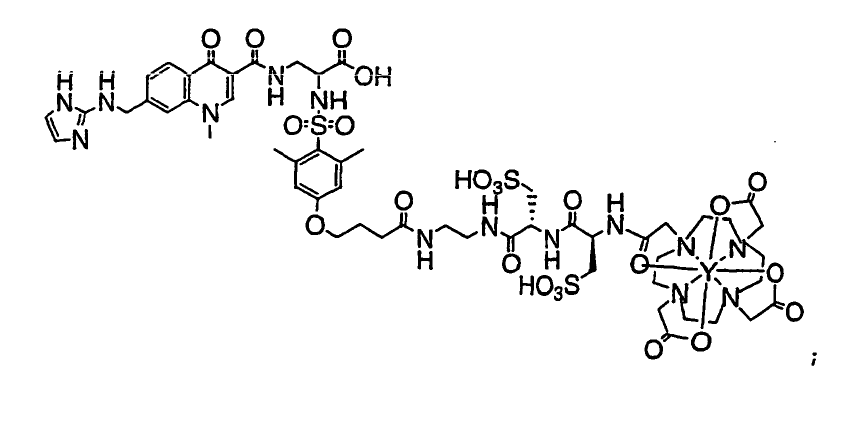 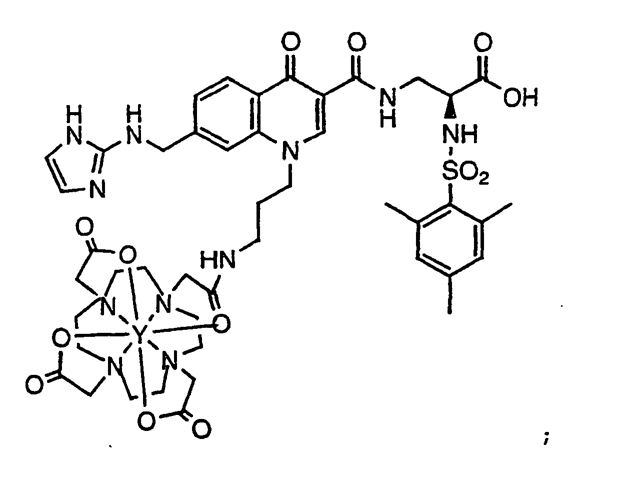 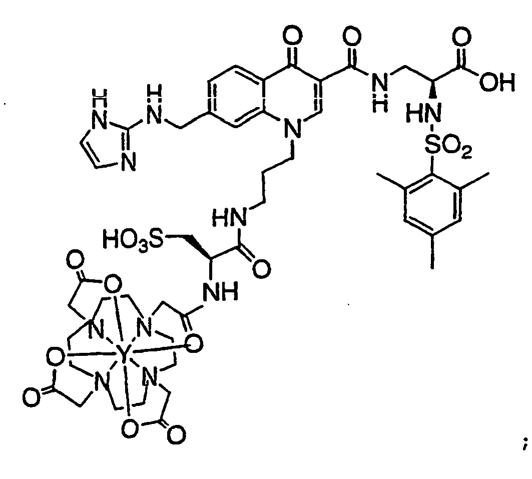 and

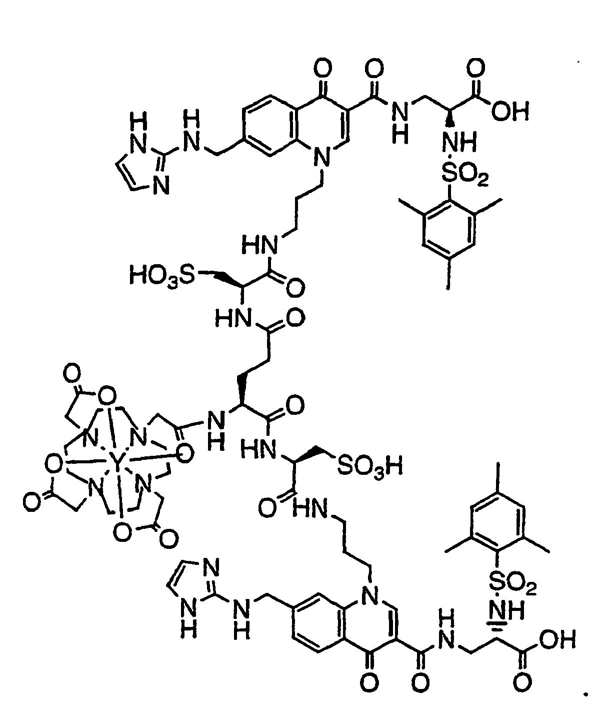

45. Use of claim 26, further comprising treating the cancer by brachytherapy, external beam radiation, laser therapy or surgical removal.

46. A kit comprising packaging material, and a therapeutic radiopharmaceutical composition of claim 12, contained within said packaging material, wherein the packaging material comprises a label or package insert which indicates that said therapeutic radiopharmaceutical composition can be used for treating cancer.

47. A therapeutic radiopharmaceutical composition of claim 12, further comprising a photosensitizing agent.

48. A therapeutic radiopharmaceutical composition according to claim 47, wherein the photosensitizing agent is selected from the group consisting of photofrin; naphthalocyanine photosensitizing agents; tetrapyrrole-based photosensitizers; porphyins; chlorins;, phthalocyanines; napthalocyanines; coumarins, psoralens, 1,3,4,6-tetramethoxyhelianthrone; 10,13-dimethyl-1,3,4,6-tetrahydroxyhelianthrone; 10,13-di(methoxycarbonyl)-1,3,4,6-tetramethoxyhelianthrone; 1,6-di-N-butylamino-3,4-dimethoxy-helianthrone; 1,6-diN-butylamino-3,4-dimethoxy-10,13-dimethyl-helianthrone; 1,6-di-(N-hydroxyethylamino)-3,4-dimethoxy-helianthrone; 2,5-dibromo-1,3,4,6-tetrahydroxyhelianthrone; and 2,5-dibromo-10,13-dimethyl-1,3,4,6-tetrahydroxyhelianthrone.

49. A kit according to claim 46, further comprising a photosensitizing agent.

50. A kit according to claim 49, wherein the photosensitizing agent is selected from the group consisting of photofrin; naphthalocyanine photosensitizing agents; tetrapyrrole-based photosensitizers; porphyins; chlorins;, phthalocyanines; napthalocyanines; coumarins, psoralens, 1,3,4,6-tetramethoxyhelianthrone; 10,13-dimethyl-1,3,4,6-tetrahydroxyhelianthrone; 10,13-di(methoxycarbonyl)-1,3,4,6-tetramethoxyhelianthrone; 1,6-di-N-butylamino-3,4-dimethoxy-helianthrone; 1,6-di-N-butylamino-3,4-dimethoxy-10,13-dimethyl-helianthrone; 1,6-di-(N-hydroxyethylamino)-3,4-dimethoxy-helianthrone; 2,5-dibromo-1,3,4,6-tetrahydroxyhelianthrone; and 2,5-dibromo-10,13-dimethyl-1,3,4,6-tetrahydroxyhelianthrone.

51. Use according to claim 26, further comprising treating the patient with photodynamic therapy.

52. Use according to claim 51,
wherein the photodynamic therapy comprises:
(a) administering a therapeutic radiopharmaceutical of the present invention and a photosensitive agent (photoreactive agent) to a patient, said photosensitive agent having a characteristic light absorption waveband and being preferentially absorbed by abnormal tissue;
(b) providing an imaging device that is integral with a plurality of light sources and produces a signal used for imaging abnormal tissue at the internal treatment site, said light sources emitting light in a waveband corresponding to the characteristic light absorption waveband of the photosensitive agent, said waveband including wavelengths sufficiently long to penetrate through a dermal layer of the patient to the internal treatment site;
(c) determining a location of the abnormal tissue at the internal targeted site within the body of the patient with the imaging device, by viewing an image of the abnormal tissue at the targeted site developed in response, to the signal produced by the imaging device; and
(d) energizing the light sources to administer light therapy to the internal targeted site at the location determined with the imaging device.

53. Use according to claim 52, wherein the photosensitive agent (photoreactive agent) is specifically targeted at the targeted tissue by including a binding agent that selectively links the photosensitive agent to the targeted tissue.

54. Use according to claim 52, wherein the photosensitizing agent is selected from the group consisting of photofrin; naphthalocyanine photosensitizing agents; tetrapyrrole-based photosensitizers; porphyins; chlorins;, phthalocyanines; napthalocyanines; coumarins, psoralens, 1,3,4,6-tetramethoxyhelianthrone; 10,13-dimethyl-1,3,4,6-tetrahydroxyhelianthrone; 10,13-di(methoxycarbonyl)-1,3,4,6-tetramethoxyhelianthrone; 1,6-di-N-butylamino-3,4-dimethoxy-helianthrone; 1,6-di-N-butylamino-3,4-dimethoxy-10,13-dimethyl-helianthrone; 1,6-di-(N-hydroxyethylamino)-3,4-dimethoxy-helianthrone; 2,5-dibromo-1,3,4,6-tetrahydroxyhelianthrone; and 2,5-dibromo-10,13-dimethyl-1,3,4,6-tetrahydroxyhelianthrone.

## Patentansprüche

1. Kit zur Behandlung von Krebs, umfassend eine Verbindung der Formel (I) und mindestens ein Mittel, ausgewählt aus der Gruppe, bestehend aus einem Antikrebsmittel und einem Radiosensibilisator, oder ein pharmazeutisch akzeptables Salz davon und einen pharmazeutisch akzeptablen Träger,
worin die Verbindung der Formel (I):
(Q)_{d}-Lₙ-Cₕ oder (Q)_{d}-Lₙ-(Cₕ)_{d'} (I),
worin Q eine Verbindung der Formel (II): 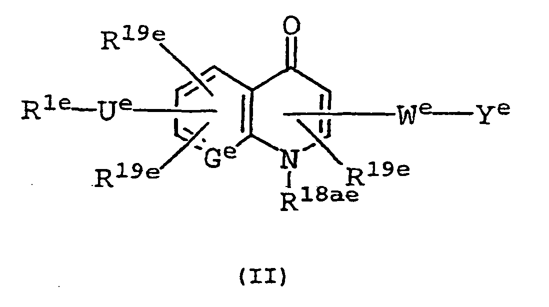 ist,
einschließlich stereoisomere Formen davon oder Gemische stereoisomerer Formen davon oder pharmazeutisch akzeptable Salz- oder Prodrug-Formen davon, worin:
R^{1e} ausgewählt ist aus: 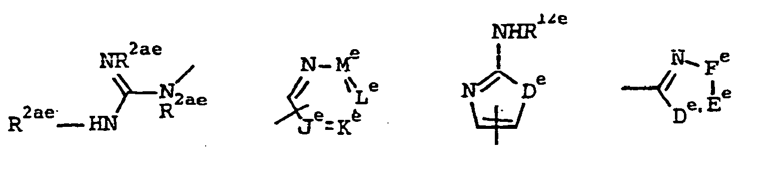 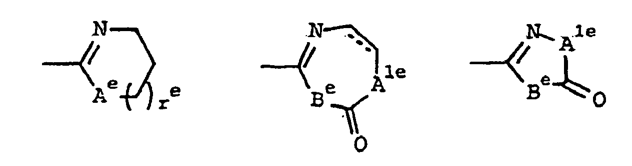 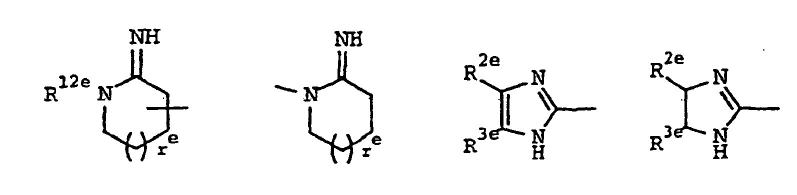 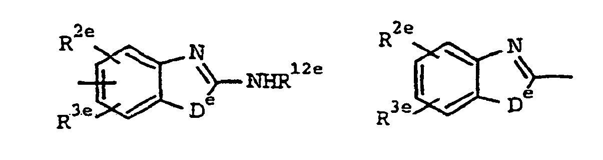 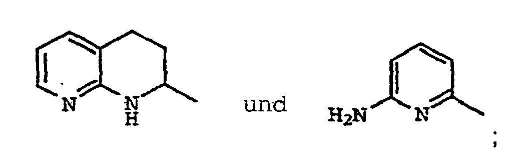
A^{e} -CH₂- oder -N(R^{10e})- ist;
A^{1e} und B^{e} unabhängig voneinander -CH₂- oder -N(R^{10e})- sind;
D^{e} -N(R^{10e})- oder -S- ist;
E^{e}-F^{e} -C(R^{2e})=C(R^{3e})- oder -C(R^{2e})₂C(R^{3e})₂- ist;
J^{e} -C(R^{2e})- oder -N- ist;
K^{e}, L^{e} und M^{e} unabhängig voneinander -C(R^{2e})- oder -C(R^{3e})- sind;
R^{2e} und R^{3e} unabhängig voneinander ausgewählt sind aus:
H, C₁-C₄-Alkoxy, NR^{11e}R^{12e}, Halogen, NO₂, CN, CF₃, C₁-C₆-Alkyl, C₃-C₆-Alkenyl, C₃-C₇-Cycloalkyl, C₃-C₇-Cycloalkyl(C₁-C₄-alkyl), Aryl(C₁-C₆-alkyl)-, (C₁-C₆-Alkyl)-carbonyl, (C₁-C₆-Alkoxy)carbonyl, Arylcarbonyl und Aryl, substituiert mit 0 - 4 R^{7e}, alternativ, wenn R^{2e} und R^{3e} Substituenten an nachbarständigen Atomen sind, diese mit den Kohlenstoffatomen, an die sie gebunden sind, unter Bildung eines 5- bis 7gliedrigen carbocyclischen oder 5- bis 7 gliedrigen heterocyclischen aromatischen oder nicht-aromatischen Ringsystems zusammengenommen werden können, wobei der carbocyclische oder heterocyclische Ring mit 0 - 2 Gruppen, ausgewählt aus C₁-C₄-Alkyl, C₁-C₄-Alkoxy, Halogen, Cyano, Amino, CF₃ und NO₂, substituiert ist;
R^{2ae} ausgewählt ist aus:
H, C₁-C₁₀-Alkyl, C₂-C₆-Alkenyl, C₃-C₁₁-Cycloalkyl, C₃-C₇-Cycloalkyl(C₁-C₄-alkyl), Aryl, Aryl(C₁-C₄-alkyl)-, (C₂-C₇-Alkyl)carbonyl, Arylcarbonyl, (C₂-C₁₀-Alkoxy)carbonyl, C₃-C₇-Cycloalkoxycarbonyl, C₇-C₁₁-Bicycloalkoxycarbonyl, Aryloxycarbonyl, Aryl(C₁-C₁₀-alkoxy)carbonyl, C₁-C₆-Alkylcarbonyloxy(C₁-C₄-alkoxy)carbonyl, Arylcarbonyloxy(C₁-C₄-alkoxy)carbonyl und C₃-C₇-Cycloalkylcarbonyloxy(C₁-C₄-alkoxy)carbonyl;
R^{7e} ausgewählt ist aus:
H, Hydroxy, C₁-C₄-Alkyl, C₁-C₄-Alkoxy, Aryl, Aryl(C₁-C₄-alkyl)-, (C₁-C₄-Alkyl)carbonyl, CO₂R^{18ae}, SO₂R^{11e}, SO₂NR^{10e}R^{11e}, OR^{10e} und N(R^{11e})R^{12e};
U^{e} ausgewählt ist aus:
-(CH₂)ₙ^{e}-, -(CH₂)ₙ^{e}O(CH₂)ₘ^{e}, -(CH₂)ₙ^{e}N(R¹²)(CH₂)ₘ^{e}-, -NH(CH₂)ₙ^{e}-, -(CH₂)ₙ^{e}C(=O)(CH₂)ₘ^{e}-, -(CH₂)ₙ^{e}S(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})- und -N(R^{10e})S(O)ₚ^{e}-;
G^{e} N oder CR^{19e} ist;
W^{e} -C(=O)-N(R^{10e})-(C₁-C₃-Alkylen)- ist, worin die Alkylengruppe durch R^{8e} und durch R^{9e} substituiert ist;
R^{8e} und R^{9e} unabhängig voneinander ausgewählt sind aus:
H, CO₂R^{18be}, C(=O)R^{18be}, CONR¹⁷R^{18be},
C₁-C₁₀-Alkyl, substituiert mit 0 - 1 R^{6e},
C₂-C₁₀-Alkenyl, substituiert mit 0 - 1 R^{6e},
C₂-C₁₀-Alkinyl, substituiert mit 0 - 1 R^{6e},
C₃-C₈-Cycloalkyl, substituiert mit 0 - 1 R^{6e},
C₅-C₆-Cycloalkenyl, substituiert mit 0 - 1 R^{6e},
(C₁-C₁₀-Alkyl)carbonyl,
C₃-C₁₀-Cycloalkyl(C₁-C₄-alkyl)-,
Phenyl, substituiert mit 0 - 3 R^{6e},
Naphthyl, substituiert mit 0 - 3 R^{6e},
einem 5- bis 10gliedrigen heterocyclischen Ring, enthaltend 1 - 3 N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist,
C₁-C₁₀-Alkoxy, substituiert mit 0 - 2 R^{7e},
Hydroxy, Nitro, -N(R^{10e})R^{11e}, -N(R^{16e})R^{17e}, Aryl(C₀-C₆-alkyl)carbonyl, Aryl(C₃-C₆-alkyl), Heteroaryl(C₁-C₆-alkyl), CONR^{18ae}R^{20e}, SO₂R^{18ae} und SO₂NR^{18ae}R^{20e},
mit der Maßgabe, daß jede der obigen Alkyl-, Cycloalkyl-, Aryl- oder Heteroarylgruppen unsubstituiert oder unabhängig mit 1 - 2 R^{7e} substituiert sein kann;
R^{6e} ausgewählt ist aus:
H, C₁-C₁₀-Alkyl, Hydroxy, C₁-C₁₀-Alkoxy, Nitro, C₁-C₁₀-Alkylcarbonyl, -N(R^{11e})R^{12e}, Cyano, Halogen, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(-O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
Aryl, substituiert mit 0 - 3 Gruppen, ausgewählt aus Halogen, C₁-C₆-Alkoxy, C₁-C₆-Alkyl, CF₃, S(O)ₘ^{e}Me und -NMe₂,
Aryl(C₁-C₄-alkyl)-, wobei das Aryl substituiert ist mit 0 - 3 Gruppen, ausgewählt aus Halogen, C₁-C₆-Alkoxy, C₁-C₆-Alkyl, CF₃, S(O)ₚ^{e}Me und -NMe₂, und
einem 5- bis 10gliedrigen heterocyclischen Ring, enthaltend 1 - 3 N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist;
R^{10e} ausgewählt ist aus:
H, -OH, CF₃, C₃-C₆-Alkenyl, C₃-C₁₁-Cycloalkyl, Aryl, (C₃-C₁₁-Cycloalkyl)methyl, Aryl(C₁-C₄-alkyl) und C₁-C₁₀-Alkyl, substituiert mit 0 - 2 R^{6e};
R^{11e} ausgewählt ist aus:
H, Hydroxy, C₁-C₈-Alkyl, C₃-C₆-Alkenyl, C₃-C₁₁-Cycloalkyl, (C₃-C₁₁-Cycloalkyl)-methyl, C₁-C₆-Alkoxy, Benzyloxy, Aryl, Heteroaryl, Heteroaryl(C₁-C₄-alkyl)-, Aryl(C₁-C₄-alkyl), Adamantylmethyl und C₁-C₁₀-Alkyl, substituiert mit 0 - 2 R^{4e};
R^{4e} ausgewählt ist aus:
H, C₁-C₆-Alkyl, C₃-C₇-Cycloalkyl, C₃-C₇-Cycloalkyl(C₁-C₄-alkyl)-, (C₁-C₁₀-Alkyl)-carbonyl, Aryl, Heteroaryl, Aryl(C₁-C₆-alkyl)- und Heteroaryl(C₁-C₆-alkyl)-, wobei die Aryl- oder Heteroarylgruppen mit 0 - 2 Substituenten, unabhängig ausgewählt aus der Gruppe, bestehend aus C₁-C₄-Alkyl, C₁-C₄-Alkoxy, F, Cl, Br, CF₃ und NO₂, substituiert sind,
alternativ, wenn R^{10e} und R^{11e} beide Substituenten an demselben Stickstoffatom sind (wie in -NR^{10e}R^{11e}), diese mit dem Stickstoffatom, an das sie gebunden sind, unter Bildung eines Heterocyclus, ausgewählt aus: 3-Azabicyclononyl, 1,2,3,4-Tetrahydro-1-chinolinyl, 1,2,3,4-Tetrahydro-2-isochinolinyl, 1-Piperidinyl, 1-Morpholinyl,1-Pyrrolidinyl, Thiamorpholinyl, Thiazolidinyl und 1-Piperazinyl, zusammengenommen werden können;
wobei der Heterocyclus mit 0 - 3 Gruppen, ausgewählt aus: C₁-C₆-Alkyl, Aryl, Heteroaryl, Aryl(C₁-C₄-alkyl)-, (C₁-C₆-Alkyl)carbonyl, (C₃-C₇-Cycloalkyl)carbonyl, (C₁-C₆-Alkoxy)carbonyl, Aryl(C₁-C₄-alkoxy)carbonyl, C₁-C₆-Alkylsulfonyl und Arylsulfonyl, substituiert ist;
R^{12e} ausgewählt ist aus:
H, C₁-C₆-Alkyl, Triphenylmethyl, Methoxymethyl, Methoxyphenyldiphenylmethyl, Trimethylsilylethoxymethyl, (C₁-C₆-Alkyl)carbonyl, (C₁-C₆-Alkoxy)carbonyl, (C₁-C₆-Alkyl)aminocarbonyl, C₃-C₆-Alkenyl, C₃-C₇-Cycloalkyl, C₃-C₇-Cycloalkyl(C₁-C₄-alkyl)-, Aryl, Heteroaryl(C₁-C₆-alkyl)carbonyl, Heteroarylcarbonyl, Aryl(C₁-C₆-alkyl)-, (C₁-C₆-Alkyl)carbonyl, Arylcarbonyl, C₁-C₆-Alkylsulfonyl, Arylsulfonyl, Aryl(C₁-C₆-alkyl)sulfonyl, Heteroarylsulfonyl, Heteroaryl(C₁-C₆-alkyl)sulfonyl, Aryloxycarbonyl und Aryl(C₁-C₆-alkoxy)carbonyl, wobei die Arylgruppen mit 0 - 2 Substituenten, ausgewählt aus der Gruppe, bestehend aus C₁-C₄-Alkyl, C₁-C₄-Alkoxy, Halogen, CF₃ und Nitro, substituiert sind;
R^{16e} ausgewählt ist aus:
-C(=O)OR^{18ae}, -C(=O)R^{18be}, -C(=O)N(R^{18be})₂, -C(=O)NHSO₂R^{18ae},
-C(=O)NHC(=O)R^{18be}, -C(=O)NHC(=O)OR^{18ae}, -C(=O)NHSO₂NHR^{18be},
-SO₂R^{18ae}, -SO₂N(R^{18be})₂ und -SO₂NHC(=O)OR^{18be},
R^{17e} ausgewählt ist aus:
H, C₁-C₆-Alkyl, C₃-C₇-Cycloalkyl, C₃-C₇-Cycloalkyl(C₁-C₄-alkyl)-, Aryl, Aryl(C₁-C₆-alkyl)- und Heteroaryl(C₁-C₆-alkyl);
R^{18ae} ausgewählt ist aus:
C₁-C₈-Alkyl, gegebenenfalls substituiert mit einer Bindung an Lₙ, C₃-C₁₁-Cycloalkyl, gegebenenfalls substituiert mit einer Bindung an Lₙ, Aryl(C₁-C₆-alkyl)-, gegebenenfalls substituiert mit einer Bindung an Lₙ, Heteroaryl(C₁-C₆-alkyl)-, gegebenenfalls substituiert mit einer Bindung an Lₙ, (C₁-C₆-Alkyl)heteroaryl, gegebenenfalls substituiert mit einer Bindung an Lₙ, Biaryl(C₁-C₆-alkyl), gegebenenfalls substituiert mit einer Bindung an Lₙ, Biaryl, gegebenenfalls substituiert mit einer Bindung an Lₙ, Heteroaryl, gegebenenfalls substituiert mit einer Bindung an Lₙ, Phenyl, substituiert mit 3 - 4 R^{19e} und gegebenenfalls substituiert mit einer Bindung an Lₙ, Naphthyl, substituiert mit 0 - 4 R^{19e} und gegebenenfalls substituiert mit einer Bindung an Lₙ, und einer Bindung an Lₙ, wobei die Aryl- oder Heteroarylgruppen gegebenenfalls mit 0 - 4 R^{19e} substituiert sind;
R^{18be} H oder R^{18ae} ist,
R^{19e} ausgewählt ist aus:
H, Halogen, CF₃, CO₂H, CN, NO₂, -NR^{11e}R^{12e}, OCF₃, C₁-C₈-Alkyl, C₂-C₆-Alkenyl, C₂-C₆-Alkinyl, C₃-C₁₁-Cycloalkyl, C₃-C₇-Cycloalkyl(C₁-C₄-alkyl)-, Aryl(C₁-C₆-alkyl)-, C₁-C₆-Alkoxy, C₁-C₄-Alkoxycarbonyl, Aryl, Aryl-O-, Aryl-SO₂-, Heteroaryl und Heteroaryl-SO₂-, wobei die Aryl- und Heteroarylgruppen mit 0 - 4 Gruppen, ausgewählt aus Wasserstoff, Halogen, CF₃, C₁-C₃-Alkyl und C₁-C₃-Alkoxy, substituiert sind;
R^{20e} ausgewählt ist aus:
Hydroxy, C₁-C₁₀-Alkyloxy, C₃-C₁₁-Cycloalkyloxy, Aryloxy, Aryl(C₁-C₄-alkyl)oxy, C₂-C₁₀-Alkylcarbonyloxy(C₁-C₂alkyl)oxy-, C₂-C₁₀-Alkoxycarbonyloxy(C₁-C₂-alkyl)oxy-, C₂-C₁₀-Alkoxycarbonyl(C₁-C₂alkyl)oxy-, C₃-C₁₀-Cycloalkylcarbonyloxy(C₁-C₂-alkyl)oxy-, C₃-C₁₀-Cycloalkoxycarbonyloxy(C₁-C₂alkyl)oxy-, C₃-C₁₀-Cycloalkoxycarbonyl(C₁-C₂-alkyl)oxy-, Aryloxycarbonyl(C₁-C₂-alkyl)oxy-, Aryloxycarbonyloxy(C₁-C₂-alkyl)oxy-, Arylcarbonyloxy(C₁-C₂-alkyl)oxy-, C₁-C₅-Alkoxy(C₁-C₅-alkyl)carbonyloxy(C₁-C₂-alkyl)oxy, (5-(C₁-C₅-Alkyl)-1,3-dioxa-cyclopenten-2-on-yl)methyloxy, (5-Aryl-1,3-dioxa-cyclopenten-2-on-yl)methyloxy und (R^{10e})(R^{11e})N-(C₁-C₁₀-Alkoxy)-;
R^{21e} ausgewählt ist aus:
C₁-C₈-Alkyl, C₂-C₆-Alkenyl, C₃-C₁₁-Cycloalkyl, (C₃-C₁₁-Cycloalkyl)methyl, Aryl, Aryl(C₁-C₄-alkyl)- und C₁-C₁₀-Alkyl, substituiert mit 0 - 2 R^{7e};
R^{22e} ausgewählt ist aus:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae} und -C(=O)NHSO₂NHR^{18be};
Y^{e} ausgewählt ist aus:
-COR^{20e}, -SO₃H, -PO₃H, -CONHNHSO₂CF₃, -CONHSO₂R^{18ae}, -CONHSO₂NHR^{18be}, -NHCOCF₃, -NHCONHSO₂R^{18ae}, -NHSO₂R^{18ae}, -OPO₃H₂, -OSO₃H, -PO₃H₂, -SO₂NHCOR^{18ae}, -SO₂NHCO₂R^{18ae}, 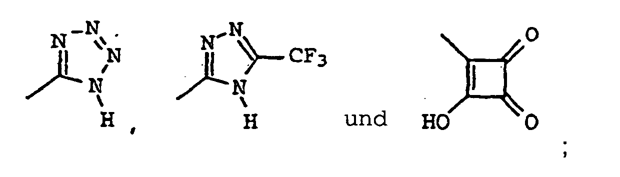
m^{e} 0 - 2 ist;
n^{e} 0 - 4 ist;
p^{e} 0 - 2 ist;
r^{e} 0 - 2 ist;
mit der folgenden Maßgabe: n^{e} und m^{e} sind so gewählt, daß die Anzahl an Atomen, die R^{1e} und Y^{e} verbinden, im Bereich von 8 - 14 liegt;
d ausgewählt ist aus 1, 2, 3, 4, 5, 6, 7, 8, 9 und 10;
d' 1 - 100 ist;
Lₙ eine Verknüpfungsgruppe mit der Formel:
((W)ₕ-(CR⁶R⁷)_{g})ₓ-(Z)ₖ((CR^{6a}R^{7a})_{g'}-(W)_{h'})_{x'}
ist;
W unabhängig bei jedem Vorkommen aus der Gruppe: O, S, NH, NHC(=O), C(=O)NH, NR⁸C(=O), C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ und (aa)_{t'} ausgewählt ist;
aa unabhängig bei jedem Vorkommen eine Aminosäure ist;
Z ausgewählt ist aus der Gruppe: Aryl, substituiert mit 0 - 3 R¹⁰, C₃-C₁₀-Cycloalkyl, substituiert mit 0 - 3 R¹⁰, und einem 5- bis 10gliedrigen heterocyclischen Ringsystem, enthaltend 1 - 4 Heteroatome, unabhängig ausgewählt aus N, S und O und substituiert mit 0 - 3 R¹⁰;
R⁶, R^{6a}, R⁷, R^{7a} und R⁸ unabhängig voneinander bei jedem Vorkommen aus der Gruppe: H, =O, COOH, SO₃H, PO₃H, C₁-C₅-Alkyl, substituiert mit 0 - 3 R¹⁰, Aryl, substituiert mit 0 - 3 R¹⁰, Benzyl, substituiert mit 0 - 3 R¹⁰, und C₁-C₅-Alkoxy, substituiert mit 0 - 3 R¹⁰, NHC(=O)R¹¹, C(=O)NHR¹¹, NHC(=O)NHR¹¹, NHR¹¹, R¹¹ und einer Bindung an Cₕ ausgewählt sind;
R¹⁰ unabhängig bei jedem Vorkommen aus der Gruppe: einer Bindung an Cₕ, COOR¹¹, C(=O)NHR¹¹, NHC(=O)R¹¹, OH, NHR¹¹, SO₃H, PO₃H, -OPO₃H₂, -OSO₃H, Aryl, substituiert mit 0 - 3 R¹¹, C₁₋₅-Alkyl, substituiert mit 0 - 1 R¹², C₁₋₅-Alkoxy, substituiert mit 0 - 1 R¹², und einem 5- bis 10gliedrigen heterocyclischen Ringsystem, enthaltend 1 - 4 Heteroatome, unabhängig ausgewählt aus N, S und O und substituiert mit 0 - 3 R¹¹, ausgewählt ist;
R¹¹ unabhängig bei jedem Vorkommen aus der Gruppe: H, -OPO₃H₂, -OSO₃H, C₁₋₅-Alkyl, substituiert mit 0 - 1 R¹², Aryl, substituiert mit 0 - 1 R¹², einem 5- bis 10gliedrigen heterocyclischen Ringsystem, enthaltend 1 - 4 Heteroatome, unabhängig ausgewählt aus N, S und O und substituiert mit 0 - 1 R¹², C₃₋₁₀-Cycloalkyl, substituiert mit 0 - 1 R¹², Polyalkylenglycol, substituiert mit 0 - 1 R¹², C₁-₅-Alkyl, substituiert mit einem Kohlenhydrat, substituiert mit 0 - 1 R¹², Cyclodextrin, substituiert mit 0 - 1 R¹², Aminosäure, substituiert mit 0 - 1 R¹², Polycarboxyalkyl, substituiert mit 0 -1 R¹², Polyazaalkyl, substituiert mit 0 - 1 R¹², Peptid, substituiert mit 0 - 1 R¹², wobei das Peptid aus 2 - 10 Aminosäuren besteht, C₁₋₅-Alkyl, substituiert mit 3,6-O-Disulfo-B-D-galactopyranosyl, Bis(phosphonomethyl)glycin und einer Bindung an Cₕ, ausgewählt ist;
R¹² eine Bindung an Cₕ ist;
k ausgewählt ist aus 0, 1 und 2;
h ausgewählt ist aus 0, 1 und 2;
h' ausgewählt ist aus 0, 1 und 2;
g ausgewählt ist aus 0, 1, 2, 3, 4, 5, 6, 7, 8, 9 und 10;
g' ausgewählt ist aus 0, 1, 2, 3, 4, 5, 6, 7, 8, 9 und 10;
s ausgewählt ist aus 0, 1, 2, 3, 4, 5, 6, 7, 8, 9 und 10;
s' ausgewählt ist aus 0, 1, 2, 3, 4, 5, 6, 7, 8, 9 und 10;
s" ausgewählt ist aus 0, 1, 2, 3, 4, 5, 6, 7, 8, 9 und 10;
t ausgewählt ist aus 0, 1, 2, 3, 4, 5, 6, 7, 8, 9 und 10;
t' ausgewählt ist aus 0, 1, 2, 3, 4, 5, 6, 7, 8, 9 und 10;
x ausgewählt ist aus 0, 1, 2, 3, 4, 5 und 6;
x' ausgewählt ist aus 0, 1, 2, 3, 4, 5 und 6;
Cₕ eine Metallbindungseinheit mit einer Formel, ausgewählt aus der Gruppe: 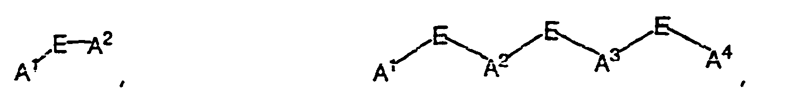 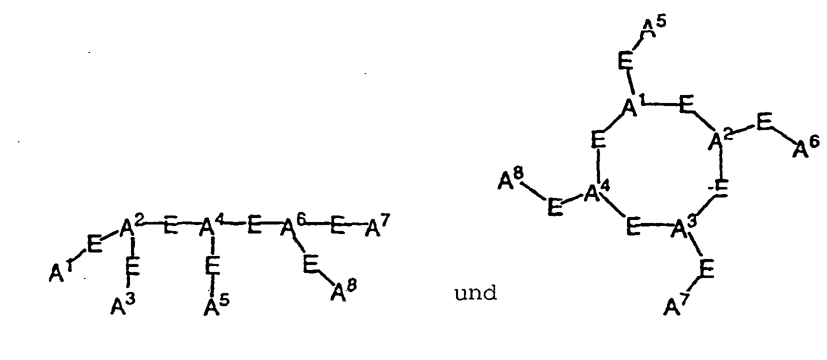 ist;
A¹, A², A³, A⁴, A⁵, A⁶, A⁷ und A⁸ unabhängig voneinander bei jedem Vorkommen aus der Gruppe: NR¹³, NR¹³R¹⁴, S, SH, S(Pg), O, OH, PR¹³, PR¹³R¹⁴, P(O)R¹⁵R¹⁶ und einer Bindung an Lₙ ausgewählt sind;
E eine Bindung, CH oder eine Spacer-Gruppe ist, unabhängig bei jedem Vorkommen ausgewählt aus der Gruppe: C₁-C₁₀-Alkyl, substituiert mit 0 - 3 R¹⁷, Aryl, substituiert mit 0 - 3 R¹⁷, C₃₋₁₀-Cycloalkyl, substituiert mit 0 - 3 R¹⁷, HeterocycloC₁₋₁₀-alkyl, substituiert mit 0 - 3 R¹⁷, wobei die Heterocyclogruppe ein 5- bis 10gliedriges heterocyclisches Ringsystem ist, enthaltend 1 - 4 Heteroatome, unabhängig ausgewählt aus N, S und O, C₆₋₁₀-Aryl-C₁₋₁₀-alkyl, substituiert mit 0 - 3 R¹⁷, C₁₋₁₀-Alkyl-C₆₋₁₀-aryl, substituiert mit 0 - 3 R¹⁷, und einem 5- bis 10-gliedrigen heterocyclischen Ringsystem, enthaltend 1 - 4 Heteroatome, unabhängig ausgewählt aus N, S und O und substituiert mit 0-3 R¹⁷;
R¹³ und R¹⁴ jeweils unabhängig voneinander ausgewählt sind aus der Gruppe: einer Bindung an Lₙ, Wasserstoff, C₁-C₁₀-Alkyl, substituiert mit 0 - 3 R¹⁷, Aryl, substituiert mit 0 - 3 R¹⁷, C₁₋₁₀-Cycloalkyl, substituiert mit 0 - 3 R¹⁷, Heterocyclo-C₁₋₁₀-alkyl, substituiert mit 0 - 3 R¹⁷, wobei die Heterocyclogruppe ein 5- bis 10gliedriges heterocyclisches Ringsystem ist, enthaltend 1-4 Heteroatom, unabhängig ausgewählt aus N, S und O, C₆₋₁₀-Aryl-C₁₋₁₀-alkyl, substituiert mit 0 - 3 R¹⁷, C₁₋₁₀-Alkyl-C₆₋₁₀-aryl, substituiert mit 0 - 3 R¹⁷, einem 5- bis 10gliedrigen heterocyclischen Ringsystem, enthaltend 1 - 4-Heteroatome, unabhängig ausgewählt aus N, S und O und substituiert mit 0 - 3 R¹⁷, und einem Elektron, vorausgesetzt, daß, wenn einer von R¹³ oder R¹⁴ ein Elektron ist, der andere auch ein Elektron ist;
alternativ R¹³ und R¹⁴ unter Bildung von =C(R²⁰)(R²¹) verbunden sind;
R¹⁵ und R¹⁶ jeweils unabhängig voneinander ausgewählt sind aus der Gruppe: einer Bindung an Lₙ, -OH, C₁-C₁₀-Alkyl, substituiert mit 0 - 3 R¹⁷, C₁-C₁₀-Alkyl, substituiert mit 0 - 3 R¹⁷, Aryl, substituiert mit 0 - 3 R¹⁷, C₃₋₁₀-Cycloalkyl, substituiert mit 0 - 3 R¹⁷, Heterocyclo-C₁₋₁₀-alkyl, substituiert mit 0 - 3 R¹⁷, wobei die Heterocyclogruppe ein 5- bis 10gliedriges heterocyclisches Ringsystem ist, enthaltend 1-4 Heteroatome, unabhängig ausgewählt aus N, S und O, C₆₋₁₀-Aryl-C₁₋₁₀-alkyl, substituiert mit 0 - 3 R¹⁷, C₁₋₁₀-Alkyl-C₆₋₁₀-aryl-, substituiert mit 0 - 3 R¹⁷, und einem 5- bis 10gliedrigen heterocyclischen Ringsystem, enthaltend 1 - 4 Heteroatom, unabhängig ausgewählt aus N, S und O und substituiert mit 0 - 3 R¹⁷;
R¹⁷ unabhängig bei jedem Vorkommen ausgewählt ist aus der Gruppe: einer Bindung an Lₙ, =O, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H, 2-(1-Morpholino)ethoxy, C₁-C₅-Alkyl, C₂-C₄-Alkenyl, C₃-C₆-Cycloalkyl, C₃-C₆-Cycloalkylmethyl, C₂-C₆-Alkoxyalkyl, Aryl, substituiert mit 0 - 2 R¹⁸, und einem 5- bis 10gliedrigen heterocyclischen Ringsystem, enthaltend 1 - 4 Heteroatome, unabhängig ausgewählt aus N, S und O;
R¹⁸, R^{18a} und R¹⁹ unabhängig voneinander bei jedem Vorkommen ausgewählt sind aus der Gruppe: einer Bindung an Lₙ, H, C₁-C₆-Alkyl, Phenyl, Benzyl, C₁-C₆-Alkoxy, Halogenid, Nitro, Cyano und Trifluormethyl;
Pg eine Thiol-Schutzgruppe ist;
R²⁰ und R²¹ unabhängig voneinander ausgewählt sind aus der Gruppe: H, C₁-C₁₀-Alkyl, -CN, -CO₂R²⁵, -C(=O)R²⁵, -C(=O)N(R²⁵)₂, C₂-C₁₀-1-Alken, substituiert mit 0-3R²³, C₂-C₁₀-1-Alkin, substituiert mit 0-3R²³, Aryl, substituiert mit 0-3R²³, einem ungesättigten 5- bis 10gliedrigen heterocyclischen Ringsystem, enthaltend 1 - 4 Heteroatome, unabhängig ausgewählt aus N, S und O und substituiert mit 0 - 3 R²³, und einem ungesättigten C₃₋₁₀-Carbocyclus, substituiert mit 0-3R²³;
alternativ R²⁰ und R²¹ zusammen mit dem zweiwertigen Kohlenstoffrest, an den sie gebunden sind: 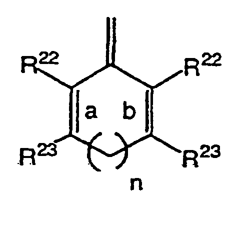 bilden;
R²² und R²³ unabhängig voneinander ausgewählt sind aus der Gruppe: H, R²⁴, C₁-C₁₀-Alkyl, substituiert mit 0-3R²⁴, C₂-C₁₀-Alkenyl, substituiert mit 0-3R²⁴, C₂-C₁₀-Alkinyl, substituiert mit 0-3R²⁴, Aryl, substituiert mit 0-3R²⁴, einem 5- bis 10gliedrigen heterocyclischen Ringsystem, enthaltend 1-4Heteroatome, unabhängig ausgewählt aus N, S und O und substituiert mit 0-3R²⁴, und einem C₃₋₁₀-Carbocyclus, substituiert mit 0-3R²⁴;
alternativ R²², R²³ zusammen ein kondensiertes aromatisches oder ein 5- bis 10gliedriges heterocyclisches Ringsystem, enthaltend 1 - 4 Heteroatome, unabhängig ausgewählt aus N, S und O, bilden;
a und b die Positionen optionaler Doppelbindungen anzeigen und n 0 oder 1 ist;
R²⁴ unabhängig bei jedem Vorkommen ausgewählt ist aus der Gruppe: =O, F, Cl, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)_{2,} =NOR²⁵, -C(=O)NHOR²⁵, -OCH₂CO₂H und 2-(1-Morpholino)ethoxy; und
R²⁵, R^{25a} und R²⁶ jeweils unabhängig voneinander bei jedem Vorkommen ausgewählt sind aus der Gruppe: Wasserstoff und C₁-C₆-Alkyl; oder ein pharmazeutisch akzeptables Salz davon ist.

2. Kit nach Anspruch 1, wobei dieses Kit viele separate Behälter umfaßt, wobei mindestens einer dieser Behälter ein oder mehrere Mittel, ausgewählt aus der Gruppe, bestehend aus einem Antikrebsmittel und einem Radiosensibilisator, oder ein pharmazeutisch akzeptables Salz davon und einen pharmazeutisch akzeptablen Träger enthält, und ein anderer dieser Behälter eine Verbindung der Formel (I):
(Q)_{d}-Lₙ-Cₕ oder (Q)_{d}-Lₙ-(Cₕ)_{d'} (I),
worin Q eine Verbindung der Formel (II): 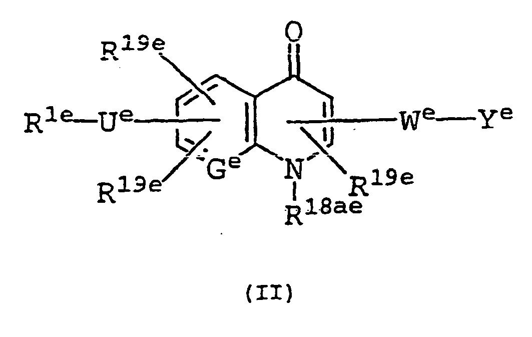 ist,
einschließlich stereoisomere Formen davon oder Gemische stereoisomerer Formen davon oder pharmazeutisch akzeptable Salz- oder Prodrug-Formen davon, worin:
R^{1e} ausgewählt ist aus: 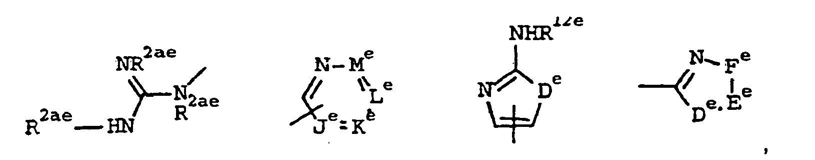 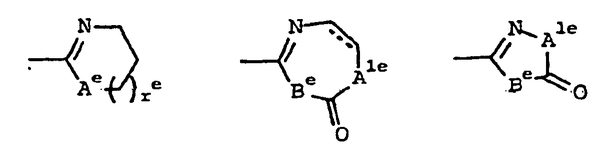 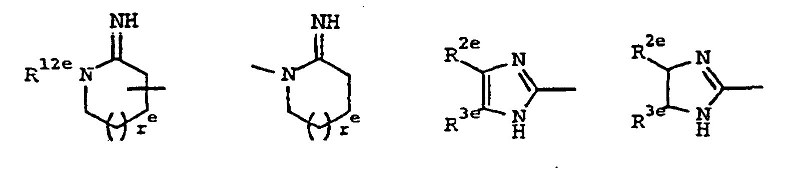 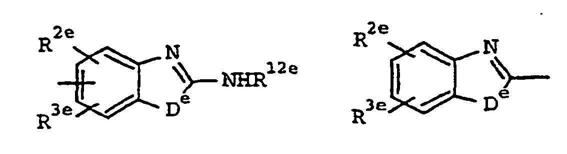 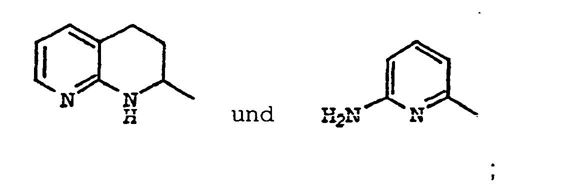
A^{e} -CH₂- oder -N(R^{10e})- ist;
A^{1e} und B^{e} unabhängig voneinander -CH₂- oder -N(R^{10e})- sind;
D^{e} -N(R^{10e})- oder -S- ist;
E^{e}-F^{e} -C(R^{2e})=C(R^{3e})- oder -C(R^{2e})₂C(R^{3e})₂- ist;
J^{e} -C(R^{2e})- oder -N- ist;
K^{e}, L^{e} und M^{e} unabhängig voneinander -C(R^{2e})- oder -C(R^{3e})- sind;
R^{2e} und R^{3e} unabhängig voneinander ausgewählt sind aus:
H, C₁-C₄-Alkoxy, NR^{11e}R^{12e}, Halogen, NO₂, CN, CF₃, C₁-C₆-Alkyl, C₃-C₆-Alkenyl, C₃-C₇-Cycloalkyl, C₃-C₇-Cycloalkyl(C₁-C₄-alkyl), Aryl(C₁-C₆-alkyl)-, (C₁-C₆-Alkyl)-carbonyl, (C₁-C₆-Alkoxy)carbonyl, Arylcarbonyl und Aryl, substituiert mit 0 - 4 R^{7e},
alternativ, wenn R^{2e} und R^{3e} Substituenten an nachbarständigen Atomen sind, diese mit den Kohlenstoffatomen, an die sie gebunden sind, unter Bildung eines 5- bis 7gliedrigen carbocyclischen oder 5- bis 7gliedrigen heterocyclischen aromatischen oder nicht-aromatischen Ringsystems zusammengenommen werden können, wobei der carbocyclische oder heterocyclische Ring mit 0 - 2 Gruppen, ausgewählt aus C₁-C₄-Alkyl, C₁-C₄-Alkoxy, Halogen, Cyano, Amino, CF₃ und NO₂, substituiert ist;
R^{2ae} ausgewählt ist aus:
H, C₁-C₁₀-Alkyl, C₂-C₆-Alkenyl, C₃-C₁₁-Cycloalkyl, C₃-C₇-Cycloalkyl(C₁-C₄-alkyl), Aryl, Aryl(C₁-C₄-alkyl)-, (C₂-C₇-Alkyl)carbonyl, Arylcarbonyl, (C₂-C₁₀-Alkoxy)carbonyl, C₃-C₇-Cycloalkoxycarbonyl, C₇-C₁₁-Bicycloalkoxycarbonyl, Aryloxycarbonyl, Aryl(C₁-C₁₀-alkoxy)carbonyl, C₁-C₆-Alkylcarbonyloxy(C₁-C₄-alkoxy)carbonyl, Arylcarbonyloxy(C₁-C₄-alkoxy)carbonyl und C₃-C₇-Cycloalkylcarbonyloxy(C₁-C₄-alkoxy)carbonyl;
R^{7e} ausgewählt ist aus:
H, Hydroxy, C₁-C₄-Alkyl, C₁-C₄-Alkoxy, Aryl, Aryl(C₁-C₄-alkyl)-, (C₁-C₄-Alkyl)carbonyl, CO₂R^{18ae}, SO₂R^{11e}, SO₂NR^{10e}R^{11e}, OR^{10e} und N(R^{11e})R^{12e};
U^{e} ausgewählt ist aus:
-(CH₂)ₙ^{e}- (CH₂)ₙ^{e}O(CH₂)ₘ^{e}-, -(CH₂)ₙ^{e}N(R¹²)(CH₂)ₘ^{e}-, -NH(CH₂)ₙ^{e}-, -(CH₂)ₙ^{e}C(=O)(CH₂)ₘ^{e}-, -(CH₂)ₙ^{e}S(O)ₚ^{e}(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})- und -N(R^{10e})S(O)ₚ^{e}-;
G^{e} N oder CR^{19e} ist;
W^{e} -C(=O)-N(R^{10e})-(C₁-C₃-Alkylen)- ist, worin die Alkylengruppe durch R^{8e} und durch R^{9e} substituiert ist;
R^{8e} und R^{9e} unabhängig voneinander ausgewählt sind aus:
H, CO₂R^{18be}, C(=O)R^{18be}, CONR¹⁷R^{18be},
C₁-C₁₀-Alkyl, substituiert mit 0-1R^{6e},
C₂-C₁₀-Alkenyl, substituiert mit 0-1R^{6e},
C₂-C₁₀-Alkinyl, substituiert mit 0-1R^{6e},
C₃-C₈-Cycloalkyl, substituiert mit 0-1R^{6e},
C₅-C₆-Cycloalkenyl, substituiert mit 0-1R^{6e},
(C₁-C₁₀-Alkyl)carbonyl,
C₃-C₁₀-Cycloalkyl(C₁-C₄-alkyl)-,
Phenyl, substituiert mit 0-3R^{6e},
Naphthyl, substituiert mit 0-3R^{6e},
einem 5- bis 10gliedrigen heterocyclischen Ring, enthaltend 1 - 3 N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist,
C₁-C₁₀-Alkoxy, substituiert mit 0-2R^{7e},
Hydroxy, Nitro, -N(R^{10e})R^{11e}, -N(R^{16e})R^{17e}, Aryl(C₀-C₆-alkyl)carbonyl, Aryl(C₃-C₆-alkyl), Heteroaryl(C₁-C₆-alkyl), CONR^{18ae}R^{20e}, SO₂R^{18ae} und SO₂NR^{18ae}R^{20e},
mit der Maßgabe, daß jede der obigen Alkyl-, Cycloalkyl-, Aryl- oder Heteroarylgruppen unsubstituiert oder unabhängig mit 1 - 2 R^{7e} substituiert sein kann;
R^{6e} ausgewählt ist aus:
H, C₁-C₁₀-Alkyl, Hydroxy, C₁-C₁₀-Alkoxy, Nitro, C₁-C₁₀-Alkylcarbonyl, -N(R^{11e})R^{12e}, Cyano, Halogen, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
Aryl, substituiert mit 0 - 3 Gruppen, ausgewählt aus Halogen, C₁-C₆-Alkoxy, C₁-C₆-Alkyl, CF₃, S(O)ₘ^{e}Me und -NMe₂,
Aryl(C₁-C₄-alkyl)-, wobei das Aryl substituiert ist mit 0 - 3 Gruppen, ausgewählt aus Halogen, C₁-C₆-Alkoxy, C₁-C₆-Alkyl, CF₃, S(O)ₚ^{e}Me und -NMe₂, und
einem 5- bis 10gliedrigen heterocyclischen Ring, enthaltend 1 - 3 N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist;
R^{10e} ausgewählt ist aus:
H, -OH, CF₃, C₃-C₆-Alkenyl, C₃-C₁₁-Cycloalkyl, Aryl, (C₃-C₁₁-Cycloalkyl)methyl, Aryl(C₁-C₄-alkyl) und C₁-C₁₀-Alkyl, substituiert mit 0 - 2 R^{6e};
R^{11e} ausgewählt ist aus:
H, Hydroxy, C₁-C₈-Alkyl, C₃-C₆-Alkenyl, C₃-C₁₁-Cycloalkyl, (C₃-C₁₁-Cycloalkyl)-methyl, C₁-C₆-Alkoxy, Benzyloxy, Aryl, Heteroaryl, Heteroaryl(C₁-C₄-alkyl)-, Aryl(C₁-C₄-alkyl), Adamantylmethyl und C₁-C₁₀-Alkyl, substituiert mit 0 - 2 R^{4e};
R^{4e} ausgewählt ist aus:
H, C₁-C₆-Alkyl, C₃-C₇-Cycloalkyl, C₃-C₇-Cycloalkyl(C₁-C₄-alkyl)-, (C₁-C₁₀-Alkyl)-carbonyl, Aryl, Heteroaryl, Aryl(C₁-C₆-alkyl)- und Heteroaryl(C₁-C₆-alkyl)-, wobei die Aryl- oder Heteroarylgruppen mit 0 - 2 Substituenten, unabhängig ausgewählt aus der Gruppe, bestehend aus C₁-C₄-Alkyl, C₁-C₄-Alkoxy, F, Cl, Br, CF₃ und NO₂, substituiert sind,
alternativ, wenn R^{10e} und R^{11e} beide Substituenten an demselben Stickstoffatom sind (wie in -NR^{10e}R^{11e}), diese mit dem Stickstoffatom, an das sie gebunden sind, unter Bildung eines Heterocyclus, ausgewählt aus: 3-Azabicyclononyl, 1,2,3,4-Tetrahydro-1-chinolinyl, 1,2,3,4-Tetrahydro-2-isochinolinyl, 1-Piperidinyl, 1-Morpholinyl,1-Pyrrolidinyl, Thiamorpholinyl, Thiazolidinyl und 1-Piperazinyl, zusammengenommen werden können;
wobei der Heterocyclus mit 0 - 3 Gruppen, ausgewählt aus:
C₁-C₆-Alkyl, Aryl, Heteroaryl, Aryl(C₁-C₄-alkyl)-, (C₁-C₆-Alkyl)carbonyl, (C₃-C₇-Cycloalkyl)carbonyl, (C₁-C₆-Alkoxy)carbonyl, Aryl(C₁-C₄-alkoxy)carbonyl, C₁-C₆-Alkylsulfonyl und Arylsulfonyl, substituiert ist;
R^{12e} ausgewählt ist aus:
H, C₁-C₆-Alkyl, Triphenylmethyl, Methoxymethyl, Methoxyphenyldiphenylmethyl, Trimethylsilylethoxymethyl, (C₁-C₆-Alkyl)carbonyl, (C₁-C₆-Alkoxy)carbonyl, (C₁-C₆-Alkyl)aminocarbonyl, C₃-C₆-Alkenyl, C₃-C₇-Cycloalkyl, C₃-C₇-Cycloalkyl-(C₁-C₄-alkyl)-, Aryl, Heteroaryl(C₁-C₆-alkyl)carbonyl, Heteroarylcarbonyl, Aryl-(C₁-C₆-alkyl)-, (C₁-C₆-Alkyl)carbonyl, Arylcarbonyl, C₁-C₆-Alkylsulfonyl, Arylsulfonyl, Aryl(C₁-C₆-alkyl)sulfonyl, Heteroarylsulfonyl, Heteroaryl(C₁-C₆-alkyl)sulfonyl, Aryloxycarbonyl und Aryl(C₁-C₆-alkoxy)carbonyl, wobei die Arylgruppen mit 0 - 2 Substituenten, ausgewählt aus der Gruppe, bestehend aus C₁-C₄-Alkyl, C₁-C₄-Alkoxy, Halogen, CF₃ und Nitro, substituiert sind;
R^{16e} ausgewählt ist aus:
-C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂ und -SO₂NHC(=O)OR^{18be},
R^{17e} ausgewählt ist aus:
H, C₁-C₆-Alkyl, C₃-C₇-Cycloalkyl, C₃-C₇-Cycloalkyl(C₁-C₄-alkyl)-, Aryl, Aryl(C₁-C₆-alkyl)- und Heteroaryl(C₁-C₆-alkyl);
R^{18ae} ausgewählt ist aus:
C₁-C₈-Alkyl, gegebenenfalls substituiert mit einer Bindung an Lₙ, C₃-C₁₁-Cycloalkyl, gegebenenfalls substituiert mit einer Bindung an Lₙ, Aryl(C₁-C₆-alkyl)-, gegebenenfalls substituiert mit einer Bindung an Lₙ, Heteroaryl(C₁-C₆-alkyl)-, gegebenenfalls substituiert mit einer Bindung an Lₙ, (C₁-C₆-Alkyl)heteroaryl, gegebenenfalls substituiert mit einer Bindung an Lₙ, Biaryl(C₁-C₆-alkyl), gegebenenfalls substituiert mit einer Bindung an Lₙ, Heteroaryl, gegebenenfalls substituiert mit einer Bindung an Lₙ, Phenyl, substituiert mit 3-4R^{19e} und gegebenenfalls substituiert mit einer Bindung an Lₙ, Naphthyl, substituiert mit 0-4 R^{19e} und gegebenenfalls substituiert mit einer Bindung an Lₙ, und einer Bindung an Lₙ, wobei die Aryl- oder Heteroarylgruppen gegebenenfalls mit 0 - 4 R^{19e} substituiert sind;
R^{18be} H oder R^{18ae} ist,
R^{19e} ausgewählt ist aus:
H, Halogen, CF₃, CO₂H, CN, NO₂, -NR^{11e}R^{12e}, OCF₃, C₁-C₈-Alkyl, C₁-C₆-Alkenyl, C₂-C₆-Alkinyl, C₃-C₁₁-Cycloalkyl, C₃-C₇-Cycloalkyl(C₁-C₄-alkyl)-, Aryl(C₁-C₆-alkyl)-, C₁-C₆-Alkoxy, C₁-C₄-Alkoxycarbonyl, Aryl, Aryl-O-, Aryl-SO₂-, Heteroaryl und Heteroaryl-SO₂-, wobei die Aryl- und Heteroarylgruppen mit 0 - 4 Gruppen, ausgewählt aus Wasserstoff, Halogen, CF₃, C₁-C₃-Alkyl und C₁-C₃-Alkoxy, substituiert sind;
R^{20e} ausgewählt ist aus:
Hydroxy, C₁-C₁₀-Alkyloxy, C₃-C₁₁-Cycloalkyloxy, Aryloxy, Aryl(C₁-C₄-alkyl)oxy, C₁-C₁₀-Alkylcarbonyloxy(C₁-C₂-alkyl)oxy-, C₂-C₁₀-Alkoxycarbonyloxy(C₁-C₂-alkyl)oxy-, C₂-C₁₀-Alkoxycarbonyl(C₁-C₂-alkyl)oxy-, C₃-C₁₀-Cycloalkylcarbonyloxy(C₁-C₂-alkyl)oxy-, C₃-C₁₀-Cycloalkoxycarbonyloxy(C₁-C₂-alkyl)oxy-, C₃-C₁₀-Cycloalkoxycarbonyl(C₁-C₂-alkyl)oxy-, Aryloxycarbonyl(C₁-C₂-alkyl)oxy-, Aryloxycarbonyloxy(C₁-C₂-alkyl)oxy-, Arylcarbonyloxy(C₁-C₂-alkyl)oxy-, C₁-C₅-Alkoxy(C₁-C₅-alkyl)carbonyloxy(C₁-C₂-alkyl)oxy, (5-(C₁-C₅-Alkyl)-1,3-dioxa-cyclopenten-2-on-yl)methyloxy, (5-Aryl-1,3-dioxa-cyclopenten-2-on-yl)methyloxy und (R^{10e})(R^{11e})N-(C₁-C₁₀-Alkoxy)-;
R^{21e} ausgewählt ist aus:
C₁-C₈-Alkyl, C₂-C₆-Alkenyl, C₃-C₁₁-Cycloalkyl, (C₃-C₁₁-Cycloalkyl)methyl, Aryl, Aryl(C₁-C₄-alkyl)- und C₁-C₁₀-Alkyl, substituiert mit 0 - 2 R^{7e};
R^{22e} ausgewählt ist aus:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae} und -C(=O)NHSO₂NHR^{18be};
Y^{e} ausgewählt ist aus:
-COR^{20e}, -SO₃H, -PO₃H, -CONHNHSO₂CF₃, -CONHSO₂R^{18ae}, -CONHSO₂NHR^{18be}, -NHCOCF₃, -NHCONHSO₂R^{18ae}, -NHSO₂R^{18ae}, -OPO₃H₂, -OSO₃H, -PO₃H₂, -SO₂NHCOR^{18ae}, -SO₂NHCO₂R^{18ae}, 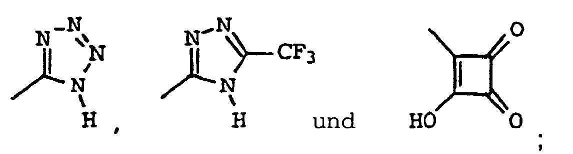
m^{e} 0 - 2 ist;
n^{e} 0 - 4 ist;
p^{e} 0 - 2 ist;
r^{e} 0 - 2 ist;
mit der folgenden Maßgabe: n^{e} und m^{e} sind so gewählt, daß die Anzahl an Atomen, die R^{1e} und Y^{e} verbinden, im Bereich von 8 - 14 liegt;
d ausgewählt ist aus 1, 2, 3, 4, 5, 6, 7, 8, 9 und 10;
d' 1 -100 ist;
Lₙ eine Verknüpfungsgruppe mit der Formel:
((W)ₕ-(CR⁶R⁷)_{g})ₓ-(Z)ₖ-((CR^{6a}R^{7a})_{g'}-(W)_{h'})_{x'}
ist;
W unabhängig bei jedem Vorkommen aus der Gruppe: O, S, NH, NHC(=O), C(=O)NH, NR⁸C(=O), C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ und (aa)_{t'} ausgewählt ist;
aa unabhängig bei jedem Vorkommen eine Aminosäure ist;
Z ausgewählt ist aus der Gruppe: Aryl, substituiert mit 0-3R¹⁰, C₃₋₁₀-Cycloalkyl, substituiert mit 0-3R¹⁰, und einem 5- bis 10gliedrigen heterocyclischen Ringsystem, enthaltend 1-4Heteroatome, unabhängig ausgewählt aus N, S und O und substituiert mit 0-3R¹⁰;
R⁶, R^{6a}, R⁷, R^{7a} und R⁸ unabhängig voneinander bei jedem Vorkommen aus der Gruppe: H, =O, COOH, SO₃H, PO₃H, C₁-C₅-Alkyl, substituiert mit 0-3R¹⁰, Aryl, substituiert mit 0-3R¹⁰, Benzyl, substituiert mit 0-3R¹⁰, und C₁-C₅-Alkoxy, substituiert mit 0-3R¹⁰, NHC(=O)R¹¹, C(=O)NHR¹¹, NHC(=O)NHR¹¹, NHR¹¹, R¹¹ und einer Bindung an Cₕ ausgewählt sind;
R¹⁰ unabhängig bei jedem Vorkommen aus der Gruppe: einer Bindung an Cₕ, COOR¹¹, C(=O)NHR¹¹, NHC(=O)R¹¹, OH, NHR¹¹, SO₃H, PO₃H, -OPO₃H₂, -OSO₃H, Aryl, substituiert mit 0-3R¹¹, C₁₋₅-Alkyl, substituiert mit 0-1R¹², C₁₋₅-Alkoxy, substituiert mit 0-1R¹², und einem 5- bis 10gliedrigen heterocyclischen Ringsystem, enthaltend 1-4Heteroatome, unabhängig ausgewählt aus N, S und O und substituiert mit 0-3R¹¹, ausgewählt ist;
R¹¹ unabhängig bei jedem Vorkommen aus der Gruppe: H, -OPO₃H₂, -OSO₃H, C₁₋₅-Alkyl, substituiert mit 0-1R¹², Aryl, substituiert mit 0-1R¹² , einem 5- bis 10gliedrigen heterocyclischen Ringsystem, enthaltend 1-4Heteroatome, unabhängig ausgewählt aus N, S und O und substituiert mit 0-1R¹², C₃₋₁₀-Cycloalkyl, substituiert mit 0-1R¹², Polyalkylenglycol, substituiert mit 0-1R¹², C₁₋₅-Alkyl, substituiert mit einem Kohlenhydrat, substituiert mit 0-1R¹², Cyclodextrin, substituiert mit 0-1R¹², Aminosäure, substituiert mit 0-1R¹², Polycarboxyalkyl, substituiert mit 0-1R¹², Polyazaalkyl, substituiert mit 0-1R¹², Peptid, substituiert mit 0-1R¹², wobei das Peptid aus 2-10Aminosäuren besteht, 3,6-O-Disulfo-B-D-galactopyranosyl, Bis(phosphonomethyl)glycin und einer Bindung an Cₕ, ausgewählt ist;
R¹² eine Bindung an Cₕ ist;
k ausgewählt ist aus 0, 1 und 2;
h ausgewählt ist aus 0,1 und 2;
h' ausgewählt ist aus 0,1 und 2;
g ausgewählt ist aus 0, 1, 2, 3, 4, 5, 6, 7, 8, 9 und 10;
g' ausgewählt ist aus 0, 1, 2, 3, 4, 5, 6, 7, 8, 9 und 10;
s ausgewählt ist aus 0, 1, 2, 3, 4, 5, 6, 7, 8, 9 und 10;
s' ausgewählt ist aus 0, 1, 2, 3, 4, 5, 6, 7, 8, 9 und 10;
s" ausgewählt ist aus 0, 1, 2, 3, 4, 5, 6, 7, 8, 9 und 10;
t ausgewählt ist aus 0, 1, 2, 3, 4, 5, 6, 7, 8, 9 und 10;
t' ausgewählt ist aus 0, 1, 2, 3, 4, 5, 6, 7, 8, 9 und 10;
x ausgewählt ist aus 0, 1, 2, 3, 4, 5 und 6;
x' ausgewählt ist aus 0, 1, 2, 3, 4, 5 und 6;
Cₕ eine Metallbindungseinheit mit einer Formel, ausgewählt aus der Gruppe: 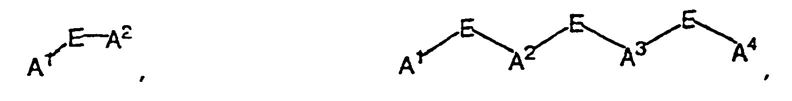 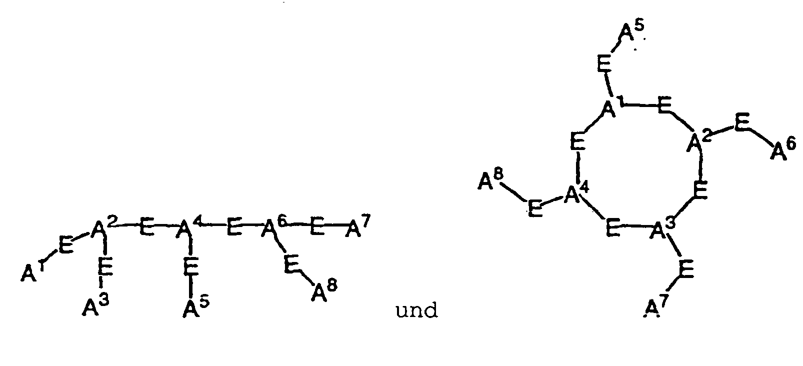 ist;
A¹, A², A³, A⁴, A⁵, A⁶, A⁷ und A⁸ unabhängig voneinander bei jedem Vorkommen aus der Gruppe: NR¹³, NR¹³R¹⁴, S, SH, S(Pg), O, OH, PR¹³, PR¹³R¹⁴, P(O)R¹⁵R¹⁶ und einer Bindung an Lₙ ausgewählt sind;
E eine Bindung, CH oder eine Spacer-Gruppe ist, unabhängig bei jedem Vorkommen ausgewählt aus der Gruppe: C₁-C₁₀-Alkyl, substituiert mit 0-3R¹⁷, Aryl, substituiert mit 0-3R¹⁷, C₃₋₁₀-Cycloalkyl, substituiert mit 0-3R¹⁷, Heterocyclo-C₁₋₁₀-alkyl, substituiert mit 0-3R¹⁷, wobei die Heterocyclogruppe ein 5- bis 10gliedriges heterocyclisches Ringsystem ist, enthaltend 1-4Heteroatome, unabhängig ausgewählt aus N, S und O, C₆₋₁₀-Aryl-C₁₋₁₀-alkyl, substituiert mit 0-3 R¹⁷, C₁₋₁₀-Alkyl-C₆₋₁₀-aryl, substituiert mit 0-3R¹⁷, und einem 5- bis 10gliedrigen heterocyclischen Ringsystem, enthaltend 1-4Heteroatome, unabhängig ausgewählt aus N, S und O und substituiert mit 0-3R¹⁷;
R¹³ und R¹⁴ jeweils unabhängig voneinander ausgewählt sind aus der Gruppe: einer Bindung an Lₙ, Wasserstoff, C₁-C₁₀-Alkyl, substituiert mit 0-3R¹⁷, Aryl, substituiert mit 0-3R¹⁷, C₁₋₁₀-Cycloalkyl, substituiert mit 0-3R¹⁷, Heterocyclo-C₁₋₁₀-alkyl, substituiert mit 0-3R¹⁷, wobei die Heterocyclogruppe ein 5- bis 10gliedriges heterocyclisches Ringsystem ist, enthaltend 1-4Heteroatome, unabhängig ausgewählt aus N, S und O, C₆₋₁₀-Aryl-C₁₋₁₀-alkyl, substituiert mit 0-3R¹⁷, C₁₋₁₀-Alkyl-C₆₋₁₀-aryl, substituiert mit 0-3R¹⁷, einem 5- bis 10gliedrigen heterocyclischen Ringsystem, enthaltend 1-4Heteroatome, unabhängig ausgewählt aus N, S und O und substituiert mit 0-3R¹⁷, und einem Elektron, vorausgesetzt, daß, wenn einer von R¹³ oder R¹⁴ ein Elektron ist, der andere auch ein Elektron ist;
alternativ R¹³ und R¹⁴ unter Bildung von =C(R²⁰)(R²¹) verbunden sind;
R¹⁵ und R¹⁶ jeweils unabhängig voneinander ausgewählt sind aus der Gruppe: einer Bindung an Lₙ, -OH, C₁-C₁₀-Alkyl, substituiert mit 0-3R¹⁷, C₁-C₁₀-Alkyl, substituiert mit 0-3R¹⁷, Aryl, substituiert mit 0-3R¹⁷, C₃₋₁₀-Cycloalkyl substituiert mit 0-3R¹⁷, Heterocyclo-C₁₋₁₀-alkyl, substituiert mit 0-3R¹⁷, wobei die Heterocyclogruppe ein 5-bis 10gliedriges heterocyclisches Ringsystem ist, enthaltend 1-4 Heteroatome, unabhängig ausgewählt aus N, S und O, C₆₋₁₀-Aryl-C₁₋₁₀-alkyl, substituiert mit 0-3R¹⁷, C₁₋₁₀-Alkyl-C₆₋₁₀-aryl-, substituiert mit 0-3R¹⁷, und einem 5- bis 10gliedrigen heterocyclischen Ringsystem, enthaltend 1-4Heteroatom, unabhängig ausgewählt aus N, S und O und substituiert mit 0-3R¹⁷;
R¹⁷ unabhängig bei jedem Vorkommen ausgewählt ist aus der Gruppe: einer Bindung an Lₙ, =O, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18ae}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO_{3H}, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H, 2-(1-Morpholino)ethoxy, C₁-C₅-Alkyl, C₂-C₄-Alkenyl, C₃-C₆-Cycloalkyl, C₃-C₆-Cycloalkylmethyl, C₂-C₆-Alkoxyalkyl, Aryl, substituiert mit 0 - 2 R¹⁸, und einem 5- bis 10gliedrigen heterocyclischen Ringsystem, enthaltend 1 - 4 Heteroatome, unabhängig ausgewählt aus N, S und O;
R¹⁸, R^{18a} und R¹⁹ unabhängig voneinander bei jedem Vorkommen ausgewählt sind aus der Gruppe: einer Bindung an Lₙ, H, C₁-C₆-Alkyl, Phenyl, Benzyl, C₁-C₆-Alkoxy, Halogenid, Nitro, Cyano und Trifluormethyl;
Pg eine Thiol-Schutzgruppe ist;
R²⁰ und R²¹ unabhängig voneinander ausgewählt sind aus der Gruppe: H, C₁-C₁₀-Alkyl, -CN, -CO₂R²⁵, -C(=O)R²⁵, -C(=O)N(R²⁵)₂, C₂-C₁₀-1-Alken, substituiert mit 0-3R²³, C₂-C₁₀-1-Alkin, substituiert mit 0-3R²³, Aryl, substituiert mit 0-3R²³, einem ungesättigten 5- bis 10gliedrigen heterocyclischen Ringsystem, enthaltend 1-4 Heteroatome, unabhängig ausgewählt aus N, S und O und substituiert mit 0-3R²³ und einem ungesättigten C₃₋₁₀-Carbocyclus, substituiert mit 0-3R²³;
alternativ R²⁰ und R²¹ zusammen mit dem zweiwertigen Kohlenstoffrest, an den sie gebunden sind: 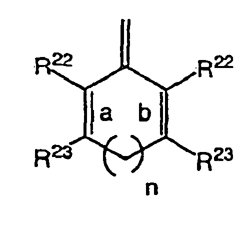 bilden,
R²² und R²³ unabhängig voneinander ausgewählt sind aus der Gruppe: H, R²⁴, C₁-C₁₀-Alkyl, substituiert mit 0-3R²⁴, C₂-C₁₀-Alkenyl, substituiert mit 0-3 R²⁴, C₂-C₁₀-Alkinyl, substituiert mit 0-3R²⁴, Aryl, substituiert mit 0-3R²⁴, einem 5- bis 10gliedrigen heterocyclischen Ringsystem, enthaltend 1-4Hetero-atome, unabhängig ausgewählt aus N, S und O und substituiert mit 0-3R²⁴, und einem C₃₋₁₀-Carbocyclus, substituiert mit 0-3R²⁴;
alternativ R²², R²³ zusammen ein kondensiertes aromatisches oder ein 5- bis 10gliedriges heterocyclisches Ringsystem, enthaltend 1 - 4 Heteroatome, unabhängig ausgewählt aus N, S und O, bilden;
a und b die Positionen optionaler Doppelbindungen anzeigen und n 0 oder 1 ist;
R²⁴ unabhängig bei jedem Vorkommen ausgewählt ist aus der Gruppe: =O, F, Cl, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H und 2-(1-Morpholino)ethoxy und
R²⁵, R^{25a} und R²⁶ jeweils unabhängig voneinander bei jedem Vorkommen ausgewählt sind aus der Gruppe: Wasserstoff und C₁-C₆-Alkyl;
oder ein pharmazeutisch akzeptables Salz davon enthält.

3. Kit nach Anspruch 1, wobei das Antikrebsmittel ausgewählt ist aus der Gruppe, bestehend aus Mitomycin, Tretinoin, Ribomustin, Gemcitabin, Vincristin, Etoposid, Cladribin, Nitobronitol, Methotrexat, Doxorubicin, Carboquon, Pentostatin, Nitracrin, Zinostatin, Cetrorelix, Letrozol, Raltitrexed, Daunorubicin, Fadrozol, Fotemustin, Thymalfasin, Sobuzoxan, Nedaplatin, Cytarabin, Bicalutamid, Vinorelbin, Vesnarinon, Aminoglutethimid, Amsacrin, Proglumid, Elliptiniumacetat, Ketanserin, Doxifluridin, Etretinat, Isotretinoin, Streptozocin, Nimustin, Vindesin, Flutamid, Drogenil, Butocin, Carmofur, Razoxan, Sizofilan, Carboplatin, Mitolactol, Tegafur, Ifosfamid, Prednimustin, Picibanil, Levamisol, Teniposid, Improsulfan, Enocitabin, Lisurid, Oxymetho-Ion, Tamoxifen, Progesteron, Mepitiostan, Epitiostanol, Formestan, Interferon-alpha, Interferon-2-alpha, Interferon-beta, Interferon-gamma, Colony-stimulating-Faktor 1, Colony-stimulating-Faktor 2, Denileukin diftitox, Interleukin-2 und Freisetzungsfaktor für Luteinisierungshormon.

4. Kit nach Anspruch 1, wobei der Radiosensibilisator ausgewählt ist aus der Gruppe, bestehend aus 2-(3-Nitro-1,2,4-triazol-1-yl)-N-(2-methoxyethyl)acetamid, N-(3-Nitro-4-chinolinyl)-4-morpholincarboxamidin, 3-Amino-1,2,4-benzotriazin-1,4-dioxid, N-(2-Hydroxyethyl)-2-nitroimidazol-1-acetamid, 1-(2-Nitroimidazol-1-yl)-3-(1-piperidinyl)-2-propanol und 1-(2-Nitro-1-imidazolyl)-3-(1-aziridino)-2-propanol.

5. Kit nach Anspruch 1, wobei Q eine Verbindung der Formel (IV): 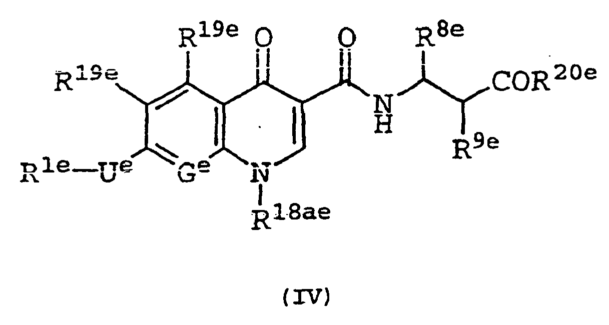 ist,
einschließlich stereoisomere Formen davon oder Gemische stereoisomerer Formen davon oder pharmazeutisch akzeptable Salz- oder Prodrug-Formen davon, worin:
R^{1e} ausgewählt ist aus: 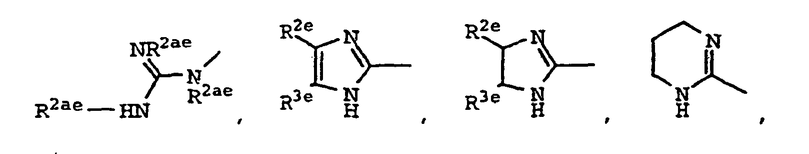 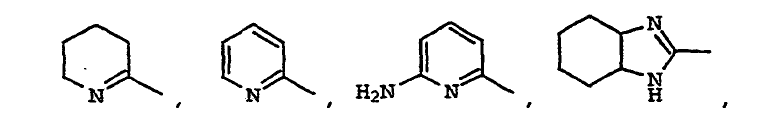 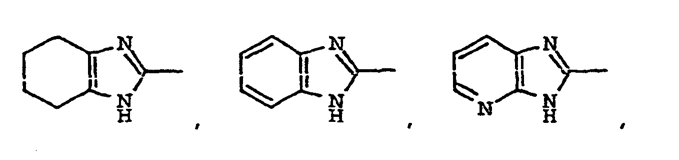 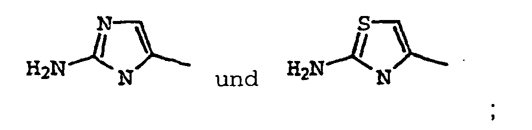
R^{2e} und R^{3e} unabhängig voneinander ausgewählt sind aus:
H, C₁-C₄-Alkoxy, NR^{11e}R^{12e}, Halogen, NO₂, CN, CF₃, C₁-C₆-Alkyl, C₃-C₆-Alkenyl, C₃-C₇-Cycloalkyl, C₃-C₇-Cycloalkyl(C₁-C₄-alkyl), Aryl(C₁-C₆-alkyl), (C₁-C₆-Alkyl)-carbonyl, (C₁-C₆-Alkoxy)carbonyl, Arylcarbonyl und Aryl, substituiert mit 0 - 4 R^{7e},
alternativ, wenn R^{2e} und R^{3e} Substituenten an nachbarständigen Atomen sind, diese mit den Kohlenstoffatomen, an die sie gebunden sind, unter Bildung eines 5- bis 7gliedrigen carbocyclischen oder 5- bis 7gliedrigen heterocyclischen aromatischen oder nicht-aromatischen Ringsystems zusammengenommen werden können, wobei der carbocyclische oder heterocyclische Ring mit 0 - 2 Gruppen, ausgewählt aus C₁-C₄-Alkyl, C₁-C₄-Alkoxy, Halogen, Cyano, Amino, CF₃ und NO₂, substituiert ist;
R^{2ae} ausgewählt ist aus:
H, C₁-C₁₀-Alkyl, C₂-C₆-Alkenyl, C₃-C₁₁-Cycloalkyl, C₃-C₇-Cycloalkyl(C₁-C₄-alkyl), Aryl, Aryl(C₁-C₄-alkyl)-, (C₂-C₇-Alkyl)carbonyl, Arylcarbonyl, (C₂-C₁₀-Alkoxy)carbonyl, C₃-C₇-Cycloalkoxycarbonyl, C₇-C₁₁-Bicycloalkoxycarbonyl, Aryloxycarbonyl, Aryl(C₁-C₁₀-alkoxy)carbonyl, C₁-C₆-Alkylcarbonyloxy(C₁-C₄-alkoxy)carbonyl, Arylcarbonyloxy(C₁-C₄-alkoxy)carbonyl und C₃-C₇-Cycloalkylcarbonyloxy(C₁-C₄-alkoxy)carbonyl;
R^{7e} ausgewählt ist aus:
H, Hydroxy, C₁-C₄-Alkyl, C₁-C₄-Alkoxy, Aryl, Aryl(C₁-C₄-alkyl)-, (C₁-C₄-Alkyl)carbonyl, CO₂R^{18ae}, SO₂R^{11e}, SO₂NR^{10e}R^{11e}, OR^{10e} und N(R^{11e})R^{12e};
U^{e} ausgewählt ist aus:
-(CH₂)ₙ^{e}-, -(CH₂)ₙ^{e}O(CH₂)ₘ^{e}-, -NH(CH₂)ₙ^{e}-, -N(R^{10e})C(=O)-, -NHC(=O)(CH₂)ₙ^{e}-und -C(=O)N(R^{10e})-;
G^{e} N oder CR^{19e} ist;
R^{8e} ausgewählt ist aus:
H, CO₂R^{18be}, C(=O)R^{18be}, CONR^{17e}R^{18be},
C₁-C₁₀-Alkyl, substituiert mit 0-1R^{6e},
C₂-C₁₀-Alkenyl, substituiert mit 0-1R^{6e},
C₂-C₁₀-Alkinyl, substituiert mit 0-1R^{6e},
C₃-C₈-Cycloalkyl, substituiert mit 0-1R^{6e},
C₅-C₆-Cycloalkenyl, substituiert mit 0-1R^{6e},
(C₁-C₁₀-Alkyl)carbonyl,
C₃-C₁₀-Cycloalkyl(C₁-C₄-alkyl)-,
Phenyl, substituiert mit 0-3R^{6e},
Naphthyl, substituiert mit 0-3R^{6e},
einem 5- bis 10gliedrigen heterocyclischen Ring, enthaltend 1-3N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist;
R^{9e} ausgewählt ist aus:
C₁-C₁₀-Alkyl, substituiert mit 0-1R^{6e},
C₁-C₁₀-Alkoxy, substituiert mit 0-2R^{7e},
H, Nitro, -N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO²NR^{10e}R^{11e},NR^{10e}SO₂R^{21e}, Hydroxy,OR^{22e}, -N(R^{10e})R^{11e}, -N(R^{16e})R^{17e}, Aryl(C₀₋C₆-alkyl)carbonyl, Aryl-(C₁-C₆-alkyl), Heteroaryl(C₁-C₆ alkyl), CONR^{18ae}R^{20e}, SO₂R^{18ae} und SO₂NR^{18ae}R^{20e},
mit der Maßgabe, daß jede der obigen Alkyl-, Cycloalkyl-, Aryl- oder Heteroarylgruppen unsubstituiert oder unabhängig mit 1 - 2 R^{7e} substituiert sein kann;
R^{6e} ausgewählt ist aus:
H, C₁-C₁₀-Alkyl, Hydroxy, C₁-C₁₀-Alkoxy, Nitro, C₁-C₁₀-Alkylcarbonyl, -N(R^{11e})R^{12e}, Cyano, Halogen, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚ^{e}R^{11e}, SO₂NR^{10e}R^{11e},
Aryl, substituiert mit 0 - 3 Gruppen, ausgewählt aus Halogen, C₁₋C₆-Alkoxy, C₁₋C₆-Alkyl, CF₃, S(O)ₘ^{e}M^{e} und -NMe₂,
Aryl(C₁-C₄-alkyl)-, wobei das Aryl substituiert ist mit 0 - 3 Gruppen, ausgewählt aus Halogen, C₁-C₆-Alkoxy, C₁-C₆-Alkyl, CF₃, S(O)ₚ^{e}M^{e} und -NMe₂, und
einem 5- bis 10gliedrigen heterocyclischen Ring, enthaltend 1 - 3 N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist;
R^{10e} ausgewählt ist aus:
H, -OH, CF₃, C₃-C₆-Alkenyl, C₃-C₁₁-Cycloalkyl, Aryl, (C₃-C₁₁-Cydoalkyl)methyl, Aryl(C₁-C₄-alkyl) und C₁-C₁₀-Alkyl, substituiert mit 0 - 2 R^{6e};
R^{11e} ausgewählt ist aus:
H, Hydroxy, C₁-C₈-Alkyl, C₃-C₆-Alkenyl, C₃-C₁₁-Cycloalkyl, (C₃-C₁₁-Cycloalkyl)-methyl, C₁-C₆-Alkoxy, Benzyloxy, Aryl, Heteroaryl, Heteroaryl(C₁-C₄-alkyl)-, Aryl-(C₁-C₄-alkyl), Adamantylmethyl und C₁-C₁₀-Alkyl, substituiert mit 0 - 2 R^{4e};
R^{4e} ausgewählt ist aus:
H, C₁-C₆-Alkyl, C₃-C₇-Cycloalkyl, C₃-C₇-Cycloalkyl(C₁-C₄-alkyl)-, Aryl, Heteroaryl, Aryl(C₁-C₆-alkyl)- und Heteroaryl(C₁-C₆-alkyl)-, wobei die Aryl- oder Heteroarylgruppen mit 0 - 2 Substituenten, unabhängig ausgewählt aus der Gruppe, bestehend aus C₁-C₄-Alkyl, C₁-C₄-Alkoxy, F, Cl, Br, CF₃ und NO₂, substituiert sind,
R^{12e} ausgewählt ist aus:
H, C₁-C₆-Alkyl, Triphenylmethyl, Methoxymethyl, Methoxyphenyldiphenylmethyl, Trimethylsilylethoxymethyl, (C₁-C₆-Alkyl)carbonyl, (C₁-C₆-Alkoxy)carbonyl, (C₁-C₆-Alkyl)aminocarbonyl, C₃-C₆-Alkenyl, C₃-C₇-Cycloalkyl, C₃-C₇-Cycloalkyl-(C₁-C₄-alkyl)-, Aryl, Heteroaryl(C₁-C₆-alkyl)carbonyl, Heteroarylcarbonyl, Aryl-(C₁-C₆-alkyl)-, (C₁-C₆-Alkyl)carbonyl, Arylcarbonyl, C₁-C₆-Alkylsulfonyl, Arylsulfonyl, Aryl(C₁-C₆-alkyl)sulfonyl, Heteroarylsulfonyl, Heteroaryl(C₁-C₆-alkyl)sulfonyl, Aryloxycarbonyl und Aryl(C₁-C₆-alkoxy)carbonyl, wobei die Arylgruppen mit 0 - 2 Substituenten, ausgewählt aus der Gruppe, bestehend aus C₁-C₄-Alkyl, C₁-C₄-Alkoxy, Halogen, CF₃ und Nitro, substituiert sind;
R^{16e} ausgewählt ist aus:
-C(=O)OR^{18ae}, -C(=O)R^{18be}, -C(=O)N(R^{18be})₂, -SO₂R^{18ae} und -SO₂N(R^{18be})_{2;}
R^{17e} ausgewählt ist aus:
H, C₁-C₆-Alkyl, C₃-C₇-Cycloalkyl, C₃-C₇-Cycloalkyl(C₁-C₄-alkyl)-, Aryl, Aryl(C₁-C₆-alkyl)- und Heteroaryl(C₁-C₆-alkyl);
R^{18ae} ausgewählt ist aus:
C₁-C₈-Alkyl, gegebenenfalls substituiert mit einer Bindung an Lₙ, C₃-C₁₁-Cycloalkyl, gegebenenfalls substituiert mit einer Bindung an Lₙ, Aryl(C₁-C₆-alkyl)-, gegebenenfalls substituiert mit einer Bindung an Lₙ, Heteroaryl(C₁-C₆-alkyl)-, gegebenenfalls substituiert mit einer Bindung an Lₙ, (C₁-C₆-Alkyl)heteroaryl, gegebenenfalls substituiert mit einer Bindung an Lₙ, Biaryl(C₁-C₆-alkyl), gegebenenfalls substituiert mit einer Bindung an Lₙ, Heteroaryl, gegebenenfalls substituiert mit einer Bindung an Lₙ, Phenyl, substituiert mit 3 - 4 R^{19e} und gegebenenfalls substituiert mit einer Bindung an Lₙ, Naphthyl, substituiert mit 0-4 R^{19e} und gegebenenfalls substituiert mit einer Bindung an Lₙ, und einer Bindung an Lₙ, wobei die Aryl- oder Heteroarylgruppen gegebenenfalls mit 0 - 4 R^{19e} substituiert sind;
R^{18be} H oder R^{18ae} ist,
R^{19e} ausgewählt ist aus:
H, Halogen, CF₃, CO₂H, CN, NO₂, -NR^{11e}R^{12e}, OCF₃, C₁-C₈-Alkyl, C₂-C₆-Alkenyl, C₂-C₆-Alkinyl, C₃-C₁₁-Cycloalkyl, C₃-C₇-Cycloalkyl(C₁-C₄-alkyl)-, Aryl(C₁-C₆-alkyl)-, C₁-C₆-Alkoxy, C₁-C₄-Alkoxycarbonyl, Aryl, Aryl-O-, Aryl-SO₂-, Heteroaryl und Heteroaryl-SO₂-, wobei die Aryl- und Heteroarylgruppen mit 0 - 4 Gruppen, ausgewählt aus Wasserstoff, Halogen, CF₃, C₁-C₃-Alkyl und C₁-C₃-Alkoxy, substituiert sind;
R^{20e} ausgewählt ist aus:
Hydroxy, C₁-C₁₀-Alkyloxy, C₃-C₁₁-Cycloalkyloxy, Aryloxy, Aryl(C₁-C₄-alkyl)oxy, C₂-C₁₀)-Alkylcarbonyloxy(C₁-C₂-alkyl)oxy**-,** C₂-C₁₀-Alkoxycarbonyloxy(C₁-C₂-alkyl)oxy-, C₂-C₁₀-Alkoxycarbonyl(C₁-C₂-alkyl)oxy-, C₃-C₁₀-Cycloalkylcarbonyloxy(C₁-C₂-alkyl)oxy-, C₃-C₁₀-Cycloalkoxycarbonyloxy(C₁-C₂-alkyl)oxy-, C₃-C₁₀-Cycloalkoxycarbonyl(C₁-C₂-alkyl)oxy-, Aryloxycarbonyl(C₁-C₂-alkyl)oxy-, Aryloxycarbonyloxy(C₁-C₂-alkyl)oxy-, Arylcarbonyloxy(C₁-C₂-alkyl)oxy-, C₁-C₅-Alkoxy(C₁-C₅-alkyl)carbonyloxy(C₁-C₂-alkyl)oxy, (5-(C₁ -C₅-Alkyl)-1,3-dioxa-cyclopenten-2-on-yl)methyloxy, (5-Aryl-1,3-dioxa-cyclopenten-2-on-yl)methyloxy und (R^{10e})(R^{11e})N-(C₁-C₁₀-Alkoxy)-;
R^{21e} ausgewählt ist aus:
C₁-C₈-Alkyl, C₂-C₆-Alkenyl, C₃-C₁₁-Cycloalkyl, (C₃-C₁₁-Cycloalkyl)methyl, Aryl, Aryl(C₁-C₄-alkyl)- und C₁-C₁₀-Alkyl, substituiert mit 0 - 2 R^{7e};
R^{22e} ausgewählt ist aus:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae} und -C(=O)NHSO₂NHR^{18be};
m^{e} 0 - 2 ist;
n^{e} 0 - 4 ist und
p^{e} 0 - 2 ist;
mit der folgenden Maßgabe: n^{e} und m^{e} sind so gewählt, daß die Anzahl an Atomen, die R^{1e} und -COR^{20e} in der Formel (IV) verbinden, im Bereich von 8 - 14 liegt;
d ausgewählt ist aus 1, 2, 3, 4 und 5;
d' 1 - 50 ist;
W unabhängig bei jedem Vorkommen aus der Gruppe: O, NH, NHC(=O), C(=O)NH, NR⁸C(=O), C(=O)NR⁸, C(=O), C(=O)O, OC(=O), NHC(=S)NH, NHC(=O)NH, SO₂, (OCH₂CH₂)ₛ, (CH₂CH₂O)ₛ, (OCH₂CH₂CH₂)_{s"}, (CH₂CH₂CH₂O)ₜ und (aa)_{t'} ausgewählt ist;
aa unabhängig bei jedem Vorkommen eine Aminosäure ist;
Z ausgewählt ist aus der Gruppe: Aryl, substituiert mit 0 - 1 R¹⁰, C₃₋₁₀-Cycloalkyl, substituiert mit 0 -1 R¹⁰, und einem 5- bis 10gliedrigen heterocyclischen Ringsystem, enthaltend 1 - 4 Heteroatome, unabhängig ausgewählt aus N, S und O und substituiert mit 0 - 1 R¹⁰;
R⁶, R^{6a}, R⁷, R^{7a} und R⁸ unabhängig bei jedem Vorkommen aus der Gruppe: H, =O, COOH, SO₃H, C₁-C₅-Alkyl, substituiert mit 0 -1 R¹⁰, Aryl, substituiert mit 0 - 1 R¹⁰, Benzyl, substituiert mit 0 - 1 R¹⁰, und C₁-C₅-Alkoxy, substituiert mit 0 - 1 R¹⁰, NHC(=O)R¹¹, C(=O)NHR¹¹, NHC(=O)NHR¹¹, NHR¹¹, R¹¹ und einer Bindung an Cₕ ausgewählt sind;
k 0 oder 1 ist;
s ausgewählt ist aus 0, 1, 2, 3, 4 und 5;
s' ausgewählt ist aus 0, 1, 2, 3, 4 und 5;
s" ausgewählt ist aus 0, 1, 2, 3, 4 und 5;
t ausgewählt ist aus 0, 1, 2, 3, 4 und 5;
A¹, A², A³, A⁴, A⁵, A⁶, A⁷ und A⁸ unabhängig voneinander bei jedem Vorkommen aus der Gruppe: NR¹³, NR¹³R¹⁴, S, SH, S(Pg), OH und einer Bindung an Lₙ ausgewählt sind;
E eine Bindung, CH oder eine Spacer-Gruppe ist, unabhängig bei jedem Vorkommen ausgewählt aus der Gruppe: C₁-C₁₀-Alkyl, substituiert mit 0 - 3 R¹⁷ , Aryl, substituiert mit 0 - 3 R¹⁷, C₃-₁₀-Cycloalkyl, substituiert mit 0 - 3 R¹⁷, und einem 5-bis 10gliedrigen heterocyclischen Ringsystem, enthaltend 1 - 4 Heteroatome, unabhängig ausgewählt aus N, S und O und substituiert mit 0 - 3 R¹⁷;
R¹³ und R¹⁴ jeweils unabhängig voneinander ausgewählt sind aus der Gruppe: einer Bindung an Lₙ, Wasserstoff, C₁-C₁₀-Alkyl, substituiert mit 0 - 3 R¹⁷ , Aryl, substituiert mit 0 - 3 R¹⁷ , einem 5- bis 10gliedrigen heterocyclischen Ringsystem, enthaltend 1 - 4 Heteroatome, unabhängig ausgewählt aus N, S und O und substituiert mit 0 - 3 R¹⁷ und einem Elektron, vorausgesetzt, daß, wenn einer von R¹³ oder R¹⁴ ein Elektron ist, der andere auch ein Elektron ist;
alternativ R¹³ und R¹⁴ unter Bildung von =C(R²⁰)(R²¹) verbunden sind;
R¹⁷ unabhängig bei jedem Vorkommen ausgewählt ist aus der Gruppe: einer Bindung an Lₙ, =O, F, Cl, Br, I, -CF₃, -CN, -CO₂R¹⁸, -C(=O)R¹⁸, -C(=O)N(R¹⁸)₂, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)₂, -N(R¹⁸)₂, -NHC(=S)NHR¹⁸, =NOR¹⁸, -C(=O)NHNR¹⁸R^{18a}, -OCH₂CO₂H und 2-(1-Morpholino)ethoxy;
R¹⁸, R^{18a} und R¹⁹ unabhängig voneinander bei jedem Vorkommen ausgewählt sind aus der Gruppe: einer Bindung an Lₙ, H und C₁-C₆-Alkyl;
R²⁰ und R²¹ unabhängig voneinander ausgewählt sind aus der Gruppe: H, C₁-C₅-Alkyl, -CO₂R²⁵, C₂-C₅-1-Alken, substituiert mit 0 - 3 R²³, C₂-C₅-1-Alkin, substituiert mit 0 - 3 R²³, Aryl, substituiert mit 0 -3 R²³, und einem ungesättigten 5- bis 10gliedrigen heterocyclischen Ringsystem, enthaltend 1 - 4 Heteroatome, unabhängig ausgewählt aus N, S und O und substituiert mit 0 - 3 R²³;
alternativ R²⁰ und R²¹ zusammen mit dem zweiwertigen Kohlenstoffrest, an den sie gebunden sind: 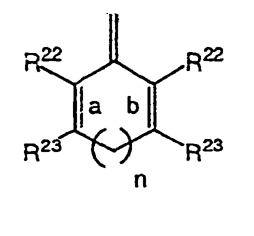 bilden,
R²² und R²³ unabhängig voneinander ausgewählt sind aus der Gruppe: H und R²⁴;
alternativ R²², R²³ zusammen ein kondensiertes aromatisches oder ein 5- bis 10gliedriges heterocyclisches Ringsystem, enthaltend 1 - 4 Heteroatome, unabhängig ausgewählt aus N, S und O, bilden;
R²⁴ unabhängig bei jedem Vorkommen ausgewählt ist aus der Gruppe: -CO₂R²⁵, -C(=O)N(R²⁵)₂, -CH₂OR²⁵, -OC(=O)R²⁵, -OR²⁵, -SO₃H, -N(R²⁵)₂ und -OCH₂CO₂H und
R²⁵ unabhängig bei jedem Vorkommen ausgewählt ist aus der Gruppe: H und C₁-C₃-Alkyl.

6. Kit nach Anspruch 1, wobei:
R^{1e} ausgewählt ist aus: 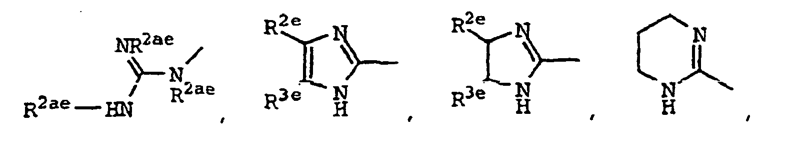 und 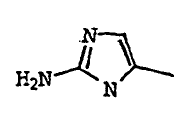
R^{2e} und R^{3e} unabhängig voneinander ausgewählt sind aus:
H, C₁-C₄-Alkoxy, NR^{11e}R^{12e}, Halogen, NO₂, CN, CF₃, C₁-C₆-Alkyl, C₃-C₆-Alkenyl, C₃-C₇-Cycloalkyl, C₃-C₇-Cycloalkyl(C₁-C₄-alkyl), Aryl(C₁-C₆-alkyl)-, (C₁-C₆-Alkyl)-carbonyl, (C₁-C₆-Alkoxy)carbonyl, Arylcarbonyl und Aryl, substituiert mit 0 - 4 R^{7e},
alternativ, wenn R^{2e} und R^{3e} Substituenten an nachbarständigen Atomen sind, diese mit den Kohlenstoffatomen, an die sie gebunden sind, unter Bildung eines 5- bis 7gliedrigen carbocyclischen oder 5- bis 7gliedrigen heterocyclischen aromatischen oder nicht-aromatischen Ringsystems zusammengenommen werden können, wobei der carbocyclische oder heterocyclische Ring mit 0 - 2 Gruppen, ausgewählt aus C₁-C₄-Alkyl, C₁-C₄-Alkoxy, Halogen, Cyano, Amino, CF₃ und NO₂, substituiert ist;
R^{2ae} ausgewählt ist aus:
H, C₁-C₁₀-Alkyl, C₂-C₆-Alkenyl, C₃-C₁₁-Cycloalkyl, C₃-C₇-Cycloalkyl(C₁-C₄-alkyl), Aryl, Aryl(C₁-C₄-alkyl)-, (C₂-C₇-Alkyl)carbonyl, Arylcarbonyl, (C₂-C₁₀-Alkoxy)carbonyl, C₃-C₇-Cycloalkoxycarbonyl, C₇-C₁₁-Bicycloalkoxycarbonyl, Aryloxycarbonyl, Aryl( C₁-C₁₀-alkoxy )carbonyl, C₁-C₆-Alkylcarbonyloxy(C₁₋C₄-alkoxy)carbonyl, Arylcarbonyloxy(C₁-C₄-alkoxy)carbonyl und C₃-C₇Cycloalkylcarbonyloxy(C₁-C₄-alkoxy)carbonyl;
R^{7e} ausgewählt ist aus:
H, Hydroxy, C₁-C₄-Alkyl, C₁-C₄-Alkoxy, Aryl, Aryl(C₁-C₄-alkyl)-, (C₁-C₄-Alkyl)carbonyl, CO₂R^{18ae}, ,SQ₂R^{11e}, SO₂NR^{10e}R^{11e}, OR^{10e} und N(R^{11e})R^{12e};
U^{e} ausgewählt ist aus:
-(CH₂)ₙ^{e}-, -NH(CH₂)ₙ^{e}-, -N(R^{10e})C(=O)- und -NHC(=O)(CH₂)ₙ^{e};
G^{e} N oder CR^{19e} ist;
R^{8e} H ist;
R^{9e} ausgewählt ist aus:
H, Nitro, -N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}_{,} NR^{10e}C(=O)NR^{10e}R^{11e}, NR^{10e}SO₂NR^{10e} R^{11e}, NR^{10e}SO₂R^{21e}, Hydroxy, OR^{22e}, -N(R^{10e})R^{11e}, -N(R^{16e})R^{17e} , Aryl(C₀-C₄-alkyl)carbonyl, Aryl-(C₁-C₄-alkyl), Heteroaryl(C₁-C₄-alkyl), CONR^{18ae}R^{20e}, SO₂R^{18ae} und SO₂NR^{18ae}R^{20e},
mit der Maßgabe, daß jede der obigen Alkyl-, Cycloalkyl-, Aryl- oder Heteroaryl-gruppen unsubstituiert oder unabhängig mit 1 - 2 R^{7e} substituiert sein kann;
R^{10e} ausgewählt ist aus:
H, -OH, CF₃, C₃-C₆-Alkenyl, C₃-C₆-Cycloalkyl, Aryl, (C₃-C₆-Cycloalkyl)methyl, Aryl(C₁-C₄-alkyl) und C₁-C₄-Alkyl, substituiert mit 0 - 2 R^{6e};
R^{6e} ausgewählt ist aus:
H, C₁-C₄-Alkyl, Hydroxy, C₁-C₄-Alkoxy, Nitro, C₁-C₄-Alkylcarbonyl, -N(R^{11e})R^{12e}, Cyano, Halogen, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
Aryl, substituiert mit 0 - 3 Gruppen, ausgewählt aus Halogen, C₁-C₄-Alkoxy, C₁-C₄-Alkyl, CF₃, S(O)ₘ^{e}Me und -NMe₂,
Aryl(C₁-C₄-alkyl)-, wobei das Aryl substituiert ist mit 0 - 3 Gruppen, ausgewählt aus Halogen, C₁-C₄-Alkoxy, C₁-C₄-Alkyl, CF₃, S(O)ₚ^{e}Me und -NMe₂, und
einem 5- bis 10gliedrigen heterocyclischen Ring, enthaltend 1 - 3 N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist;
R^{11e} ausgewählt ist aus:
H, Hydroxy, C₁-C₄-Alkyl, C₃-C₆-Alkenyl, C₃-C₆-Cycloalkyl, (C₃-C₆-Cycloalkyl)-methyl, C₁-C₄-Alkoxy, Benzyloxy, Aryl, Heteroaryl, Heteroaryl(C₁-C₄-alkyl)-, Aryl(C₁-C₄-alkyl), Adamantylmethyl und C₁-C₄-Alkyl, substituiert mit 0 - 2 R^{4e};
R^{4e} ausgewählt ist aus:
H, C₁-C₄-Alkyl, C₃-C₇-Cycloalkyl, C₃-C₇-Cycloalkyl(C₁-C₄-alkyl)-, Aryl, Heteroaryl, Aryl(C₁-C₄-alkyl)- und Heteroaryl(C₁-C₄-alkyl)-, wobei die Aryl- oder Heteroaryl-gruppen mit 0 - 2 Substituenten, unabhängig ausgewählt aus der Gruppe, bestehend aus C₁-C₄-Alkyl, C₁-C₄-Alkoxy, F, Cl, Br, CF₃ und NO₂, substituiert sind,
R^{12e} ausgewählt ist aus:
H, C₁-C₄-Alkyl, (C₁-C₄-Alkyl)carbonyl, (C₁-C₄-Alkoxy)carbonyl, Phenyl(C₁-C₄-alkyl)-, Phenylsulfonyl, Phenyloxycarbonyl und Phenyl(C₁-C₄-alkoxy)carbonyl, wobei die Phenylgruppen mit 0 - 2 Substituenten, ausgewählt aus der Gruppe, bestehend aus C₁-C₄-Alkyl, C₁-C₄-Alkoxy, Halogen, CF₃ und Nitro, substituiert sind;
R^{16e} ausgewählt ist aus:
-C(=O)OR^{18ae}, -C(=O)R^{18be}, -C(=O)N(R^{18e})2, -SO₂R^{18ae} und -SO₂N(R^{18be})₂;
R^{17e} ausgewählt ist aus:
H, C₁-C₄-Alkyl, C₃-C₆-Cycloalkyl, C₃-C₆-Cycloalkyl(C₁-C₄-alkyl)-, Aryl, Aryl(C₁-C₆-alkyl)- und Heteroaryl(C₁-C₆-alkyl);
R^{18ae} ausgewählt ist aus:
C₁-C₈-Alkyl, gegebenenfalls substituiert mit einer Bindung an Lₙ, C₃-C₁₁-Cycloalkyl, gegebenenfalls substituiert mit einer Bindung an Lₙ, Aryl(C₁-C₆-alkyl, gegebenenfalls substituiert mit einer Bindung an Lₙ, Heteroaryl(C₁-C₆-alkyl)-, gegebenenfalls substituiert mit einer Bindung an Lₙ, (C₁-C₆-Alkyl)heteroaryl, gegebenenfalls substituiert mit einer Bindung an Lₙ, Biaryl(C₁-C₆-alkyl), gegebenenfalls substituiert mit einer Bindung an Lₙ, Heteroaryl, gegebenenfalls substituiert mit einer Bindung an Lₙ, Phenyl, substituiert mit 3 - 4 R^{19e} und gegebenenfalls substituiert mit einer Bindung an Lₙ, Naphthyl, substituiert mit 0-4 R^{19e} und gegebenenfalls substituiert mit einer Bindung an Lₙ, und einer Bindung an Lₙ, wobei die Aryl- oder Heteroarylgruppen gegebenenfalls mit 0 - 4 R^{19e} substituiert sind;
R^{18be} H oder R^{18ae} ist,
R^{19e} ausgewählt ist aus:
H, Halogen, CF₃, CO₂H, CN, NO₂, -NR^{11e}R^{12e}, OCF₃, C₁-C₆-Alkyl, C₂-C₆-Alkenyl, C₂-C₆-Alkinyl, C₃-C₆-Cycloalkyl, C₃-C₆-Cycloalkyl(C₁-C₄-alkyl)-, Aryl(C₁-C₄-alkyl)-, C₁-C₆-Alkoxy, C₁-C₄-Alkoxycarbonyl, Aryl, Aryl-O-, Aryl-SO₂-, Heteroaryl und Heteroaryl-SO₂-, wobei die Aryl- und Heteroarylgruppen mit 0 - 4 Gruppen, ausgewählt aus Wasserstoff, Halogen, CF₃, C₁-C₃-Alkyl und C₁-C₃-Alkoxy, substituiert sind;
R^{20e} ausgewählt ist aus:
Hydroxy, C₁-C₆-Alkyloxy, C₃-C₆-Cycloalkyloxy, Aryloxy, Aryl(C₁-C₄-alkyl)oxy, C₂-C₁₀-Alkylcarbonyloxy(C₁-C₂-alkyl)oxy-, C₂-C₁₀-Alkoxycarbonyloxy(C₁-C₂-alkyl)oxy-, C₂-C₁₀-Alkoxycarbonyl(C₁-C₂-alkyl)oxy-, C₃-C₁₀-Cycloalkylcarbonyloxy-(C₁-C₂-alkyl)oxy-, C₃-C₁₀-Cycloalkoxycarbonyloxy(C₁-C₂-alkyl)oxy-, C₃-C₁₀-Cyclo-alkoxycarbonyl(C₁-C₂-alkyl)oxy-, Aryloxycarbonyl(C₁-C₂-alkyl)oxy-, Aryloxycarbonyloxy(C₁-C₂-alkyl)oxy-, Arylcarbonyloxy( C₁-C₂-alkyl)oxy-, C₁-C₅-Alkoxy( C₁-C₅-alkyl)carbonyloxy(C₁-C₂-alkyl)oxy, (5-(C₁-C₅-Alkyl)-1,3-dioxa-cyclopenten-2-on-yl)methyloxy, (5-Aryl-1,3-dioxa-cyclopenten-2-on-yl)methyloxy und (R^{10e})(R^{11e})N-(C₁-C₁₀-Alkoxy)-;
R^{21e} ausgewählt ist aus:
C₁-C₄-Alkyl, C₂-C₆-Alkenyl, C₃-C₆-Cycloalkyl, (C₃-C₆-Cycloalkyl)methyl, Aryl, Aryl(C₁-C₄-alkyl)- und C₁-C₁₀-Alkyl, substituiert mit 0 - 2 R^{7e};
R^{22e} ausgewählt ist aus:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae} und -C(=O)NHSO₂NHR^{18be};
m^{e} 0 - 2 ist;
n^{e} 0 - 4 ist;
p^{e} 0 - 2 ist;
Cₕ 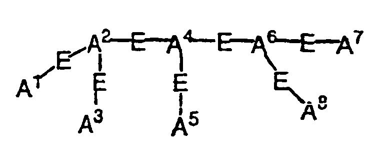 ist;
A¹ ausgewählt ist aus der Gruppe: OH und einer Bindung an Lₙ;
A², A⁴ und A⁶ jeweils N sind;
A³, A⁵ und A⁸ jeweils OH sind;
A⁷ eine Bindung an Lₙ oder eine NH-Bindung an Lₙ ist;
E ein C₂-Alkyl, substituiert mit 0 - 1 R¹⁷, ist;
R¹⁷ =O ist;
alternativ Cₕ 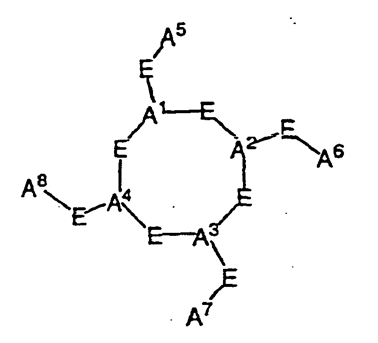 ist;
A¹ ausgewählt ist aus der Gruppe: OH und einer Bindung an Lₙ;
A², A³ und A⁴ jeweils N sind;
A⁵, A⁶ und A⁸ jeweils OH sind;
A⁷ eine Bindung an Lₙ ist;
E ein C₂Alkyl, substituiert mit 0 - 1 R¹⁷, ist;
R¹⁷ =O ist;
alternativ Cₕ 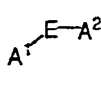 ist;
A¹ NH₂ oder N=C(R²⁰)(R²¹) ist;
E eine Bindung ist;
A² NHR¹³ ist;
R¹³ ein Heterocyclus, substituiert mit R¹⁷, ist, wobei der Heterocyclus aus Pyridin und Pyrimidin ausgewählt ist;
R¹⁷ aus einer Bindung an Lₙ, C(=O)NHR¹⁸ und C(=O)R¹⁸ ausgewählt ist;
R¹⁸ eine Bindung an Lₙ ist;
R²⁴ ausgewählt ist aus der Gruppe: -CO₂R²⁵, -OR²⁵, -SO₃H und -N(R²⁵)₂ und
R²⁵ unabhängig bei jedem Vorkommen ausgewählt ist aus der Gruppe: Wasserstoff und Methyl.

7. Kit nach Anspruch 1, wobei: .
Q aus der Gruppe ausgewählt ist:
3-[7-[(Imidazolin-2-ylamino)methyl]-1-methyl-6,8-difluorchinolin-4-on-3-ylcarbonylamino]-2-(3,5-dimethylisoxazol-4-.ylsulfonylamino)propionsäure,
3-[7-[(Imidazolin-2-ylamino)methyl]-1-methyl-6,8-difluorchinolin-4-on-3-ylcarbonylamino]-2-(benzyloxycarbonylamino)propionsäure,
3-[7-[(Imidazolin-2-ylamino)methyl]-1-methyl-6,8-difluorchinolin-4-on-3-ylcarbonylamino]-2-(n-butyloxycarbonylamino)propionsäure,
3-[7-[(Imidazolin-2-ylamino)methyl]-1-methyl-6,8-difluorchinolin-4-on-3-ylcarbonylamino]-2-(n-butylsulfonylamino)propionsäure,
3-[7-[(Tetrahydropyrimid-2-ylamino)methyl]-1-methyl-6,8-difluorchinolin-4-on-3-ylcarbonylamino]-2-(benzyloxycarbonylamino)propionsäure,
3-[7-[(Tetrahydropyrimid-2-ylamino)methyl]-1-methyl-6,8-difluorchinolin-4-on-3-ylcarbonylamino]-2-(n-butyloxycarbonylamino)propionsäure,
3-[7-[(Tetrahydropyrimid-2-ylamino)methyl]-1-methyl-6,8-difluorchinolin-4-on-3-ylcarbonylamino]-2-(phenylsulfonylamino)propionsäure,
3-[7-[(Tetrahydropyrimid-2-ylamino)methyl]-1-methyl-6,8-difluorchinolin-4-on-3-ylcarbonylamino]-2-(n-butyl-sulfonyl)aminopropionsäure,
3-[7-[(2-Aminothiazol-4-yl)methyl]-1-methyl-6,8-difluorchinolin-4-on-3-ylcarbonylamino]-2-(benzyloxycarbonylamino)propionsäure,
3-[7-[(Imidazolin-2-ylamino)methyl]-1-methyl-6,8-difluorchinolin-4-on-3-ylcarbonylamino]-2-((2,4,6-trimethylphenyl)sulfonylamino)propionsäure,
3-[7-[(Tetrahydropyrimid-2-ylamino)methyl]-1-methyl-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-methyl-6,8-difluorchinolin-4-on-3-ylcarbonylamino]-2-(3,5-dimethylisoxazol-4-ylsulfonylamino)propionsäure,
3-[7-[(Imidazol-2-ylamino)methyl]-1-methyl-6,8-difluorchinolin-4-on-3-ylcarbonylamino]-2-(benzyloxycarbonylamino)propionsäure,
3-[7-[(Imidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-methyl-6,8-difluorchinolin-4-on-3-ylcarbonylamino]-2-((4-biphenyl)sulfonylamino)propionsäure,
3-[7-[(Imidazol-2-ylamino)methyl]-1-methyl-6,8-difluorchinolin-4-on-3-ylcarbonylamino]-2-(1-naphthylsulfonylamino)propionsäure,
3-[7-[(Benzimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(4-Methylimidazol-2-ylamino)methyl]-1-methyl-6,8=difluorchinolin-4-on-3-ylcarbonylamino]-2-((2,4,6-trimethylphenyl)sulfonylamino)propionsäure,
3-[7-[(4,5-Dimethylimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(4,5,6,7-Tetrahydrobenzimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(Pyridin-2-ylamino)methyl]-1-methyl-6,8-difluorchinolin-4-on-3-ylcarbonylamino]-2-((2,4,6-trimethylphenyl)sulfonylamino)propionsäure,
3-[7-(2-Aminopyridin-6-yl)-1-methyl-6,8-difluorchinolin-4-on-3-ylcarbonylamino]-2-((2,4,6-trimethylphenyl)sulfonylamino)propionsäure,
3-[7-[(7-Azabenzimidazol-2-yl)methyl]-1-methyl-6,8-difluorchinolin-4-on-3-ylcarbonylamino]-2-((2,4,6 trimethylphenyl)sulfonylamino)propionsäure,
3-[7-[(Benzimidazol-2-ylamino)methyl]-1-(2-phenylethyl)-6,8-difluorchinolin-4-on-3-ylcarbonylamino]propionsäure,
3-[7-[(Pyridin-2-ylamino)methyl]-1-(2-phenylethyl)-6,8-difluorchinolin-4-on-3-ylcarbonylamino]propionsäure,
3-[7-[(Imidazolin-2-ylamino)methyl]-1-(2-phenylethyl)-6,8-difluorchinolin-4-on-3-ylcarbonylamino]propionsäure,
3-[7-[(Imidazol-2-ylamino)methyl]-1-(2-phenylethyl-6,8-difluorchinolin-4-on-3-ylcarbonylamino]propionsäure,
3-[7-[(Imidazolin-2-ylamino)methyl]-1-(2-phenylethyl)-6,8-difluorchinolin-4-on-3-ylcarbonylamino]-2-(benzyloxycarbonylamino)propionsäure,
3-[7-[(Imidazolin-2-ylamino)methyl]-1-(2-phenylethyl)-6,8-difluorchinolin-4-on-3-ylcarbonylamino]-2-(n-butyloxycarbonylamino)propionsäure,
3-[7-[(Imidazolin-2-ylamino)methyl]-1-(2-phenylethyl)-6,8-difluorchinolin-4-on-3-ylcarbonylamino]-2-(phenylsulfonylamino)propionsäure,
3-[7-[(Imidazolin-2-ylamino)methyl]-1-(2-phenylethyl)-6,8-difluorchinolin-4-on-3-ylcarbonylamino]-2-(n-butylsulfonylamino)propionsäure,
3-[7-[(Tetrahydropyrimid-2-ylamino)methyl]-1-(2-phenylethyl)-6,8-difluorchinolin-4-on-3-ylcarbonylamino]-2-(benzyloxycarbonylamino)propionsäure,
3-[7-[(Tetrahydropyrimid-2-ylamino)methyl]-1-(2-phenylethyl)-6,8-difluorchinolin-4-on-3-ylcarbonylamino]-2-(n-butyloxycarbonylamino)propionsäure,
3-[7-[(Tetrahydropyrimid-2-ylamino)methyl]-1-(2-phenylethyl)-6,8-difluorchinolin-4-on-3-ylcarbonylamino]-2-(phenylsulfonylamino)propionsäure,
3-[7-[(Tetrahydropyrimid-2-ylamino)methyl]-1-(2-phenylethyl)-6,8-difluorchinolin-4-on-3-ylcarbonylamino]-2-(n-butylsulfonyl)aminopropionsäure,
3-[7-[(2-Aminothiazol-4-yl)methyl]-1-(2-phenylethyl)-6,8-difluorchinolin-4-on-3-ylcarbonylamino]-2-(phenylsulfonylamino)propionsäure,
3-[7-[(2-Aminothiazol-4-yl)methyl]-1-(2-phenylethyl)-6,8-difluorchinolin-4-on-3-ylcarbonylamino]-2-(benzyloxycarbonylamino)propionsäure,
3-[7-[(Imidazolin-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(Tetrahydropyrimid-2-ylamino)methyl]-1-(2-phenylethyl)-6,8-difluorchinolin-4.-on-3-ylcarbonylamino]-2-((2,4,6-trimethylphenyl)sulfonylamino)propionsäure,
3-[7-[(Imidazol-2-ylamino)methyl]-1-(2-phenylethyl)-6,8-difluorchinolin-4-on-3-ylcarbonylamino]-2-(benzyloxycarbonylamino)propionsäure,
3-[7-[(Imidazol-2-ylamino)methyl]-1-(2-phenylethyl)-6,8-difluorchinolin-4-on-3-ylcarbonylamino]-2-(phenylsulfonylamino)propionsäure,
3-[7-[(Imidazol-2-ylamino)methyl]-1-(2-phenylethyl)-6,8-difluorchinolin-4-on-3-ylcarbonylamino]-2-((2,6-dichlorphenyl)sulfonylamino)propionsäure,
3-[7-[(Imidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-(2-phenylethyl)-6,8-difluorchinolin-4-on-3-yl-carbonylamin-2-((4-biphenyl)sulfonylamino)propionsäure,
3-[7-[(Benzimidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(4-Methylimidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(4,5-Dimethylimidazol-2-ylamino)methyl]-1 -(2-phenylethyl)-6,8-difluorchinolin-4-on-3-ylcarbonylamino]-2-((2,4,6-trimethylphenyl)sulfonylamino)propionsäure,
3-[7-[(4,5,6,7-Tetrahydrobenzimidazol-2-ylamino)methyl]-1-(2-phenylethyl)-6,8-di-fluorchinolin-4-on-3-ylcarbonylamino]-2-((2,4,6-trimethylphenyl)sulfonylamino)-propionsäure,
3-[7-[(Pyridin-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(2-Aminopyrid in-6-yl)-1-(2-phenylethyl)-6,8-difluorchinolin-4-on-3-ylcarbonylamino]-2-((2,4,6-trimethylphenyl)sulfonylamino)propionsäure und
3-[7-[(7-Azabenzimidazol-2-yl)methyl]-1-(2-phenylethyl)-6,8-difluorchinolin-4-on-3-ylcarbonylamino]-2-((2,4,6-trimethylphenyl)sulfonylamino)propionsäure.

8. Kit nach Anspruch 1, wobei die Verbindung aus der Gruppe ausgewählt ist:
2-(((4-(4-(((3-(2-(2-(3-((6-((1-Aza-2-(2-sulfophenyl)vinyl)amino)(3-pyridyl))carbonylamino)propoxy)ethoxy)ethoxy)propyl)amino)sulfonyl)phenyl)phenyl)sulfonyl)-amino)-3-((7-((imidazol-2-ylamino)methyl)-1-methyl-4-oxo(3-hydrochinolyl))carbonylamino)propionsäure;
3-((7-((Imidazol-2-ylamino)methyl)-1-methyl-4-oxo(3-hydrochinolyl))carbonylamino)-2-(((4-(4-(((3-(2-(2-(3-(2-(1,4,7,10-tetraaza-4,7,10-tris(carboxylmethyl)cyclododecyl)acetylamino)propoxy)ethoxy)ethoxy)propyl)amino)sulfonyl)phenyl)phenyl)-sulfonyl)amino)propionsäure;
2-(((4-(3-(N-(3-(2-(2-(3-((6-((1-Aza-2-(2-sulfophenyl)vinyl)amino)(3-pyridyl))carbonylamino)propoxy)ethoxy)ethoxy)propyl)carbamoyl)propoxy)-2,6-dimethylphenyl)-sulfonyl)amino)-3-((7-((imidazol-2-ylamino)methyl)-1-methyl-4-oxo(3-hydrochinolyl))carbonylamino)propionsäure;
3-((1-(3-((6-((1-Aza-2-(2-sulfophenyl)vinyl)amino)(3-pyridyl))carbonylamino)propyl)-7-((imidazol-2-ylamino)methyl)-4-oxo(3-hydrochinolyl))carbonylamino)-2-(((2,4,6-trimethylphenyl)sulfonyl)amino)propionsäure;
3-((1-(3-((6-((1-Aza-2-(2-sulfophenyl)vinyl)amino)(3-pyridyl))carbonylamino)propyl)-7-(((1-hydroxyimidazol-2-yl)amino)methyl)-4-oxo(3-hydrochinolyl))carbonylamino)-2-(((2,4,6-trimethylphenyl)sulfonyl)amino)propionsäure;
3-((1-(3-(3-(N-(3-(2-(2-(3-((6-((1-Aza-2-(2-sulfophenyl)vinyl)amino)(3-pyridyl))carbonylamino)propoxy)ethoxy)ethoxy)propyl)carbamoy))propanoy)amino)propy))-7-((imidazol-2-ylamino)methyl)-4-oxo(3-hydrochinolyl))carbonylamino)-2-(((2,4,6-trimethylphenyl)sulfonyl)amino)propionsäure;
2-(2-Aza-2-(5-(N-(1,3-bis(3-(2-(2-(3-(3-(N-(3-(3-(N-(3-carboxy-2-(((2,4,6-trimethylphenyl)sulfonyl)amino)ethyl)carbamoyl)-7-((imidazol-2-ylamino)methyl)4-oxohydrochinolyl)propyl)carbamoyl)propanoylamino)propoxy)ethoxy)ethoxy)-propyl)-carbamoyl)(2-pyridyl))amino)vinyl)benzolsulfonsäure; 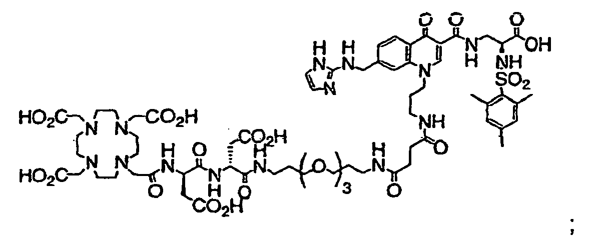 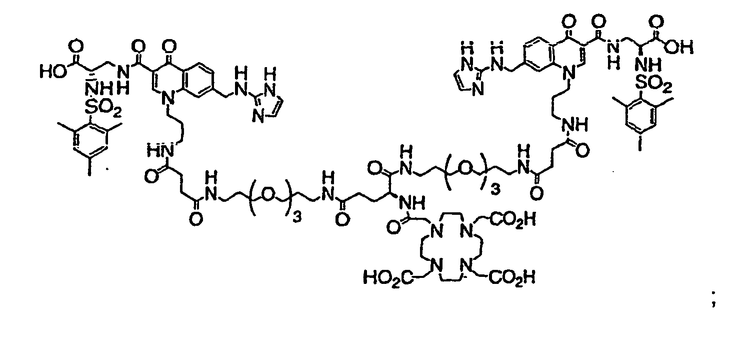 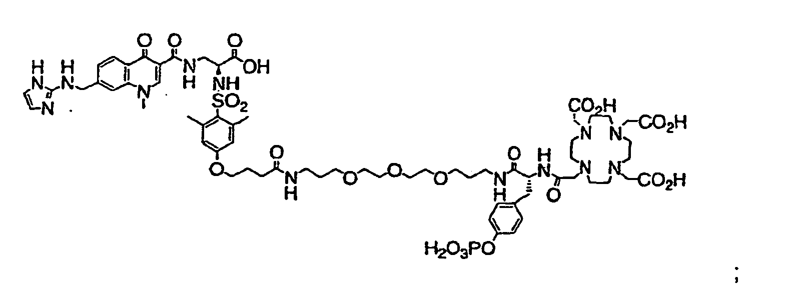 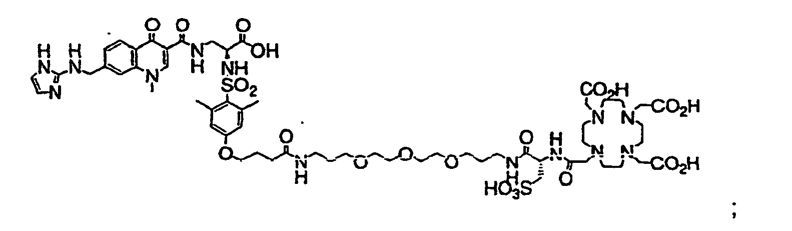 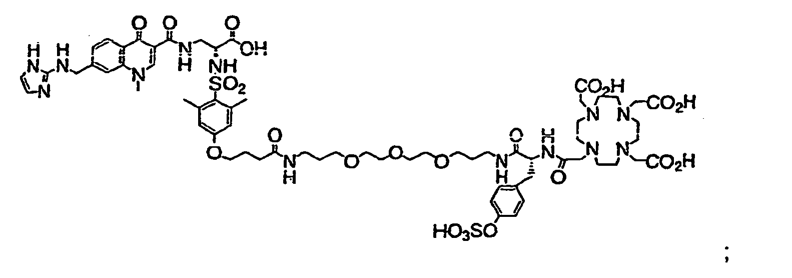 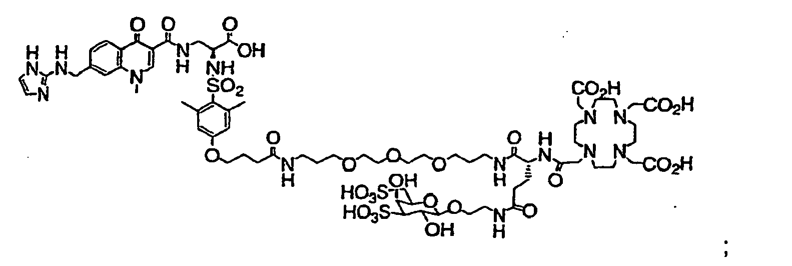 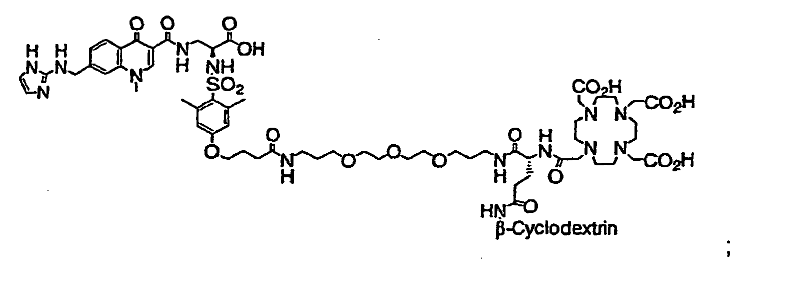 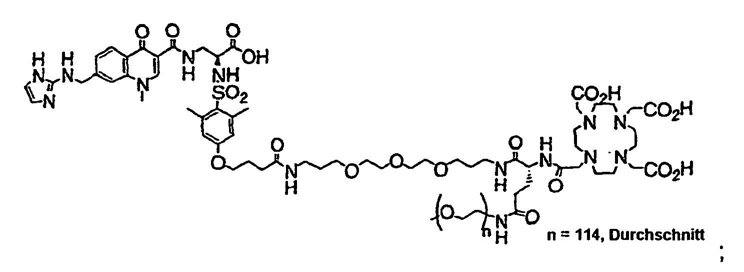
2-(((4-(3-(N-(3-(2-(2-(3-(2-(1,4,7,10-Tetraaza-4,7,10-tris(carboxymethyl)cyclododecylacetylamino)-6-aminohexanoylamino)propoxy)ethoxy)ethoxy)propyl)carbamoyl)-propoxy)-2,6-dimethylphenyl)sulfonyl)amino)-3-((7-((imidazol-2-ylamino)methyl)-1-methyl-4-oxo(3-hydrochinolyl))carbonylamino)propionsäure;
2-(((4-(3-(N-(3-(2-(2-(3-(2-(1,4,7,10-Tetraaza-4,7,10-tris(carboxymethyl)cyclododecylacetylamino)-6-(2-(bis(phosphonomethyl)amino)acetylamino)hexanoylamino)-propoxy)ethoxy)ethoxy)propyl)carbamoyl)propoxy)-2,6-dimethylphenyl)sulfonyl)-amino)-3-((7-((imidazol-2-ylamino)methyl)-1-methyl-4-oxo(3-hydrochinolyl))carbonylamino)propionsäurekonjugat und
2-(((4-(3-(N-(3-(2-(2-(3-(2-(2-((2-((2-(Bis(carboxymethyl)amino)ethyl)(carboxymethyl)-amino)ethyl)(carboxymethyl)amino)acetylamino)-3-sulfopropyl)propoxy)ethoxy)-ethoxy)propyl)carbamoyl)propoxy)-2,6-dimethylphenyl)sulfonyl)amino)-3-((7-((imidazol-2-ylamino)methyl)-1-methyl-4-oxo(3-hydrochinolyl))carbonylamino)propionsäure; 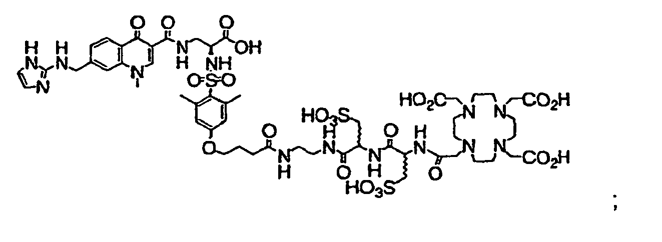 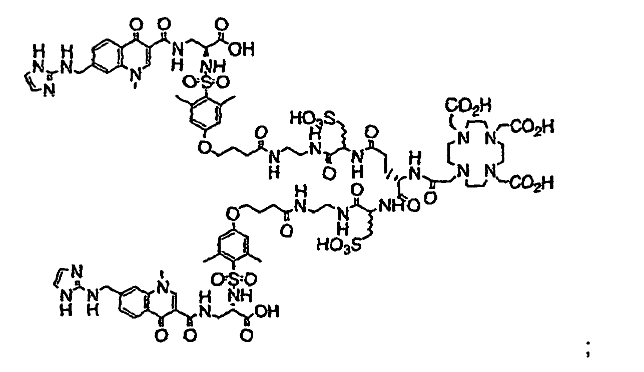 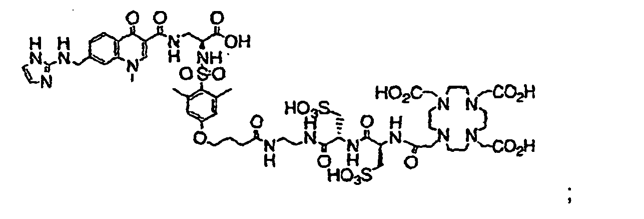 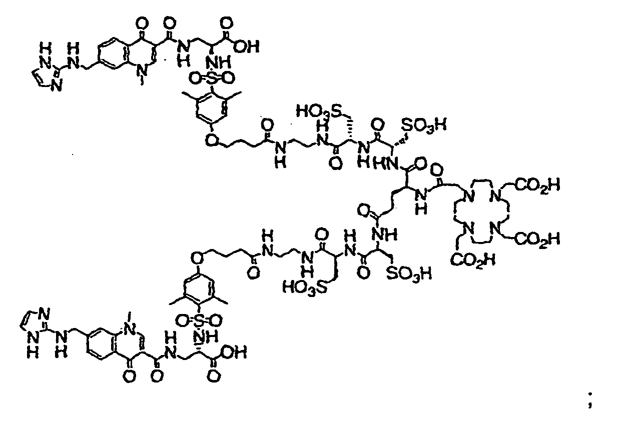 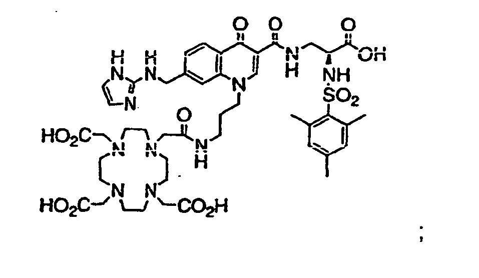 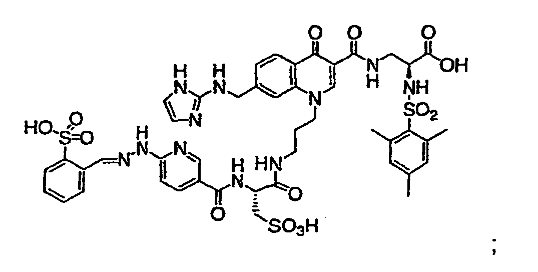 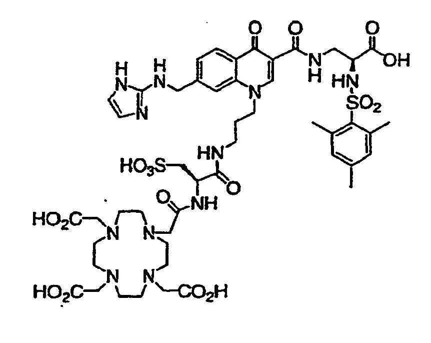 und 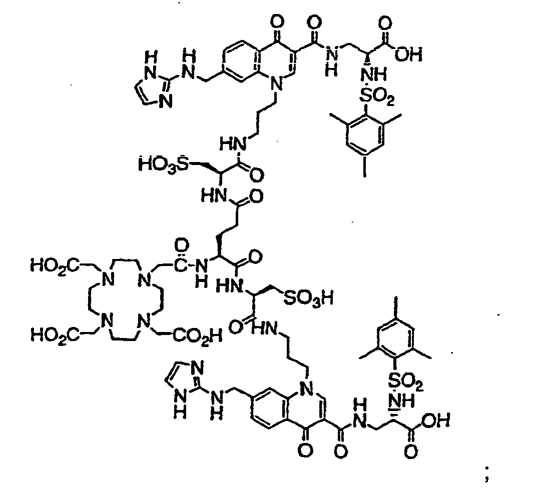
oder eine pharmazeutisch akzeptable Salzform davon.

9. Kit nach Anspruch 1, wobei das Kit ferner einen oder mehrere zusätzliche Liganden und ein Reduktionsmittel umfaßt.

10. Kit nach Anspruch 9, wobei die zusätzlichen Liganden Tricin und TPPTS sind.

11. Kit nach Anspruch 9, wobei das Reduktionsmittel Zinn(II) ist.

12. Therapeutische radiopharmazeutische Zusammensetzung, umfassend mindestens ein Mittel, ausgewählt aus der Gruppe, bestehend aus einem Antikrebsmittel und einem Radiosensibilisator, oder ein pharmazeutisch akzeptables Salz davon und ein Radiopharmazeutikum, umfassend:
a) ein Metall;
b) einen Chelatbildner, der das Metall chelatisieren kann, und
c) eine Targetierungskomponente;
wobei die Targetierungskomponente über 0 - Verknüpfungsgruppe(n) an den Chelatbildner gebunden ist, und die Targetierungskomponente Chinolon-Nicht-Peptid ist, das an einen Rezeptor bindet, der während der Angiogenese hochreguliert wird.

13. Therapeutische radiopharmazeutische Zusammensetzung nach Anspruch 12, wobei das Antikrebsmittel ausgewählt ist aus der Gruppe, bestehend aus Mitomycin, Tretinoin, Ribomustin, Gemcitabin, Vincristin, Etoposid, Cladribin, Nitobronitol, Methotrexat, Doxorubicin, Carboquon, Pentostatin, Nitracrin, Zinostatin, Cetrorelix, Letrozol, Raltitrexed, Daunorubicin, Fadrozol, Fotemustin, Thymalfasin, Sobuzoxan, Nedaplatin, Cytarabin, Bicalutamid, Vinorelbin, Vesnarinon, Aminoglutethimid, Amsacrin, Proglumid, Elliptiniumacetat, Ketanserin, Doxifluridin, Etretinat, Isotretinoin, Streptozocin, Nimustin, Vindesin, Flutamid, Drogenil, Butocin, Carmofur, Razoxan, Sizofilan, Carboplatin, Mitolactol, Tegafur, Ifosfamid, Prednimustin, Picibanil, Levamisol, Teniposid, Improsulfan, Enocitabin, Lisurid, Oxymetholon, Tamoxifen, Progesteron, Mepitiostan, Epitiostanol, Formestan, Interferon-alpha, Interferon-2-alpha, Interferon-beta, Interferon-gamma, Colony-stimulating-Faktor 1, Colony-stimulating-Faktor 2, Denileukin diftitox, Interleukin-2 und Freisetzungsfaktor für Luteinisierungshormon.

14. Therapeutische radiopharmazeutische Zusammensetzung nach Anspruch 12, wobei der Radiosensibilisator ausgewählt ist aus der Gruppe, bestehend aus 2-(3-Nitro-1,2,4-triazol-1-yl)-N-(2-methoxyethyl)acetamid, N-(3-Nitro-4-chinolinyl)-4-morpholincarboxamidin, 3-Amino-1,2,4-benzotriazin-1,4-dioxid, N-(2-Hydroxyethyl)-2-nitroimidazol-1-acetamid, 1-(2-Nitroimidazol-1-yl)-3-(1-piperidinyl)-2-propanol und 1-(2-Nitro-1-imidazolyl)-3-(1-aziridino)-2-propanol.

15. Therapeutische radiopharmazeutische Zusammensetzung nach Anspruch 12, wobei das Metall ausgewählt ist aus the Gruppe: ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd,¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag und ¹⁹²Ir und die Verknüpfungsgruppe zwischen der Nicht-Peptid-Targetierungskomponente und dem Chelatbildner vorliegt.

16. Therapeutische radiopharmazeutische Zusammensetzung nach Anspruch 15, wobei die Targetierungskomponente ein Chinolon-Nicht-Peptid und der Rezeptor αᵥβ₃ oder αᵥβ₅ ist.

17. Therapeutische radiopharmazeutische Zusammensetzung nach Anspruch 12,
wobei das Radiopharmazeutikum
a) ein Metall, ausgewählt ist aus der Gruppe: ³³p, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag und ¹⁹²Ir und
b) eine Verbindung der Formel (I):
(Q)_{d}-Lₙ-Cₕ oder (Q)_{d}-Lₙ-(Cₕ)_{d}' (I),
worin Q eine Verbindung der Formel (II): 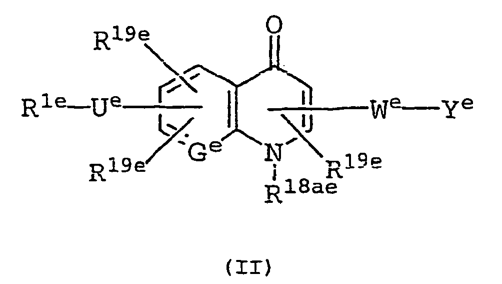 ist,
einschließlich stereoisomere Formen davon oder Gemische stereoisomerer Formen davon oder pharmazeutisch akzeptable Salz- oder Prodrug-Formen davon, worin:
R^{1e} ausgewählt ist aus: 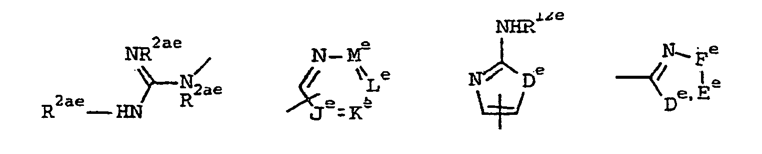 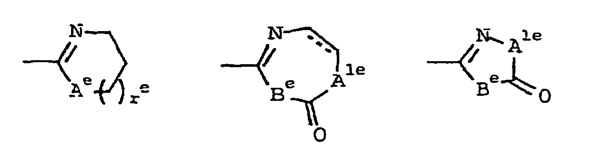 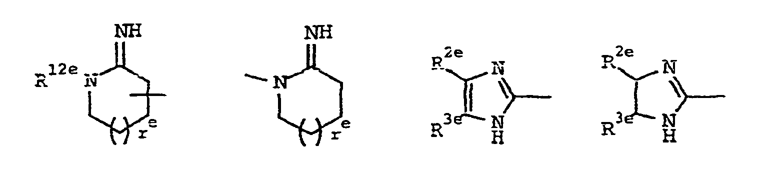 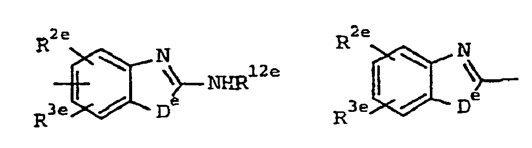 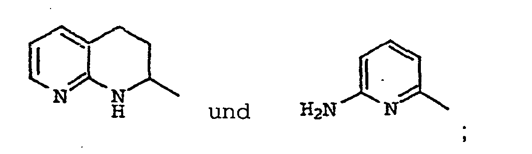
A^{e} -CH₂- oder -N(R^{10e})- ist;
A^{1e} und B^{e} unabhängig voneinander-CH₂- oder -N(R^{10e})- sind;
D^{e} -N(R^{10e})- oder -S- ist;
E^{e}-F^{e} -C(R^{2e})=C(R^{3e})- oder -C(R^{2e})₂C(R^{3e})₂- ist;
J^{e} -C(R^{2e})- oder -N- ist;
K^{e}, L^{e} und M^{e} unabhängig voneinander -C(R^{2e})- oder -C(R^{3e})- sind;
R^{2e} und R^{3e} unabhängig voneinander ausgewählt sind aus:
H, C₁-C₄-Alkoxy, NR^{11e}R^{12e}, Halogen, NO₂, CN, CF₃, C₁-C₆-Alkyl, C₃-C₆-Alkenyl, C₃-C₇-Cycloalkyl, C₃-C₇-Cycloalkyl(C₁-C₄-alkyl), Aryl(C₁-C₆-alkyl)-, (C₁-C₆-Alkyl)-carbonyl, (C₁-C₆-Alkoxy)carbonyl, Arylcarbonyl und Aryl, substituiert mit 0-4R^{7e},
alternativ, wenn R^{2e} und R^{3e} Substituenten an nachbarständigen Atomen sind, diese mit den Kohlenstoffatomen, an die sie gebunden sind, unter Bildung eines 5- bis 7gliedrigen carbocyclischen oder 5- bis 7gliedrigen heterocyclischen aromatischen oder nicht-aromatischen Ringsystems zusammengenommen werden können, wobei der carbocyclische oder heterocyclische Ring mit 0 - 2 Gruppen, ausgewählt aus C₁-C₄-Alkyl, C₁-C₄-Alkoxy, Halogen, Cyano, Amino, CF₃ und NO₂, substituiert ist;
R^{2ae} ausgewählt ist aus:
H, C₁-C₁₀-Alkyl, C₂-C₆-Alkenyl, C₃-C₁₁-Cycloalkyl, C₃-C₇-Cycloalkyl(C₁-C₄-alkyl), Aryl, Aryl(C₁-C₄-alkyl)-, (C₂-C₇-Alkyl)carbonyl, Arylcarbonyl, (C₂-C₁₀-Alkoxy)carbonyl, C₃-C₇-Cycloalkoxycarbonyl, C₇-C₁₁-Bicycloalkoxycarbonyl, Aryloxycarbonyl, Aryl(C₁-C₁₀-alkoxy)carbonyl, C₁-C₆-Alkylcarbonyloxy(C₁-C₄-alkoxy)carbonyl, Arylcarbonyloxy(C₁-C₄-alkoxy)carbonyl und C₃-C₇-Cycloalkylcarbonyloxy(C₁-C₄-alkoxy)carbonyl;
R^{7e} ausgewählt ist aus:
H, Hydroxy, C₁-C₄-Alkyl, C₁-C₄-Alkoxy, Aryl, Aryl(C₁-C₄-alkyl)-, (C₁-C₄-Alkyl)-carbonyl, CO₂R^{18ae}, SO₂R^{11e}, SO₂NR^{10e}R^{11e}, OR^{10e} und N(R^{11e})R^{12e};
U^{e} ausgewählt ist aus:
-(CH₂)ₙ^{e}-, -(CH₂)ₙ^{e}O(CH₂)ₘ^{e}-, -(CH₂)ₙ^{e}N(R¹²)(CH₂)ₘ^{e}-, -NH(CH₂)ₙ^{e}-, -(CH₂)ₙ^{e}C(=O)(CH₂)ₘ^{e}-, -(CH₂)ₙ^{e}S(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})- und -N(R^{10e})S(O)ₚ^{e}-;
G^{e} N oder CR^{19e} ist;
W^{e} -C(=O)-N(R^{10e})-(C₁-C₃-Alkylen)- ist, worin die Alkylengruppe durch R^{8e} und durch R^{9e} substituiert ist;
R^{8e} und R^{9e} unabhängig voneinander ausgewählt sind aus:
H, C(=O₂)R^{18be}, C(=O)R^{18b}, CONR¹⁷R^{18be}
C₁-C₁₀-Alkyl, substituiert mit 0-1R^{6e},
C₂-C₁₀-Alkenyl, substituiert mit 0-1R^{6e},
C₂-C₁₀-Alkinyl, substituiert mit 0-1R^{6e},
C₃-C₈-Cycloalkyl, substituiert mit 0-1R^{6e},
C₅-C₆-Cycloalkenyl, substituiert mit 0-1R^{6e},
(C₁-C₁₀-Alkyl)carbonyl,
C₃-C₁₀-Cycloalkyl(C₁-C₄-alkyl)-,
Phenyl, substituiert mit 0-3R^{6e},
Naphthyl, substituiert mit 0-3R^{6e},
einem 5- bis 10gliedrigen heterocyclischen Ring, enthaltend 1-3N-, O- oder S-Heteroatome, wobei der heterocyclische Ring gesättigt, teilweise gesättigt oder vollständig ungesättigt sein kann, wobei der heterocyclische Ring mit 0-2R^{7e} substituiert ist,
C₁-C₁₀-Alkoxy, substituiert mit 0-2R^{7e},
Hydroxy, Nitro, -N(R^{10e})R^{11e}, -N(R^{16e})R^{17e} , Aryl(C₀-C₆-alkyl)carbonyl, Aryl(C₃-C₆-alkyl), Heteroaryl(C₁-C₆-alkyl), CONR^{18ae}R^{20e}, SO₂R^{18ae} und SO₂NR^{18ae}R^{20e} ,
mit der Maßgabe, daß jede der obigen Alkyl-, Cycloalkyl-, Aryl- oder Heteroarylgruppen unsubstituiert oder unabhängig mit 1-2R^{7e} substituiert sein kann;
R^{6e} ausgewählt ist aus:
H, C₁-C₁₀-Alkyl, Hydroxy, C₁-C₁₀-Alkoxy, Nitro, C₁-C₁₀-Alkylcarbonyl, -N(R^{11e})R^{12e} Cyano, Halogen, CF₃, CHO, CO₂R^{18be}, C(=O)R^{18be} , CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
Aryl, substituiert mit 0 - 3 Gruppen, ausgewählt aus Halogen, C₁-C₆-Alkoxy, C₁-C₆-Alkyl, CF₃, S(O)ₘ^{e}Me und -NMe₂,
Aryl(C₁-C₄-alkyl)-, wobei das Aryl substituiert ist mit 0 - 3 Gruppen, ausgewählt aus Halogen, C₁-C₆-Alkoxy, C₁-C₆-Alkyl, CF₃, S(O)ₚ^{e} Me und -NMe₂;
und einem 5- bis 10gliedrigen heterocyclischen Ring, enthaltend 1-3N-, O-oder S-Heteroatome, wobei der heterocyclische Ring gesättigt, teilweise gesättigt oder vollständig ungesättigt sein kann, wobei der heterocyclische Ring mit 0-2R^{7e} substituiert ist;
R^{10e} ausgewählt ist aus:
H, -OH, CF₃, C₃-C₆-Alkenyl, C₃-C₁₁-Cycloalkyl, Aryl, (C₃-C₁₁-Cycloalkyl)methyl, Aryl(C₁-C₄-alkyl) und C₁-C₁₀-Alkyl, substituiert mit 0-2R^{6e};
R^{11e} ausgewählt ist aus:
H, Hydroxy, C₁-C₈-Alkyl, C₃-C₆-Alkenyl, C₃-C₁₁-Cycloalkyl, (C₃-C₁₁-Cycloalkyl)-methyl, C₁-C₆-Alkoxy, Benzyloxy, Aryl, Heteroaryl, Heteroaryl(C₁-C₄-alkyl)-, Aryl-(C₁-C₄-alkyl), Adamantylmethyl und C₁-C₁₀-Alkyl, substituiert mit 0-2R^{4e};
R^{4e} ausgewählt ist aus:
H, C₁-C₆-Alkyl, C₃-C₇-Cycloalkyl, C₃-C₇-Cycloalkyl(C₁-C₄-alkyl)-, (C₁-C₁₀-Alkyl)-carbonyl, Aryl, Heteroaryl, Aryl(C₁-C₆-alkyl)- und Heteroaryl(C₁-C₆-alkyl)-, wobei die Aryl- oder Heteroarylgruppen mit 0 - 2 Substituenten, unabhängig ausgewählt aus der Gruppe, bestehend aus C₁-C₄-Alkyl, C₁-C₄-Alkoxy, F, Cl, Br, CF₃ und NO₂, substituiert sind,
alternativ, wenn R^{10e} und R^{11e} beide Substituenten an demselben Stickstoffatom sind (wie in -NR^{10e}R^{11e}), diese mit dem Stickstoffatom, an das sie gebunden sind, unter Bildung eines Heterocyclus, ausgewählt aus: 3-Azabicyclononyl, 1,2,3,4-Tetrahydro-1-chinolinyl, 1,2,3,4-Tetrahydro-2-isochinolinyl, 1-Piperidinyl, 1-Morpholinyl,1-Pyrrolidinyl, Thiamorpholinyl, Thiazolidinyl und 1-Piperazinyl, zusammengenommen werden können;
wobei der Heterocyclus mit 0-3Gruppen, ausgewählt aus: C₁-C₆-Alkyl, Aryl, Heteroaryl, Aryl(C₁-C₄-alkyl)-, (C₁-C₆-Alkyl)carbonyl, (C₃-C₇-Cycloalkyl)carbonyl, (C₁-C₆-Alkoxy)carbonyl, Aryl(C₁-C₄-alkoxy)carbonyl, C₁-C₆-Alkylsulfonyl und Arylsulfonyl substituiert ist;
R^{12e} ausgewählt ist aus:
H, C₁-C₆-Alkyl, Triphenylmethyl, Methoxymethyl, Methoxyphenyldiphenylmethyl, Trimethylsilylethoxymethyl, (C₁-C₆-Alkyl)carbonyl, (C₁-C₆-Alkoxy)carbonyl, (C₁-C₆-Alkyl)aminocarbonyl, C₃-C₆-Alkenyl, C₃-C₇-Cycloalkyl, C₃-C₇-Cycloalkyl-(C₁-C₄-alkyl)-, Aryl, Heteroaryl(C₁-C₆-alkyl)carbonyl, Heteroarylcarbonyl, Aryl-(C₁-C₆-alkyl)-, (C₁-C₆-Alkyl)carbonyl, Arylcarbonyl, C₁-C₆-Alkylsulfonyl, Arylsulfonyl, Aryl(C₁-C₆-alkyl)sulfonyl, Heteroarylsulfonyl, Heteroaryl(C₁-C₆-alkyl)sulfonyl, Aryloxycarbonyl und Aryl(C₁-C₆-alkoxy)carbonyl, wobei die Arylgruppen mit 0 - 2 Substituenten, ausgewählt aus der Gruppe, bestehend aus C₁-C₄-Alkyl, C₁-C₄-Alkoxy, Halogen, CF₃ und Nitro substituiert sind;
R^{16e} ausgewählt ist aus:
-C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂ und -SO₂NHC(=O)OR^{18be},
R^{17e} ausgewählt ist aus:
H, C₁-C₆-Alkyl, C₃-C₇-Cycloalkyl, C₃-C₇-Cycloalkyl(C₁-C₄-alkyl)-, Aryl, Aryl(C₁-C₆-alkyl)- und Heteroaryl(C₁-C₆-alkyl);
R^{18ae} ausgewählt ist aus:
C₁-C₈-Alkyl, gegebenenfalls substituiert mit einer Bindung an Lₙ, C₃-C₁₁-Cycloalkyl, gegebenenfalls substituiert mit einer Bindung an Lₙ, Aryl(C₁-C₆-alkyl)-, gegebenenfalls substituiert mit einer Bindung an Lₙ, Heteroaryl(C₁-C₆-alkyl)-, gegebenenfalls substituiert mit einer Bindung an Lₙ, (C₁-C₆-Alkyl)heteroaryl, gegebenenfalls substituiert mit einer Bindung an Lₙ, Biaryl(C₁-C₆-alkyl), gegebenenfalls substituiert mit einer Bindung an Lₙ, Heteroaryl, gegebenenfalls substituiert mit einer Bindung an Lₙ, Phenyl, substituiert mit 3 - 4 R^{19e} und gegebenenfalls substituiert mit einer Bindung an Lₙ, Naphthyl, substituiert mit 0-4R^{19e} und gegebenenfalls substituiert mit einer Bindung an Lₙ, und einer Bindung an Lₙ, wobei die Aryl- oder Heteroarylgruppen gegebenenfalls mit 0 - 4 R^{19e} substituiert sind;
R^{18be} H oder R^{18ae} ist,
R^{19e} ausgewählt ist aus:
H, Halogen, CF₃, CO₂H, CN, NO₂, -NR^{11e}R^{12e}, OCF₃, C₁-C₈-Alkyl, C₂-C₆-Alkenyl, C₂-C₆-Alkinyl, C₃-C₁₁-Cycloalkyl, C₃-C₇-Cycloalkyl(C₁-C₄-alkyl)-, Aryl(C₁-C₆-alkyl)-, C₁-C₆-Alkoxy, C₁-C₄-Alkoxycarbonyl, Aryl, Aryl-O-, Aryl-SO₂-, Heteroaryl und Heteroaryl-SO₂-, wobei die Aryl- und Heteroarylgruppen mit 0 - 4 Gruppen, ausgewählt aus Wasserstoff, Halogen, CF₃, C₁-C₃-Alkyl und C₁-C₃-Alkoxy, substituiert sind;
R^{20e} ausgewählt ist aus:
Hydroxy, C₁-C₁₀-Alkyloxy, C₃-C₁₁-Cycloalkyloxy, Aryloxy, Aryl(C₁-C₄-alkyl)oxy, C₂-C₁₀-Alkylcarbonyloxy(C₁-C₂-alkyl)oxy-, C₂-C₁₀-Alkoxycarbonyloxy(C₁-C₂-alkyl)oxy-, C₂-C₁₀-Alkoxycarbonyl(C₁-C₂-alkyl)oxy-, C₃-C₁₀-Cycloalkylcarbonyloxy(C₁-C₂-alkyl)oxy-, C₃-C₁₀-Cycloalkoxycarbonyloxy(C₁-C₂-alkyl)oxy-, C₃-C₁₀-Cycloalkoxycarbonyl(C₁-C₂-alkyl)oxy-, Aryloxycarbonyl(C₁-C₂-alkyl)oxy-, Aryloxycarbonyloxy(C₁-C₂-alkyl)oxy-, Arylcarbonyloxy(C₁-C₂-alkyl)oxy-, C₁-C₅-Alkoxy(C₁-C₅-alkyl)carbonyloxy(C₁-C₂-alkyl)oxy, (5-(C₁-C₅-Alkyl)-1,3-dioxa-cyclopenten-2-on-yl)methyloxy, (5-Aryl-1,3-dioxa-cyclopenten-2-on-yl)methyloxy und (R^{10e})(R^{11e})N-(C₁-C₁₀-Alkoxy)-;
R^{21e} ausgewählt ist aus:
C₁-C₈-Alkyl, C₂-C₆-Alkenyl, C₃-C₁₁-Cycloalkyl, (C₃-C₁₁-Cycloalkyl)methyl, Aryl, Aryl(C₁-C₄-alkyl)- und C₁-C₁₀-Alkyl, substituiert mit 0 - 2 R^{7e};
R^{22e} ausgewählt ist aus:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae} und -C(=O)NHSO₂NHR^{18be}
Y^{e} ausgewählt ist aus:
-COR^{20e}, -SO₃H, -PO₃H, -CONHNHSO₂CF₃, -CONHSO₂R^{18ae}, -CONHSO₂NHR^{18be}, -NHCOCF₃, -NHCONHSO₂R^{18ae}, -NHSO₂R^{18ae}, -OPO₃H₂, -OSO₃H, -PO₃H₂, -SO₂NHCOR^{18ae}, -SO₂NHCO₂R^{18ae}, 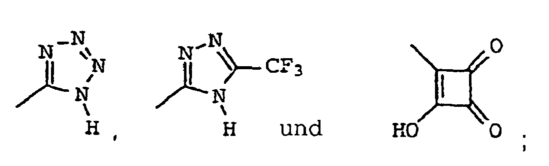
m^{e} 0 - 2 ist;
n^{e} 0 - 4 ist;
p^{e} 0 - 2 ist;
r^{e} 0 - 2 ist;
mit der folgenden Maßgabe: n^{e} und m^{e} sind so gewählt, daß die Anzahl an Atomen, die R^{1e} und Y^{e} verbinden, im Bereich von 8 - 14 liegt;
d ausgewählt ist aus 1, 2, 3, 4, 5, 6, 7, 8, 9 und 10;
d' 1 - 100 ist;
Lₙ eine Verknüpfungsgruppe mit der Formel:
((W)ₕ-(CR⁶R⁷)_{g})ₓ-(Z)ₖ-((CR^{6a}R^{7a})_{g},-(W)_{h'})_{x'}
ist;
W unabhängig bei jedem Vorkommen aus der Gruppe: O, S, NH, NHC(=O), C(=O)NH, NR⁸C(=O), C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ und (aa)_{t'} ausgewählt ist;
aa unabhängig bei jedem Vorkommen eine Aminosäure ist;
Z ausgewählt ist aus der Gruppe: Aryl, substituiert mit 0-3R¹⁰, C₃₋₁₀-Cycloalkyl, substituiert mit 0 - 3 R¹⁰, und einem 5- bis 10gliedrigen heterocyclischen Ringsystem, enthaltend 1-4Heteroatome, unabhängig ausgewählt aus N, S und O und substituiert mit 0-3R¹⁰;
R⁶, R^{6a}, R⁷, R^{7a} und R⁸ unabhängig bei jedem Vorkommen aus der Gruppe: H, =O, COOH, SO₃H, PO₃H, C₁-C₅-Alkyl, substituiert mit 0-3R¹⁰, Aryl, substituiert mit 0-3R¹⁰, Benzyl, substituiert mit 0-3R¹⁰, und C₁-C₅-Alkoxy, substituiert mit 0 - 3 R¹⁰, NHC(=O)R¹¹, C(=O)NHR¹¹, NHC(=O)NHR¹¹, NHR¹¹, R¹¹ und einer Bindung an Cₕ ausgewählt sind;
R¹⁰ unabhängig bei jedem Vorkommen aus der Gruppe: einer Bindung an Cₕ, COOR¹¹, C(=O)NHR¹¹, NHC(=O)R¹¹, OH, NHR¹¹, SO₃H, PO₃H, -OPO₃H₂, -OSO₃H, Aryl, substituiert mit 0-3R¹¹, C₁₋₅-Alkyl, substituiert mit 0-1R¹², C₁-C₅-Alkoxy, substituiert mit 0-1R¹², und einem 5- bis 10gliedrigen heterocyclischen Ringsystem, enthaltend 1-4Heteroatome, unabhängig ausgewählt aus N, S und O und substituiert mit 0-3R¹¹, ausgewählt ist;
R¹¹ unabhängig bei jedem Vorkommen aus der Gruppe: H, -OPO₃H₂, -OSO₃H, C₁₋₅-Alkyl, substituiert mit 0-1R¹² , Aryl, substituiert mit 0-1R¹² , einem 5- bis 10gliedrigen heterocyclischen Ringsystem, enthaltend 1-4Heteroatome, unabhängig ausgewählt aus N, S und O und substituiert mit 0-1R¹², C₃₋₁₀-Cycloalkyl, substituiert mit 0-1R¹², Polyalkylenglycol, substituiert mit 0-1R¹², C₁₋₅-Alkyl, substituiert mit einem Kohlenhydrat, substituiert mit 0-1R¹², Cyclodextrin, substituiert mit 0-1R¹², Aminosäure, substituiert mit 0-1R¹², Polycarboxyalkyl, substituiert mit 0-1R¹²; Polyazaalkyl, substituiert mit 0-1R¹², Peptid, substituiert mit 0-1R¹², wobei das Peptid aus 2 -10 Aminosäuren besteht, 3,6-O-Disulfo-B-D-galactopyranosyl, Bis(phosphonomethyl)glycin und einer Bindung an Cₕ, ausgewählt ist;
R¹² eine Bindung an Cₕ ist;
k ausgewählt ist aus 0, 1 und 2;
h ausgewählt ist aus 0, 1 und 2;
h' ausgewählt ist aus 0, 1 und 2;
g ausgewählt ist aus 0, 1, 2, 3, 4, 5, 6, 7, 8, 9 und 10;
g' ausgewählt ist aus 0, 1, 2, 3, 4, 5, 6, 7, 8, 9 und 10;
s ausgewählt ist aus 0, 1, 2, 3, 4, 5, 6, 7, 8, 9 und 10;
s' ausgewählt ist aus 0, 1, 2, 3, 4, 5, 6, 7, 8, 9 und 10;
s" ausgewählt ist aus 0, 1, 2, 3, 4, 5, 6, 7, 8, 9 und 10;
t ausgewählt ist aus 0, 1, 2, 3, 4, 5, 6, 7, 8, 9 und 10;
t' ausgewählt ist aus 0, 1, 2, 3, 4, 5, 6, 7, 8, 9 und 10;
x ausgewählt ist aus 0, 1, 2, 3, 4, 5 und 6;
x' ausgewählt ist aus 0, 1, 2, 3, 4, 5 und 6;
Cₕ eine Metallbindungseinheit mit einer Formel, ausgewählt aus der Gruppe: 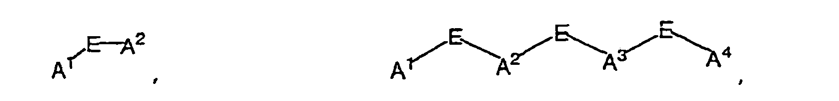 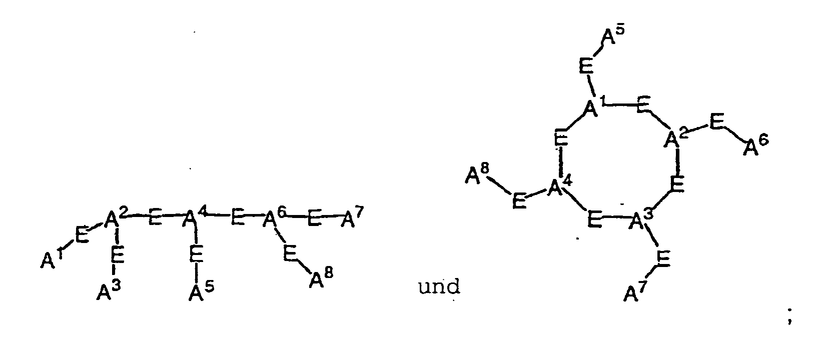
A¹, A², A³, A⁴, A⁵, A⁶, A⁷ und A⁸ unabhängig bei jedem Vorkommen aus der Gruppe: NR¹³, NR¹³R¹⁴, S, SH, S(Pg), O, OH, PR¹³, PR¹³R¹⁴, P(O)R¹⁵R¹⁶ und einer Bindung an Lₙ ausgewählt sind;
E eine Bindung, CH oder eine Spacer-Gruppe ist, unabhängig bei jedem Vorkommen ausgewählt aus der Gruppe: C₁-C₁₀-Alkyl, substituiert mit 0-3R¹⁷, Aryl, substituiert mit 0-3R¹⁷, C₃₋₁₀-Cycloalkyl, substituiert mit 0-3R¹⁷, Heterocyclo-C₁₋₁₀-alkyl, substituiert mit 0-3R¹⁷, wobei die Heterocyclogruppe ein 5- bis 10gliedriges heterocyclisches Ringsystem ist, enthaltend 1 - 4 Heteroatome, unabhängig ausgewählt aus N, S und O, C₆₋₁₀-Aryl-C₁₋₁₀-alkyl, substituiert mit 0 - 3 R¹⁷, C₁₋₁₀-Alkyl-C₆₋₁₀-aryl, substituiert mit 0-3R¹⁷, und einem 5- bis 10gliedrigen heterocyclischen Ringsystem, enthaltend 1-4Heteroatome, unabhängig ausgewählt aus N, S und O und substituiert mit 0-3R¹⁷;
R¹³ und R¹⁴ jeweils unabhängig voneinander ausgewählt sind aus der Gruppe: einer Bindung an Lₙ, Wasserstoff, C₁-C₁₀-Alkyl, substituiert mit 0-3R¹⁷, Aryl, substituiert mit 0-3R¹⁷, C₁₋₁₀-Cycloalkyl, substituiert mit 0-3R¹⁷, Heterocyclo-C₁₋₁₀-alkyl, substituiert mit 0-3R¹⁷, wobei die Heterocyclogruppe ein 5- bis 10gliedriges heterocyclisches Ringsystem ist, enthaltend 1 - 4 Heteroatome, unabhängig ausgewählt aus N, S und O, C₆₋₁₀-Aryl-C₁₋₁₀-alkyl, substituiert mit 0-3R¹⁷, C₁₋₁₀-Alkyl-C₆₋₁₀-aryl, substituiert mit 0-3R¹⁷, einem 5- bis 10gliedrigen heterocyclischen Ringsystem, enthaltend 1-4Heteroatome, unabhängig ausgewählt aus N, S und O und substituiert mit 0-3R¹⁷, und einem Elektron, vorausgesetzt, daß, wenn einer von R¹³ oder R¹⁴ ein Elektron ist, der andere auch ein Elektron ist;
alternativ R¹³ und R¹⁴ unter Bildung von =C(R²⁰)(R²¹) verbunden sind;
R¹⁵ und R¹⁶ jeweils unabhängig voneinander ausgewählt sind aus der Gruppe: einer Bindung an Lₙ, -OH, C₁-C₁₀-Alkyl, substituiert mit 0-3R¹⁷, C₁-C₁₀-Alkyl, substituiert mit 0-3R¹⁷, Aryl, substituiert mit 0-3R¹⁷, C₃₋₁₀-Cycloalkyl, substituiert mit 0-3R¹⁷, Heterocyclo-C₁₋₁₀-alkyl, substituiert mit 0-3R¹⁷, wobei die Heterocyclogruppe ein 5- bis 10gliedriges heterocyclisches Ringsystem ist, enthaltend 1-4 Heteroatome, unabhängig ausgewählt aus N, S und O, C₆₋₁₀-Aryl-C₁₋₁₀-alkyl, substituiert mit 0-3R¹⁷, C₁₋₁₀-Alkyl-C₆₋₁₀-aryl-, substituiert mit 0-3R¹⁷, und einem 5- bis 10gliedrigen heterocyclischen Ringsystem, enthaltend 1-4Heteroatome, unabhängig ausgewählt aus N, S und O und substituiert mit 0-3R¹⁷;
R¹⁷ unabhängig bei jedem Vorkommen ausgewählt ist aus der Gruppe: einer Bindung an Lₙ, =O, F, Cl, Br, l, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H, 2-(1-Morpholino)ethoxy, C₁-C₅-Alkyl, C₂-C₄-Alkenyl, C₃-C₆-Cycloalkyl, C₃-C₆-Cycloalkylmethyl, C₂-C₆-Alkoxyalkyl, Aryl, substituiert mit 0-2R¹⁸, und einem 5- bis 10gliedrigen heterocyclischen Ringsystem, enthaltend 1-4Heteroatome, unabhängig ausgewählt aus N, S und O;
R¹⁸, R^{18a} und R¹⁹ unabhängig bei jedem Vorkommen ausgewählt sind aus der Gruppe: einer Bindung an Lₙ, H, C₁-C₆-Alkyl, Phenyl, Benzyl, C₁-C₆-Alkoxy, Halogenid, Nitro, Cyano und Trifluormethyl;
Pg eine Thiol-Schutzgruppe ist;
R²⁰ und R²¹ unabhängig voneinander ausgewählt sind aus der Gruppe: H, C₁-C₁₀-Alkyl, -CN, -CO₂R²⁵, -C(=O)R²⁵, -C(=O)N(R²⁵)₂, C₂-C₁₀-1-Alken, substituiert mit 0-3R²³, C₂-C₁₀-1-Alkin, substituiert mit 0-3R²³, Aryl, substituiert mit 0-3R²³, einem ungesättigten 5- bis 10gliedrigen heterocyclischen Ringsystem, enthaltend 1-4Heteroatome, unabhängig ausgewählt aus N, S und O und substituiert mit 0-3R²³, und einem ungesättigten C₃₋₁₀-Carbocyclus, substituiert mit 0-3R²³;
alternativ R²⁰ und R²¹ zusammen mit dem zweiwertigen Kohlenstoffrest, an den sie gebunden sind: 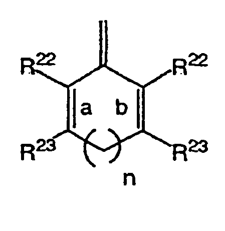 bilden;
R²² und R²³ unabhängig voneinander ausgewählt sind aus der Gruppe: H, R²⁴, C₁-C₁₀-Alkyl, substituiert mit 0-3R²⁴, C₂-C₁₀-Alkenyl, substituiert mit 0-3 R²⁴, C₂-C₁₀-Alkinyl, substituiert mit 0-3R²⁴, Aryl, substituiert mit 0-3R²⁴, einem 5-bis 10gliedrigen heterocyclischen Ringsystem, enthaltend 1-4Heteroatome, unabhängig ausgewählt aus N, S und O und substituiert mit 0-3R²⁴, und einem C₃₋₁₀-Carbocyclus, substituiert mit 0-3R²⁴;
alternativ R²², R²³ zusammen ein kondensiertes aromatisches oder ein 5- bis 10gliedriges heterocyclisches Ringsystem, enthaltend 1-4Heteroatome, unabhängig ausgewählt aus N, S und O, bilden;
a und b die Positionen optionaler Doppelbindungen anzeigen und n 0 oder 1 ist;
R²⁴ unabhängig bei jedem Vorkommen ausgewählt ist aus der Gruppe: =O, F, Cl, Br, l, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)_{2,} -N(R²⁵)₂, =NOR²⁵, -C(=O)NHOR²⁵, -OCH₂CO₂H und 2-(1-Morpholino)ethoxy und
R²⁵ , R^{25a} und R²⁶ jeweils unabhängig voneinander bei jedem Vorkommen ausgewählt sind aus der Gruppe: Wasserstoff und C₁-C₆-Alkyl;
oder ein pharmazeutisch akzeptables Salz davon umfaßt.

18. Therapeutische radiopharmazeutische Zusammensetzung nach Anspruch 17,
wobei Q eine Verbindung der Formel (IV): 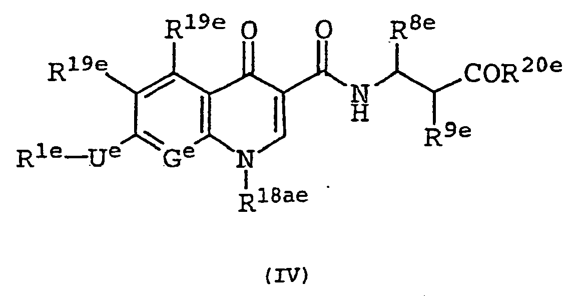 ist,
einschließlich stereoisomere Formen davon oder Gemische stereoisomerer Formen davon oder pharmazeutisch akzeptable Salz- oder Prodrug-Formen davon, worin:
R^{1e} ausgewählt ist aus: 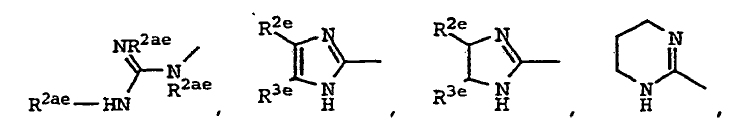 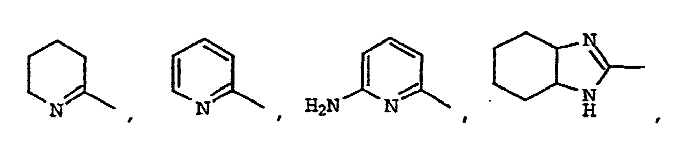 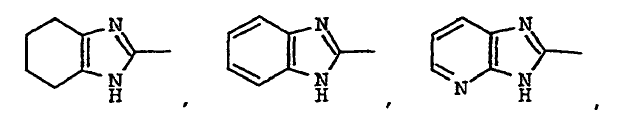 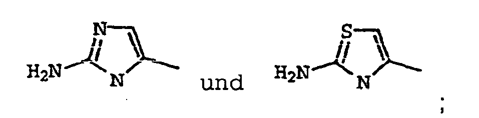
R^{2e} und R^{3e} unabhängig voneinander ausgewählt sind aus:
H, C₁-C₄-Alkoxy, NR^{11e}R^{12e}, Halogen, NO₂, CN, CF₃, C₁-C₆-Alkyl, C₃-C₆-Alkenyl, C₃-C₇-Cycloalkyl, C₃-C₇-Cycloalkyl(C₁-C₄-alkyl), Aryl(C₁-C₆-alkyl)-, (C₁-C₆-Alkyl)-carbonyl, (C₁-C₆-Alkoxy)carbonyl, Arylcarbonyl und Aryl, substituiert mit 0-4R^{7e},
alternativ, wenn R^{2e} und R^{3e} Substituenten an nachbarständigen Atomen sind, diese mit den Kohlenstoffatomen, an die sie gebunden sind, unter Bildung eines 5- bis 7gliedrigen carbocyclischen oder 5- bis 7gliedrigen heterocyclischen aromatischen oder nicht-aromatischen Ringsystems zusammengenommen werden können, wobei der carbocyclische oder heterocyclische Ring mit 0 - 2 Gruppen, ausgewählt aus C₁-C₄-Alkyl, C₁-C₄-Alkoxy, Halogen, Cyano, Amino, CF₃ und NO₂, substituiert ist;
R^{2ae} ausgewählt ist aus:
H, C₁-C₁₀-Alkyl, C₂-C₆-Alkenyl, C₃-C₁₁-Cycloalkyl, C₃-C₇-Cycloalkyl(C₁-C₄-alkyl), Aryl, Aryl(C₁-C₄-alkyl)-, (C₂-C₇-Alkyl)carbonyl, Arylcarbonyl, (C₂-C₁₀-Alkoxy)carbonyl, C₃-C₇-Cycloalkoxycarbonyl, C₇-C₁₁-Bicycloalkoxycarbonyl, Aryloxycarbonyl, Aryl(C₁-C₁₀-alkoxy)carbonyl, C₁-C₆-Alkylcarbonyloxy(C₁-C₄-alkoxy)carbonyl, Arylcarbonyloxy(C₁-C₄-alkoxy)carbonyl und C₃-C₇-Cycloalkylcarbonyloxy(C₁-C₄-alkoxy)carbonyl;
R^{7e} ausgewählt ist aus:
H, Hydroxy, C₁-C₄-Alkyl, C₁-C₄-Alkoxy, Aryl, Aryl(C₁-C₄-alkyl)-, (C₁-C₄-Alkyl)-carbonyl, CO₂R^{18ae}, SO₂R^{11e}, SO₂NR^{10e}R^{11e}, OR^{10e} und N(R^{11e})R^{12e};
U^{e} ausgewählt ist aus:
-(CH₂)ₙ^{e}-, -(CH₂)ₙ^{e}O(CH₂)ₘ^{e}-, -NH(CH₂)ₙ^{e}-, -N(R^{10e})C(=O)-, -NHC(=O)(CH₂)ₙ^{e}-und C(=O)N(R¹⁰e)-;
G^{e} N oder CR^{19e} ist;
R^{8e} ausgewählt ist aus:
H, CO₂R^{18be}, C(=O)R^{18be}, CONR^{17e}R^{18be},
C₁-C₁₀-Alkyl, substituiert mit 0-1R^{6e},
C₂-C₁₀-Alkenyl, substituiert mit 0-1R^{6e},
C₂-C₁₀-Alkinyl, substituiert mit 0-1R^{6e},
C₃-C₈-Cycloalkyl, substituiert mit 0-1R^{6e},
C₅-C₆-Cycloalkenyl, substituiert mit 0-1R^{6e},
(C₁-C₁₀-Alkyl)carbonyl,
C₃-C₁₀-Cycloalkyl(C₁-C₄-alkyl)-,
Phenyl, substituiert mit 0-3R^{6e},
Naphthyl, substituiert mit 0-3R^{6e},
einem 5- bis 10gliedrigen heterocyclischen Ring, enthaltend 1-3N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist;
R^{9e} ausgewählt ist aus:
C₁-C₁₀-Alkyl, substituiert mit 0-1R^{6e},
C₁-C₁₀-Alkoxy, substituiert mit 0-2R^{7e},
H, Nitro, -N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, Hydroxy, OR^{22e}, -N(R^{10e})R^{11e}, -N(R^{16e})R^{17e} , Aryl(C₀-C₆-alkyl)carbonyl, Aryl-(C₁-C₆-alkyl), Heteroaryl(C₁-C₆ alkyl), CONR^{18ae}R^{20e}, SO₂R^{18ae} und SO₂NR^{18ae}R^{20e},
mit der Maßgabe, daß jede der obigen Alkyl-, Cycloalkyl-, Aryl- oder Heteroarylgruppen unsubstituiert oder unabhängig mit 1-2R^{7e} substituiert sein kann;
R^{6e} ausgewählt ist aus:
H, C₁-C₁₀-Alkyl, Hydroxy, C₁-C₁₀-Alkoxy, Nitro, C₁-C₁₀-Alkylcarbonyl, -N(R^{11e})R^{12e}, Cyano, Halogen, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚ^{e}R^{11e}, SO₂NR^{10e}R^{11e},
Aryl, substituiert mit 0 - 3 Gruppen, ausgewählt aus Halogen, C₁-C₆-Alkoxy, C₁-C₆-Alkyl, CF₃, S(O)ₘ^{e}Me und -NMe₂,
Aryl(C₁-C₄-alkyl)-, wobei das Aryl substituiert ist mit 0 - 3 Gruppen, ausgewählt aus Halogen, C₁-C₆-Alkoxy, C₁-C₆-Alkyl, CF₃, S(O)ₚ^{e}Me und -NMe₂, und
einem 5- bis 10gliedrigen heterocyclischen Ring, enthaltend 1-3N-, O- oder S-Heteroatome, wobei der heterocyclische Ring gesättigt, teilweise gesättigt oder vollständig ungesättigt sein kann, wobei der heterocyclische Ring mit 0-2R^{7e} substituiert ist;
R^{10e} ausgewählt ist aus:
H, -OH, CF₃, C₃-C₆-Alkenyl, C₃-C₁₁-Cycloalkyl, Aryl, (C₃-C₁₁-Cycloalkyl)methyl, Aryl(C₁-C₄-alkyl) und C₁-C₁₀-Alkyl, substituiert mit 0-2R^{6e};
R^{11e} ausgewählt ist aus:
H, Hydroxy, C₁-C₈-Alkyl, C₃-C₆-Alkenyl, C₃-C₁₁-Cycloalkyl, (C₃-C₁₁-Cycloalkyl)-methyl, C₁-C₆-Alkoxy, Benzyloxy, Aryl, Heteroaryl, Heteroaryl(C₁-C₄-alkyl)-, Aryl-(C₁-C₄-alkyl), Adamantylmethyl und C₁-C₁₀-Alkyl, substituiert mit 0-2R^{4e};
R^{4e} ausgewählt ist aus:
H, C₁-C₆-Alkyl, C₃-C₇-Cycloalkyl, C₃-C₇-Cycloalkyl(C₁-C₄-alkyl)-, Aryl, Heteroaryl, Aryl(C₁-C₆-alkyl)- und Heteroaryl(C₁-C₆-alkyl)-, wobei die Aryl- oder Heteroarylgruppen mit 0 - 2 Substituenten, unabhängig ausgewählt aus der Gruppe, bestehend aus C₁-C₄-Alkyl, C₁-C₄-Alkoxy, F, Cl, Br, CF₃ und NO₂, substituiert sind,
R^{12e} ausgewählt ist aus:
H, C₁-C₆-Alkyl, Triphenylmethyl, Methoxymethyl, Methoxyphenyldiphenylmethyl, Trimethylsilylethoxymethyl, (C₁-C₆-Alkyl)carbonyl, (C₁-C₆-Alkoxy)carbonyl, (C₁-C₆-Alkyl)aminocarbonyl, C₃-C₆-Alkenyl, C₃-C₇-Cycloalkyl, C₃-C₇-Cycloalkyl-(C₁-C₄-alkyl)-, Aryl, Heteroaryl(C₁-C₆-alkyl)carbonyl, Heteroarylcarbonyl, Aryl-(C₁-C₆-alkyl)-, (C₁-C₆-Alkyl)carbonyl, Arylcarbonyl, C₁-C₆-Alkylsulfonyl, Arylsulfonyl, Aryl(C₁-C₆-alkyl)sulfonyl, Heteroarylsulfonyl, Heteroaryl(C₁-C₆-alkyl)sulfonyl, Aryloxycarbonyl und Aryl(C₁-C₆-alkoxy)carbonyl, wobei die Arylgruppen mit 0 - 2 Substituenten, ausgewählt aus der Gruppe, bestehend aus C₁-C₄-Alkyl, C₁-C₄-Alkoxy, Halogen, CF₃ und Nitro, substituiert sind;
R^{16e} ausgewählt ist aus:
-C(=O)OR^{18ae}, -C(=O)R^{18be}, -C(=O)N(R^{18be})₂, -SO₂R^{18ae} und -SO₂N(R^{18be})₂;
R^{17e} ausgewählt ist aus:
H, C₁-C₆-Alkyl, C₃-C₇-Cycloalkyl, C₃-C₇-Cycloalkyl(C₁-C₄-alkyl)-, Aryl, Aryl(C₁-C₆-alkyl)- und Heteroaryl(C₁-C₆-alkyl);
R^{18ae} ausgewählt ist aus:
C₁-C₈-Alkyl, gegebenenfalls substituiert mit einer Bindung an Lₙ, C₃-C₁₁-Cycloalkyl, gegebenenfalls substituiert mit einer Bindung an Lₙ, Aryl(C₁-C₆-alkyl)-, gegebenenfalls substituiert mit einer Bindung an Lₙ, Heteroaryl(C₁-C₆-alkyl)-, gegebenenfalls substituiert mit einer Bindung an Lₙ, (C₁-C₆-Alkyl)heteroaryl, gegebenenfalls substituiert mit einer Bindung an Lₙ, Biaryl(C₁-C₆-alkyl), gegebenenfalls substituiert mit einer Bindung an Lₙ, Heteroaryl, gegebenenfalls substituiert mit einer Bindung an Lₙ, Phenyl, substituiert mit 3-4R^{19e} und gegebenenfalls substituiert mit einer Bindung an Lₙ, Naphthyl, substituiert mit 0-4R^{19e} und gegebenenfalls substituiert mit einer Bindung an Lₙ, und einer Bindung an Lₙ, wobei die Aryl- oder Heteroarylgruppen gegebenenfalls mit 0 - 4 R^{19e} substituiert sind;
R^{18be} H oder R^{18ae} ist,
R^{19e} ausgewählt ist aus:
H, Halogen, CF₃, CO₂H, CN, NO₂, -NR^{11e}R^{12e}, OCF₃, C₁-C₈-Alkyl, C₂-C₆-Alkenyl, C₂-C₆-Alkinyl, C₃-C₁₁-Cycloalkyl, C₃-C₇-Cycloalkyl(C₁-C₄-alkyl)-, Aryl(C₁-C₆-alkyl)-, C₁-C₆-Alkoxy, C₁-C₄-Alkoxycarbonyl, Aryl, Aryl-O-, Aryl-SO₂-, Heteroaryl und Heteroaryl-SO₂-, wobei die Aryl- und Heteroarylgruppen mit 0 - 4 Gruppen, ausgewählt aus Wasserstoff, Halogen, CF₃, C₁-C₃-Alkyl und C₁-C₃-Alkoxy, substituiert sind;
R^{20e} ausgewählt ist aus:
Hydroxy, C₁-C₁₀-Alkyloxy, C₃-C₁₁-Cycloalkyloxy, Aryloxy, Aryl(C₁-C₄-alkyl)oxy, C₂-C₁₀-Alkylcarbonyloxy(C₁-C₂-alkyl)oxy-, C₂-C₁₀-Alkoxycarbonyloxy(C₁-C₂-alkyl)oxy-, C₂-C₁₀-Alkoxycarbonyl(C₁-C₂-alkyl)oxy-, C₃-C₁₀-Cycloalkylcarbonyloxy-(C₁-C₂-alkyl)oxy-, C₃-C₁₀-Cycloalkoxycarbonyloxy(C₁-C₂-alkyl)oxy-, C₃-C₁₀-Cycloalkoxycarbonyl(C₁-C₂-alkyl)oxy-, Aryloxycarbonyl(C₁-C₂-alkyl)oxy-, Aryloxycarbonyloxy(C₁-C₂-alkyl)oxy-, Arylcarbonyloxy(C₁-C₂-alkyl)oxy-, C₁-C₅-Alkoxy(C₁-C₅-alkyl)carbonyloxy(C₁-C₂-alkyl)oxy, (5-(C₁-C₅-Alkyl)-1,3-dioxa-cyclopenten-2-on-yl)methyloxy, (5-Aryl-1,3-dioxa-cyclopenten-2-on-yl)methyloxy und (R^{10e})(R^{11e})N-(C₁-C₁₀-Alkoxy)-;
R^{21e} ausgewählt ist aus:
C₁-C₈-Alkyl, C₂-C₆-Alkenyl, C₃-C₁₁-Cycloalkyl, (C₃-C₁₁-Cycloalkyl)methyl, Aryl, Aryl(C₁-C₄-alkyl)- und C₁-C₁₀-Alkyl, substituiert mit 0 - 2 R^{7e};
R^{22e} ausgewählt ist aus:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae} und -C(=O)NHSO₂NHR^{18be};
m^{e} 0 - 2 ist;
n^{e} 0 - 4 ist und
p^{e} 0 - 2 ist;
mit der folgenden Maßgabe: n^{e} und m^{e} sind so gewählt, daß die Anzahl an Atomen, die R^{1e} und -COR^{20e} in der Formel (IV) verbinden, im Bereich von 8 - 14 liegt;
d ausgewählt ist aus 1, 2, 3, 4 und 5;
d' 1 - 50 ist;
W unabhängig bei jedem Vorkommen aus der Gruppe: O, NH, NHC(=O), C(=O)NH, NR⁸C(=O), C(=O)NR⁸, C(=O), C(=O)O, OC(=O), NHC(=S)NH, NHC(=O)NH, SO₂, (OCH₂CH₂)ₛ, (CH₂CH₂O)_{s'}, (OCH₂CH₂CH₂)_{s"}, (CH₂CH₂CH₂O)ₜ und (aa)_{t'} ausgewählt ist;
aa unabhängig bei jedem Vorkommen eine Aminosäure ist;
Z ausgewählt ist aus der Gruppe: Aryl, substituiert mit 0 - 1 R¹⁰, C₃₋₁₀-Cycloalkyl, substituiert mit 0 - 1 R¹⁰, und einem 5- bis 10gliedrigen heterocyclischen Ringsystem, enthaltend 1 - 4 Heteroatome, unabhängig ausgewählt aus N, S und O und substituiert mit 0 - 1 R¹⁰;
R⁶, R^{6a} , R⁷, R^{7a} und R⁸ unabhängig voneinander bei jedem Vorkommen aus der Gruppe: H, =O, COOH, SO₃H, C₁-C₅-Alkyl, substituiert mit 0 - 1 R¹⁰, Aryl, substituiert mit 0 - 1 R¹⁰, Benzyl, substituiert mit 0 - 1 R¹⁰, und C₁-C₅-Alkoxy, substituiert mit 0 - 1 R¹⁰, NHC(=O)R¹¹, C(=O)NHR¹¹, NHC(=O)NHR¹¹, NHR¹¹, R¹¹ und einer Bindung an Cₕ ausgewählt sind;
k 0 oder 1 ist;
s ausgewählt ist aus 0, 1, 2, 3, 4 und 5;
s' ausgewählt ist aus 0, 1, 2, 3, 4 und 5;
s" ausgewählt ist aus 0, 1, 2, 3, 4 und 5;
t ausgewählt ist aus 0, 1, 2, 3, 4 und 5;
A¹, A², A³, A⁴, A⁵, A⁶, A⁷ und A⁸ unabhängig voneinander bei jedem Vorkommen aus der Gruppe: NR¹³, NR¹³R¹⁴, S, SH, S(Pg), OH und einer Bindung an Lₙ ausgewählt sind;
E eine Bindung, CH oder eine Spacer-Gruppe ist, unabhängig bei jedem Vorkommen ausgewählt aus der Gruppe: C₁-C₁₀-Alkyl, substituiert mit 0 - 3 R¹⁷ , Aryl, substituiert mit 0 - 3 R¹⁷, C₃₋₁₀-Cycloalkyl, substituiert mit 0 - 3 R¹⁷ , und einem 5-bis 10gliedrigen heterocyclischen Ringsystem, enthaltend 1 - 4 Heteroatome, unabhängig ausgewählt aus N, S und O und substituiert mit 0 - 3 R¹⁷;
R¹³ und R¹⁴ jeweils unabhängig voneinander ausgewählt sind aus der Gruppe: einer Bindung an Lₙ, Wasserstoff, C₁-C₁₀-Alkyl, substituiert mit 0 - 3 R¹⁷ , Aryl, substituiert mit 0 - 3 R¹⁷ , einem 5- bis 10gliedrigen heterocyclischen Ringsystem, enthaltend 1 - 4 Heteroatome, unabhängig ausgewählt aus N, S und O und substituiert mit 0 - 3 R¹⁷, und einem Elektron, vorausgesetzt, daß, wenn einer von R¹³ oder R¹⁴ ein Elektron ist, der andere auch ein Elektron ist;
alternativ R¹³ und R¹⁴ unter Bildung von =C(R²⁰)(R²¹) verbunden sind;
R¹⁷ unabhängig bei jedem Vorkommen ausgewählt ist aus der Gruppe: einer Bindung an Lₙ, =O, F, Cl, Br, I, -CF₃, -CN, -CO₂R¹⁸, -C(=O)R¹⁸, -C(=O)N(R¹⁸)₂, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)₂, -N(R¹⁸)₂, -NHC(=S)NHR¹⁸, =NOR¹⁸, -C(=O)NHNR¹⁸R^{18a}, -OCH₂CO₂H und 2-(1-Morpholino)ethoxy;
R¹⁸, R^{18a} und R¹⁹ unabhängig voneinander bei jedem Vorkommen ausgewählt sind aus der Gruppe: einer Bindung an Lₙ, H und C₁-C₆-Alkyl;
R²⁰ und R²¹ unabhängig voneinander ausgewählt sind aus der Gruppe:
H, C₁-C₅-Alkyl, -CO₂R²⁵, C₂-C₅-1-Alken, substituiert mit 0 - 3 R²³, C₂-C₅-1-Alkin, substituiert mit 0 - 3 R²³, Aryl, substituiert mit 0 - 3 R²³, und einem ungesättigten 5- bis 10gliedrigen heterocyclischen Ringsystem, enthaltend 1 - 4 Heteroatome, unabhängig ausgewählt aus N, S und O und substituiert mit 0 - 3 R²³;
alternativ R²⁰ und R²¹ zusammen mit dem zweiwertigen Kohlenstoffrest, an den sie gebunden sind: 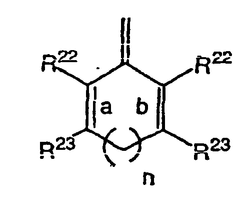 bilden,
R²² und R²³ unabhängig voneinander ausgewählt sind aus der Gruppe: H und R²⁴;
alternativ R²², R²³ zusammen ein kondensiertes aromatisches oder ein 5- bis 10gliedriges heterocyclisches Ringsystem, enthaltend 1 - 4 Heteroatome, unabhängig ausgewählt aus N, S und O, bilden;
R²⁴ unabhängig bei jedem Vorkommen ausgewählt ist aus der Gruppe: -CO₂R²⁵, -C(=O)N(R²⁵)₂, -CH₂OR²⁵, -OC(=O)R²⁵, -OR²⁵, -SO₃H, -N(R²⁵)₂ und -OCH₂CO₂H und
R²⁵ unabhängig bei jedem Vorkommen ausgewählt ist aus der Gruppe: H und C₁-C₃-Alkyl.

19. Therapeutische radiopharmazeutische Zusammensetzung nach Anspruch 17, worin:
R^{1e} ausgewählt ist aus: 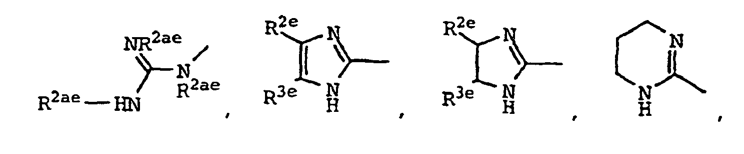 und 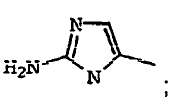
R^{2e} und R^{3e} unabhängig voneinander ausgewählt sind aus:
H, C₁-C₄-Alkoxy, NR^{11e}R^{12e}, Halogen, NO₂, CN, CF₃, C₁-C₆-Alkyl, C₃-C₆-Alkenyl, C₃-C₇-Cycloalkyl, C₃-C₇-Cycloalkyl(C₁-C₄-alkyl), Aryl(C₁-C₆-alkyl)-, (C₁-C₆-Alkyl)-carbonyl, (C₁-C₆-Alkoxy)carbonyl, Arylcarbonyl und Aryl, substituiert mit 0 - 4 R^{7e},
alternativ, wenn R^{2e} und R^{3e} Substituenten an nachbarständigen Atomen sind, diese mit den Kohlenstoffatomen, an die sie gebunden sind, unter Bildung eines 5- bis 7gliedrigen carbocyclischen oder 5- bis 7gliedrigen heterocyclischen aromatischen oder nicht-aromatischen Ringsystems zusammengenommen werden können, wobei der carbocyclische oder heterocyclische Ring mit 0 - 2 Gruppen, ausgewählt aus C₁-C₄-Alkyl, C₁-C₄-Alkoxy, Halogen, Cyano, Amino, CF₃ und NO₂, substituiert ist;
R^{2ae} ausgewählt ist aus:
H, C₁-C₁₀-Alkyl, C₂-C₆-Alkenyl, C₃-C₁₁-Cycloalkyl, C₃-C₇-Cycloalkyl(C₁-C₄-alkyl), Aryl, Aryl(C₁-C₄-alkyl)-, (C₂-C₇-Alkyl)carbonyl, Arylcarbonyl, (C₂-C₁₀-Alkoxy)carbonyl, C₃-C₇-Cycloalkoxycarbonyl, C₇-C₁₁-Bicycloalkoxycarbonyl, Aryloxycarbonyl, Aryl(C₁-C₁₀-alkoxy)carbonyl, C₁-C₆-Alkylcarbonyloxy(C₁-C₄-alkoxy)carbonyl, Arylcarbonyloxy(C₁-C₄-alkoxy)carbonyl und C₃-C₇-Cycloalkylcarbonyloxy(C₁-C₄-alkoxy)carbonyl;
R^{7e} ausgewählt ist aus:
H, Hydroxy, C₁-C₄-Alkyl, C₁-C₄-Alkoxy, Aryl, Aryl(C₁-C₄-alkyl)-, (C₁-C₄-Alkyl)carbonyl, CO₂R^{18ae}, SO₂R^{11e}, SO₂NR^{10e}R^{11e}, OR^{10e} und N(R^{11e})R^{12e};
U^{e} ausgewählt ist aus:
-(CH₂)ₙ^{e}-, -NH(CH₂)ₙ^{e}-, -N(R^{10e})C(=O)- und -NHC(=O)(CH₂)ₙ^{e};
G^{e} N oder CR^{19e} ist;
R^{8e} H ist;
R^{9e} ausgewählt ist aus:
H, Nitro, -N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, Hydroxy, OR^{22e}, -N(R^{10e})R^{11e}, -N(R^{16e})R^{17e}, Aryl(C₀-C₄-alkyl)carbonyl, Aryl-(C₁-C₄-alkyl), Heteroaryl(C₁-C₄-alkyl), CONR^{18ae}R^{20e}, SO₂R^{18ae} und SO₂NR^{18ae}R^{20e},
mit der Maßgabe, daß jede der obigen Alkyl-, Cycloalkyl-, Aryl- oder Heteroarylgruppen unsubstituiert oder unabhängig mit 1 - 2 R^{7e} substituiert sein kann;
R^{10e} ausgewählt ist aus:
H, -OH, CF₃, C₃-C₆-Alkenyl, C₃-C₆-Cycloalkyl, Aryl, (C₃-C₆-Cycloalkyl)methyl, Aryl(C₁-C₄-alkyl) und C₁-C₄-Alkyl, substituiert mit 0 - 2 R^{6e};
R^{6e} ausgewählt ist aus:
H, C₁-C₄-Alkyl, Hydroxy, C₁-C₄-Alkoxy, Nitro, C₁-C₄-Alkylcarbonyl, -N(R^{11e})R^{12e}, Cyano, Halogen, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e} NR^{10e}SO₂NR^{10e}R^{11e}, NR^{10e}SO₂R^{21e},S(O)ₚR^{11e}, SO₂NR^{10e}R^{11e},
Aryl, substituiert mit 0 - 3 Gruppen, ausgewählt aus Halogen, C₁-C₄-Alkoxy, C₁-C₄-Alkyl, CF₃, S(O)ₘ^{e}Me und -NMe₂,
Aryl(C₁-C₄-alkyl)-, wobei das Aryl substituiert ist mit 0 - 3 Gruppen, ausgewählt aus Halogen, C₁-C₄-Alkoxy, C₁-C₄-Alkyl, CF₃, S(O)ₚ^{e}Me und -NMe₂, und
einem 5- bis 10gliedrigen heterocyclischen Ring, enthaltend 1 - 3 N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist;
R^{11e} ausgewählt ist aus:
H, Hydroxy, C₁-C₄-Alkyl, C₃-C₆-Alkenyl, C₃-C₆-Cycloalkyl, (C₃-C₆-Cycloalkyl)-methyl, C₁-C₄-Alkoxy, Benzyloxy, Aryl, Heteroaryl, Heteroaryl(C₁-C₄-alkyl)-, Aryl(C₁-C₄-alkyl), Adamantylmethyl und C₁-C₄-Alkyl, substituiert mit 0 - 2 R^{4e};
R^{4e} ausgewählt ist aus:
H, C₁-C₄-Alkyl, C₃-C₇-Cycloalkyl, C₃-C₇-Cycloalkyl(C₁-C₄-alkyl)-, Aryl, Heteroaryl, Aryl(C₁-C₄-alkyl)- und Heteroaryl(C₁-C₄-alkyl)-, wobei die Aryl- oder Heteroarylgruppen mit 0 - 2 Substituenten, unabhängig ausgewählt aus der Gruppe, bestehend aus C₁-C₄-Alkyl, C₁-C₄-Alkoxy, F, Cl, Br, CF₃ und NO₂, substituiert sind,
R^{12e} ausgewählt ist aus:
H, C₁-C₄-Alkyl, (C₁-C₄-Alkyl)carbonyl, (C₁-C₄-Alkoxy)carbonyl, Phenyl(C₁-C₄-alkyl)-, Phenylsulfonyl, Phenyloxycarbonyl und Phenyl(C₁-C₄-alkoxy)carbonyl, wobei die Phenylgruppen mit 0 - 2 Substituenten, ausgewählt aus der Gruppe, bestehend aus C₁-C₄-Alkyl, C₁-C₄-Alkoxy, Halogen, CF₃ und Nitro, substituiert sind;
R^{16e} ausgewählt ist aus:
-C(=O)OR^{18ae}, -C(=O)R^{18be}, -C(=O)N(R^{18be})₂, -SO₂R^{18ae} und -SO₂N(R^{18be})₂;
R^{17e} ausgewählt ist aus:
H, C₁-C₄-Alkyl, C₃-C₆-Cycloalkyl, C₃-C₆-Cycloalkyl(C₁-C₄-alkyl)-, Aryl, Aryl(C₁-C₆-alkyl)- und Heteroaryl(C₁-C₆-alkyl);
R^{18ae} ausgewählt ist aus:
C₁-C₈-Alkyl, gegebenenfalls substituiert mit einer Bindung an Lₙ, C₃-C₁₁-Cycloalkyl, gegebenenfalls substituiert mit einer Bindung an Lₙ, Aryl(C₁-C₆-alkyl)-, gegebenenfalls substituiert mit einer Bindung an Lₙ, Heteroaryl(C₁-C₆-alkyl)-, gegebenenfalls substituiert mit einer Bindung an Lₙ, (C₁-C₆-Alkyl)heteroaryl, gegebenenfalls substituiert mit einer Bindung an Lₙ, Biaryl(C₁-C₆-alkyl), gegebenenfalls substituiert mit einer Bindung an Lₙ, Heteroaryl, gegebenenfalls substituiert mit einer Bindung an Lₙ, Phenyl, substituiert mit 3 - 4 R^{19e} und gegebenenfalls substituiert mit einer Bindung an Lₙ, Naphthyl, substituiert mit 0-4 R^{19e} und gegebenenfalls substituiert mit einer Bindung an Lₙ, und einer Bindung an Lₙ, wobei die Aryl- oder Heteroarylgruppen gegebenenfalls mit 0 - 4 R^{19e} substituiert sind;
R^{18be} H oder R^{18ae} ist,
R^{19e} ausgewählt ist aus:
H, Halogen, CF₃, CO₂H, CN, NO₂, -NR^{11e}R^{12e}, OCF₃, C₁-C₆-Alkyl, C₂-C₆-Alkenyl, C₂-C₆-Alkinyl, C₃-C₆-Cycloalkyl, C₃-C₆-Cycloalkyl(C₁-C₄-alkyl)-, Aryl(C₁-C₄-alkyl)-, C₁-C₆-Alkoxy, C₁-C₄-Alkoxycarbonyl, Aryl, Aryl-O-, Aryl-SO₂-, Heteroaryl und Heteroaryl-SO₂-, wobei die Aryl- und Heteroarylgruppen mit 0 - 4 Gruppen, ausgewählt aus Wasserstoff, Halogen, CF₃, C₁-C₃-Alkyl und C₁-C₃-Alkoxy, substituiert sind;
R^{20e} ausgewählt ist aus:
Hydroxy, C₁-C₆-Alkyloxy, C₃-C₆-Cycloalkyloxy, Aryloxy, Aryl(C₁-C₄-alkyl)oxy, C₂-C₁₀-Alkylcarbonyloxy(C₁-C₂-alkyl)oxy-, C₂-C₁₀-Alkoxycarbonyloxy(C₁-C₂-alkyl)oxy-, C₂-C₁₀-Alkoxycarbonyl(C₁-C₂-alkyl)oxy-, C₃-C₁₀-Cycloalkylcarbonyloxy(C₁-C₂-alkyl)oxy-, C₃-C₁₀-Cycloalkoxycarbonyloxy(C₁-C₂-alkyl)oxy-, C₃-C₁₀-Cycloalkoxycarbonyl(C₁-C₂-alkyl)oxy-, Aryloxycarbonyl(C₁-C₂-alkyl)oxy-, Aryloxycarbonyloxy(C₁-C₂-alkyl)oxy-, Arylcarbonyloxy(C₁-C₂-alkyl)oxy-, C₁-C₅-Alkoxy(C₁-C₅-alkyl)carbonyloxy(C₁-C₂-alkyl)oxy, (5-(C₁-C₅-Alkyl)-1,3-dioxa-cyclopenten-2-on-yl)methyloxy, (5-Aryl-1,3-dioxa-cyclopenten-2-on-yl)methyloxy und (R^{10e})(R^{11e})N-(C₁-C₁₀-Alkoxy)-;
R^{21e} ausgewählt ist aus:
C₁-C₄-Alkyl, C₂-C₆-Alkenyl, C₃-C₆-Cycloalkyl, (C₃-C₆-Cycloalkyl)methyl, Aryl, Aryl(C₁-C₄-alkyl)- und C₁-C₁₀-Alkyl, substituiert mit 0 - 2 R^{7e};
R^{22e} ausgewählt ist aus:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae} und -C(=O)NHSO₂NHR^{18be};
m^{e} 0 - 2 ist;
n^{e} 0 - 4 ist;
p^{e} 0 - 2 ist;
Cₕ 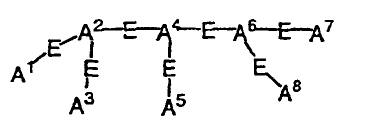 ist;
A¹ ausgewählt ist aus der Gruppe: OH und einer Bindung an Lₙ;
A², A⁴ und A⁶ jeweils N sind;
A³, A⁵ und A⁸ jeweils OH sind;
A⁷ eine Bindung an Lₙ oder eine NH-Bindung an Lₙ ist;
E ein C₂-Alkyl, substituiert mit 0 - 1 R¹⁷, ist;
R¹⁷ =O ist;
alternativ Cₕ 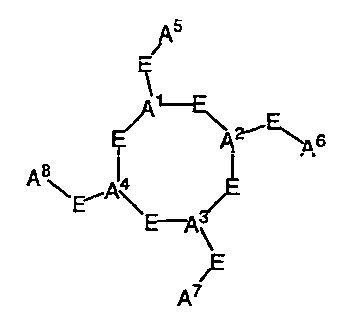 ist;
A¹ ausgewählt ist aus der Gruppe: OH und einer Bindung an Lₙ;
A², A³ und A⁴ jeweils N sind;
A⁵, A⁶ und A⁸ jeweils OH sind;
A⁷ eine Bindung an Lₙ ist;
E ein C₂-Alkyl, substituiert mit 0 - 1 R¹⁷, ist;
R¹⁷ =O ist;
alternativ Cₕ 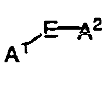 ist;
A¹ NH₂ oder N=C(R²⁰)(R²¹) ist;
E eine Bindung ist;
A² NHR¹³ ist;
R¹³ ein Heterocyclus, substituiert mit R¹⁷, ist, wobei der Heterocyclus aus Pyridin und Pyrimidin ausgewählt ist;
R¹⁷ aus einer Bindung an Lₙ, C(=O)NHR¹⁸ und C(=O)R¹⁸ ausgewählt ist;
R¹⁸ eine Bindung an Lₙ ist;
R²⁴ ausgewählt ist aus der Gruppe: -CO₂R²⁵, -OR²⁵, -SO₃H und -N(R²⁵)₂ und
R²⁵ unabhängig bei jedem Vorkommen ausgewählt ist aus der Gruppe: Wasserstoff und Methyl.

20. Therapeutische radiopharmazeutische Zusammensetzung nach Anspruch 17, wobei Q aus der Gruppe ausgewählt ist:
3-[7-[(Imidazolin-2-ylamino)methyl]-1-methyl-6,8-difluorchinolin-4-on-3-ylcarbonylamino]-2-(3,5-dimethylisoxazol-4-ylsulfonylamino)propionsäure,
3-[7-[(Imidazolin-2-ylamino)methyl]-1-methyl-6,8-difluorchinolin-4-on-3-ylcarbonylamino]-2-(benzyloxycarbonylamino)propionsäure,
3-[7-[(Imidazolin-2-ylamino)methyl]-1-methyl-6,8-difluorchinolin-4-on-3-ylcarbonylamino]-2-(n-butyloxycarbonylamino)propionsäure,
3-[7-[(Imidazolin-2-ylamino)methyl]-1-methyl-6,8-difluorchinolin-4-on-3-ylcarbonylamino]-2-(n-butylsulfonylamino)propionsäure,
3-[7-[(Tetrahydropyrimid-2-ylamino)methyl]-1-methyl-6,8-difluorchinolin-4-on-3-ylcarbonylamino]-2-(benzyloxycarbonylamino)propionsäure,
3-[7-[(Tetrahydropyrimid-2-ylamino)methyl]-1-methyl-6,8-difluorchinolin-4-on-3-ylcarbonylamino]-2-(n-butyloxycarbonylamino)propionsäure,
3-[7-[(Tetrahydropyrimid-2-ylamino)methyl]-1-methyl-6,8-difluorchinolin-4-on-3-ylcarbonylamino]-2-(phenylsulfonylamino)propionsäure,
3-[7-[(Tetrahydropyrimid-2-ylamino)methyl]-1-methyl-6,8-difluorchinolin-4-on-3-ylcarbonylamino]-2-(n-butylsulfonyl)aminopropionsäure,
3-[7-[(2-Aminothiazol-4-yl)methyl]-1-methyl-6,8-difluorchinolin-4-on-3-ylcarbonylamino]-2-(benzyloxycarbonylamino)propionsäure,
3-[7-[(Imidazolin-2-ylamino)methyl]-1-methyl-6,8-difluorchinolin-4-on-3-ylcarbonylamino]-2-((2,4,6-trimethylphenyl)sulfonylamino)propionsäure,
3-[7-[(Tetrahydropyrimid-2-ylamino)methyl]-1-methyl-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-methyl-6,8-difluorchinolin-4-on-3-ylcarbonylamino]-2-(3,5-dimethylisoxazol-4-ylsulfonylamino)propionsäure,
3-[7-[(Imidazol-2-ylamino)methyl]-1-methyl-6,8-difluorchinolin-4-on-3-ylcarbonylamino]-2-(benzyloxycarbonylamino)propionsäure,
3-[7-[(Imidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-methyl-6,8-difluorchinolin-4-on-3-ylcarbonylamino]-2-((4-biphenyl)sulfonylamino)propionsäure,
3-[7-[(Imidazol-2-ylamino)methyl]-1-methyl-6,8-difluorchinolin-4-on-3-ylcarbonylamino]-2-(1-naphthylsulfonylamino)propionsäure,
3-[7-[(Benzimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(4-Methylimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(4,5-Dimethylimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(4,5,6,7-T trahydrobenzimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(Pyridin-2-ylamino)methyl]-1-methyl-6,8-difluorchinolin-4-on-3-ylcarbonylamino]-2-((2,4,6-trimethylphenyl)sulfonylamino)propionsäure,
3-[7-(2-Aminopyridin-6-yl)-1-methyl-6,8-difluorchinolin-4-on-3-ylcarbonylamino]-2-((2,4,6-trimethylphenyl)sulfonylamino)propionsäure,
3-[7-[(7-Azabenzimidazol-2-yl)methyl]-1-methyl-6,8-difluorchinolin-4-on-3-ylcarbonylamino]-2-((2,4,6-trimethylphenyl)sulfonylamino)propionsäure,
3-[7-[(Benzimidazol-2-ylamino)methyl]-1-(2-phenylethyl)-6,8-difluorchinolin-4-on-3-ylcarbonylamino]propionsäure,
3-[7-[(Pyridin-2-ylamino)methyl]-1-(2-phenylethyl)-6,8-difluorchinolin-4-on-3-ylcarbonylamino]propionsäure,
3-[7-[(Imidazolin-2-ylamino)methyl]-1-(2-phenylethyl)-6,8-difluorchinolin-4-on-3-ylcarbonylamino]propionsäure,
3-[7-[(Imidazol-2-ylamino)methyl]-1-(2-phenylethyl)-6,8-difluorchinolin-4-on-3-ylcarbonylamino]propionsäure,
3-[7-[(Imidazolin-2-ylamino)methyl]-1-(2-phenylethyl)-6,8-difluorchinolin-4-on-3-ylcarbonylamino]-2-(benzyloxycarbonylamino)propionsäure,
3-[7-[(Imidazolin-2-ylamino)methyl]-1-(2-phenylethyl)-6,8-difluorchinolin-4-on-3-ylcarbonylamino]-2-(n-butyloxycarbonylamino)propionsäure,
3-[7-[(Imidazolin-2-ylamino)methyl]-1-(2-phenylethyl)-6,8-difluorchinolin-4-on-3-ylcarbonylamino]-2-(phenylsulfonylamino)propionsäure,
3-[7-[(Imidazolin-2-ylamino)methyl]-1-(2-phenylethyl)-6,8-difluorchinolin-4-on-3-ylcarbonylamino]-2-(n-butylsulfonylamino)propionsäure,
3-[7-[(Tetrahydropyrimid-2-ylamino)methyl]-1-(2-phenylethyl)-6,8-difluorchinolin-4-on-3-ylcarbonylamino]-2-(benzyloxycarbonylamino)propionsäure,
3-[7-[(Tetrahydropyrimid-2-ylamino)methyl]-1-(2-phenylethyl)-6,8-difluorchinolin-4-on-3-ylcarbonylamino]-2-(n-butyloxy-carbonylamino)propionsäure,
3-[7-[(Tetrahydropyrimid-2-ylamino)methyl]-1-(2-phenylethyl)-6,8-difluorchinolin-4-on-3-ylcarbonylamino]-2-(phenylsulfonylamino)propionsäure,
3-[7-[(Tetrahydropyrimid-2-ylamino)methyl]-1-(2-phenylethyl)-6,8-difluorchinolin-4-on-3-ylcarbonylamino]-2-(n-butylsulfonyl)aminopropionsäure,
3-[7-[(2-Aminothiazol-4-yl)methyl]-1-(2-phenylethyl)-6,8-difluorchinolin-4-on-3-ylcarbonylamino]-2-(phenylsulfonylamino)propionsäure,
3-[7-[(2-Aminothiazol-4-yl)methyl]-1-(2-phenylethyl)-6,8-difluorchinolin-4-on-3-ylcarbonylamino]-2-(benzyloxycarbonylamino)propionsäure,
3-[7-[(Imidazolin-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(Tetrahydropyrimid-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-(2-phenylethyl)-6,8-difluorchinolin-4-on-3-ylcarbonylamino]-2-(benzyloxycarbonylamino)propionsäure,
3-[7-[(Imidazol-2-ylamino)methyl]-1-(2-phenylethyl)-6,8-difluorchinolin-4-on-3-ylcarbonylamino]-2-(phenylsulfonylamino)propionsäure,
3-[7-[(Imidazol-2-ylamino)methyl]-1-(2-phenylethyl)-6,8-difluorchinolin-4-on-3-ylcarbonylamino]-2-((2,6-dichlorphenyl)sulfonylamino)propionsäure,
3-[7-[(Imidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-(2-phenylethyl)-6,8-difluorchinolin-4-on-3-ylcarbonylamino]-2-((4-biphenyl)sulfonylamino)propionsäure,
3-[7-[(Benzimidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(4-Methylimidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(4,5-Dimethylimidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(4,5,6,7-Tetrahydrobenzimidazol-2-ylamino)methyl]-1-(2-phenylethyl)-6,8-di-fluorchinolin-4-on-3-ylcarbonylamino]-2-((2,4,6-trimethylphenyl)sulfonylamino)-propionsäure,
3-[7-[(Pyridin-2-ylamino)methyl]-1-(2-phenylethyl)-6,8-difluorchinolin-4-on-3-ylcarbonylamino]-2-((2,4,6 trimethylphenyl)sulfonylamino)propionsäure,
3-[7-(2-Aminopyridin-6-yl)-1-(2-phenylethyl)-6,8-difluorchinolin-4-on-3-ylcarbonylamino]-2-((2,4,6-trimethylphenyl)sulfonylamino)propionsäure und
3-[7-[(7-Azabenzimidazol-2-yl)methyl]-1-(2-phenylethyl)-6,8-difluorchinolin-4-on-3-ylcarbonylamino]-2-((2,4,6-trimethylphenyl)sulfonylamino)propionsäure.

21. Zusammensetzung nach Anspruch 15, wobei das Radioisotop ¹⁵³Sm ist.

22. Zusammensetzung nach Anspruch 15, wobei das Radioisotop ¹⁷⁷Lu ist.

23. Zusammensetzung nach Anspruch 22, wobei das Radiopharmazeutikum aus der Gruppe ausgewählt ist: 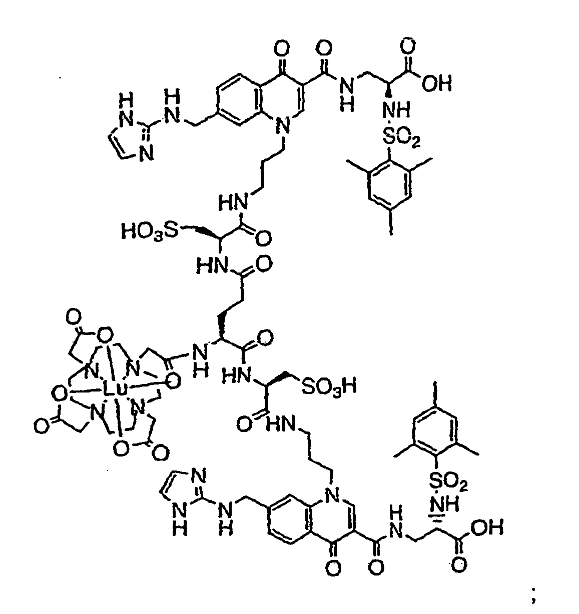 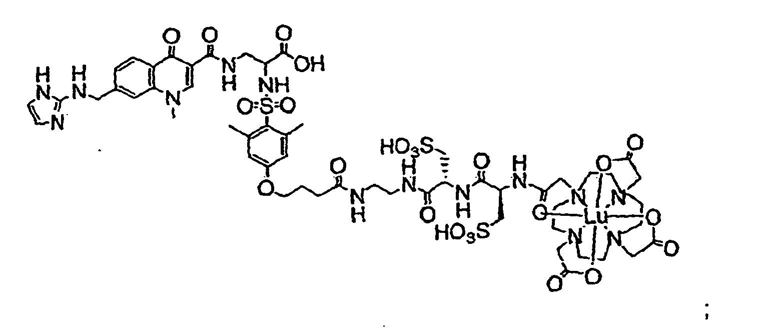 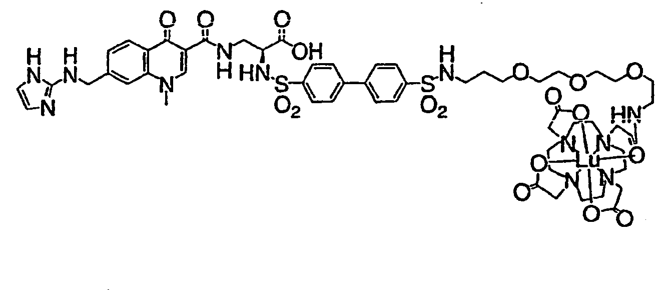 und

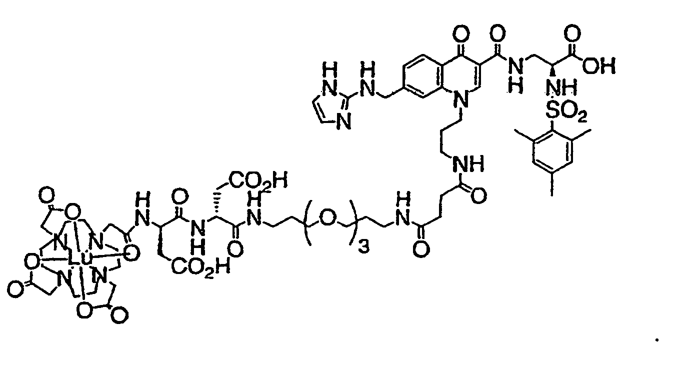

24. Zusammensetzung nach Anspruch 15, wobei das Radioisotop ⁹⁰Y ist.

25. Zusammensetzung nach Anspruch 24, wobei das Radiopharmazeutikum aus der Gruppe ausgewählt ist: 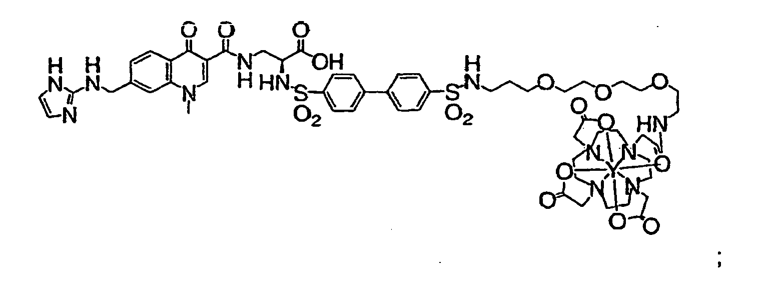 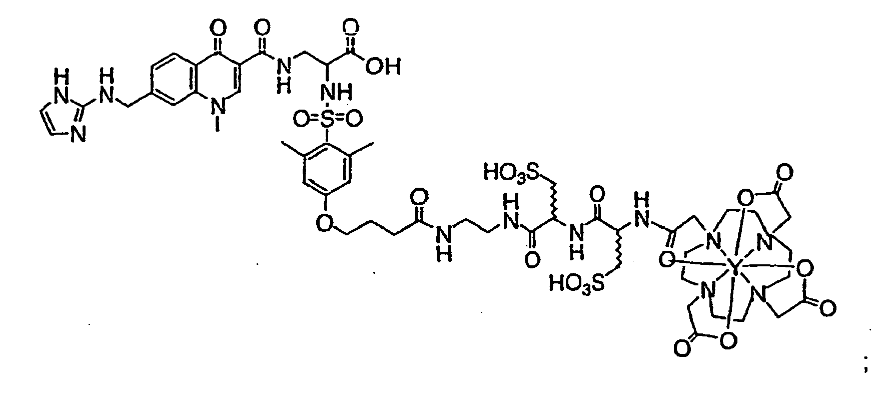 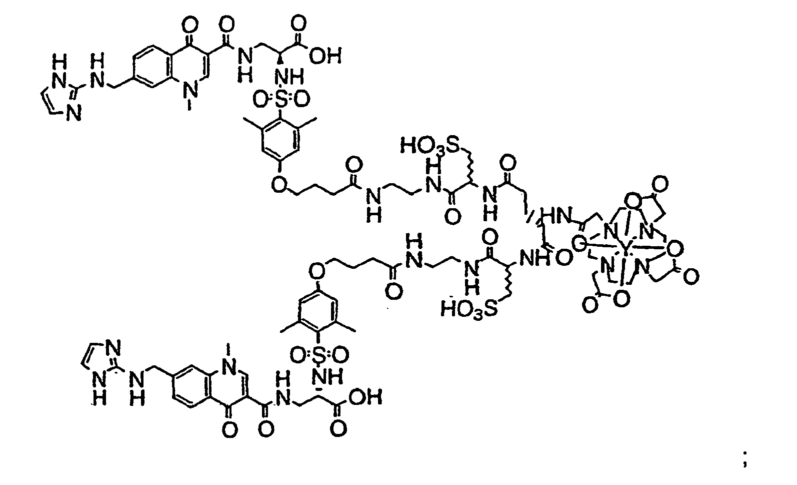 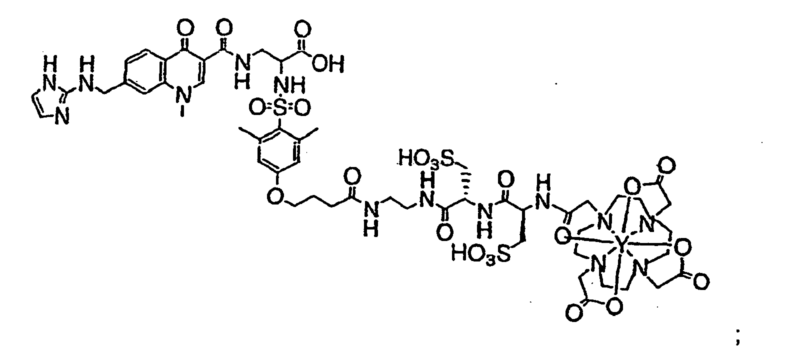 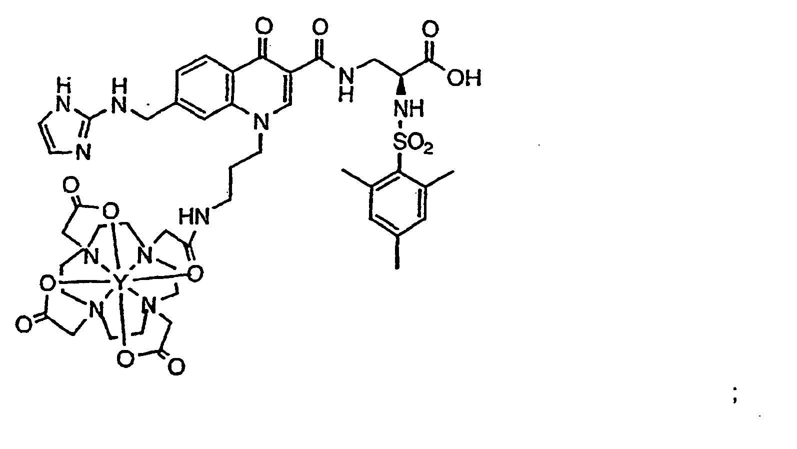 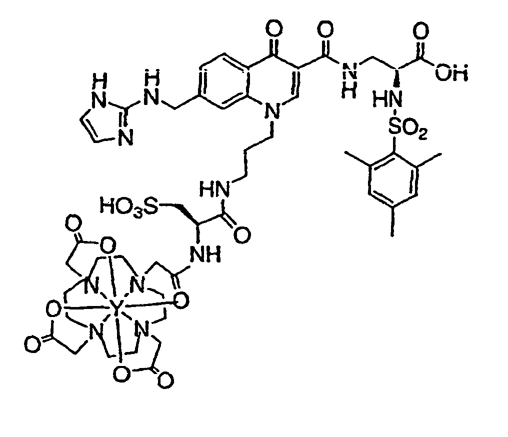 und

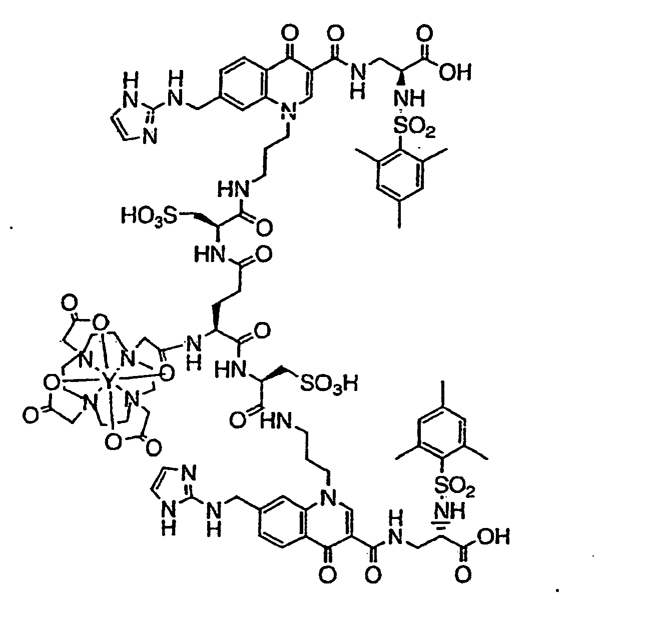

26. Verwendung eines therapeutischen Radiopharmazeutikums, umfassend
a) ein Metall;
b) einen Chelatbildner, der das Metall chelatisieren kann, und
c) eine Targetierungskomponente,
wobei die Targetierungskomponente über eine Verknüpfungsgruppe an den Chelatbildner gebunden ist und die Targetierungskomponente ein Chinolon-Nicht-Peptid ist, das an einen Rezeptor bindet, der während der Angiogenese hochreguliert wird, und das Metall ein Radioisotop, ausgewählt aus der Gruppe: ³³P,¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag und ¹⁹²Ir oder ein pharmazeutisch akzeptables Salz davon ist,
und mindestens ein Mittel, ausgewählt aus der Gruppe, bestehend aus einen Antikrebsmittel und einem Radiosensibilisator, oder ein pharmazeutisch akzeptables Salz davon
zur Herstellung eines Medikaments zur Verabreichung an einen Patienten, der einer solchen bedarf.

27. Verwendung nach Anspruch 26, wobei die Verabreichung des therapeutischen Radiopharmazeutikums und des Mittels gleichzeitig stattfindet.

28. Verwendung nach Anspruch 26, wobei die Verabreichung des therapeutischen Radiopharmazeutikums und des Mittels nacheinander stattfindet.

29. Verwendung nach Anspruch 26, wobei der Krebs ausgewählt ist aus der Gruppe, bestehend aus Karzinomen der Lunge, der Brust, des Eierstocks, des Magens, des Pankreas, des Kehlkopfes, der Speiseröhre, des Hodens, der Leber, der Parotis, der Gallenwege, des Darms, des Mastdarms, der Zervix, des Uterus, des Endometriums, der Niere, der Blase, der Prostata, der Schilddrüse, Plattenepithelkarzinomen, Adenokarzinomen, kleinzelligen Karzinomen, Melanomen, Gliomen und Neuroblastomen.

30. Verwendung nach Anspruch 26, wobei das Antikrebsmittel ausgewählt ist aus der Gruppe, bestehend aus Mitomycin, Tretinoin, Ribomustin, Gemcitabin, Vincristin, Etoposid, Cladribin, Nitobronitol, Methotrexat, Doxorubicin, Carboquon, Pentostatin, Nitracrin, Zinostatin, Cetrorelix, Letrozol, Raltitrexed, Daunorubicin, Fadrozol, Fotemustin, Thymalfasin, Sobuzoxan, Nedaplatin, Cytarabin, Bicalutamid, Vinorelbin, Vesnarinon, Aminoglutethimid, Amsacrin, Proglumid, Elliptiniumacetat, Ketanserin, Doxifluridin, Etretinat, Isotretinoin, Streptozocin, Nimustin, Vindesin, Flutamid, Drogenil, Butocin, Carmofur, Razoxan, Sizofilan, Carboplatin, Mitolactol, Tegafur, Ifosfamid, Prednimustin, Picibanil, Levamisol, Teniposid, Improsulfan, Enocitabin, Lisurid, Oxymetholon, Tamoxifen, Progesteron, Mepitiostan, Epitiostanol, Formestan, Interferon-alpha, Interferon-2-alpha, Interferon-beta, Interferon-gamma, Colony-stimulating-Faktor 1, Colony-stimulating-Faktor 2, Denileukin diftitox, Interleukin-2 und Freisetzungsfaktor für Luteinisierungshormon.

31. Verwendung nach Anspruch 26, wobei der Radiosensibilisator ausgewählt ist aus der Gruppe, bestehend aus 2-(3-Nitro-1,2,4-triazol-1-yl)-N-(2-methoxyethyl)acetamid, N-(3-Nitro-4-chinolinyl)-4-morpholincarboxamidin, 3-Amino-1,2,4-benzotriazin-1,4-dioxid, N-(2-Hydroxyethyl)-2-nitroimidazol-1-acetamid, 1-(2-Nitroimidazol-1-yl)-3-(1-piperidinyl)-2-propanol und 1-(2-Nitro-1-imidazolyl)-3-(1-aziridino)-2-propanol.

32. Verwendung nach Anspruch 26, wobei das Antikrebsmittel ein Chemotherapeutikum ist.

33. Verwendung nach Anspruch 26, wobei die Verabreichung durch Injektion oder Infusion stattfindet.

34. Verwendung nach Anspruch 26, wobei das therapeutische Radiopharmazeutikum:
a) ein Metall;
b) einen Chelatbildner, der das Metall chelatisieren kann, und
c) eine Targetierungskomponente umfaßt;
wobei die Targetierungskomponente über eine Verknüpfungsgruppe an den Chelatbildner gebunden ist und die Targetierungskomponente ein Chinolon-Nicht-Peptid ist, das an einen Rezeptor bindet, der während der Angiogenese hochreguliert wird, und das Metall ein Radioisotop ist, ausgewählt aus der Gruppe: ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag und ¹⁹²Ir.

35. Verwendung nach Anspruch 34, wobei die Targetierungskomponente ein Chino-Ion-Nicht-Peptid und der Rezeptor αᵥβ₃ oder αᵥβ₅ ist.

36. Verwendung nach Anspruch 34, wobei das therapeutische Radiopharmazeutikum
a) ein Radioisotop, ausgewählt aus der Gruppe: ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag und ¹⁹²Ir, und
b) eine Verbindung der Formel (I):
(Q)_{d}-Lₙ-Cₕ oder (Q)_{d}-Lₙ-(Cₕ)_{d'} (I),
worin Q eine Verbindung der Formel (II): 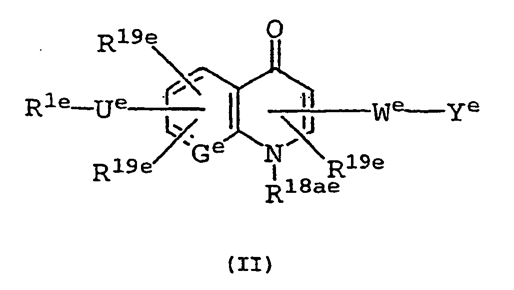 ist,
einschließlich stereoisomere Formen davon oder Gemische stereoisomerer Formen davon oder pharmazeutisch akzeptable Salz- oder Prodrug-Formen davon, worin:
R^{1e} ausgewählt ist aus: 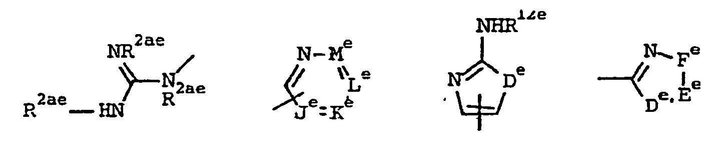 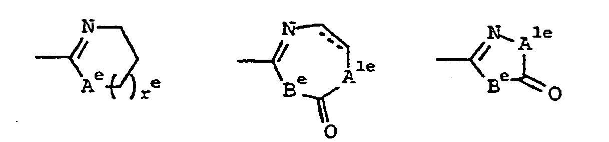 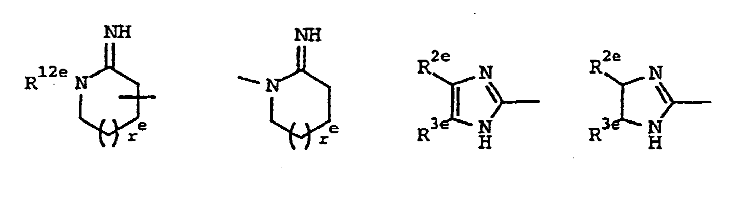 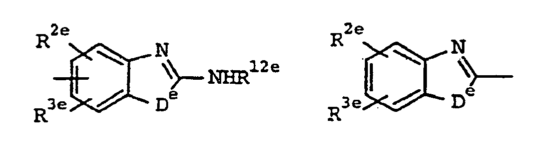 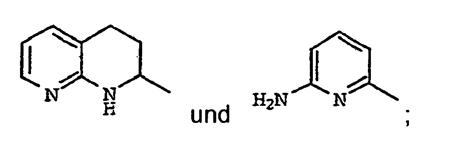
A^{e} -CH₂- oder -N(R^{10e})- ist;
A^{1e} und B^{e} unabhängig voneinander -CH₂- oder -N(R^{10e})- sind;
D^{e} -N(R^{10e})- oder -S- ist;
E^{e}-F^{e} -C(R^{2e})=C(R^{3e})- oder -C(R^{2e} )₂C(R^{3e})₂- ist;
J^{e} -C(R^{2e})- oder -N- ist;
K^{e}, L^{e} und M^{e} unabhängig voneinander -C(R^{2e})- oder -C(R^{3e})- sind;
R^{2e} und R^{3e} unabhängig voneinander ausgewählt sind aus:
H, C₁-C₄-Alkoxy, NR^{11e}R^{12e}, Halogen, NO₂, CN, CF₃, C₁-C₆-Alkyl, C₃-C₆-Alkenyl, C₃-C₇-Cycloalkyl, C₃-C₇-Cycloalkyl(C₁-C₄-alkyl), Aryl(C₁-C₆-alkyl)-, (C₁-C₆-Alkyl)-carbonyl, (C₁-C₆-Alkoxy)carbonyl, Arylcarbonyl und Aryl, substituiert mit 0 - 4 R^{7e},
alternativ, wenn R^{2e} und R^{3e} Substituenten an nachbarständigen Atomen sind, diese mit den Kohlenstoffatomen, an die sie gebunden sind, unter Bildung eines 5- bis 7gliedrigen carbocyclischen oder 5- bis 7gliedrigen heterocyclischen aromatischen oder nicht-aromatischen Ringsystems zusammengenommen werden können, wobei der carbocyclische oder heterocyclische Ring mit 0 - 2 Gruppen, ausgewählt aus C₁-C₄-Alkyl, C₁-C₄-Alkoxy, Halogen, Cyano, Amino, CF₃ und NO₂, substituiert ist;
R^{2ae} ausgewählt ist aus:
H, C₁-C₁₀-Alkyl, C₂-C₆-Alkenyl, C₃-C₁₁-Cycloalkyl, C₃-C₇-Cycloalkyl(C₁-C₄-alkyl), Aryl, Aryl(C₁-C₄-alkyl)-, (C₂-C₇-Alkyl)carbonyl, Arylcarbonyl, (C₂-C₁₀-Alkoxy)carbonyl, C₃-C₇-Cycloalkoxycarbonyl, C₇-C₁₁-Bicycloalkoxycarbonyl, Aryloxycarbonyl, Aryl(C₁-C₁₀-alkoxy)carbonyl, C₁-C₆-Alkylcarbonyloxy(C₁-C₄-alkoxy)carbonyl, Arylcarbonyloxy(C₁-C₄-alkoxy)carbonyl und C₃-C₇-Cycloalkylcarbonyloxy(C₁-C₄-alkoxy)carbonyl;
R^{7e} ausgewählt ist aus:
H, Hydroxy, C₁-C₄-Alkyl, C₁-C₄-Alkoxy, Aryl, Aryl(C₁-C₄-alkyl)-, (C₁-C₄-Alkyl)-Carbonyl, CO₂R^{18ea}, SO₂R^{11e}, SO₂NR^{10e}R^{11e}, OR^{10e} und N(R^{11e})R^{12e};
U^{e} ausgewählt ist aus:
-(CH₂)ₙ^{e}-, -(CH₂)ₙ^{e}O(CH₂)ₘ^{e}-, -(CH₂)ₙ^{e}N(R¹²)(CH₂)ₘ^{e}-, -NH(CH₂)ₙ^{e}-, -(CH₂)ₙ^{e}C(=O)(CH₂)ₘ^{e}-, -(CH₂)ₙ^{e}S(O)ₚ^{e}(CH₂)ₘ^{e}-, -(CH₂)ₙ^{e}NHNH(CH₂)ₘ^{e}-, -N(R^{10e})C(=O)-, -NHC(=O)(CH₂)ₙ^{e}-, -C(=O)N(R^{10e})- und -N(R^{10e})S(O)ₚ^{e}-;
G^{e} N oder CR^{19e} ist;
W^{e} -C(=O)-N(R^{10e})-(C₁-C₃-Alkylen)- ist, worin die Alkylengruppe durch R^{8e} und durch R^{9e} substituiert ist;
R^{8e} und R^{9e} unabhängig voneinander ausgewählt sind aus:
H, CO₂R^{18be}, C(=O)R^{18be}, CONR¹⁷R^{18be},
C₁-C₁₀-Alkyl, substituiert mit 0 - 1 R^{6e},
C₂-C₁₀-Alkenyl, substituiert mit 0 - 1 R^{6e},
C₂-C₁₀-Alkinyl, substituiert mit 0 - 1 R^{6e},
C₃-C₈-Cycloalkyl, substituiert mit 0 - 1 R^{6e},
C₅-C₆-Cycloalkenyl, substituiert mit 0 - 1 R^{6e},
(C₁-C₁₀-Alkyl)carbonyl,
C₃-C₁₀-Cycloalkyl(C₁-C₄-alkyl)-,
Phenyl, substituiert mit 0 - 3 R^{6e},
Naphthyl, substituiert mit 0 - 3 R^{6e},
einem 5- bis 10gliedrigen heterocyclischen Ring, enthaltend 1 - 3 N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist,
C₁-C₁₀-Alkoxy, substituiert mit 0 - 2 R^{7e},
Hydroxy, Nitro, -N(R^{10e})R^{11e}, -N(R^{16e})R^{17e}, Aryl(C₀-C₆-alkyl)carbonyl, Aryl(C₃-C₆-alkyl), Heteroaryl(C₁-C₆-alkyl), CONR^{18ae}R^{20e}, SO₂R^{18ae} und SO₂NR^{18ea}R^{20e},
mit der Maßgabe, daß jede der obigen Alkyl-, Cycloalkyl-, Aryl- oder Heteroaryl-gruppen unsubstituiert oder unabhängig mit 1 - 2 R^{7e} substituiert sein kann;
R^{6e} ausgewählt ist aus:
H, C₁-C₁₀-Alkyl, Hydroxy, C₁-C₁₀-Alkoxy, Nitro, C₁-C₁₀-Alkylcarbonyl, -N(R^{11e})R^{12e}, Cyano, Halogen, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
Aryl, substituiert mit 0 - 3 Gruppen, ausgewählt aus Halogen, C₁-C₆-Alkoxy, C₁-C₆-Alkyl, CF₃, S(O)ₘ^{e}Me und -NMe₂,
Aryl(C₁-C₄-alkyl)-, wobei das Aryl mit 0 - 3 Gruppen, ausgewählt aus Halogen, C₁-C₆-Alkoxy, C₁-C₆-Alkyl, CF₃, S(O)ₚ^{e}Me und -NMe₂, substituiert ist;
und einem 5- bis 10gliedrigen heterocyclischen Ring, enthaltend 1 - 3 N-, O-oder S-Heteroatome, wobei der heterocyclische Ring gesättigt, teilweise gesättigt oder vollständig ungesättigt sein kann, wobei der heterocyclische Ring mit 0 - 2 R^{7e} substituiert ist;
R^{10e} ausgewählt ist aus:
H, -OH, CF₃, C₃-C₆-Alkenyl, C₃-C₁₁-Cycloalkyl, Aryl, (C₃-C₁₁-Cycloalkyl)methyl, Aryl(C₁-C₄-alkyl) und C₁-C₁₀-Alkyl, substituiert mit 0 - 2 R^{6e};
R^{11e} ausgewählt ist aus:
H, Hydroxy, C₁-C₈-Alkyl, C₃-C₆-Alkenyl, C₃-C₁₁-Cycloalkyl, (C₃-C₁₁-Cycloalkyl)-methyl, C₁-C₆-Alkoxy, Benzyloxy, Aryl, Heteroaryl, Heteroaryl(C₁-C₄-alkyl)-, Aryl-(C₁-C₄-alkyl), Adamantylmethyl und C₁-C₁₀-Alkyl, substituiert mit 0 - 2 R^{4e};
R^{4e} ausgewählt ist aus:
H, C₁-C₆-Alkyl, C₃-C₇-Cycloalkyl, C₃-C₇-Cycloalkyl(C₁-C₄-alkyl)-, (C₁-C₁₀-Alkyl)-carbonyl, Aryl, Heteroaryl, Aryl(C₁-C₆-alkyl)- und Heteroaryl(C₁-C₆-alkyl)-, wobei die Aryl- oder Heteroarylgruppen mit 0 - 2 Substituenten, unabhängig ausgewählt aus der Gruppe, bestehend aus C₁-C₄-Alkyl, C₁-C₄-Alkoxy, F, Cl, Br, CF₃ und NO₂, substituiert sind,
alternativ, wenn R^{10e} und R^{11e} beide Substituenten an demselben Stickstoffatom sind (wie in -NR^{10e}R^{11e}), diese mit dem Stickstoffatom, an das sie gebunden sind, unter Bildung eines Heterocyclus, ausgewählt aus: 3-Azabicyclononyl, 1,2,3,4-Tetrahydro-1-chinolinyl, 1,2,3,4-Tetrahydro-2-isochinolinyl, 1-Piperidinyl, 1-Morpholinyl,1-Pyrrolidinyl, Thiamorpholinyl, Thiazolidinyl und 1-Piperazinyl, zusammengenommen werden können;
wobei der Heterocyclus mit 0 - 3 Gruppen, ausgewählt aus: C₁-C₆-Alkyl, Aryl, Heteroaryl, Aryl(C₁-C₄-alkyl)-, (C₁-C₆-Alkyl)carbonyl, (C₃-C₇-Cycloalkyl)carbonyl, (C₁-C₆-Alkoxy)carbonyl, Aryl(C₁-C₄-alkoxy)carbonyl, C₁-C₆-Alkylsulfonyl und Arylsulfonyl, substituiert ist;
R^{12e} ausgewählt ist aus:
H, C₁-C₆-Alkyl, Triphenylmethyl, Methoxymethyl, Methoxyphenyldiphenylmethyl, Trimethylsilylethoxymethyl, (C₁-C₆-Alkyl)carbonyl, (C₁-C₆-Alkoxy)carbonyl, (C₁-C₆-Alkyl)aminocarbonyl, C₃-C₆-Alkenyl, C₃-C₇-Cycloalkyl, C₃-C₇-Cycloalkyl-(C₁-C₄-alkyl)-, Aryl, Heteroaryl(C₁-C₆-alkyl)carbonyl, Heteroarylcarbonyl, Aryl-(C₁-C₆-alkyl)-, (C₁-C₆-Alkyl)carbonyl, Arylcarbonyl, C₁-C₆-Alkylsulfonyl, Arylsulfonyl, Aryl(C₁-C₆-alkyl)sulfonyl, Heteroarylsulfonyl, Heteroaryl(C₁-C₆-alkyl)sulfonyl, Aryloxycarbonyl und Aryl(C₁-C₆-alkoxy)carbonyl, wobei die Arylgruppen mit 0 - 2 Substituenten, ausgewählt aus der Gruppe, bestehend aus C₁-C₄-Alkyl, C₁-C₄-Alkoxy, Halogen, CF₃ und Nitro, substituiert sind;
R^{16e} ausgewählt ist aus:
-C(=O)OR^{18ae}, -C(=O)R^{18be}, -C(=O)N(R^{18be})₂, -C(=O)NHSO₂R^{18ae},
-C(=O)NHC(=O)R^{18be}, -C(=O)NHC(=O)OR^{18ae}, -C(=O)NHSO₂NHR^{18be},
-SO₂R^{18ae}, -SO₂N(R^{18be})₂ und -SO₂NHC(=O)OR^{18be},
R^{17e} ausgewählt ist aus:
H, C₁-C₆-Alkyl, C₃-C₇-Cycloalkyl, C₃-C₇-Cycloalkyl(C₁-C₄-alkyl)-, Aryl, Aryl(C₁-C₆-alkyl)- und Heteroaryl(C₁-C₆-alkyl);
R^{18ae} ausgewählt ist aus:
C₁-C₈-Alkyl, gegebenenfalls substituiert mit einer Bindung an Lₙ, C₃-C₁₁-Cycloalkyl, gegebenenfalls substituiert mit einer Bindung an Lₙ, Aryl(C₁-C₆-alkyl)-, gegebenenfalls substituiert mit einer Bindung an Lₙ, Heteroaryl(C₁-C₆-alkyl)-, gegebenenfalls substituiert mit einer Bindung an Lₙ, (C₁-C₆-Alkyl)heteroaryl, gegebenenfalls substituiert mit einer Bindung an Lₙ, Biaryl(C₁-C₆-alkyl), gegebenenfalls substituiert mit einer Bindung an Lₙ, Heteroaryl, gegebenenfalls substituiert mit einer Bindung an Lₙ, Phenyl, substituiert mit 3 - 4 R^{19e} und gegebenenfalls substituiert mit einer Bindung an Lₙ, Naphthyl, substituiert mit 0-4 R^{19e} und gegebenenfalls substituiert mit einer Bindung an Lₙ, und einer Bindung an Lₙ, wobei die Aryl- oder Heteroarylgruppen gegebenenfalls mit 0 - 4 R^{19e} substituiert sind;
R^{18be} H oder R^{18ae} ist,
R^{19e} ausgewählt ist aus:
H, Halogen, CF₃, CO₂H, CN, NO₂, -NR^{11e}R^{12e}, OCF₃, C₁-C₈-Alkyl, C₂-C₆-Alkenyl, C₂-C₆-Alkinyl, C₃-C₁₁-Cycloalkyl, C₃-C₇-Cycloalkyl(C₁-C₄-alkyl)-, Aryl(C₁-C₆-alkyl)-, C₁-C₆-Alkoxy, C₁-C₄-Alkoxycarbonyl, Aryl, Aryl-O-, Aryl-SO₂-, Heteroaryl und Heteroaryl-SO₂-, wobei die Aryl- und Heteroarylgruppen mit 0 - 4 Gruppen, ausgewählt aus Wasserstoff, Halogen, CF₃, C₁-C₃-Alkyl und C₁-C₃-Alkoxy, substituiert sind;
R^{20e} ausgewählt ist aus:
Hydroxy, C₁-C₁₀-Alkyloxy, C₃-C₁₁-Cycloalkyloxy, Aryloxy, Aryl(C₁-C₄-alkyl)oxy, C₂-C₁₀-Alkylcarbonyloxy(C₁-C₂-alkyl)oxy-, C₂-C₁₀-Alkoxycarbonyloxy(C₁-C₂-alkyl)oxy-, C₂-C₁₀-Alkoxycarbonyl(C₁-C₂-alkyl)oxy-, C₃-C₁₀-Cycloalkylcarbonyloxy(C₁-C₂-alkyl)oxy-, C₃-C₁₀-Cycloalkoxycarbonyloxy(C₁-C₂alkyl)oxy-, C₃-C₁₀-Cycloalkoxycarbonyl(C₁-C₂-alkyl)oxy-, Aryloxycarbonyl(C₁-C₂-alkyl)oxy-, Aryloxycarbonyloxy(C₁-C₂-alkyl)oxy-, Arylcarbonyloxy(C₁-C₂-alkyl)oxy-, C₁-C₅-Alkoxy(C₁-C₅-alkyl)carbonyloxy(C₁-C₂-alkyl)oxy, (5-(C₁-C₅-Alkyl)-1,3-dioxa-cyclopenten-2-on-yl)methyloxy, (5-Aryl-1,3-dioxa-cyclopenten-2-on-yl)methyloxy und (R^{10e})(R^{11e})N-(C₁-C₁₀-Alkoxy)-;
R^{21e} ausgewählt ist aus:
C₁-C₈-Alkyl, C₂-C₆-Alkenyl, C₃-C₁₁-Cycloalkyl, (C₃-C₁₁-Cycloalkyl)methyl, Aryl, Aryl(C₁-C₄-alkyl)- und C₁-C₁₀-Alkyl, substituiert mit 0 - 2 R^{7e};
R^{22e} ausgewählt ist aus:
-C(=O)-R^{18be}, -C(=O)N(R^{18be})₂, -C(=O)NHSO₂R^{18ae}, -C(=O)NHC(=O)R^{18be},
-C(=O)NHC(=O)OR^{18ae} und -C(=O)NHSO₂NHR^{18be};
Y^{e} ausgewählt ist aus:
-COR^{20e}, -SO₃H, -PO₃H, -CONHNHSO₂CF₃, -CONHSO₂R^{18ae},
-CONHSO₂NHR^{18be}, -NHCOCF₃, -NHCONHSO₂R^{18ae}, -NHSO₂R^{18ae}, -OPO₃H₂, -OSO₃H, -PO₃H₂, -SO₂NHCOR^{18ae}, -SO₂NHCO₂R^{18ae}, 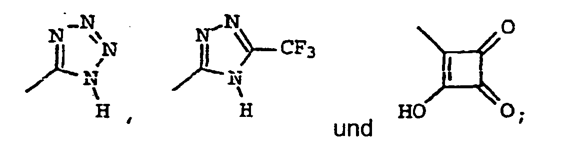
m^{e} 0 - 2 ist;
n^{e} 0 - 4 ist;
p^{e} 0 - 2 ist;
r^{e} 0 - 2 ist;
mit der folgenden Maßgabe: n^{e} und m^{e} sind so gewählt, daß die Anzahl an Atomen, die R^{1e} und Y^{e} verbinden, im Bereich von 8 - 14 liegt;
d ausgewählt ist aus 1, 2, 3, 4, 5, 6, 7, 8, 9 und 10;
d' 1 - 100 ist;
Lₙ eine Verknüpfungsgruppe mit der Formel:
((W)ₕ-(CR⁶R⁷)_{g})ₓ-(Z)ₖ-((CR^{6a}R^{7a})_{g'}-(W)_{h'})_{x'}
ist;
W unabhängig bei jedem Vorkommen aus der Gruppe: O, S, NH, NHC(=O), C(=O)NH, NR⁸C(=O), C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ und (aa)_{t'} ausgewählt ist;
aa unabhängig bei jedem Vorkommen eine Aminosäure ist;
Z ausgewählt ist aus der Gruppe: Aryl, substituiert mit 0-3R¹⁰, C₃₋₁₀-Cycloalkyl, substituiert mit 0-3R¹⁰, und einem 5- bis 10gliedrigen heterocyclischen Ringsystem, enthaltend 1-4Heteroatome, unabhängig ausgewählt aus N, S und O und substituiert mit 0-3R¹⁰;
R⁶, R^{6a}, R⁷, R^{7a} und R⁸ unabhängig voneinander bei jedem Vorkommen aus der Gruppe: H, =O, COOH, SO₃H, PO₃H, C₁-C₅-Alkyl, substituiert mit 0-3R¹⁰, Aryl, substituiert mit 0-3R¹⁰, Benzyl, substituiert mit 0-3R¹⁰, und C₁-C₅-Alkoxy, substituiert mit 0-3R¹⁰, NHC(=O)R¹¹, C(=O)NHR¹¹, NHC(=O)NHR¹¹, NHR¹¹, R¹¹ und einer Bindung an Cₕ ausgewählt sind;
R¹⁰ unabhängig bei jedem Vorkommen aus der Gruppe: einer Bindung an Cₕ, COOR¹¹, C(=O)NHR¹¹, NHC(=O)R¹¹, OH, NHR¹¹, SO₃H, PO₃H, -OPO₃H₂, -OSO₃H, Aryl, substituiert mit 0-3R¹¹, C₁₋₅-Alkyl, substituiert mit 0-1R¹², C₁₋₅-Alkoxy, substituiert mit 0-1R¹², und einem 5- bis 10gliedrigen heterocyclischen Ringsystem, enthaltend 1-4Heteroatome, unabhängig ausgewählt aus N, S und O und substituiert mit 0-3R¹¹, ausgewählt ist;
R¹¹ unabhängig bei jedem Vorkommen aus der Gruppe: H, -OPO₃H₂, -OSO₃H, C₁₋₅-Alkyl, substituiert mit 0-1R¹², Aryl, substituiert mit 0-1R¹² , einem 5-bis 10gliedrigen heterocyclischen Ringsystem, enthaltend 1-4Heteroatome, unabhängig ausgewählt aus N, S und O und substituiert mit 0-1R¹², C₃-₁₀-Cycloalkyl, substituiert mit 0-1R¹², Polyalkylenglycol, substituiert mit 0-1R¹², C₁₋₅-Alkyl, substituiert mit einem Kohlenhydrat, substituiert mit 0-1R¹², Cyclodextrin, substituiert mit 0-1R¹², Aminosäure, substituiert mit 0-1R¹², Polycarboxyalkyl, substituiert mit 0-1R¹², Polyazaalkyl, substituiert mit 0-1R¹², Peptid, substituiert mit 0-1R¹², wobei das Peptid aus 2 - 10 Aminosäuren besteht, 3,6-O-Disulfo-B-D-galactopyranosyl, Bis(phosphonomethyl)glycin und einer Bindung an Cₕ, ausgewählt ist;
R¹² eine Bindung an Cₕ ist;
k ausgewählt ist aus 0, 1 und 2;
h ausgewählt ist aus 0,1 und 2;
h' ausgewählt ist aus 0,1 und 2;
g ausgewählt ist aus 0,1, 2, 3, 4, 5, 6, 7, 8, 9 und 10;
g' ausgewählt ist aus 0, 1, 2, 3, 4, 5, 6, 7, 8, 9 und 10;
s ausgewählt ist aus 0, 1, 2, 3, 4, 5, 6, 7, 8, 9 und 10;
s' ausgewählt ist aus 0, 1, 2, 3, 4, 5, 6, 7, 8, 9 und 10;
s" ausgewählt ist aus 0, 1, 2, 3, 4, 5, 6, 7, 8, 9 und 10;
t ausgewählt ist aus 0, 1, 2, 3, 4, 5, 6, 7, 8, 9 und 10;
t' ausgewählt ist aus 0, 1, 2, 3, 4, 5, 6, 7, 8, 9 und 10;
x ausgewählt ist aus 0, 1, 2, 3, 4, 5 und 6;
x' ausgewählt ist aus 0, 1, 2, 3, 4, 5 und 6;
Cₕ eine Metallbindungseinheit mit einer Formel, ausgewählt aus der Gruppe: 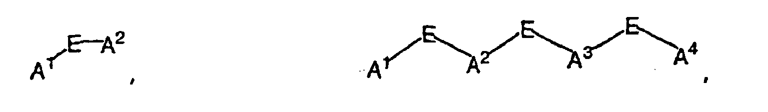 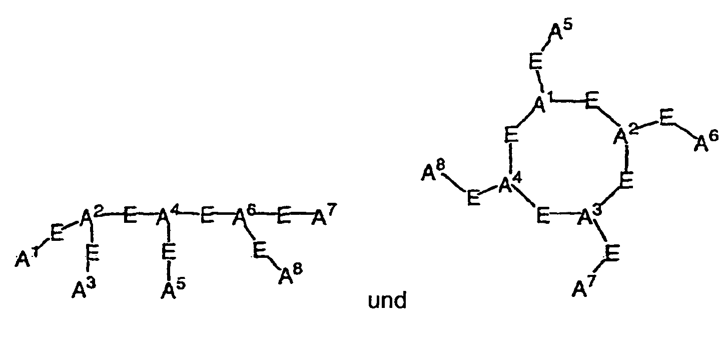 ist;
A¹, A², A³, A⁴, A⁵, A⁶, A⁷ und A⁸ unabhängig voneinander bei jedem Vorkommen aus der Gruppe: NR¹³, NR¹³R¹⁴, S, SH, S(Pg), O, OH, PR¹³, PR¹³R¹⁴, P(O)R¹⁵R¹⁶ und einer Bindung an Lₙ ausgewählt sind;
E eine Bindung, CH oder eine Spacer-Gruppe ist, unabhängig bei jedem Vorkommen ausgewählt aus der Gruppe: C₁-C₁₀-Alkyl, substituiert mit 0-3R¹⁷, Aryl, substituiert mit 0-3R¹⁷, C₃₋₁₀-Cycloalkyl, substituiert mit 0-3R¹⁷, Heterocyclo-C₁-₁₀-alkyl, substituiert mit 0-3R¹⁷, wobei die Heterocyclogruppe ein 5- bis 10gliedriges heterocyclisches Ringsystem ist, enthaltend 1 - 4 Heteroatome, unabhängig ausgewählt aus N, S und O, C₆₋₁₀-Aryl-C₁₋₁₀-alkyl, substituiert mit 0-3 R¹⁷, C₁₋₁₀-Alkyl-C₆₋₁₀-aryl-, substituiert mit 0-3R¹⁷, und einem 5-bis 10gliedrigen heterocyclischen Ringsystem, enthaltend 1-4Heteroatome, unabhängig ausgewählt aus N, S und O und substituiert mit 0-3R¹⁷;
R¹³ und R¹⁴ jeweils unabhängig voneinander ausgewählt sind aus der Gruppe: einer Bindung an Lₙ, Wasserstoff, C₁-C₁₀-Alkyl, substituiert mit 0-3R¹⁷, Aryl, substituiert mit 0-3R¹⁷, C₁₋₁₀-Cycloalkyl, substituiert mit 0-3R¹⁷, Heterocyclo-C₁₋₁₀-alkyl, substituiert mit 0-3R¹⁷, wobei die Heterocyclogruppe ein 5- bis 10gliedriges heterocyclisches Ringsystem ist, enthaltend 1-4Heteroatom, unabhängig ausgewählt aus N, S und O, C₆₋₁₀-Aryl-C₁₋₁₀-alkyl, substituiert mit 0-3R¹⁷, C₁₋₁₀-Alkyl-C₆₋₁₀-aryl, substituiert mit 0-3R¹⁷, einem 5- bis 10gliedrigen heterocyclischen Ringsystem, enthaltend 1-4Heteroatome, unabhängig ausgewählt aus N, S und O und substituiert mit 0-3R¹⁷, und einem Elektron, vorausgesetzt, daß, wenn einer von R¹³ oder R¹⁴ ein Elektron ist, der andere auch ein Elektron ist;
alternativ R¹³ und R¹⁴ unter Bildung von =C(R²⁰)(R²¹) verbunden sind;
R¹⁵ und R¹⁶ jeweils unabhängig voneinander ausgewählt sind aus der Gruppe: einer Bindung an Lₙ, -OH, C₁-C₁₀-Alkyl, substituiert mit 0-3R¹⁷, C₁-C₁₀-Alkyl, substituiert mit 0-3R¹⁷, Aryl, substituiert mit 0-3R¹⁷, C₃₋₁₀-Cycloalkyl, substituiert mit 0-3R¹⁷, Heterocyclo-C₁₋₁₀-alkyl, substituiert mit 0-3R¹⁷, wobei die Heterocyclogruppe ein 5- bis 10gliedriges heterocyclisches Ringsystem ist, enthaltend 1-4 Heteroatome, unabhängig ausgewählt aus N, S und O, C₆₋₁₀-Aryl-C₁₋₁₀-alkyl, substituiert mit 0-3R¹⁷, C₁₋₁₀-Alkyl-C₆₋₁₀-aryl-, substituiert mit 0-3R¹⁷, und einem 5- bis 10gliedrigen heterocyclischen Ringsystem, enthaltend 1 - 4 Heteroatome, unabhängig ausgewählt aus N, S und O und substituiert mit 0-3R¹⁷;
R¹⁷ unabhängig bei jedem Vorkommen ausgewählt ist aus der Gruppe: einer Bindung an Lₙ, =O, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H, 2-(1-Morpholino)ethoxy, C₁-C₅-Alkyl, C₂-C₄-Alkenyl, C₃-C₆-Cycloalkyl, C₃-C₆-Cycloalkylmethyl, C₂-C₆-Alkoxyalkyl, Aryl, substituiert mit 0-2R¹⁸, und einem 5- bis 10gliedrigen heterocyclischen Ringsystem, enthaltend 1-4Heteroatome, unabhängig ausgewählt aus N, S und O;
R¹⁸, R^{18a} und R¹⁹ unabhängig voneinander bei jedem Vorkommen ausgewählt sind aus der Gruppe: einer Bindung an Lₙ, H, C₁-C₆-Alkyl, Phenyl, Benzyl, C₁-C₆-Alkoxy, Halogenid, Nitro, Cyano und Trifluormethyl;
Pg eine Thiol-Schutzgruppe ist;
R²⁰ und R²¹ unabhängig voneinander ausgewählt sind aus der Gruppe: H, C₁-C₁₀-Alkyl, -CN, -CO₂R²⁵, -C(=O)R²⁵, -C(=O)N(R²⁵)₂, C₂-C₁₀-1-Alken, substituiert mit 0-3R²³, C₂-C₁₀-1-Alkin, substituiert mit 0-3R²³, Aryl, substituiert mit 0-3R²³, einem ungesättigten 5- bis 10gliedrigen heterocyclischen Ringsystem, enthaltend 1-4Heteroatome, unabhängig ausgewählt aus N, S und O und substituiert mit 0-3R²³, und einem ungesättigten C₃₋₁₀-Carbocyclus, substituiert mit 0-3R²³;
alternativ R²⁰ und R²¹ zusammen mit dem zweiwertigen Kohlenstoffrest, an den sie gebunden sind: 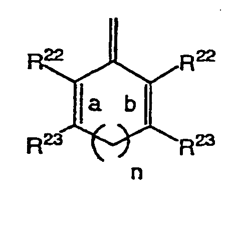 bilden;
R²² und R²³ unabhängig voneinander ausgewählt sind aus der Gruppe:
H, R²⁴, C₁-C₁₀-Alkyl, substituiert mit 0-3R²⁴, C₂-C₁₀-Alkenyl, substituiert mit 0-3 R²⁴, C₂-C₁₀-Alkinyl, substituiert mit 0-3R²⁴, Aryl, substituiert mit 0-3R²⁴, einem 5- bis 10gliedrigen heterocyclischen Ringsystem, enthaltend 1-4Heteroatome, unabhängig ausgewählt aus N, S und O und substituiert mit 0 - 3 R²⁴, und einem C₃₋₁₀-Carbocyclus, substituiert mit 0-3R²⁴;
alternativ R²², R²³ zusammen ein kondensiertes aromatisches oder ein 5- bis 10gliedriges heterocyclisches Ringsystem, enthaltend 1 - 4 Heteroatome, unabhängig ausgewählt aus N, S und O, bilden;
a und b die Positionen optionaler Doppelbindungen anzeigen und n 0 oder 1 ist;
R²⁴ unabhängig bei jedem Vorkommen ausgewählt ist aus der Gruppe: =O, F, Cl, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H und 2-(1-Morpholino)ethoxy; und
R²⁵, R^{25a} und R²⁶ jeweils unabhängig voneinander bei jedem Vorkommen ausgewählt sind aus der Gruppe: Wasserstoff und C₁-C₆-Alkyl; oder ein pharmazeutisch akzeptables Salz davon umfaßt.

37. Verwendung nach Anspruch 36, wobei:
Q eine Verbindung der Formel (IV): 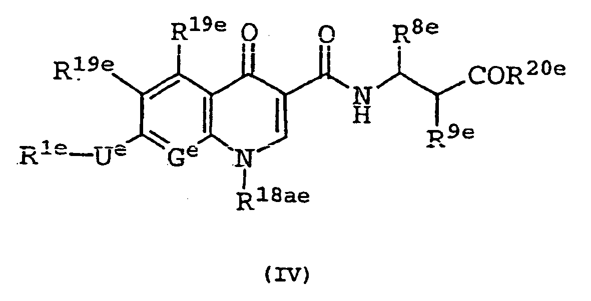 ist,
einschließlich stereoisomere Formen davon oder Gemische stereoisomerer Formen davon oder pharmazeutisch akzeptable Salz- oder Prodrug-Formen davon, worin:
R^{1e} ausgewählt ist aus: 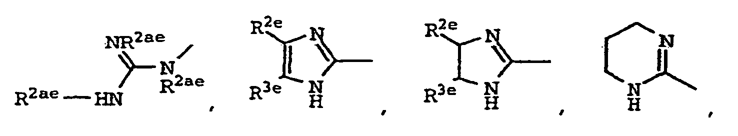 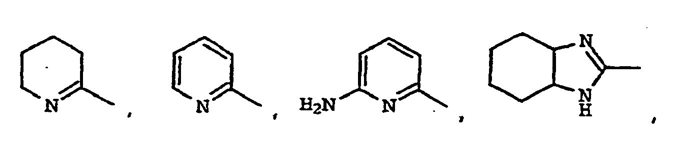 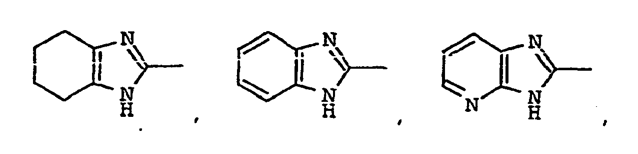 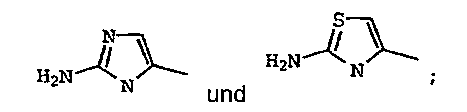
R^{2e} und R^{3e} unabhängig voneinander ausgewählt sind aus:
H, C₁-C₄-Alkoxy, NR^{11e}R^{12e}, Halogen, NO₂, CN, CF₃, C₁-C₆-Alkyl, C₃-C₆-Alkenyl, C₃-C₇-Cycloalkyl, C₃-C₇-Cycloalkyl(C₁-C₄-alkyl), Aryl(C₁-C₆-alkyl)-, (C₁-C₆-Alkyl)-carbonyl, (C₁-C₆-Alkoxy)carbonyl, Arylcarbonyl und Aryl, substituiert mit 0 - 4 R^{7e},
alternativ, wenn R^{2e} und R^{3e} Substituenten an nachbarständigen Atomen sind, diese mit den Kohlenstoffatomen, an die sie gebunden sind, unter Bildung eines 5- bis 7gliedrigen carbocyclischen oder 5- bis 7gliedrigen heterocyclischen aromatischen oder nicht-aromatischen Ringsystems zusammengenommen werden können, wobei der carbocyclische oder heterocyclische Ring mit 0-2Gruppen, ausgewählt aus C₁-C₄-Alkyl, C₁-C₄-Alkoxy, Halogen, Cyano, Amino, CF₃ und NO₂, substituiert ist;
R^{2ae} ausgewählt ist aus:
H, C₁-C₁₀-Alkyl, C₂-C₆-Alkenyl, C₃-C₁₁-Cycloalkyl, C₃-C₇-Cycloalkyl(C₁-C₄-alkyl), Aryl, Aryl(C₁-C₄-alkyl)-, (C₂-C₇-Alkyl)carbonyl, Arylcarbonyl, (C₂-C₁₀-Alkoxy)carbonyl, C₃-C₇-Cycloalkoxycarbonyl, C₇-C₁₁-Bicycloalkoxycarbonyl, Aryloxycarbonyl, Aryl(C₁-C₁₀-alkoxy)carbonyl, C₁-C₆-Alkylcarbonyloxy(C₁-C₄-alkoxy)carbonyl, Arylcarbonyloxy(C₁-C₄-alkoxy)carbonyl und C₃-C₇Cycloalkylcarbonyloxy(C₁-C₄-alkoxy)carbonyl;
R^{7e} ausgewählt ist aus:
H, Hydroxy, C₁-C₄-Alkyl, C₁-C₄-Alkoxy, Aryl, Aryl(C₁-C₄-alkyl)-, (C₁-C₄-Alkyl)-carbonyl, CO₂R^{18ae}, SO₂R^{11e}, SO₂NR^{10e}R^{11e}, OR^{10e} und N(R^{11e})R^{12e};
U^{e} ausgewählt ist aus:
-(CH₂)ₙ^{e}-, -(CH₂)ₙ^{e}O(CH₂)m^{e}-, -NH(CH₂)ₙ^{e}-, -N(R^{10e})C(=O)-, -NHC(=O)(CH₂)ₙ^{e}-und -C(=O)N(R^{10e})-;
G^{e} N oder CR^{19e} ist;
R^{8e} ausgewählt ist aus:
H, CO₂R^{18be}, C(=O)R^{18be}, CONR^{17e}R^{18be},
C₁-C₁₀-Alkyl, substituiert mit 0-1R^{6e},
C₂-C₁₀-Alkenyl, substituiert mit 0-1R^{6e},
C₂-C₁₀-Alkinyl, substituiert mit 0-1R^{6e},
C₃-C₈-Cycloalkyl, substituiert mit 0-1R^{6e},
C₅-C₆-Cycloalkenyl, substituiert mit 0-1R^{6e},
(C₁-C₁₀-Alkyl)carbonyl,
C₃-C₁₀-Cycloalkyl(C₁-C₄-alkyl)-,
Phenyl, substituiert mit 0-3R^{6e},
Naphthyl, substituiert mit 0-3R^{6e},
einem 5-bis 10gliedrigen heterocyclischen Ring, enthaltend 1-3N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist;
R^{9e} ausgewählt ist aus:
C₁-C₁₀-Alkyl, substituiert mit 0-1R^{6e},
C₁-C₁₀-Alkoxy, substituiert mit 0-2R^{7e},
H, Nitro, -N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, Hydroxy, OR^{22e}, -N(R^{10e})R^{11e}, -N(R^{16e})R^{17e}, Aryl(C₀-C₆-alkyl)carbonyl, Aryl-(C₁-C₆-alkyl), Heteroaryl(C₁-C₆ alkyl), CONR^{18ae}R^{20e}, SO₂R^{18ae} und SO₂NR^{18ae}R^{20e},
mit der Maßgabe, daß jede der obigen Alkyl-, Cycloalkyl-, Aryl- oder Heteroarylgruppen unsubstituiert oder unabhängig mit 1-2R^{7e} substituiert sein kann;
R^{6e} ausgewählt ist aus:
H, C₁-C₁₀-Alkyl, Hydroxy, C₁-C₁₀-Alkoxy, Nitro, C₁-C₁₀-Alkylcarbonyl, -N(R^{11e})R^{12e}, Cyano, Halogen, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)p^{e}R^{11e}, SO₂NR^{10e}R^{11e},
Aryl, substituiert mit 0 - 3 Gruppen, ausgewählt aus Halogen, C₁-C₆-Alkoxy, C₁-C₆-Alkyl, CF₃, S(O)ₘ^{e}Me und -NMe₂,
Aryl(C₁-C₄-alkyl)-, wobei das Aryl substituiert ist mit 0-3Gruppen, ausgewählt aus Halogen, C₁-C₆-Alkoxy, C₁-C₆-Alkyl, CF₃, S(O)ₚ^{e}Me und -NMe₂, und
einem 5- bis 10gliedrigen heterocyclischen Ring, enthaltend 1 - 3 N-, O- oder S-Heteroatome, wobei der heterocyclische Ring gesättigt, teilweise gesättigt oder vollständig ungesättigt sein kann, wobei der heterocyclische Ring mit 0-2R^{7e} substituiert ist;
R^{10e} ausgewählt ist aus:
H, -OH, CF₃, C₃-C₆-Alkenyl, C₃-C₁₁-Cycloalkyl, Aryl, (C₃-C₁₁-Cycloalkyl)methyl, Aryl(C₁-C₄-alkyl) und C₁-C₁₀-Alkyl, substituiert mit 0-2R^{6e};
R^{11e} ausgewählt ist aus:
H, Hydroxy, C₁-C₈-Alkyl, C₃-C₆-Alkenyl, C₃-C₁₁-Cycloalkyl, (C₃-C₁₁-Cycloalkyl)-methyl, C₁-C₆-Alkoxy, Benzyloxy, Aryl, Heteroaryl, Heteroaryl(C₁-C₄-alkyl)-, Aryl(C₁-C₄-alkyl), Adamantylmethyl und C₁-C₁₀-Alkyl, substituiert mit 0-2R^{4e};
R^{4e} ausgewählt ist aus:
H, C₁-C₆-Alkyl, C₃-C₇-Cycloalkyl, C₃-C₇-Cycloalkyl(C₁-C₄-alkyl)-, Aryl, Heteroaryl, Aryl(C₁-C₆-alkyl)- und Heteroaryl(C₁-C₆-alkyl)-, wobei die Aryl- oder Heteroarylgruppen mit 0 - 2 Substituenten, unabhängig ausgewählt aus der Gruppe, bestehend aus C₁-C₄-Alkyl, C₁-C₄-Alkoxy, F, Cl, Br, CF₃ und NO₂, substituiert sind,
R^{12e} ausgewählt ist aus:
H, C₁-C₆-Alkyl, Triphenylmethyl, Methoxymethyl, Methoxyphenyldiphenylmethyl, Trimethylsilylethoxymethyl, (C₁-C₆-Alkyl)carbonyl, (C₁-C₆-Alkoxy)carbonyl, (C₁-C₆-Alkyl)aminocarbonyl, C₃-C₆-Alkenyl, C₃-C₇-Cycloalkyl, C₃-C₇-Cycloalkyl-(C₁-C₄-alkyl)-, Aryl, Heteroaryl(C₁-C₆-alkyl)carbonyl, Heteroarylcarbonyl, Aryl-(C₁-C₆-alkyl)-, (C₁-C₆-Alkyl)carbonyl, Arylcarbonyl, C₁-C₆-Alkylsulfonyl, Arylsulfonyl, Aryl(C₁-C₆-alkyl)sulfonyl, Heteroarylsulfonyl, Heteroaryl(C₁-C₆-alkyl)sulfonyl, Aryloxycarbonyl und Aryl(C₁-C₆-alkoxy)carbonyl, wobei die Arylgruppen mit 0 - 2 Substituenten, ausgewählt aus der Gruppe, bestehend aus C₁-C₄-Alkyl, C₁-C₄-Alkoxy, Halogen, CF₃ und Nitro, substituiert sind;
R^{16e} ausgewählt ist aus:
-C(=O)OR^{18ae}, -C(=O)R^{18be}, -C(=O)N(R^{18be})₂, -SO₂R^{18ae} und -SO₂N(R^{18be})₂;
R^{17e} ausgewählt ist aus:
H, C₁-C₆-Alkyl, C₃-C₇-Cycloalkyl, C₃-C₇-Cycloalkyl(C₁-C₄-alkyl)-, Aryl, Aryl(C₁-C₆-alkyl)- und Heteroaryl(C₁-C₆-alkyl);
R¹⁸ae ausgewählt ist aus:
C₁-C₈-Alkyl, gegebenenfalls substituiert mit einer Bindung an Lₙ, C₃-C₁₁-Cycloalkyl, gegebenenfalls substituiert mit einer Bindung an Lₙ, Aryl(C₁-C₆-alkyl)-, gegebenenfalls substituiert mit einer Bindung an Lₙ, Heteroaryl(C₁-C₆-alkyl)-, gegebenenfalls substituiert mit einer Bindung an Lₙ, (C₁-C₆-Alkyl)heteroaryl, gegebenenfalls substituiert mit einer Bindung an Lₙ, Biaryl(C₁-C₆-alkyl), gegebenenfalls substituiert mit einer Bindung an Lₙ, Heteroaryl, gegebenenfalls substituiert mit einer Bindung an Lₙ, Phenyl, substituiert mit 3 - 4 R^{19e} und gegebenenfalls substituiert mit einer Bindung an Lₙ, Naphthyl, substituiert mit 0-4 R^{19e} und gegebenenfalls substituiert mit einer Bindung an Lₙ, und einer Bindung an Lₙ, wobei die Aryl- oder Heteroarylgruppen gegebenenfalls mit 0 - 4 R^{19e} substituiert sind;
R^{18be} H oder R^{18ae} ist,
R^{19e} ausgewählt ist aus:
H, Halogen, CF₃, CO₂H, CN, NO₂, -NR^{11e}R^{12e}, OCF₃, C₁-C₈-Alkyl, C₂-C₆-Alkenyl, C₂-C₆-Alkinyl, C₃-C₁₁-Cycloalkyl, C₃-C₇-Cycloalkyl(C₁-C₄-alkyl)-, Aryl(C₁-C₆-alkyl)-, C₁-C₆-Alkoxy, C₁-C₄-Alkoxycarbonyl, Aryl, Aryl-O-, Aryl-SO₂-, Heteroaryl und Heteroaryl-SO₂-, wobei die Aryl- und Heteroarylgruppen mit 0 - 4 Gruppen, ausgewählt aus Wasserstoff, Halogen, CF₃, C₁-C₃-Alkyl und C₁-C₃-Alkoxy, substituiert sind;
R^{20e} ausgewählt ist aus:
Hydroxy, C₁-C₁₀-Alkyloxy, C₃-C₁₁-Cycloalkyloxy, Aryloxy, Aryl(C₁-C₄-alkyl)oxy, C₂-C₁₀-Alkylcarbonyloxy(C₁-C₂-alkyl)oxy-, C₂-C₁₀-Alkoxycarbonyloxy(C₁-C₂-alkyl)oxy-, C₂₋C₁₀-Alkoxycarbonyl(C₁-C₂alkyl)oxy-, C₃-C₁₀-Cycloalkylcarbonyloxy-(C₁-C₂-alkyl)oxy-, C₃-C₁₀-Cycloalkoxycarbonyloxy(C₁-C₂-alkyl)oxy-, C₃-C₁₀-Cycloalkoxycarbonyl(C₁-C₂-alkyl)oxy-, Aryloxycarbonyl(C₁-C₂-alkyl)oxy-, Aryloxycarbonyloxy(C₁-C₂-alkyl)oxy-, Arylcarbonyloxy(C₁-C₂-alkyl)oxy-, C₁-C₅-Alkoxy-(C₁-C₅-alkyl)carbonyloxy(C₁-C₂-alkyl)oxy, (5-(C₁-C₅-Alkyl)-1,3-dioxa-cyclopenten-2-on-yl)methyloxy, (5-Aryl-1,3-dioxa-cyclopenten-2-on-yl)methyloxy und (R^{10e})(R^{11e})N-(C₁-C₁₀-Alkoxy)-;
R^{21e} ausgewählt ist aus:
C₁-C₈-Alkyl, C₂-C₆-Alkenyl, C₃-C₁₁-Cycloalkyl, (C₃-C₁₁-Cycloalkyl)methyl, Aryl, Aryl(C₁-C₄-alkyl)- und C₁-C₁₀-Alkyl, substituiert mit 0 - 2 R^{7e};
R^{22e} ausgewählt ist aus:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae} und -C(=O)NHSO₂NHR^{18be};
m^{e} 0 - 2 ist;
n^{e} 0 - 4 ist und
p^{e} 0 - 2 ist;
mit der folgenden Maßgabe: n^{e} und m^{e} sind so gewählt, daß die Anzahl an Atomen, die R^{1e} und -COR^{20e} in der Formel (IV) verbinden, im Bereich von 8 - 14 liegt;
d ausgewählt ist aus 1, 2, 3, 4 und 5;
d' 1 - 50 ist;
W unabhängig bei jedem Vorkommen aus der Gruppe: O, NH, NHC(=O), C(=O)NH, NR⁸C(=O), C(=O)NR⁸, C(=O), C(=O)O, OC(=O), NHC(=S)NH, NHC(=O)NH, SO₂, (OCH₂CH₂)ₛ, (CH₂CH₂O)_{s'}, (OCH₂CH₂CH₂)_{s"}, (CH₂CH₂CH₂O)ₜ und (aa)_{t'} ausgewählt ist;
aa unabhängig bei jedem Vorkommen eine Aminosäure ist;
Z ausgewählt ist aus der Gruppe: Aryl, substituiert mit 0-1R¹⁰, C₃-₁₀-Cycloalkyl, substituiert mit 0-1R¹⁰, und einem 5- bis 10gliedrigen heterocyclischen Ringsystem, enthaltend 1-4Heteroatome, unabhängig ausgewählt aus N, S und O und substituiert mit 0-1R¹⁰;
R⁶, R^{6a} , R⁷, R^{7a} und R⁸ unabhängig voneinander bei jedem Vorkommen aus der Gruppe: H, =O, COOH, SO₃H, C₁-C₅-Alkyl, substituiert mit 0-1R¹⁰, Aryl, substituiert mit 0 - 1 R¹⁰, Benzyl, substituiert mit 0-1R¹⁰, und C₁-C₅-Alkoxy, substituiert mit 0 - 1R¹⁰, NHC(=O)R¹¹, C(=O)NHR¹¹, NHC(=O)NHR¹¹, NHR¹¹, R¹¹ und einer Bindung an Cₕ ausgewählt sind;
k 0 oder 1 ist;
s ausgewählt ist aus 0, 1, 2, 3, 4 und 5;
s' ausgewählt ist aus 0, 1, 2, 3, 4 und 5;
s" ausgewählt ist aus 0, 1, 2, 3, 4 und 5;
t ausgewählt ist aus 0, 1, 2, 3, 4 und 5;
A¹, A², A³, A⁴, A⁵, A⁶, A⁷ und A⁸ unabhängig voneinander bei jedem Vorkommen aus der Gruppe: NR¹³, NR¹³R¹⁴, S, SH, S(Pg), OH und einer Bindung an Lₙ ausgewählt sind;
E eine Bindung, CH oder eine Spacer-Gruppe ist, unabhängig bei jedem Vorkommen ausgewählt aus der Gruppe: C₁-C₁₀-Alkyl, substituiert mit 0-3R¹⁷, Aryl, substituiert mit 0-3R¹⁷, C₃₋₁₀-Cycloalkyl, substituiert mit 0-3R¹⁷, und einem 5-bis 10gliedrigen heterocyclischen Ringsystem, enthaltend 1-4Heteroatome, unabhängig ausgewählt aus N, S und O und substituiert mit 0-3R¹⁷;
R¹³ und R¹⁴ jeweils unabhängig voneinander ausgewählt sind aus der Gruppe: einer Bindung an Lₙ, Wasserstoff, C₁-C₁₀-Alkyl, substituiert mit 0 - 3 R¹⁷, Aryl, substituiert mit 0-3R¹⁷, einem 5- bis 10gliedrigen heterocyclischen Ringsystem, enthaltend 1-4Heteroatome, unabhängig ausgewählt aus N, S und O und substituiert mit 0-3R¹⁷, und einem Elektron, vorausgesetzt, daß, wenn einer von R¹³ oder R¹⁴ ein Elektron ist, der andere auch ein Elektron ist;
alternativ R¹³ und R¹⁴ unter Bildung von =C(R²⁰)(R²¹) verbunden sind;
R¹⁷ unabhängig bei jedem Vorkommen ausgewählt ist aus der Gruppe: einer Bindung an Lₙ, =O, F, Cl, Br, I, -CF₃, -CN, -CO₂R¹⁸, -C(=O)R¹⁸, -C(=O)N(R¹⁸)₂, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)₂, -N(R¹⁸)₂, -NHC(=S)NHR¹⁸, =NOR¹⁸, -C(=O)NHNR¹⁸R^{18a}, -OCH₂CO₂H und 2-(1-Morpholino)ethoxy;
R¹⁸, R^{18a} und R¹⁹ unabhängig voneinander bei jedem Vorkommen ausgewählt sind aus der Gruppe: einer Bindung an Lₙ, H und C₁-C₆-Alkyl;
R²⁰ und R²¹ unabhängig voneinander ausgewählt sind aus der Gruppe:
H, C₁-C₅-Alkyl, -CO₂R²⁵, C₂-C₅-1-Alken, substituiert mit 0-3R²³, C₂-C₅-1-Alkin, substituiert mit 0-3R²³, Aryl, substituiert mit 0-3R²³, und einem ungesättigten 5- bis 10gliedrigen heterocyclischen Ringsystem, enthaltend 1-4Heteroatome, unabhängig ausgewählt aus N, S und O und substituiert mit 0-3R²³;
alternativ R²⁰ und R²¹ zusammen mit dem zweiwertigen Kohlenstoffrest, an den sie gebunden sind: 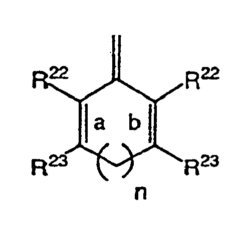 bilden,
R²² und R²³ unabhängig voneinander ausgewählt sind aus der Gruppe:
H und R²⁴;
alternativ R²², R²³ zusammen ein kondensiertes aromatisches oder ein 5- bis 10gliedriges heterocyclisches Ringsystem, enthaltend 1 - 4 Heteroatome, unabhängig ausgewählt aus N, S und O, bilden;
R²⁴ unabhängig bei jedem Vorkommen ausgewählt ist aus der Gruppe: -CO₂R²⁵, -C(=O)N(R²⁵)₂, -CH₂OR²⁵, -OC(=O)R²⁵, -OR²⁵, -SO₃H, -N(R²⁵)₂ und -OCH₂CO₂H und
R²⁵ unabhängig bei jedem Vorkommen ausgewählt ist aus der Gruppe: H und C₁-C₃-Alkyl.

38. Verwendung nach Anspruch 36, wobei:
R^{1e} ausgewählt ist aus: 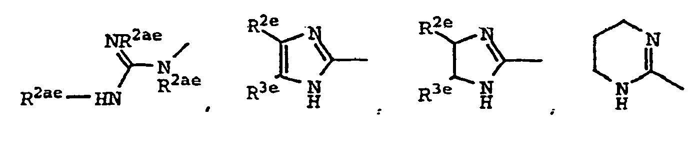 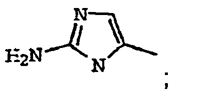 und
R^{2e} und R^{3e} unabhängig voneinander ausgewählt sind aus:
H, C₁-C₄-Alkoxy, NR^{11e}R^{12e}, Halogen, NO₂, CN, CF₃, C₁-C₆-Alkyl, C₃-C₆-Alkenyl, C₃-C₇-Cycloalkyl, C₃-C₇-Cycloalkyl(C₁-C₄-alkyl), Aryl(C₁-C₆-alkyl)-, (C₁-C₆-Alkyl)-carbonyl, (C₁-C₆-Alkoxy)carbonyl, Arylcarbonyl und Aryl, substituiert mit 0 - 4 R^{7e},
alternativ, wenn R^{2e} und R^{3e} Substituenten an nachbarständigen Atomen sind, diese mit den Kohlenstoffatomen, an die sie gebunden sind, unter Bildung eines 5- bis 7gliedrigen carbocyclischen oder 5- bis 7gliedrigen heterocyclischen aromatischen oder nicht-aromatischen Ringsystems zusammengenommen werden können, wobei der carbocyclische oder heterocyclische Ring mit 0 - 2 Gruppen, ausgewählt aus C₁-C₄-Alkyl, C₁-C₄-Alkoxy, Halogen, Cyano, Amino, CF₃ und NO₂, substituiert ist;
R^{2ae} ausgewählt ist aus:
H, C₁-C₁₀-Alkyl, C₂-C₆-Alkenyl, C₃-C₁₁-Cycloalkyl, C₃-C₇-Cycloalkyl(C₁-C₄-alkyl), Aryl, Aryl(C₁-C₄-alkyl)-, (C₂-C₇-Alkyl)carbonyl, Arylcarbonyl, (C₂-C₁₀-Alkoxy)carbonyl, C₃-C₇-Cycloalkoxycarbonyl, C₇-C₁₁-Bicycloalkoxycarbonyl, Aryloxycarbonyl, Aryl(C₁-C₁₀-alkoxy)carbonyl, C₁-C₆-Alkylcarbonyloxy(C₁-C₄-alkoxy)carbonyl, Arylcarbonyloxy(C₁-C₄-alkoxy)carbonyl und C₃-C₇-Cycloalkylcarbonyloxy(C₁-C₄-alkoxy)carbonyl;
R^{7e} ausgewählt ist aus:
H, Hydroxy, C₁-C₄-Alkyl, C₁-C₄-Alkoxy, Aryl, Aryl(C₁-C₄-alkyl)-, (C₁-C₄-Alkyl)carbonyl, CO₂R^{18ae}, SO₂R^{11e}, SO₂NR^{10e}R^{11e}, OR^{10e} und N(R^{11e})R^{12e};
U^{e} ausgewählt ist aus:
-(CH₂)ₙ^{e}-, -NH(CH₂)ₙ^{e}-, -N(R^{10e})C(=O)- und -NHC(=O)(CH₂)ₙ^{e};
G^{e} N oder CR^{19e} ist;
R^{8e} H ist;
R^{9e} ausgewählt ist aus:
H, Nitro, -N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, Hydroxy, OR^{22e}, -N(R^{10e})R^{11e}, -N(R^{16e})R^{17e}, Aryl(C₀-C₄-alkyl)carbonyl, Aryl-(C₁-C₄-alkyl), Heteroaryl(C₁-C₄-alkyl), CONR^{18ae}R^{20e}, SO₂R^{18ae} und SO₂NR^{18ae}R^{20e},
mit der Maßgabe, daß jede der obigen Alkyl-, Cycloalkyl-, Aryl- oder Heteroarylgruppen unsubstituiert oder unabhängig mit 1-2R^{7e} substituiert sein kann;
R^{10e} ausgewählt ist aus:
H, -OH, CF₃, C₃-C₆-Alkenyl, C₃-C₆-Cycloalkyl, Aryl, (C₃-C₆-Cycloalkyl)methyl, Aryl(C₁-C₄-alkyl) und C₁-C₄-Alkyl, substituiert mit 0-2R^{6e};
R^{6e} ausgewählt ist aus:
H, C₁-C₄-Alkyl, Hydroxy, C₁-C₄-Alkoxy, Nitro, C₁-C₄-Alkylcarbonyl, -N(R^{11e})R^{12e}, Cyano, Halogen, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e},
Aryl, substituiert mit 0 - 3 Gruppen, ausgewählt aus Halogen, C₁-C₄-Alkoxy, C₁-C₄-Alkyl, CF₃, S(O)ₘ^{e}Me und -NMe₂,
Aryl(C₁-C₄-alkyl)-, wobei das Aryl substituiert ist mit 0 - 3 Gruppen, ausgewählt aus Halogen, C₁-C₄-Alkoxy, C₁-C₄-Alkyl, CF₃, S(O)ₚ^{e}Me und -NMe₂, und
einem 5- bis 10gliedrigen heterocyclischen Ring, enthaltend 1 - 3 N-, O- oder S-Heteroatome, wobei der heterocyclische Ring gesättigt, teilweise gesättigt oder vollständig ungesättigt sein kann, wobei der heterocyclische Ring mit 0-2 R^{7e} substituiert ist;
R^{11e} ausgewählt ist aus:
H, Hydroxy, C₁-C₄-Alkyl, C₃-C₆-Alkenyl, C₃-C₆-Cycloalkyl, (C₃-C₆-Cycloalkyl)-methyl, C₁-C₄-Alkoxy, Benzyloxy, Aryl, Heteroaryl, Heteroaryl(C₁-C₄-alkyl)-, Aryl(C₁-C₄-alkyl), Adamantylmethyl und C₁-C₄-Alkyl, substituiert mit 0-2R^{4e};
R^{4e} ausgewählt ist aus:
H, C₁-C₄-Alkyl, C₃-C₇-Cycloalkyl, C₃-C₇-Cycloalkyl(C₁-C₄-alkyl)-, Aryl, Heteroaryl, Aryl(C₁-C₄-alkyl)- und Heteroaryl(C₁-C₄-alkyl)-, wobei die Aryl- oder Heteroarylgruppen mit 0 - 2 Substituenten, unabhängig ausgewählt aus der Gruppe, bestehend aus C₁-C₄-Alkyl, C₁-C₄-Alkoxy, F, Cl, Br, CF₃ und NO₂, substituiert sind,
R^{12e} ausgewählt ist aus:
H, C₁-C₄-Alkyl, (C₁-C₄-Alkyl)carbonyl, (C₁-C₄-Alkoxy)carbonyl, Phenyl(C₁-C₄-alkyl)-, Phenylsulfonyl, Phenyloxycarbonyl und Phenyl(C₁-C₄-alkoxy)carbonyl, wobei die Phenylgruppen mit 0 - 2 Substituenten, ausgewählt aus der Gruppe, bestehend aus C₁-C₄-Alkyl, C₁-C₄-Alkoxy, Halogen, CF₃ und Nitro, substituiert sind;
R^{16e} ausgewählt ist aus:
-C(=O)OR^{18ae}, -C(=O)R^{18be}, -C(=O)N(R^{18be})₂, -SO₂R^{18ae} und -SO₂N(R^{18be})₂;
R^{17e} ausgewählt ist aus:
H, C₁-C₄-Alkyl, C₃-C₆-Cycloalkyl, C₃-C₆-Cycloalkyl(C₁-C₄-alkyl)-, Aryl, Aryl(C₁-C₆-alkyl)- und Heteroaryl(C₁-C₆-alkyl);
R^{18ae} ausgewählt ist aus:
C₁-C₈-Alkyl, gegebenenfalls substituiert mit einer Bindung an Lₙ, C₃-C₁₁-Cycloalkyl, gegebenenfalls substituiert mit einer Bindung an Lₙ, Aryl(C₁-C₆-alkyl)-, gegebenenfalls substituiert mit einer Bindung an Lₙ, Heteroaryl(C₁-C₆-alkyl)-, gegebenenfalls substituiert mit einer Bindung an Lₙ, (C₁-C₆-Alkyl)heteroaryl, gegebenenfalls substituiert mit einer Bindung an Lₙ, Biaryl(C₁-C₆-alkyl), gegebenenfalls substituiert mit einer Bindung an Lₙ, Heteroaryl, gegebenenfalls substituiert mit einer Bindung an Lₙ, Phenyl, substituiert mit 3 - 4 R^{19e} und gegebenenfalls substituiert mit einer Bindung an Lₙ, Naphthyl, substituiert mit 0-4 R^{19e} und gegebenenfalls substituiert mit einer Bindung an Lₙ, und einer Bindung an Lₙ, wobei die Aryl- oder Heteroarylgruppen gegebenenfalls mit 0 - 4 R^{19e} substituiert sind;
R^{18be} H oder R^{18ae} ist,
R^{19e} ausgewählt ist aus:
H, Halogen, CF₃, CO₂H, CN, NO₂, -NR^{11e}R^{12e}, OCF₃, C₁-C₆-Alkyl, C₂-C₆-Alkenyl, C₂-C₆-Alkinyl, C₃-C₆-Cycloalkyl, C₃-C₆-Cycloalkyl(C₁-C₄-alkyl)-, Aryl(C₁-C₄-alkyl)-, C₁-C₆-Alkoxy, C₁-C₄-Alkoxycarbonyl, Aryl, Aryl-O-, Aryl-SO₂-, Heteroaryl und Heteroaryl-SO₂-, wobei die Aryl- und Heteroarylgruppen mit 0 - 4 Gruppen, ausgewählt aus Wasserstoff, Halogen, CF₃, C₁-C₃-Alkyl und C₁-C₃-Alkoxy, substituiert sind;
R^{20e} ausgewählt ist aus:
Hydroxy, C₁-C₆-Alkyloxy, C₃-C₆-Cycloalkyloxy, Aryloxy, Aryl(C₁-C₄-alkyl)oxy, C₂-C₁₀-Alkylcarbonyloxy(C₁-C₂-alkyl)oxy-, C₂-C₁₀-Alkoxycarbonyloxy(C₁-C₂-alkyl)oxy-, C₂-C₁₀-Alkoxycarbonyl(C₁-C₂-alkyl)oxy-, C₃-C₁₀-Cycloalkylcarbonyloxy-(C₁-C₂-alkyl)oxy-, C₃-C₁₀-Cycloalkoxycarbonyloxy(C₁-C₂-alkyl)oxy-, C₃-C₁₀-Cycloalkoxycarbonyl(C₁-C₂-alkyl)oxy-, Aryloxycarbonyl(C₁-C₂-alkyl)oxy-, Aryloxycarbonyloxy(C₁-C₂-alkyl)oxy-, Arylcarbonyloxy(C₁-C₂-alkyl)oxy-, C₁-C₅-Alkoxy(C₁-C₅-alkyl)carbonyloxy(C₁-C₂-alkyl)oxy, (5-(C₁-C₅-Alkyl)-1,3-dioxa-cyclopenten-2-on-yl)methyloxy, (5-Aryl-1,3-dioxa-cyclopenten-2-on-yl)methyloxy und (R^{10e})(R^{11e})N-(C₁-C₁₀-Alkoxy)-;
R^{21e} ausgewählt ist aus:
C₁-C₄-Alkyl, C₂-C₆-Alkenyl, C₃-C₆-Cycloalkyl, (C₃-C₆-Cycloalkyl)methyl, Aryl, Aryl(C₁-C₄-alkyl)- und C₁-C₁₀-Alkyl, substituiert mit 0 - 2 R^{7e};
R^{22e} ausgewählt ist aus:
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae} und -C(=O)NHSO₂NHR^{18be};
m^{e} 0 - 2 ist;
n^{e} 0 - 4 ist;
p^{e} 0 - 2 ist;
Cₕ 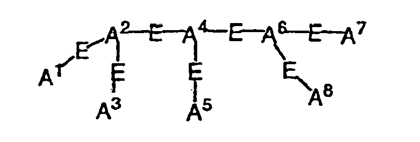 ist;
A¹ ausgewählt ist aus der Gruppe: OH und einer Bindung an Lₙ;
A², A⁴ und A⁶ jeweils N sind;
A³, A⁵ und A⁸ jeweils OH sind;
A⁷ eine Bindung an Lₙ oder eine NH-Bindung an Lₙ ist;
E ein C₂-Alkyl, substituiert mit 0 - 1 R¹⁷, ist;
R¹⁷ =O ist;
alternativ Cₕ 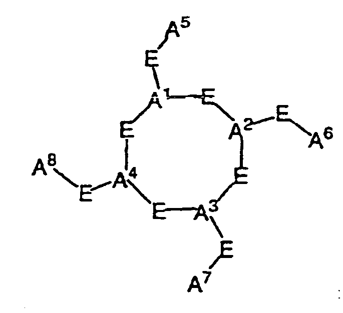 ist;
A¹ ausgewählt ist aus der Gruppe: OH und einer Bindung an Lₙ;
A², A³ und A⁴ jeweils N sind;
A⁵, A⁶ und A⁸ jeweils OH sind;
A⁷ eine Bindung an Lₙ ist;
E ein C₂-Alkyl, substituiert mit 0-1R¹⁷, ist;
R¹⁷ =O ist;
alternativ Cₕ 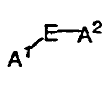 ist;
A¹ NH₂ oder N=C(R²⁰)(R²¹) ist;
E eine Bindung ist;
A² NHR¹³ ist;
R¹³ ein Heterocyclus, substituiert mit R¹⁷, ist, wobei der Heterocyclus aus Pyridin und Pyrimidin ausgewählt ist;
R¹⁷ aus einer Bindung an Lₙ, C(=O)NHR¹⁸ und C(=O)R¹⁸ ausgewählt ist;
R¹⁸ eine Bindung an Lₙ ist;
R²⁴ ausgewählt ist aus der Gruppe: -CO₂R²⁵, -OR²⁵, -SO₃H und -N(R²⁵)₂ und
R²⁵ unabhängig bei jedem Vorkommen ausgewählt ist aus der Gruppe: Wasserstoff und Methyl.

39. Verwendung nach Anspruch 36, wobei
Q aus der Gruppe ausgewählt ist:
3-[7-[(Imidazolin-2-ylamino)methyl]-1-methyl-6,8-difluorchinolin-4-on-3-ylcarbonylamino]-2-(3,5-dimethylisoxazol-4-ylsulfonylamino)propionsäure,
3-[7-[(Imidazolin-2-ylamino)methyl]-1-methyl-6,8-difluorchinolin-4-on-3-ylcarbonylamino]-2-(benzyloxycarbonylamino)propionsäure,
3-[7-[(Imidazolin-2-ylamino)methyl]-1-methyl-6,8-difluorchinolin-4-on-3-ylcarbonylamino]-2-(n-butyloxycarbonylamino)propionsäure,
3-[7-[(Imidazolin-2-ylamino)methyl]-1-methyl-6,8-difluorchinolin-4-on-3-ylcarbonylamino]-2-(n-butylsulfonylamino)propionsäure,
3-[7-[(Tetrahydropyrimid-2-ylamino)methyl]-1-methyl-6,8-difluorchinolin-4-on-3-ylcarbonylamino]-2-(benzyloxycarbonylamino)propionsäure,
3-[7-[(Tetrahydropyrimid-2-ylamino)methyl]-1-methyl-6,8-difluorchinolin-4-on-3-ylcarbonylamino]-2-(n-butyloxycarbonylamino)propionsäure,
3-[7-[(Tetrahydropyrimid-2-ylamino)methyl]-1-methyl-6,8-difluorchinolin-4-on-3-ylcarbonylamino]-2-(phenylsulfonylamino)propionsäure,
3-[7-[(Tetrahydropyrimid-2-ylamino)methyl]-1-methyl-6,8-difluorchinolin-4-on-3-ylcarbonylamino]-2-(n-butylsulfonyl)aminopropionsäure,
3-[7-[(2-Aminothiazol-4-yl)methyl]-1-methyl-6,8-difluorchinolin-4-on-3-ylcarbonylamino]-2-(benzyloxycarbonylamino)propionsäure,
3-[7-[(Imidazolin-2-ylamino)methyl]-1-methyl-6,8-difluorchinolin-4-on-3-ylcarbonylamino]-2-((2,4,6-trimethylphenyl)sulfonylamino)propionsäure,
3-[7-[(Tetrahydropyrimid-2-ylamino)methyl]-1-methyl-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-methyl-6,8-difluorchinolin-4-on-3-ylcarbonylamino]-2-(3,5-dimethylisoxazol-4-ylsulfonylamino)propionsäure,
3-[7-[(Imidazol-2-ylamino)methyl]-1-methyl-6,8-difluorchinolin-4-on-3-ylcarbonylamino]-2-(benzyloxycarbonylamino)propionsäure,
3-[7-[(Imidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-methyl-6,8-difluorchinolin-4-on-3-ylcarbonylamino]-2-((4-biphenyl)sulfonylamino)propionsäure,
3-[7-[(Imidazol-2-ylamino)methyl]-1-methyl-6,8-difluorchinolin-4-on-3-ylcarbonylamino]-2-(1-naphthylsulfonylamino)propionsäure,
3-[7-[(Benzimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(4-Methylimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(4,5-Dimethylimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(4,5,6,7-Tetrahydrobenzimidazol-2-ylamino)methyl]-1-methyl-6,8-difluorchinolin-4-on-3-ylcarbonylamino]-2-((2,4,6-trimethylphenyl)sulfonylamino)propionsäure,
3-[7-[(Pyridin-2-ylamino)methyl]-1-methyl-6,8-difluorchinolin-4-on-3-ylcarbonylamino]-2-((2,4,6-trimethylphenyl)sulfonylamino)propionsäure,
3-[7-(2-Aminopyridin-6-yl)-1-methyl-6,8-difluorchinolin-4-on-3-ylcarbonylamino]-2-((2,4,6-trimethylphenyl)sulfonylamino)propionsäure,
3-[7-[(7-Azabenzimidazol-2-yl)methyl]-1-methyl-6,8-difluorchinolin-4-on-3-ylcarbonylamino]-2-((2,4,6-trimethylphenyl)sulfonylamino)propionsäure,
3-[7-[(Benzimidazol-2-ylamino)methyl]-1-(2-phenylethyl)-6,8-difluorchinolin-4-on-3-ylcarbonylamino]propionsäure,
3-[7-[(Pyridin-2-ylamino)methyl]-1-(2-phenylethyl)-6,8-difluorchinolin-4-on-3-ylcarbonylamino]propionsäure,
3-[7-[(Imidazolin-2-ylamino)methyl]-1-(2-phenylethyl)-6,8-difluorchinolin-4-on-3-yl carbonylamino]propionsäure,
3-[7-[(Imidazol-2-ylamino)methyl]-1-(2-phenylethyl)6,8-difluorchinolin-4-on-3-ylcarbonylamino]propionsäure,
3-[7-[(Imidazolin-2-ylamino)methyl]-1-(2-phenylethyl)-6,8-difluorchinolin-4-on-3-ylcarbonylamino]-2-(benzyloxycarbonylamino)propionsäure,
3-[7-[(Imidazolin-2-ylamino)methyl]-1-(2-phenylethyl)-6,8-difluorchinolin-4-on-3-ylcarbonylamino]-2-(n-butyloxycarbonylamino)propionsäure,
3-[7-[(Imidazolin-2-ylamino)methyl]-1-(2-phenylethyl)-6,8-difluorchinolin-4-on-3-ylcarbonylamino]-2-(phenylsulfonylamino)propionsäure,
3-[7-[(Imidazolin-2-ylamino)methyl]-1-(2-phenylethyl)-6,8-difluorchinolin-4-on-3-ylcarbonylamino]-2-(n-butylsulfonylamino)propionsäure,
3-[7-[(Tetrahydropyrimid-2-ylamino)methyl]-1-(2-phenylethyl)-6,8-difluorchinolin-4-on-3-ylcarbonylamino]-2-(benzyloxycarbonylamino)propionsäure,
3-[7-[(Tetrahydropyrimid-2-ylamino)methyl]-1-(2-phenylethyl)6,8-difluorchinolin-4-on-3-ylcarbonylamino]-2-(n-butyloxycarbonylamino)propionsäure,
3-[7-[(Tetrahydropyrimid-2-ylamino)methyl]-1-(2-phenylethyl)-6,8-difluorchinolin-4-on-3-ylcarbonylamino]-2-(phenylsulfonylamino)propionsäure,
3-[7-[(Tetrahydropyrimid-2-ylamino)methyl]-1-(2-phenylethyl)-6,8-difluorchinolin-4-on-3-ylcarbonylamino]-2-(n-butylsulfonyl)aminopropionsäure,
3-[7-[(2-Aminothiazol-4-yl)methyl]-1-(2-phenylethyl)-6,8-difluorchinolin-4-on-3-ylcarbonylamino]-2-(phenylsulfonylamino)propionsäure,
3-[7-[(2-Aminothiazol-4-yl)methyl]-1-(2-phenylethyl)-6,8-difluorchinolin-4-on-3-ylcarbonylamino]-2-(benzyloxycarbonylamino)propionsäure,
3-[7-[(Imidazolin-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(Tetrahydropyrimid-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-(2-phenylethyl)-6,8-difluorchinolin-4-on-3-ylcarbonylamino]-2-(benzyloxycarbonylamino)propionsäure,
3-[7-[(Imidazol-2-ylamino)methyl]-1-(2-phenylethyl)-6,8-difluorchinolin-4-on-3-ylcarbonylamino]-2-(phenylsulfonylamino)propionsäure,
3-[7-[(Imidazol-2-ylamino)methyl]-1-(2-phenylethyl)-6,8-difluorchinolin-4-on-3-ylcarbonylamino]-2-((2,6-dichlorphenyl)sulfonylamino)propionsäure,
3-[7-[(Imidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(Imidazol-2-ylamino)methyl]-1-(2-phenylethyl)-6,8-difluorchinolin-4-on-3-ylcarbonylamino]-2-((4-biphenyl)sulfonylamino)propionsäure,
3-[7-[(Benzimidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(4-Methylimidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(4,5-Dimethylimidazol-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-[(4,5,6,7-Tetrahydrobenzimidazol-2-ylamino)methyl]-1-(2-phenylethyl)-6,8-difluorchinolin-4-on-3-ylcarbonylamino]-2-((2,4,6-trimethylphenyl)sulfonylamino)-propionsäure,
3-[7-[(Pyridin-2-ylamino)methyl]-1-(2-phenylethyl)-6,8-difluorchinolin-4-on-3-ylcarbonylamino]-2-((2,4,6-trimethylphenyl)sulfonylamino)propionsäure,
3-[7-(2-Aminopyridin-6-yl)-1-(2-phenylethyl)-6,8-difluorchinolin-4-on-3-ylcarbonylamino]-2-((2,4,6-trimethylphenyl)sulfonylamino)propionsäure und
3-[7-[(7-Azabenzimidazol-2-yl)methyl]-1-(2-phenylethyl)-6,8-difluorchinolin-4-on-3-ylcarbonylamino]-2-((2,4,6-trimethylphenyl)sulfonylamino)propionsäure.

40. Verwendung nach Anspruch 34, wobei das Radioisotop ¹⁵³Sm ist.

41. Verwendung nach Anspruch 34, wobei das Radioisotop ¹⁷⁷Lu ist.

42. Verwendung nach Anspruch 41, wobei das Radiopharmazeutikum aus der Gruppe ausgewählt ist: 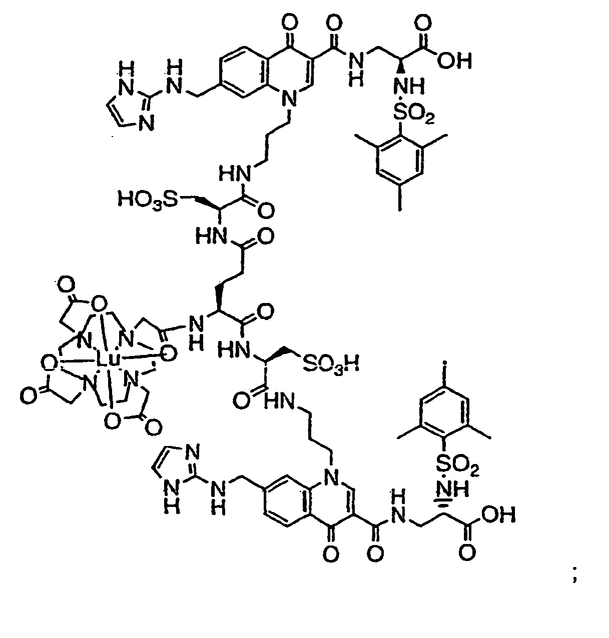 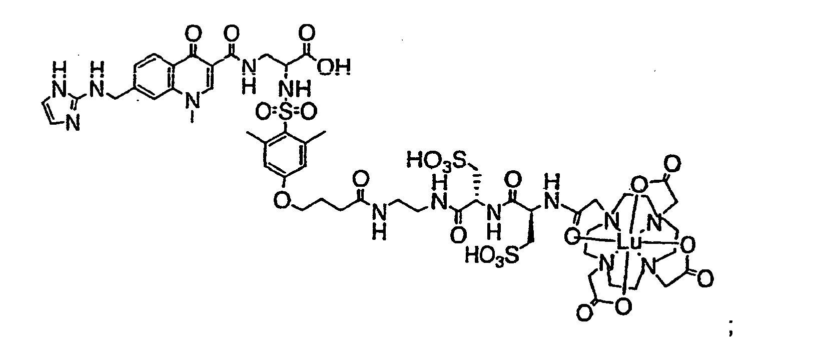 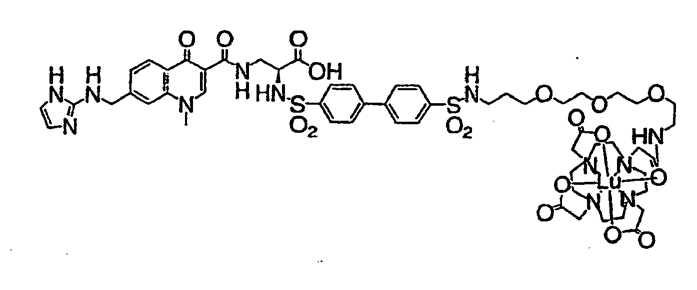 und

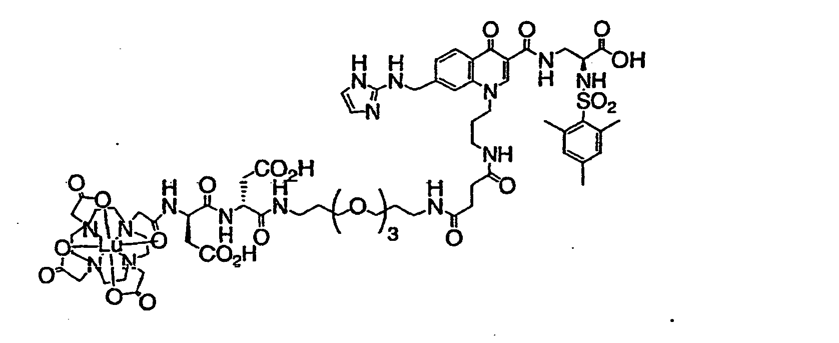

43. Verwendung nach Anspruch 34, wobei das Radioisotop ⁹⁰Y ist.

44. Verwendung nach Anspruch 43, wobei das Radiopharmazeutikum aus der Gruppe ausgewählt ist: 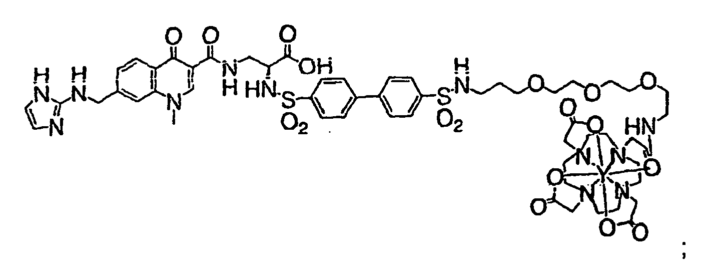 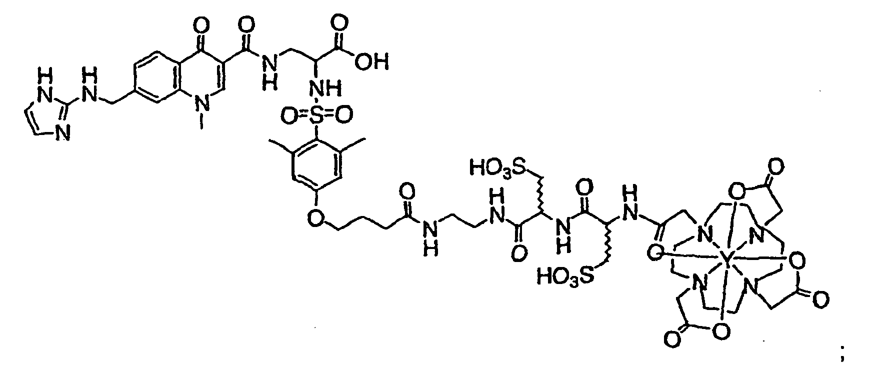 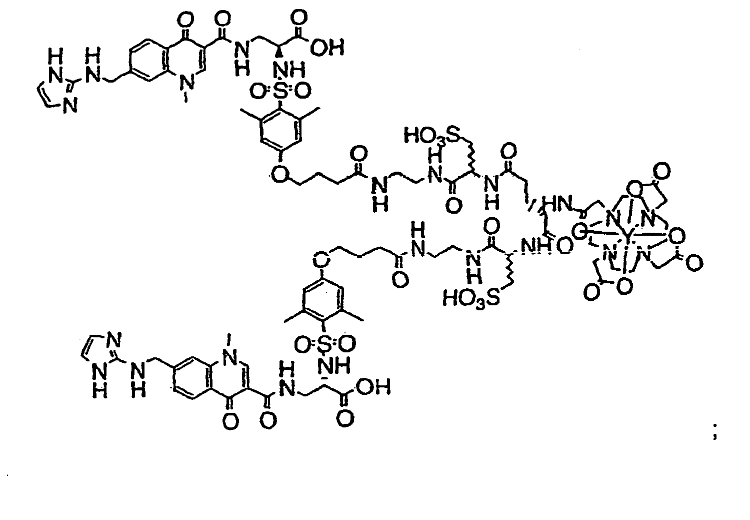 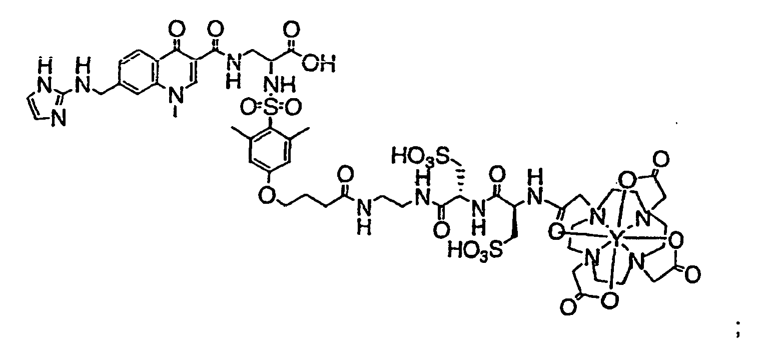 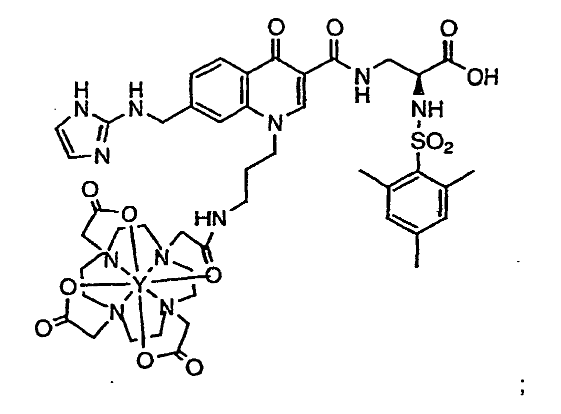 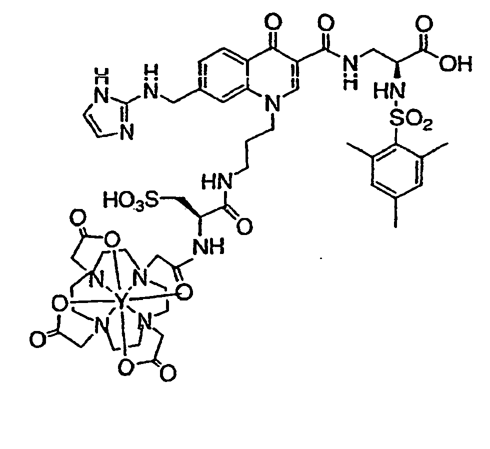 und

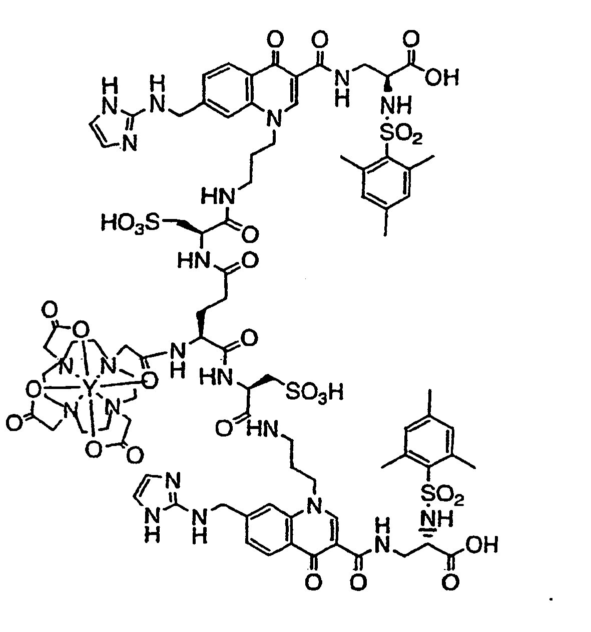

45. Verwendung nach Anspruch 26, ferner umfassend die Behandlung von Krebs mittels Brachytherapie, externer Bestrahlung, Lasertherapie oder chirurgischer Entfernung.

46. Kit, umfassend Verpackungsmaterial und eine therapeutische radiopharmazeutische Zusammensetzung nach Anspruch 12, enthalten in dem Verpackungsmaterial, wobei das Verpackungsmaterial ein Etikett oder eine Packungsbeilage umfaßt, das/die anzeigt, daß die therapeutische radiopharmazeutische Zusammensetzung zur Behandlung von Krebs verwendet werden kann.

47. Therapeutische radiopharmazeutische Zusammensetzung nach Anspruch 12, ferner umfassend einen Photosensibilisator.

48. Therapeutische radiopharmazeutische Zusammensetzung nach Anspruch 47, wobei der Photosensibilisator ausgewählt ist aus der Gruppe, bestehend aus Photofrin; Naphthalocyanin-Photosensibilisatoren; Tetrapyrrol-basierenden Photosensibilisator; Porphyrinen; Chlorinen; Phthalocyaninen; Naphthalocyaninen; Coumarinen, Psoralenen, 1,3,4,6-Tetramethoxyhelianthron; 10,13-Dimethyl-1,3,4,6-tetrahydroxyhelianthron; 10,13-Di(methoxycarbonyl)-1,3,4,6-tetramethoxyhelianthron; 1,6-Di-N-butylamino-3,4-dimethoxy-helianthron; 1,6-Di-N-butylamino-3,4-dimethoxy-10,13-dimethyl-helianthron; 1,6-Di-(N-hydroxyethylamino)-3,4-dimethoxy-helianthron; 2,5-Dibrom-1,3,4,6-tetrahydroxyhelianthron und 2,5-Dibrom-10,13-dimethyl-1,3,4,6-tetrahydroxyhelianthron.

49. Kit nach Anspruch 46, ferner umfassend einen Photosensibilisator.

50. Kit nach Anspruch 49, wobei der Photosensibilisator ausgewählt ist aus der Gruppe, bestehend aus Photofrin; Naphthalocyanin-Photosensibilisatoren; Tetrapyrrol-basierenden Photosensibilisatoren; Porphyrinen; Chlorinen; Phthalocyaninen; Naphthalocyaninen; Coumarinen, Psoralenen, 1,3,4,6-Tetramethoxyhelianthron; 10,13-Dimethyl-1,3,4,6-tetrahydroxyhelianthron; 10,13-Di(methoxycarbonyl)-1,3,4,6-tetramethoxyhelianthron; 1,6-Di-N-butylamino-3,4-dimethoxy-helianthron; 1,6-Di-N-butylamino-3,4-dimethoxy-10,13-dimethyl-helianthron; 1,6-Di-(N-hydroxyethylamino)-3,4-dimethoxy-helianthron; 2,5-Dibrom-1,3,4,6-tetrahydroxyhelianthron und 2,5-Dibrom-10,13-dimethyl-1,3,4,6-tetrahydroxyhelianthron.

51. Verwendung nach Anspruch 26, ferner umfassend das Behandeln des Patienten mit photodynamischer Therapie.

52. Verwendung nach Anspruch 51, wobei die photodynamische Therapie:
a) das Verabreichen eines therapeutischen Radiopharmazeutikums der vorliegenden Erfindung und eines Photosensibilisators (photoreaktives Mittel) an einen Patienten, wobei der Photosensibilisator ein charakteristisches Lichtabsorptionswellenband aufweist und vorzugsweise von anomalem Gewebe absorbiert wird;
(b) das Bereitstellen einer Bildgebungsvorrichtung, die mit einer Vielzahl von Lichtquellen eine Einheit bildet und ein Signal erzeugt, das zur bildlichen Darstellung von anomalem Gewebe an der innen liegenden Behandlungsstelle verwendet wird, wobei die Lichtquellen Licht in einem Wellenband emittieren, das dem charakteristischen Lichtabsorptionswellenband des Photosensibilisators entspricht, wobei das Wellenband Wellenlängen umfaßt, die ausreichend lang sind, daß sie durch die Hautschicht des Patienten zu der innen liegenden Behandlungsstelle vordringen können;
(c) das Bestimmen einer Position des anomalen Gewebes an der innen liegenden Zielstelle im Körper des Patienten mit der Bildgebungsvorrichtung, indem ein Bild von dem anomalen Gewebe an der Zielstelle, welches als Reaktion auf das durch die Bildgebungsvorrichtung erzeugte Signal entwickelt wurde, betrachtet wird; und
(d) das Aktivieren der Lichtquellen, damit die innen liegende Zielstelle an der mit der Bildgebungsvorrichtung bestimmten Position mit Licht behandelt werden kann, umfaßt.

53. Verwendung nach Anspruch 52, wobei der Photosensibilisator (photoreaktives Mittel) speziell auf das betroffene Gewebe gerichtet wird, indem ein Bindemittel, das selektiv den Photosensibilisator an das betroffene Gewebe bindet, einbezogen wird.

54. Verwendung nach Anspruch 52, wobei der Photosensibilisator ausgewählt ist aus der Gruppe, bestehend aus Photofrin; Naphthalocyanin-Photosensibilisatoren; Tetrapyrrol-basierenden Photosensibilisatoren; Porphyrinen; Chloinen; Phthalocyaninen; Naphthalocyaninen; Coumarinen, Psoralenen, 1,3,4,6-Tetramethoxyhelianthron; 10,13-Dimethyl-1,3,4,6-tetrahydroxyhelianthron; 10,13-Di(methoxycarbonyl)-1,3,4,6-tetramethoxyhelianthron; 1,6-Di-N-butylamino-3,4-dimethoxy-helianthron; 1,6-Di-N-butylamino-3,4-dimethoxy-10,13-dimethyl-helianthron; 1,6-Di-(N-hydroxyethylamino)-3,4-dimethoxy-helianthron; 2,5-Dibrom-1,3,4,6-tetrahydroxyhelianthron und 2,5-Dibrom-10,13-dimethyl-1,3,4,6-tetrahydroxyhelianthron.

## Revendications

1. Trousse destinée au traitement du cancer, comprenant un composé de formule (I) et au moins un agent choisi dans le groupe constitué par un agent anticancéreux et un agent de radiosensibilisation, ou un sel de ceux-ci acceptable sur le plan pharmaceutique, et un support acceptable sur le plan pharmaceutique, dans laquelle le composé de formule (I) est :
(Q)_{d}-Lₙ-Cₕ ou (Q)_{d}-Lₙ-(Cₕ)_{d'}
dans laquelle Q est un composé de formule (II) : 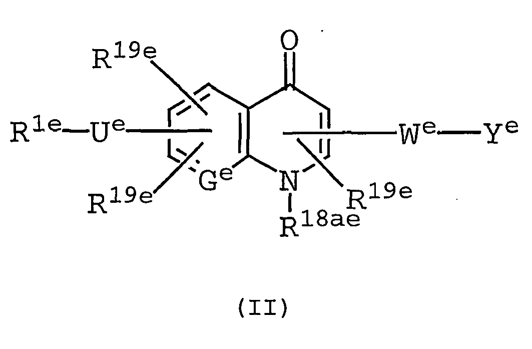 incluant les formes stéréoisomériques de celle-ci, ou les mélanges des formes stéréoisomériques de celle-ci, ou les formes de sel ou de promédicament de celle-ci acceptables sur le plan pharmaceutique, dans laquelle :
R^{1e} est choisi parmi : 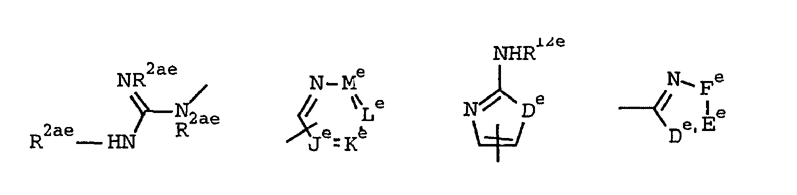 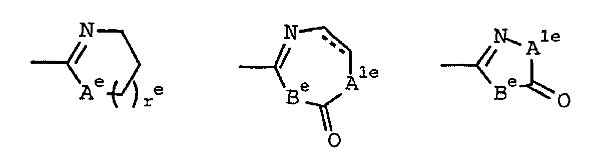 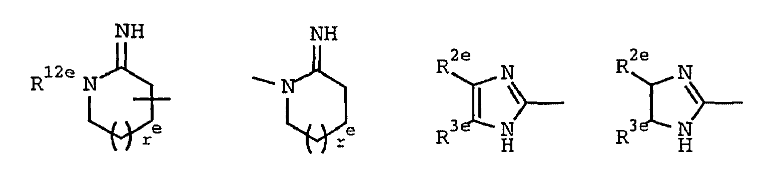 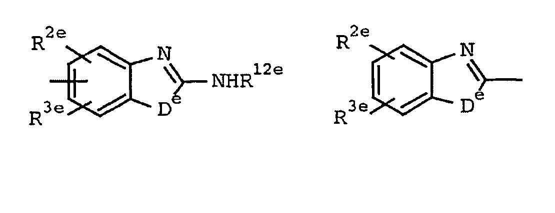 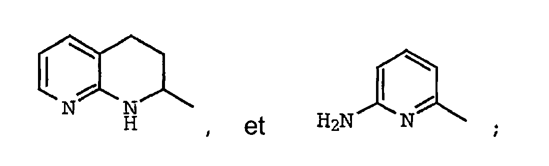
A^{e} représente -CH₂- ou -N(R^{10e})- ;
A^{1e} et B^{e} représentent indépendamment -CH₂- ou -N(R^{10e})- ;
De représente -N(R^{10e})- ou -S- ;
E^{e}-F^{e} représente -C(R^{2e})=C(R^{3e})- ou -C(R^{2e})₂C(R^{3e})₂- ;
J^{e} représente -C(R^{2e})- ou -N- ;
K^{e}, L^{e} et M^{e} représentent indépendamment -C(R^{2e})- ou -C(R^{3e})- ;
R^{2e} et R^{3e} sont indépendamment choisis parmi :
H, un groupe alcoxy en C₁-C₄, NR^{11e}R^{12e}, un halogène, NO₂, CN, CF₃, un groupe alkyle en C₁-C₆, un groupe alcényle en C₃-C₆, un groupe cycloalkyle en C₃-C₇, un groupe cycloalkyle(alkyle en C₁-C₄) en C₃-C₇, un groupe aryle(alkyle en C₁-C₆), un groupe (alkyle en C₁-C₆)carbonyle, un groupe (alcoxy en C₁-C₆)carbonyle, un groupe arylcarbonyle, et un groupe aryle substitué par 0 à 4 R^{7e},
en variante, lorsque R^{2e} et R^{3e} sont des substituants liés à des atomes adjacents, ils peuvent former conjointement avec les atomes de carbone auxquels ils sont liés un carbocycle à 5-7 chaînons ou un hétérocycle à 5-7 chaînons, aromatique ou non aromatique, ledit carbocycle ou hétérocycle étant substitué par 0 à 2 groupes choisis parmi un groupe alkyle en C₁-C₄, un groupe alcoxy en C₁-C₄, un groupe halogéno, un groupe cyano, un groupe amino, CF₃ et NO₂ ;
R^{2ae} est choisi parmi :
H, un groupe alkyle en C₁-C₁₀, un groupe alcényle en C₂-C₆, un groupe cycloalkyle en C₃-C₁₁, un groupe cycloalkyl(alkyle en C₁-C₄) en C₃-C₇, un groupe aryle, un groupe aryle(alkyle en C₁-C₄), un groupe (alkyle en C₂-C₇)carbonyle, un groupe arylcarbonyle, un groupe (alcoxy en C₂-C₁₀)carbonyle, un groupe cycloalcoxycarbonyle en C₃-C₇, un groupe bicycloalcoxycarbonyle en C₇-C₁₁, un groupe aryloxycarbonyle, un groupe aryl(alcoxy en C₁-C₁₀)carbonyle, un groupe alkylcarbonyloxy(alcoxy en C₁-C₄)carbonyle en C₁-C₆, un groupe arylcarbonyloxy(alcoxy en C₁-C₄)carbonyle, et un groupe cycloalkylcarbonyloxy(alcoxy en C₁-C₄)carbonyle en C₃-C₇ ;
R^{7e} est choisi parmi :
H, un groupe hydroxy, un groupe alkyle en C₁-C₄, un groupe alcoxy en C₂-C₄, un groupe aryle, un groupe aryle(alkyle en C₁-C₄), un groupe (alkyle en C₁-C₄)carbonyle, CO₂R^{18ae}, SO₂R^{11e}, SO₂NR^{10e}R^{11e}, OR^{10e}, et N(R^{11e})R^{12e};
U^{e} est choisi parmi :
-(CH₂)ₙe-, -(CH₂)ₙeO(CH₂)ₘe-, -(CH₂)ₙeN(R¹²)(CH₂)ₘe-, -NH(CH₂)ₙe-, -(CH₂)ₙeC(=O)(CH₂)ₘe-, -(CH₂)ₙeS(O)ₚe(CH₂)ₘe-, -(CH₂)ₙeNHNH(CH₂)ₘe-, -N(R^{10e})C(=O)-, -NHC(=O)(CH₂)ₙe-, -C(=O)N(R^{10e})-, et -N(R^{10e})S(O)ₚe-;
G^{e} représente N ou CR^{19e} ;
W^{e} représente -C(=O)-N(R^{10e})-(alkylène en C₁-C₃)-, où le groupe alkylène est substitué par R^{8e} et par R^{9e} :
R^{8e} et R^{9e} sont indépendamment choisis parmi :
H, CO₂R^{18be}, C(=O)R^{18be}, CONR¹⁷R^{18be}, un groupe alkyle en C₁-C₁₀ substitué par 0 à 1 R^{6e}, un groupe alcényle en C₂-C₁₀ substitué par 0 à 1 R^{6e}, un groupe alcynyle en C₂-C₁₀ substitué par 0 à 1 R^{6e}, un groupe cycloalkyle en C₃-C₈ substitué par 0 à 1 R^{6e}, un groupe cycloalcényle en C₅-C₆ substitué par 0 à 1 R^{6e}, un groupe (alkyle en C₃-C₁₀)carbonyle, un groupe cycloalkyle(alkyle en C₁-C₄) en C₃-C₁₀, un groupe phényle substitué par 0 à 3 R^{6e}, un groupe naphtyle substitué par 0 à 3 R^{6e}, un hétérocycle à 5-10 chaînons contenant 1 à 3 hétéroatomes N, O ou S, ledit hétérocycle pouvant être saturé, partiellement saturé, ou entièrement insaturé, et ledit hétérocycle étant substitué par 0 à 2 R^{7e}, un groupe alcoxy en C₁-C₁₀ substitué par 0 à 2 R^{7e}, un groupe hydroxy, un groupe nitro, -N(R^{10e})R^{11e}, -N(R^{16e})R^{17e}, un groupe aryl(alkyle en C₀-C₆)carbonyle, aryl(alkyle en C₃-C₆), hétéroaryl(alkyle en C₁-C₆), CONR^{18ae}R^{20e}, SO₂R^{18ae}, et SO₂NR^{18ae}R^{20e},
sachant que chacun desdits groupes alkyles, cycloalkyles, aryles ou hétéroaryles peut être non substitué ou substitué de manière dépendante par 1 à 2 R^{17e} ;
R^{6e} est choisi parmi :
H, un groupe alkyle en C₁-C₁₀, un groupe hydroxy, un groupe alcoxy en C₁-C₁₀, un groupe nitro, un groupe alkylcarbonyle en C₁-C₁₀, -N(R^{11e})R^{12e}, un groupe cyano, un groupe halogéno, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e}, un groupe aryle substitué par 0 à 3 groupes choisis parmi un halogène, un groupe alcoxy en C₁-C₆, un groupe alkyle en C₁-C₆, CF₃, S(O)ₘeMe, et -NMe₂, un groupe aryle(alkyle en C₁-C₄), ledit groupe aryle étant substitué par 0 à 3 groupes choisis parmi un halogène, un groupe alcoxy en C₁-C₆, un groupe alkyle en C₁-C₆, CF₃, S(O)ₘeMe, et -NMe₂, et un hétérocycle à 5-10 chaînons contenant 1 à 3 hétéroatomes N, O, ou S, ledit hétérocycle pouvant être saturé, partiellement saturé ou entièrement insaturé, ledit hétérocycle étant substitué par 0 à 2 R^{7e}:
R^{10e} est choisi parmi :
H, -OH, CF₃, un groupe alcényle en C₃-C₆, un groupe cycloalkyle en C₃-C₁₁, un groupe aryle, un groupe (cycloalkyle en C₃-C₁₁)méthyle, un groupe aryle(alkyle en C₁-C₄), et un groupe alkyle en C₁-C₁₀ substitué par 0 à 2 R^{6e} ;
R^{11e} est choisi parmi :
H, un groupe hydroxy, un groupe alkyle en C₁-C₈, un groupe alcényle en C₃-C₆, un groupe cycloalkyle en C₃-C₁₁, un groupe (cycloalkyle en C₃-C₁₁)méthyle, un groupe alcoxy en C₁-C₆, un groupe benzyloxy, un groupe aryle, un groupe hétéroaryle, un groupe hétéroaryle(alkyle en C₁-C₄), un groupe aryle(alkyle en C₁-C₄), un groupe adamantylméthyle, et un groupe alkyle en C₁-C₁₀ substitué par 0 à 2 R^{4e} ;
R^{4e} est choisi parmi :
H, un groupe alkyle en C₁-C₆, un groupe cycloalkyle en C₃-C₇, un groupe cycloalkyle(alkyle en C₁-C₄) en C₃-C₇, un groupe (alkyle en C₁-C₁₀)carbonyle, un groupe aryle, un groupe hétéroaryle, un groupe aryle(alkyle en C₁-C₆), et un groupe hétéroaryle(alkyle en C₁-C₆), lesdits groupes aryles ou hétéroaryles étant substitués par 0 à 2 substituants indépendamment choisis dans le groupe constitué par un groupe alkyle en C₁-C₄, un groupe alcoxy en C₁-C₄, F, Cl, Br, CF₃ et NO₂,
en variante, lorsque, R^{10e} et R^{11e} sont tous deux des substituants liés au même atome d'azote (comme dans -NR^{10e}R^{11e}), ils peuvent former conjointement avec l'atome d'azote auquel ils sont liés un hétérocycle choisi parmi :
le 3-azabicyclononyle, le 1,2,3,4-tétrahydro-1-quinolinyle, le 1,2,3,4-tétrahydro-2-isoquinolinyle, le 1-pipéridinyle, le 1-morpholinyle, le 1- pyrrolidinyle, le thiamorpholinyle, le thiazolidinyle, et le 1-pipérazinyle ; ledit hétérocycle étant substitué par 0 à 3 groupes choisis parmi :
un groupe alkyle en C₁-C₆, un groupe aryle, un groupe hétéroaryle, un groupe aryle(alkyle en C₁-C₄), un groupe (alkyle en C₁-C₆)carbonyle, un groupe (cycloalkyle en C₃-C₇)carbonyle, un groupe (alcoxy en C₁-C₆)carbonyle, un groupe aryle(alcoxy en C₁-C₄)carbonyle, un groupe alkylsulfonyle en C₁-C₆, et un groupe arylsulfonyle ;
R^{12e} est choisi parmi :
H, un groupe alkyle en C₁-C₆, un groupe triphénylméthyle, un groupe méthoxyméthyle, un groupe méthoxyphényldiphénylméthyle, un groupe triméthylsilyléthoxyméthyle, un groupe (alkyle en C₁-C₆)carbonyle, un groupe (alcoxy en C₁-C₆)carbonyle, un groupe (alkyle en C₁-C₆)aminocarbonyle, un groupe alcényle en C₃-C₆, un groupe cycloalkyle en C₃-C₇, un groupe cycloalkyle(alkyle en C₁-C₄) en C₃-C₇, un groupe aryle, un groupe hétéroaryle(alkyle en C₁-C₆)carbonyle, un groupe hétéroarylcarbonyle, un groupe aryle(alkyle en C₁-C₆), un groupe (alkyle en C₁-C₆)carbonyle, un groupe arylcarbonyle, un groupe alkylsulfonyle en C₁-C₆, un groupe arylsulfonyle, un groupe aryle(alkyle en C₁-C₆)sulfonyle, un groupe hétéroarylsulfonyle, un groupe hétéroaryle(alkyle en C₁-C₆)sulfonyle, un groupe aryloxycarbonyle, et un groupe aryle(alcoxy en C₁-C₆)carbonyle, lesdits groupes aryles étant substitués par 0 à 2 substituants choisis dans le groupe constitué par un groupe alkyle en C₁-C₄, un groupe alcoxy en C₁-C₄, un groupe halogéno, CF₃, et un groupe nitro ;
R^{16e} est choisi parmi :
-C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, et -SO₂NHC(=O)OR^{18be} ;
R^{17e} est choisi parmi :
H, un groupe alkyle en C₁-C₆, un groupe cycloalkyle en C₃-C₇, un groupe cycloalkyle(alkyle en C₁-C₄) en C₃-C₇, un groupe aryle, un groupe aryle(alkyle en C₁-C₆), et un groupe hétéroaryle(alkyle en C₁-C₆);
R^{18ae} est choisi parmi :
un groupe alkyle en C₁-C₈ éventuellement substitué par une liaison à Lₙ, un groupe cycloalkyle en C₃-C₁₁ éventuellement substitué par une liaison à Lₙ, un groupe aryle(alkyle en C₁-C₆) éventuellement substitué par une liaison à Lₙ, un groupe hétéroaryle(alkyle en C₁-C₆) éventuellement substitué par une liaison à Lₙ, un groupe (alkyle en C₁-C₆)hétéroaryle éventuellement substitué par une liaison à Lₙ, un groupe biaryle(alkyle en C₁-C₆) éventuellement substitué par une liaison à Lₙ, un groupe biaryle éventuellement substitué par une liaison à Lₙ, un groupe hétéroaryle éventuellement substitué par une liaison à Lₙ, un groupe phényle substitué par 3 à 4 R^{19e} et éventuellement substitué par une liaison à Lₙ, un groupe naphtyle substitué par 0 à 4 R^{19e} et éventuellement substitué par une liaison à Lₙ, et une liaison à Lₙ, lesdits groupes aryles ou hétéroaryles étant éventuellement substitués par 0 à 4 R^{19e} ;
R^{18be} représente H ou R^{18ae};
R^{19e} est choisi parmi :
H, un halogène, CF₃, CO₂H, CN, NO₂, -NR^{11e}R^{12e}, OCF3, un groupe alkyle en C₁-C₈, un groupe alcényle en C₂-C₆, un groupe alcynyle en C₂-C₆, un groupe cycloalkyle en C₃-C₁₁, un groupe cycloalkyle(alkyle en C₁-C₄) en C₃-C₇, un groupe aryle(alkyle en C₁-C₆), un groupe alcoxy en C₁-C₆, un groupe alcoxycarbonyle en C₁-C₄, un groupe aryle, un groupe aryle-O-, un groupe aryle-SO₂-, un groupe hétéroaryle, et un groupe hétéroaryle-SO₂-, lesdits groupes aryles et hétéroaryles étant substitués par 0 à 4 groupes choisis parmi l'hydrogène, un halogène, CF₃, un groupe alkyle en C₁-C₃, et un groupe alcoxy en C₁-C₃;
R^{20e} est choisi parmi les groupes :
hydroxy, alkyloxy en C₁-C₁₀, cycloalkyloxy en C₃-C₁₁, aryloxy, aryl(alkyle en C₁-C₄)oxy, alkylcarbonyloxy(alkyle en C₁-C₂)oxy en C₂-C₁₀, alcoxycarbonyloxy(alkyle en C₁-C₂)oxy en C₂-C₁₀, alcoxycarbonyl(alkyle en C₁-C₂)oxy en C₂-C₁₀, cycloalkylcarbonyloxy(alkyle en C₁-C₂)oxy en C₃-C₁₀, cycloalcoxycarbonyloxy(alkyle en C₁-C₂)oxy en C₃-C₁₀, cycloalcoxycarbonyl(alkyle en C₁-C₂)oxy en C₃-C₁₀, aryloxycarbonyl(alkyle en C₁-C₂)oxy, aryloxycarbonyloxy(alkyle en C₁-C₂)oxy, arylcarbonyloxy(alkyle en C₁-C₂)oxy, alcoxy(alkyle en C₁-C₅)carbonyloxy(alkyle en C₁-C₂)oxy en C₁-C₅, (5-(alkyle en C₁-C₅)-1,3-dioxa-cyclopentèn-2-one-yl)méthyloxy, (5-aryl-1,3-dioxa-cyclopentèn-2-one-yl)méthyloxy, et (R^{10e})(R^{11e})N(alcoxy en C₁-C₁₀) ;
R^{21e} est choisi parmi les groupes :
alkyle en C₁-C₈, alcényle en C₂-C₆, cycloalkyle en C₃-C₁₁, (cycloalkyle en C₃-C₁₁)méthyle, aryle, aryle(alkyle en C₁-C₄), et alkyle en C₁-C₁₀ substitué par 0 à 2 R^{7e};
R^{22e} est choisi parmi :
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, et -C(=O)NHSO₂NHR^{18be} ;
Y^{e} est choisi parmi :
-COR^{20e}, -SO₃H -PO₃H, -CONHNHSO₂CF₃, -CONHSO₂R^{18ae}, -CONHSO₂NHR^{18be}, -NHCOCF₃, -NHCONHSO₂R^{18ae}, -NHSO₂R^{18ae}, -OPO₃H₂, -OSO₃H, -PO₃H₂, -SO₂NHCOR^{18ae}, -SO²NHCO₂R^{18ae}, 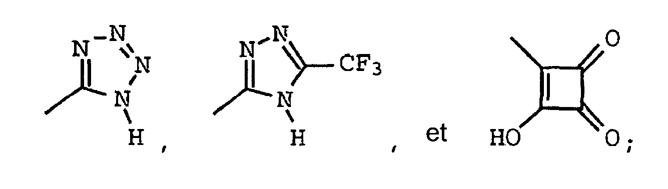
m^{e} est 0-2 ;
n^{e} est 0-4 ;
p^{e} est 0-2 ;
r^{e} est 0-2 ;
avec la clause suivante :
n^{e} et m^{e} sont choisis de telle sorte que le nombre d'atomes reliant R^{1e} et Y^{e} est dans la plage comprise entre 8 et 14 ;
d est choisi parmi 1, 2, 3, 4, 5, 6, 7, 8, 9 et 10 ;
d' est 1-100 ;
Lₙ est un groupe de liaison de formule :
((W)ₕ-(CR⁶R⁷)_{g})ₓ-(Z)ₖ-((CR^{6a}R^{7a})_{g'}-(W)_{h'})_{x'} ;
W est indépendamment choisi à chaque occurrence dans le groupe : O, S, NH, NHC(=O), C(=O)NH, NR⁸C(=O), C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'} (OCH₂CH₂CH₂)_{s"}, (CH₂CH₂CH₂O)ₜ, et (aa)_{t'} ;
aa est indépendamment à chaque occurrence un acide aminé ;
z est choisi dans le groupe :
groupe aryle substitué par 0 à 3 R¹⁰, groupe cycloalkyle en C₃-C₁₀ substitué par 0 à 3 R¹⁰, et hétérocycle à 5-10 chaînons contenant 1 à 4 hétéroatomes indépendamment choisis parmi N, S, et O et substitué par 0 à 3 R¹⁰ ;
R⁶, R^{6a}, R⁷, R^{7a}, et R⁸ sont indépendamment choisis à chaque occurrence dans le groupe :
H, =O, COOH, SO₃H, PO₃H, un groupe alkyle en C₁-C₅ substitué par 0 à 3 R¹⁰, un groupe aryle substitué par 0 à 3 R¹⁰, un groupe benzyle substitué par 0 à 3 R¹⁰, et un groupe alcoxy en C₁-C₅ substitué par 0 à 3 R¹⁰, NHC(=O)R¹¹, C(=O)NHR¹¹, NHC(=O)NHR¹¹, NHR¹¹, R¹¹, et une liaison à Cₕ ;
R¹⁰ est indépendamment choisi à chaque occurrence dans le groupe :
une liaison à Cₕ, COOR¹¹, C(=O)NHR¹¹, NHC(=O)R¹¹, OH, NHR¹¹, SO₃H, PO₃H, OPO₃H₂, OSO₃H, un groupe aryle substitué par 0 à 3 R¹¹, un groupe alkyle en C₁-C₅ substitué par 0 à 1 R¹², un groupe alcoxy en C₁-C₅ substitué par 0 à 1 R¹² , et un hétérocycle à 5-10 chaînons contenant 1 à 4 hétéroatomes indépendamment choisis parmi N, S, et O et substitué par 0 à 3 R¹¹ ;
R¹¹ est indépendamment choisi à chaque occurrence dans le groupe :
H, -OPO₃H₂, -OSO₃H, un groupe alkyle en C₁-C₅ substitué par 0 à 1 R¹², un groupe aryle substitué par 0 à 1 R¹², un hétérocycle à 5-10 chaînons contenant 1 à 4 hétéroatomes indépendamment choisis parmi N, S, et O et substitué par 0 à 1 R¹², un groupe cycloalkyle en C₃-C₁₀ substitué par 0 à 1 R¹², un polyalkylène glycol substitué par 0 à 1 R¹², un groupe alkyle en C₁-C₅ substitué par un hydrate de carbone substitué par 0 à 1 R¹², une cyclodextrine substituée par 0 à 1 R¹², un acide aminé substitué par 0 à 1 R¹², un groupe polycarboxyalkyle substitué par 0 à 1 R¹², un groupe polyazaalkyle substitué par 0 à 1 R¹², un peptide substitué par 0 à 1 R¹², ledit peptide étant constitué de 2 à 10 acides aminés, un groupe alkyle en C₁-C₅ substitué par un groupe 3,6-O-disulfo-B-D-galactopyranosyle, une bis(phosphonométhyl)glycine, et une liaison à Cₕ;
R¹² est une liaison à Cₕ ;
k est choisi parmi 0,1, et 2 ;
h est choisi parmi 0, 1, et 2 ;
h' est choisi parmi 0,1, et 2 ;
g est choisi parmi 0,1, 2, 3, 4, 5, 6, 7, 8, 9 et 10;
g' est choisi parmi 0, 1, 2, 3, 4, 5, 6, 7, 8, 9 et 10 ;
s est choisi parmi 0, 1, 2, 3, 4, 5, 6, 7, 8, 9 et 10 ;
s' est choisi parmi 0, 1, 2, 3, 4, 5, 6, 7, 8, 9 et 10 ;
s" est choisi parmi 0, 1, 2, 3, 4, 5, 6, 7, 8, 9 et 10 ;
t est choisi parmi 0, 1, 2, 3, 4, 5, 6, 7, 8, 9 et 10 ;
t' est choisi parmi 0, 1, 2, 3, 4, 5, 6, 7, 8, 9 et 10 ;
x est choisi parmi 0, 1, 2, 3, 4, 5, et 6 ;
x' est choisi parmi 0, 1, 2, 3, 4, 5, et 6 ;
Cₕ est un groupement permettant de lier un métal, dont la formule est choisie dans le groupe : 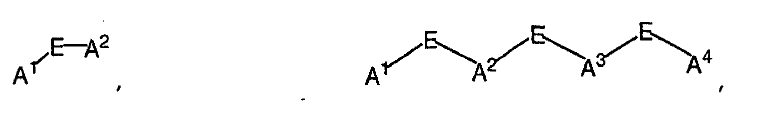 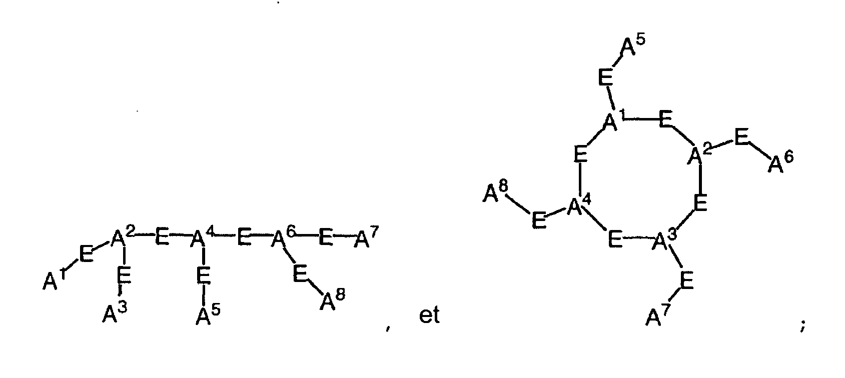
A¹, A², A³, A⁴, A⁵, A⁶, A⁷ et A⁸ sont indépendamment choisis à chaque occurrence dans le groupe :
NR¹³, NR¹³R¹⁴, S, SH, S(Pg), O, OH, PR¹³ , PR¹³R¹⁴, P(O)R¹⁵R¹⁶, et une liaison à Lₙ ;
E est une liaison, CH, ou un groupe espaceur indépendamment choisi à chaque occurrence dans le groupe :
un groupe alkyle en C₁-C₁₀ substitué par 0 à 3 R¹⁷, un groupe aryle substitué par 0 à 3 R¹⁷, un groupe cycloalkyle en C₃-C₁₀ substitué par 0 à 3 R¹⁷, un groupe hétérocyclo(C₁-C₁₀)alkyle substitué par 0 à 3 R¹⁷, ledit groupe hétérocyclo étant un hétérocycle à 5-10 chaînons contenant 1 à 4 hétéroatomes indépendamment choisis parmi N, S, et O, un groupe aryle(C₁-C₁₀)alkyle en C₆-C₁₀ substitué par 0 à 3 R¹⁷, un groupe alkyle(C₆-C₁₀)alkyle en C₁-C₁₀ substitué par 0 à 3 R¹⁷, et un hétérocycle à 5-10 chaînons contenant 1 à 4 hétéroatomes indépendamment choisis parmi N, S, et O et substitué par 0 à 3 R¹⁷ ;
R¹³ et R¹⁴ sont chacun indépendamment choisis dans le groupe :
une liaison à Lₙ, un hydrogène, un groupe alkyle en C₁-C₁₀ substitué par 0 à 3 R¹⁷, un groupe aryle substitué par 0 à 3 R¹⁷, un groupe cycloalkyle en C₁-C₁₀ substitué par 0 à 3 R¹⁷, un groupe hétérocyclo(C₁-C₁₀)alkyle substitué par 0 à 3 R¹⁷, ledit groupe hétérocyclo étant un hétérocycle à 5-10 chaînons contenant 1 à 4 hétéroatomes indépendamment choisis parmi N, S et O, un groupe aryl(C₁-C₁₀)alkyle en C₆-C₁₀ substitué par 0 à 3 R¹⁷, un groupe alkyl(C₆-C₁₀)aryle en C₁-C₁₀ substitué par 0 à 3 R¹⁷, un hétérocyle à 5-10 chaînons contenant 1 à 4 hétéroatomes indépendamment choisis parmi N, S et O et substitués par 0 à 3 R¹⁷ , et un électron, sachant que lorsque l'un parmi R¹³ et R¹⁴ est un électron, alors l'autre est aussi un électron ;
en variante, R¹³ et R¹⁴ se combinent pour former =C(R²⁰)(R²¹) ;
R¹⁵ et R¹⁶ sont chacun indépendamment choisis dans le groupe :
une liaison à Lₙ, -OH, un groupe alkyle en C₁-C₁₀ substitué par 0 à 3 R¹⁷, un groupe aryle substitué par 0 à 3 R¹⁷, un groupe cycloalkyle en C₃-C₁₀ substitué par 0 à 3 R¹⁷, un groupe hétérocyclo(C₁-C₁₀)alkyle substitué par 0 à 3 R¹⁷, ledit groupe hétérocyclo étant un hétérocycle à 5-10 chaînons contenant 1 à 4 hétéroatomes indépendamment choisis parmi N, S, et O, un groupe aryle(C₁-C₁₀)alkyle en C₆-C₁₀ substitué par 0 à 3 R¹⁷, un groupe alkyle(C₁-C₁₀)aryle en C₁-C₁₀ substitué par 0 à 3 R¹⁷ , et un hétérocycle à 5-10 chaînons contenant 1 à 4 hétéroatomes indépendamment choisis parmi N, S, et O et substitué par 0 à 3 R¹⁷;
R¹⁷ est indépendamment choisi à chaque occurrence dans le groupe :
une liaison à Lₙ, =O, F, CI, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a} , -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H, le groupe 2-(1-morpholino)éthoxy, un groupe alkyle en C₁-C₅, un groupe alcényle en C₂-C₄, un groupe cycloalkyle en C₃-C₆, un groupe cycloalkylméthyle en C₃-C₆, un groupe alcoxyalkyle en C₂-C₆, un groupe aryle substitué par 0 à 2 R¹⁸, et un hétérocycle à 5-10 chaînons contenant 1 à 4 hétéroatomes indépendamment choisis parmi N, S et O ;
R¹⁸, R^{18a} et R¹⁹ sont indépendamment choisis à chaque occurrence dans le groupe :
une liaison à Lₙ, H, un groupe alkyle en C₁-C₆, un groupe phényle, un groupe benzyle, un groupe alcoxy en C₁-C₆, un halogénure, un groupe nitro, un groupe cyano, et un groupe trifluorométhyle ;
Pg est un groupe protecteur de thiol ;
R²⁰ et R²¹ sont indépendamment choisis dans le groupe :
H, un groupe alkyle en C₁-C₁₀, -CN, -CO₂R²⁵, -C(=O)R²⁵, -C(=O)N(R²⁵)2, un 1-alcène en C₂-C₁₀, substitué par 0 à 3 R²³, un 1-alcyne en C₂-C₁₀ substitué par 0 à 3 R²³, un groupe aryle substitué par 0 à 3 R²³, un hétérocycle insaturé à 5-10 chaînons contenant 1 à 4 hétéroatomes indépendamment choisis parmi N, S, et O et substitué par 0 à 3 R²³, et un carbocycle insaturé en C₃-C₁₀ substitué par 0 à 3 R²³ ;
en variante, R²⁰ et R²¹ forment conjointement avec le radical carboné divalent auquel ils sont liés : 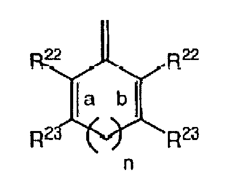
R²² et R²³ sont indépendamment choisis parmi le groupe :
H, R²⁴, un groupe alkyle en C₁-C₁₀ substitué par 0 à 3 R²⁴, un groupe alcényle en C₂-C₁₀ substitué par 0 à 3 R²⁴, un groupe alcynyle en C₂-C₁₀ substitué par 0 à 3 R²⁴, un groupe aryle substitué par 0 à 3 R²⁴, un hétérocycle à 5-10 chaînons contenant 1 à 4 hétéroatomes indépendamment choisis parmi N, S, et O et substitué par 0 à 3 R²⁴, et un carbocycle en C₃-C₁₀ substitué par 0 à 3 R²⁴ ; en variante, R²² et R²³ forment ensemble un cycle aromatique ou un hétérocycle à 5-10 chaînons contenant 1 à 4 hétéroatomes indépendamment choisis parmi N, S, et O ;
a et b indiquent les positions de doubles liaisons éventuelles, et n vaut 0 ou 1 ;
R²⁴ est indépendamment choisi à chaque occurrence dans le groupe : =0, F, CI, Br, I, -CF₃, -CN, -CO₂R²⁵, -C(=O)R²⁵, -C(=O)N(R²⁵)2, -N(R²⁵)₃⁺, -CH₂OR²⁵, -OC(=O)R²⁵, -OC(=O)OR^{25a}, -OR²⁵, -OC(=O)N(R²⁵)_{2,} -NR²⁶C(=O)R²⁵, -NR²⁶C(=O)OR^{25a}, -NR²⁶C(=O)N(R²⁵)₂, -N²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, et le groupe 2-(1-morpholino)éthoxy ; et,
R²⁵ , R^{25a}, et R²⁶ sont chacun indépendamment choisis à chaque occurrence dans le groupe : un hydrogène et un groupe alkyle en C₁-C₆ ; ou un sel de ceux-ci acceptable sur le plan pharmaceutique.

2. Trousse selon la revendication 1, ladite trousse comprenant une pluralité de contenants séparés, au moins l'un desdits contenants contenant un ou plusieurs agents choisis dans le groupe constitué par un agent anticancéreux et un agent de radiosensibilisation, ou un sel de ceux-ci acceptable sur le plan pharmaceutique, et un support acceptable sur le plan pharmaceutique, et un autre desdits contenants contenant un composé de formule (I) :
(Q)_{d}-Lₙ-Cₕ ou (Q)_{d}-Lₙ-(Cₕ)_{d'} (I)
dans laquelle Q est un composé de formule (II) : 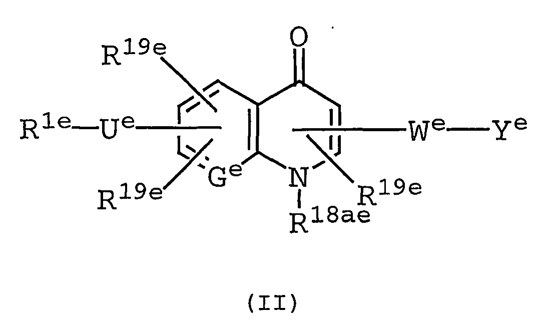 incluant les formes stéréoisomériques de celle-ci, ou les mélanges des formes stéréoisomériques de celle-ci, ou les formes de sel ou de promédicament de celle-ci acceptables sur le plan pharmaceutique, dans laquelle:
R^{1e} est choisi parmi : 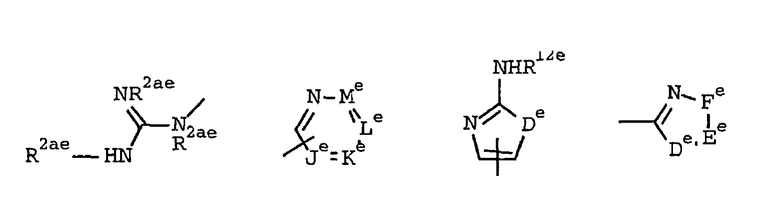 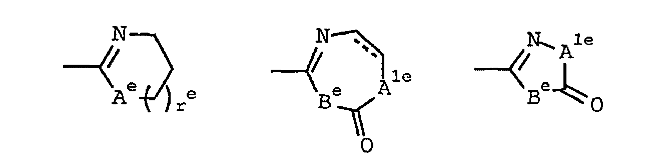 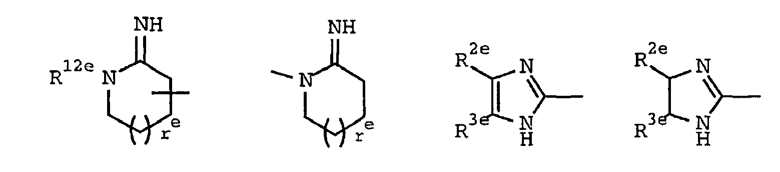 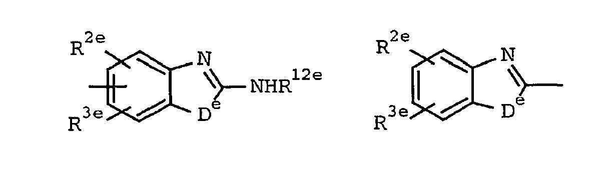 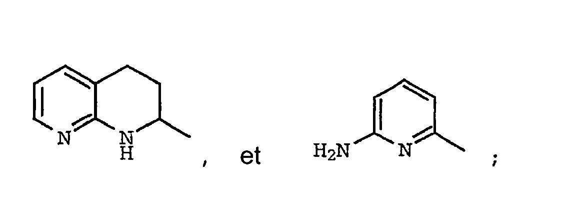
A^{e} représente -CH₂- ou -N(R^{10e})-;
A^{1e} et B^{e} représentent indépendamment -CH₂- ou -N(R^{10e})-;
D^{e} représente -N(R^{10e})- ou -S- ;
E^{e}-F^{e} représente -C(R^{2e})=C(R^{3e})- ou -C(R^{2e})₂C(R^{3e})₂-;
J^{e} représente -C(R^{2e})- ou -N- ;
K^{e}, L^{e} et M^{e} représentent indépendamment -C(R^{2e})- ou -C(R^{3e})- ;
R^{2e} et R^{3e} sont indépendamment choisis parmi :
H, un groupe alcoxy en C₁-C₄, NR^{11e}R^{12e}, un halogène, NO₂, CN, CF₃, un groupe alkyle en C₁-C₆, un groupe alcényle en C₃-C₆, un groupe cycloalkyle en C₃-C₇, un groupe cycloalkyle(alkyle en C₁-C₄) en C₃-C₇, un groupe aryle(alkyle en C₁-C₆), un groupe (alkyle en C₁-C₆)carbonyle, un groupe (alcoxy en C₁-C₆)carbonyle, un groupe arylcarbonyle, et un groupe aryle substitué par 0 à 4 R^{7e},
en variante, lorsque R^{2e} et R^{3e} sont des substituants liés à des atomes adjacents, ils peuvent former conjointement avec les atomes de carbone auxquels ils sont liés un carbocycle à 5-7 chaînons ou un hétérocycle à 5-7 chaînons, aromatique ou non aromatique, ledit carbocycle ou hétérocycle étant substitué par 0 à 2 groupes choisis parmi un groupe alkyle en C₁-C₄, un groupe alcoxy en C₁-C₄, un groupe halogéno, un groupe cyano, un groupe amino, CF₃ et NO₂ ;
R^{2ae} est choisi parmi :
H, un groupe alkyle en C₁-C₁₀, un groupe alcényle en C₂-C₆, un groupe cycloalkyle en C₃-C₁₁, un groupe cycloalkyl(alkyle en C₁-C₄) en C₃-C₇, un groupe aryle, un groupe aryle(alkyle en C₁-C₄), un groupe (alkyle en C₂-C₇)carbonyle, un groupe arylcarbonyle, un groupe (alcoxy en C₂-C₁₀)carbonyle, un groupe cycloalcoxycarbonyle en C₃-C₇, un groupe bicycloalcoxycarbonyle en C₇-C₁₁, un groupe aryloxycarbonyle, un groupe aryl(alcoxy en C₁-C₁₀)carbonyle, un groupe alkylcarbonyloxy(alcoxy en C₁-C₄)carbonyle en C₁-C₆, un groupe arylcarbonyloxy(alcoxy en C₁-C₄)carbonyle, et un groupe cycloalkylcarbonyloxy(alcoxy en C₁-C₄)carbonyle en C₃-C₇ ;
R^{7e} est choisi parmi :
H, un groupe hydroxy, un groupe alkyle en C₁-C₄, un groupe alcoxy en C₂-C₄, un groupe aryle, un groupe aryle(alkyle en C₁-C₄), un groupe (alkyle en C₁-C₄)carbonyle, CO₂R^{18ae}, SO₂R^{11e}, SO₂NR^{10e}R^{11e}, OR^{10e}, et N(R^{11e})R^{12e} ;
U^{e} est choisi parmi :
-(CH₂)ₙe-, -(CH₂)ₙeO(CH₂)ₘe-, -(CH₂)ₙeN(R¹²)(CH₂)ₘe-, -NH(CH₂)ₙe-, -(CH₂)ₙec(₌O)(CH₂)ₘe-, -(CH₂)ₙeS(O)ₚe(CH₂)ₘe-, -(CH₂)ₙeNHNH(CH₂)ₘe-, -N(R^{10e})C(=O)-, -NHC(=O)(CH₂)ₙe-, -C(=O)N(R^{10e})-, et -N(R^{10e})S(O)ₚe-;
G^{e} représente N ou CR^{19e} ;
W^{e} représente -C(=O)-N(R^{10e})-(alkylène en C₁-C₃)-, où le groupe alkylène est substitué par R^{8e} et par R^{9e} :
R^{8e} et R^{9e} sont indépendamment choisis parmi :
H, CO₂R^{18be}, C(=O)R^{18be}, CONR¹⁷R^{18be}, un groupe alkyle en C₁-C₁₀ substitué par 0 à 1 R^{6e}, un groupe alcényle en C₂-C₁₀ substitué par 0 à 1 R^{6e}, un groupe alcynyle en C₂-C₁₀ substitué par 0 à 1 R^{6e}, un groupe cycloalkyle en C₃-C₈ substitué par 0 à 1 R^{6e}, un groupe cycloalcényle en C₅-C₆ substitué par 0 à 1 R^{6e}, un groupe (alkyle en C₃-C₁₀)carbonyle, un groupe cycloalkyle(alkyle en C₁-C₄) en C₃-C₁₀, un groupe phényle substitué par 0 à 3 R^{6e}, un groupe naphtyle substitué par 0 à 3 R^{6e}, un hétérocycle à 5-10 chaînons contenant 1 à 3 hétéroatomes N, O ou S, ledit hétérocycle pouvant être saturé, partiellement saturé, ou entièrement insaturé, et ledit hétérocycle étant substitué par 0 à 2 R^{7e}, un groupe alcoxy en C₁-C₁₀ substitué par 0 à 2 R^{7e}, un groupe hydroxy, un groupe nitro, -N(R^{10e})R11^{e}, -N(R^{16e})R^{17e}, un groupe aryl(alkyle en C₀-C₆)carbonyle, aryl(alkyle en C₃-C₆), hétéroaryl(alkyle en C₁-C₆), CONR^{18ae}R^{20e}, SO₂R^{18ae}, et SO₂NR^{18ae}R^{20e},
sachant que chacun desdits groupes alkyles, cycloalkyles, aryles ou hétéroaryles peut être non substitué ou substitué de manière indépendante par 1 à 2 R^{7e} ;
R^{6e} est choisi parmi :
H, un groupe alkyle en C₁-C₁₀, un groupe hydroxy, un groupe alcoxy en C₁-C₁₀, un groupe nitro, un groupe alkylcarbonyle en C₁-C₁₀, -N(R11^{e})R^{12e}, un groupe cyano, un groupe halogéno, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e}, un groupe aryle substitué par 0 à 3 groupes choisis parmi un halogène, un groupe alcoxy en C₁-C₆, un groupe alkyle en C₁-C₆, CF₃, S(O)ₘeMe, et -NMe₂, un groupe aryle(alkyle en C₁-C₄), ledit groupe aryle étant substitué par 0 à 3 groupes choisis parmi un halogène, un groupe alcoxy en C₁-C₆, un groupe alkyle en C₁-C₆, CF₃, S(O)ₘeMe, et -NMe₂, et un hétérocycle à 5-10 chaînons contenant 1 à 3 hétéroatomes N, O, ou S, ledit hétérocycle pouvant être saturé, partiellement saturé ou entièrement insaturé, ledit hétérocycle étant substitué par 0 à 2 R^{7e};
R^{10e} est choisi parmi :
H, -OH, CF₃, un groupe alcényle en C₃-C₆, un groupe cycloalkyle en C₃-C₁₁, un groupe aryle, un groupe (cycloalkyle en C₃-C₁₁)méthyle, un groupe aryle(alkyle en C₁-C₄), et un groupe alkyle en C₁-C₁₀ substitué par 0 à 2 R^{6e} ;
R^{11e} est choisi parmi :
H, un groupe hydroxy, un groupe alkyle en C₁-C₈, un groupe alcényle en C₃-C₆, un groupe cycloalkyle en C₃-C₁₁, un groupe (cycloalkyle en C₃-C₁₁)méthyle, un groupe alcoxy en C₁-C₆, un groupe benzyloxy, un groupe aryle, un groupe hétéroaryle, un groupe hétéroaryle(alkyle en C₁-C₄), un groupe aryle(alkyle en C₁-C₄), un groupe adamantylméthyle, et un groupe alkyle en C₁-C₁₀ substitué par 0 à 2 R^{4e};
R^{4e} est choisi parmi :
H, un groupe alkyle en C₁-C₆, un groupe cycloalkyle en C₃-C₇, un groupe cycloalkyle(alkyle en C₁-C₄) en C₃-C₇, un groupe (alkyle en C₁-C₁₀)carbonyle, un groupe aryle, un groupe hétéroaryle, un groupe aryle(alkyle en C₁-C₆), et un groupe hétéroaryle(alkyle en C₁-C₆), lesdits groupes aryles ou hétéroaryles étant substitués par 0 à 2 substituants indépendamment choisis dans le groupe constitué par un groupe alkyle en C₁-C₄, un groupe alcoxy en C₁-C₄, F, CI, Br, CF₃ et NO₂,
en variante, lorsque, R^{10e} et R^{11e} sont tous deux des substituants liés au même atome d'azote (comme dans -NR^{10e}R^{11e}), ils peuvent former conjointement avec l'atome d'azote auquel ils sont liés un hétérocycle choisi parmi :
le 3-azabicyclononyle, le 1,2,3,4-tétrahydro-1-quinolinyle, le 1,2,3,4-tétrahydro-2-isoquinolinyle, le 1-pipéridinyle, le 1-morpholinyle, le 1- pyrrolidinyle, le thiamorpholinyle, le thiazolidinyle, et le 1-pipérazinyle ; ledit hétérocycle étant substitué par 0 à 3 groupes choisis parmi :
un groupe alkyle en C₁-C₆, un groupe aryle, un groupe hétéroaryle, un groupe aryle(alkyle en C₁-C₄), un groupe (alkyle en C₁-C₆)carbonyle, un groupe (cycloalkyle en C₃-C₇)carbonyle, un groupe (alcoxy en C₁-C₆)carbonyle, un groupe aryle(alcoxy en C₁-C₄)carbonyle, un groupe alkylsulfonyle en C₁-C₆, et un groupe arylsulfonyle ;
R^{12e} est choisi parmi :
H, un groupe alkyle en C₁-C₆, un groupe triphénylméthyle, un groupe méthoxyméthyle, un groupe méthoxyphényldiphénylméthyle, un groupe triméthylsilyléthoxyméthyle, un groupe (alkyle en C₁-C₆)carbonyle, un groupe (alcoxy en C₁-C₆)carbonyle, un groupe (alkyle en C₁-C₆)aminocarbonyle, un groupe alcényle en C₃-C₆, un groupe cycloalkyle en C₃-C₇, un groupe cycloalkyle(alkyle en C₁-C₄) en C₃-C₇, un groupe aryle, un groupe hétéroaryle(alkyle en C₁-C₆)carbonyle, un groupe hétéroarylcarbonyle, un groupe aryle(alkyle en C₁-C₆), un groupe (alkyle en C₁-C₆)carbonyle, un groupe arylcarbonyle, un groupe alkylsulfonyle en C₁-C₆, un groupe arylsulfonyle, un groupe aryle(alkyle en C₁-C₆)sulfonyle, un groupe hétéroarylsulfonyle, un groupe hétéroaryle(alkyle en C₁-C₆)sulfonyle, un groupe aryloxycarbonyle, et un groupe aryle(alcoxy en C₁-C₆)carbonyle, lesdits groupes aryles étant substitués par 0 à 2 substituants choisis dans le groupe constitué par un groupe alkyle en C₁-C₄, un groupe alcoxy en C₁-C₄, un groupe halogéno, CF₃, et un groupe nitro ;
R^{16e} est choisi parmi :
-C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, et -SO₂NHC(=O)OR^{18be};
R^{17e} est choisi parmi :
H, un groupe alkyle en C₁-C₆, un groupe cycloalkyle en C₃-C₇, un groupe cycloalkyle(alkyle en C₁-C₄) en C₃-C₇, un groupe aryle, un groupe aryle(alkyle en C₁-C₆), et un groupe hétéroaryle(alkyle en C₁-C₆) ;
R^{18ae} est choisi parmi :
un groupe alkyle en C₁-C₈ éventuellement substitué par une liaison à Lₙ, un groupe cycloalkyle en C₃-C₁₁ éventuellement substitué par une liaison à Lₙ, un groupe aryle(alkyle en C₁-C₆) éventuellement substitué par une liaison à Lₙ, un groupe hétéroaryle(alkyle en C₁-C₆) éventuellement substitué par une liaison à Lₙ, un groupe (alkyle en C₁-C₆)hétéroaryle éventuellement substitué par une liaison à Lₙ, un groupe biaryle(alkyle en C₁-C₆) éventuellement substitué par une liaison à Lₙ, un groupe hétéroaryle éventuellement substitué par une liaison à Lₙ, un groupe phényle substitué par 3 à 4 R^{19e} et éventuellement substitué par une liaison à Lₙ, un groupe naphtyle substitué par 0 à 4 R^{19e} et éventuellement substitué par une liaison à Lₙ, et une liaison à Lₙ, lesdits groupes aryles ou hétéroaryles étant éventuellement substitués par 0 à 4 R^{19e} ;
R^{18be} représente H ou R^{18ae};
R^{19e} est choisi parmi :
H, un halogène, CF₃, CO₂H, CN, NO₂, -NR^{11e}R^{12e}, OCF₃, un groupe alkyle en C₁-C₈, un groupe alcényle en C₂-C₆, un groupe alcynyle en C₂-C₆, un groupe cycloalkyle en C₃-C₁₁, un groupe cycloalkyle(alkyle en C₁-C₄) en C₃-C₇, un groupe aryle(alkyle en C₁-C₆), un groupe alcoxy en C₁-C₆, un groupe alcoxycarbonyle en C₁-C₄, un groupe aryle, un groupe aryle-O-, un groupe aryle-SO₂-, un groupe hétéroaryle, et un groupe hétéroaryle-SO₂-, lesdits groupes aryles et hétéroaryles étant substitués par 0 à 4 groupes choisis parmi l'hydrogène, un halogène, CF₃, un groupe alkyle en C₁-C₃, et un groupe alcoxy en C₁-C₃ ;
R^{20e} est choisi parmi les groupes :
hydroxy, alkyloxy en C₁-C₁₀, cycloalkyloxy en C₃-C₁₁, aryloxy, aryl(alkyle en C₁-C₄)oxy, alkylcarbonyloxy(alkyle en C₁-C₂)oxy en C₂-C₁₀, alcoxycarbonyloxy(alkyle en C₁-C₂)oxy en C₂-C₁₀, alcoxycarbonyl(alkyle en C₁-C₂)oxy en C₂-C₁₀, cycloalkylcarbonyloxy(alkyle en C₁-C₂)oxy en C₃-C₁₀, cycloalcoxycarbonyloxy(alkyle en C₁-C₂)oxy en C₃-C₁₀ cycloalcoxycarbonyl(alkyle en C₁-C₂)oxy en C₃-C₁₀, aryloxycarbonyl(alkyle en C₁-C₂)oxy, aryloxycarbonyloxy(alkyle en C₁-C₂)oxy, arylcarbonyloxy(alkyle en C₁-C₂)oxy, alcoxy(alkyle en C₁-C₅)carbonyloxy(alkyle en C₁-C₂)oxy en C₁-C₅, (5-(alkyle en C₁-C₅)-1,3-dioxa-cyclopentèn-2-one-yl)méthyloxy, (5-aryl-1,3-dioxa-cyclopentèn-2-one-yl)méthyloxy, et (R^{10e})(R11^{e})N(alcoxy en C₃-C₁₀) ;
R^{21e} est choisi parmi les groupes :
alkyle en C₁-C₈, alcényle en C₂-C₆, cycloalkyle en C₃-C₁₁, (cycloalkyle en C₃-C₁₁)méthyle, aryle, aryle(alkyle en C₁-C₄), et alkyle en C₁-C₁₀ substitué par 0 à 2 R^{7e};
R^{22e} est choisi parmi :
C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, et -C(=O)NHSO₂NHR^{18be} ;
Y^{e} est choisi parmi :
-COR^{20e}, -SO₃H -PO₃H, -CONHNHSO₂CF₃, -CONHSO₂R^{18ae}, -CONHSO₂NHR^{18be}, -NHCOCF₃, -NHCONHSO₂R^{18ae}, -NHSO₂R^{18ae}, -OPO₃H₂, -OSO₃H, -PO₃H₂, -SO₂NHCOR^{18ae}, -SO₂NHCO₂R^{18ae}, 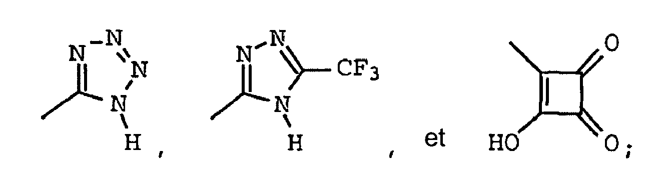
m^{e} est 0-2 ;
n^{e} est 0-4 ;
p^{e} est 0-2 ;
r^{e} est 0-2 ;
avec la clause suivante :
n^{e} et m^{e} sont choisis de telle sorte que le nombre d'atomes reliant R^{1e} et Y^{e} est dans la plage comprise entre 8 et 14 ;
d est choisi parmi 1, 2, 3, 4, 5, 6, 7, 8, 9 et 10 ;
d' est 1-100 ;
Lₙ est un groupe de liaison de formule :
((W)ₕ-(CR⁶R⁷)_{g})ₓ-(Z)ₖ-((CR^{6a}R^{7a})_{g'}-(W)_{h'})_{x'} ;
W est indépendamment choisi à chaque occurrence dans le groupe : O, S, NH, NHC(=O), C(=O)NH, NR⁸C(=O), C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ, et (aa)_{t'} ;
aa est indépendamment à chaque occurrence un acide aminé ;
z est choisi dans le groupe :
groupe aryle substitué par 0 à 3 R¹⁰, groupe cycloalkyle en C₃-C₁₀ substitué par 0 à 3 R¹⁰, et hétérocycle à 5-10 chaînons contenant 1 à 4 hétéroatomes indépendamment choisis parmi N, S, et O et substitué par 0 à 3 R¹⁰;
R⁶, R^{6a}, R⁷, R^{7a}, et R⁸ sont indépendamment choisis à chaque occurrence dans le groupe :
H, =O, COOH, SO₃H PO₃H, un groupe alkyle en C₁-C₅ substitué par 0 à 3 R¹⁰, un groupe aryle substitué par 0 à 3 R¹⁰, un groupe benzyle substitué par 0 à 3 R¹⁰, et un groupe alcoxy en C₁-C₅ substitué par 0 à 3 R¹⁰, NHC(=O)R¹¹, C(=O)NHR¹¹, NHC(=O)NHR¹¹, NHR¹¹, R¹¹, et une liaison à Cₕ ;
R¹⁰ est indépendamment choisi à chaque occurrence dans le groupe :
une liaison à Cₕ, COOR¹¹, C(=O)NHR¹¹, NHC(=O)R¹¹, OH, NHR¹¹, SO₃H, PO₃H, OPO₃H₂, OSO₃H, un groupe aryle substitué par 0 à 3 R¹¹, un groupe alkyle en C₁-C₅ substitué par 0 à 1 R¹², un groupe alcoxy en C₁-C₅ substitué par 0 à 1 R¹² , et un hétérocycle à 5-10 chaînons contenant 1 à 4 hétéroatomes indépendamment choisis parmi N, S, et O et substitué par 0 à 3 R¹¹ ;
R¹¹ est indépendamment choisi à chaque occurrence dans le groupe :
H, -OPO₃H₂, -OSO₃H, un groupe alkyle en C₁-C₅ substitué par 0 à 1 R¹², un groupe aryle substitué par 0 à 1 R¹², un hétérocycle à 5-10 chaînons contenant 1 à 4 hétéroatomes indépendamment choisis parmi N, S, et O et substitué par 0 à 1 R¹², un groupe cycloalkyle en C₃-C₁₀ substitué par 0 à 1 R¹², un polyalkylène glycol substitué par 0 à 1 R¹², un groupe alkyle en C₁-C₅ substitué par un hydrate de carbone substitué par 0 à 1 R¹², une cyclodextrine substituée par 0 à 1 R¹², un acide aminé substitué par 0 à 1 R¹², un groupe polycarboxyalkyle substitué par 0 à 1 R¹², un groupe polyazaalkyle substitué par 0 à 1 R¹², un peptide substitué par 0 à 1 R¹², ledit peptide étant constitué de 2 à 10 acides aminés, un groupe 3,6-O-disulfo-B-D-galactopyranosyle, une bis(phosphonométhyl)glycine, et une liaison à Cₕ ;
R¹² est une liaison à Cₕ ;
k est choisi parmi 0, 1, et 2 ;
h est choisi parmi 0, 1, et 2 ;
h' est choisi parmi 0, 1, et 2 ;
g est choisi parmi 0, 1, 2, 3, 4, 5, 6, 7, 8, 9 et 10 ;
g' est choisi parmi 0, 1, 2, 3, 4, 5, 6, 7, 8, 9 et 10 ;
s est choisi parmi 0, 1, 2, 3, 4, 5, 6, 7, 8, 9 et 10 ;
s' est choisi parmi 0, 1, 2, 3, 4, 5, 6, 7, 8, 9 et 10 ;
s" est choisi parmi 0, 1, 2, 3, 4, 5, 6, 7, 8, 9 et 10 ;
t est choisi parmi 0, 1, 2, 3, 4, 5, 6, 7, 8, 9 et 10 ;
t' est choisi parmi 0, 1, 2, 3, 4, 5, 6, 7, 8, 9 et 10 ;
x est choisi parmi 0, 1, 2, 3, 4, 5, et 6 ;
x' est choisi parmi 0, 1, 2, 3, 4, 5, et 6 ;
Cₕ est un groupement permettant de lier un métal, dont la formule est choisie dans le groupe : 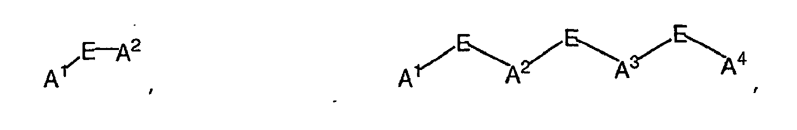 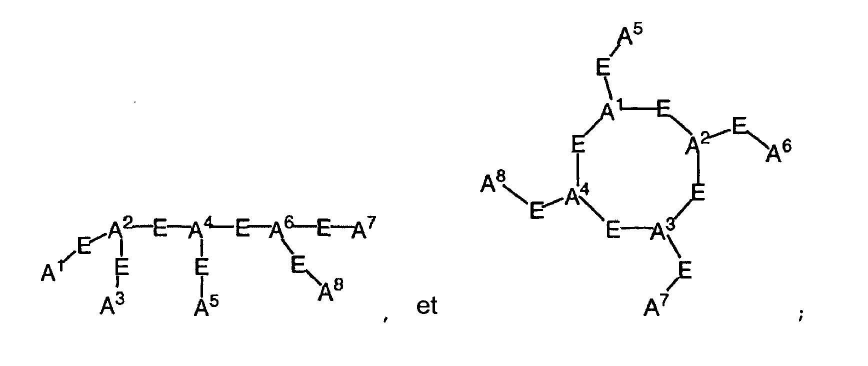
A¹, A², A³, A⁴, A⁵, A⁶, A⁷ et A⁸ sont indépendamment choisis à chaque occurrence dans le groupe :
NR¹³ , NR¹³R¹⁴, S, SH, S(Pg), O, OH, PR¹³, PR¹³R¹⁴, P(O)R¹⁵R¹⁶, et une liaison à Lₙ ;
E est une liaison, CH, ou un groupe espaceur indépendamment choisi à chaque occurrence dans le groupe :
un groupe alkyle en C₁-C₁₀ substitué par 0 à 3 R¹⁷, un groupe aryle substitué par 0 à 3 R¹⁷ un groupe cycloalkyle en C₃-C₁₀ substitué par 0 à 3 R¹⁷, un groupe hétérocyclo(C₁-C₁₀)alkyle substitué par 0 à 3 R¹⁷, ledit groupe hétérocyclo étant un hétérocycle à 5-10 chaînons contenant 1 à 4 hétéroatomes indépendamment choisis parmi N, S, et O, un groupe aryle(C₁-C₁₀)alkyle en C₆-C₁₀ substitué par 0 à 3 R¹⁷, un groupe alkyle(C₆-C₁₀)alkyle en C₁-C₁₀ substitué par 0 à 3 R¹⁷, et un hétérocycle à 5-10 chaînons contenant 1 à 4 hétéroatomes indépendamment choisis parmi N, S, et O et substitué par 0 à 3 R¹⁷;
R¹³ et R¹⁴ sont chacun indépendamment choisis dans le groupe :
une liaison à Lₙ, un hydrogène, un groupe alkyle en C₁-C₁₀ substitué par 0 à 3 R¹⁷, un groupe aryle substitué par 0 à 3 R¹⁷, un groupe cycloalkyle en C₁-C₁₀ substitué par 0 à 3 R¹⁷, un groupe hétérocyclo(C₁-C₁₀)alkyle substitué par 0 à 3R¹⁷, ledit groupe hétérocyclo étant un hétérocycle à 5-10 chaînons contenant 1 à 4 hétéroatomes indépendamment choisis parmi N, S et O, un groupe aryl(C₁-C₁₀)alkyle en C₆-C₁₀ substitué par 0 à 3 R¹⁷, un groupe alkyl(C₆-C₁₀)aryle en C₁-C₁₀ substitué par 0 à 3 R¹⁷, un hétérocyle à 5-10 chaînons contenant 1 à 4 hétéroatomes indépendamment choisis parmi N, S et O et substitués par 0 à 3 R¹⁷, et un électron, sachant que lorsque l'un parmi R¹³ et R¹⁴ est un électron, alors l'autre est aussi un électron ;
en variante, R¹³ et R¹⁴ se combinent pour former =C(R²⁰)(R²¹) ;
R¹⁵ et R¹⁶ sont chacun indépendamment choisis dans le groupe :
une liaison à Lₙ, -OH, un groupe alkyle en C₁-C₁₀ substitué par 0 à 3 R¹⁷, un groupe aryle substitué par 0 à 3 R¹⁷, un groupe cycloalkyle en C₃-C₁₀ substitué par 0 à 3 R¹⁷ un groupe hétérocyclo(C₁-C₁₀)alkyle substitué par 0 à 3 R¹⁷ ledit groupe hétérocyclo étant un hétérocycle à 5-10 chaînons contenant 1 à 4 hétéroatomes indépendamment choisis parmi N, S, et O, un groupe aryle(C₁-C₁₀)alkyle en C₆-C₁₀ substitué par 0 à 3 R¹⁷, un groupe alkyle(C₁-C₁₀)aryle en C₁-C₁₀ substitué par 0 à 3 R¹⁷, et un hétérocycle à 5-10 chaînons contenant 1 à 4 hétéroatomes indépendamment choisis parmi N, S, et O et substitué par 0 à 3 R¹⁷;
R¹⁷ est indépendamment choisi à chaque occurrence dans le groupe :
une liaison à Lₙ, =O, F, CI, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H, le groupe 2-(1-morpholino)éthoxy, un groupe alkyle en C₁-C₅, un groupe alcényle en C₂-C₄, un groupe cycloalkyle en C₃-C₆, un groupe cycloalkylméthyle en C₃-C₆, un groupe alcoxyalkyle en C₂-C₆, un groupe aryle substitué par 0 à 2 R¹⁸, et un hétérocycle à 5-10 chaînons contenant 1 à 4 hétéroatomes indépendamment choisis parmi N, S et O ;
R¹⁸, R^{18a} et R¹⁹ sont indépendamment choisis à chaque occurrence dans le groupe :
une liaison à Lₙ, H, un groupe alkyle en C₁-C₆, un groupe phényle, un groupe benzyle, un groupe alcoxy en C₁-C₆, un halogénure, un groupe nitro, un groupe cyano, et un groupe trifluorométhyle ;
Pg est un groupe protecteur de thiol ;
R²⁰ et R²¹ sont indépendamment choisis dans le groupe :
H, un groupe alkyle en C₁-C₁₀, -CN, -CO₂R²⁵, -C(=O)R²⁵, -C(=O)N(R²⁵)₂, un 1-alcène en C₂-C₁₀, substitué par 0 à 3 R²³, un 1-alcyne en C₂-C₁₀ substitué par 0 à 3 R²³, un groupe aryle substitué par 0 à 3 R²³, un hétérocycle insaturé à 5-10 chaînons contenant 1 à 4 hétéroatomes indépendamment choisis parmi N, S, et O et substitué par 0 à 3 R²³, et un carbocycle insaturé en C₃-C₁₀ substitué par 0 à 3 R²³;
en variante, R²⁰ et R²¹ forment conjointement avec le radical carboné divalent auquel ils sont liés : 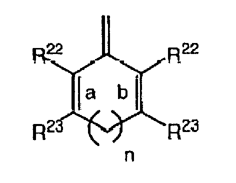
R²² et R²³ sont indépendamment choisis parmi le groupe :
H, R²⁴, un groupe alkyle en C₁-C₁₀ substitué par 0 à 3 R²⁴, un groupe alcényle en C₂-C₁₀ substitué par 0 à 3 R²⁴, un groupe alcynyle en C₂-C₁₀ substitué par 0 à 3 R²⁴, un groupe aryle substitué par 0 à 3 R²⁴, un hétérocycle à 5-10 chaînons contenant 1 à 4 hétéroatomes indépendamment choisis parmi N, S, et O et substitué par 0 à 3 R²⁴, et un carbocycle en C₃-C₁₀ substitué par 0 à 3 R²⁴ ;
en variante, R²² et R²³ forment ensemble un cycle aromatique ou un hétérocycle à 5-10 chaînons contenant 1 à 4 hétéroatomes indépendamment choisis parmi N, S, et O;
a et b indiquent les positions de doubles liaisons éventuelles, et n vaut 0 ou 1 ;
R²⁴ est indépendamment choisi à chaque occurrence dans le groupe :
=O, F, CI, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, et le groupe 2-(1-morpholino)éthoxy ; et,
R²⁵, R^{25a}, et R²⁶ sont chacun indépendamment choisis à chaque occurrence dans le groupe : un hydrogène et un groupe alkyle en C₁-C₆; ou un sel de ceux-ci acceptable sur le plan pharmaceutique.

3. Trousse selon la revendication 1, dans laquelle l'agent anticancéreux est choisi dans le groupe constitué par la mitomycine, la trétinoïne, la ribomustine, la gemcitabine, la vincristine, l'étoposide, la cladribine, le mitobronitol, le méthotrexate, la doxorubicine, la carboquone, la pentostatine, la nitracrine, la zinostatine, le cétrorélix, le létrozole, le raltitrexed, la daunorubicine, la fadrozole, la fotémustine, la thymalfasine, le sobuzoxane, le nédaplatine, la cytarabine, la bicalutamide, la vinorelbine, la vesnarinone, l'aminoglutéthimide, l'amsacrine, le proglumide, l'acétate d'elliptinium, la kétansérine, la doxifluridine, l'étrétinate, l'isotrétinoïne, la streptozocine, la nimustine, la vindésine, la flutamide, le drogénil, la butocine, le carmofur, le razoxane, la sizofilane, le carboplatine, le mitolactol, le tegafur, l'ifosfamide, la prednimustine, le picibanil, le levamisole, le téniposide, l'improsulfane, l'énocitabine, le lisuride, l'oxymétholone, le tamoxifène, la progestérone, le mépitiostane, l'épitiostanol, le formestane, l'interféron-alpha, l'interféron-2 alpha, l'interféron-beta, l'interféron-gamma, le facteur 1 de stimulation de colonies, le facteur 2 de stimulation de colonies, le dénileukine diftitox, l'interleukine 2, et le facteur de libération de l'hormone lutéinisante.

4. Trousse selon la revendication 1, dans laquelle l'agent de radiosensibilisation est choisi dans le groupe constitué par le 2-(3-nitro-1,2,4-triazol-1-yl)-N-(2-méthoxyéthyl)acétamide, la N-(3-nitro-4-quinolinyl)-4-morpholinecarboxamidine, le 3-amino-1,2,4-benzotriazine-1,4-dioxide, le N-(2-hydroxyéthyl)-2-nitroimidazole-1-acétamide, le 1-(2-nitroimidazol-1-yl)-3-(1-pipéridinyl)-2-propanol, et le 1-(2-nitro-1-imidazolyl)-3-(1-aziridino)-2-propanol.

5. Trousse selon la revendication 1, dans laquelle Q est un composé de formule (IV) : 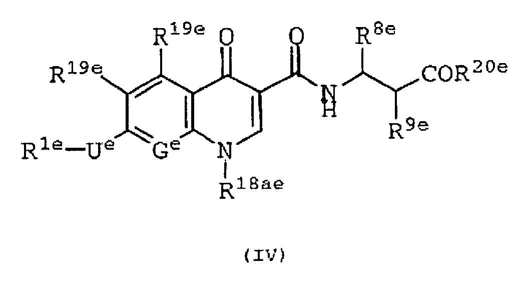 incluant les formes stéréoisomériques de celle-ci, ou les mélanges des formes stéréoisomériques de celle-ci, ou les formes de sel ou de promédicament de celle-ci acceptables sur le plan pharmaceutique, dans laquelle :
R^{1e} est choisi parmi : 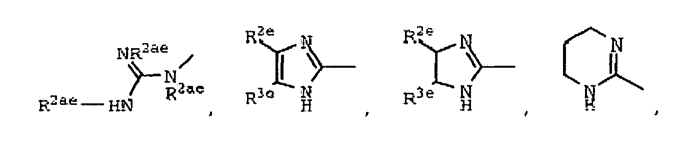 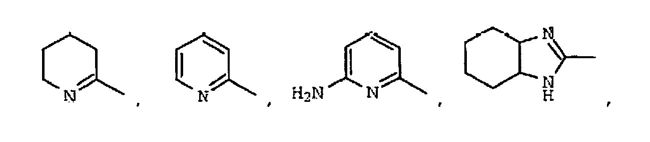 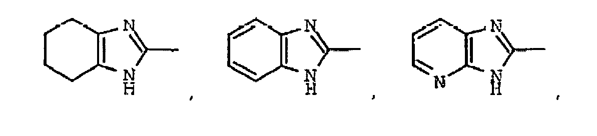 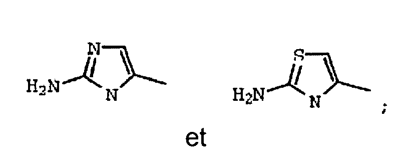
R^{2e} et R^{3e} sont indépendamment choisis parmi :
H, un groupe alcoxy en C₁-C₄, NR^{11e}R^{12e}, un halogène, NO₂, CN, CF₃, un groupe alkyle en C₁-C₆, un groupe alcényle en C₃-C₆, un groupe cycloalkyle en C₃-C₇, un groupe cycloalkyle(alkyle en C₁-C₄) en C₃-C₇, un groupe aryle(alkyle en C₁-C₆), un groupe (alkyle en C₁-C₆)carbonyle, un groupe (alcoxy en C₁-C₆)carbonyle, un groupe arylcarbonyle, et un groupe aryle substitué par 0 à 4 R^{7e},
en variante, lorsque R^{2e} et R^{3e} sont des substituants liés à des atomes adjacents, ils peuvent former conjointement avec les atomes de carbone auxquels ils sont liés un carbocycle à 5-7 chaînons ou un hétérocycle à 5-7 chaînons, aromatique ou non aromatique, ledit carbocycle ou hétérocycle étant substitué par 0 à 2 groupes choisis parmi un groupe alkyle en C₁-C₄, un groupe alcoxy en C₁-C₄, un groupe halogéno, un groupe cyano, un groupe amino, CF₃ et NO₂ ;
R^{2ae} est choisi parmi :
H, un groupe alkyle en C₁-C₁₀, un groupe alcényle en C₂-C₆, un groupe cycloalkyle en C₃-C₁₁, un groupe cycloalkyl(alkyle en C₁-C₄) en C₃-C₇, un groupe aryle, un groupe aryle(alkyle en C₁-C₄), un groupe (alkyle en C₂-C₇)carbonyle, un groupe arylcarbonyle, un groupe (alcoxy en C₂-C₁₀)carbonyle, un groupe cycloalcoxycarbonyle en C₃-C₇, un groupe bicycloalcoxycarbonyle en C₇-C₁₁, un groupe aryloxycarbonyle, un groupe aryl(alcoxy en C₁-C₁₀)carbonyle, un groupe alkylcarbonyloxy(alcoxy en C₁-C₄)carbonyle en C₁-C₆, un groupe arylcarbonyloxy(alcoxy en C₁-C₄)carbonyle, et un groupe cycloalkylcarbonyloxy(alcoxy en C₁-C₄)carbonyle en C₃-C₇ ;
R^{7e} est choisi parmi :
H, un groupe hydroxy, un groupe alkyle en C₁-C₄, un groupe alcoxy en C₂-C₄, un groupe aryle, un groupe aryle(alkyle en C₁-C₄), un groupe (alkyle en C₁-C₄)carbonyle, CO₂R^{18ae}, SO₂R^{11e}, SO₂NR^{10e}R^{11e}, OR^{10e}, et N(R^{11e})R^{12e} ;
U^{e} est choisi parmi :
-(CH₂)ₙe-, -(CH₂)ₙeO(CH₂)ₘe-, -NH(CH₂)ₙe-,
-N(R^{10e})C(=O)-, -NHC(=O)(CH₂)ₙe-, et -C(=O)N(R^{10e})- ;
G^{e} représente N ou CR^{19e} ;
R^{8e} est choisi parmi :
H, CO₂R^{18be}, C(=O)R^{18be}, CONR¹⁷R^{18be}, un groupe alkyle en C₁-C₁₀ substitué par 0 à 1 R^{6e}, un groupe alcényle en C₂-C₁₀ substitué par 0 à 1 R^{6e}, un groupe alcynyle en C₂-C₁₀ substitué par 0 à 1 R^{6e}, un groupe cycloalkyle en C₃-C₈ substitué par 0 à 1 R^{6e}, un groupe cycloalcényle en C₅-C₆ substitué par 0 à 1 R^{6e}, un groupe (alkyle en C₁-C₁₀)carbonyle, un groupe cycloalkyle(alkyle en C₁-C₄) en C₃-C₁₀, un groupe phényle substitué par 0 à 3 R^{6e}, un groupe naphtyle substitué par 0 à 3 R^{6e}, un hétérocycle à 5-10 chaînons contenant 1 à 3 hétéroatomes N, O ou S, ledit hétérocycle pouvant être saturé, partiellement saturé, ou entièrement insaturé, et ledit hétérocycle étant substitué par 0 à 2 R^{7e};
R^{9e} est choisi parmi :
un groupe alkyle en C₁-C₁₀ substitué par 0 à 1 R^{6e}, un groupe alcoxy en C₁-C₁₀ substitué par 0 à 2 R^{7e}, H, un groupe nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, un groupe hydroxy, OR^{22e}, -N(R^{10e})R^{11e}, -N(R^{16e})R^{17e}, un groupe aryl(alkyle en C₀-C₆)carbonyle, un groupe aryle(alkyle en C₁-C₆), un groupe hétéroaryle(alkyle en C₁-C₆), CONR^{18ae}R^{20e}, SO₂R^{18ae}, et SO₂NR^{18ae}R^{20e},
sachant que chacun desdits groupes alkyles, cycloalkyles, aryles ou hétéroaryles peut être non substitué ou substitué de manière indépendante par 1 à 2 R^{7e};
R^{6e} est choisi parmi :
H, un groupe alkyle en C₁-C₁₀, un groupe hydroxy, un groupe alcoxy en C₁-C₁₀, un groupe nitro, un groupe alkylcarbonyle en C₁-C₁₀, -N(R^{11e})R^{12e}, un groupe cyano, un groupe halogéno, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚeR^{11e}, SO₂NR^{10e}R^{11e}, un groupe aryle substitué par 0 à 3 groupes choisis parmi un halogène, un groupe alcoxy en C₁-C₆, un groupe alkyle en C₁-C₆, CF₃, S(O)ₘeMe, et -NMe₂, un groupe aryle(alkyle en C₁-C₄), ledit groupe aryle étant substitué par 0 à 3 groupes choisis parmi un halogène, un groupe alcoxy en C₁-C₆, un groupe alkyle en C₁-C₆, CF₃, S(O)ₚeMe, et -NMe₂, et un hétérocycle à 5-10 chaînons contenant 1 à 3 hétéroatomes N, O, ou S, ledit hétérocycle pouvant être saturé, partiellement saturé, ou entièrement insaturé, ledit hétérocycle étant substitué par 0 à 2 R^{7e} ;
R^{10e} est choisi parmi :
H, -OH, CF₃, un groupe alcényle en C₃-C₆, un groupe cycloalkyle en C₃-C₁₁, un groupe aryle, un groupe (cycloalkyle en C₃-C₁₁)méthyle, un groupe aryle(alkyle en C₁-C₄), et un groupe alkyle en C₁-C₁₀ substitué par 0 à 2 R^{6e};
R^{11e} est choisi parmi :
H, un groupe hydroxy, un groupe alkyle en C₁-C₈, un groupe alcényle en C₃-C₆, un groupe cycloalkyle en C₃-C₁₁, un groupe (cycloalkyle en C₃-C₁₁)méthyle, un groupe alcoxy en C₁-C₆, un groupe benzyloxy, un groupe aryle, un groupe hétéroaryle, un groupe hétéroaryle(alkyle en C₁-C₄), un groupe aryle(alkyle en C₁-C₄), un groupe adamantylméthyle, et un groupe alkyle en C₁-C₁₀ substitué par 0 à 2 R^{4e};
R^{4e} est choisi parmi :
H, un groupe alkyle en C₁-C₆, un groupe cycloalkyle en C₃-C₇, un groupe cycloalkyle(alkyle en C₁-C₄) en C₃-C₇, un groupe aryle, un groupe hétéroaryle, un groupe aryle(alkyle en C₁-C₆), et un groupe hétéroaryle(alkyle en C₁-C₆), lesdits groupes aryles ou hétéroaryles étant substitués par 0 à 2 substituants indépendamment choisis dans le groupe constitué par un groupe alkyle en C₁-C₄, un groupe alcoxy en C₁-C₄, F, CI, Br, CF₃ et NO₂ ;
R^{12e} est choisi parmi :
H, un groupe alkyle en C₁-C₆, un groupe triphénylméthyle, un groupe méthoxyméthyle, un groupe méthoxyphényldiphénylméthyle, un groupe triméthylsilyléthoxyméthyle, un groupe (alkyle en C₁-C₆)carbonyle, un groupe (alcoxy en C₁-C₆)carbonyle, un groupe (alkyle en C₁-C₆)aminocarbonyle, un groupe alcényle en C₃-C₆, un groupe cycloalkyle en C₃-C₇, un groupe cycloalkyle(alkyle en C₁-C₄) en C₃-C₇, un groupe aryle, un groupe hétéroaryle(alkyle en C₁-C₆)carbonyle, un groupe hétéroarylcarbonyle, un groupe aryle(alkyle en C₁-C₆), un groupe (alkyle en C₁-C₆)carbonyle, un groupe arylcarbonyle, un groupe alkylsulfonyle en C₁-C₆, un groupe arylsulfonyle, un groupe aryle(alkyle en C₁-C₆)sulfonyle, un groupe hétéroarylsulfonyle, un groupe hétéroaryle(alkyle en C₁-C₆)sulfonyle, un groupe aryloxycarbonyle, et un groupe aryle(alcoxy en C₁-C₆)carbonyle, lesdits groupes aryles étant substitués par 0 à 2 substituants choisis dans le groupe constitué par un groupe alkyle en C₁-C₄, un groupe alcoxy en C₁-C₄, un groupe halogéno, CF₃, et un groupe nitro ;
R^{16e} est choisi parmi :
-C(=O)OR^{18ae}, -C(=O)R^{18be}, -C(=O)N(R^{18be})₂, -3O₂R^{18ae}, et -SO₂N(R^{18be})₂;
R^{17e} est choisi parmi :
H, un groupe alkyle en C₁-C₆, un groupe cycloalkyle en C₃-C₇, un groupe cycloalkyle(alkyle en C₁-C₄) en C₃-C₇, un groupe aryle, un groupe aryle(alkyle en C₁-C₆), et un groupe hétéroaryle(alkyle en C₁-C₆) ;
R^{18ae} est choisi parmi :
un groupe alkyle en C₁-C₈ éventuellement substitué par une liaison à Lₙ, un groupe cycloalkyle en C₃-C₁₁ éventuellement substitué par une liaison à Lₙ, un groupe aryle(alkyle en C₁-C₆) éventuellement substitué par une liaison à Lₙ, un groupe hétéroaryle(alkyle en C₁-C₆) éventuellement substitué par une liaison à Lₙ, un groupe (alkyle en C₁-C₆)hétéroaryle éventuellement substitué par une liaison à Lₙ, un groupe biaryle(alkyle en C₁-C₆) éventuellement substitué par une liaison à Lₙ, un groupe hétéroaryle éventuellement substitué par une liaison à Lₙ, un groupe phényle substitué par 3 à 4 R^{19e} et éventuellement substitué par une liaison à Lₙ, un groupe naphtyle substitué par 0 à 4 R^{19e} et éventuellement substitué par une liaison à Lₙ, et une liaison à Lₙ, lesdits groupes aryles ou hétéroaryles étant éventuellement substitués par 0 à 4 R^{19e} ;
R^{18be} représente H ou R^{18ae};
R^{19e} est choisi parmi :
H, un halogène, CF₃, CO₂H, CN, NO₂, -NR^{11e}R^{12e}, OCF₃, un groupe alkyle en C₁-C₈, un groupe alcényle en C₂-C₆, un groupe alcynyle en C₂-C₆, un groupe cycloalkyle en C₃-C₁₁, un groupe cycloalkyle(alkyle en C₁-C₄) en C₃-C₇, un groupe aryle(alkyle en C₁-C₆), un groupe alcoxy en C₁-C₆, un groupe alcoxycarbonyle en C₁-C₄, un groupe aryle, un groupe aryle-O-, un groupe aryle-SO₂-, un groupe hétéroaryle, et un groupe hétéroaryle-SO₂-, lesdits groupes aryles et hétéroaryles étant substitués par 0 à 4 groupes choisis parmi l'hydrogène, un halogène, CF₃, un groupe alkyle en C₁-C₃, et un groupe alcoxy en C₁-C₃ ;
R^{20e} est choisi parmi les groupes :
hydroxy, alkyloxy en C₁-C₁₀, cycloalkyloxy en C₃-C₁₁, aryloxy, aryl(alkyle en C₁-C₄)oxy, alkylcarbonyloxy(alkyle en C₁-C₂)oxy en C₂-C₁₀, alcoxycarbonyloxy(alkyle en C₁-C₂)oxy en C₂-C₁₀, alcoxycarbonyl(alkyle en C₁-C₂)oxy en C₂-C₁₀, cycloalkylcarbonyloxy(alkyle en C₁-C₂)oxy en C₃-C₁₀, cycloalcoxycarbonyloxy(alkyle en C₁-C₂)oxy en C₃-C₁₀, cycloalcoxycarbonyl(alkyle en C₁-C₂)oxy en C₃-C₁₀, aryloxycarbonyl(alkyle en C₁-C₂)oxy, aryloxycarbonyloxy(alkyle en C₁-C₂)oxy, arylcarbonyloxy(alkyle en C₁-C₂)oxy, alcoxy(alkyle en C₁-C₅)carbonyloxy(alkyle en C₁-C₂)oxy en C₁-C₅, (5-(alkyle en C₁-C₅)-1,3-dioxa-cyclopentèn-2-one-yl)méthyloxy, (5-aryl-1,3-dioxa-cyclopentèn-2-one-yl)méthyloxy, et (R^{10e})(R^{11e})N(alcoxy en C₃-C₁₀) ;
R^{21e} est choisi parmi les groupes :
alkyle en C₁-C₈, alcényle en C₂-C₆, cycloalkyle en C₃-C₁₁, (cycloalkyle en C₃-C₁₁)méthyle, aryle, aryle(alkyle en C₁-C₄), et alkyle en C₁-C₁₀ substitué par 0 à 2 R^{7e};
R^{22e} est choisi parmi :
-C(=O)-R^{18be}, -C(=O)N(R^{18be})₂, -C(=O)NHSO₂R^{18ae}, -C(=O)NHC(=O)R^{18be}, -C(₌O)NHC(=O)OR^{18ae}, et -C(=O)NHSO₂NHR^{18be} ;
m^{e} est 0-2 ;
n^{e} est 0-4 ; et
p^{e} est 0-2 ;
avec la clause suivante :
n^{e} et m^{e} sont choisis de telle sorte que le nombre d'atomes reliant R¹ et -COR^{20e} dans la formule (IV) est dans la plage comprise entre 8 et 14 ;
d est choisi parmi 1, 2, 3, 4, et 5 ;
d' est 1-50 ;
W est indépendamment choisi à chaque occurrence dans le groupe : O, NH, NHC(=O), C(=O)NH, NR⁸C(=O), C(=O)NR⁸, C(=O), C(=O)O, OC(=O), NHC(=S)NH, NHC(=O)NH, SO₂, (OCH₂CH₂)ₛ, (CH₂CH₂O)_{s'}, (OCH₂CH₂CH₂)_{s"}, (CH₂CH₂CH₂O)ₜ, et (aa)_{t'} ;
aa est indépendamment à chaque occurrence un acide aminé ;
z est choisi dans le groupe :
groupe aryle substitué par 0 à 1 R¹⁰, groupe cycloalkyle en C₃-C₁₀ substitué par 0 à 1 R¹⁰, et hétérocycle à 5-10 chaînons contenant 1 à 4 hétéroatomes indépendamment choisis parmi N, S, et O et substitué par 0 à 1 R¹⁰;
R⁶, R^{6a}, R⁷, R^{7a}, et R⁸ sont indépendamment choisis à chaque occurrence dans le groupe :
H, =O, COOH, SO₃H, un groupe alkyle en C₁-C₅ substitué par 0 à 1 R¹⁰, un groupe aryle substitué par 0 à 1 R¹⁰, un groupe benzyle substitué par 0 à 1 R¹⁰, et un groupe alcoxy en C₁-C₅ substitué par 0 à 1 R¹⁰, NHC(=O)R¹¹, C(=O)NHR¹¹, NHC(=O)NHR¹¹, NHR¹¹, R¹¹, et une liaison à Cₕ ;
k vaut 0 ou 1 ;
s est choisi parmi 0, 1, 2, 3, 4, et 5 ;
s' est choisi parmi 0, 1, 2, 3, 4, et 5 ;
s" est choisi parmi 0, 1, 2, 3, 4, et 5 ;
t est choisi parmi 0, 1, 2, 3, 4, et 5 ;
A¹, A², A³, A⁴, A⁵, A⁶, A⁷ et A⁸ sont indépendamment choisis à chaque occurrence dans le groupe :
NR¹³, NR¹³R¹⁴, S, SH, S(Pg), OH, et une liaison à Lₙ ;
E est une liaison, CH, ou un groupe espaceur indépendamment choisi à chaque occurrence dans le groupe :
un groupe alkyle en C₁-C₁₀ substitué par 0 à 3 R¹⁷, un groupe aryle substitué par 0 à 3 R¹⁷ un groupe cycloalkyle en C₃-C₁₀ substitué par 0 à 3 R¹⁷, et un hétérocycle à 5-10 chaînons contenant 1 à 4 hétéroatomes indépendamment choisis parmi N, S, et O et substitué par 0 à 3 R¹⁷ ;
R¹³ et R¹⁴ sont chacun indépendamment choisis dans le groupe :
une liaison à Lₙ, un hydrogène, un groupe alkyle en C₁-C₁₀ substitué par 0 à 3 R¹⁷, un groupe aryle substitué par 0 à 3 R¹⁷, un hétérocyle à 5-10 chaînons contenant 1 à 4 hétéroatomes indépendamment choisis parmi N, S et O et substitués par 0 à 3 R¹⁷, et un électron, sachant que lorsque l'un parmi R¹³ et R¹⁴ est un électron, alors l'autre est aussi un électron ;
en variante, R¹³ et R¹⁴ se combinent pour former =C(R²⁰)(R²¹) ;
R¹⁷ est indépendamment choisi à chaque occurrence dans le groupe :
une liaison à Lₙ, =O, F, CI, Br, I, -CF₃, -CN, -CO₂R¹⁸, -C(=O)R¹⁸, -C(=O)N(R¹⁸)₂, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)₂, -N(R¹⁸)₂, -NHC(=S)NHR¹⁸, =NOR¹⁸, -C(=O)NHNR¹⁸R^{18a}, -OCH₂CO₂H, et le groupe 2-(1-morpholino)éthoxy ;
R¹⁸, R^{18a} et R¹⁹ sont indépendamment choisis à chaque occurrence dans le groupe :
une liaison à Lₙ, H, et un groupe alkyle en C₁-C₆ ;
R²⁰ et R²¹ sont indépendamment choisis dans le groupe :
H, un groupe alkyle en C₁-C₅, -CO₂R²⁵, un 1-alcène en C₂-C₅ substitué par 0 à 3 R²³, un 1-alcyne en C₂-C₅ substitué par 0 à 3 R²³, un groupe aryle substitué par 0 à 3 R²³, et un hétérocycle insaturé à 5-10 chaînons contenant 1 à 4 hétéroatomes indépendamment choisis parmi N, S, et O et substitué par 0 à 3 R²³;
en variante, R²⁰ et R²¹ forment conjointement avec le radical carboné divalent auquel ils sont liés : 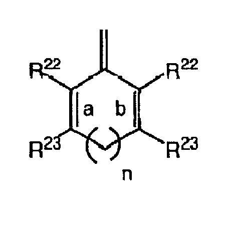
R²² et R²³ sont indépendamment choisis parmi le groupe : H, et R²⁴ ;
en variante, R²² et R²³ forment ensemble un cycle aromatique ou un hétérocycle à 5-10 chaînons contenant 1 à 4 hétéroatomes indépendamment choisis parmi N, S, et O ;
R²⁴ est indépendamment choisi à chaque occurrence dans le groupe : -CO₂R²⁵, -C(=O)N(R²⁵)₂, -CH₂OR²⁵, -OC(=O)R²⁵, -OR²⁵, -SO₃H, -N(R²⁵)₂, et -OCH₂CO₂H ; et,
R²⁵ est indépendamment choisi à chaque occurrence dans le groupe : un hydrogène et un groupe alkyle en C₁-C₃.

6. Trousse selon la revendication 1, dans laquelle :
R^{1e} est choisi parmi : 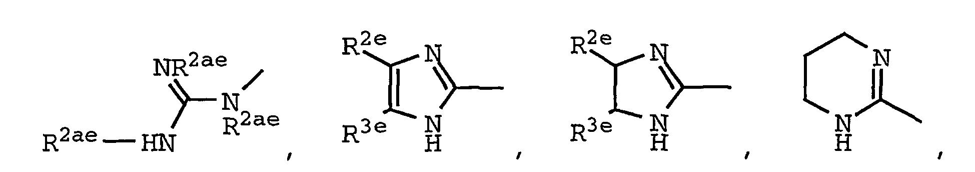 et 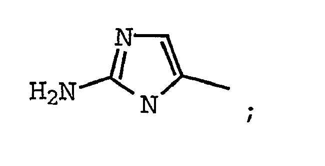
R^{2e} et R^{3e} sont indépendamment choisis parmi :
H, un groupe alcoxy en C₁-C₄, NR^{11e}R^{12e}, un halogène, NO₂, CN, CF₃, un groupe alkyle en C₁-C₆, un groupe alcényle en C₃-C₆, un groupe cycloalkyle en C₃-C₇, un groupe cycloalkyle(alkyle en C₁-C₄) en C₃-C₇, un groupe aryle(alkyle en C₁-C₆), un groupe (alkyle en C₁-C₆)carbonyle, un groupe (alcoxy en C₁-C₆)carbonyle, un groupe arylcarbonyle, et un groupe aryle substitué par 0 à 4 R^{7e},
en variante, lorsque R^{2e} et R^{3e} sont des substituants liés à des atomes adjacents, ils peuvent former conjointement avec les atomes de carbone auxquels ils sont liés un carbocycle à 5-7 chaînons ou un hétérocycle à 5-7 chaînons, aromatique ou non aromatique, ledit carbocycle ou hétérocycle étant substitué par 0 à 2 groupes choisis parmi un groupe alkyle en C₁-C₄, un groupe alcoxy en C₁-C₄, un groupe halogéno, un groupe cyano, un groupe amino, CF₃ et NO₂ ;
R^{2ae} est choisi parmi :
H, un groupe alkyle en C₁-C₁₀, un groupe alcényle en C₂-C₆, un groupe cycloalkyle en C₃-C₁₁, un groupe cycloalkyle(alkyle en C₁-C₄) en C₃-C₇, un groupe aryle, un groupe aryle(alkyle en C₁-C₄), un groupe (alkyle en C₂-C₇)carbonyle, un groupe arylcarbonyle, un groupe (alcoxy en C₂-C₁₀)carbonyle, un groupe cycloalcoxycarbonyle en C₃-C₇, un groupe bicycloalcoxycarbonyle en C₇-C₁₁, un groupe aryloxycarbonyle, un groupe aryle(alcoxy en C₁-C₁₀)carbonyle, un groupe alkylcarbonyloxy(alcoxy en C₁-C₄)carbonyle en C₁-C₆, un groupe arylcarbonyloxy(alcoxy en C₁-C₄)carbonyle, et un groupe cycloalkylcarbonyloxy(alcoxy en C₁-C₄)carbonyle en C₃-C₇ ;
R^{7e} est choisi parmi :
H, un groupe hydroxy, un groupe alkyle en C₁-C₄, un groupe alcoxy en C₁-C₄, un groupe aryle, un groupe aryle(alkyle en C₁-C₄), un groupe (alkyle en C₁-C₄)carbonyle, CO₂R^{18ae}, SO₂R^{11e}, SO₂NR^{10e}R^{11e}, OR^{10e}, et N(R^{11e})R¹²e;
U^{e} est choisi parmi :
-(CH₂)ₙe-, -NH(CH₂)ₙe-, -N(R^{10e})C(=O)-, et -NHC(=O)(CH₂)ₙe- ;
G^{e} représente N ou CR^{19e} ;
R^{8e} représente H ;
R^{9e} est choisi parmi :
H, un groupe nitro, N(R^{11e})R^{12e}, OC(=O)R^{10e}, OR^{10e}, OC(=O)R^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, un groupe hydroxy, OR^{22e}, -N(R^{10e})R^{11e}, - N(R^{16e})R^{17e}, un groupe aryle(alkyle en C₀-C₄)carbonyle, un groupe aryle(alkyle en C₁-C₄), un groupe hétéroaryle(alkyle en C₁-C₄), CONR^{18ae}R^{20e}, SO₂R^{18ae}, et SO₂NR^{18ae}R^{20e}, sachant que chacun desdits groupes alkyles, cycloalkyles, aryles ou hétéroaryles peut être non substitué ou substitué de manière indépendante par 1 à 2 R^{7e};
R^{10e} est choisi parmi :
H, -OH, CF₃, un groupe alcényle en C₃-C₆, un groupe cycloalkyle en C₃-C₆, un groupe aryle, un groupe (cycloalkyle en C₃-C₆)méthyle, un groupe aryle(alkyle en C₁-C₄), et un groupe alkyle en C₁-C₄ substitué par 0 à 2 R^{6e} ;
R^{6e} est choisi parmi :
H, un groupe alkyle en C₁-C₄, un groupe hydroxy, un groupe alcoxy en C₁-C₄, un groupe nitro, un groupe alkylcarbonyle en C₁-C₄, -N(R^{11e})R^{12e}, un groupe cyano, un groupe halogéno, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e}, un groupe aryle substitué par 0 à 3 groupes choisis parmi un halogène, un groupe alcoxy en C₁-C₄, un groupe alkyle en C₁-C₄, CF₃, S(O)ₘeMe, et -NMe₂, un groupe aryle(alkyle en C₁-C₄), ledit groupe aryle étant substitué par 0 à 3 groupes choisis parmi un halogène, un groupe alcoxy en C₁-C₄, un groupe alkyle en C₁-C₄, CF₃, S(O)ₚeMe, et -NMe₂, et un hétérocycle à 5-10 chaînons contenant 1 à 3 hétéroatomes N, O, ou S, ledit hétérocycle pouvant être saturé, partiellement saturé ou entièrement insaturé, ledit hétérocycle étant substitué par 0 à 2 R^{7e};
R^{11e} est choisi parmi les groupes :
H, hydroxy, alkyle en C₁-C₄, alcényle en C₃-C₆, cycloalkyle en C₃-C₆, (cycloalkyle en C₃-C₆)méthyle, alcoxy en C₁-C₄, benzyloxy, aryle, hétéroaryle, hétéroaryle(alkyle en C₁-C₄), aryle(alkyle en C₁-C₄), adamantylméthyle, et alkyle en C₁-C₄ substitué par 0 à 2 R^{4e} ;
R^{4e} est choisi parmi les groupes :
H, alkyle en C₁-C₄, cycloalkyle en C₃-C₇, cycloalkyle(alkyle en C₁-C₄) en C₃-C₇, aryle, hétéroaryle, aryle(alkyle en C₁-C₄), et hétéroaryle(alkyle en C₁-C₄), lesdits groupes aryles ou hétéroaryles étant substitués par 0 à 2 substituants indépendamment choisis dans le groupe constitué par un groupe alkyle en C₁-C₄, un groupe alcoxy en C₁-C₄, F, CI, Br, CF₃, et NO₂,
R^{12e} est choisi parmi les groupes :
H, alkyle en C₁-C₄, (alkyle en C₁-C₄)carbonyle, (alcoxy en C₁-C₄)carbonyle, phényle(alkyle en C₁-C₄), phénylsulfonyle, phényloxycarbonyle, et phényle(alcoxy en C₁-C₄)carbonyle, lesdits groupes phényles étant substitués par 0 à 2 substituants choisis dans le groupe constitué par un groupe alkyle en C₁-C₄, un groupe alcoxy en C₁-C₄, un groupe halogéno, CF₃, et un groupe nitro ;
R^{16e} est choisi parmi :
-C(=O)OR^{18ae}, -C(=O)R^{18be}, -C(=O)N(R^{18be})₂, -SO₂R^{18ae}, et -SO₂N(R^{18be})₂;
R^{17e} est choisi parmi :
H, un groupe alkyle en C₁-C₄, un groupe cycloalkyle en C₃-C₆, un groupe cycloalkyle(alkyle en C₁-C₄) en C₃-C₆, un groupe aryle, un groupe aryle(alkyle en C₁-C₆), et un groupe hétéroaryle(alkyle en C₁-C₆) ;
R^{18ae} est choisi parmi :
un groupe alkyle en C₁-C₈ éventuellement substitué par une liaison à Lₙ, un groupe cycloalkyle en C₃-C₁₁ éventuellement substitué par une liaison à Lₙ, un groupe aryle(alkyle en C₁-C₆) éventuellement substitué par une liaison à Lₙ, un groupe hétéroaryle(alkyle en C₁-C₆) éventuellement substitué par une liaison à Lₙ, un groupe (alkyle en C₁-C₆)hétéroaryle éventuellement substitué par une liaison à Lₙ, un groupe biaryle(alkyle en C₁-C₆) éventuellement substitué par une liaison à Lₙ, un groupe hétéroaryle éventuellement substitué par une liaison à Lₙ, un groupe phényle substitué par 3 à 4 R^{19e} et éventuellement substitué par une liaison à Lₙ, un groupe naphtyle substitué par 0 à 4 R^{19e} et éventuellement substitué par une liaison à Lₙ, et une liaison à Lₙ, lesdits groupes aryles ou hétéroaryles étant éventuellement substitués par 0 à 4 R^{19e} ;
R^{18be} représente H ou R^{18ae};
R^{19e} est choisi parmi :
H, un halogène, CF₃, CO₂H, CN, NO₂, -NR^{11e}R^{12e}, OCF₃, un groupe alkyle en C₁-C₆, un groupe alcényle en C₂-C₆, un groupe alcynyle en C₂-C₆, un groupe cycloalkyle en C₃-C₆, un groupe cycloalkyle(alkyle en C₁-C₄) en C₃-C₆, un groupe aryle(alkyle en C₁-C₄), un groupe alcoxy en C₁-C₆, un groupe alcoxycarbonyle en C₁-C₄, un groupe aryle, un groupe aryle-O-, un groupe aryle-SO₂-, un groupe hétéroaryle, et un groupe hétéroaryle-SO₂-, lesdits groupes aryles et hétéroaryles étant substitués par 0 à 4 groupes choisis parmi l'hydrogène, un halogène, CF₃, un groupe alkyle en C₁-C₃, et un groupe alcoxy en C₁-C₃ ;
R^{20e} est choisi parmi les groupes :
hydroxy, alkyloxy en C₁-C₆, cycloalkyloxy en C₃-C₆, aryloxy, aryl(alkyle en C₁-C₄)oxy, alkylcarbonyloxy(alkyle en C₁-C₂)oxy en C₂-C₁₀, alcoxycarbonyloxy(alkyle en C₁-C₂)oxy en C₂-C₁₀, alcoxycarbonyl(alkyle en C₁-C₂)oxy en C₂-C₁₀, cycloalkylcarbonyloxy(alkyle en C₁-C₂)oxy en C₃-C₁₀, cycloalcoxycarbonyloxy(alkyle en C₁-C₂)oxy en C₃-C₁₀, cycloalcoxycarbonyl(alkyle en C₁-C₂)oxy en C₃-C₁₀, aryloxycarbonyl(alkyle en C₁-C₂)oxy, aryloxycarbonyloxy(alkyle en C₁-C₂)oxy, arylcarbonyloxy(alkyle en C₁-C₂)oxy, alcoxy(alkyle en C₁-C₅)carbonyloxy(alkyle en C₁-C₂)oxy en C₁-C₅, (5-(alkyle en C₁-C₅)-1,3-dioxa-cyclopentèn-2-one-yl)méthyloxy, (5-aryl-1,3-dioxa-cyclopentèn-2-one-yl)méthyloxy, et (R^{10e})(R^{11e})N(alcoxy en C₁-C₁₀) ;
R^{21e} est choisi parmi les groupes :
alkyle en C₁-C₄, alcényle en C₂-C₆, cycloalkyle en C₃-C₆, (cycloalkyle en C₃-C₆)méthyle, aryle, aryle(alkyle en C₁-C₄), et alkyle en C₁-C₁₀ substitué par 0 à 2 R^{7e};
R^{22e} est choisi parmi :
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, et-C(=O)NHSO₂NHR^{18be};
m^{e} est 0-2 ;
n^{e} est 0-4 ;
p^{e} est 0-2 ;
Cₕ est 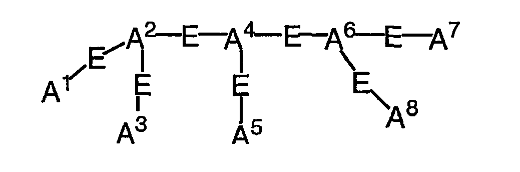
A¹est choisi dans le groupe :
OH, et une liaison à Lₙ ;
A², A⁴ et A⁶ sont chacun N ;
A³, A⁵ et A⁸ sont chacun OH ;
A⁷ est une liaison à Lₙ ou une liaison NH à Lₙ ;
E est un groupe alkyle en C₂ substitué par 0 à 1 R¹⁷;
R¹⁷ représente =O ;
en variante, Cₕ représente 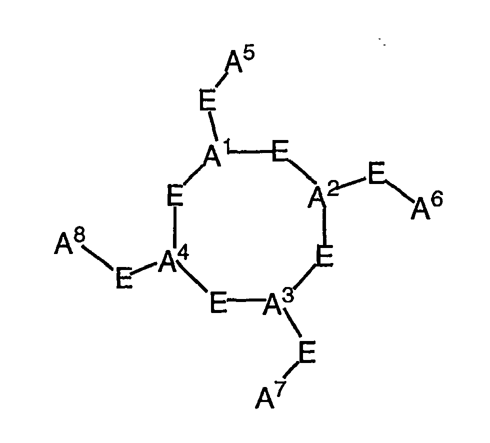
A¹est choisi dans le groupe :
OH, et une liaison à Lₙ ;
A², A³ et A⁴ sont chacun N ;
A⁵, A⁶ et A⁸ sont chacun OH ;
A⁷ est une liaison à Lₙ ;
E est un alkyle en C₂ substitué par 0 à 1 R¹⁷ ;
R¹⁷ représente =O;
en variante, Cₕ est 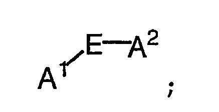
A¹ est NH₂ ou N=C(R²⁰)(R²¹) ;
E est une liaison ;
A² est NHR¹³;
R¹³ est un hétérocycle substitué par R¹⁷, ledit hétérocycle étant choisi parmi la pyridine et la pyrimidine ;
R¹⁷ est choisi parmi une liaison à Lₙ, C(=O)NHR¹⁸ et C(=O)R¹⁸ ;
R¹⁸ est une liaison à Lₙ ;
R²⁴ est choisi dans le groupe :
-CO₂R²⁵, -OR²⁵, -SO₃H, et -N(R²⁵)2 ; et,
R²⁵ est indépendamment choisi à chaque occurrence dans le groupe : hydrogène et groupe méthyle.

7. Trousse selon la revendication 1, dans laquelle Q est choisi dans le groupe :
acide 3-[7-[(imidazolin-2-ylamino)méthyl]-1-méthyl-6,8-difluoroquinoline-4-one-3-ylcarbonylamino]-2-(3,5-diméthylisoxazol-4-ylsulfonylamino)propionique,
acide 3-[7-[(imidazolin-2-ylamino)méthyl]-1-méthyl-6,8-difluoroquinoline-4-one-3-ylcarbonylamino]-2-(benzyloxycarbonylamino)propionique,
acide 3-[7-[(imidazolin-2-ylamino)méthyl]-1-méthyl-6,8-difluoroquinoline-4-one-3-ylcarbonylamino]-2-(n-butyloxycarbonylamino)propionique,
acide 3-[7-[(imidazolin-2-ylamino)méthyl]-1-méthyl-6,8-difluoroquinoline-4-one-3-ylcarbonylamino]-2-(n-butylsulfonylamino)propionique,
acide 3-[7-[(tétrahydropyrimid-2-ylamino)méthyl)-1-méthyl-6,8-difluoroquinoline-4-one-3-ylcarbonylamino]-2-(benzyloxycarbonylamino)propionique,
acide 3-[7-[(tétrahydropyrimid-2-ylamino)méthyl]-1-méthyl-6,8-difluoroquinoline-4-one-3-ylcarbonylamino]-2-(n-butyloxycarbonylamino)propionique,
acide 3-[7-[(tétrahydropyrimid-2-ylamino)méthyl]-1-méthyl-6,8-difluoroquinoline-4-one-3-ylcarbonylamino]-2-(phénylsulfonylamino)propionique,
acide 3-[7-[(tétrahydropyrimid-2-ylamino)méthyl]-1-méthyl-6,8-difluoroquinoline-4-one-3-ylcarbonylamino]-2-(n-butylsulfonyl)aminopropionique,
acide 3-[7-[(2-aminothiazol-4-yl)méthyl]-1-méthyl-6,8-difluoroquinoline-4-one-3-ylcarbonylamino]-2-(benzyloxycarbonylamino)propionique,
acide 3-[7-[(imidazolin-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(tétrahydropyrimid-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-méthyl-6,8-difluoroquinoline-4-one-3-ylcarbonylamino]-2-(3,5-diméthylisoxazol-4-ylsulfonylamino)propionique,
acide 3-[7-[(imidazol-2-ylamino)méthyl]-1-méthyl-6,8-difluoroquinoline-4-one-3-ylcarbonylamino]-2-(benzyloxycarbonylamino)propionique,
acide 3-[7-[(imidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-méthyl-6,8-difluoroquinoline-4-one-3-ylcarbonylamino]-2-((4-biphényl)sulfonylamino)propionique,
acide 3-[7-[(imidazol-2-ylamino)méthyl]-1-méthyl-6,8-difluoroquinoline-4-one-3-ylcarbonylamino]-2-(1-naphtylsulfonylamino)propionique,
acide 3-[7-[(benzimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(4-méthylimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(4,5-diméthylimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(4,5,6,7-tétrahydrobenzimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(pyridin-2-ylamino)méthyl]-1-méthyl-6,8-difluoroquinoline-4-one-3-ylcarbonylamino]-2-((2,4,6-triméthylphényl)sulfonylamino)propionique,
acide 3-[7- (2-aminopyridin-6-yl)-1-méthyl-6,8-difluoroquinoline-4-one-3-ylcarbonylamino]-2-((2,4,6-triméthylphényl)sulfonylamino)propionique,
acide 3-[7-[(7-azabenzimidazol-2-yl)méthyl]-1-méthyl-6,8-difluoroquinoline-4-one-3-ylcarbonylamino]-2-((2,4,6-triméthylphényl)sulfonylamino)propionique,
acide 3-[7-[(benzimidazol-2-ylamino)méthyl]-1-(2-phényléthyl)-6,8-difluoroquinoline-4-one-3-ylcarbonylamino]propionique,
acide 3-[7-[(pyridin-2-ylamino)méthyl]-1-(2-phényléthyl)- 6,8-difluoroquinoline-4-one-3-ylcarbonylamino]propionique,
acide 3-[7-[(imidazolin-2-ylamino)méthyl]-1-(2-phényléthyl)-6,8-difluoroquinoline-4-one-3-ylcarbonylamino]propionique,
acide 3-[7-[(imidazol-2-ylamino)méthyl]-1-(2-phényléthyl)-6,8-difluoroquinoline-4-one-3-ylcarbonylamino]propionique,
acide 3-[7-[(imidazolin-2-ylamino)méthyl]-1-(2-phényléthyl)-6,8-difluoroquinoline-4-one-3-ylcarbonylamino]-2-(benzyloxycarbonylamino)propionique,
acide 3-[7-[(imidazolin-2-ylamino)méthyl]-1-(2-phényléthyl)-6,8-difluoroquinoline-4-one-3-ylcarbonylamino]-2-(n-butyloxycarbonylamino)propionique,
acide 3-[7-[(imidazolin-2-ylamino)méthyl]-1-(2-phényléthyl)-6,8-difluoroquinoline-4-one-3-ylcarbonylamino]-2-(phénylsulfonylamino)propionique,
acide 3-[7-[(imidazolin-2-ylamino)méthyl]-1-(2-phényléthyl)-6,8-difluoroquinoline-4-one-3-ylcarbonylamino]-2-(n-butylsulfonylamino)propionique,
acide 3-[7-[(tétrahydropyrimid-2-ylamino)méthyl]-1-(2-phényléthyl)-6,8-difluoroquinoline-4-one-3-ylcarbonylamino]-2-(benzyloxycarbonylamino)propionique,
acide 3-[7-[(tétrahydropyrimid-2-ylamino)méthyl]-1-(2-phényléthyl))-6,8-difluoroquinoline-4-one-3-ylcarbonylamino]-2-(n-butyloxycarbonylamino)propionique,
acide 3-[7-[(tétrahydropyrimid-2-ylamino)méthyl]-1-(2-phényléthyl)-6,8-difluoroquinoline-4-one-3-ylcarbonylamino]-2-(phénylsulfonylamino)propionique,
acide 3-[7-[(tétrahydropyrimid-2-ylamino)méthyl]-1-(2-phényléthyl)-6,8-difluoroquinoline-4-one-3-ylcarbonylamino]-2-(n-butylsulfonyl)aminopropionique,
acide 3-[7-[(2-aminothiazol-4-yl)méthyl]-1-(2-phényléthyl)-6,8-difluoroquinoline-4-one-3-ylcarbonylamino]-2-(phénylsulfonylamino)propionique,
acide 3-[7-[(2-aminothiazol-4-yl)méthyl]-1-(2-phényléthyl)-6,8-difluoroquinoline-4-one-3-ylcarbonylamino]-2-(benzyloxycarbonylamino)propionique,
acide 3-[7-[(imidazolin-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(tétrahydropyrimid-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-(2-phényléthyl)-6,8-difluoroquinoline-4-one-3-ylcarbonylamino]-2-(benzyloxycarbonylamino)propionique,
acide 3-[7-[(imidazol-2-ylamino)méthyl]-I- (2-phényléthyl)-6,8-difluoroquinoline-4-one-3-ylcarbonylamino]-2-(phénylsulfonylamino)propionique,
acide 3-[7-[(imidazol-2-ylamino)méthyl]-1-(2-phényléthyl)-6,8-difluoroquinoline-4-one-3-ylcarbonylamino]-2-((2,6-dichlorophényl)sulfonylamino)propionique,
acide 3-[7-[(imidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-(2-phényléthyl)-6,8-difluoroquinoline-4-one-3-ylcarbonylamino]-2-((4-biphényl)sulfonylamino)propionique,
acide 3-[7-[(benzimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(4-méthylimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(4, 5-diméthylimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(4,5,6,7-tétrahydrobenzimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(pyridin-2-ylamino)méthyl]-1-(2-phényléthyl)- 6,8-difluoroquinoline-4-one-3-ylcarbonylamino]-2-((2,4,6-triméthylphényl)sulfonylamino)propionique,
acide 3-[7-(2-aminopyridin-6-yl)-1-(2-phényléthyl)-6,8-difluoroquinoline-4-one-3-ylcarbonylamino]-2-((2,4,6-triméthylphényl)sulfonylamino)propionique, et
acide 3-[7-(7-azabenzimidazol-2-yl)méthyl]-1-(2-phényléthyl)-6,8-difluoroquinoline-4-one-3-ylcarbonylamino]-2-((2,4,6-triméthylphényl)sulfonylamino)propionique.

8. Trousse selon la revendication 1, dans laquelle le composé est choisi dans le groupe :
acide 2-(((4-(4-(((3-(2-(2-(3-((6-((1-aza-2-(2-sulfophényl)vinyl)amino)(3-pyridyl))carbonylamino)propoxy)éthoxy)éthoxy)propyl)amino)sulfonyl)phényl)phén yl)sulfonyl)amino)-3-((7-((imidazol-2-ylamino)méthyl)-1-méthyl-4-oxo(3-hydroquinolyl))carbonylamino)propanoïque ;
acide 3-((7-((imidazol-2-ylamino)méthyl)-1-méthyl-4-oxo(3-hydroquinolyl))carbonylamino)-2-(((4-(4-(((3-(2-(2-(3-(2-(1,4,7,10-tétraaza-4,7,10-tris(carboxylméthyl)cyclododécyl)acétylamino)propoxy)éthoxy)éthoxy)propyl)amin o)sulfonyl)phényl)phényl)sulfonyl)amino)propanoïque ;
acide 2-(((4-(3-(N-(3-(2-(2-(3-((6-((1-aza-2-(2-sulfophényl)vinyl)amino)(3-pyridyl))carbonylamino)propoxy)éthoxy)éthoxy)propyl)carbomoyl)propoxy)-2,6-diméthylphényl)sulfonyl)amino)-3-((7-((imidazol-2-ylamino)méthyl)-1-méthyl-4-oxo(3-hydroquinolyl))carbonylamino)propanoïque ;
acide 3-((1-(3-((6-((1-aza-2-(2-sulfophényl)vinyl)amino)(3-pyridyl))carbonylamino)propyl)-7-((imidazole-2-ylamino)méthyl)-4-oxo(3-hydroquinolyl))carbonylamino)-2-(((2,4,6-triméthylphényl)sulfonyl)amino)propanoïque ;
acide 3-((1-(3-((6-((1-aza-2-(2-sulfophényl)vinyl)amino)(3-pyridyl))carbonylamino)propyl)-7-(((1-hydroxyimidazole-2-yl)amino)méthyl)-4-oxo(3-hydroquinolyl))carbonylamino)-2-(((2,4,6-triméthylphényl)sulfonyl)amino)propanoïque ;
acide 3-((1-(3-(3-(N-(3-(2-(2-(3-((6-((1-aza-2-(2-sulfophényl)vinyl)amino)(3-pyridyl))carbonylamino)propoxy)éthoxy)éthoxy)propyl)carbamoyl)propanoylamino) propyl)-7-((imidazole-2-ylamino)méthyl)-4-oxo(3-hydroquinolyl))carbonylamino)-2-(((2,4,6-triméthylphényl)sulfonyl)amino)propanoïque ;
acide 2-(2-aza-2-(5-(N-(1,3-bis(3-(2-(2-(3-(3-(N-(3-(3-(N-(3-carboxy-2-(((2,4,6-triméthylphényl)sulfonyl)amino)éthyl)carbamoyl)-7-((imidazole-2-ylamino)méthyl)-4-oxohydroquinolyl)propyl)carbamoyl)propanoylamino)propoxy)éthoxy)éthoxy)propy I)carbomoyl)(2-pyridyl))amino)vinyl)benzènesulfonique ; 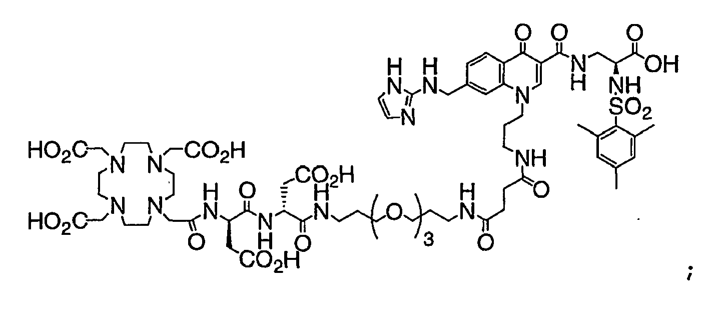 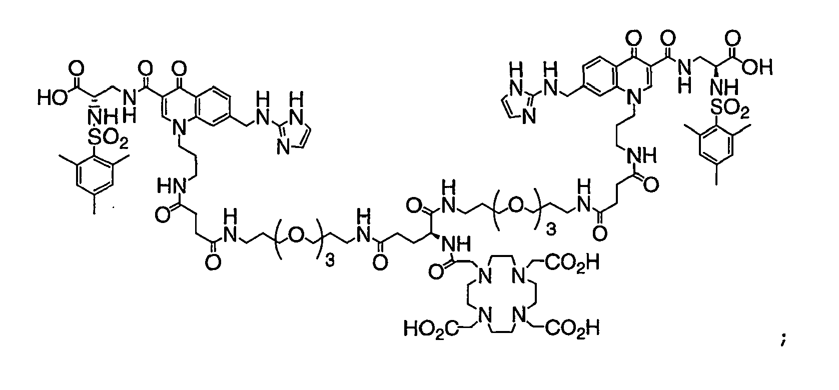 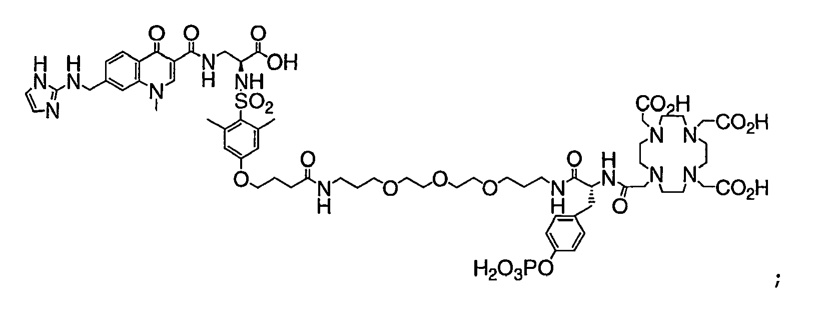 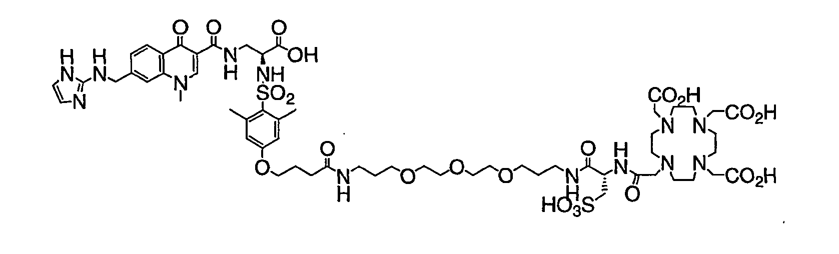 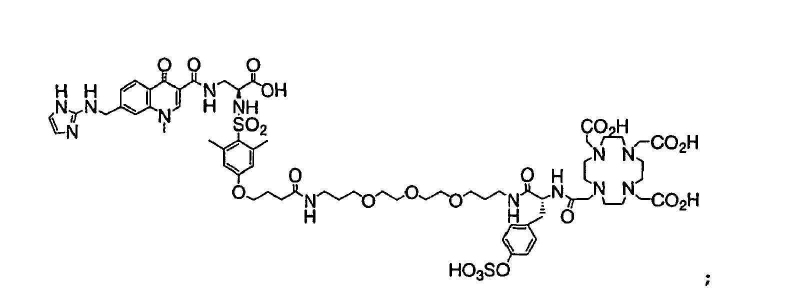 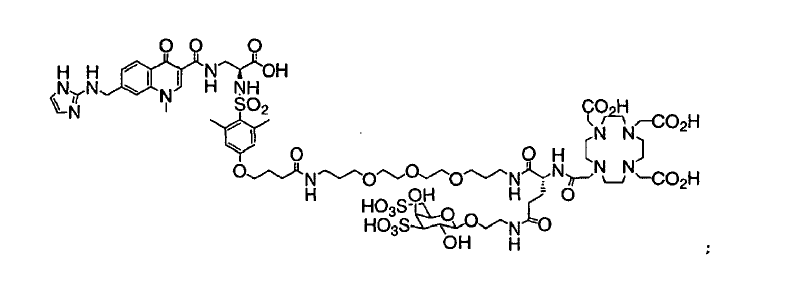 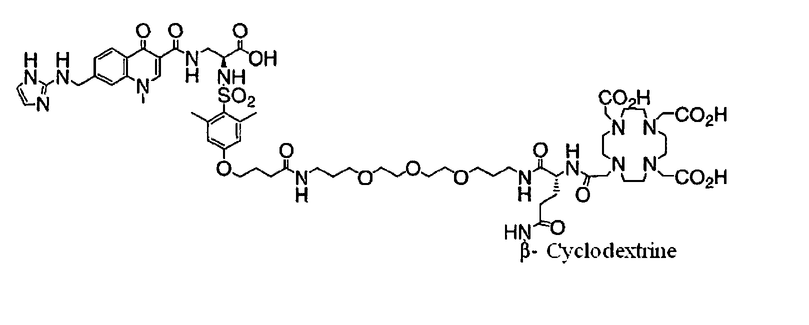 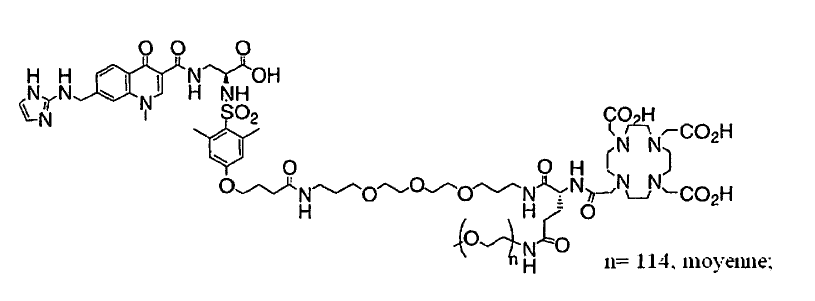
acide 2-(((4-(3-(N-(3-(2-(2-(3-(2-(1,4,7,10-tétraaza-4,7,10-tris(carboxyméthyl)cyclododécylacétylamino)-6-aminohexanoylamino)propoxy)éthoxy)éthoxy)propyl)carbamoyl)propoxy)-2,6-diméthylphényl)sulfonyl)amino)-3-((7-((imidazol-2-ylamino)méthyl)-1-méthyl-4-oxo(3-hydroquinolyl))carbonylamino)propanoïque ;
acide conjugué 2-(((4-(3-(N-(3-(2-(2-(3-(2-(1,4,7,10-tétraaza-4,7,10-tris(carboxyméthyl)cyclododécylacétylamino)-6-(2-(bis(phosphonométhyl)amino)acétylamino)hexanoylamino)propoxy)éthoxy)éthoxy )propyl)carbamoyl)propoxy)-2,6-diméthylphényl)sulfonyl)amino)-3-((7-((imidazol-2-ylamino)méthyl)-1-méthyl-4-oxo(3-hydroquinolyl))carbonylamino)propanoïque; et
acide 2-(((4-(3-(N-(3-(2-(2-(3-(2-(2-((2-((2-(bis(carboxyméthyl)amino)éthyl)(carboxyméthyl)amino)éthyl)(carboxyméthyl)amin o)acétylamino)-3-sulfopropyl)propoxy)éthoxy)éthoxy)propyl)carbamoyl)propoxy)-2,6-diméthylphényl)sulfonyl)amino)-3-((7-((imidazol-2-ylamino)méthyl)-1-méthyl-4-oxo(3-hydroquinolyl))carbonylamino)propanoïque ; 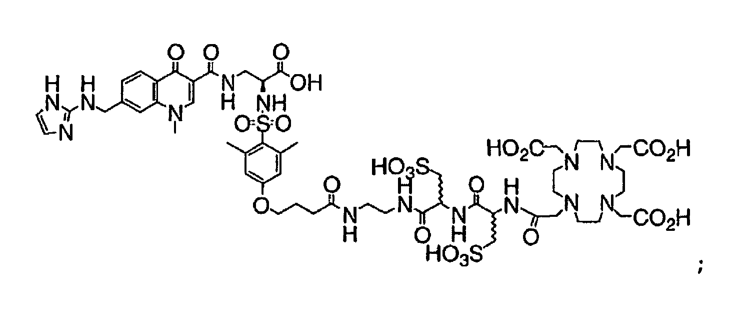 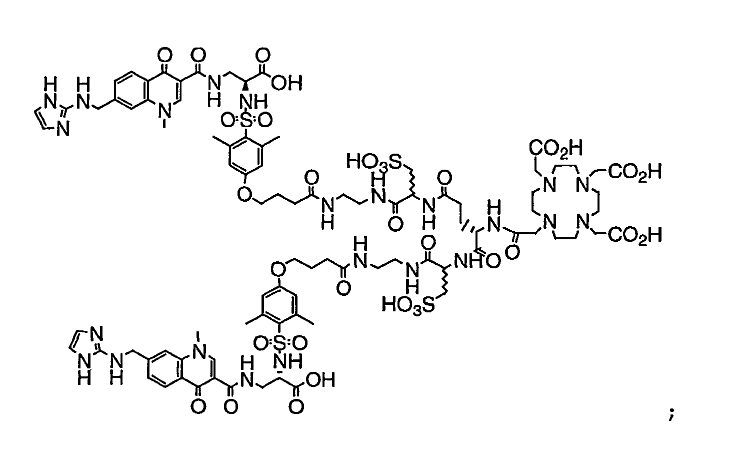 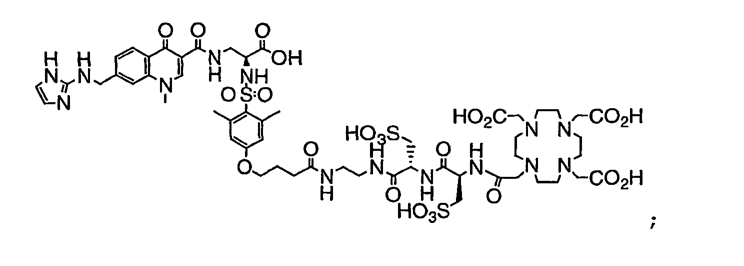 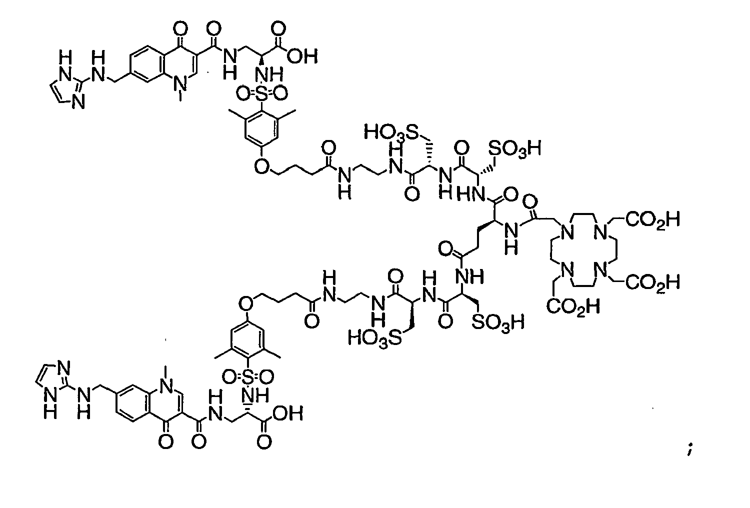 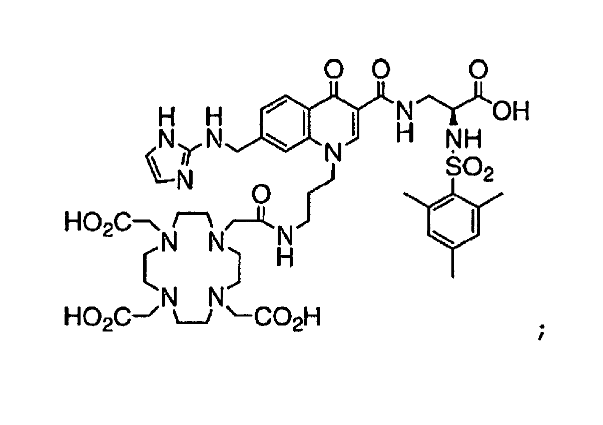 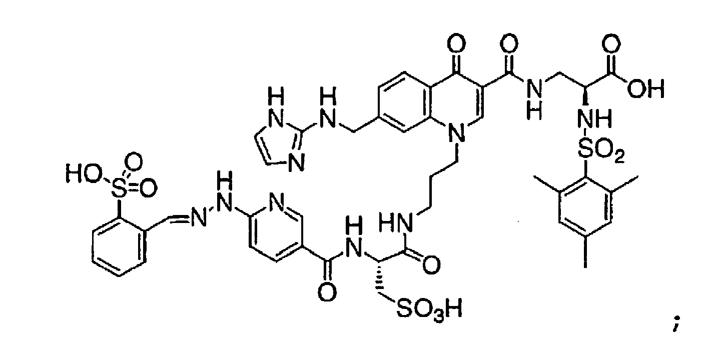 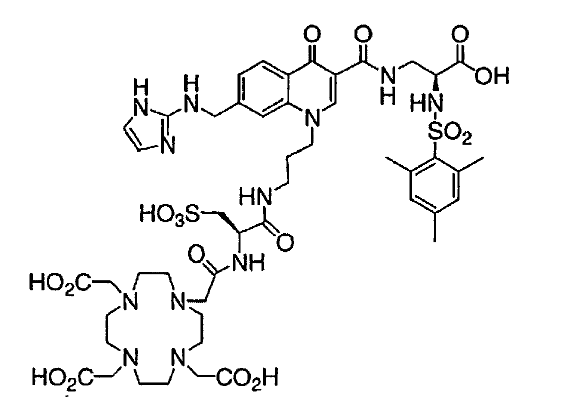 ; et 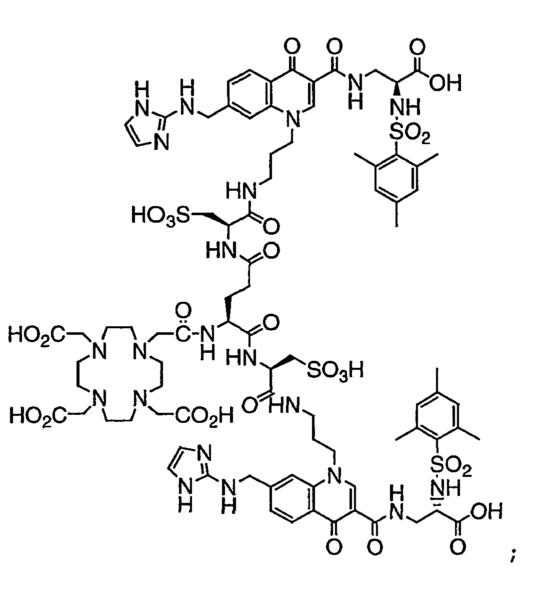
ou sous la forme d'un sel de ceux-ci acceptable sur le plan pharmaceutique.

9. Trousse selon la revendication 1, laquelle trousse comprend en outre un ou plusieurs ligands auxiliaires et un agent réducteur.

10. Trousse selon la revendication 9, dans laquelle les ligands auxiliaires sont la tricine et la TPPTS.

11. Trousse selon la revendication 9, dans laquelle l'agent réducteur est l'étain (II).

12. Composition radiopharmaceutique thérapeutique comprenant au moins un agent choisi dans le groupe constitué par un agent anticancéreux et un agent de radiosensibilisation, ou un sel de ceux-ci acceptable sur le plan pharmaceutique, et un agent radiopharmaceutique comprenant :
a) un métal ;
b) un chélatant capable de chélater le métal ; et
c) un motif de ciblage ;
où le motif de ciblage est lié au chélatant par 0 à 1 groupe de liaison, et le motif de ciblage est une quinolone non-peptidique qui se lie à un récepteur qui est régulé en amont pendant l'angiogénèse.

13. Composition radiopharmaceutique thérapeutique selon la revendication 12, dans laquelle l'agent anticancéreux est choisi dans le groupe constitué par la mytomycine, la trétinoïne, la ribomustine, la gemcitabine, la vincristine, l'étoposide, la cladribine, le mitobronitol, le méthotrexate, la doxorubicine, la carboquone, la pentostatine, la nitracrine, la zinostatine, le cétrorélix, le létrozole, le raltitrexed, la daunorubicine, la fadrozole, la fotémustine, la thymalfasine, le sobuzoxane, le nédaplatine, la cytarabine, le bicalutamide, la vinorelbine, la vesnarinone, l'aminoglutéthimide, l'amsacrine, le proglumide, l'acétate d'elliptinium, la kétansérine, la doxifluridine, l'étrétinate, l'isotrétinoïne, la streptozocine, la nimustine, la vindésine, le flutamide, le drogénil, la butocine, le carmofur, le razoxane, la sizofilane, le carboplatine, le mitolactol, le tegafur, l'ifosfamide, la prednimustine, le picibanil, le lévamisole, le téniposide, l'improsulfane, l'énocitabine, le lisuride, l'oxymétholone, le tamoxifène, la progestérone, le mépitiostane, l'épitiostanol, le formestane, l'interféron-alpha, l'interféron-2 alpha, l'interféron-beta, l'interféron-gamma, le facteur 1 de stimulation de colonie, le facteur 2 de stimulation de colonie, le dénileukine diftitox, l'interleukine-2, et le facteur de libération de l'hormone lutéinisante.

14. Composition radiopharmaceutique thérapeutique selon la revendication 12, dans laquelle l'agent de radiosensibilisation est choisi dans le groupe constitué par le 2-(3-nitro-1,2,4-triazol-1-yl)-N-(2-méthoxyéthyl)acétamide, la N-(3-nitro-4-quinolinyl)-4-morpholinecarboxamidine, le 3-amino-1,2,4-benzotriazine-1,4-dioxide, le N-(2-hydroxyéthyl)-2-nitroimidazole-1-acétamide, le 1-(2-nitroimidazol-1-yl)-3-(1-pipéridinyl)-2-propanol, et le 1-(2-nitro-1-imidazolyl)-3-(1-aziridino)-2-propanol.

15. Composition radiopharmaceutique thérapeutique selon la revendication 12, dans laquelle le métal est choisi dans le groupe : ³³P, ²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd , ¹⁵⁹Gd, ¹⁴⁰_{La}, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, et ¹⁹²Ir, et le groupe de liaison est présent entre le motif de ciblage non-peptidique et le chélatant.

16. Composition radiopharmaceutique thérapeutique selon la revendication 15, dans laquelle le motif de ciblage est une quinolone non-peptidique et le récepteur est αᵥβ₃ ou αᵥβ₅.

17. Composition radiopharmaceutique thérapeutique selon la revendication 12, dans laquelle l'agent radiopharmaceutique comprend :
a) un métal choisi dans le groupe : ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au , ¹⁶⁹Yb, ¹⁷⁵Yb, ¹⁶⁵Dy, ¹⁶⁶Dy, ⁶⁷Cu, ¹⁰⁵Rh, ¹¹¹Ag, et ¹⁹²Ir; et
b) un composé de formule (I) :
(Q)_{d}-Lₙ-Cₕ ou (Q)_{d}-Lₙ-(Cₕ)_{d'}
dans laquelle Q est un composé de formule (II) : 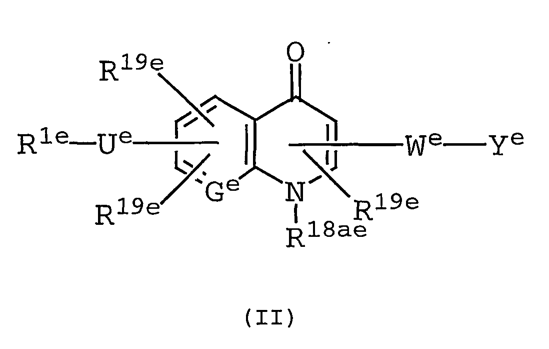 incluant les formes stéréoisomériques de celle-ci, ou les mélanges des formes stéréoisomériques de celle-ci, ou les formes de sel ou de promédicament de celle-ci acceptables sur le plan pharmaceutique, dans laquelle :
R^{1e} est choisi parmi : 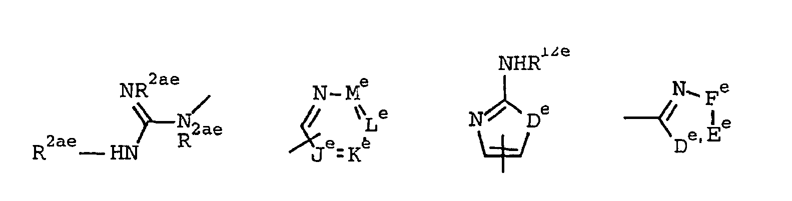 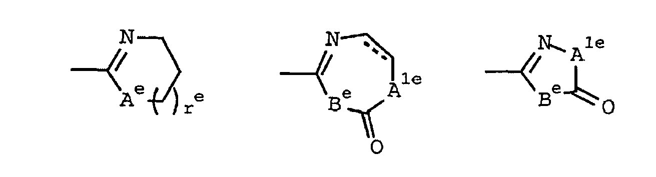 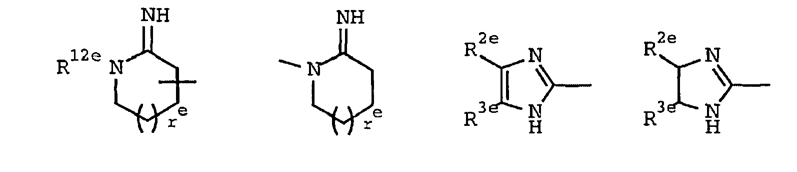 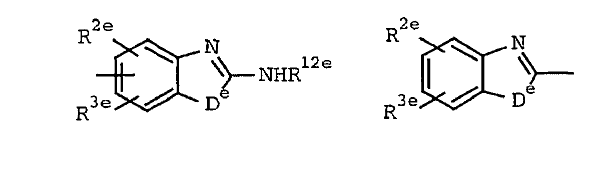 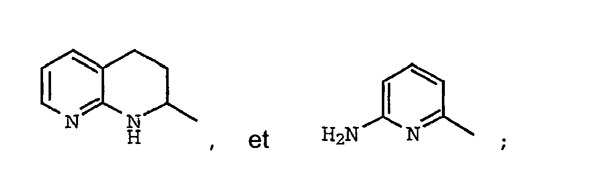
A^{e} représente -CH₂- ou -N(R^{10e})- ;
A^{1e} et B^{e} représentent indépendamment -CH₂- ou -N(R^{10e})- ;
De représente -N(R^{10e})- ou -S- ;
E^{e}-F^{e} représente -C(R^{2e})=C(R^{3e})- ou -C(R^{2e})₂C(R^{3e})₂- ;
J^{e} représente -C(R^{2e})- ou -N- ;
K^{e}, Le et M^{e} représentent indépendamment -C(R^{2e})- ou -C(R^{3e})-;
R^{2e} et R^{3e} sont indépendamment choisis parmi :
H, un groupe alcoxy en C₁-C₄, NR^{11e}R^{12e}, un halogène, NO₂, CN, CF₃, un groupe alkyle en C₁-C₆, un groupe alcényle en C₃-C₆, un groupe cycloalkyle en C₃-C₇, un groupe cycloalkyle(alkyle en C₁-C₄) en C₃-C₇, un groupe aryle(alkyle en C₁-C₆), un groupe (alkyle en C₁-C₆)carbonyle, un groupe (alcoxy en C₁-C₆)carbonyle, un groupe arylcarbonyle, et un groupe aryle substitué par 0 à 4 R^{7e},
en variante, lorsque R^{2e} et R^{3e} sont des substituants liés à des atomes adjacents, ils peuvent former conjointement avec les atomes de carbone auxquels ils sont liés un carbocycle à 5-7 chaînons ou un hétérocycle à 5-7 chaînons, aromatique ou non aromatique, ledit carbocycte ou hétérocycle étant substitué par 0 à 2 groupes choisis parmi un groupe alkyle en C₁-C₄, un groupe alcoxy en C₁-C₄, un groupe halogéno, un groupe cyano, un groupe amino, CF₃ et NO₂ ;
R^{2ae} est choisi parmi :
H, un groupe alkyle en C₁-C₁₀, un groupe alcényle en C₂-C₆, un groupe cycloalkyle en C₃-C₁₁, un groupe cycloalkyl(alkyle en C₁-C₄) en C₃-C₇, un groupe aryle, un groupe aryle(alkyle en C₁-C₄), un groupe (alkyle en C₂-C₇)carbonyle, un groupe arylcarbonyle, un groupe (alcoxy en C₂-C₁₀)carbonyle, un groupe cycloalcoxycarbonyle en C₃-C₇, un groupe bicycloalcoxycarbonyle en C₇-C₁₁, un groupe aryloxycarbonyle, un groupe aryl(alcoxy en C₁-C₁₀)carbonyle, un groupe alkylcarbonyloxy(alcoxy en C₁-C₄)carbonyle en C₁-C₆, un groupe arylcarbonyloxy(alcoxy en C₁-C₄)carbonyle, et un groupe cycloalkylcarbonyloxy(alcoxy en C₁-C₄)carbonyle en C₃-C₇ ;
R^{7e} est choisi parmi :
H, un groupe hydroxy, un groupe alkyle en C₁-C₄, un groupe alcoxy en C₁-C₄, un groupe aryle, un groupe aryle(alkyle en C₁-C₄), un groupe (alkyle en C₁-C₄)carbonyle, CO2R^{18ae}, SO₂R^{11e}, SO₂NR^{10e}R^{11e}, OR^{10e}, et N(R^{11e})R^{12e} _{;}
U^{e} est choisi parmi :
-(CH₂)ₙe-, -(CH₂)ₙeO(CH₂)ₘe-, -(CH₂)ₙeN(R¹²)(CH₂)ₘe-, -NH(CH₂)ₙe-, -(CH2)ₙeC(=O)(CH₂)ₘe-, -(CH₂)ₙeS(O)ₚe(CH₂)ₘe-, -(CH₂)ₙeNHNH(CH₂)ₘe-, -N(R^{10e})C(=O)-, -NHC(=O)(CH₂)ₙe-, -C(=O)N(R^{10e})-, et -N(R^{10e})S(O)ₚe-;
G^{e} représente N ou CR^{19e} ;
W^{e} représente -C(=O)-N(R^{10e})-alkylène en C₁-C₃)-, où le groupe alkylène est substitué par R^{8e} et par R^{9e} :
R^{8e} et R^{9e} sont indépendamment choisis parmi :
H, CO₂R^{18be}, C(=O)R^{18be}, CONR¹⁷R^{18be}, un groupe alkyle en C₁-C₁₀ substitué par 0 à 1 R^{6e}, un groupe alcényle en C₂-C₁₀ substitué par 0 à 1 R^{6e}, un groupe alcynyle en C₂-C₁₀ substitué par 0 à 1 R^{6e}, un groupe cycloalkyle en C₃-C₈ substitué par 0 à 1 R^{6e}, un groupe cycloalcényle en C₅-C₆ substitué par 0 à 1 R^{6e}, un groupe (alkyle en C₁-C₁₀)carbonyle, un groupe cycloalkyle(alkyle en C₁-C₄) en C₃-C₁₀, un groupe phényle substitué par 0 à 3 R^{6e}, un groupe naphtyle substitué par 0 à 3 R^{6e}, un hétérocycle à 5-10 chaînons contenant 1 à 3 hétéroatomes N, O ou S, ledit hétérocycle pouvant être saturé, partiellement saturé, ou entièrement insaturé, et ledit hétérocycle étant substitué par 0 à 2 R^{7e}, un groupe alcoxy en C₁-C₁₀ substitué par 0 à 2 R^{7e}, un groupe hydroxy, un groupe nitro, -N(R^{10e})R^{11e}, -N(R^{16e})R^{17e}, un groupe aryl(alkyle en C₀-C₆)carbonyle, aryl(alkyle en C₃-C₆), hétéroaryl(alkyle en C₁-C₆), CONR^{18ae}R^{20e}, SO₂R^{18ae} et SO₂NR^{18ae}R^{20e},
sachant que chacun desdits groupes alkyles, cycloalkyles, aryles ou hétéroaryles peut être non substitué ou substitué de manière indépendante par 1 à 2 R^{7e};
R^{6e} est choisi parmi :
H, un groupe alkyle en C₁-C₁₀, un groupe hydroxy, un groupe alcoxy en C₁-C₁₀, un groupe nitro, un groupe alkylcarbonyle en C₁-C₁₀, -N(R^{11e})R^{12e}, un groupe cyano, un groupe halogéno, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e}, un groupe aryle substitué par 0 à 3 groupes choisis parmi un halogène, un groupe alcoxy en C₁-C₆, un groupe alkyle en C₁-C₆, CF₃, S(O)ₘeMe, et -NMe₂, un groupe aryle(alkyle en C₁-C₄), ledit groupe aryle étant substitué par 0 à 3 groupes choisis parmi un halogène, un groupe alcoxy en C₁-C₆, un groupe alkyle en C₁-C₆, CF₃, S(O)ₚeMe, et -NMe₂, et un hétérocycle à 5-10 chaînons contenant 1 à 3 hétéroatomes N, O, ou S, ledit hétérocycle pouvant être saturé, partiellement saturé ou entièrement insaturé, ledit hétérocycle étant substitué par 0 à 2 R^{7e} ;
R^{10e} est choisi parmi :
H, -OH, CF₃, un groupe alcényle en C₃-C₆, un groupe cycloalkyle en C₃-C₁₁ , un groupe aryle, un groupe (cycloalkyle en C₃-C₁₁)méthyle, un groupe aryle(alkyle en C₁-C₄), et un groupe alkyle en C₁-C₁₀ substitué par 0 à 2 R^{6e};
R11^{e} est choisi parmi :
H, un groupe hydroxy, un groupe alkyle en C₁-C₈, un groupe alcényle en C₃-C₆, un groupe cycloalkyle en C₃-C₁₁, un groupe (cycloalkyle en C₃-C₁₁ )méthyle, un groupe alcoxy en C₁-C₆, un groupe benzyloxy, un groupe aryle, un groupe hétéroaryle, un groupe hétéroaryle(alkyle en C₁-C₄), un groupe aryle(alkyle en C₁-C₄), un groupe adamantylméthyle, et un groupe alkyle en C₁-C₁₀ substitué par 0 à 2 R^{4e};
R^{4e} est choisi parmi :
H, un groupe alkyle en C₁-C₆, un groupe cycloalkyle en C₃-C₇, un groupe cycloalkyle(alkyle en C₁-C₄) en C₃-C₇, un groupe (alkyle en C₁-C₁₀)carbonyle, un groupe aryle, un groupe hétéroaryle, un groupe aryle(alkyle en C₁-C₆), et un groupe hétéroaryle(alkyle en C₁-C₆), lesdits groupes aryles ou hétéroaryles étant substitués par 0 à 2 substituants indépendamment choisis dans le groupe constitué par un groupe alkyle en C₁-C₄, un groupe alcoxy en C₁-C₄, F, CI, Br, CF₃ et NO₂,
en variante, lorsque R^{10e} et R^{11e} sont tous deux des substituants liés au même atome d'azote (comme dans -NR^{10e}R^{11e}), ils peuvent former conjointement avec l'atome d'azote auquel ils sont liés un hétérocycle choisi parmi :
le 3-azabicyclononyle, le 1,2,3,4-tétrahydro-1-quinolinyle, le 1,2,3,4-tétrahydro-2-isoquinolinyle, le 1-pipéridinyle, le 1-morpholinyle, le 1-pyrrolidinyle, le thiamorpholinyle, le thiazolidinyle, et le 1-pipérazinyle ; ledit hétérocycle étant substitué par 0 à 3 groupes choisis parmi :
un groupe alkyle en C₁-C₆, un groupe aryle, un groupe hétéroaryle, un groupe aryle(alkyle en C₁-C₄), un groupe (alkyle en C₁-C₆)carbonyle, un groupe (cycloalkyle en C₃-C₇)carbonyle, un groupe (alcoxy en C₁-C₆)carbonyle, un groupe aryle(alcoxy en C₁-C₄)carbonyle, un groupe alkylsulfonyle en C₁-C₆, et un groupe arylsulfonyle ;
R^{12e} est choisi parmi :
H, un groupe alkyle en C₁-C₆, un groupe triphénylméthyle, un groupe méthoxyméthyle, un groupe méthoxyphényldiphénylméthyle, un groupe triméthylsilyléthoxyméthyle, un groupe (alkyle en C₁-C₆)carbonyle, un groupe (alcoxy en C₁-C₆)carbonyle, un groupe (alkyle en C₁-C₆)aminocarbonyle, un groupe alcényle en C₃-C₆, un groupe cycloalkyle en C₃-C₇, un groupe cycloalkyle(alkyle en C₁-C₄) en C₃-C₇, un groupe aryle, un groupe hétéroaryle(alkyle en C₁-C₆)carbonyle, un groupe hétéroarylcarbonyle, un groupe aryle(alkyle en C₁-C₆), un groupe (alkyle en C₁-C₆)carbonyle, un groupe arylcarbonyle, un groupe alkylsulfonyle en C₁-C₆, un groupe arylsulfonyle, un groupe aryle(alkyle en C₁-C₆)sulfonyle, un groupe hétéroarylsulfonyle, un groupe hétéroaryle(alkyle en C₁-C₆)sulfonyle, un groupe aryloxycarbonyle, et un groupe aryle(alcoxy en C₁-C₆)carbonyle, lesdits groupes aryles étant substitués par 0 à 2 substituants choisis dans le groupe constitué par un groupe alkyle en C₁-C₄, un groupe alcoxy en C₁-C₄, un groupe halogéno, CF₃, et un groupe nitro ;
R^{16e} est choisi parmi :
-C(=O)OR^{18ae}, -C(=O)R^{18be},-C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, et -SO₂NHC(=O)OR^{18be};
R^{17e} est choisi parmi :
H, un groupe alkyle en C₁-C₆, un groupe cycloalkyle en C₃-C₇, un groupe cycloalkyle(alkyle en C₁-C₄) en C₃-C₇, un groupe aryle, un groupe aryle(alkyle en C₁-C₆), et un groupe hétéroaryle(alkyle en C₁-C₆) ;
R^{18ae} est choisi parmi :
un groupe alkyle en C₁-C₈ éventuellement substitué par une liaison à Lₙ, un groupe cycloalkyle en C₃-C₁₁ éventuellement substitué par une liaison à Lₙ, un groupe aryle(alkyle en C₁-C₆) éventuellement substitué par une liaison à Lₙ, un groupe hétéroaryle(alkyle en C₁-C₆) éventuellement substitué par une liaison à Lₙ, un groupe (alkyle en C₁-C₆)hétéroaryle éventuellement substitué par une liaison à Lₙ, un groupe biaryle(alkyle en C₁-C₆) éventuellement substitué par une liaison à Lₙ, un groupe hétéroaryle éventuellement substitué par une liaison à Lₙ, un groupe phényle substitué par 3 à 4 R^{19e} et éventuellement substitué par une liaison à Lₙ, un groupe naphtyle substitué par 0 à 4 R^{19e} et éventuellement substitué par une liaison à Lₙ, et une liaison à Lₙ, lesdits groupes aryles ou hétéroaryles étant éventuellement substitués par 0 à 4 R^{19e} ;
R^{18be} représente H ou R^{18ae}
R^{19e} est choisi parmi :
H, un halogène, CF₃, CO₂H, CN, NO₂, -NR^{11e}R^{12e}, OCF₃, un groupe alkyle en C₁-C₈, un groupe alcényle en C₂-C₆, un groupe alcynyle en C₂-C₆, un groupe cycloalkyle en C₃-C₁₁, un groupe cycloalkyle(alkyle en C₁-C₄) en C₃-C₇, un groupe aryle(alkyle en C₁-C₆), un groupe alcoxy en C₁-C₆, un groupe alcoxycarbonyle en C₁-C₄, un groupe aryle, un groupe aryle-O-, un groupe aryle-SO₂-, un groupe hétéroaryle, et un groupe hétéroaryle-SO₂-, lesdits groupes aryles et hétéroaryles étant substitués par 0 à 4 groupes choisis parmi l'hydrogène, un halogène, CF₃, un groupe alkyle en C₁-C₃, et un groupe alcoxy en C₁-C₃ ;
R^{20e} est choisi parmi les groupes :
hydroxy, alkyloxy en C₁-C₁₀, cycloalkyloxy en C₃-C₁₁ , aryloxy, aryl(alkyle en C₁-C₄)oxy, alkylcarbonyloxy(alkyle en C₁-C₂)oxy en C₂-C₁₀, alcoxycarbonyloxy(alkyle en C₁-C₂)oxy en C₂-C₁₀, alcoxycarbonyl(alkyle en C₁-C₂)oxy en C₂-C_{10,} cycloalkylcarbonyloxy(alkyle en C₁-C₂)oxy en C₃-C₁₀, cycloalcoxycarbonyloxy(alkyle en C₁-C₂)oxy en C₃-C₁₀ cycloalcoxycarbonyl(alkyle en C₁-C₂)oxy en C₃-C₁₀, aryloxycarbonyl(alkyle en C₁-C₂)oxy, aryloxycarbonyloxy(alkyle en C₁-C₂)oxy, arylcarbonyloxy(alkyle en C₁-C₂)oxy, alcoxy(alkyle en C₁-C₅)carbonyloxy(alkyle en C₁-C₂)oxy en C₁-C₅, (5-(alkyle en C₁-C₅)-1,3-dioxa-cyclopentèn-2-one-yl)méthyloxy, (5-aryl-1,3-dioxa-cyclopentèn-2-one-yl)méthyloxy, et (R^{10e})(R^{11e})N(alcoxy en C₃-C₁₀) ;
R^{21e} est choisi parmi les groupes :
alkyle en C₁-C₈, alcényle en C₂-C₆, cycloalkyle en C₃-C₁₁, (cycloalkyle en C₃-C₁₁)méthyle, aryle, aryle(alkyle en C₁-C₄), et alkyle en C₁-C₁₀ substitué par 0 à 2 R^{7e};
R^{22e} est choisi parmi :
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, et -C(=O)NHSO₂NHR^{18be} ;
Y^{e} est choisi parmi :
-COR^{20e}, -SO₃H, -PO₃H, -CONHNHSO₂CF₃, -CONHSO₂R^{18ae}, -CONHSO₂NHR^{18be}, -NHCOCF₃, -NHCONHSO₂R^{18ae}, -NHSO₂R^{18ae}, -OPO₃H₂, -OSO₃H, -PO₃H₂, -SO₂NHCOR^{18ae}, -SO₂NHCO₂R^{18ae}, 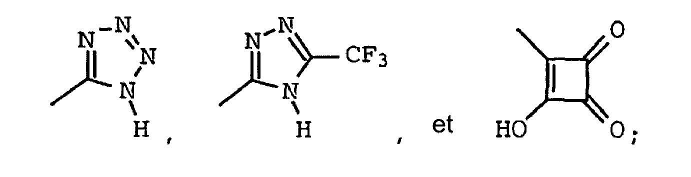
m^{e} est 0-2 ;
ne est 0-4 ;
p^{e} est 0-2 ;
r^{e} est 0-2 ;
avec la clause suivante :
n^{e} et m^{e} sont choisis de telle sorte que le nombre d'atomes reliant R^{1e} et Y^{e} est dans la plage comprise entre 8 et 14 ;
d est choisi parmi 1, 2, 3, 4, 5, 6, 7, 8, 9 et 10 ;
d' est 1-100 ;
Lₙ est un groupe de liaison de formule :
((W)ₕ-(CR⁶R⁷)_{g})ₓ-(Z)ₖ-((CR^{6a}R^{7a})_{g}^{,}-(W)_{h'},)_{x'} ;
W est indépendamment choisi à chaque occurrence dans le groupe : O, S, NH, NHC(=O), C(=O)NH, NR⁸C(=O), C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)ₛ", (CH₂CH₂CH₂O)ₜ, et (aa)t' ;
aa est indépendamment à chaque occurrence un acide aminé ;
z est choisi dans le groupe :
groupe aryle substitué par 0 à 3 R¹⁰, groupe cycloalkyle en C₃-C₁₀ substitué par 0 à 3 R¹⁰, et hétérocycle à 5-10 chaînons contenant 1 à 4 hétéroatomes indépendamment choisis parmi N, S, et O et substitué par 0 à 3 R¹⁰;
R⁶, R^{6a}, R⁷, R^{7a}, et R⁸ sont indépendamment choisis à chaque occurrence dans le groupe :
H, =O, COOH, SO₃H, PO₃H, un groupe alkyle en C₁-C₅ substitué par 0 à 3 R¹⁰, un groupe aryle substitué par 0 à 3 R¹⁰, un groupe benzyle substitué par 0 à 3 R¹⁰, et un groupe alcoxy en C₁-C₅ substitué par 0 à 3 R¹⁰, NHC(=O)R¹¹, C(=O)NHR¹¹, NHC(=O)NHR¹¹, NHR¹¹, R¹¹ et une liaison à C_{h;}
R¹⁰ est indépendamment choisi à chaque occurrence dans le groupe :
une liaison à Cₕ, COOR¹¹, C(=O)NHR¹¹, NHC(=O)R¹¹, OH, NHR¹¹, SO₃H, PO₃H, OPO₃H₂, OSO₃H, un groupe aryle substitué par 0 à 3 R¹¹, un groupe alkyle en C₁-C₅ substitué par 0 à 1 R¹², un groupe alcoxy en C₁-C₅ substitué par 0 à 1 R¹² , et un hétérocycle à 5-10 chaînons contenant 1 à 4 hétéroatomes indépendamment choisis parmi N, S, et O et substitué par 0 à 3 R¹¹ ;
R¹¹ est indépendamment choisi à chaque occurrence dans le groupe :
H, -OPO₃H₂, -OSO₃H, un groupe alkyle en C₁-C₅ substitué par 0 à 1 R¹², un groupe aryle substitué par 0 à 1 R¹², un hétérocycle à 5-10 chaînons contenant 1 à 4 hétéroatomes indépendamment choisis parmi N, S, et O et substitué par 0 à 1 R¹², un groupe cycloalkyle en C₃-C₁₀ substitué par 0 à 1 R¹², un polyalkylène glycol substitué par 0 à 1 R¹², un groupe alkyle en C₁-C₅ substitué par un hydrate de carbone substitué par 0 à 1 R¹², une cyclodextrine substituée par 0 à 1 R¹², un acide aminé substitué par 0 à 1 R¹², un groupe polycarboxyalkyle substitué par 0 à 1 R¹², un groupe polyazaalkyle substitué par 0 à 1 R¹², un peptide substitué par 0 à 1 R¹², ledit peptide étant constitué de 2 à 10 acides aminés, un groupe 3,6-O-disulfo-B-D-galactopyranosyle, une bis(phosphonométhyl)glycine, et une liaison à Cₕ ;
R¹² est une liaison à Cₕ ;
k est choisi parmi 0, 1, et 2 ;
h est choisi parmi 0, 1, et 2 ;
h' est choisi parmi 0, 1, et 2 ;
g est choisi parmi 0, 1, 2, 3, 4, 5, 6, 7, 8, 9 et 10 ;
g' est choisi parmi 0, 1, 2, 3, 4, 5, 6, 7, 8, 9 et 10 ;
s est choisi parmi 0, 1, 2, 3, 4, 5, 6, 7, 8, 9 et 10 ;
s' est choisi parmi 0, 1, 2, 3, 4, 5, 6, 7, 8, 9 et 10 ;
s" est choisi parmi 0, 1, 2, 3, 4, 5, 6, 7, 8, 9 et 10 ;
t est choisi parmi 0, 1, 2, 3, 4, 5, 6, 7, 8, 9 et 10 ;
t' est choisi parmi 0, 1, 2, 3, 4, 5, 6, 7, 8, 9 et 10 ;
x est choisi parmi 0, 1, 2, 3, 4, 5, et 6 ;
x' est choisi parmi 0, 1, 2, 3, 4, 5, et 6 ;
Cₕ est un groupement permettant de lier un métal, dont la formule est choisie dans le groupe: 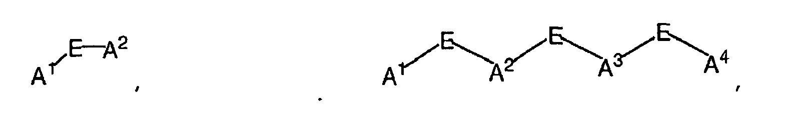 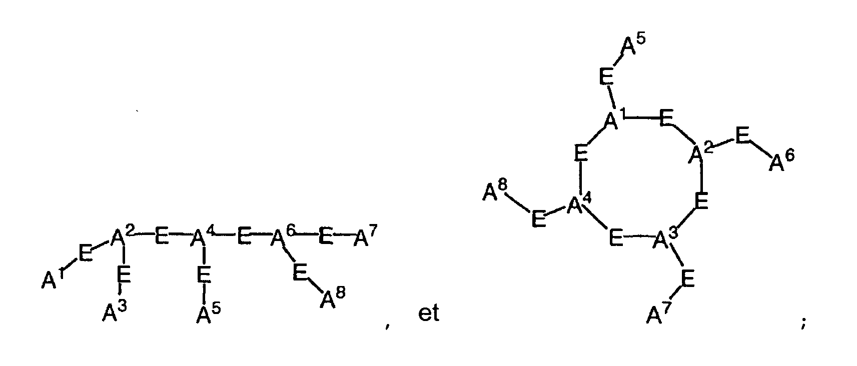
A¹, A², A³, A⁴, A⁵, A⁶, A⁷ et A⁸ sont indépendamment choisis à chaque occurrence dans le groupe :
NR¹³, NR¹³R¹⁴, S, SH, S(Pg), O, OH, PR¹³, PR¹³R¹⁴, P(O)R¹⁵R¹⁶, et une liaison àLₙ;
E est une liaison, CH, ou un groupe espaceur indépendamment choisi à chaque occurrence dans le groupe :
un groupe alkyle en C₁-C₁₀ substitué par 0 à 3 R¹⁷, un groupe aryle substitué par 0 à 3 R¹⁷, un groupe cycloalkyle en C₃-C₁₀ substitué par 0 à 3 R¹⁷, un groupe hétérocyclo(C₁-C₁₀)alkyle substitué par 0 à 3 R¹⁷, ledit groupe hétérocyclo étant un hétérocycle à 5-10 chaînons contenant 1 à 4 hétéroatomes indépendamment choisis parmi N, S, et O, un groupe aryle(C₁-C₁₀)alkyle en C₆-C₁₀ substitué par 0 à 3 R¹⁷, un groupe alkyle(C₆-C₁₀)alkyle en C₁-C₁₀ substitué par 0 à 3 R¹⁷, et un hétérocycle à 5-10 chaînons contenant 1 à 4 hétéroatomes indépendamment choisis parmi N, S, et O et substitué par 0 à 3 R¹⁷ ;
R¹³ et R¹⁴ sont chacun indépendamment choisis dans le groupe :
une liaison à Lₙ, un hydrogène, un groupe alkyle en C₁-C₁₀ substitué par 0 à 3 R¹⁷, un groupe aryle substitué par 0 à 3 R¹⁷, un groupe cycloalkyle en C₁-C₁₀ substitué par 0 à 3 R¹⁷, un groupe hétérocyclo(C₁-C₁₀)alkyle substitué par 0 à 3 R¹⁷, ledit groupe hétérocyclo étant un hétérocycle à 5-10 chaînons contenant 1 à 4 hétéroatomes indépendamment choisis parmi N, S et O, un groupe aryl(C₁-C₁₀)alkyle en C₆-C₁₀ substitué par 0 à 3 R¹⁷, un groupe alkyl(C₆-C₁₀)aryle en C₁-C₁₀ substitué par 0 à 3 R¹⁷, un hétérocyle à 5-10 chaînons contenant 1 à 4 hétéroatomes indépendamment choisis parmi N, S et O et substitués par 0 à 3 R¹⁷ et un électron, sachant que lorsque l'un parmi R¹³ et R¹⁴ est un électron, alors l'autre est aussi un électron ;
en variante, R¹³ et R¹⁴ se combinent pour former =C(R²⁰)(R²¹);
R¹⁵ et R¹⁶ sont chacun indépendamment choisis dans le groupe :
une liaison à Lₙ, -OH, un groupe alkyle en C₁-C₁₀ substitué par 0 à 3 R¹⁷ un groupe aryle substitué par 0 à 3 R¹⁷, un groupe cycloalkyle en C₃-C₁₀ substitué par 0 à 3 R¹⁷ un groupe hétérocyclo(C₁-C₁₀)alkyle substitué par 0 à 3 R¹⁷ ledit groupe hétérocyclo étant un hétérocycle à 5-10 chaînons contenant 1 à 4 hétéroatomes indépendamment choisis parmi N, S, et O, un groupe aryle(C₁-C₁₀)alkyle en C₆-C₁₀ substitué par 0 à 3 R¹⁷, un groupe alkyle(C₁-C₁₀)aryle en C₁-C₁₀ substitué par 0 à 3 R¹⁷, et un hétérocycle à 5-10 chaînons contenant 1 à 4 hétéroatomes indépendamment choisis parmi N, S, et O et substitué par 0 à 3 R¹⁷ ;
R¹⁷ est indépendamment choisi à chaque occurrence dans le groupe :
une liaison à Lₙ, =O, F, CI, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸ , NO₂, -C(=O)NHOR¹⁸, -C(=O)NHNR¹⁸R^{18a}, -OCH₂CO₂H, le groupe 2-(1-morpholino)éthoxy, un groupe alkyle en C₁-C₅, un groupe alcényle en C₂-C₄, un groupe cycloalkyle en C₃-C₆, un groupe cycloalkylméthyle en C₃-C₆, un groupe alcoxyalkyle en C₂-C₆, un groupe aryle substitué par 0 à 2 R¹⁸, et un hétérocycle à 5-10 chaînons contenant 1 à 4 hétéroatomes indépendamment choisis parmi N, S et O ;
R¹⁸, R^{18a} et R¹⁹ sont indépendamment choisis à chaque occurrence dans le groupe :
une liaison à Lₙ, H, un groupe alkyle en C₁-C₆, un groupe phényle, un groupe benzyle, un groupe alcoxy en C₁-C₆, un halogénure, un groupe nitro, un groupe cyano, et un groupe trifluorométhyle ;
Pg est un groupe protecteur de thiol ;
R²⁰ et R²¹ sont indépendamment choisis dans le groupe :
H, un groupe alkyle en C₁-C₁₀, -CN, -CO₂R²⁵, -C(=O)R²⁵, -C(=O)N(R²⁵)₂, un 1-alcène en C₂-C₁₀, substitué par 0 à 3 R²³, un 1-alcyne en C₂-C₁₀ substitué par 0 à 3 R²³, un groupe aryle substitué par 0 à 3 R²³, un hétérocycle insaturé à 5-10 chaînons contenant 1 à 4 hétéroatomes indépendamment choisis parmi N, S, et O et substitué par 0 à 3 R²³, et un carbocycle insaturé en C₃-C₁₀ substitué par 0 à 3 R²³ ;
en variante, R²⁰ et R²¹ forment conjointement avec le radical carboné divalent auquel ils sont liés : 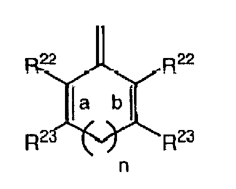
R²² et R²³ sont indépendamment choisis parmi le groupe :
H, R²⁴, un groupe alkyle en C₁-C₁₀ substitué par 0 à 3 R²⁴, un groupe alcényle en C₂-C₁₀ substitué par 0 à 3 R²⁴, un groupe alcynyle en C₂-C₁₀ substitué par 0 à 3 R²⁴, un groupe aryle substitué par 0 à 3 R²⁴, un hétérocycle à 5-10 chaînons contenant 1 à 4 hétéroatomes indépendamment choisis parmi N, S, et O et substitué par 0 à 3 R²⁴, et un carbocycle en C₃-C₁₀ substitué par 0 à 3 R²⁴ ;
en variante, R²² et R²³ forment ensemble un cycle aromatique ou un hétérocycle à 5-10 chaînons contenant 1 à 4 hétéroatomes indépendamment choisis parmi N, S, et O ;
a et b indiquent les positions de doubles liaisons éventuelles, et n vaut 0 ou 1 ;
R²⁴ est indépendamment choisi à chaque occurrence dans le groupe : =O, F, Cl, Br, l, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁶SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, et le groupe 2-(1-morpholino)éthoxy ; et,
R²⁵, R^{25a}, et R²⁶ sont chacun indépendamment choisis à chaque occurrence dans le groupe : un hydrogène et un groupe alkyle en C₁-C₆ ; ou un sel de ceux-ci acceptable sur le plan pharmaceutique.

18. Composition radiopharmaceutique thérapeutique selon la revendication 17, dans laquelle Q est un composé de formule (IV) : 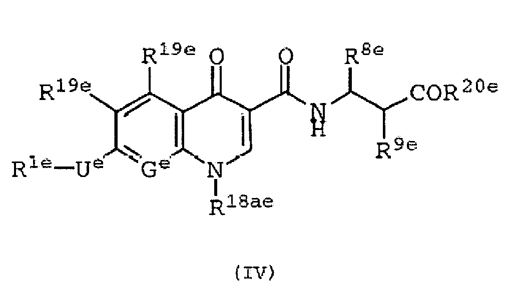 incluant les formes stéréoisomériques de celle-ci, ou les mélanges des formes stéréoisomériques de celle-ci, ou les formes de sel ou de promédicament de celle-ci acceptables sur le plan pharmaceutique, dans laquelle :
R^{1e} est choisi parmi : 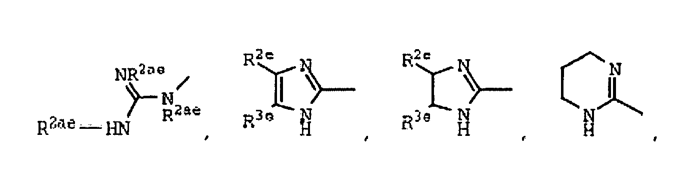 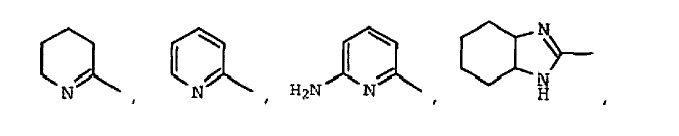 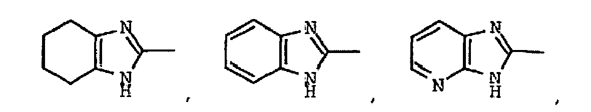 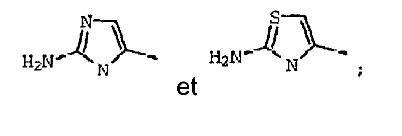
R^{2e} et R^{3e} sont indépendamment choisis parmi :
H, un groupe alcoxy en C₁-C₄, NR^{11e}R^{12e}, un halogène, NO₂, CN, CF₃, un groupe alkyle en C₁-C₆, un groupe alcényle en C₃-C₆, un groupe cycloalkyle en C₃-C₇, un groupe cycloalkyle(alkyle en C₁-C₄) en C₃-C₇, un groupe aryle(alkyle en C₁-C₆), un groupe (alkyle en C₁-C₆)carbonyle, un groupe (alcoxy en C₁-C₆)carbonyle, un groupe arylcarbonyle, et un groupe aryle substitué par 0 à 4 R^{7e},
en variante, lorsque R^{2e} et R^{3e} sont des substituants liés à des atomes adjacents, ils peuvent former conjointement avec les atomes de carbone auxquels ils sont liés un carbocycle à 5-7 chaînons ou un hétérocycle à 5-7 chaînons, aromatique ou non aromatique, ledit carbocycle ou hétérocycle étant substitué par 0 à 2 groupes choisis parmi un groupe alkyle en C₁-C₄, un groupe alcoxy en C₁-C₄, un groupe halogéno, un groupe cyano, un groupe amino, CF₃ et NO₂ ;
R^{2ae} est choisi parmi :
H, un groupe alkyle en C₁-C₁₀, un groupe alcényle en C₂-C₆, un groupe cycloalkyle en C₃-C₁₁, un groupe cycloalkyl(alkyle en C₁-C₄) en C₃-C₇, un groupe aryle, un groupe aryle(alkyle en C₁-C₄), un groupe (alkyle en C₂-C₇)carbonyle, un groupe arylcarbonyle, un groupe (alcoxy en C₂-C₁₀)carbonyle, un groupe cycloalcoxycarbonyle en C₃-C₇, un groupe bicycloalcoxycarbonyle en C₇-C₁₁, un groupe aryloxycarbonyle, un groupe aryl(alcoxy en C₁-C₁₀)carbonyle, un groupe alkylcarbonyloxy(alcoxy en C₁-C₄)carbonyle en C₁-C₆, un groupe arylcarbonyloxy(alcoxy en C₁-C₄)carbonyle, et un groupe cycloalkylcarbonyloxy(alcoxy en C₁-C₄)carbonyle en C₃-C₇ ;
R^{7e} est choisi parmi :
H, un groupe hydroxy, un groupe alkyle en C₁-C₄, un groupe alcoxy en C₁-C₄, un groupe aryle, un groupe aryle(alkyle en C₁-C₄), un groupe (alkyle en C₁-C₄)carbonyle, CO₂R^{18ae}, SO₂R^{11e}, SO₂NR^{10e}R^{11e}, OR^{10e}, et N(R^{11e})R^{12e} ;
U^{e} est choisi parmi :
-(CH₂)ₙe-, -(CH₂)ₙeO(CH₂)ₘe-, -NH(CH₂)ₙe-, -N(R^{10e})C(=O)-, -NHC(=O)(CH₂)ₙe-, et -C(=O)N(R^{10e})- ;
G^{e} représente N ou CR^{19e} ;
R^{8e} est choisi parmi :
H, CO₂R^{18be}, C(=O)R^{18be}, CONR^{17e}R^{18be}, un groupe alkyle en C₁-C₁₀ substitué par 0 à 1 R^{6e}, un groupe alcényle en C₂-C₁₀ substitué par 0 à 1 R^{6e}, un groupe alcynyle en C₂-C₁₀ substitué par 0 à 1 R^{6e}, un groupe cycloalkyle en C₃-C₈ substitué par 0 à 1 R^{6e}, un groupe cycloalcényle en C₅-C₆ substitué par 0 à 1 R^{6e}, un groupe (alkyle en C₁-C₁₀)carbonyle, un groupe cycloalkyle(alkyle en C₁-C₄) en C₃-C₁₀, un groupe phényle substitué par 0 à 3 R^{6e}, un groupe naphtyle substitué par 0 à 3 R^{6e}, un hétérocycle à 5-10 chaînons contenant 1 à 3 hétéroatomes N, O ou S, ledit hétérocycle pouvant être saturé, partiellement saturé, ou entièrement insaturé, et ledit hétérocycle étant substitué par 0 à 2 R^{7e} ;
R^{9e} est choisi parmi :
un groupe alkyle en C₁-C₁₀ substitué par 0 à 1 R^{6e}, un groupe alcoxy en C₁-C₁₀ substitué par 0 à 1 R^{6e}, H, un groupe nitro, -N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, un groupe hydroxy, OR^{22e}, -N(R^{10e})R^{11e}, -N(R^{16e})R^{17e}, un groupe aryl(alkyle en C₀-C₆)carbonyle, aryl(alkyle en C₁-C₆), hétéroaryl(alkyle en C₁-C₆), CONR^{18ae}R^{20e}, SO₂R^{18ae} et SO₂NR^{18ae}R^{20e}, sachant que chacun desdits groupes alkyles, cycloalkyles, aryles ou hétéroaryles peut être non substitué ou substitué de manière indépendante par 1 à 2 R^{7e} ;
R^{6e} est choisi parmi :
H, un groupe alkyle en C₁-C₁₀, un groupe hydroxy, un groupe alcoxy en C₁-C₁₀, un groupe nitro, un groupe alkylcarbonyle en C₁-C₁₀, -N(R^{11e})R^{12e}, un groupe cyano, un groupe halogéno, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚeR^{11e}, SO₂NR^{10e}R^{11e}, un groupe aryle substitué par 0 à 3 groupes choisis parmi un halogène, un groupe alcoxy en C₁-C₆, un groupe alkyle en C₁-C₆, CF₃, S(O)ₘeMe, et -NMe₂, un groupe aryle(alkyle en C₁-C₄), ledit groupe aryle étant substitué par 0 à 3 groupes choisis parmi un halogène, un groupe alcoxy en C₁-C₆, un groupe alkyle en C₁-C₆, CF₃, S(O)ₚeMe, et -NMe₂, et un hétérocycle à 5-10 chaînons contenant 1 à 3 hétéroatomes N, O, ou S, ledit hétérocycle pouvant être saturé, partiellement saturé ou entièrement insaturé, ledit hétérocycle étant substitué par 0 à 2 R^{7e},
R^{10e} est choisi parmi :
H, -OH, CF₃, un groupe alcényle en C₃-C₆, un groupe cycloalkyle en C₃-C₁₁, un groupe aryle, un groupe (cycloalkyle en C₃-C₁₁)méthyle, un groupe aryle(alkyle en C₁-C₄), et un groupe alkyle en C₁-C₁₀ substitué par 0 à 2 R^{6e} ;
R^{11e} est choisi parmi :
H, un groupe hydroxy, un groupe alkyle en C₁-C₈, un groupe alcényle en C₃-C₆, un groupe cycloalkyle en C₃-C₁₁, un groupe (cycloalkyle en C₃-C₁₁)méthyle, un groupe alcoxy en C₁-C₆, un groupe benzyloxy, un groupe aryle, un groupe hétéroaryle, un groupe hétéroaryle(alkyle en C₁-C₄), un groupe aryle(alkyle en C₁-C₄), un groupe adamantylméthyle, et un groupe alkyle en C₁-C₁₀ substitué par 0 à 2 R^{4e} ;
R^{4e} est choisi parmi :
H, un groupe alkyle en C₁-C₆, un groupe cycloalkyle en C₃-C₇, un groupe cycloalkyle(alkyle en C₁-C₄) en C₃-C₇, un groupe aryle, un groupe hétéroaryle, un groupe aryle(alkyle en C₁-C₆), et un groupe hétéroaryle(alkyle en C₁-C₆), lesdits groupes aryles ou hétéroaryles étant substitués par 0 à 2 substituants indépendamment choisis dans le groupe constitué par un groupe alkyle en C₁-C₄, un groupe alcoxy en C₁-C₄, F, Cl, Br, CF₃ et NO₂ ;
R^{12e} est choisi parmi :
H, un groupe alkyle en C₁-C₆, un groupe triphénylméthyle, un groupe méthoxyméthyle, un groupe méthoxyphényldiphénylméthyle, un groupe triméthylsilyléthoxyméthyle, un groupe (alkyle en C₁-C₆)carbonyle, un groupe (alcoxy en C₁-C₆)carbonyle, un groupe (alkyle en C₁-C₆)aminocarbonyle, un groupe alcényle en C₃-C₆, un groupe cycloalkyle en C₃-C₇, un groupe cycloalkyle(alkyle en C₁-C₄) en C₃-C₇, un groupe aryle, un groupe hétéroaryle(alkyle en C₁-C₆)carbonyle, un groupe hétéroarylcarbonyle, un groupe aryle(alkyle en C₁-C₆), un groupe (alkyle en C₁-C₆)carbonyle, un groupe arylcarbonyle, un groupe alkylsulfonyle en C₁-C₆, un groupe arylsulfonyle, un groupe aryle(alkyle en C₁-C₆)sulfonyle, un groupe hétéroarylsulfonyle, un groupe hétéroaryle(alkyle en C₁-C₆)sulfonyle, un groupe aryloxycarbonyle, et un groupe aryle(alcoxy en C₁-C₆)carbonyle, lesdits groupes aryles étant substitués par 0 à 2 substituants choisis dans le groupe constitué par un groupe alkyle en C₁-C₄, un groupe alcoxy en C₁-C₄, un groupe halogéno, CF₃, et un groupe nitro ;
R^{16e} est choisi parmi :
-C(=O)OR^{18ae}, -C(=O)R^{18be}, -C(=O)N(R^{18be})₂, -SO₂R^{18ae}, et -SO₂N(R^{18be})₂ ;
R^{17e} est choisi parmi :
H, un groupe alkyle en C₁-C₆, un groupe cycloalkyle en C₃-C₇, un groupe cycloalkyle(alkyle en C₁-C₄) en C₃-C₇, un groupe aryle, un groupe aryle(alkyle en C₁-C₆), et un groupe hétéroaryle(alkyle en C₁-C₆) ;
R^{18ae} est choisi parmi :
un groupe alkyle en C₁-C₈ éventuellement substitué par une liaison à Lₙ, un groupe cycloalkyle en C₃-C₁₁ éventuellement substitué par une liaison à Lₙ, un groupe aryle(alkyle en C₁-C₆) éventuellement substitué par une liaison à Lₙ, un groupe hétéroaryle(alkyle en C₁-C₆) éventuellement substitué par une liaison à Lₙ, un groupe (alkyle en C₁-C₆)hétéroaryle éventuellement substitué par une liaison à Lₙ, un groupe biaryle(alkyle en C₁-C₆) éventuellement substitué par une liaison à Lₙ, un groupe hétéroaryle éventuellement substitué par une liaison à Lₙ, un groupe phényle substitué par 3 à 4 R^{19e} et éventuellement substitué par une liaison à Lₙ, un groupe naphtyle substitué par 0 à 4 R^{19e} et éventuellement substitué par une liaison à Lₙ, et une liaison à Lₙ, lesdits groupes aryles ou hétéroaryles étant éventuellement substitués par 0 à 4 R^{19e} ;
R^{18be} représente H ou R^{18ae} ;
R^{19e} est choisi parmi :
H, un halogène, CF₃, CO₂H, CN, NO₂, -NR^{11e}R^{12e}, OCF₃, un groupe alkyle en C₁-C₈, un groupe alcényle en C₂-C₆, un groupe alcynyle en C₂-C₆, un groupe cycloalkyle en C₃-C₁₁, un groupe cycloalkyle(alkyle en C₁-C₄) en C₃-C₇, un groupe aryle(alkyle en C₁-C₆), un groupe alcoxy en C₁-C₆, un groupe alcoxycarbonyle en C₁-C₄, un groupe aryle, un groupe aryle-O-, un groupe aryle-SO₂-, un groupe hétéroaryle, et un groupe hétéroaryle-SO₂-, lesdits groupes aryles et hétéroaryles étant substitués par 0 à 4 groupes choisis parmi l'hydrogène, un halogène, CF₃, un groupe alkyle en C₁-C₃, et un groupe alcoxy en C₁-C₃ ;
R^{20e} est choisi parmi les groupes :
hydroxy, alkyloxy en C₁-C₁₀, cycloalkyloxy en C₃-C₁₁, aryloxy, aryl(alkyle en C₁-C₄)oxy, alkylcarbonyloxy(alkyle en C₁-C₂)oxy en C₂-C₁₀, alcoxycarbonyloxy(alkyle en C₁-C₂)oxy en C₂-C₁₀, alcoxycarbonyl(alkyle en C₁-C₂)oxy en C₂-C₁₀, cycloalkylcarbonyloxy(alkyle en C₁-C₂)oxy en C₃-C₁₀, cycloalcoxycarbonyloxy(alkyle en C₁-C₂)oxy en C₃-C₁₀, cycloalcoxycarbonyl(alkyle en C₁-C₂)oxy en C₃-C₁₀, aryloxycarbonyl(alkyle en C₁-C₂)oxy, aryloxycarbonyloxy(alkyle en C₁-C₂)oxy, arylcarbonyloxy(alkyle en C₁-C₂)oxy, alcoxy(alkyle en C₁-C₅)carbonyloxy(alkyle en C₁-C₂)oxy en C₁-C₅, (5-(alkyle en C₁-C₅)-1,3-dioxa-cyclopentèn-2-one-yl)méthyloxy, (5-aryl-1,3-dioxa-cyclopentèn-2-one-yl)méthyloxy, et (R^{10e})(R^{11e})N(alcoxy en C₁-C₁₀) ;
R^{21e} est choisi parmi les groupes :
alkyle en C₁-C₈, alcényle en C₂-C₆, cycloalkyle en C₃-C₁₁, (cycloalkyle en C₃-C₁₁)méthyle, aryle, aryle(alkyle en C₁-C₄), et alkyle en C₁-C₁₀ substitué par 0 à 2 R^{7e} ;
R^{22e} est choisi parmi :
-C(=O)-R^{18be}, -C(=O)N(R^{18be})₂, -C(=O)NHSO₂R^{18ae}, -C(=O)NHC(=O)R18^{be}, -C(=O)NHC(=O)OR^{18ae}, et -C(=O)NHSO₂NHR^{18be} ;
m^{e} est 0-2 ;
ne est 0-4 ; et
p^{e} est 0-2 ;
avec la clause suivante :
ne et m^{e} sont choisis de telle sorte que le nombre d'atomes reliant R¹ et -COR^{20e} dans la formule (IV) est dans la plage comprise entre 8 et 14 ;
d est choisi parmi 1, 2, 3, 4, et 5 ;
d'est 1-50 ;
W est indépendamment choisi à chaque occurrence dans le groupe : O, NH, NHC(=O), C(=O)NH, NR⁸C(=O), C(=O)NR⁸, C(=O), C(=O)O, OC(=O), NHC(=S)NH, NHC(=O)NH, SO₂, (OCH₂CH₂)ₛ, (CH₂CH₂O)_{s'}, (OCH₂CH₂CH₂)_{s"}, (CH₂CH₂CH₂O)ₜ, et (aa)_{t'} ;
aa est indépendamment à chaque occurrence un acide aminé ;
z est choisi dans le groupe :
groupe aryle substitué par 0 à 1 R¹⁰, groupe cycloalkyle en C₃-C₁₀ substitué par 0 à 1 R¹⁰, et hétérocycle à 5-10 chaînons contenant 1 à 4 hétéroatomes indépendamment choisis parmi N, S, et O et substitué par 0 à 1 R¹⁰ ;
R⁶, R^{6a}, R⁷, R^{7a}, et R⁸ sont indépendamment choisis à chaque occurrence dans le groupe :
H, =O, COOH, SO₃H, un groupe alkyle en C₁-C₅ substitué par 0 à 1 R¹⁰, un groupe aryle substitué par 0 à 1 R¹⁰, un groupe benzyle substitué par 0 à 1 R¹⁰, et un groupe alcoxy en C₁-C₅ substitué par 0 à 1 R¹⁰, NHC(=O)R¹¹, C(=O)NHR¹¹, NHC(=O)NHR¹¹, NHR¹¹, R¹¹, et une liaison à Cₕ ;
k est 0 ou 1 ;
s est choisi parmi 0, 1, 2, 3, 4, et 5 ;
s' est choisi parmi 0, 1, 2, 3, 4, et 5 ;
s" est choisi parmi 0, 1, 2, 3, 4, et 5 ;
t est choisi parmi 0, 1, 2, 3, 4, et 5 ;
A¹, A², A³, A⁴, A⁵, A⁶, A⁷ et A⁸ sont indépendamment choisis à chaque occurrence dans le groupe :
NR¹³, NR¹³R¹⁴, S, SH, S(Pg), OH, et une liaison à Lₙ ;
E est une liaison, CH, ou un groupe espaceur indépendamment choisi à chaque occurrence dans le groupe :
un groupe alkyle en C₁-C₁₀ substitué par 0 à 3 R¹⁷ un groupe aryle substitué par 0 à 3 R¹⁷, un groupe cycloalkyle en C₃-C₁₀ substitué par 0 à 3 R¹⁷ , et un hétérocycle à 5-10 chaînons contenant 1 à 4 hétéroatomes indépendamment choisis parmi N, S, et O et substitué par 0 à 3 R¹⁷ ;
R¹³ et R¹⁴ sont chacun indépendamment choisis dans le groupe :
une liaison à Lₙ, un hydrogène, un groupe alkyle en C₁-C₁₀ substitué par 0 à 3 R¹⁷, un groupe aryle substitué par 0 à 3 R¹⁷, un hétérocycle à 5-10 chaînons contenant 1 à 4 hétéroatomes indépendamment choisis parmi N, S et O et substitués par 0 à 3 R¹⁷, et un électron, sachant que lorsque l'un parmi R¹³ et R¹⁴ est un électron, alors l'autre est aussi un électron ;
en variante, R¹³ et R¹⁴ se combinent pour former =C(R²⁰)(R²¹) ;
R¹⁷ est indépendamment choisi à chaque occurrence dans le groupe :
une liaison à Lₙ, =O, F, Cl, Br, l, -CF₃, -CN, -CO₂R¹⁸, -C(=O)R¹⁸ -C(=O)N(R¹⁸)₂, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)₂, -N(R¹⁸)₂, -NHC(=S)NHR¹⁸, =NOR¹⁸, -C(=O)NHNR¹⁸R^{18a}, -OCH₂CO₂H, et le groupe 2-(1-morpholino)éthoxy ;
R¹⁸, R^{18a} et R¹⁹ sont indépendamment choisis à chaque occurrence dans le groupe :
une liaison à Lₙ, H, et un groupe alkyle en C₁-C₆ ;
R²⁰ et R²¹ sont indépendamment choisis dans le groupe :
H, un groupe alkyle en C₁-C₅, -CO₂R²⁵, un 1-alcène en C₂-C₅ substitué par 0 à 3 R²³, un 1-alcyne en C₂-C₅ substitué par 0 à 3 R²³, un groupe aryle substitué par 0 à 3 R²³, un hétérocycle insaturé à 5-10 chaînons contenant 1 à 4 hétéroatomes indépendamment choisis parmi N, S, et O et substitué par 0 à 3 R²³,
en variante, R²⁰ et R²¹ forment conjointement avec le radical carboné divalent auquel ils sont liés : 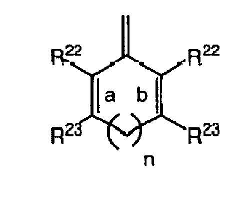
R²² et R²³ sont indépendamment choisis parmi le groupe : H, et R²⁴ ;
en variante, R²² et R²³ forment ensemble un cycle aromatique ou un hétérocycle à 5-10 chaînons contenant 1 à 4 hétéroatomes indépendamment choisis parmi N, S, et O ;
R²⁴ est indépendamment choisi à chaque occurrence dans le groupe : -CO₂R²⁵, -C(=O)N(R²⁵)₂, -CH₂OR²⁵, -OC(=O)R²⁵, -OR²⁵, -SO₃H, -N(R²⁵)₂, et -OCH₂CO₂H ; et,
R²⁵ est indépendamment choisi à chaque occurrence dans le groupe : un hydrogène et un groupe alkyle en C₁-C₃.

19. Composition radiopharmaceutique thérapeutique selon la revendication 17, dans laquelle :
R^{1e} est choisi parmi : 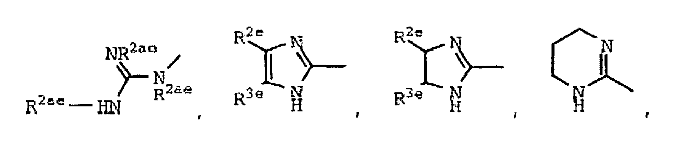 et 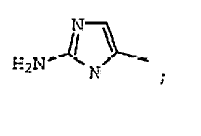
R^{2e} et R^{3e} sont indépendamment choisis parmi :
H, un groupe alcoxy en C₁-C₄, NR^{11e}R^{12e}, un halogène, NO₂, CN, CF₃, un groupe alkyle en C₁-C₆, un groupe alcényle en C₃-C₆, un groupe cycloalkyle en C₃-C₇, un groupe cycloalkyle(alkyle en C₁-C₄) en C₃-C₇, un groupe aryle(alkyle en C₁-C₆), un groupe (alkyle en C₁-C₆)carbonyle, un groupe (alcoxy en C₁-C₆)carbonyle, un groupe arylcarbonyle, et un groupe aryle substitué par 0 à 4 R^{7e}
en variante, lorsque R^{2e} et R^{3e} sont des substituants liés à des atomes adjacents, ils peuvent former conjointement avec les atomes de carbone auxquels ils sont liés un carbocycle à 5-7 chaînons ou un hétérocycle à 5-7 chaînons, aromatique ou non aromatique, ledit carbocycle ou hétérocycle étant substitué par 0 à 2 groupes choisis parmi un groupe alkyle en C₁-C₄, un groupe alcoxy en C₁-C₄, un groupe halogéno, un groupe cyano, un groupe amino, CF₃ et NO₂ ;
R^{2ae} est choisi parmi :
H, un groupe alkyle en C₁-C₁₀, un groupe alcényle en C₂-C₆, un groupe cycloalkyle en C₃-C₁₁, un groupe cycloalkyl(alkyle en C₁-C₄) en C₃-C₇, un groupe aryle, un groupe aryle(alkyle en C₁-C₄), un groupe (alkyle en C₂-C₇)carbonyle, un groupe arylcarbonyle, un groupe (alcoxy en C₂-C₁₀)carbonyle, un groupe cycloalcoxycarbonyle en C₃-C₇, un groupe bicycloalcoxycarbonyle en C₇C₁₁, un groupe aryloxycarbonyle, un groupe aryl(alcoxy en C₁-C₁₀)carbonyle, un groupe alkylcarbonyloxy(alcoxy en C₁-C₄)carbonyle en C₁-C₆, un groupe arylcarbonyloxy(alcoxy en C₁-C₄)carbonyle, et un groupe cycloalkylcarbonyloxy(alcoxy en C₁-C₄)carbonyle en C₃-C₇ ;
R^{7e} est choisi parmi :
H, un groupe hydroxy, un groupe alkyle en C₁-C₄, un groupe alcoxy en C₁-C₄, un groupe aryle, un groupe aryle(alkyle en C₁-C₄), un groupe (alkyle en C₁-C₄)carbonyle, CO₂R^{18ae}, SO₂R^{11e}, SO₂NR^{10e}R^{11e}, OR^{10e}, et N(R^{11e})R^{12e} ;
U^{e} est choisi parmi :
-(CH₂)ₙe-, -NH(CH₂)ₙe-, -N(R^{10e})C(=O)-, et -NHC(=O)(CH₂)ₙe;
G^{e} représente N ou CR^{19e} ;
R^{8e} représente H ;
R^{9e} est choisi parmi :
H, un groupe nitro, -N(R11^{e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, un groupe hydroxy, OR^{22e}, -N(R^{10e})R^{11e}, -N(R^{16e})R^{17e}, un groupe aryl(alkyle en C₀-C₄)carbonyle, aryl(alkyle en C₁-C₄), hétéroaryl(alkyle en C₁-C₄), CONR^{18ae}R^{20e}, SO₂R^{18ae}, et SO₂NR^{18ae}R^{20e}, sachant que chacun desdits groupes alkyles, cycloalkyles, aryles ou hétéroaryles peut être non substitué ou substitué de manière indépendante par 1 à 2 R^{7e} ;
R^{10e} est choisi parmi :
H, -OH, CF₃, un groupe alcényle en C₃-C₆, un groupe cycloalkyle en C₃-C₆, un groupe aryle, un groupe (cycloalkyle en C₃-C₆)méthyle, un groupe aryle(alkyle en C₁-C₄), et un groupe alkyle en C₁-C₄ substitué par 0 à 2 R^{6e} ;
R^{6e} est choisi parmi :
H, un groupe alkyle en C₁-C₄, un groupe hydroxy, un groupe alcoxy en C₁-C₄, un groupe nitro, un groupe alkylcarbonyle en C₁-C₄, -N(R^{11e})R^{12e}, un groupe cyano, un groupe halogéno, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e} , NR^{10e}SO₂NR^{10e}R^{11e} , NR^{10e}SO₂R^{21e} , S(O)ₚR^{11e} , SO₂NR^{10e}R^{11e}, un groupe aryle substitué par 0 à 3 groupes choisis parmi un halogène, un groupe alcoxy en C₁-C₄, un groupe alkyle en C₁-C₄, CF₃, S(O)ₘeMe, et -NMe₂, un groupe aryle(alkyle en C₁-C₄), ledit groupe aryle étant substitué par 0 à 3 groupes choisis parmi un halogène, un groupe alcoxy en C₁-C₄, un groupe alkyle en C₁-C₄, CF₃, S(O)ₚeMe, et -NMe₂, et un hétérocycle à 5-10 chaînons contenant 1 à 3 hétéroatomes N, O, ou S, ledit hétérocycle pouvant être saturé, partiellement saturé ou entièrement insaturé, ledit hétérocycle étant substitué par 0 à 2 R^{7e};
R^{11e} est choisi parmi :
H, un groupe hydroxy, un groupe alkyle en C₁-C₄, un groupe alcényle en C₃-C₆, un groupe cycloalkyle en C₃-C₆, un groupe (cycloalkyle en C₃-C₆)méthyle, un groupe alcoxy en C₁-C₄, un groupe benzyloxy, un groupe aryle, un groupe hétéroaryle, un groupe hétéroaryle(alkyle en C₁-C₄), un groupe aryle(alkyle en C₁-C₄), un groupe adamantylméthyle, et un groupe alkyle en C₁-C₄ substitué par 0 à 2 R^{4e} ;
R^{4e} est choisi parmi :
H, un groupe alkyle en C₁-C₄, un groupe cycloalkyle en C₃-C₇, un groupe cycloalkyle(alkyle en C₁-C₄) en C₃-C₇, un groupe aryle, un groupe hétéroaryle, un groupe aryle(alkyle en C₁-C₄), et un groupe hétéroaryle(alkyle en C₁-C₄), lesdits groupes aryles ou hétéroaryles étant substitués par 0 à 2 substituants indépendamment choisis dans le groupe constitué par un groupe alkyle en C₁-C₄, un groupe alcoxy en C₁-C₄, F, Cl, Br, CF₃ et NO₂,
R^{12e} est choisi parmi :
H, un groupe alkyle en C₁-C₄, un groupe (alkyle en C₁-C₄)carbonyle, un groupe (alcoxy en C₁-C₄)carbonyle, un groupe phényle(alkyle en C₁-C₄), un groupe phénylsulfonyle, un groupe phényloxycarbonyle, un groupe phényle(alcoxy en C₁-C₄)carbonyle, lesdits groupes phényles étant substitués par 0 à 2 substituants choisis dans le groupe constitué par un groupe alkyle en C₁-C₄, un groupe alcoxy en C₁-C₄, un groupe halogéno, CF₃, et un groupe nitro ;
R^{16e} est choisi parmi :
-C(=O)OR^{18e}, -C(=O)R^{18be}, -C(=O)N(R^{18be})₂, -SO₂R^{18ae}, et -SO₂N(R^{18be})₂ ;
R^{17e} est choisi parmi :
H, un groupe alkyle en C₁-C₄, un groupe cycloalkyle en C₃-C₆, un groupe cycloalkyle(alkyle en C₁-C₄) en C₃-C₆, un groupe aryle, un groupe aryle(alkyle en C₁-C₆), et un groupe hétéroaryle(alkyle en C₁-C₆) ;
R^{18ae} est choisi parmi :
un groupe alkyle en C₁-C₈ éventuellement substitué par une liaison à Lₙ, un groupe cycloalkyle en C₃-C₁₁ éventuellement substitué par une liaison à Lₙ, un groupe aryle(alkyle en C₁-C₆) éventuellement substitué par une liaison à Lₙ, un groupe hétéroaryle(alkyle en C₁-C₆) éventuellement substitué par une liaison à Lₙ, un groupe (alkyle en C₁-C₆)hétéroaryle éventuellement substitué par une liaison à Lₙ, un groupe biaryle(alkyle en C₁-C₆) éventuellement substitué par une liaison à Lₙ, un groupe hétéroaryle éventuellement substitué par une liaison à Lₙ, un groupe phényle substitué par 3 à 4 R^{19e} et éventuellement substitué par une liaison à Lₙ, un groupe naphtyle substitué par 0 à 4 R^{19e} et éventuellement substitué par une liaison à Lₙ, et une liaison à Lₙ, lesdits groupes aryles ou hétéroaryles étant éventuellement substitués par 0 à 4 R^{19e} ;
R^{18be} représente H ou R^{18ae} ;
R^{19e} est choisi parmi :
H, un halogène, CF₃, CO₂H, CN, NO₂, -NR^{11e}R^{12e}, OCF₃, un groupe alkyle en C₁-C₆, un groupe alcényle en C₂-C₆, un groupe alcynyle en C₂-C₆, un groupe cycloalkyle en C₃-C₆, un groupe cycloalkyle(alkyle en C₁-C₄) en C₃-C₆, un groupe aryle(alkyle en C₁-C₄), un groupe alcoxy en C₁-C₆, un groupe alcoxycarbonyle en C₁-C₄, un groupe aryle, un groupe aryle-O-, un groupe aryle-SO₂-, un groupe hétéroaryle, et un groupe hétéroaryle-SO₂-, lesdits groupes aryles et hétéroaryles étant substitués par 0 à 4 groupes choisis parmi l'hydrogène, un halogène, CF₃, un groupe alkyle en C₁-C₃, et un groupe alcoxy en C₁-C₃ ;
R^{20e} est choisi parmi les groupes :
hydroxy, alkyloxy en C₁-C₆, cycloalkyloxy en C₃-C₆, aryloxy, aryl(alkyle en C₁-C₄)oxy, alkylcarbonyloxy(alkyle en C₁-C₂)oxy en C₂-C₁₀, alcoxycarbonyloxy(alkyle en C₁-C₂)oxy en C₂-C₁₀, alcoxycarbonyl(alkyle en C₁-C₂)oxy en C₂-C₁₀, cycloalkylcarbonyloxy(alkyle en C₁-C₂)oxy en C₃-C₁₀, cycloalcoxycarbonyloxy(alkyle en C₁-C₂)oxy en C₃-C₁₀, cycloalcoxycarbonyl(alkyle en C₁-C₂)oxy en C₃-C₁₀, aryloxycarbonyl(alkyle en C₁-C₂)oxy, aryloxycarbonyloxy(alkyle en C₁-C₂)oxy, arylcarbonyloxy(alkyle en C₁-C₂)oxy, alcoxy(alkyle en C₁-C₅)carbonyloxy(alkyle en C₁-C₂)oxy en C₁-C₅, (5-(alkyle en C₁-C₅)-1,3-dioxa-cyclopentèn-2-one-yl)méthyloxy, (5-aryl-1,3-dioxa-cyclopentèn-2-one-yl)méthyloxy, et (R^{10e})(R^{11e})N(alcoxy en C₁-C₁₀) ;
R^{21e} est choisi parmi les groupes :
alkyle en C₁-C₄, alcényle en C₂-C₆, cycloalkyle en C₃-C₆, (cycloalkyle en C₃-C₆)méthyle, aryle, aryle(alkyle en C₁-C₄), et alkyle en C₁-C₁₀ substitué par 0 à 2 R^{7e} ;
R^{22e} est choisi parmi :
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, et -C(=O)NHSO₂NHR^{18be};
m^{e} est 0-2 ;
n^{e} est 0-4 ;
p^{e} est 0-2 ;
Cₕ est 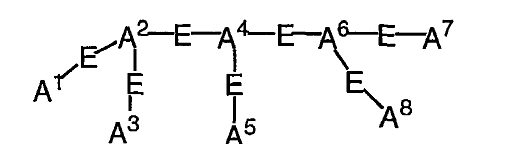
A¹ est choisi dans le groupe :
OH, et une liaison à Lₙ ;
A², A⁴ et A⁶ sont chacun N ;
A³, A⁵ et A⁸ sont chacun OH ;
A⁷ est une liaison à Lₙ ou une liaison NH à Lₙ ;
E est un groupe alkyle en C₂ substitué par 0 à 1 R¹⁷ ;
R¹⁷ représente =O ;
en variante, Cₕ représente 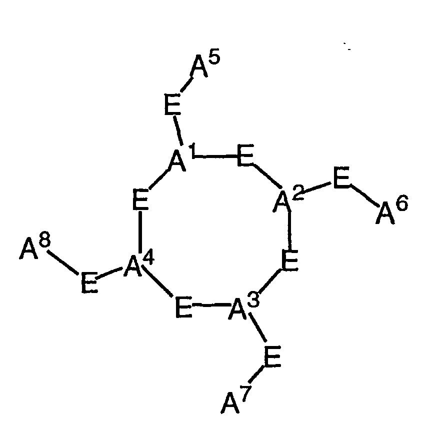
A¹ est choisi dans le groupe :
OH, et une liaison à Lₙ ;
A², A³ et A⁴ sont chacun N ;
A⁵, A⁶ et A⁸ sont chacun OH ;
A⁷ est une liaison à Lₙ ;
E est un alkyle en C₂ substitué par 0 à 1 R¹⁷ ;
R¹⁷ représente =O ;
en variante, Cₕ est 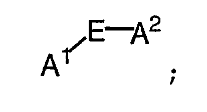
A¹ est NH₂ ou N=C(R²⁰)(R²¹) ;
E est une liaison ;
A² est NHR¹³ ;
R¹³ est un hétérocycle substitué par R¹⁷, ledit hétérocycle étant choisi parmi la pyridine et la pyrimidine ;
R¹⁷ est choisi parmi une liaison à Lₙ, C(=O)_{NHR}¹⁸ et C(=O)R¹⁸ ;
R¹⁸ est une liaison à Lₙ ;
R²⁴ est choisi dans le groupe :
-CO₂R²⁵, -OR²⁵, -SO₃H, et -N(R²⁵)₂ ; et,
R²⁵ est indépendamment choisi à chaque occurrence dans le groupe : hydrogène et groupe méthyle.

20. Composition radiopharmaceutique thérapeutique selon la revendication 17, dans laquelle Q est choisi dans le groupe :
acide 3-[7-[(imidazolin-2-ylamino)méthyl]-1-méthyl-6,8-difluoroquinoline-4-one-3-ylcarbonylamino]-2-(3,5-diméthylisoxazol-4-ylsulfonylamino)propionique,
acide 3-[7-[(imidazolin-2-ylamino)méthyl]-1-méthyl-6,8-difluoroquinoline-4-one-3-ylcarbonylamino]-2-(benzyloxycarbonylamino)propionique,
acide 3-[7-[(imidazolin-2-ylamino)méthyl]-1-méthyl-6,8-difluoroquinoline-4-one-3-ylcarbonylamino]-2-(n-butyloxycarbonylamino)propionique,
acide 3-[7-[(imidazolin-2-ylamino)méthyl]-1-méthyl-6,8-difluoroquinoline-4-one-3-ylcarbonylamino]-2-(n-butylsulfonylamino)propionique,
acide 3-[7-[(tétrahydropyrimid-2-ylamino)méthyl)-1-méthyl-6,8-difluoroquinoline-4-one-3-ylcarbonylamino]-2-(benzyloxycarbonylamino)propionique,
acide 3-[7-[(tétrahydropyrimid-2-ylamino)méthyl]-1-méthyl-6,8-difluoroquinoline-4-one-3-ylcarbonylamino]-2-(n-butyloxycarbonylamino)propionique,
acide 3-[7-[(tétrahydropyrimid-2-ylamino)méthyl]-1-méthyl-6,8-difluoroquinoline-4-one-3-ylcarbonylamino]-2-(phénylsulfonylamino)propionique,
acide 3-[7-[(tétrahydropyrimid-2-ylamino)méthyl]-1-méthyl-6,8-difluoroquinoline-4-one-3-ylcarbonylamino]-2-(n-butylsulfonyl)aminopropionique,
acide 3-[7-[(2-aminothiazol-4-yl)méthyl]-1-méthyl-6,8-difluoroquinoline-4-one-3-ylcarbonylamino]-2-(benzyloxycarbonylamino)propionique,
acide 3-[7-[(imidazolin-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(tétrahydropyrimid-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-méthyl-6,8-difluoroquinoline-4-one-3-ylcarbonylamino]-2-(3,5-diméthylisoxazol-4-ylsulfonylamino)propionique,
acide 3-[7-[(imidazol-2-ylamino)méthyl]-1-méthyl-6,8-difluoroquinoline-4-one-3-ylcarbonylamino]-2-(benzyloxycarbonylamino)propionique,
acide 3-[7-[(imidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-méthyl-6,8-difluoroquinoline-4-one-3-ylcarbonylamino]-2-((4-biphényl)sulfonylamino)propionique,
acide 3-[7-[(imidazol-2-ylamino)méthyl]-1-méthyl-6,8-difluoroquinoline-4-one-3-ylcarbonylamino]-2-(1-naphtylsulfonylamino)propionique,
acide 3-[7-[(benzimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(4-méthylimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(4,5-diméthylimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(4,5,6,7-tétrahydrobenzimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(pyridin-2-ylamino)méthyl]-1-méthyl-6,8-difluoroquinoline-4-one-3-ylcarbonylamino]-2-((2,4,6-triméthylphényl)sulfonylamino)propionique,
acide 3-[7- (2-aminopyridin-6-yl)-1-méthyl-6,8-difluoroquinoline-4-one-3-ylcarbonylamino]-2-((2,4,6-triméthylphényl)sulfonylamino)propionique,
acide 3-[7-[(7-azabenzimidazol-2-yl)méthyl]-1-méthyl-6,8-difluoroquinoline-4-one-3-ylcarbonylamino]-2-((2,4,6-triméthylphényl)sulfonylamino)propionique,
acide 3-[7-[(benzimidazol-2-ylamino)méthyl]-1-(2-phényléthyl)-6,8-difluoroquinoline-4-one-3-ylcarbonylamino]propionique,
acide 3-[7-[(pyridin-2-ylamino)méthyl]-1-(2-phényléthyl)- 6,8-difluoroquinoline-4-one-3-ylcarbonylamino]propionique,
acide 3-[7-[(imidazolin-2-ylamino)méthyl]-1-(2-phényléthyl)-6,8-difluoroquinoline-4-one-3-ylcarbonylamino]propionique,
acide 3-[7-[(imidazol-2-ylamino)méthyl]-1-(2-phényléthyl)-6,8-difluoroquinoline-4-one-3-ylcarbonylamino]propionique,
acide 3-[7-[(imidazolin-2-ylamino)méthyl]-1-(2-phényléthyl)-6,8-difluoroquinoline-4-one-3-ylcarbonylamino]-2-(benzyloxycarbonylamino)propionique,
acide 3-[7-[(imidazolin-2-ylamino)méthyl]-1-(2-phényléthyl)-6,8-difluoroquinoline-4-one-3-ylcarbonylamino]-2-(n-butyloxycarbonylamino)propionique,
acide 3-[7-[(imidazolin-2-ylamino)méthyl]-1-(2-phényléthyl)-6,8-difluoroquinoline-4-one-3-ylcarbonylamino]-2-(phénylsulfonylamino)propionique,
acide 3-[7-[(imidazolin-2-ylamino)méthyl]-1-(2-phényléthyl)-6,8-difluoroquinoline-4-one-3-ylcarbonylamino]-2-(n-butylsulfonylamino)propionique,
acide 3-[7-[(tétrahydropyrimid-2-ylamino)méthyl]-1-(2-phényléthyl)-6,8-difluoroquinoline-4-one-3-ylcarbonylamino]-2-(benzyloxycarbonylamino)propionique,
acide 3-[7-[(tétrahydropyrimid-2-ylamino)méthyl]-1-(2-phényléthyl))-6,8-difluoroquinoline-4-one-3-ylcarbonylamino]-2-(n-butyloxycarbonylamino)propionique,
acide 3-[7-[(tétrahydropyrimid-2-ylamino)méthyl]-1-(2-phényléthyl)-6,8-difluoroquinoline-4-one-3-ylcarbonylamino]-2-(phénylsulfonylamino)propionique,
acide 3-[7-[(tétrahydropyrimid-2-ylamino)méthyl]-1-(2-phényléthyl)-6,8-difluoroquinoline-4-one-3-ylcarbonylamino]-2-(n-butylsulfonyl)aminopropionique,
acide 3-[7-[(2-aminothiazol-4-yl)méthyl]-1-(2-phényléthyl)-6,8-difluoroquinoline-4-one-3-ylcarbonylamino]-2-(phénylsulfonylamino)propionique,
acide 3-[7-[(2-aminothiazol-4-yl)méthyl]-1-(2-phényléthyl)-6,8-difluoroquinoline-4-one-3-ylcarbonylamino]-2-(benzyloxycarbonylamino)propionique,
acide 3-[7-[(imidazolin-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(tétrahydropyrimid-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-(2-phényléthyl)-6,8-difluoroquinoline-4-one-3-ylcarbonylamino]-2-(benzyloxycarbonylamino)propionique,
acide 3-[7-[(imidazol-2-ylamino)méthyl]-I-(2-phényléthyl)-6,8-difluoroquinoline-4-one-3-ylcarbonylamino]-2-(phénylsulfonylamino)propionique,
acide 3-[7-[(imidazol-2-ylamino)méthyl]-1-(2-phényléthyl)-6,8-difluoroquinoline-4-one-3-ylcarbonylamino]-2-((2,6-dichlorophényl)sulfonylamino)propionique,
acide 3-[7-[(imidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-(2-phényléthyl)-6,8-difluoroquinoline-4-one-3-ylcarbonylamino]-2-((4-biphényl)sulfonylamino)propionique,
acide 3-[7-[(benzimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(4-méthylimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(4,5-diméthylimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(4,5,6,7-tétrahydrobenzimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(pyridin-2-ylamino)méthyl]-1-(2-phényléthyl)- 6,8-difluoroquinoline-4-one-3-ylcarbonylamino]-2-((2,4,6-triméthylphényl)sulfonylamino)propionique, acide 3-[7-(2-aminopyridin-6-yl)-1-(2-phényléthyl)-6,8-difluoroquinoline-4-one-3-ylcarbonylamino]-2-((2,4,6-triméthylphényl)sulfonylamino)propionique, et
acide 3-[7-(7-azabenzimidazol-2-yl)méthyl]-1-(2-phényléthyl)-6,8-difluoroquinoline-4-one-3-ylcarbonylamino]-2-((2,4,6-triméthylphényl)sulfonylamino)propionique.

21. Composition selon la revendication 15, dans laquelle le radioisotope est ¹⁵³Sm.

22. Composition selon la revendication 15, dans laquelle le radioisotope est ¹⁷⁷Lu.

23. Composition selon la revendication 22, dans laquelle l'agent radiopharmaceutique est choisi dans le groupe : 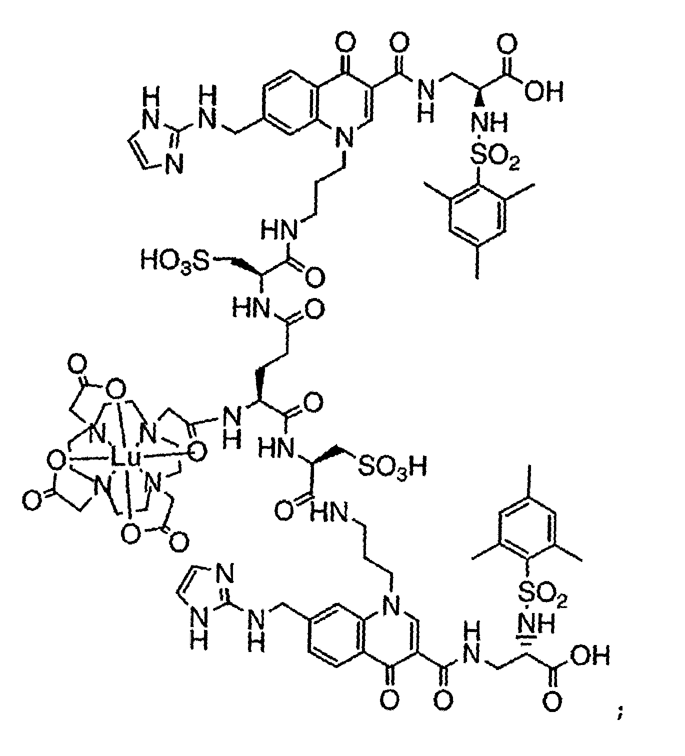 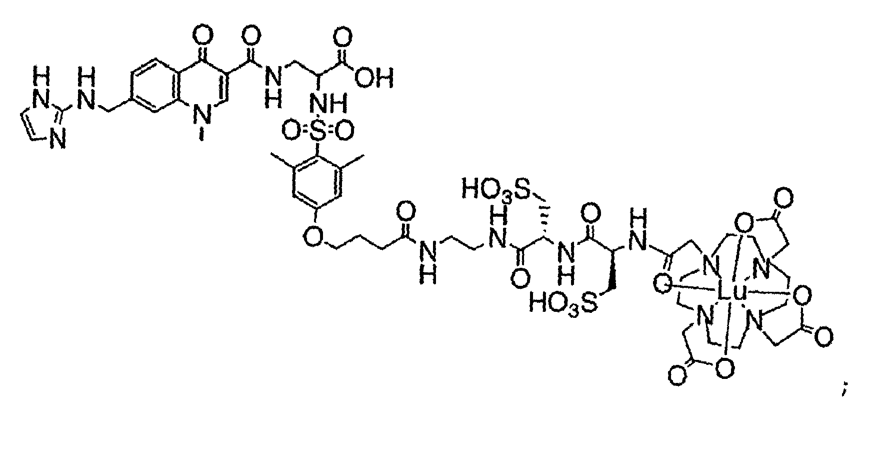 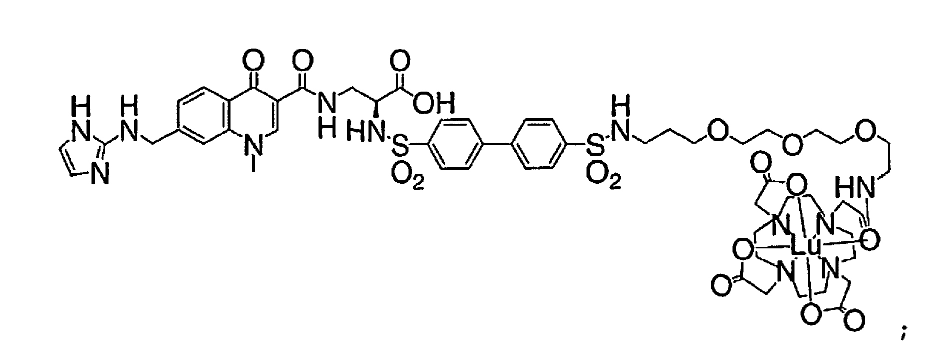 et

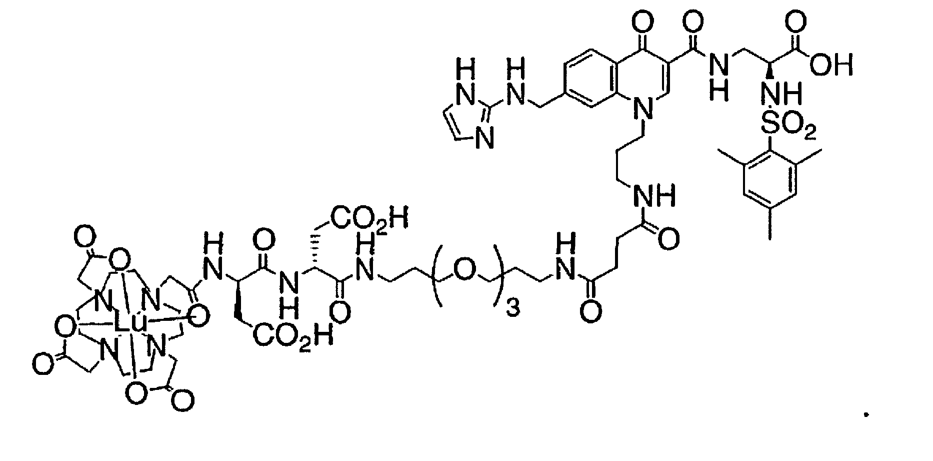

24. Composition selon la revendication 15, dans laquelle le radioisotope est ⁹⁰Y.

25. Composition selon la revendication 24, dans laquelle l'agent radiopharmaceutique est choisi dans le groupe : 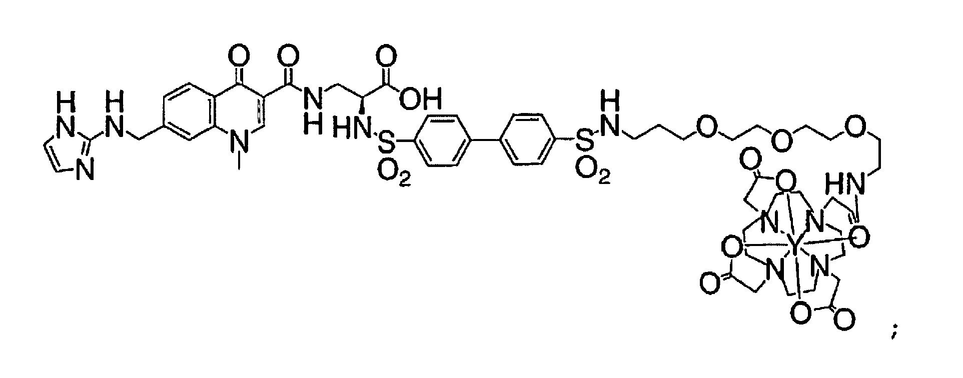 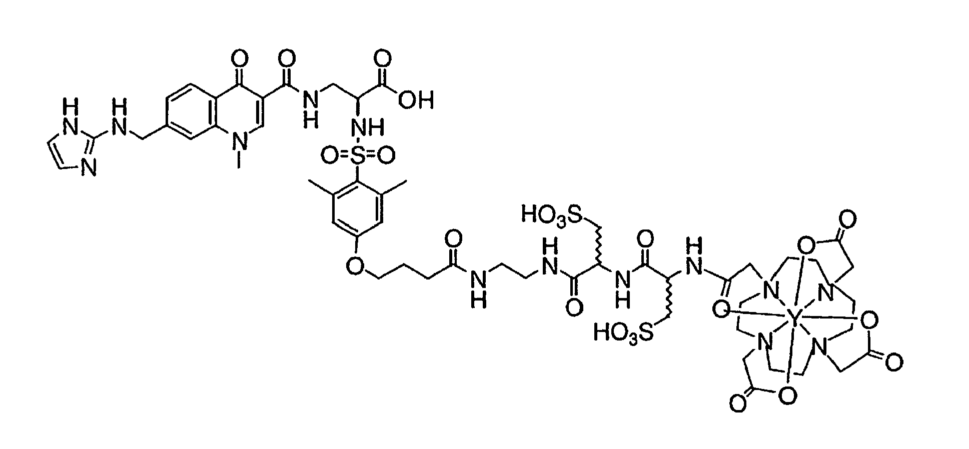 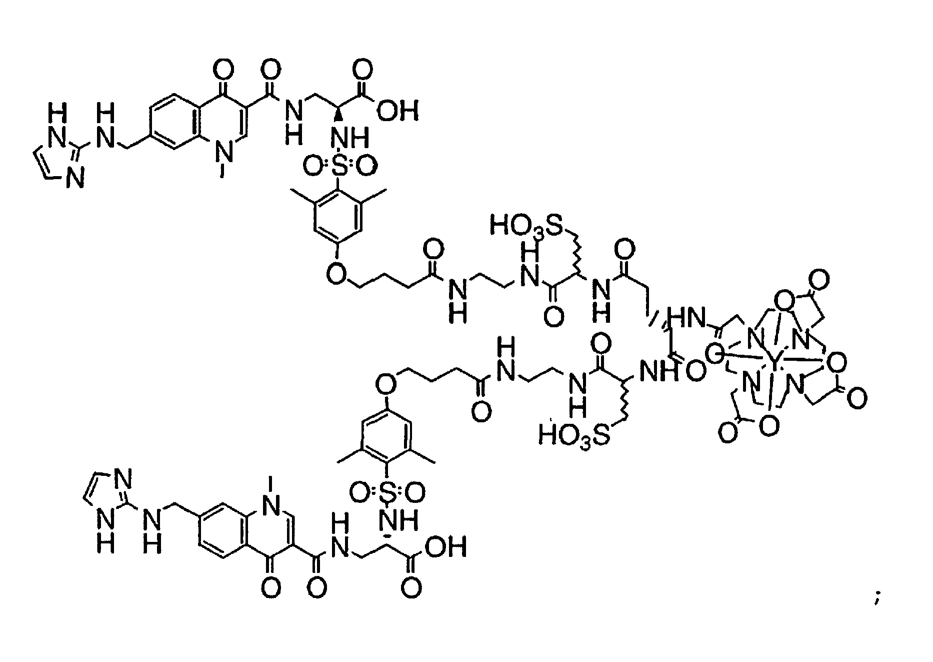 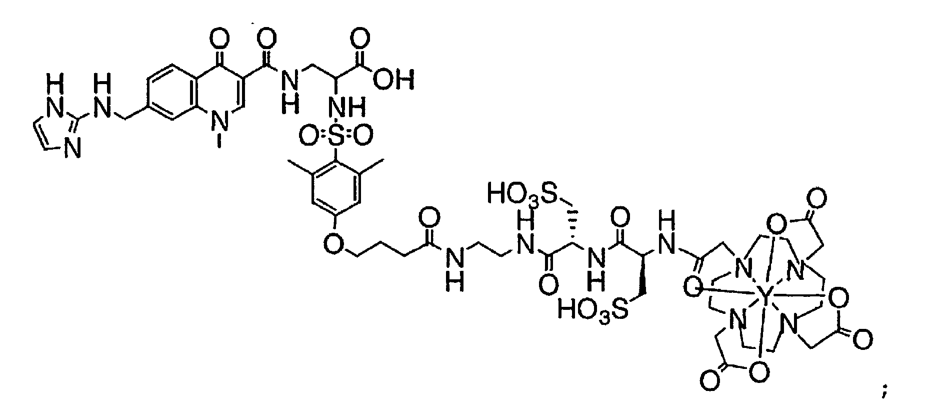 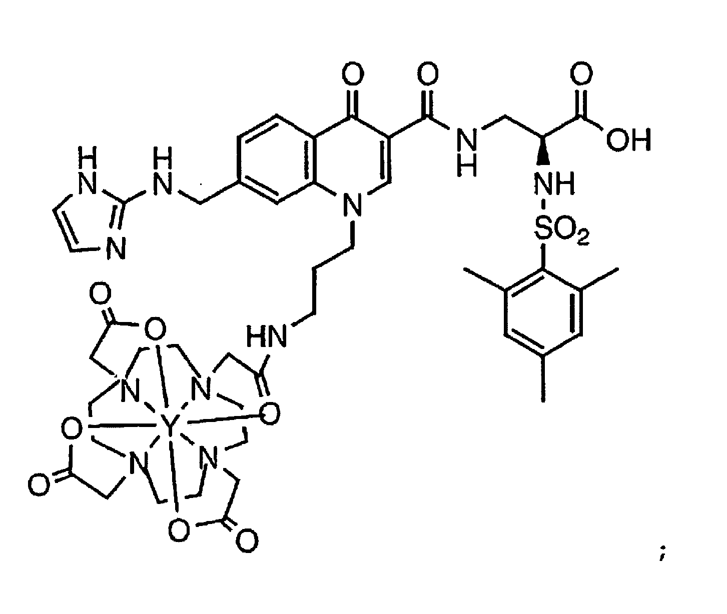 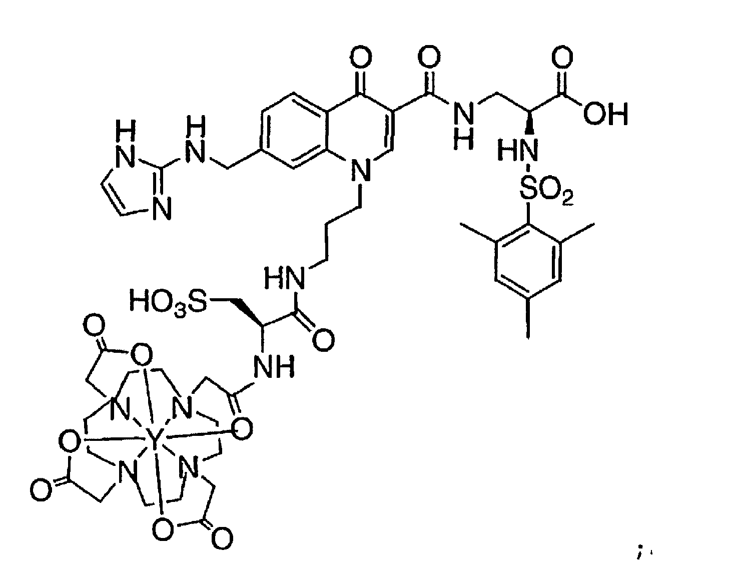 et

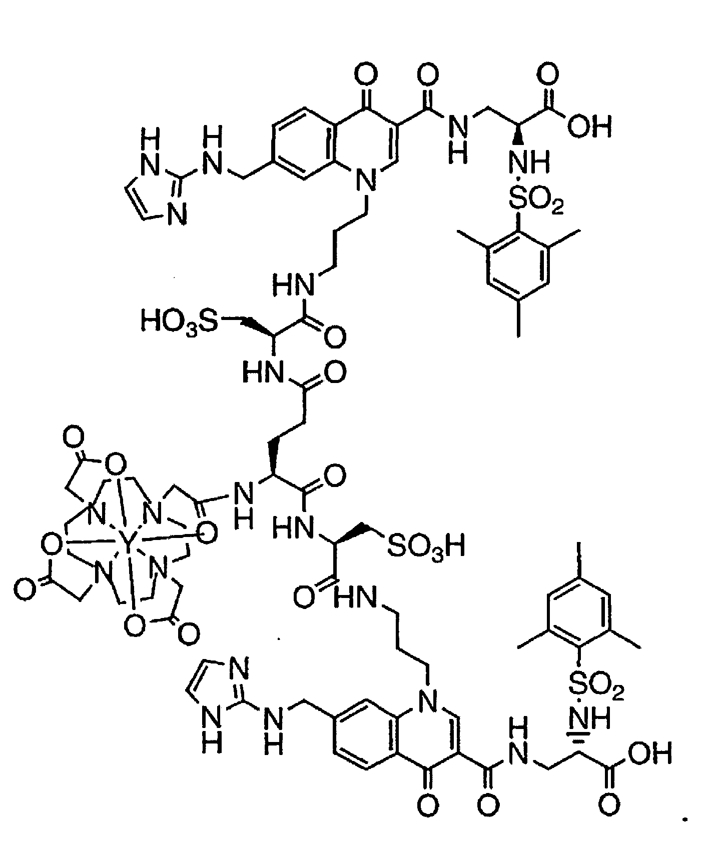

26. Utilisation d'une composition radiopharmaceutique thérapeutique comprenant :
a) un métal ;
b) un chélatant capable de chélater le métal ; et
c) un motif de ciblage ;
où le motif de ciblage est lié au chélatant par un groupe de liaison, et le motif de ciblage est une quinolone non-peptidique qui se lie à un récepteur qui est régulé en amont pendant l'angiogénèse, et le métal est un radioisotope choisi dans le groupe : ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, et ¹⁹²Ir ; ou un sel de celui-ci acceptable sur le plan pharmaceutique, et au moins un agent choisi dans le groupe constitué par un agent anticancéreux et un agent de radiosensibilisation, ou un sel de ceux-ci acceptable sur le plan pharmaceutique, pour la fabrication d'un médicament à administrer à un patient le nécessitant.

27. Utilisation selon la revendication 26, dans laquelle l'administration du radiopharmaceutique thérapeutique et celle de l'agent sont simultanées.

28. Utilisation selon la revendication 26, dans laquelle l'administration du radiopharmaceutique thérapeutique et celle de l'agent sont consécutives.

29. Utilisation selon la revendication 26, dans laquelle le cancer est choisi parmi le groupe constitué par les carcinomes du poumon, du sein, de l'ovaire, de l'estomac, du pancréas, du larynx, de l'oesophage, des testicules, du foie, de la glande parotide, des voies biliaires, du colon, du rectum, du col de l'utérus, de l'utérus, de l'endomètre, du rein, de la vessie, de la prostate, de la thyroïde, les carcinomes de l'épithélium pavimenteux, les adénocarcinomes, les carcinomes à petites cellules, les mélanomes, les gliomes et les neuroblastomes.

30. Utilisation selon la revendication 26, dans laquelle l'agent anticancéreux est choisi dans le groupe constitué par la mitomycine, la trétinoïne, la ribomustine, la gemcitabine, la vincristine, l'étoposide, la cladribine, le mitobronitol, le méthotrexate, la doxorubicine, la carboquone, la pentostatine, la nitracrine, la zinostatine, le cétrorélix, le létrozole, le raltitrexed, la daunorubicine, la fadrozole, la fotémustine, la thymalfasine, le sobuzoxane, le nédaplatine, la cytarabine, la bicalutamide, la vinorelbine, la vesnarinone, l'aminoglutéthimide, l'amsacrine, le proglumide, l'acétate d'elliptinium, la kétansérine, la doxifluridine, l'étrétinate, l'isotrétinoïne, la streptozocine, la nimustine, la vindésine, la flutamide, le drogénil, la butocine, le carmofur, le razoxane, la sizofilane, le carboplatine, le mitolactol, le tegafur, l'ifosfamide, la prednimustine, le picibanil, le lévamisole, le téniposide, l'improsulfane, l'énocitabine, le lisuride, l'oxymétholone, le tamoxifène, la progestérone, le mépitiostane, l'épitiostanol, le formestane, l'interféron-alpha, l'interféron-2 alpha, l'interféron-beta, l'interféron-gamma, le facteur 1 de stimulation de colonies, le facteur 2 de stimulation de colonies, le dénileukine diftitox, l'interleukine 2, et le facteur de libération de l'hormone lutéinisante.

31. Utilisation selon la revendication 26, dans laquelle l'agent de radiosensibilisation est choisi dans le groupe constitué par le 2-(3-nitro-1,2,4-triazol-1-yl)-N-(2-méthoxyéthyl)acétamide, la N-(3-nitro-4-quinolinyl)-4-morpholinecarboxamidine, le 3-amino-1,2,4-benzotriazine-1,4-dioxide, le N-(2-hydroxyéthyl)-2-nitroimidazole-1-acétamide, le 1-(2-nitroimidazol-1-yl)-3-(1-pipéridinyl)-2-propanol, et le 1-(2-nitro-1-imidazolyl)-3-(1-aziridino)-2-propanol.

32. Utilisation selon la revendication 26, dans laquelle l'agent anticancéreux est un agent chimiothérapeutique.

33. Utilisation selon la revendication 26, dans laquelle l'administration est effectuée par injection ou perfusion.

34. Utilisation selon la revendication 26, dans laquelle le radiopharmaceutique thérapeutique comprend :
a) un métal ;
b) un chélatant capable de chélater le métal ; et
c) un motif de ciblage ;
où le motif de ciblage est lié au chélatant par un groupe de liaison, et le motif de ciblage est une quinolone non-peptidique qui se lie à un récepteur qui est régulé en amont pendant l'angiogénèse, et le métal est un radioisotope choisi dans le groupe : ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹¹¹Ag, et ¹⁹²Ir.

35. Utilisation selon la revendication 34, dans laquelle le motif de ciblage est une quinolone non-peptidique et le récepteur est αᵥβ₃ ou αᵥβ₅.

36. Utilisation selon la revendication 34, dans laquelle le radiopharmaceutique thérapeutique comprend :
a) un radioisotope choisi dans le groupe : ³³P, ¹²⁵I, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, et ¹⁹²Ir ; et
b) un composé de formule (I) :
(Q)_{d}-Lₙ-Cₕ ou (Q)_{d}-Lₙ-(Cₕ)_{d'}
dans laquelle Q est un composé de formule (II) : 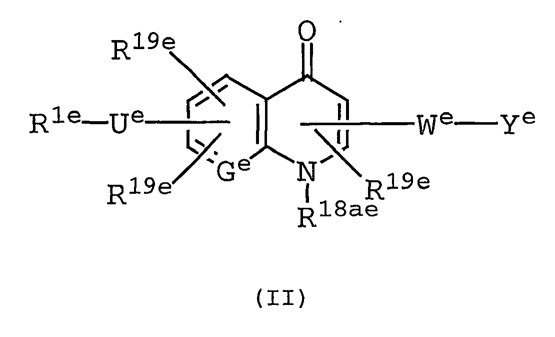 incluant les formes stéréoisomériques de celle-ci, ou les mélanges des formes stéréoisomériques de celle-ci, ou les formes de sel ou de promédicament de celle-ci acceptables sur le plan pharmaceutique, dans laquelle :
R^{1e} est choisi parmi : 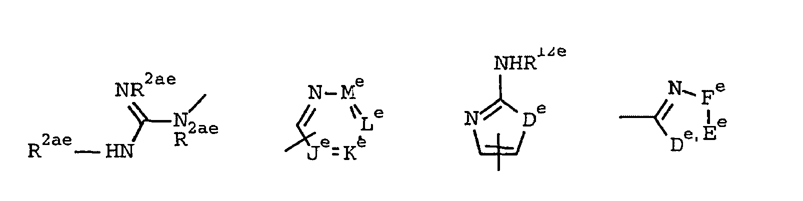 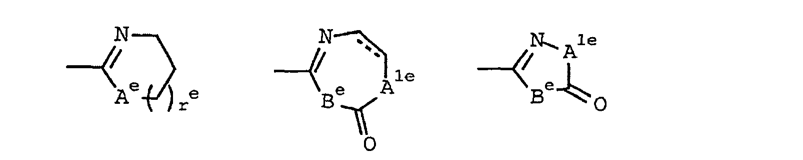 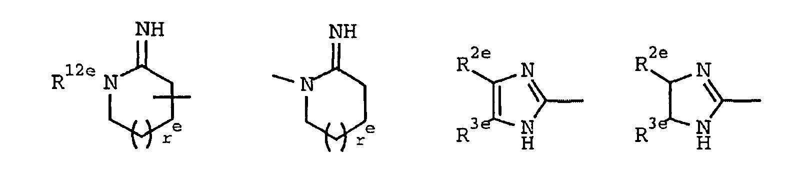 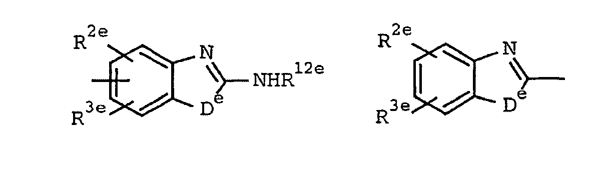 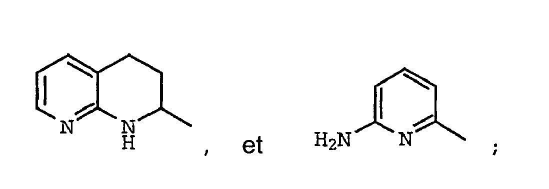
A^{e} représente -CH₂- ou -N(R^{10e})- ;
A^{1e} et B^{e} représentent indépendamment -CH₂- ou -N(R^{10e})- ;
D^{e} représente -N(R^{10e})- ou -S- ;
E^{e}-F^{e} représente -C(R^{2e})=C(R^{3e})- ou -C(R^{2e})₂C(R^{3e})₂- ;
J^{e} représente -C(R^{2e})- ou -N- ;
K^{e}, L^{e} et M^{e} représentent indépendamment -C(R^{2e})- ou -C(R^{3e})- ;
R^{2e} et R^{3e} sont indépendamment choisis parmi :
H, un groupe alcoxy en C₁-C₄, NR^{11e}R^{12e}, un halogène, NO₂, CN, CF₃, un groupe alkyle en C₁-C₆, un groupe alcényle en C₃-C₆, un groupe cycloalkyle en C₃-C₇, un groupe cycloalkyle(alkyle en C₁-C₄) en C₃-C₇, un groupe aryle(alkyle en C₁-C₆), un groupe (alkyle en C₁-C₆)carbonyle, un groupe (alcoxy en C₁-C₆)carbonyle, un groupe arylcarbonyle, et un groupe aryle substitué par 0 à 4 R^{7e},
en variante, lorsque R^{2e} et R^{3e} sont des substituants liés à des atomes adjacents, ils peuvent former conjointement avec les atomes de carbone auxquels ils sont liés un carbocycle à 5-7 chaînons ou un hétérocycle à 5-7 chaînons, aromatique ou non aromatique, ledit carbocycle ou hétérocycle étant substitué par 0 à 2 groupes choisis parmi un groupe alkyle en C₁-C₄, un groupe alcoxy en C₁-C₄, un groupe halogéno, un groupe cyano, un groupe amino, CF₃ et NO₂ ;
R^{2ae} est choisi parmi :
H, un groupe alkyle en C₁-C₁₀, un groupe alcényle en C₂-C₆, un groupe cycloalkyle en C₃-C₁₁, un groupe cycloalkyl(alkyle en C₁-C₄) en C₃-C₇, un groupe aryle, un groupe aryle(alkyle en C₁-C₄), un groupe (alkyle en C₂-C₇)carbonyle, un groupe arylcarbonyle, un groupe (alcoxy en C₂-C₁₀)carbonyle, un groupe cycloalcoxycarbonyle en C₃-C₇, un groupe bicycloalcoxycarbonyle en C₇-C₁₁, un groupe aryloxycarbonyle, un groupe aryl(alcoxy en C₁-C₁₀)carbonyle, un groupe alkylcarbonyloxy(alcoxy en C₁-C₄)carbonyle en C₁-C₆, un groupe arylcarbonyloxy(alcoxy en C₁-C₄)carbonyle, et un groupe cycloalkylcarbonyloxy(alcoxy en C₁-C₄)carbonyle en C₃-C₇ ;
R^{7e} est choisi parmi :
H, un groupe hydroxy, un groupe alkyle en C₁-C₄, un groupe alcoxy en C₁-C₄, un groupe aryle, un groupe aryle(alkyle en C₁-C₄), un groupe (alkyle en C₁-C₄)carbonyle, CO₂R^{18ae}, SO₂R^{11e}, SO₂NR^{10e}R^{11e}, OR^{10e}, et N(R^{11e})R^{12e} ;
U^{e} est choisi parmi :
-(CH₂)ₙe-, -(CH₂)ₙeO(CH₂)ₘe-, -(CH₂)ₙeN(R¹²)(CH₂)ₘe-, -NH(CH₂)ₙe-, -(CH₂)ₙeC(=O)(CH₂)ₘe-, -(CH₂)ₙeS(O)ₚe(CH₂)ₘe-, -(CH₂)ₙeNHNH(CH₂)ₘe-, -N(R^{10e})C(=O)-, -NHC(=O)(CH₂)ₙe-, -C(=O)N(R^{10e})-, et -N(R^{10e})S(O)ₚe-;
G^{e} représente N ou CR^{19e} ;
W^{e} représente -C(=O)-N(R^{10e})-(alkylène en C₁-C₃)-, où le groupe alkylène est substitué par R^{8e} et par R^{9e} :
R^{8e} et R^{9e} sont indépendamment choisis parmi :
H, CO₂R^{18be}, C(=O)R^{18be}, CONR¹⁷R^{18be}, un groupe alkyle en C₁-C₁₀ substitué par 0 à 1 R^{6e}, un groupe alcényle en C₂-C₁₀ substitué par 0 à 1 R^{6e}, un groupe alcynyle en C₂-C₁₀ substitué par 0 à 1 R^{6e}, un groupe cycloalkyle en C₃-C₈ substitué par 0 à 1 R^{6e}, un groupe cycloalcényle en C₅-C₆ substitué par 0 à 1 R^{6e}, un groupe (alkyle en C₁-C₁₀)carbonyle, un groupe cycloalkyle(alkyle en C₁-C₄) en C₃-C₁₀, un groupe phényle substitué par 0 à 3 R^{6e}, un groupe naphtyle substitué par 0 à 3 R^{6e}, un hétérocycle à 5-10 chaînons contenant 1 à 3 hétéroatomes N, O ou S, ledit hétérocycle pouvant être saturé, partiellement saturé, ou entièrement insaturé, et ledit hétérocycle étant substitué par 0 à 2 R^{7e}, un groupe alcoxy en C₁-C₁₀ substitué par 0 à 2 R^{7e}, un groupe hydroxy, un groupe nitro, -N(R^{10e})R^{11e}, -N(R^{16e})R^{17e}, un groupe aryl(alkyle en C₀-C₆)carbonyle, aryl(alkyle en C₃-C₆), hétéroaryl(alkyle en C₁-C₆), CONR^{18ae}R^{20e}, SO₂R^{18ae}, et SO₂NR^{18ae}R^{2e},
sachant que chacun desdits groupes alkyles, cycloalkyles, aryles ou hétéroaryles peut être non substitué ou substitué de manière indépendante par 1 à 2 R^{7e} ;
R^{6e} est choisi parmi :
H, un groupe alkyle en C₁-C₁₀, un groupe hydroxy, un groupe alcoxy en C₁-C₁₀, un groupe nitro, un groupe alkylcarbonyle en C₁-C₁₀, -N(R^{11e})R^{12e}, un groupe cyano, un groupe halogéno, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e}, un groupe aryle substitué par 0 à 3 groupes choisis parmi un halogène, un groupe alcoxy en C₁-C₆, un groupe alkyle en C₁-C₆, CF₃, S(O)ₘeMe, et -NMe₂, un groupe aryle(alkyle en C₁-C₄), ledit groupe aryle étant substitué par 0 à 3 groupes choisis parmi un halogène, un groupe alcoxy en C₁-C₆, un groupe alkyle en C₁-C₆, CF₃, S(O)ₚeMe, et -NMe₂, et un hétérocycle à 5-10 chaînons contenant 1 à 3 hétéroatomes N, O, ou S, ledit hétérocycle pouvant être saturé, partiellement saturé ou entièrement insaturé, ledit hétérocycle étant substitué par 0 à 2 R^{7e} ;
R^{10e} est choisi parmi :
H, -OH, CF₃, un groupe alcényle en C₃-C₆, un groupe cycloalkyle en C₃-C₁₁, un groupe aryle, un groupe (cycloalkyle en C₃-C₁₁)méthyle, un groupe aryle(alkyle en C₁-C₄), et un groupe alkyle en C₁-C₁₀ substitué par 0 à 2 R^{6e} ;
R^{11e} est choisi parmi :
H, un groupe hydroxy, un groupe alkyle en C₁-C₈, un groupe alcényle en C₃-C₆, un groupe cycloalkyle en C₃-C₁₁, un groupe (cycloalkyle en C₃-C₁₁)méthyle, un groupe alcoxy en C₁-C₆, un groupe benzyloxy, un groupe aryle, un groupe hétéroaryle, un groupe hétéroaryle(alkyle en C₁-C₄), un groupe aryle(alkyle en C₁-C₄), un groupe adamantylméthyle, et un groupe alkyle en C₁-C₁₀ substitué par 0 à 2 R^{4e} ;
R^{4e} est choisi parmi :
H, un groupe alkyle en C₁-C₆, un groupe cycloalkyle en C₃-C₇, un groupe cycloalkyle(alkyle en C₁-C₄) en C₃-C₇, un groupe (alkyle en C₁-C₁₀)carbonyle, un groupe aryle, un groupe hétéroaryle, un groupe aryle(alkyle en C₁-C₆), et un groupe hétéroaryle(alkyle en C₁-C₆), lesdits groupes aryles ou hétéroaryles étant substitués par 0 à 2 substituants indépendamment choisis dans le groupe constitué par un groupe alkyle en C₁-C₄, un groupe alcoxy en C₁-C₄, F, CI, Br, CF₃ et NO₂,
en variante, lorsque R^{10e} et R^{11e} sont tous deux des substituants liés au même atome d'azote (comme dans -NR^{10e}R^{11e}), ils peuvent former conjointement avec l'atome d'azote auquel ils sont liés un hétérocycle choisi parmi :
le 3-azabicyclononyle, le 1,2,3,4-tétrahydro-1-quinolinyle, le 1,2,3,4-tétrahydro-2-isoquinolinyle, le 1-pipéridinyle, le 1-morpholinyle, le 1-pyrrolidinyle, le thiamorpholinyle, le thiazolidinyle, et le 1-pipérazinyle ; ledit hétérocycle étant substitué par 0 à 3 groupes choisis parmi :
un groupe alkyle en C₁-C₆, un groupe aryle, un groupe hétéroaryle, un groupe aryle(alkyle en C₁-C₄), un groupe (alkyle en C₁-C₆)carbonyle, un groupe (cycloalkyle en C₃-C₇)carbonyle, un groupe (alcoxy en C₁-C₆)carbonyle, un groupe aryle(alcoxy en C₁-C₄)carbonyle, un groupe alkylsulfonyle en C₁-C₆, et un groupe arylsulfonyle ;
R^{12e} est choisi parmi :
H, un groupe alkyle en C₁-C₆, un groupe triphénylméthyle, un groupe méthoxyméthyle, un groupe méthoxyphényldiphénylméthyle, un groupe triméthylsilyléthoxyméthyle, un groupe (alkyle en C₁-C₆)carbonyle, un groupe (alcoxy en C₁-C₆)carbonyle, un groupe (alkyle en C₁-C₆)aminocarbonyle, un groupe alcényle en C₃-C₆, un groupe cycloalkyle en C₃-C₇, un groupe cycloalkyle(alkyle en C₁-C₄) en C₃-C₇, un groupe aryle, un groupe hétéroaryle(alkyle en C₁-C₆)carbonyle, un groupe hétéroarylcarbonyle, un groupe aryle(alkyle en C₁-C₆), un groupe (alkyle en C₁-C₆)carbonyle, un groupe arylcarbonyle, un groupe alkylsulfonyle en C₁-C₆, un groupe arylsulfonyle, un groupe aryle(alkyle en C₁-C₆)sulfonyle, un groupe hétéroarylsulfonyle, un groupe hétéroaryle(alkyle en C₁-C₆)sulfonyle, un groupe aryloxycarbonyle, et un groupe aryle(alcoxy en C₁-C₆)carbonyle, lesdits groupes aryles étant substitués par 0 à 2 substituants choisis dans le groupe constitué par un groupe alkyle en C₁-C₄, un groupe alcoxy en C₁-C₄, un groupe halogéno, CF₃, et un groupe nitro ;
R^{16e} est choisi parmi :
-C(=O)OR^{18ae}, -C(=O)R^{18be}, -C(=O)N(R^{18be})₂, -C(=O)NHSO₂R^{18ae}, -C(=O)NHC(=O)R^{18be}, -C(=O)NHC(=O)OR^{18ae}, -C(=O)NHSO₂NHR^{18be}, -SO₂R^{18ae}, -SO₂N(R^{18be})₂, et -SO₂NHC(=O)OR^{18be} ;
R^{17e} est choisi parmi :
H, un groupe alkyle en C₁-C₆, un groupe cycloalkyle en C₃-C₇, un groupe cycloalkyle(alkyle en C₁-C₄) en C₃-C₇, un groupe aryle, un groupe aryle(alkyle en C₁-C₆), et un groupe hétéroaryle(alkyle en C₁-C₆) ;
R^{18ae} est choisi parmi :
un groupe alkyle en C₁-C₈ éventuellement substitué par une liaison à Lₙ, un groupe cycloalkyle en C₃-C₁₁ éventuellement substitué par une liaison à Lₙ, un groupe aryle(alkyle en C₁-C₆) éventuellement substitué par une liaison à Lₙ, un groupe hétéroaryle(alkyle en C₁-C₆) éventuellement substitué par une liaison à Lₙ, un groupe (alkyle en C₁-C₆)hétéroaryle éventuellement substitué par une liaison à Lₙ, un groupe biaryle(alkyle en C₁-C₆) éventuellement substitué par une liaison à Lₙ, un groupe hétéroaryle éventuellement substitué par une liaison à Lₙ, un groupe phényle substitué par 3 à 4 R^{19e} et éventuellement substitué par une liaison à Lₙ, un groupe naphtyle substitué par 0 à 4 R^{19e} et éventuellement substitué par une liaison à Lₙ, et une liaison à Lₙ, lesdits groupes aryles ou hétéroaryles étant éventuellement substitués par 0 à 4 R^{19e} ;
R^{18be} représente H ou R^{18ae};
R^{19e} est choisi parmi :
H, un halogène, CF₃, CO₂H, CN, NO₂, -NR^{11e}R^{12e}, OCF₃, un groupe alkyle en C₁-C₈, un groupe alcényle en C₂-C₆, un groupe alcynyle en C₂-C₆, un groupe cycloalkyle en C₃-C₁₁, un groupe cycloalkyle(alkyle en C₁-C₄) en C₃-C₇, un groupe aryle(alkyle en C₁-C₆), un groupe alcoxy en C₁-C₆, un groupe alcoxycarbonyle en C₁-C₄, un groupe aryle, un groupe aryle-O-, un groupe aryle-SO₂-, un groupe hétéroaryle, et un groupe hétéroaryle-SO₂-, lesdits groupes aryles et hétéroaryles étant substitués par 0 à 4 groupes choisis parmi l'hydrogène, un halogène, CF₃, un groupe alkyle en C₁-C₃, et un groupe alcoxy en C₁-C₃;
R^{20e} est choisi parmi les groupes :
hydroxy, alkyloxy en C₁-C₁₀, cycloalkyloxy en C₃-C₁₁, aryloxy, aryl(alkyle en C₁-C₄)oxy, alkylcarbonyloxy(alkyle en C₁-C₂)oxy en C₂-C₁₀, alcoxycarbonyloxy(alkyle en C₁-C₂)oxy en C₂-C₁₀, alcoxycarbonyl(alkyle en C₁-C₂)oxy en C₂-C₁₀, cycloalkylcarbonyloxy(alkyle en C₁-C₂)oxy en C₃-C₁₀, cycloalcoxycarbonyloxy(alkyle en C₁-C₂)oxy en C₃-C₁₀, cycloalcoxycarbonyl(alkyle en C₁-C₂)oxy en C₃-C₁₀, aryloxycarbonyl(alkyle en C₁-C₂)oxy, aryloxycarbonyloxy(alkyle en C₁-C₂)oxy, arylcarbonyloxy(alkyle en C₁-C₂)oxy, alcoxy(alkyle en C₁-C₅)carbonyloxy(alkyle en C₁-C₂)oxy en C₁-C₅, (5-(alkyle en C₁-C₅)-1,3-dioxa-cyclopentèn-2-one-yl)méthyloxy, (5-aryl-1,3-dioxa-cyclopentèn-2-one-yl)méthyloxy, et (R^{10e})(R^{11e})N(alcoxy en C₁-C₁₀) ;
R^{21e} est choisi parmi les groupes :
alkyle en C₁-C₈, alcényle en C₂-C₆, cycloalkyle en C₃-C₁₁, (cycloalkyle en C₃-C₁₁)méthyle, aryle, aryle(alkyle en C₁-C₄), et alkyle en C₁-C₁₀ substitué par 0 à 2 R^{7e};
R^{22e} est choisi parmi :
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, et -C(=O)NHSO₂NHR^{18be} ;
Y^{e} est choisi parmi :
-COR^{20e}, -SO₃H, -PO₃H, -CONHNHSO₂CF₃, -CONHSO₂R^{18ae}, -CONHSO₂NHR^{18be}, -NHCOCF₃, -NHCONHSO₂R^{18ae}, -NHSO₂R^{18ae}, -OPO₃H₂, -OSO₃H, -PO₃H₂, -SO₂NHCOR^{18ae}, -SO₂NHCO₂R^{18ae}, 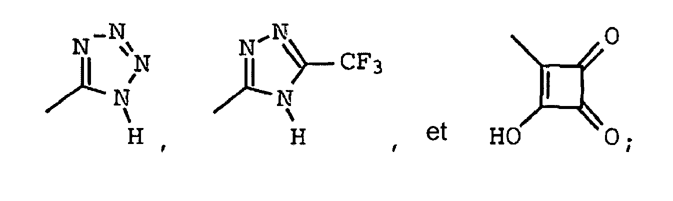
m^{e} est 0-2 ;
n^{e} est 0-4 ;
p^{e} est 0-2 ;
r^{e} est 0-2 ;
avec la clause suivante :
n^{e} et m^{e} sont choisis de telle sorte que le nombre d'atomes reliant R^{1e} et Y^{e} est dans la plage comprise entre 8 et 14 ;
d est choisi parmi 1, 2, 3, 4, 5, 6, 7, 8, 9 et 10 ;
d' est 1-100 ;
Lₙ est un groupe de liaison de formule :
((W)ₕ-(CR⁶R⁷)_{g})ₓ-(Z)ₖ-((CR^{6a}R^{7a})_{g'}-(W)_{h'})_{x'} ;
W est indépendamment choisi à chaque occurrence dans le groupe : O, S, NH, NHC(=O), C(=O)NH, NR⁸C(=O), C(=O)NR⁸, C(=O), C(=O)O, OC(=O), NHC(=S)NH, NHC(=O)NH, SO₂, SO₂NH, (OCH₂CH₂)ₛ, (CH₂CH₂O)_{s'}, (OCH₂CH₂CH₂)_{s"}, (CH₂CH₂CH₂O)ₜ, et (aa)_{t'} ;
aa est indépendamment à chaque occurrence un acide aminé ;
z est choisi dans le groupe :
groupe aryle substitué par 0 à 3 R¹⁰, groupe cycloalkyle en C₃-C₁₀ substitué par 0 à 3 R¹⁰, et hétérocycle à 5-10 chaînons contenant 1 à 4 hétéroatomes indépendamment choisis parmi N, S, et O et substitué par 0 à 3 R¹⁰ ;
R⁶, R^{6a}, R⁷, R^{7a}, et R⁸ sont indépendamment choisis à chaque occurrence dans le groupe :
H, =O, COOH, SO₃H, PO₃H, un groupe alkyle en C₁-C₅ substitué par 0 à 3 R¹⁰, un groupe aryle substitué par 0 à 3 R¹⁰, un groupe benzyle substitué par 0 à 3 R¹⁰, et un groupe alcoxy en C₁-C₅ substitué par 0 à 3 R¹⁰, NHC(=O)R¹¹, C(=O)NHR¹¹, NHC(=O)NHR¹¹, NHR¹¹, R¹¹, et une liaison à Cₕ;
R¹⁰ est indépendamment choisi à chaque occurrence dans le groupe :
une liaison à Cₕ, COOR¹¹, C(=O)NHR¹¹, NHC(=O)R¹¹, OH, NHR¹¹, SO₃H, PO₃H, OPO₃H₂, OSO₃H, un groupe aryle substitué par 0 à 3 R¹¹, un groupe alkyle en C₁-C₅ substitué par 0 à 1 R¹², un groupe alcoxy en C₁-C₅ substitué par 0 à 1 R¹², et un hétérocycle à 5-10 chaînons contenant 1 à 4 hétéroatomes indépendamment choisis parmi N, S, et O et substitué par 0 à 3 R¹¹ ;
R¹¹ est indépendamment choisi à chaque occurrence dans le groupe :
H, -OPO₃H₂, -OSO₃H, un groupe alkyle en C₁-C₅ substitué par 0 à 1 R¹², un groupe aryle substitué par 0 à 1 R¹², un hétérocycle à 5-10 chaînons contenant 1 à 4 hétéroatomes indépendamment choisis parmi N, S, et O et substitué par 0 à 1 R¹², un groupe cycloalkyle en C₃-C₁₀ substitué par 0 à 1 R¹², un polyalkylène glycol substitué par 0 à 1 R¹², un groupe alkyle en C₁-C₅ substitué par un hydrate de carbone substitué par 0 à 1 R¹², une cyclodextrine substituée par 0 à 1 R¹², un acide aminé substitué par 0 à 1 R¹², un groupe polycarboxyalkyle substitué par 0 à 1 R¹², un groupe polyazaalkyle substitué par 0 à 1 R¹², un peptide substitué par 0 à 1 R¹², ledit peptide étant constitué de 2 à 10 acides aminés, un groupe 3,6-O-disulfo-B-D-galactopyranosyle, une bis(phosphonométhyl)glycine, et une liaison à Cₕ ;
R¹² est une liaison à Cₕ ;
k est choisi parmi 0, 1, et 2 ;
h est choisi parmi 0, 1, et 2 ;
h' est choisi parmi 0, 1, et 2 ;
g est choisi parmi 0, 1, 2, 3, 4, 5, 6, 7, 8, 9 et 10 ;
g' est choisi parmi 0, 1, 2, 3, 4, 5, 6, 7, 8, 9 et 10 ;
s est choisi parmi 0, 1, 2, 3, 4, 5, 6, 7, 8, 9 et 10 ;
s' est choisi parmi 0, 1, 2, 3, 4, 5, 6, 7, 8, 9 et 10 ;
s" est choisi parmi 0, 1, 2, 3, 4, 5, 6, 7, 8, 9 et 10 ;
t est choisi parmi 0, 1, 2, 3, 4, 5, 6, 7, 8, 9 et 10 ;
t' est choisi parmi 0, 1, 2, 3, 4, 5, 6, 7, 8, 9 et 10 ;
x est choisi parmi 0, 1, 2, 3, 4, 5, et 6 ;
x' est choisi parmi 0, 1, 2, 3, 4, 5, et 6 ;
Cₕ est un groupement permettant de lier un métal, dont la formule est choisie dans le groupe : 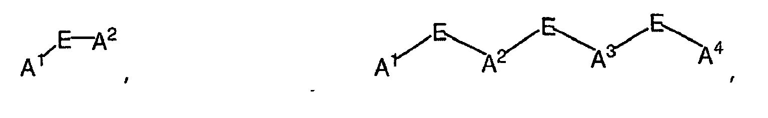 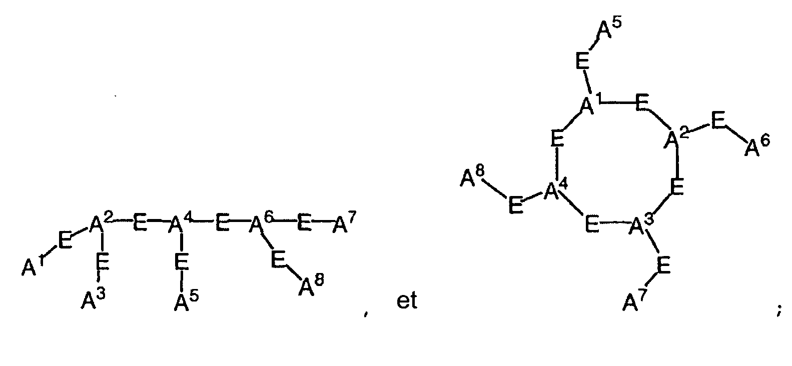
A¹, A², A³, A⁴, A⁵, A⁶, A⁷ et A⁸ sont indépendamment choisis à chaque occurrence dans le groupe :
NR¹³, NR¹³R¹⁴, S, SH, S(Pg), O, OH, PR¹³, PR¹³R¹⁴, P(O)R¹⁵R¹⁶, et une liaison à Lₙ ;
E est une liaison, CH, ou un groupe espaceur indépendamment choisi à chaque occurrence dans le groupe :
un groupe alkyle en C₁-C₁₀ substitué par 0 à 3 R¹⁷, un groupe aryle substitué par 0 à 3 R¹⁷, un groupe cycloalkyle en C₃-C₁₀ substitué par 0 à 3 R¹⁷, un groupe hétérocyclo(C₁-C₁₀)alkyle substitué par 0 à 3 R¹⁷, ledit groupe hétérocyclo étant un hétérocycle à 5-10 chaînons contenant 1 à 4 hétéroatomes indépendamment choisis parmi N, S, et O, un groupe aryle(C₁-C₁₀)alkyle en C₆-C₁₀ substitué par 0 à 3 R¹⁷, un groupe alkyle(C₆-C₁₀)aryle en C₁-C₁₀ substitué par 0 à 3 R¹⁷, et un hétérocycle à 5-10 chaînons contenant 1 à 4 hétéroatomes indépendamment choisis parmi N, S, et O et substitué par 0 à 3 R¹⁷ ;
R¹³ et R¹⁴ sont chacun indépendamment choisis dans le groupe :
une liaison à Lₙ, un hydrogène, un groupe alkyle en C₁-C₁₀ substitué par 0 à 3 R¹⁷, un groupe aryle substitué par 0 à 3 R¹⁷, un groupe cycloalkyle en C₁-C₁₀ substitué par 0 à 3 R¹⁷, un groupe hétérocyclo(C₁-C₁₀)alkyle substitué par 0 à 3 R¹⁷, ledit groupe hétérocyclo étant un hétérocycle à 5-10 chaînons contenant 1 à 4 hétéroatomes indépendamment choisis parmi N, S et O, un groupe aryl(C₁-C₁₀)alkyle en C₆-C₁₀ substitué par 0 à 3 R¹⁷, un groupe alkyl(C₆-C₁₀)aryle en C₁-C₁₀ substitué par 0 à 3 R¹⁷, un hétérocyle à 5-10 chaînons contenant 1 à 4 hétéroatomes indépendamment choisis parmi N, S et O et substitués par 0 à 3 R¹⁷, et un électron, sachant que lorsque l'un parmi R¹³ et R¹⁴ est un électron, alors l'autre est aussi un électron ;
en variante, R¹³ et R¹⁴ se combinent pour former =C(R²⁰)(R²¹) ;
R¹⁵ et R¹⁶ sont chacun indépendamment choisis dans le groupe :
une liaison à Lₙ, -OH, un groupe alkyle en C₁-C₁₀ substitué par 0 à 3 R¹⁷, un groupe aryle substitué par 0 à 3 R¹⁷, un groupe cycloalkyle en C₃-C₁₀ substitué par 0 à 3 R¹⁷, un groupe hétérocyclo(C₁-C₁₀)alkyle substitué par 0 à 3 R¹⁷, ledit groupe hétérocyclo étant un hétérocycle à 5-10 chaînons contenant 1 à 4 hétéroatomes indépendamment choisis parmi N, S, et O, un groupe aryle(C₁-C₁₀)alkyle en C₆-C₁₀ substitué par 0 à 3 R¹⁷, un groupe alkyle(C₆-C₁₀)aryle en C₁-C₁₀ substitué par 0 à 3 R¹⁷, et un hétérocycle à 5-10 chaînons contenant 1 à 4 hétéroatomes indépendamment choisis parmi N, S, et O et substitué par 0 à 3 R¹⁷ ;
R¹⁷ est indépendamment choisi à chaque occurrence dans le groupe :
une liaison à Lₙ, =O, F, Cl, Br, I, -CF₃, -CN, -CO₂R¹⁸, -C(=O)R¹⁸, -C(=O)N(R¹⁸)₂, -CHO, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -SR¹⁸, -S(=O)R^{18a}, -SO₂N(R¹⁸)₂, -N(R¹⁸)₂, -NHC(=S)NHR¹⁸, =NOR¹⁸, NO₂, -C(=O)NHOR¹⁸, -C(=O)NHNR¹⁸R^{18a}, -OCH₂CO₂H, le groupe 2-(1-morpholino)éthoxy, un groupe alkyle en C₁-C₅, un groupe alcényle en C₂-C₄, un groupe cycloalkyle en C₃-C₆, un groupe cycloalkylméthyle en C₃-C₆, un groupe alcoxyalkyle en C₂-C₆, un groupe aryle substitué par 0 à 2 R¹⁸, et un hétérocycle à 5-10 chaînons contenant 1 à 4 hétéroatomes indépendamment choisis parmi N, S et O ;
R¹⁸, R^{18a} et R¹⁹ sont indépendamment choisis à chaque occurrence dans le groupe :
une liaison à Lₙ, H, un groupe alkyle en C₁-C₆, un groupe phényle, un groupe benzyle, un groupe alcoxy en C₁-C₆, un halogénure, un groupe nitro, un groupe cyano, et un groupe trifluorométhyle ;
Pg est un groupe protecteur de thiol ;
R²⁰ et R²¹ sont indépendamment choisis dans le groupe :
H, un groupe alkyle en C₁-C₁₀, -CN, -CO₂R²⁵, -C(=O)R²⁵, -C(=O)N(R²⁵)₂, un 1-alcène en C₂-C₁₀, substitué par 0 à 3 R²³, un 1-alcyne en C₂-C₁₀ substitué par 0 à 3 R²³, un groupe aryle substitué par 0 à 3 R²³, un hétérocycle insaturé à 5-10 chaînons contenant 1 à 4 hétéroatomes indépendamment choisis parmi N, S, et O et substitué par 0 à 3 R²³, et un carbocycle insaturé en C₃-C₁₀ substitué par 0 à 3 R²³ ;
en variante, R²⁰ et R²¹ forment conjointement avec le radical carboné divalent auquel ils sont liés : 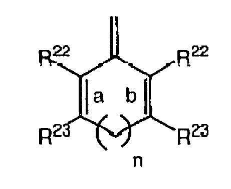
R²² et R²³ sont indépendamment choisis parmi le groupe :
H, R²⁴, un groupe alkyle en C₁-C₁₀ substitué par 0 à 3 R²⁴, un groupe alcényle en C₂-C₁₀ substitué par 0 à 3 R²⁴, un groupe alcynyle en C₂-C₁₀ substitué par 0 à 3 R²⁴, un groupe aryle substitué par 0 à 3 R²⁴, un hétérocycle à 5-10 chaînons contenant 1 à 4 hétéroatomes indépendamment choisis parmi N, S, et O et substitué par 0 à 3 R²⁴, et un carbocycle en C₃-C₁₀ substitué par 0 à 3 R²⁴ ;
en variante, R²² et R²³ forment ensemble un cycle aromatique ou un hétérocycle à 5-10 chaînons contenant 1 à 4 hétéroatomes indépendamment choisis parmi N, S, et O ;
a et b indiquent les positions de doubles liaisons éventuelles, et n vaut 0 ou 1 ;
R²⁴ est indépendamment choisi à chaque occurrence dans le groupe :
=O, F, CI, Br, I, -CF₃, -CN, -CO₂R²⁵, -C(=O)R²⁵, -C(=O)N(R²⁵)₂, -N(R²⁵)₃⁺, -CH₂OR²⁵, -OC(=O)R²⁵, -OC(=O)OR^{25a}, -OR²⁵, -OC(=O)N(R²⁵)₂, -NR²⁶C(=O)R²⁵, -NR²⁶C(=O)OR^{25a}, -NR²⁶C(=O)N(R²⁵)₂, -NR²⁶SO₂N(R²⁵)₂, -NR²⁵SO₂R^{25a}, -SO₃H, -SO₂R^{25a}, -SR²⁵, -S(=O)R^{25a}, -SO₂N(R²⁵)₂, -N(R²⁵)₂, =NOR²⁵, -C(=O)NHOR²⁵, -OCH₂CO₂H, et le groupe 2-(1-morpholino)éthoxy ; et,
R²⁵, R^{25a}, et R²⁶ sont chacun indépendamment choisis à chaque occurrence dans le groupe : un hydrogène et un groupe alkyle en C₁-C₆ ; ou un sel de ceux-ci acceptable sur le plan pharmaceutique.

37. Utilisation selon la revendication 36, dans laquelle Q est un composé de formule (IV) : 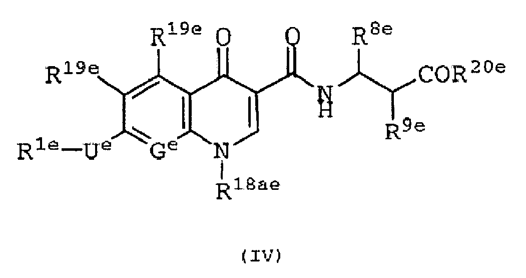 incluant les formes stéréoisomériques de celle-ci, ou les mélanges des formes stéréoisomériques de celle-ci, ou les formes de sel ou de promédicament de celle-ci acceptables sur le plan pharmaceutique, dans laquelle :
R^{1e} est choisi parmi : 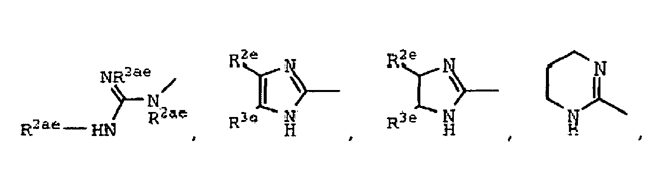 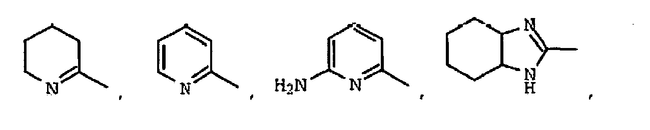 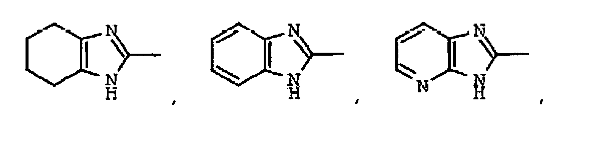 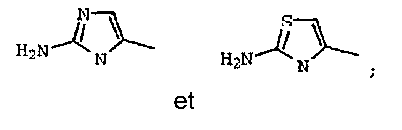
R^{2e} et R^{3e} sont indépendamment choisis parmi :
H, un groupe alcoxy en C₁-C₄, NR^{11e}R^{12e}, un halogène, NO₂, CN, CF₃, un groupe alkyle en C₁-C₆, un groupe alcényle en C₃-C₆, un groupe cycloalkyle en C₃-C₇, un groupe cycloalkyle(alkyle en C₁-C₄) en C₃-C₇, un groupe aryle(alkyle en C₁-C₆), un groupe (alkyle en C₁-C₆)carbonyle, un groupe (alcoxy en C₁-C₆)carbonyle, un groupe arylcarbonyle, et un groupe aryle substitué par 0 à 4 R^{7e},
en variante, lorsque R^{2e} et R^{3e} sont des substituants liés à des atomes adjacents, ils peuvent former conjointement avec les atomes de carbone auxquels ils sont liés un carbocycle à 5-7 chaînons ou un hétérocycle à 5-7 chaînons, aromatique ou non aromatique, ledit carbocycle ou hétérocycle étant substitué par 0 à 2 groupes choisis parmi un groupe alkyle en C₁-C₄, un groupe alcoxy en C₁-C₄, un groupe halogéno, un groupe cyano, un groupe amino, CF₃ et NO₂ ;
R^{2ae} est choisi parmi :
H, un groupe alkyle en C₁-C₁₀, un groupe alcényle en C₂-C₆, un groupe cycloalkyle en C₃-C₁₁, un groupe cycloalkyl(alkyle en C₁-C₄) en C₃-C₇, un groupe aryle, un groupe aryle(alkyle en C₁-C₄), un groupe (alkyle en C₂-C₇)carbonyle, un groupe arylcarbonyle, un groupe (alcoxy en C₂-C₁₀)carbonyle, un groupe cycloalcoxycarbonyle en C₃-C₇, un groupe bicycloalcoxycarbonyle en C₇-C₁₁, un groupe aryloxycarbonyle, un groupe aryl(alcoxy en C₁-C₁₀)carbonyle, un groupe alkylcarbonyloxy(alcoxy en C₁-C₄)carbonyle en C₁-C₆, un groupe arylcarbonyloxy(alcoxy en C₁-C₄)carbonyle, et un groupe cycloalkylcarbonyloxy(alcoxy en C₁-C₄)carbonyle en C₃-C₇ ;
R^{7e} est choisi parmi :
H, un groupe hydroxy, un groupe alkyle en C₁-C₄, un groupe alcoxy en C₁-C₄, un groupe aryle, un groupe aryle(alkyle en C₁-C₄), un groupe (alkyle en C₁-C₄)carbonyle, CO₂R^{18ae}, SO₂R^{11e}, SO₂NR^{10e}R^{11e}, OR^{10e}, et N(R^{11e})R^{12e};
U^{e} est choisi parmi :
-(CH₂)ₙe-, -(CH₂)ₙeO(CH₂)ₘe-, -NH(CH₂)ₙe-, -N(R^{10e})C(=O)-, -NHC(=O)(CH₂)ₙe-, et -C(=O)N(R^{10e})- ;
G^{e} représente N ou CR^{19e} ;
R^{8e} est choisi parmi :
H, CO₂R^{18be}, C(=O)R^{18be}, CONR¹⁷R^{18be}, un groupe alkyle en C₁-C₁₀ substitué par 0 à 1 R^{6e}, un groupe alcényle en C₂-C₁₀ substitué par 0 à 1 R^{6e}, un groupe alcynyle en C₂-C₁₀ substitué par 0 à 1 R^{6e}, un groupe cycloalkyle en C₃-C₈ substitué par 0 à 1 R^{6e}, un groupe cycloalcényle en C₅-C₆ substitué par 0 à 1 R^{6e}, un groupe (alkyle en C₁-C₁₀)carbonyle, un groupe cycloalkyle(alkyle en C₁-C₄) en C₃-C₁₀, un groupe phényle substitué par 0 à 3 R^{6e}, un groupe naphtyle substitué par 0 à 3 R^{6e}, un hétérocycle à 5-10 chaînons contenant 1 à 3 hétéroatomes N, O ou S, ledit hétérocycle pouvant être saturé, partiellement saturé, ou entièrement insaturé, et ledit hétérocycle étant substitué par 0 à 2 R^{7e} ;
R^{9e} est choisi parmi :
un groupe alkyle en C₁-C₁₀ substitué par 0 à 1 R^{6e}, un groupe alcoxy en C₁-C₁₀ substitué par 0 à 2 R^{7e}, H, un groupe nitro, N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, un groupe hydroxy, OR^{22e}, -N(R^{10e})R^{11e}, -N(R^{16e})R^{17e}, un groupe aryl(alkyle en C₀-C₆)carbonyle, un groupe aryle(alkyle en C₁-C₆), un groupe hétéroaryle(alkyle en C₁-C₆), CONR^{18ae}R^{20e}, SO₂R^{18ae}, et SO₂NR^{18ae}R^{20e},
sachant que chacun desdits groupes alkyles, cycloalkyles, aryles ou hétéroaryles peut être non substitué ou substitué de manière indépendante par 1 à 2 R^{7e} ;
R^{6e} est choisi parmi :
H, un groupe alkyle en C₁-C₁₀, un groupe hydroxy, un groupe alcoxy en C₁-C₁₀, un groupe nitro, un groupe alkylcarbonyle en C₁-C₁₀, -N(R^{11e})R^{12e}, un groupe cyano, un groupe halogéno, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚeR^{11e}, SO₂NR^{10e}R^{11e}, un groupe aryle substitué par 0 à 3 groupes choisis parmi un halogène, un groupe alcoxy en C₁-C₆, un groupe alkyle en C₁-C₆, CF₃, S(O)ₘeMe, et -NMe₂, un groupe aryle(alkyle en C₁-C₄), ledit groupe aryle étant substitué par 0 à 3 groupes choisis parmi un halogène, un groupe alcoxy en C₁-C₆, un groupe alkyle en C₁-C₆, CF₃, S(O)ₚeMe, et -NMe₂, et un hétérocycle à 5-10 chaînons contenant 1 à 3 hétéroatomes N, O, ou S, ledit hétérocycle pouvant être saturé, partiellement saturé, ou entièrement insaturé, ledit hétérocycle étant substitué par 0 à 2 R^{7e} ;
R^{10e} est choisi parmi :
H, -OH, CF₃, un groupe alcényle en C₃-C₆, un groupe cycloalkyle en C₃-C₁₁, un groupe aryle, un groupe (cycloalkyle en C₃-C₁₁)méthyle, un groupe aryle(alkyle en C₁-C₄), et un groupe alkyle en C₁-C₁₀ substitué par 0 à 2 R^{6e} ;
R^{11e} est choisi parmi :
H, un groupe hydroxy, un groupe alkyle en C₁-C₈, un groupe alcényle en C₃-C₆, un groupe cycloalkyle en C₃-C₁₁, un groupe (cycloalkyle en C₃-C₁₁)méthyle, un groupe alcoxy en C₁-C₆, un groupe benzyloxy, un groupe aryle, un groupe hétéroaryle, un groupe hétéroaryle(alkyle en C₁-C₄), un groupe aryle(alkyle en C₁-C₄), un groupe adamantylméthyle, et un groupe alkyle en C₁-C₁₀ substitué par 0 à 2 R^{4e} ;
R^{4e} est choisi parmi :
H, un groupe alkyle en C₁-C₆, un groupe cycloalkyle en C₃-C₇, un groupe cycloalkyle(alkyle en C₁-C₄) en C₃-C₇, un groupe aryle, un groupe hétéroaryle, un groupe aryle(alkyle en C₁-C₆), et un groupe hétéroaryle(alkyle en C₁-C₆), lesdits groupes aryles ou hétéroaryles étant substitués par 0 à 2 substituants indépendamment choisis dans le groupe constitué par un groupe alkyle en C₁-C₄, un groupe alcoxy en C₁-C₄, F, CI, Br, CF₃ et NO₂ ;
R^{12e} est choisi parmi :
H, un groupe alkyle en C₁-C₆, un groupe triphénylméthyle, un groupe méthoxyméthyle, un groupe méthoxyphényldiphénylméthyle, un groupe triméthylsilyléthoxyméthyle, un groupe (alkyle en C₁-C₆)carbonyle, un groupe (alcoxy en C₁-C₆)carbonyle, un groupe (alkyle en C₁-C₆)aminocarbonyle, un groupe alcényle en C₃-C₆, un groupe cycloalkyle en C₃-C₇, un groupe cycloalkyle(alkyle en C₁-C₄) en C₃-C₇, un groupe aryle, un groupe hétéroaryle(alkyle en C₁-C₆)carbonyle, un groupe hétéroarylcarbonyle, un groupe aryle(alkyle en C₁-C₆), un groupe (alkyle en C₁-C₆)carbonyle, un groupe arylcarbonyle, un groupe alkylsulfonyle en C₁-C₆, un groupe arylsulfonyle, un groupe aryle(alkyle en C₁-C₆)sulfonyle, un groupe hétéroarylsulfonyle, un groupe hétéroaryle(alkyle en C₁-C₆)sulfonyle, un groupe aryloxycarbonyle, et un groupe aryle(alcoxy en C₁-C₆)carbonyle, lesdits groupes aryles étant substitués par 0 à 2 substituants choisis dans le groupe constitué par un groupe alkyle en C₁-C₄, un groupe alcoxy en C₁-C₄, un groupe halogéno, CF₃, et un groupe nitro ;
R^{16e} est choisi parmi :
-C(=O)OR^{18ae}, -C(=O)R^{18be}, -C(=O)N(R^{18be})₂, -SO₂R^{18ae}, et -SO₂N(R^{18be})₂ ;
R^{17e} est choisi parmi :
H, un groupe alkyle en C₁-C₆, un groupe cycloalkyle en C₃-C₇, un groupe cycloalkyle(alkyle en C₁-C₄) en C₃-C₇, un groupe aryle, un groupe aryle(alkyle en C₁-C₆), et un groupe hétéroaryle(alkyle en C₁-C₆) ;
R^{18ae} est choisi parmi :
un groupe alkyle en C₁-C₈ éventuellement substitué par une liaison à Lₙ, un groupe cycloalkyle en C₃-C₁₁ éventuellement substitué par une liaison à Lₙ, un groupe aryle(alkyle en C₁-C₆) éventuellement substitué par une liaison à Lₙ, un groupe hétéroaryle(alkyle en C₁-C₆) éventuellement substitué par une liaison à Lₙ, un groupe (alkyle en C₁-C₆)hétéroaryle éventuellement substitué par une liaison à Lₙ, un groupe biaryle(alkyle en C₁-C₆) éventuellement substitué par une liaison à Lₙ, un groupe hétéroaryle éventuellement substitué par une liaison à Lₙ, un groupe phényle substitué par 3 à 4 R^{19e} et éventuellement substitué par une liaison à Lₙ, un groupe naphtyle substitué par 0 à 4 R^{19e} et éventuellement substitué par une liaison à Lₙ, et une liaison à Lₙ, lesdits groupes aryles ou hétéroaryles étant éventuellement substitués par 0 à 4 R^{19e} ;
R^{18be} représente H ou R^{18ae} ;
R^{19e} est choisi parmi :
H, un halogène, CF₃, CO₂H, CN, NO₂, -NR^{11e}R^{12e}, OCF₃, un groupe alkyle en C₁-C₈, un groupe alcényle en C₂-C₆, un groupe alcynyle en C₂-C₆, un groupe cycloalkyle en C₃-C₁₁, un groupe cycloalkyle(alkyle en C₁-C₄) en C₃-C₇, un groupe aryle(alkyle en C₁-C₆), un groupe alcoxy en C₁-C₆, un groupe alcoxycarbonyle en C₁-C₄, un groupe aryle, un groupe aryle-O-, un groupe aryle-SO₂-, un groupe hétéroaryle, et un groupe hétéroaryle-SO₂-, lesdits groupes aryles et hétéroaryles étant substitués par 0 à 4 groupes choisis parmi l'hydrogène, un halogène, CF₃, un groupe alkyle en C₁-C₃, et un groupe alcoxy en C₁-C₃ ;
R^{20e} est choisi parmi les groupes :
hydroxy, alkyloxy en C₁-C₁₀, cycloalkyloxy en C₃-C₁₁, aryloxy, aryl(alkyle en C₁-C₄)oxy, alkylcarbonyloxy(alkyle en C₁-C₂)oxy en C₂-C₁₀, alcoxycarbonyloxy(alkyle en C₁-C₂)oxy en C₂-C₁₀, alcoxycarbonyl(alkyle en C₁-C₂)oxy en C₂-C₁₀, cycloalkylcarbonyloxy(alkyle en C₁-C₂)oxy en C₃-C₁₀, cycloalcoxycarbonyloxy(alkyle en C₁-C₂)oxy en C₃-C₁₀, cycloalcoxycarbonyl(alkyle en C₁-C₂)oxy en C₃-C₁₀, aryloxycarbonyl(alkyle en C₁-C₂)oxy, aryloxycarbonyloxy(alkyle en C₁-C₂)oxy, arylcarbonyloxy(alkyle en C₁-C₂)oxy, alcoxy(alkyle en C₁-C₅)carbonyloxy(alkyle en C₁-C₂)oxy en C₁-C₅, (5-(alkyle en C₁-C₅)-1,3-dioxa-cydopentèn-2-one-yl)méthyloxy, (5-aryl-1,3-dioxa-cyclopentèn-2-one-yl)méthyloxy, et (R^{10e})(R^{11e})N(alcoxy en C₁-C₁₀) ;
R^{21e} est choisi parmi les groupes :
alkyle en C₁-C₈, alcényle en C₂-C₆, cycloalkyle en C₃-C₁₁, (cycloalkyle en C₃-C₁₁)méthyle, aryle, aryle(alkyle en C₁-C₄), et alkyle en C₁-C₁₀ substitué par 0 à 2 R^{7e} ;
R^{22e} est choisi parmi :
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, et -C(=O)NHSO₂NHR^{18be} ;
m^{e} est 0-2 ;
ne est 0-4 ; et
p^{e} est 0-2 ;
avec la clause suivante :
ne et m^{e} sont choisis de telle sorte que le nombre d'atomes reliant R¹ et -COR^{20e} dans la formule (IV) est dans la plage comprise entre 8 et 14 ;
d est choisi parmi 1, 2, 3, 4, et 5 ;
d'est 1-50 ;
W est indépendamment choisi à chaque occurrence dans le groupe : O, NH, NHC(=O), C(=O)NH, NR⁸C(=O), C(=O)NR⁸, C(=O), C(=O)O, OC(=O), NHC(=S)NH, NHC(=O)NH, SO₂, (OCH₂CH₂)ₛ, (CH₂CH₂O)_{s'}, (OCH₂CH₂CH₂)_{s"}, (CH₂CH₂CH₂O)ₜ, et (aa)_{t'} ;
aa est indépendamment à chaque occurrence un acide aminé ;
z est choisi dans le groupe :
groupe aryle substitué par 0 à 1 R¹⁰, groupe cycloalkyle en C₃-C₁₀ substitué par 0 à 1 R¹⁰, et hétérocycle à 5-10 chaînons contenant 1 à 4 hétéroatomes indépendamment choisis parmi N, S, et O et substitué par 0 à 1 R¹⁰ ;
R⁶, R^{6a} , R⁷, R^{7a}, et R⁸ sont indépendamment choisis à chaque occurrence dans le groupe :
H, =O, COOH, SO₃H, un groupe alkyle en C₁-C₅ substitué par 0 à 1 R¹⁰, un groupe aryle substitué par 0 à 1 R¹⁰, un groupe benzyle substitué par 0 à 1 R¹⁰, et un groupe alcoxy en C₁-C₅ substitué par 0 à 1 R¹⁰, NHC(=O)R¹¹, C(=O)NHR¹¹, NHC(=O)NHR¹¹, NHR¹¹, R¹¹, et une liaison à Cₕ ;
k vaut 0 ou 1 ;
s est choisi parmi 0, 1, 2, 3, 4, et 5 ;
s' est choisi parmi 0, 1, 2, 3, 4, et 5 ;
s" est choisi parmi 0, 1, 2, 3, 4, et 5 ;
t est choisi parmi 0, 1, 2, 3, 4, et 5 ;
A¹, A², A³, A⁴, A⁵, A⁶, A⁷ et A⁸ sont indépendamment choisis à chaque occurrence dans le groupe :
NR¹³, NR¹³R¹⁴, S, SH, S(Pg), OH, et une liaison à Lₙ ;
E est une liaison, CH, ou un groupe espaceur indépendamment choisi à chaque occurrence dans le groupe :
un groupe alkyle en C₁-C₁₀ substitué par 0 à 3 R¹⁷, un groupe aryle substitué par 0 à 3 R¹⁷, un groupe cycloalkyle en C₃-C₁₀ substitué par 0 à 3 R¹⁷, et un hétérocycle à 5-10 chaînons contenant 1 à 4 hétéroatomes indépendamment choisis parmi N, S, et O et substitué par 0 à 3 R¹⁷ ;
R¹³ et R¹⁴ sont chacun indépendamment choisis dans le groupe :
une liaison à Lₙ, un hydrogène, un groupe alkyle en C₁-C₁₀ substitué par 0 à 3 R¹⁷, un groupe aryle substitué par 0 à 3 R¹⁷, un hétérocyle à 5-10 chaînons contenant 1 à 4 hétéroatomes indépendamment choisis parmi N, S et O et substitués par 0 à 3 R¹⁷, et un électron, sachant que lorsque l'un parmi R¹³ et R¹⁴ est un électron, alors l'autre est aussi un électron ;
en variante, R¹³ et R¹⁴ se combinent pour former =C(R²⁰)(R²¹) ;
R¹⁷ est indépendamment choisi à chaque occurrence dans le groupe :
une liaison à Lₙ, =O, F, CI, Br, I, -CF₃, -CN, -CO₂R¹⁸, -C(=O)R¹⁸, -C(=O)N(R¹⁸)₂, -CH₂OR¹⁸, -OC(=O)R¹⁸, -OC(=O)OR^{18a}, -OR¹⁸, -OC(=O)N(R¹⁸)₂, -NR¹⁹C(=O)R¹⁸, -NR¹⁹C(=O)OR^{18a}, -NR¹⁹C(=O)N(R¹⁸)₂, -NR¹⁹SO₂N(R¹⁸)₂, -NR¹⁹SO₂R^{18a}, -SO₃H, -SO₂R^{18a}, -S(=O)R^{18a}, -SO₂N(R¹⁸)₂, -N(R¹⁸)₂, -NHC(=S)NHR¹⁸, =NOR¹⁸, -C(=O)NHNR¹⁸R^{18a}, -OCH₂CO₂H, et le groupe 2-(1-morpholino)éthoxy ;
R¹⁸, R^{18a} et R¹⁹ sont indépendamment choisis à chaque occurrence dans le groupe :
une liaison à Lₙ, H, et un groupe alkyle en C₁-C₆ ;
R²⁰ et R²¹ sont indépendamment choisis dans le groupe :
H, un groupe alkyle en C₁-C₅, -CO₂R²⁵, un 1-alcène en C₂-C₅ substitué par 0 à 3 R²³, un 1-alcyne en C₂-C₅ substitué par 0 à 3 R²³, un groupe aryle substitué par 0 à 3 R²³, et un hétérocycle insaturé à 5-10 chaînons contenant 1 à 4 hétéroatomes indépendamment choisis parmi N, S, et O et substitué par 0 à 3 R²³ ;
en variante, R²⁰ et R²¹ forment conjointement avec le radical carboné divalent auquel ils sont liés : 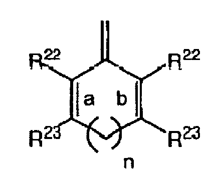
R²² et R²³ sont indépendamment choisis parmi le groupe : H, et R²⁴ ;
en variante, R²² et R²³ forment ensemble un cycle aromatique ou un hétérocycle à 5-10 chaînons contenant 1 à 4 hétéroatomes indépendamment choisis parmi N, S, et O ;
R²⁴ est indépendamment choisi à chaque occurrence dans le groupe : -CO₂R²⁵, -C(=O)N(R²⁵)₂, -CH₂OR²⁵, -OC(=O)R²⁵, -OR²⁵, -SO₃H, -N(R²⁵)₂, et -OCH₂CO₂H ; et,
R²⁵ est indépendamment choisi à chaque occurrence dans le groupe : un hydrogène et un groupe alkyle en C₁-C₃.

38. Utilisation selon la revendication 36, dans laquelle :
R^{1e} est choisi parmi : 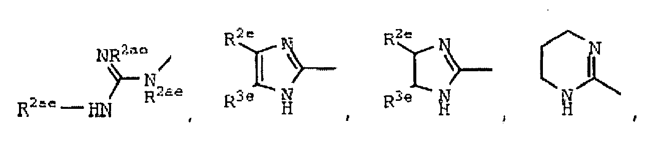 et 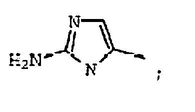
R^{2e} et R^{3e} sont indépendamment choisis parmi :
H, un groupe alcoxy en C₁-C₄, NR^{11e}R^{12e}, un halogène, NO₂, CN, CF₃, un groupe alkyle en C₁-C₆, un groupe alcényle en C₃-C₆, un groupe cycloalkyle en C₃-C₇, un groupe cycloalkyle(alkyle en C₁-C₄) en C₃-C₇, un groupe aryle(alkyle en C₁-C₆), un groupe (alkyle en C₁-C₆)carbonyle, un groupe (alcoxy en C₁-C₆)carbonyle, un groupe arylcarbonyle, et un groupe aryle substitué par 0 à 4 R^{7e},
en variante, lorsque R^{2e} et R^{3e} sont des substituants liés à des atomes adjacents, ils peuvent former conjointement avec les atomes de carbone auxquels ils sont liés un carbocycle à 5-7 chaînons ou un hétérocycle à 5-7 chaînons, aromatique ou non aromatique, ledit carbocycle ou hétérocycle étant substitué par 0 à 2 groupes choisis parmi un groupe alkyle en C₁-C₄, un groupe alcoxy en C₁-C₄, un groupe halogéno, un groupe cyano, un groupe amino, CF₃ et NO₂ ;
R^{2ae} est choisi parmi :
H, un groupe alkyle en C₁-C₁₀, un groupe alcényle en C₂-C₆, un groupe cycloalkyle en C₃-C₁₁, un groupe cycloalkyl(alkyle en C₁-C₄) en C₃-C₇, un groupe aryle, un groupe aryle(alkyle en C₁-C₄), un groupe (alkyle en C₂-C₇)carbonyle, un groupe arylcarbonyle, un groupe (alcoxy en C₂-C₁₀)carbonyle, un groupe cycloalcoxycarbonyle en C₃-C₇, un groupe bicycloalcoxycarbonyle en C₇-C₁₁, un groupe aryloxycarbonyle, un groupe aryl(alcoxy en C₁-C₁₀)carbonyle, un groupe alkylcarbonyloxy(alcoxy en C₁-C₄)carbonyle en C₁-C₆, un groupe arylcarbonyloxy(alcoxy en C₁-C₄)carbonyle, et un groupe cycloalkylcarbonyloxy(alcoxy en C₁-C₄)carbonyle en C₃-C₇ ;
R^{7e} est choisi parmi :
H, un groupe hydroxy, un groupe alkyle en C₁-C₄, un groupe alcoxy en C₁-C₄, un groupe aryle, un groupe aryle(alkyle en C₁-C₄), un groupe (alkyle en C₁-C₄)carbonyle, CO₂R^{18ae}, SO₂R^{11e}, SO₂NR^{10e}R^{11e}, OR^{10e}, et N(R^{11e})R^{12e} ;
U^{e} est choisi parmi :
-(CH₂)ₙe-, -NH(CH₂)ₙe-, -N(R^{10e})C(=O)-, et -NHC(=O)(CH₂)ₙe ;
G^{e} représente N ou CR^{19e} ;
R^{8e} représente H ;
R^{9e} est choisi parmi :
H, un groupe nitro, -N(R^{11e})R^{12e}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, un groupe hydroxy, OR^{22e}, -N(R^{10e})R^{11e}, -N(R^{16e})R^{17e}, un groupe aryl(alkyle en C₀-C₄)carbonyle, aryl(alkyle en C₁-C₄), hétéroaryl(alkyle en C₁-C₄), CONR^{18ae}R^{20e}, SO₂R^{18ae}, et SO₂NR^{18ae}R^{20e}, sachant que chacun desdits groupes alkyles, cycloalkyles, aryles ou hétéroaryles peut être non substitué ou substitué de manière indépendante par 1 à 2 R^{7e} ;
R^{10e} est choisi parmi :
H, -OH, CF₃, un groupe alcényle en C₃-C₆, un groupe cycloalkyle en C₃-C₆, un groupe aryle, un groupe (cycloalkyle en C₃-C₆)méthyle, un groupe aryle(alkyle en C₁-C₄), et un groupe alkyle en C₁-C₄ substitué par 0 à 2 R^{6e} ;
R^{6e} est choisi parmi :
H, un groupe alkyle en C₁-C₄, un groupe hydroxy, un groupe alcoxy en C₁-C₄, un groupe nitro, un groupe alkylcarbonyle en C₁-C₄, -N(R^{11e})R^{12e}, un groupe cyano, un groupe halogéno, CF₃, CHO, CO₂R^{18be}, C(=O)R^{18be}, CONR^{17e}R^{18be}, OC(=O)R^{10e}, OR^{10e}, OC(=O)NR^{10e}R^{11e}, NR^{10e}C(=O)R^{10e}, NR^{10e}C(=O)OR^{21e}, NR^{10e}C(=O)NR^{10e}R^{11e}, NR^{10e}SO₂NR^{10e}R^{11e}, NR^{10e}SO₂R^{21e}, S(O)ₚR^{11e}, SO₂NR^{10e}R^{11e}, un groupe aryle substitué par 0 à 3 groupes choisis parmi un halogène, un groupe alcoxy en C₁-C₄, un groupe alkyle en C₁-C₄, CF₃, S(O)ₘeMe, et -NMe₂, un groupe aryle(alkyle en C₁-C₄), ledit groupe aryle étant substitué par 0 à 3 groupes choisis parmi un halogène, un groupe alcoxy en C₁-C₄, un groupe alkyle en C₁-C₄, CF₃, S(O)ₚeMe, et -NMe₂, et un hétérocycle à 5-10 chaînons contenant 1 à 3 hétéroatomes N, O, ou S, ledit hétérocycle pouvant être saturé, partiellement saturé ou entièrement insaturé, ledit hétérocycle étant substitué par 0 à 2 R^{7e} ;
R^{11e} est choisi parmi :
H, un groupe hydroxy, un groupe alkyle en C₁-C₄, un groupe alcényle en C₃-C₆, un groupe cycloalkyle en C₃-C₆, un groupe (cycloalkyle en C₃-C₆)méthyle, un groupe alcoxy en C₁-C₄, un groupe benzyloxy, un groupe aryle, un groupe hétéroaryle, un groupe hétéroaryle(alkyle en C₁-C₄), un groupe aryle(alkyle en C₁-C₄), un groupe adamantylméthyle, et un groupe alkyle en C₁-C₄ substitué par 0 à 2 R^{4e} ;
R^{4e} est choisi parmi :
H, un groupe alkyle en C₁-C₄, un groupe cycloalkyle en C₃-C₇, un groupe cycloalkyle(alkyle en C₁-C₄) en C₃-C₇, un groupe aryle, un groupe hétéroaryle, un groupe aryle(alkyle en C₁-C₄), et un groupe hétéroaryle(alkyle en C₁-C₄), lesdits groupes aryles ou hétéroaryles étant substitués par 0 à 2 substituants indépendamment choisis dans le groupe constitué par un groupe alkyle en C₁-C₄, un groupe alcoxy en C₁-C₄, F, CI, Br, CF₃ et NO₂,
R^{12e} est choisi parmi :
H, un groupe alkyle en C₁-C₄, un groupe (alkyle en C₁-C₄)carbonyle, un groupe (alcoxy en C₁-C₄)carbonyle, un groupe phényle(alkyle en C₁-C₄), un groupe phénylsulfonyle, un groupe phényloxycarbonyle, un groupe phényle(alcoxy en C₁-C₄)carbonyle, lesdits groupes phényles étant substitués par 0 à 2 substituants choisis dans le groupe constitué par un groupe alkyle en C₁-C₄, un groupe alcoxy en C₁-C₄, un groupe halogéno, CF₃, et un groupe nitro ;
R^{16e} est choisi parmi :
-C(=O)OR^{18ae}, -C(=O)R^{18be}, -C(=O)N(R^{18be})₂, -SO₂R^{18ae}, et -SO₂N(R^{18be})₂ ;
R^{17e} est choisi parmi :
H, un groupe alkyle en C₁-C₄, un groupe cycloalkyle en C₃-C₆, un groupe cycloalkyle(alkyle en C₁-C₄) en C₃-C₆, un groupe aryle, un groupe aryle(alkyle en C₁-C₆), et un groupe hétéroaryle(alkyle en C₁-C₆) ;
R^{18ae} est choisi parmi :
un groupe alkyle en C₁-C₈ éventuellement substitué par une liaison à Lₙ, un groupe cycloalkyle en C₃-C₁₁ éventuellement substitué par une liaison à Lₙ, un groupe aryle(alkyle en C₁-C₆) éventuellement substitué par une liaison à Lₙ, un groupe hétéroaryle(alkyle en C₁-C₆) éventuellement substitué par une liaison à Lₙ, un groupe (alkyle en C₁-C₆)hétéroaryle éventuellement substitué par une liaison à Lₙ, un groupe biaryle(alkyle en C₁-C₆) éventuellement substitué par une liaison à Lₙ, un groupe hétéroaryle éventuellement substitué par une liaison à Lₙ, un groupe phényle substitué par 3 à 4 R^{19e} et éventuellement substitué par une liaison à Lₙ, un groupe naphtyle substitué par 0 à 4 R^{19e} et éventuellement substitué par une liaison à Lₙ, et une liaison à Lₙ, lesdits groupes aryles ou hétéroaryles étant éventuellement substitués par 0 à 4 R^{19e} ;
R^{18be} représente H ou R^{18ae} ;
R^{19e} est choisi parmi :
H, un halogène, CF₃, CO₂H, CN, NO₂, -NR^{11e}R^{12e}, OCF₃, un groupe alkyle en C₁-C₆, un groupe alcényle en C₂-C₆, un groupe alcynyle en C₂-C₆, un groupe cycloalkyle en C₃-C₆, un groupe cycloalkyle(alkyle en C₁-C₄) en C₃-C₆, un groupe aryle(alkyle en C₁-C₄), un groupe alcoxy en C₁-C₆, un groupe alcoxycarbonyle en C₁-C₄, un groupe aryle, un groupe aryle-O-, un groupe aryle-SO₂-, un groupe hétéroaryle, et un groupe hétéroaryle-SO₂-, lesdits groupes aryles et hétéroaryles étant substitués par 0 à 4 groupes choisis parmi l'hydrogène, un halogène, CF₃, un groupe alkyle en C₁-C₃, et un groupe alcoxy en C₁-C₃ ;
R^{20e} est choisi parmi les groupes :
hydroxy, alkyloxy en C₁-C₆, cycloalkyloxy en C₃-C₆, aryloxy, aryl(alkyle en C₁-C₄)oxy, alkylcarbonyloxy(alkyle en C₁-C₂)oxy en C₂-C₁₀, alcoxycarbonyloxy(alkyle en C₁-C₂)oxy en C₂-C₁₀, alcoxycarbonyl(alkyle en C₁-C₂)oxy en C₂-C₁₀, cycloalkylcarbonyloxy(alkyle en C₁-C₂)oxy en C₃-C₁₀, cycloalcoxycarbonyloxy(alkyle en C₁-C₂)oxy en C₃-C₁₀, cycloalcoxycarbonyl(alkyle en C₁-C₂)oxy en C₃-C₁₀, aryloxycarbonyl(alkyle en C₁-C₂)oxy, aryloxycarbonyloxy(alkyle en C₁-C₂)oxy, arylcarbonyloxy(alkyle en C₁-C₂)oxy, alcoxy(alkyle en C₁-C₅)carbonyloxy(alkyle en C₁-C₂)oxy en C₁-C₅, (5-(alkyle en C₁-C₅)-1,3-dioxa-cyclopentèn-2-one-yl)méthyloxy, (5-aryl-1,3-dioxa-cyclopentèn-2-one-yl)méthyloxy, et (R^{10e})(R^{11e})N(alcoxy en C₁-C₁₀) ;
R^{21e} est choisi parmi les groupes :
alkyle en C₁-C₄, alcényle en C₂-C₆, cycloalkyle en C₃-C₆, (cycloalkyle en C₃-C₆)méthyle, aryle, aryle(alkyle en C₁-C₄), et alkyle en C₁-C₁₀ substitué par 0 à 2 R^{7e} ;
R^{22e} est choisi parmi :
-C(=O)-R^{18be}, -C(=O)N(R^{18be})₂, -C(=O)NHSO₂R^{18ae}, -C(=O)NHC(=O)R^{18be}, -C(=O)NHC(=O)OR^{18ae}, et -C(=O)NHSO₂NHR^{18be} ;
m^{e} est 0-2 ;
n^{e} est 0-4 ;
p^{e} est 0-2 ;
Cₕ est 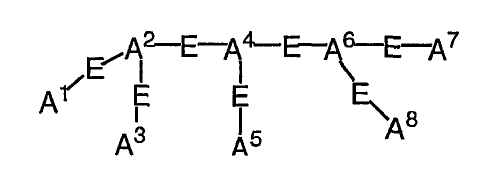
A¹ est choisi dans le groupe :
OH, et une liaison à Lₙ ;
A², A⁴ et A⁶ sont chacun N ;
A³, A⁵ et A⁸ sont chacun OH ;
A⁷ est une liaison à Lₙ ou une liaison NH à Lₙ ;
E est un groupe alkyle en C₂ substitué par 0 à 1 R¹⁷ ;
R¹⁷ représente =O ;
en variante, Cₕ représente 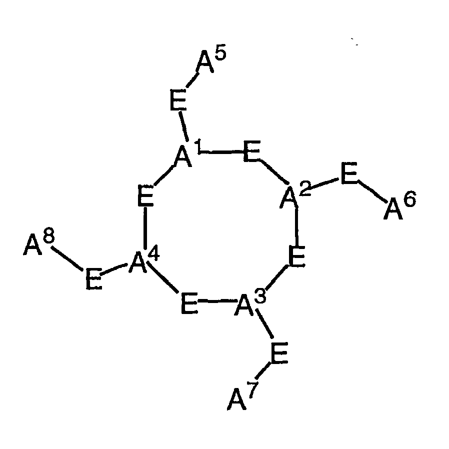
A¹ est choisi dans le groupe :
OH, et une liaison à Lₙ ;
A², A³ et A⁴ sont chacun N ;
A⁵, A⁶ et A⁸ sont chacun OH ;
A⁷ est une liaison à Lₙ ;
E est un alkyle en C₂ substitué par 0 à 1 R¹⁷ ;
R¹⁷ représente =O ;
en variante, Cₕ est 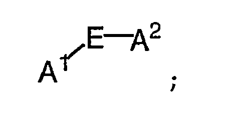
A¹ est NH₂ ou N=C(R²⁰)(R²¹) ;
E est une liaison ;
A² est NHR¹³;
R¹³ est un hétérocycle substitué par R¹⁷, ledit hétérocycle étant choisi parmi la pyridine et la pyrimidine ;
R¹⁷ est choisi parmi une liaison à Lₙ, C(=O)NHR¹⁸ et C(=O)R¹⁸ ;
R¹⁸ est une liaison à Lₙ ;
R²⁴ est choisi dans le groupe :
-CO₂R²⁵, -OR²⁵, -SO₃H, et -N(R²⁵)₂ ; et,
R²⁵ est indépendamment choisi à chaque occurrence dans le groupe : hydrogène et groupe méthyle.

39. Utilisation selon la revendication 36, dans laquelle Q est choisi dans le groupe :
acide 3-[7-[(imidazolin-2-ylamino)méthyl]-1-méthyl-6,8-difluoroquinoline-4-one-3-ylcarbonylamino]-2-(3,5-diméthylisoxazol-4-ylsulfonylamino)propionique,
acide 3-[7-[(imidazolin-2-ylamino)méthyl]-1-méthyl-6,8-difluoroquinoline-4-one-3-ylcarbonylamino]-2-(benzyloxycarbonylamino)propionique,
acide 3-[7-[(imidazolin-2-ylamino)méthyl]-1-méthyl-6,8-difluoroquinoline-4-one-3-ylcarbonylamino]-2-(n-butyloxycarbonylamino)propionique,
acide 3-[7-[(imidazolin-2-ylamino)méthyl]-1-méthyl-6,8-difluoroquinoline-4-one-3-ylcarbonylamino]-2-(n-butylsulfonylamino)propionique,
acide 3-[7-[(tétrahydropyrimid-2-ylamino)méthyl)-1-méthyl-6,8-difluoroquinoline-4-one-3-ylcarbonylamino]-2-(benzyloxycarbonylamino)propionique,
acide 3-[7-[(tétrahydropyrimid-2-ylamino)méthyl]-1-méthyl-6,8-difluoroquinoline-4-one-3-ylcarbonylamino]-2-(n-butyloxycarbonylamino)propionique,
acide 3-[7-[(tétrahydropyrimid-2-ylamino)méthyl]-1-méthyl-6,8-difluoroquinoline-4-one-3-ylcarbonylamino]-2-(phénylsulfonylamino)propionique,
acide 3-[7-[(tétrahydropyrimid-2-ylamino)méthyl]-1-méthyl-6,8-difluoroquinoline-4-one-3-ylcarbonylamino]-2-(n-butylsulfonyl)aminopropionique,
acide 3-[7-[(2-aminothiazol-4-yl)méthyl]-1-méthyl-6,8-difluoroquinoline-4-one-3-ylcarbonylamino]-2-(benzyloxycarbonylamino)propionique,
acide 3-[7-[(imidazolin-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(tétrahydropyrimid-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-méthyl-6,8-difluoroquinoline-4-one-3-ylcarbonylamino]-2-(3,5-diméthylisoxazol-4-ylsulfonylamino)propionique,
acide 3-[7-[(imidazol-2-ylamino)méthyl]-1-méthyl-6,8-difluoroquinoline-4-one-3-ylcarbonylamino]-2-(benzyloxycarbonylamino)propionique,
acide 3-[7-[(imidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-méthyl-6,8-difluoroquinoline-4-one-3-ylcarbonylamino]-2-((4-biphényl)sulfonylamino)propionique,
acide 3-[7-[(imidazol-2-ylamino)méthyl]-1-méthyl-6,8-difluoroquinoline-4-one-3-ylcarbonylamino]-2-(1-naphtylsulfonylamino)propionique,
acide 3-[7-[(benzimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(4-méthylimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(4,5-diméthylimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(4,5,6,7-tétrahydrobenzimidazol-2-ylamino)méthyl]-1-méthyl-6,8-difluoroquinoline-4-one-3-ylcarbonylamino]-2-((2,4,6-triméthylphényl)sulfonylamino)propionique,
acide 3-[7-[(pyridin-2-ylamino)méthyl]-1-méthyl-6,8-difluoroquinoline-4-one-3-ylcarbonylamino]-2-((2,4,6-triméthylphényl)sulfonylamino)propionique,
acide 3-[7- (2-aminopyridin-6-yl)-1-méthyl-6,8-difluoroquinoline-4-one-3-ylcarbonylamino]-2-((2,4,6-triméthylphényl)sulfonylamino)propionique,
acide 3-[7-[(7-azabenzimidazol-2-yl)méthyl]-1-méthyl-6,8-difluoroquinoline-4-one-3-ylcarbonylamino]-2-((2,4,6-triméthylphényl)sulfonylamino)propionique,
acide 3-[7-[(benzimidazol-2-ylamino)méthyl]-1-(2-phényléthyl)-6,8-difluoroquinoline-4-one-3-ylcarbonylamino]propionique,
acide 3-[7-[(pyridin-2-ylamino)méthyl]-1-(2-phényléthyl)-6,8-difluoroquinoline-4-one-3-ylcarbonylamino]propionique,
acide 3-[7-[(imidazolin-2-ylamino)méthyl]-1-(2-phényléthyl)-6,8-difluoroquinoline-4-one-3-ylcarbonylamino]propionique,
acide 3-[7-[(imidazol-2-ylamino)méthyl]-1-(2-phényléthyl)-6,8-difluoroquinoline-4-one-3-ylcarbonylamino]propionique,
acide 3-[7-[(imidazolin-2-ylamino)méthyl]-1-(2-phényléthyl)-6,8-difluoroquinoline-4-one-3-ylcarbonylamino]-2-(benzyloxycarbonylamino)propionique,
acide 3-[7-[(imidazolin-2-ylamino)méthyl]-1-(2-phényléthyl)-6,8-difluoroquinoline-4-one-3-ylcarbonylamino]-2-(n-butyloxycarbonylamino)propionique,
acide 3-[7-[(imidazolin-2-ylamino)méthyl]-1-(2-phényléthyl)-6,8-difluoroquinoline-4-one-3-ylcarbonylamino]-2-(phénylsulfonylamino)propionique,
acide 3-[7-[(imidazolin-2-ylamino)méthyl]-1-(2-phényléthyl)-6,8-difluoroquinoline-4-one-3-ylcarbonylamino]-2-(n-butylsulfonylamino)propionique,
acide 3-[7-[(tétrahydropyrimid-2-ylamino)méthyl]-1-(2-phényléthyl)-6,8-difluoroquinoline-4-one-3-ylcarbonylamino]-2-(benzyloxycarbonylamino)propionique,
acide 3-[7-[(tétrahydropyrimid-2-ylamino)méthyl]-1-(2-phényléthyl))-6,8-difluoroquinoline-4-one-3-ylcarbonylamino]-2-(n-butyloxycarbonylamino)propionique,
acide 3-[7-[(tétrahydropyrimid-2-ylamino)méthyl]-1-(2-phényléthyl)-6,8-difluoroquinoline-4-one-3-ylcarbonylamino]-2-(phénylsulfonylamino)propionique,
acide 3-[7-[(tétrahydropyrimid-2-ylamino)méthyl]-1-(2-phényléthyl)-6,8-difluoroquinoline-4-one-3-ylcarbonylamino]-2-(n-butylsulfonyl)aminopropionique,
acide 3-[7-[(2-aminothiazol-4-yl)méthyl]-1-(2-phényléthyl)-6,8-difluoroquinoline-4-one-3-ylcarbonylamino]-2-(phénylsulfonylamino)propionique,
acide 3-[7-[(2-aminothiazol-4-yl)méthyl]-1-(2-phényléthyl)-6,8-difluoroquinoline-4-one-3-ylcarbonylamino]-2-(benzyloxycarbonylamino)propionique,
acide 3-[7-[(imidazolin-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(tétrahydropyrimid-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-(2-phényléthyl)-6,8-difluoroquinoline-4-one-3-ylcarbonylamino]-2-(benzyloxycarbonylamino)propionique,
acide 3-[7-[(imidazol-2-ylamino)méthyl]-I-(2-phényléthyl)-6,8-difluoroquinoline-4-one-3-ylcarbonylamino]-2-(phénylsulfonylamino)propionique,
acide 3-[7-[(imidazol-2-ylamino)méthyl]-1-(2-phényléthyl)-6,8-difluoroquinoline-4-one-3-ylcarbonylamino]-2-((2,6-dichlorophényl)sulfonylamino)propionique,
acide 3-[7-[(imidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(imidazol-2-ylamino)méthyl]-1-(2-phényléthyl)-6,8-difluoroquinoline-4-one-3-ylcarbonylamino]-2-((4-biphényl)sulfonylamino)propionique,
acide 3-[7-[(benzimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(4-méthylimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(4,5-diméthylimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(4,5,6,7-tétrahydrobenzimidazol-2-ylamino)méthyl]-1-(2-phényléthyl)-6,8-difluoroquinoline-4-one-3-ylcarbonylamino]-2-((2,4,6-triméthylphényl)sulfonylamino)propionique,
acide 3-[7-[(pyridin-2-ylamino)méthyl]-1-(2-phényléthyl)-6,8-difluoroquinoline-4-one-3-ylcarbonylamino]-2-((2,4,6-triméthylphényl)sulfonylamino)propionique, acide 3-[7-(2-aminopyridin-6-yl)-1-(2-phényléthyl)-6,8-difluoroquinoline-4-one-3-ylcarbonylamino]-2-((2,4,6-triméthylphényl)sulfonylamino)propionique, et
acide 3-[7-(7-azabenzimidazol-2-yl)méthyl]-1-(2-phényléthyl)-6,8-difluoroquinoline-4-one-3-ylcarbonylamino]-2-((2,4,6-triméthylphényl)sulfonylamino)propionique.

40. Utilisation selon la revendication 34, dans laquelle le radioisotope est ¹⁵³Sm.

41. Utilisation selon la revendication 34, dans laquelle le radioisotope est ¹⁷⁷Lu.

42. Utilisation selon la revendication 41, dans laquelle l'agent radiopharmaceutique est sélectionné dans le groupe : 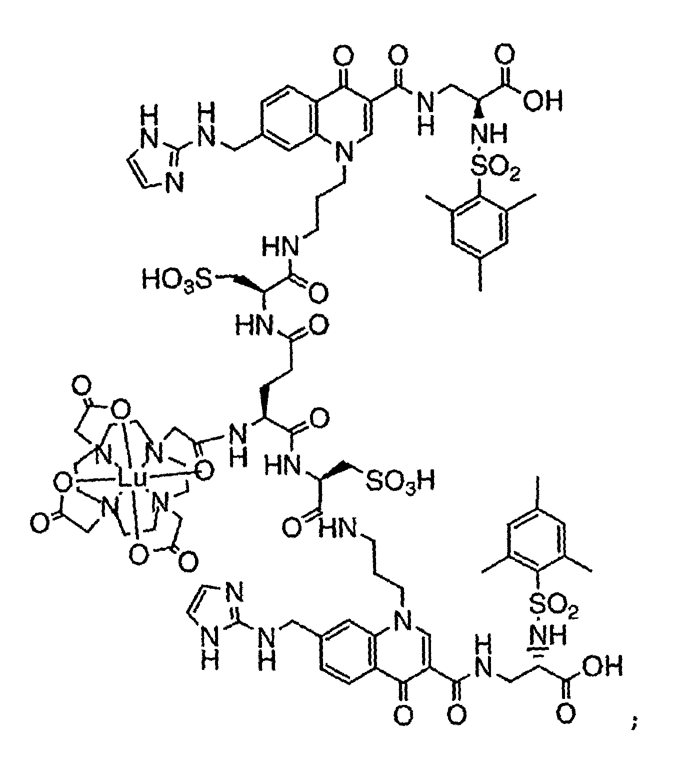 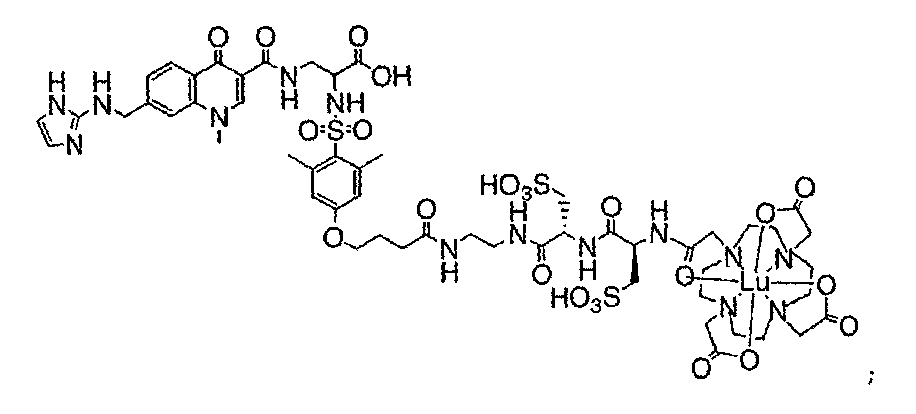 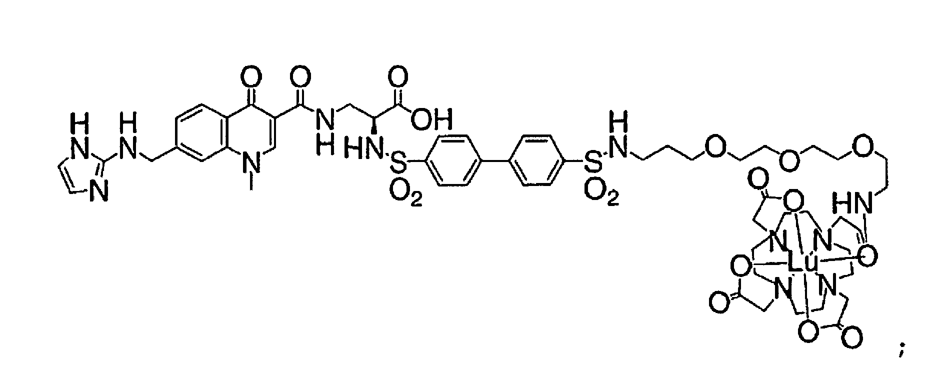 et

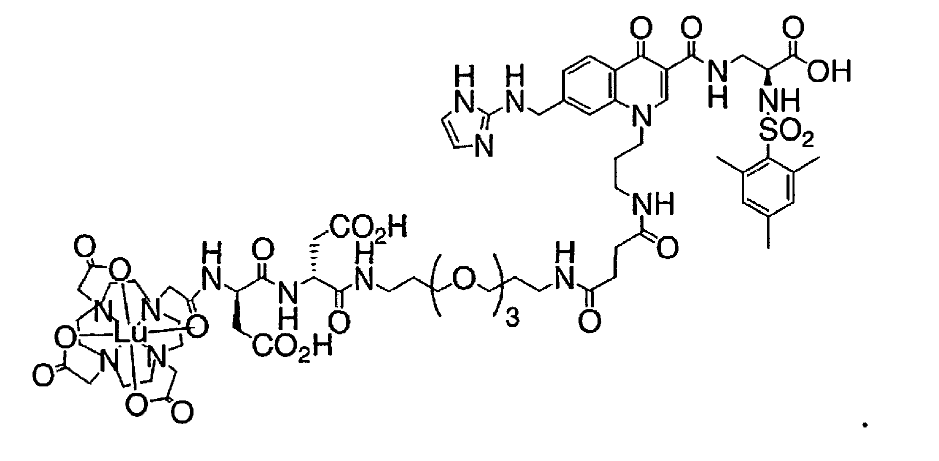

43. Utilisation selon la revendication 34, dans laquelle le radioisotope est ⁹⁰Y;

44. Utilisation selon la revendication 43, dans laquelle l'agent radiopharmaceutique est choisi dans le groupe : 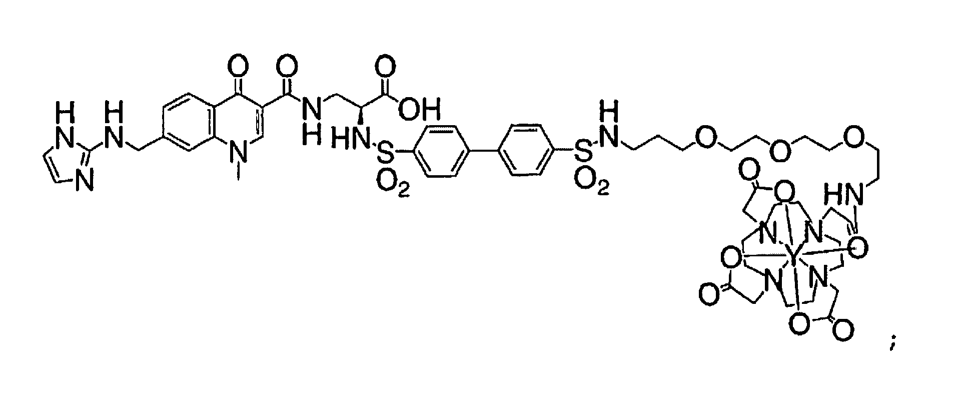 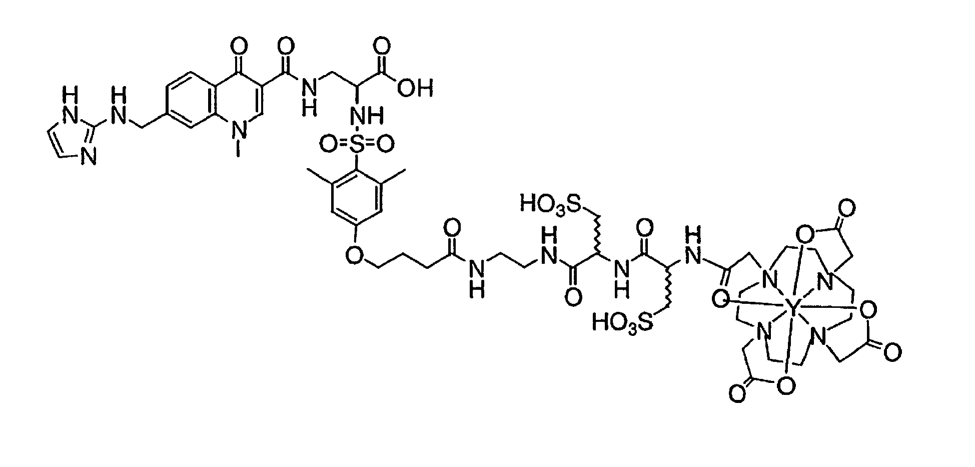 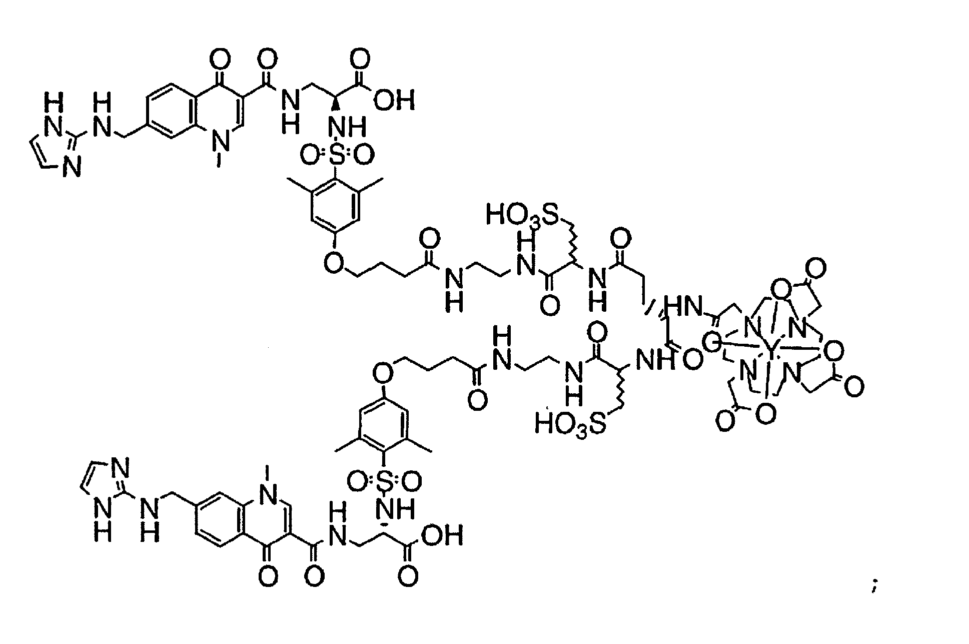 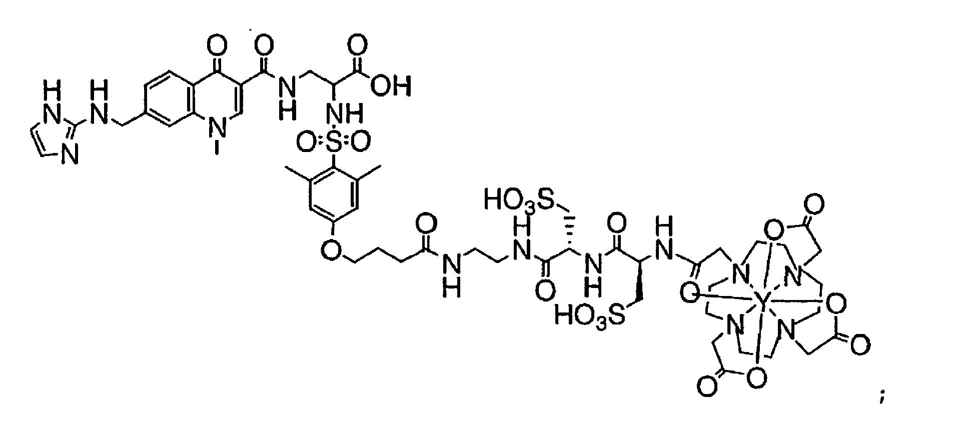 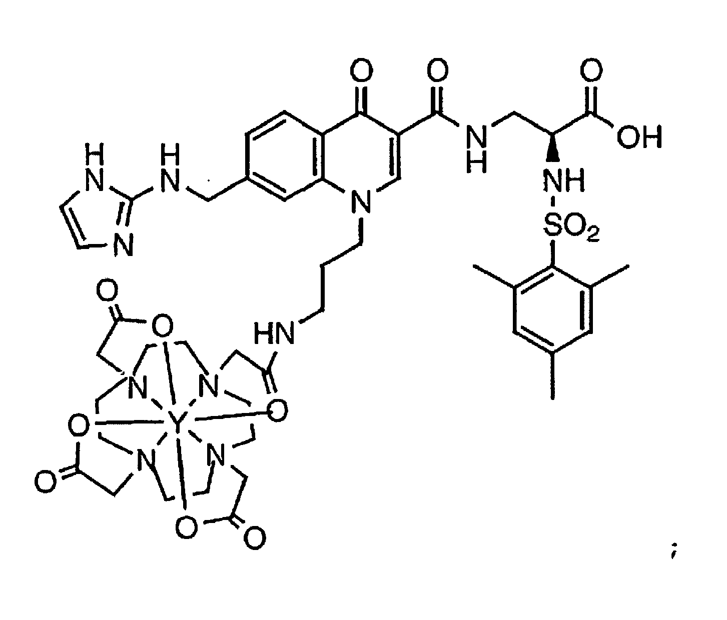 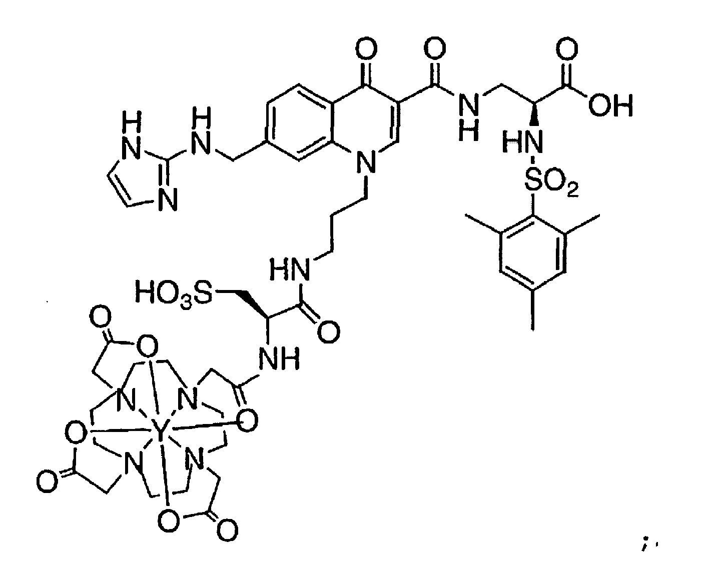 et

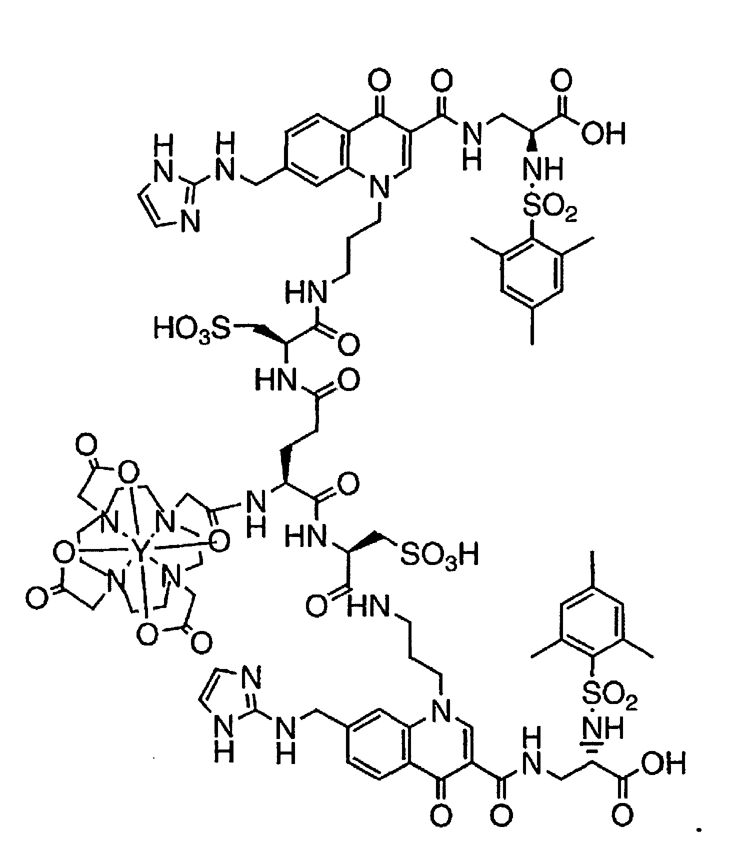

45. Utilisation selon la revendication 26 comprenant en outre le traitement du cancer par brachythérapie, rayonnement externe, thérapie au laser ou retrait chirurgical.

46. Trousse comprenant un matériau d'emballage et une composition radiopharmaceutique thérapeutique selon la revendication 12, contenue au sein dudit matériau d'emballage, dans laquelle le matériau d'emballage comprend une étiquette ou un insert d'emballage qui indique que ladite composition radiopharmaceutique thérapeutique peut être utilisée pour traiter un cancer.

47. Composition radiopharmaceutique thérapeutique selon la revendication 12, comprenant en outre un agent de photosensibilisation.

48. Composition radiopharmaceutique thérapeutique selon la revendication 47, dans laquelle l'agent de photosensibilisation est sélectionné dans le groupe constitué par : la photofrine ; des agents de photosensibilisation à base de naphtalocyanine ; des agents de photosensibilisation à base de tétrapyrrole ; des porphyrines ; des chlorines ; des phtalocyanines ; des naphtalocyanines ; des coumarines ; des psoralènes ; la 1,3,4,6-tétraméthoxyhélianthrone ; la 10,13-diméthyl-1,3,4,6-tétrahydroxyhélianthrone ; la 10,13-di(méthoxycarbonyl)-1,3,4,6-tétraméthoxyhélianthrone ; la 1,6-di-N-butylamino-3,4-diméthoxyhélianthrone ; la 1,6-di-N-butylamino-3,4-diméthoxy-10,13-diméthylhélianthrone ; la 1,6-di-(N-hydroxyéthylamino)-3,4-diméthoxyhélianthrone ; la 2,5-dibromo-1,3,4,6-tétrahydroxyhélianthrone ; et la 2,5-dibromo-10,13-diméthyl-1,3,4,6-tétrahydroxyhélianthrone.

49. Trousse selon la revendication 46, comprenant en outre un agent de photosensibilisation.

50. Trousse selon la revendication 49, dans laquelle l'agent de photosensibilisation est sélectionné dans le groupe constitué par : la photofrine ; des agents de photosensibilisation à base de naphtalocyanine ; des agents de photosensibilisation à base de tétrapyrrole ; des porphyrines ; des chlorines ; des phtalocyanines ; des naphtalocyanines ; des coumarines ; des psoralènes ; la 1,3,4,6-tétraméthoxyhélianthrone ; la 10,13-diméthyl-1,3,4,6-tétrahydroxyhélianthrone ; la 10,13-di(méthoxycarbonyl)-1,3,4,6-tétraméthoxyhélianthrone ; la 1,6-di-N-butylamino-3,4-diméthoxyhélianthrone ; la 1,6-di-N-butylamino-3,4-diméthoxy-10,13-diméthylhélianthrone ; la 1,6-di-(N-hydroxyéthylamino)-3,4-diméthoxyhélianthrone ; la 2,5-dibromo-1,3,4,6-tétrahydroxyhélianthrone ; et la 2,5-dibromo-10,13-diméthyl-1,3,4,6-tétrahydroxyhélianthrone.

51. Utilisation selon la revendication 26, comprenant en outre le traitement du patient avec une thérapie photodynamique.

52. Utilisation selon la revendication 51, dans laquelle la thérapie photodynamique comprend les étapes suivantes :
(a) administrer un radiopharmaceutique thérapeutique de la présente invention et d'un agent photosensible (agent photoréactif) à un patient, ledit agent photosensible présentant une gamme de longueurs d'onde d'absorption de lumière caractéristique et étant de préférence absorbé par un tissu anormal ;
(b) fournir un dispositif d'imagerie intégral comportant plusieurs sources lumineuses et produisant un signal utilisé pour imager un tissu anormal au niveau du site de traitement interne, lesdites sources lumineuses émettant de la lumière dans une gamme de longueurs d'onde qui correspond à la gamme de longueurs d'onde caractéristique de l'agent photosensible, ladite gamme de longueurs d'onde comprenant des longueurs d'onde suffisamment longues pour pénétrer par la couche du derme du patient vers le site de traitement interne ;
(c) déterminer un emplacement du tissu anormal au niveau du site interne ciblé au sein du corps du patient à l'aide du dispositif d'imagerie, en visualisant une image du tissu anormal au niveau du site développée en réponse au signal produit par le dispositif d'imagerie ; et
(d) alimenter les sources lumineuses pour administrer une luminothérapie vers le site interne ciblé, au niveau de l'emplacement déterminé avec le dispositif d'imagerie.

53. Utilisation selon la revendication 52, dans laquelle l'agent photosensible (agent photoréactif) est ciblé de façon spécifique au niveau du tissu ciblé en incluant un agent de liaison qui lie de façon sélective l'agent photosensible au tissu ciblé.

54. Utilisation selon la revendication 52, dans laquelle l'agent de photosensibilisation est sélectionné dans le groupe constitué par : la photofrine ; des agents de photosensibilisation à base de naphtalocyanine ; des agents de photosensibilisation à base de tétrapyrrole ; des porphyrines ; des chlorines ; des phtalocyanines ; des naphtalocyanines ; des coumarines ; des psoralènes ; la 1,3,4,6-tétraméthoxyhélianthrone ; la 10,13-diméthyl-1,3,4,6-tétrahydroxyhélianthrone ; la 10,13-di(méthoxycarbonyl)-1,3,4,6-tétraméthoxyhélianthrone ; la 1,6-di-N-butylamino-3,4-diméthoxyhélianthrone ; la 1,6-di-N-butylamino-3,4-diméthoxy-10,13-diméthylhélianthrone ; la 1,6-di-(N-hydroxyéthylamino)-3,4-diméthoxyhélianthrone ; la 2,5-dibromo-1,3,4,6-tétrahydroxyhélianthrone ; et la 2,5-dibromo-10,13-diméthyl-1,3,4,6-tétrahydroxyhélianthrone.
